# EUROPEAN PATENT APPLICATION

(11) **EP 4 155 296 A1**
(43) Date of publication of application: **29.03.2023**
(21) Application number: 21199013.0
(22) Date of filing: 24.09.2021
(51) Int. Cl.: C07D 273/00, A61P 35/00, A61K 31/33

(54) **MACROCYCLIC COMPOUNDS AS PROTEASOME SUBUNIT BETA TYPE-5 INHIBITORS**

(71) Applicant: Lead Discovery Center GmbH, 44227 Dortmund (DE); QLi5 Therapeutics GmbH, 44227 Dortmund (DE)
(72) Inventor: The designation of the inventor has not yet been filed
(74) Representative: Arth, Hans-Lothar

(57) **Abstract**

The present invention relates to certain macrocyclic compounds of the formula (I) and pharmaceutically acceptable salts thereof. These compounds are useful in the treatment or prevention of a disease associated with and/or caused by proteasome or immunoproteasome, selected from a cancer, an infectious disease, an inflammatory diease, and autoimmune disease.

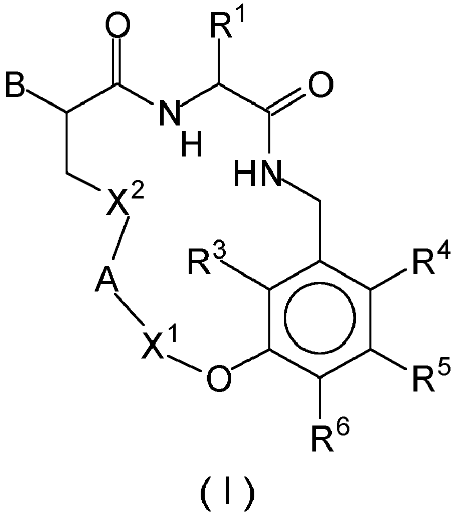

## Description

The present invention relates to certain macrocyclic compounds of the formula (I) and pharmaceutically acceptable salts thereof. These compounds are useful in the treatment or prevention of a disease treatable by proteasome inhibition, selected from cancer, infectious diseases, inflammatory dieases, and autoimmune diseases.

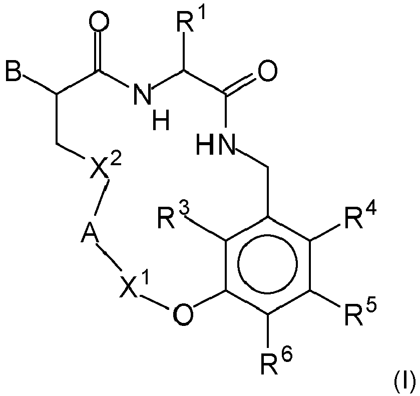

### Background of the invention

The proteasome is a multicatalytic proteinase complex with a highly ordered ringshaped 20S core structure. The core structure is composed of 4 rings of 28 non-identical subunits; 2 rings are composed of 7 alpha subunits and 2 rings are composed of 7 beta subunits. Proteasomes are distributed throughout eukaryotic cells at a high concentration and cleave peptides in an ATP/ubiquitin-dependent process in a non-lysosomal pathway.. Two isoforms of the proteasome are the constitutive proteasome and the immunoproteasome which is constitutively expressed in hematopoietic cells and can be induced in non-immune cells by inflammatory cytokines or oxidadtive stress. Both differ from each other in the subunit composition. The constitutive proteasome has three subunits with chymotrypsin-like (beta type-5), caspase-like (beta type-1) and trypsin-like (beta type-2) enzymatic activity. In the immunoproteasome these subunits are replaced by the beta type-5i, beta type-1i and beta type-2i catalytic subunits, which have different substrate preferences and an increased proteolytic activity compared to the corresponding subunits in the constitutive proteasome. An essential function of the immunoproteasome, is the processing of class I MHC peptides for antigen presentation.

The proteasomes form a pivotal component for the ubiquitin-proteasome system (UPS) and corresponding cellular Protein Quality Control (PQC). Protein ubiquitination and subsequent proteolysis and degradation by the proteasome are important mechanisms in the regulation of the cell cycle, cell growth and differentiation, gene transcription, signal transduction and apoptosis.

The proteasome plays a central role in the cellular protein degradation pathway. Protein degradation is of particular importance for cancer cells with a high protein turnover, which is usually associated with the formation of misfolded proteins. Inhibition of the proteasome in such cells leads to an accumulation of misfolded proteins activating the caspase cascade and thus contributing to apoptosis. Alternative events induced by proteasome inhibition contributing to induce cell death are: Inhibition of the pro-survival NFkB pathway and induction of pro-apoptotic proteins. Thus proteasome inhibition leads to apoptosis of cells with a high protein turnover such as tumor and myeloma cells. Also plasma cells which have a high protein turnover due to the constant secretion of antibodies show sensitivity to proteasome inhibition. Accordingly proteasome inhibition has been shown to have a signficant effect on autoantibody mediated autoimmune diseases such as systemic lupus erythematosus (SLE) or myasthenia gravis (MG). Since proteasome inhibitors are effective at targeting antibody producing B-cells they are also evaluated as therapies in antibody-mediated allograft rejection. The inhibition of the protozoan proteasome by proteasome inhibitors has been shown to be effective in treating malaria, killing selectively plasmodium falciparium while sparing human cells. In addition proteasome inhibitors have shown potential as antibiotics for example against mycobacterium tuberculosis.

It is object to the present invention to provide novel compounds as proteasome subunit beta type-5 inhibitors and/or pharmaceutically acceptable salts thereof, which can be used as pharmaceutically active agents, and use thereof especially for prophylaxis and/or treatment of a disease associated with and/or caused by proteasome subunit beta type-5 selected from a cancer, a neurodegenerative disease, an infectious disease, and an inflammatory diease as well as compositions comprising at least one of those compounds and/or pharmaceutically acceptable salts thereof as pharmaceutically active ingredients. The objective of the present invention is solved by the teaching of the independent claims. Further advantageous features, aspects and details of the invention are evident from the dependent claims, the description, and the examples of the present application.

### Description of the invention

The present invention relates to a compound of the general formula (I) 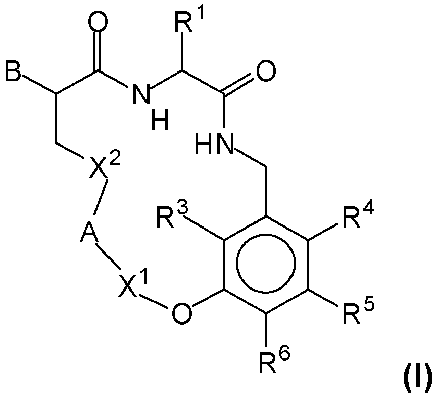 wherein
A represents -CO-N(**R^{N6}**)-, 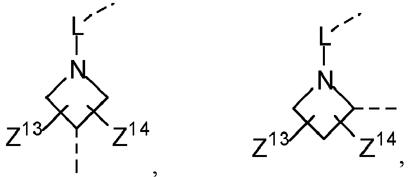 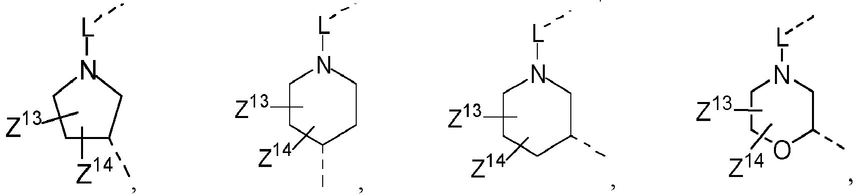 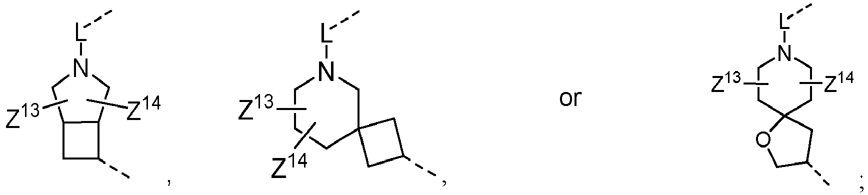
B represents -H, -NH(**R²**), -N(**R²**)(**R^{N5}**), 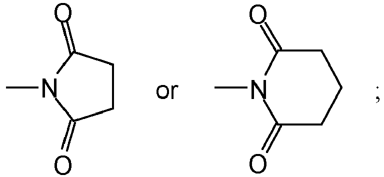
L represents -CO-, -CO-NH-, -CO-N(**R^{N3}**)-, or -CO-O-;
**R¹** represents -H, -(CH₂)ₚ-**R⁷**, -(CH₂)ₚ-NH-**R⁷**, -(CH₂)ₚ-**R⁹**, or -(CH₂)ₚ-**NR^{N4}**-**R⁹**;
**R²** represents -H, -**R⁸**, -**R¹¹**, -**L¹**-**R¹¹**, -**L¹**-(CH₂)ᵣ-**R⁸**, -**L¹**-**R¹⁰**, -**L¹**-(C₂H₄O)ₛ-**R¹¹**, -**L¹**-(CH₂)ₜ-O-**R¹¹**, -**L¹**-(CH₂)ₜ-NH-(CH₂)ᵣ-**R⁸**, -**L¹**-(CH₂)ₜ-O-(CH₂)ᵣ-**R⁸**, -**L¹**-(CH₂)t-**NR^{N6}R¹⁰**, -**L¹**-(CH₂)t-O-(CH₂)ᵤ-**NR^{N6}R¹⁰**, - **L¹**-(CH₂)ᵣ-**R¹⁴**, -CO-C(**R¹²**)(**R¹³**)-**R⁸**, or -CO-C(**R¹²**)(**R¹³**)-(CH₂)ᵤ-**R⁸**;
**L¹** represents a bond, -CO-, -CO₂-, -CONH-, or -SO₂-;
**R³** - **R⁶** represent independently of each other -H, -CH₃, -OCH₃, -F, or -CI;
or **R ⁵** and **R⁶** form 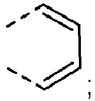
**R⁸** and **R⁹** represents independently of each other
   C₆-C₁₄ aryl, C₁-C₁₀ heteroaryl, C₃-C₈ carbocyclyl, C₁-C₉ heterocyclyl, C₄-C₁₁ bicyclic carbocyclyl, C₄-C₁₁ bridged carbocyclyl, C₁-C₁₀ bicyclic heterocyclyl, C₁-C₁₀ bridged heterocyclyl, C₇-C₁₆-spiroalkyl, C₅-C₁₄-spiroheterocyclyl,
   wherein all afore-mentioned ring systems can be substituted with 1 to 5 substituents selected from Z¹, Z², Z³, Z⁴, Z⁵, Z⁶, Z⁷, Z⁸, Z⁹, Z¹⁰, Z¹¹, Z¹² and R^{N1}, R^{N2}; and
   C₁-C₁₀ heteroaryl, C₁-C₉ heterocyclyl, C₁-C₁₀ bicyclic heterocyclyl, C₁-C₁₀ bridged heterocyclyl, C₅-C₁₄-spiroheterocyclyl ring systems contain at least one of heteroatoms N, O, and S;
   said C₃-C₈ carbocyclyl, C₁-C₉ heterocyclyl, C₁-C₁₀ bicyclic heterocyclyl, C₄-C₁₁ bridged carbocyclyl, C₁-C₁₀ bicyclic heterocyclyl, C₁-C₁₀ bridged heterocyclyl, C₇-C₁₆-spiroalkyl, C₅-C₁₄-spiroheterocyclyl ring systems can be partly saturated or unsaturated,
**R⁷** and **R¹⁰** represent independently of each other -H, -CH₃, -C₂H₅, -C₃H₇, -CH(CH₃)₂, -CH₂F, -CHF₂, -CF₃, -CH₂CN, -C(CH₃)₂-CN, -CH₂-C(CH₃)₂-CN, 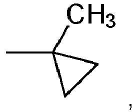 cyclo-C₃H₅, cyclo-C₄H₇, cyclo-C₅H₉, cyclo-C₆H₁₁, cyclo-C₇H₁₃, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -Ph, -CH₂-Ph, -CH₂-CH₂-Ph, -C₂H₄-CH=CH₂, -CH₂-CH=CH-CH₃, -CH₂-C(CH₃)=CH₂, -CH(CH₃)-CH=CH₂, -C(CH₃)=CH-CH₃, -CH₂-CH=C(CH₃)₂, -CO-CH=C(CH₃)₂, -CH₂-C=CH, -C₂H₄-C=CH, -CH₂-C=C-CH₃, -CH₂-OCF₃, -C₂H₄-OCF₃, -C₃H₆-OCF₃, -CH₂-OCHF₂, -C₂H₄-OCHF₂, -C₃H₆-OCHF₂, -CH₂,-OCH₃, -C₂H₄-OCH₃, -C₃H₆-OCH₃, -CH₂-OC₂H₅, -C₂H₄-OC₂H₅, -C₃H₆-OC₂H₅, -CH₂-OH, -C₂H₄-OH, -C₃H₆-OH, -C(CH₃)₂-CN, -C(CH₃)₂-OH, -C(CH₃)₂-CH₂-OH, -C(C₂H₅)₂-CH₂-OH, -C(CH₂-OH)₂-CH₃, -C(CH₂-OH)₂-C₂H₅, -C(CH₃)₂-CH₂-SH, -C(C₂H₅)₂-CH₂-SH, -C(CH₂-SH)₂-CH₃, or -C(CH₂-SH)₂-C₂H₅;
**R¹¹** represents -H, -CH₃, -C₂H₅, -C₃H₇, -CH(CH₃)₂, -CH₂F, -CHF₂, -CF₃, -CH₂CN, -C(CH₃)₂-CN, -CH₂-C(CH₃)₂-CN, 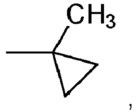 cyclo-C₃H₅, cyclo-C₄H₇, cyclo-C₅H₉, cyclo-C₆H₁₁, cyclo-C₇H₁₃, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -Ph, -CH₂-Ph, -CH₂-CH₂-Ph, -CH=CH₂, -CH₂-CH=CH₂, -C(CH₃)=CH₂, -CH=CH-CH₃, -C(CH₃)=CH-CH₃, -CH=C(CH₃)₂, -C(CH₃)=C(CH₃)₂, -C₂H₄-CH=CH₂, -CH₂-CH=CH-CH₃, -CH₂-C(CH₃)=CH₂, -CH(CH₃)-CH=CH₂, -C(CH₃)=CH-CH₃, -CH₂-CH=C(CH₃)₂, -C=CH, -CH₂-C=CH, -C₂H₄-C=CH, -CH₂-C=C-CH₃, -C≡C-CH₃, -C≡C-C₂H₅, -CH₂-OCF₃, -C₂H₄-OCF₃, -C₃H₆-OCF₃, -CH₂-OCHF₂, -C₂H₄-OCHF₂, -C₃H₆-OCHF₂, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OH, -C₂H₄-OH, -C₃H₆-OH, -C(CH₃)₂-CH₂-OH, -C(C₂H₅)₂-CH₂-OH, -C(CH₂-OH)₂-CH₃, -C(CH₂-OH)₂-C₂H₅, -C(CH₃)₂-CH₂-SH, -C(C₂H₅)₂-CH₂-SH, -C(CH₂-SH)₂-CH₃, or -C(CH₂-SH)₂-C₂H₅;
**R¹²** and **R¹³** represent independently of each other -H, -CH₃, -C₂H₅, -C₃H₇, -CH(CH₃)₂, -Ph, -CH₂-Ph, -COOH, -NH₂, -NHCO₂(CCH₃)₃, -CH₂-NH₂, -CHF₂, -CF₃, -OCF₃, -OCHF₂, -OH, -OCH₃, -OC₂H₅, -OC₃H₇, and -OCH(CH₃)₂; or
**R¹²** and **R¹³** form together 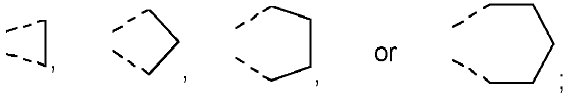
**R¹⁴** represents 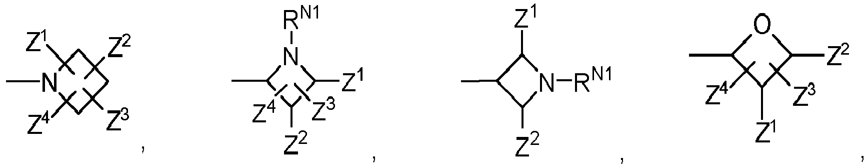 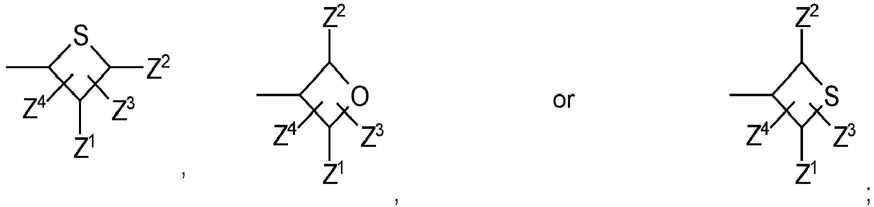
**R¹⁵** and **R¹⁶** represent independently of each other **-X³-L²-R¹⁷**, or -(OCH₂CH₂)_{w}-**R¹⁷**;
**L²** represents -(CH₂)ᵥ-, -(CH₂CH₂-O)_{w}-CH₂-, or -(CH₂CH₂-O)_{w}-CH₂CH₂-;
**R¹⁷** represents -OH, -SH, -SO₃H, -NH₂, or -CO₂H;
**R^{N1}**, **R^{N2}**, **R^{N3}** and **R^{N4}** represent independently of each other -H, -CH₃, -C₂H₅, -C₃H₇, -CH(CH₃)₂, -CHF₂, -CF₃, 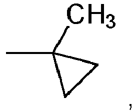 cyclo-C₃H₅, cyclo-C₄H₇, cyclo-C₅H₉, cyclo-C₆H₁₁, cyclo-C₇H₁₃, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -Ph, -CH₂-Ph, -CH₂-CH₂-Ph, -C₂H₄-CH=CH₂, -CH₂-CH=CH-CH₃, -CH₂-C(CH₃)=CH₂, -CH(CH₃)-CH=CH₂, -CH₂-CH=C(CH₃)₂, -CH₂-C=CH, -C₂H₄-C=CH, -CH₂-C=C-CH₃, -COCH₃, -COC₂H₅, -COC₃H₇, -CO-cyclo-C₃H₅, -COCH(CH₃)₂, -COC(CH₃)₃, -COOH, -COOCH₃, -COOC₂H₅, -COOC₃H₇, -COO-cyclo-C₃H₅, -COOCH(CH₃)₂, -COOC(CH₃)₃, -COOCH₂Ph, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -SO₃H, or -**R¹⁵**;
**R^{N5}** and **R^{N6}** represent independently of each other -H, -CH₃, -C₂H₅, -C₃H₇, -CH(CH₃)₂, cyclo-C₃H₅, -COOC(CH₃)₃, or -COOCH₂Ph;
**X¹** represents -(CH₂)ₘ-;
**X²** represents -(CH₂)ₙ-;
**X³** represents a bond, -O-, -NH-, or -S-;
**Z¹** - **Z¹⁴** represent independently of each other 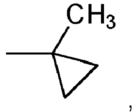 cyclo-C₃H₅, cyclo-C₄H₇, cyclo-C₅H₉, cyclo-C₆H₁₁, cyclo-C₇H₁₃, -**R¹⁶**, -H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -SH, -SCH₃, -SC₂H₅, -SC₃H₇, -S-cyclo-C₃H₅, -SCH(CH₃)₂, -SC(CH₃)₃, -F, -Cl, -Br, -I, -CN, -COCH₃, -COC₂H₅, -COC₃H₇, -CO-cyclo-C₃H₅, -COCH(CH₃)₂, -COC(CH₃)₃, -COOH, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cycl0-C₃H₅)₂, -SO₂N[CH(CH₃)₂]₂, -SO₂N[C(CH₃)₃]₂, -O-S(=O)CH₃, -O-S(=O)C₂H₅, -O-S(=O)C₃H₇, -O-S(=O)-cyclo-C₃H₅, -O-S(=O)CH(CH₃)₂, -O-S(=O)C(CH₃)₃, -S(=O)(=NH)CH₃, -S(=O)(=NH)C₂H₅, -S(=O)(=NH)C₃H₇, -S(=O)(=NH)-cyclo-C₃H₅, -S(=O)(=NH)CH(CH₃)₂, -S(=O)(=NH)C(CH₃)₃, -NH-SO₂-CH₃, -NH-SO₂-C₂H₅, -NH-SO₂-C₃H₇, -NH-SO₂-cyclo-C₃H₅, -NH-SO₂-CH(CH₃)₂, -NH-SO₂-C(CH₃)₃, -O-SO₂-CH₃, -O-SO₂-C₂H₅, -O-SO₂-C₃H₇, -O-SO₂-cyclo-C₃H₅, -O-SO₂-CH(CH₃)₂, -O-SO₂-C(CH₃)₃, -OCH₂F, -OCHF₂, -OCF₃, -CH₂-OCF₃, -C₂H₄-OCF₃, -C₃H₆-OCF₃, -CH₂-OCHF₂, -C₂H₄-OCHF₂, -C₃H₆-OCHF₂, -OC₂F₅, -CH₂-OC₂F₅, -C₂H₄-OC₂F₅, -C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-C(=NH)-NH₂, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-C(=NH)-NHCH₃, -NH-C(=NH)-NHC₂H₅, -NH-C(=NH)-NHC₃H₇, -O-CO-NH-cyclo-C₃H₅, -NH-C(=NH)-NH-cyclo-C₃H₅, -NH-C(=NH)-NH[CH(CH₃)₂], -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₂F, -CHF₂, -CF₃, -CH₂-CH₂F, -CH₂-CHF₂, -CH₂-CF₃, cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH=CH-CH=CH-CH₃, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C=C-CH₃, -CH₂-CH=CH-CH=CH-CH₃, -CH=CH-CH=CH-C₂H₅, -CH(CH₃)-CH₂-C=CH, -CH(CH₃)-C≡C-CH₃, -C₂H₄-CH(CH₃)-C≡CH, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C=CH, -C(CH₃)=CH-CH=CH-CH₃, -C=CH, -C≡C-CH₃, -CH₂-C=CH, -C₂H₄-C=CH, -CH₂-C=C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-CH₂-CH(CH₃)₂, -CH(CH₃)-C₂H₄-C≡CH, -CH₂-CH(CH₃)-C≡C-CH₃, -C(CH₃)(C₂H₅)-C≡CH, -CH(CH₃)-CH₂-C≡C-CH₃, -CH(CH₃)-C≡C-C₂H₅, -CH₂-C≡C-CH(CH₃)₂, -C≡C-CH(CH₃)-C₂Hₛ, -CH₂-C≡C-C≡C-CH₃, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -CH₂-CH(C≡CH)₂, -C=C-C=CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -C≡C-C(CH₃)₃, -C≡C-CH₂-C≡C-CH₃, -C≡C-C≡C-C₂H₅, 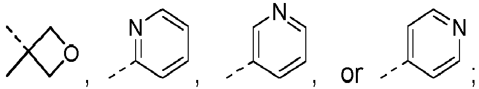
**Z³** and **Z⁴** may form together 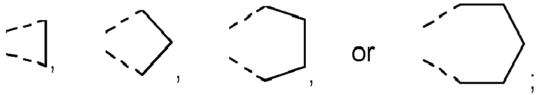
**Z¹³** and **Z¹⁴** may form together 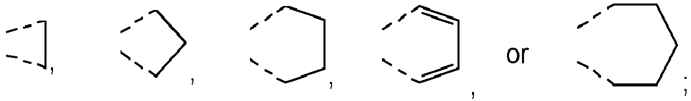
   m is an integer selected from 0, 1, 2, 3, 4, 5, or 6;
   n is an integer selected from 0, 1, 2, 3, 4, 5, or 6;
   p is an integer selected from 0, 1, 2, 3, 4, 5, or 6;
   r is an integer selected from 0, 1, 2, 3, or 4;
   s is an integer selected from 1, 2, 3, 4, 5, 6, 7, 8, 9, or 10;
   t is an integer selected from 1, 2, 3, or 4;
   u is an integer selected from 1, 2, 3, or 4;
   v is an integer selected from 0, 1, 2, 3, 4, 5, or 6;
   w is an integer selected from 0, 1, 2, or 3;
or an enantiomer, a stereoisomeric form, a mixture of enantiomers, a diastereomer, a mixture of diastereomers, a hydrate, a solvate, an acid salt form, a tautomer, a racemate of the above mentioned compounds, or a pharmaceutically acceptable salt thereof.

As used herein, the term **"C₆-C₁₄-aryl"** refers to aromatic residues or more specific to aromatic carbocyclic residues with one, two or three aromatic rings and refers preferably to phenyl and naphthyl, wherein these phenyl and naphthyl residues can be substituted with 1 to 5 substituents selected from Z¹ to Z¹². However it is clear to a skilled person that the term "can be substituted" refers to the replacement of a hydrogen atom by one of the substituents Z¹ to Z¹². The carbon atom number of **C₆-C₁₄** refers only to the carbon atoms of the aromatic ring system (aryl) and does not include the carbon atoms of the substituents Z¹ to Z¹².

Examples of preferred C₆-C₁₄-aryl groups and substituted C₆-C₁₄-aryl residues are

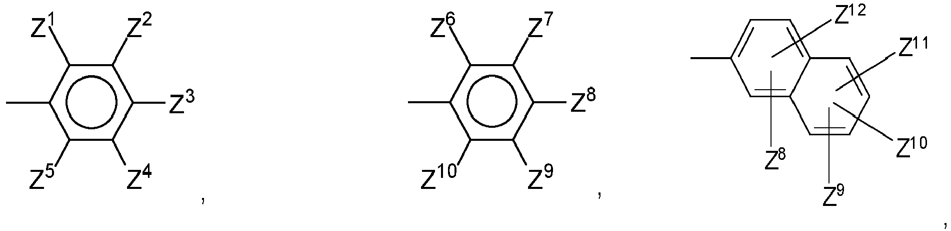

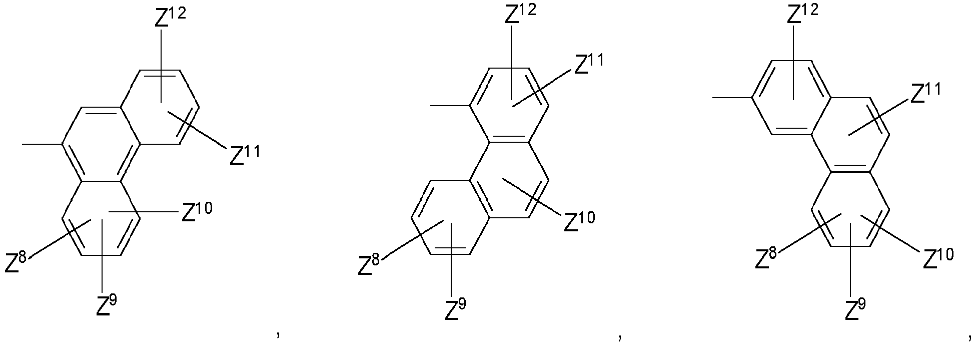

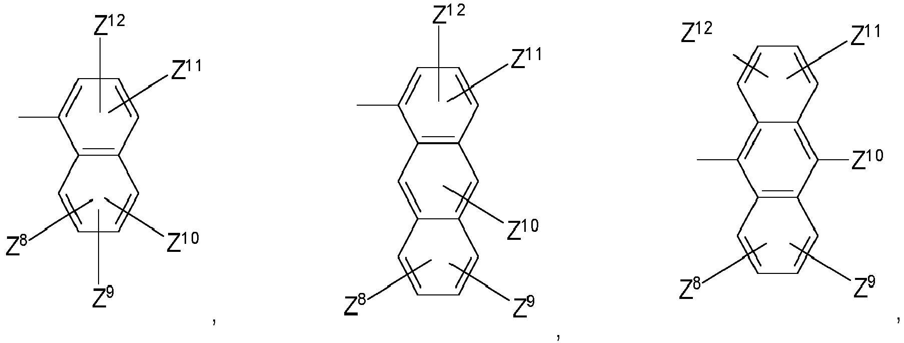

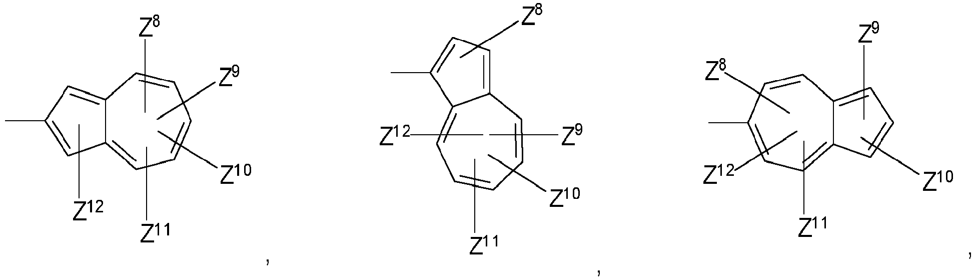

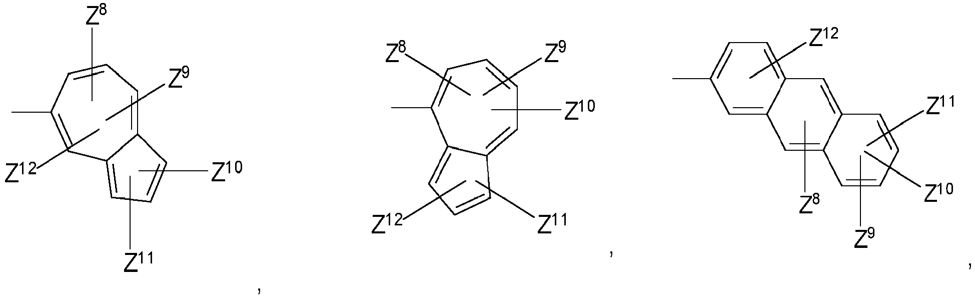

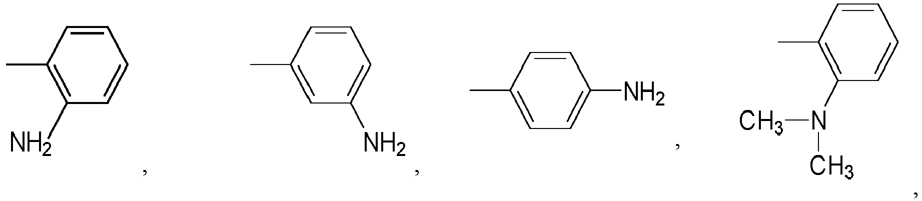

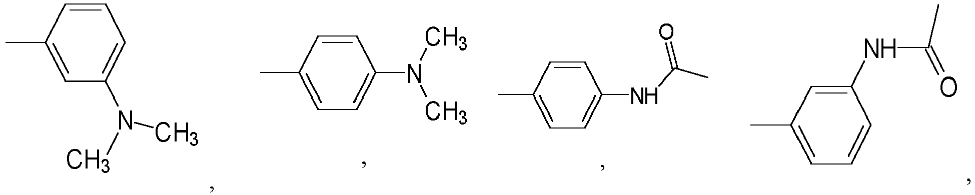

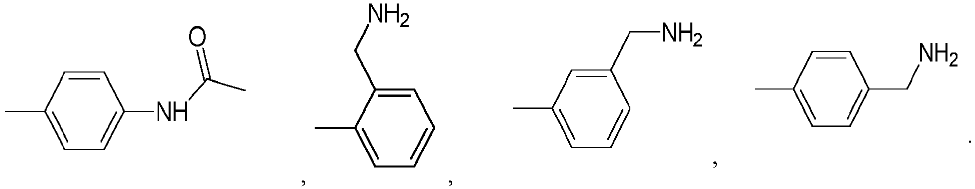

As used herein, the term **"C₁-C₁₀-heteroaryl"** refers to aromatic residues with one or more heteroatoms such as O, S, N and especially N and refers preferably to 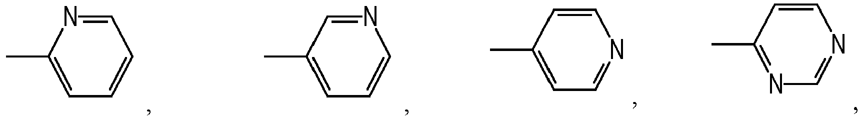 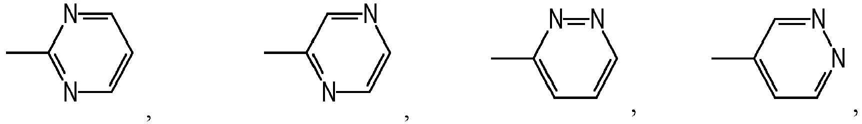 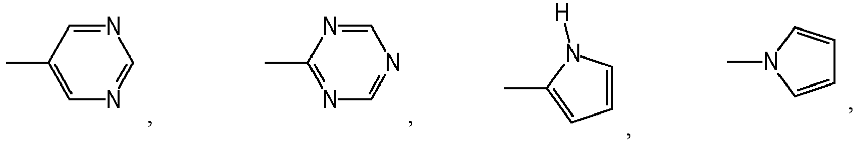 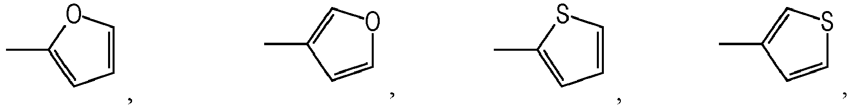 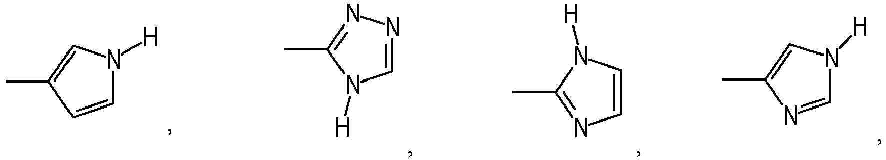 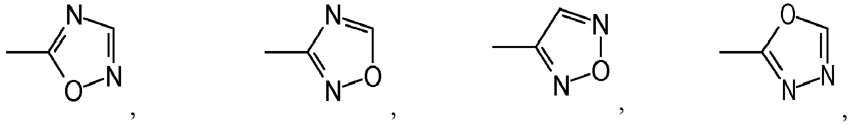 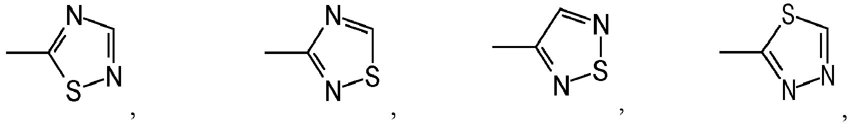 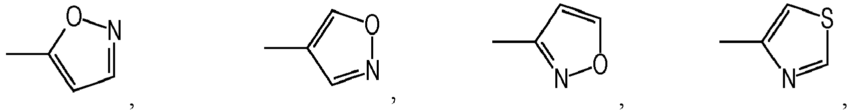 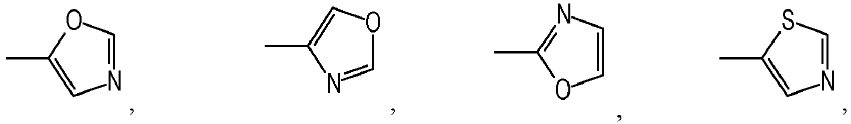 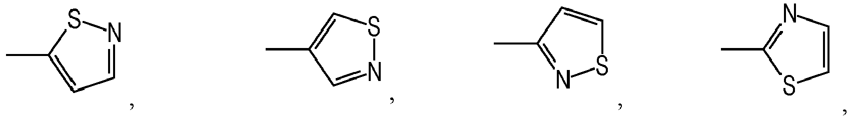 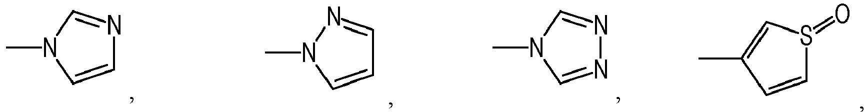 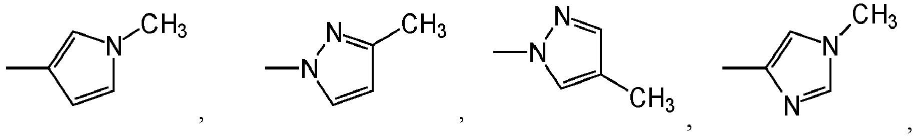 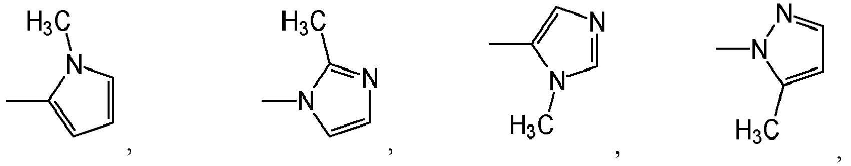 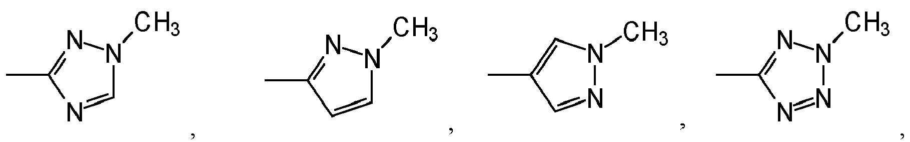 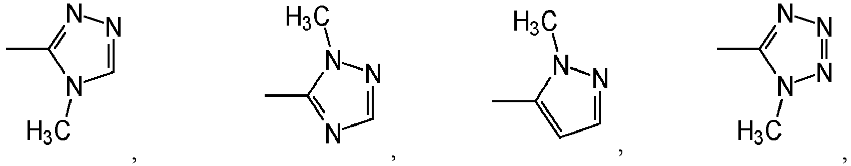 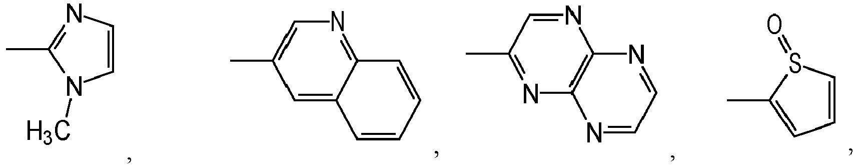 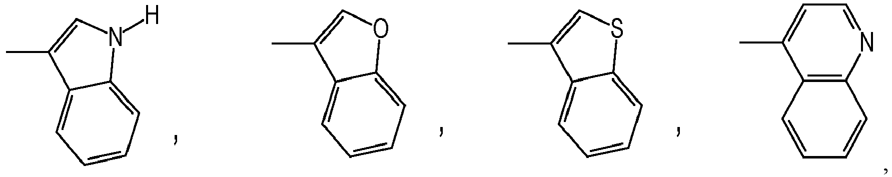 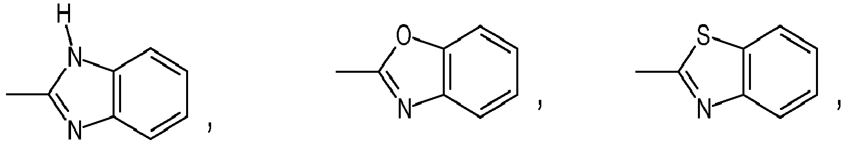 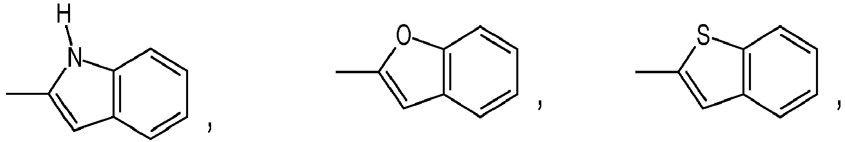 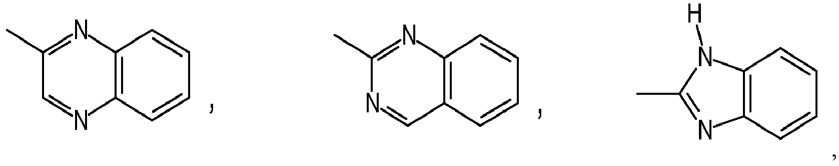 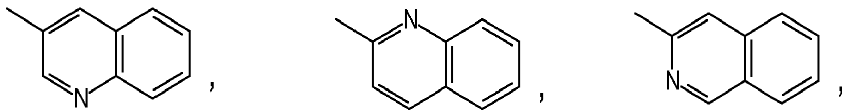 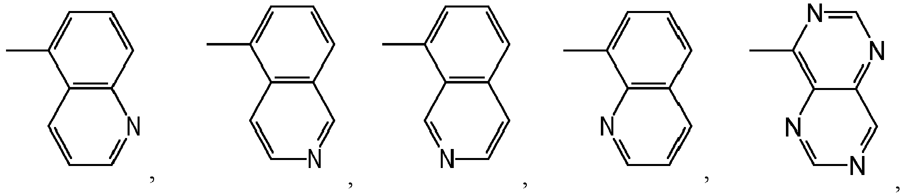 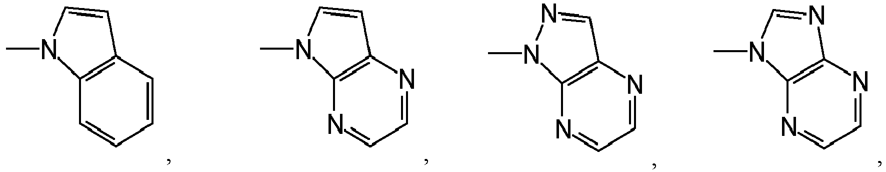 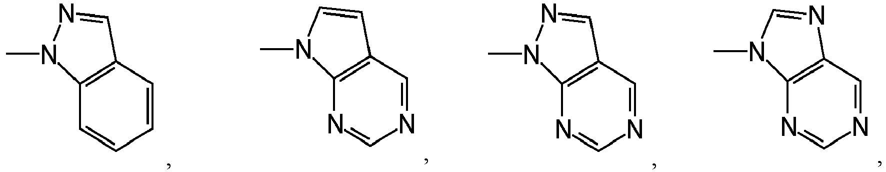 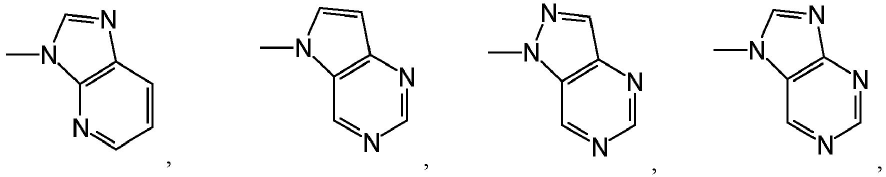 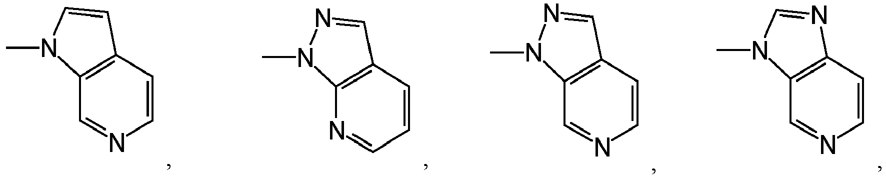 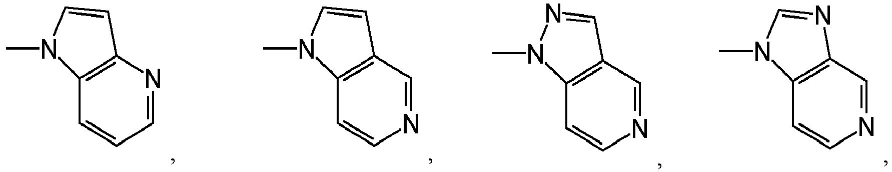 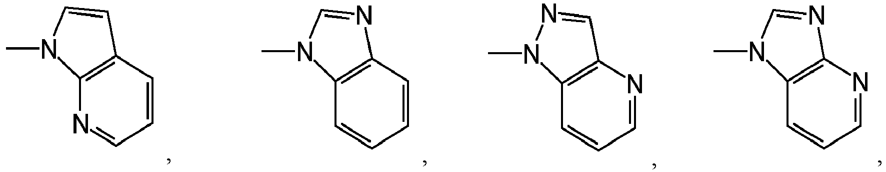 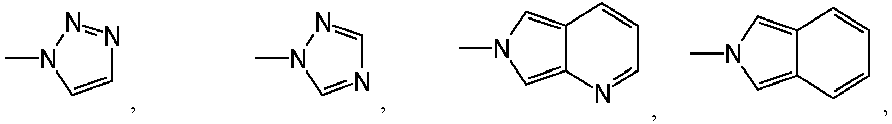 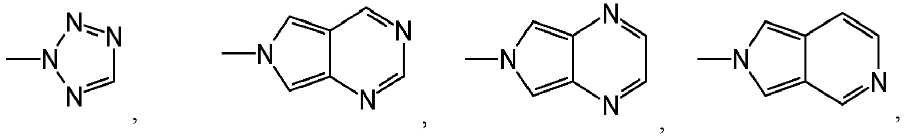 wherein these residues can be substituted with 1 to 5 substituents selected from R^{N1}, R^{N2}, Z¹ to Z¹². However it is clear to a skilled person that the term "can be substituted" refers to the replacement of a hydrogen atom by one of the substituents R^{N1}, R^{N2}, Z¹ to Z¹². Moreover it is clear to a skilled person that only these hydrogen atoms which are present in the residue can be replaced by the substituents R^{N1}, R^{N2}, Z¹ to Z¹². In case of secondary amine group in these heteroaryl residues, a hydrogen atom of secondary amine group is replaced by the substituent R^{N1} or R^{N2}. Thus, since the oxadiazole group has only one hydrogen atom, only one hydrogen atom can be replaced by one substituent selected from Z¹ to Z¹². The carbon atom number of **C₁-C₁₀** refers only to the carbon atoms of the heteroaromatic ring system (heteroaryl) and does not include the carbon atoms of the substituents R^{N1}, R^{N2}, Z¹ to Z¹².

Examples of preferred substituted C₁-C₁₀-heteroaryl residues are

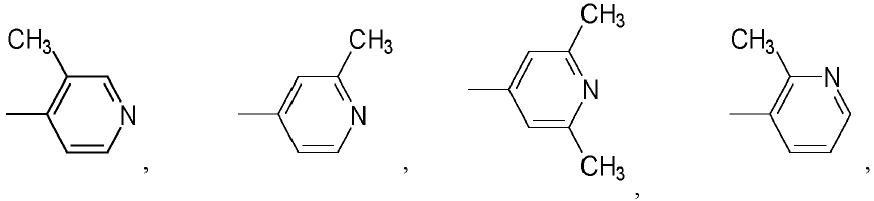

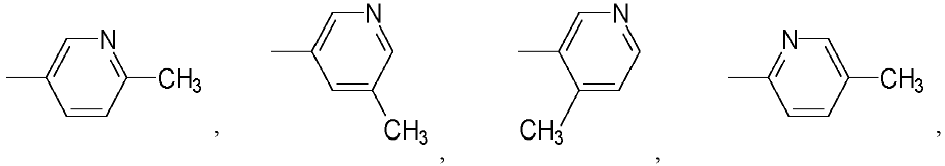

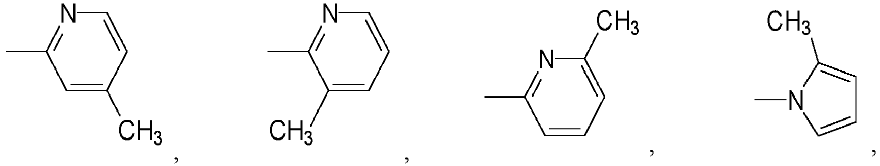

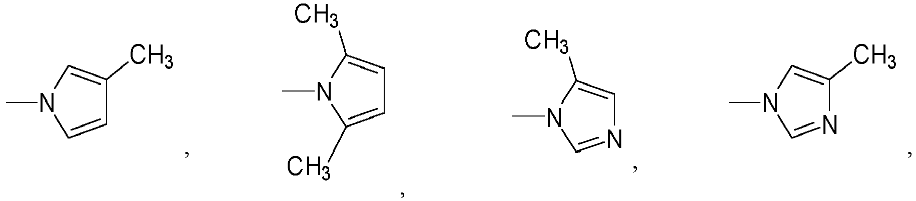

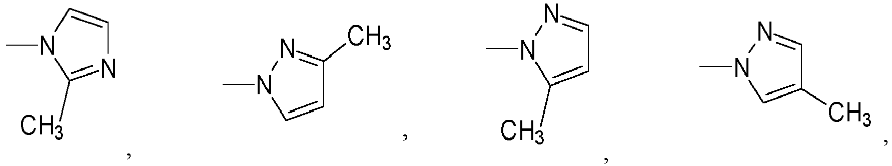

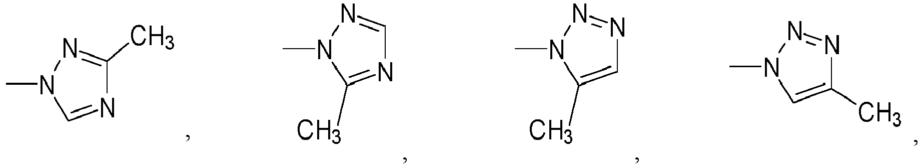

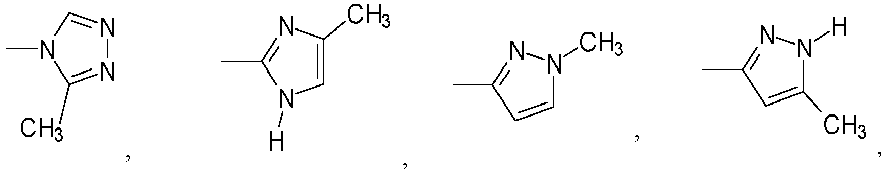

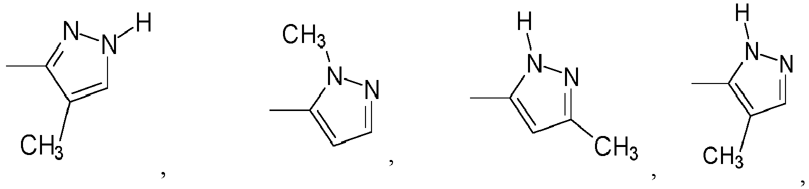

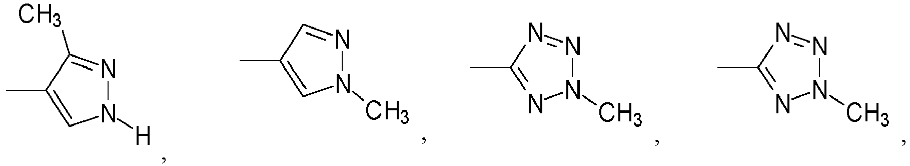

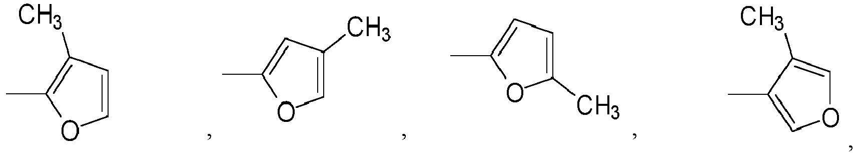

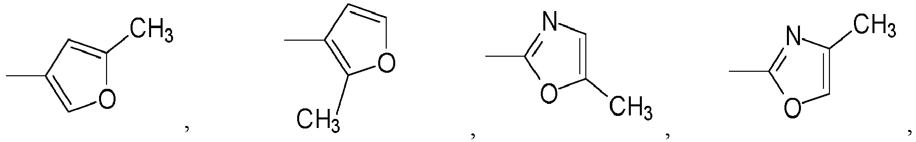

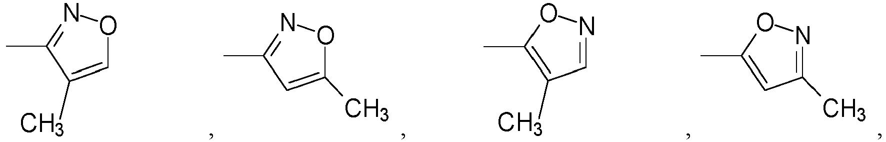

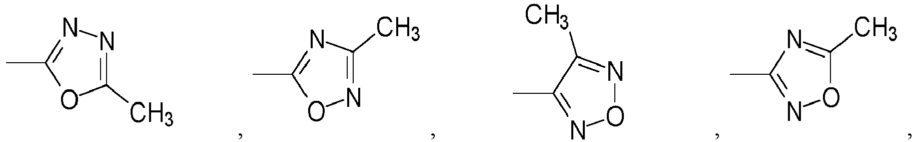

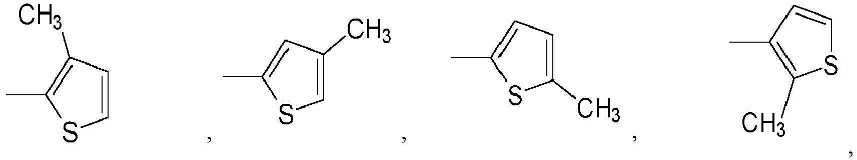

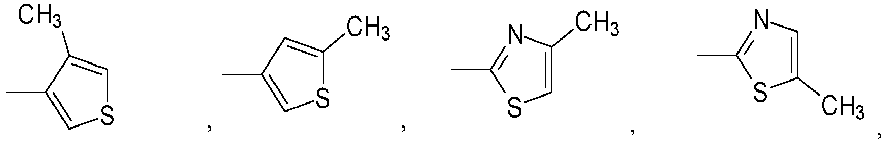

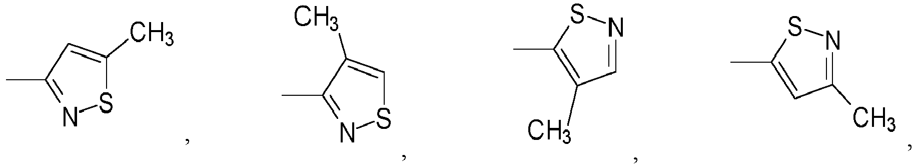

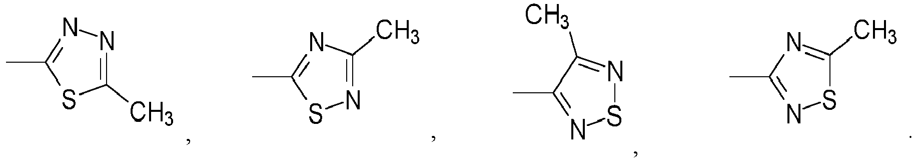

As used herein, **C₃-C₈-carbocyclyl** refers to cyclo-C₃H₅, cyclo-C₄H₇, cyclo-C₅H₉, cyclo-C₆H₁₁, cyclo-C₇H₁₃, and cyclo-C₈H₁₅, wherein these residues can be substituted with 1 to 5 substituents selected from Z¹ to Z¹², preferably, Z¹, Z², Z³, Z⁴ and Z⁵. However it is clear to a skilled person that the term "can be substituted" refers to the replacement of a hydrogen atom by one of the substituents Z¹ to Z¹², preferably, Z¹, Z², Z³, Z⁴ and Z⁵. The carbon atom number of **C₃-C₈** refers only to the carbon atoms of the cycloalkyl residue and does not include the carbon atoms of the substituents Z¹ to Z¹².

Examples of preferred substituted C₃-C₈-cycloalkyl residues are

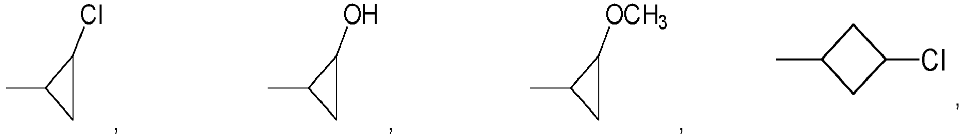

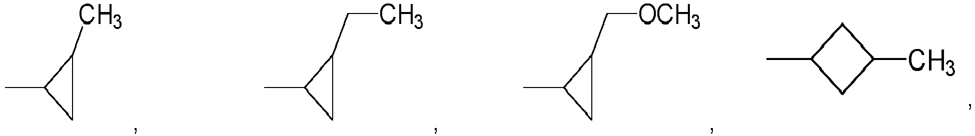

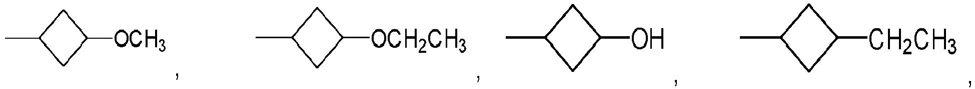

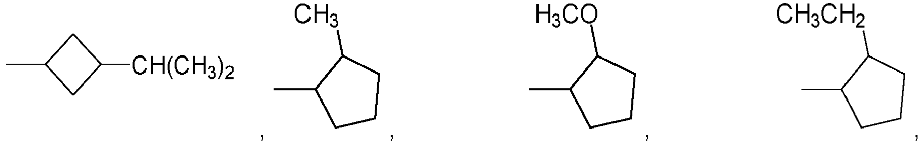

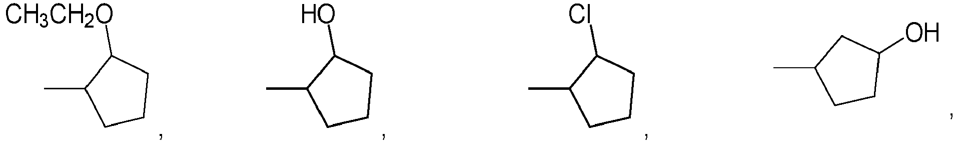

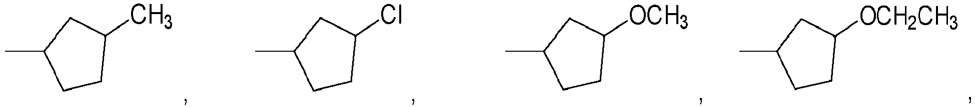

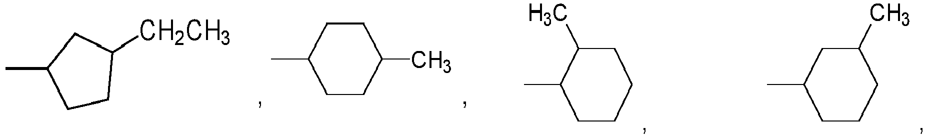

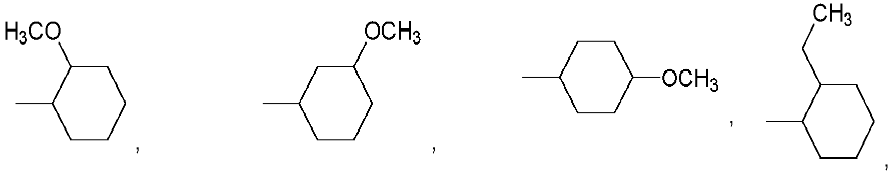

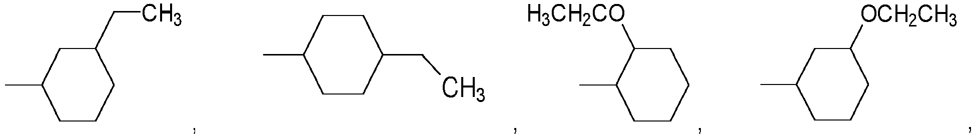

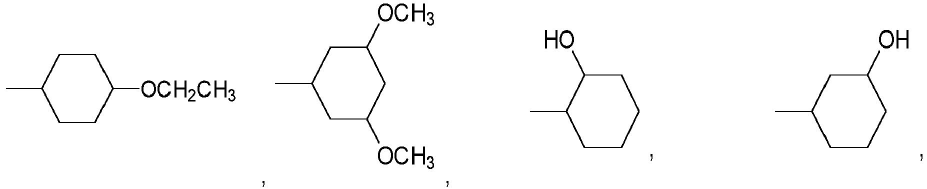

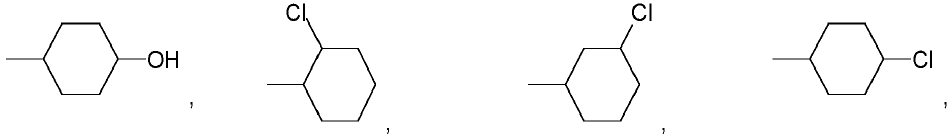

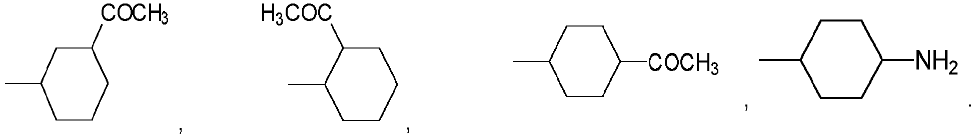

As used herein, the term **"C₁-C₉-heterocyclyl"** covers saturated or partly unsaturated heterocyclic residues with 1 to 9 ring carbon atoms, but not aromatic residues and covers also bicyclic saturated or partly unsaturated residues with 1 to 9 ring carbon atoms, but preferably not fully aromatic residues which are aromatic throughout the bicyclic system but may comprise partly aromatic ring systems, wherein one ring of the bicyclic ring system is aromatic.

Examples of preferred substituted C₁-C₉-heterocyclyl residues are 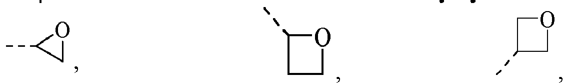 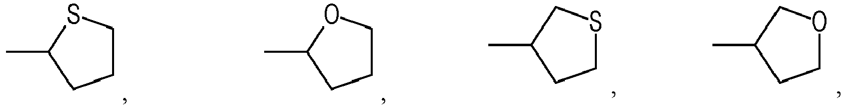 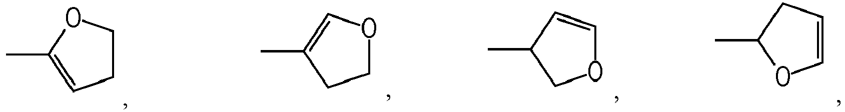 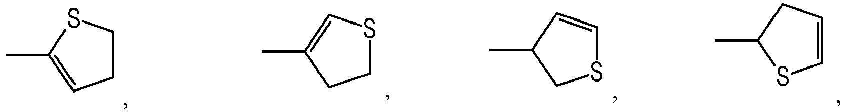 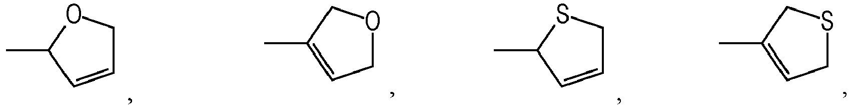 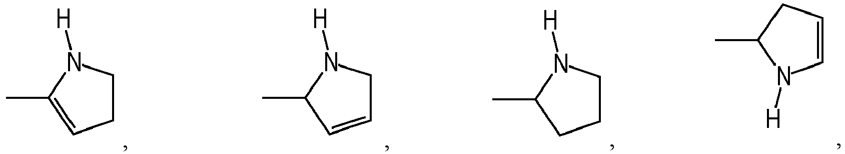 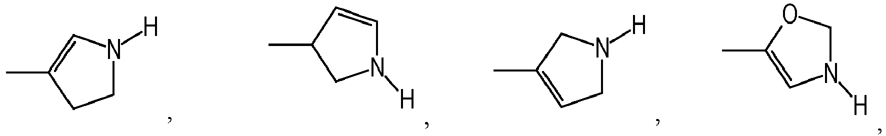 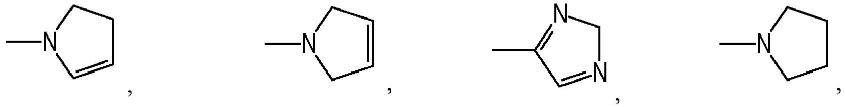 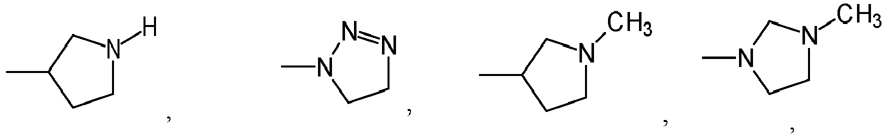 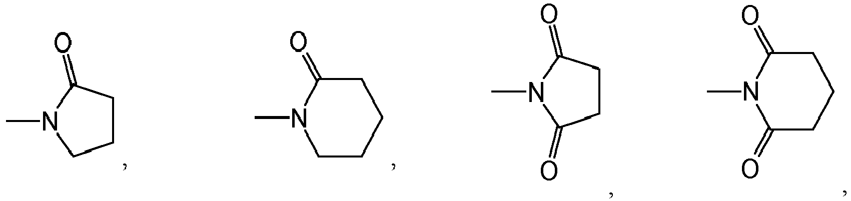 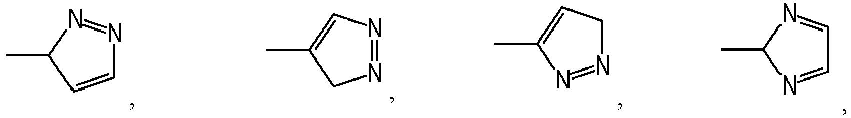 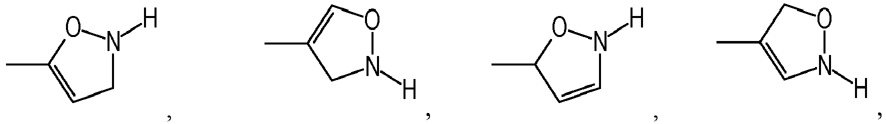 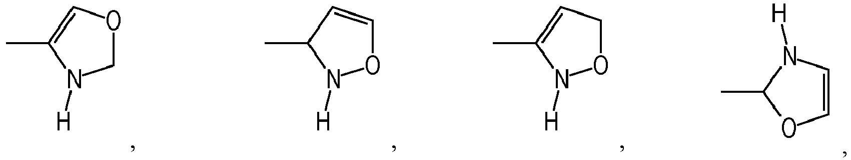 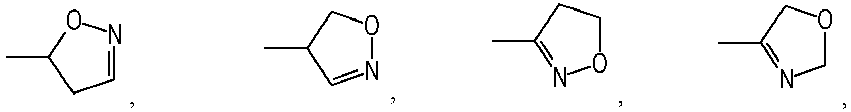 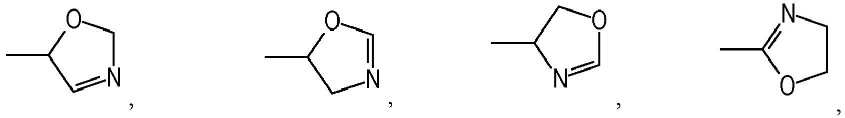 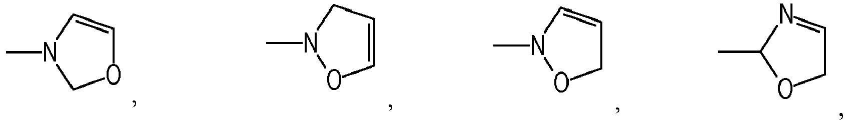 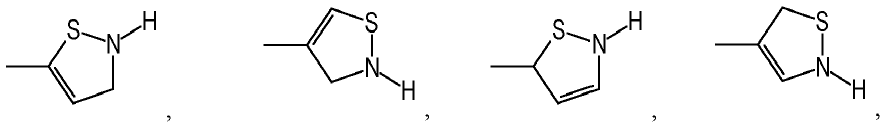 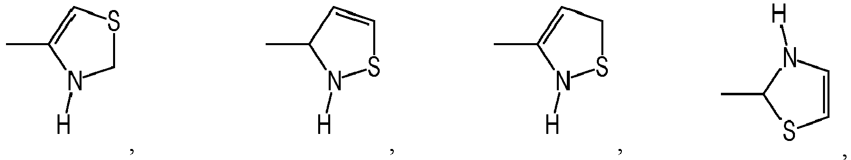 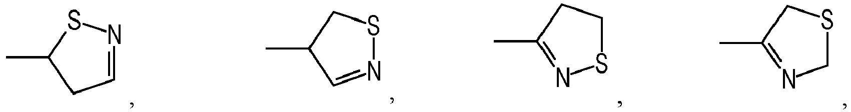 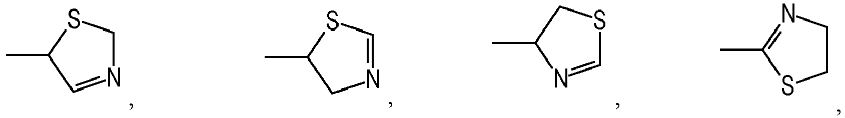 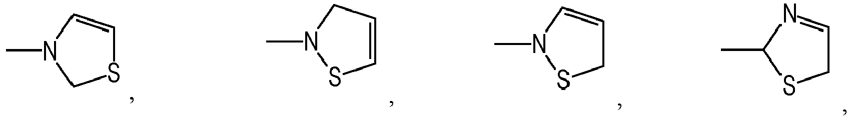 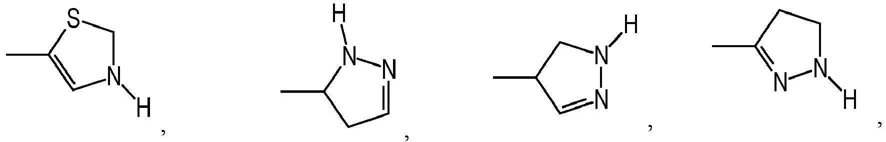 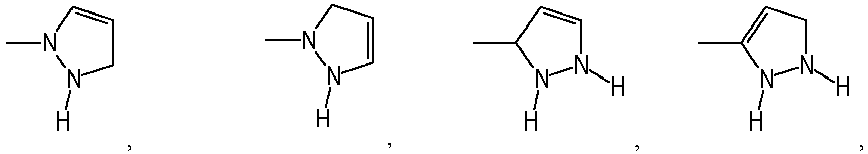 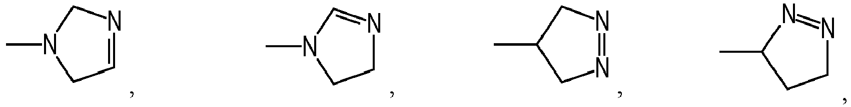 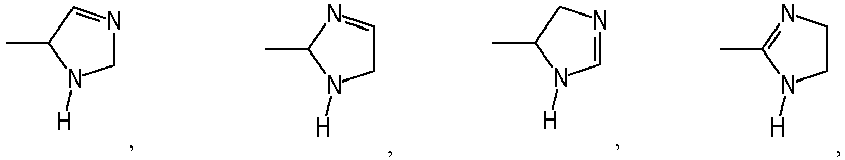 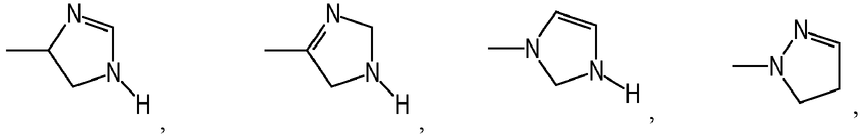 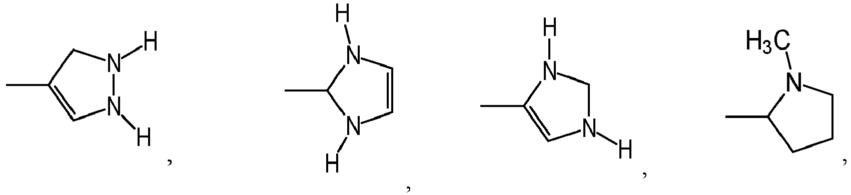 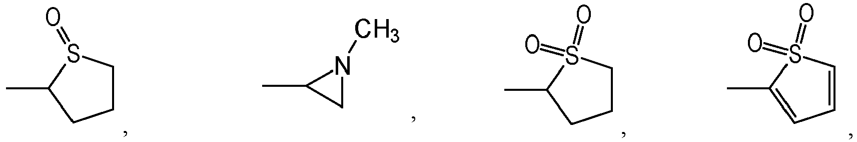 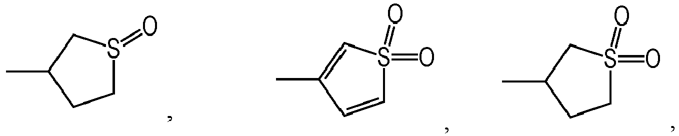 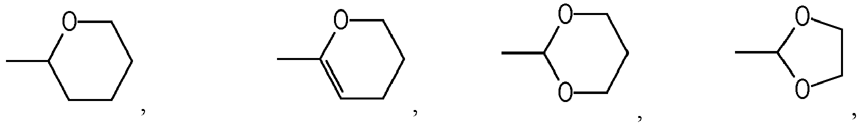 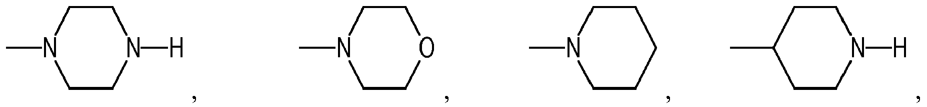 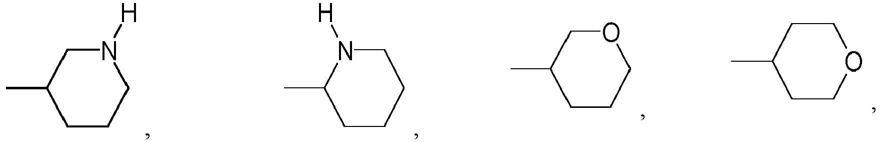 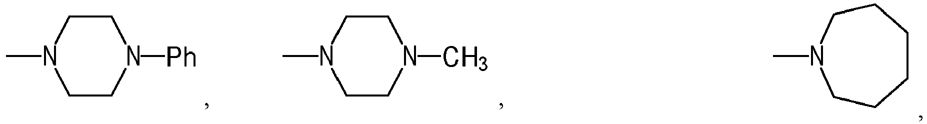 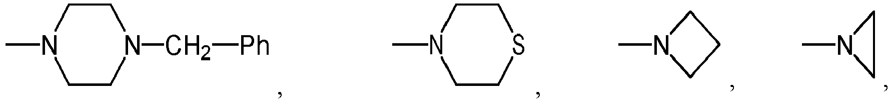 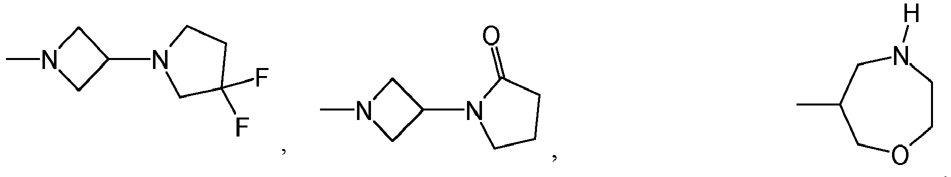 wherein these residues can be substituted with 1 to 5 substituents selected from R^{N1}, R^{N2}, Z¹ to Z¹². However it is clear to a skilled person that the term "can be substituted" refers to the replacement of a hydrogen atom by one of the substituents R^{N1}, R^{N2}, Z¹ to Z¹². Moreover it is clear to a skilled person that only these hydrogen atoms which are present in the residue can be replaced by the substituents R^{N1}, R^{N2}, Z¹ to Z¹². In case of secondary amine group in these heteroaryl residues, a hydrogen atom of secondary amine group is replaced by the substituent R^{N1} or R^{N2}. Thus, since the oxirane group (also named as ethylene oxide group) has only three hydrogen atoms, only three hydrogen atoms can be replaced by three substituents selected from Z¹ to Z¹². The carbon atom number of **C₁-C₉** refers only to the carbon atoms of the heterocyclic ring system (heterocyclyl) and does not include the carbon atoms of the substituents R^{N1}, R^{N2}, Z¹ to Z¹².

As used herein, **C₄-C₁₁ bicyclic carbocyclyl** is represented by generalized formula **(a1),** 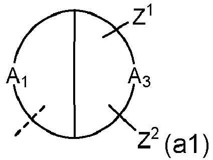
wherein, A₁, and A₃ represent independently C₁-C₇ alkylene; and
C₅-C₁₁ bicyclic carbocyclic ring may be optionally substituted with 1 to 5 substituents selected from Z¹ to Z¹², preferably substituted by Z¹ and Z² and have 0 - 2 double bonds

Examples of preferred C₅-C₁₁ bicyclic carbocylcic ring are

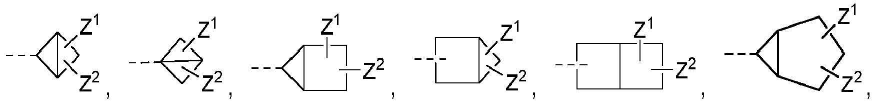

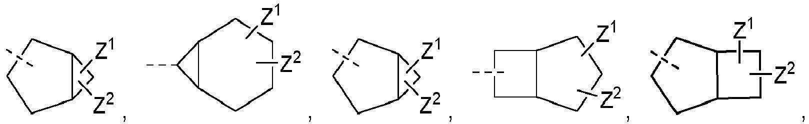

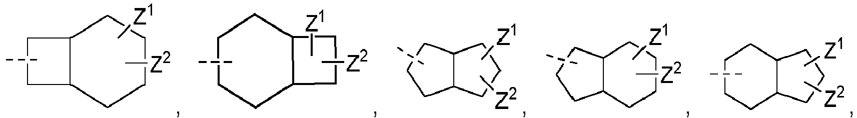

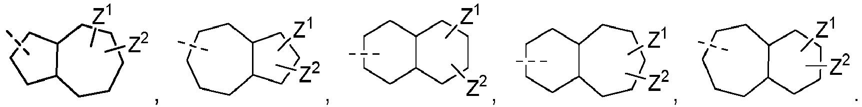

As used herein, **C₁-C₁₀ bicyclic heterocyclyl** may be represented by generalized formula **(a2)** 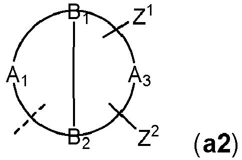
wherein, A₁, and A₃ represent independently C₁-C₅ alkylene and at least one carbon atom of said C₁-C₅ alkylene is replaced with heteroatoms selected from O, N, and S; and
B₁, and B₂ represent independently CH, or N;
C₅-C₁₁ bicyclic heterocylcic ring may be optionally substituted with 1 to 5 substituents selected from Z¹ to Z¹², R^{N1} and R^{N2}, preferably substituted by Z¹, Z² and R^{N1}, and have 0 - 3 double bonds.

Examples of preferred C₅-C₁₁ bicyclic heterocylcic ring are

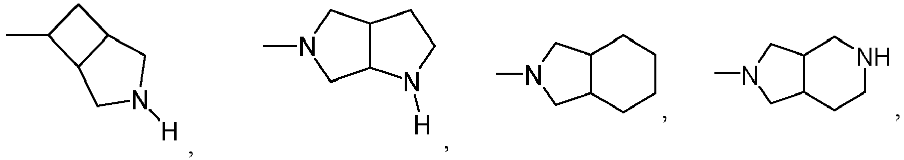

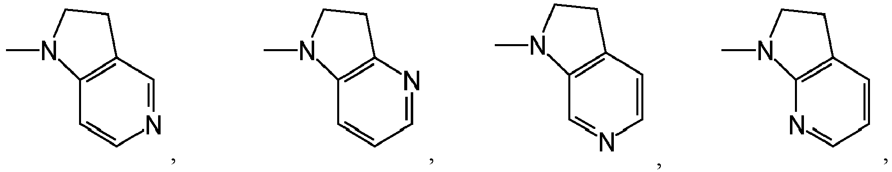

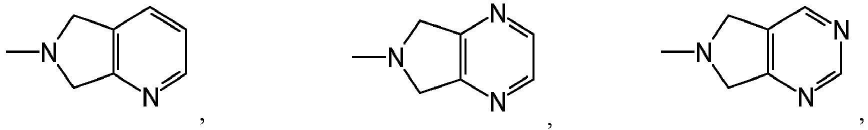

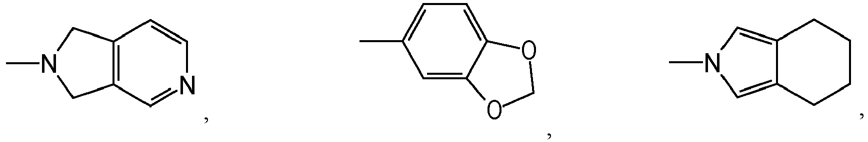

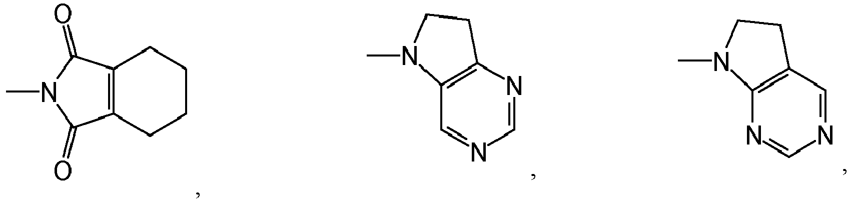

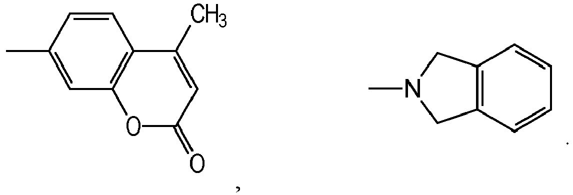

As used herein, **C₄-C₁₁ bridged carbocyclyl** may be represented by generalaized formula **(b1)** 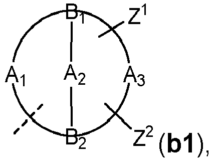
wherein, A₁, A₂, and A₃ represent independently C1-C3 alkylene; and
B₁, B₂, represent independently CH, or B₁ and B₂ form a bond;
C₅-C₁₁ bridged carbocylcic ring may be optionally substituted with 1 to 5 substituents selected from Z¹ to Z¹², preferably substituted by Z¹ and Z² and have 0 - 2 double bonds.

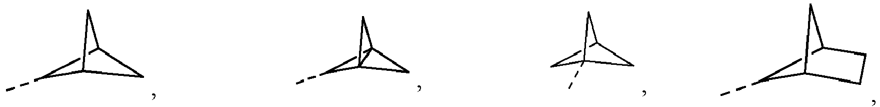

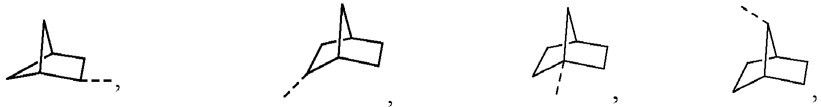

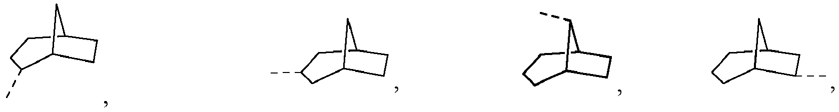

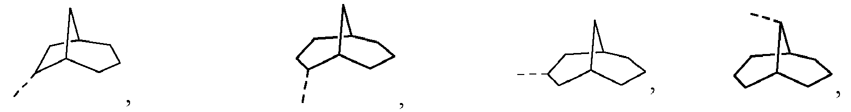

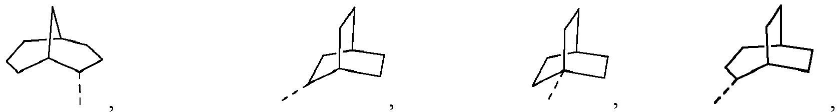

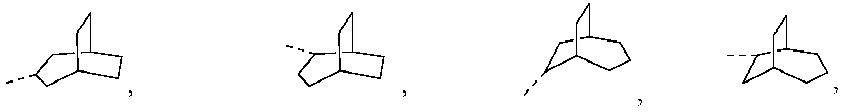

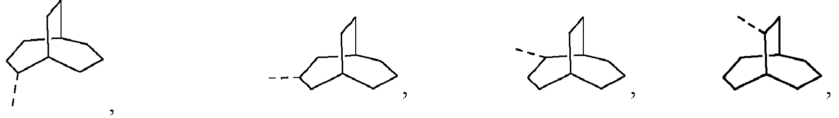

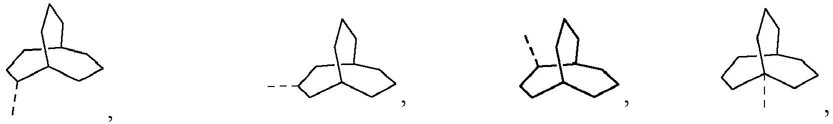

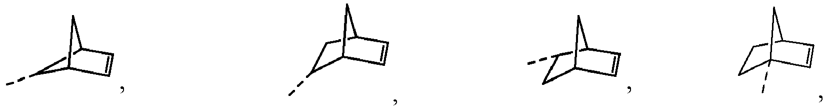

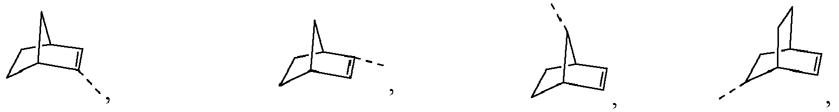

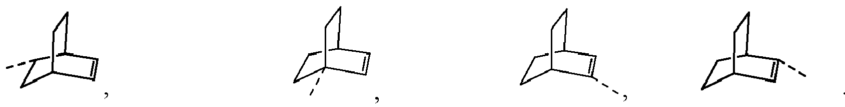

As used herein, **C₁-C₁₀ bridged heterocylyl** may be represented by generalaized formula **(b2)** 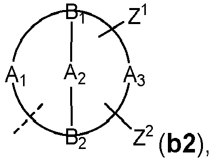
wherein, A₁, A₂, and A₃ represent independently C₁-C₃ alkylene and at least one carbon atom of said C₁-C₃ alkylene is replaced with heteroatoms selected from O, N, and S;and
B₁, and B₂, represent independently CH, or **N;**
C₅-C₁₁ bridged heterocyclyl ring may be optionally substituted with 1 to 5 substituents selected from Z¹ to Z¹², R^{N1} and R^{N2}, preferably substituted by Z¹, Z² and R^{N1}, and have 0 - 2 double bonds.

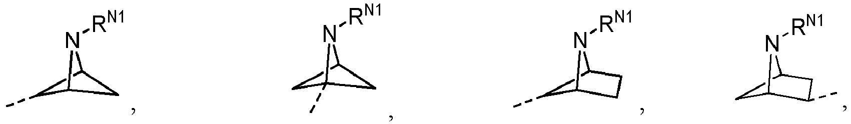

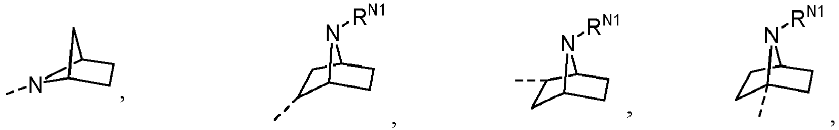

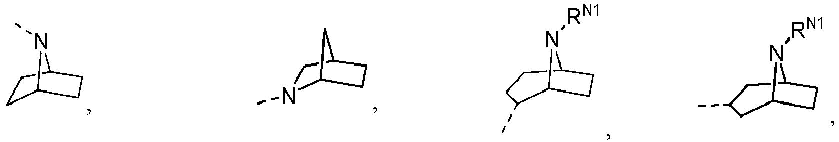

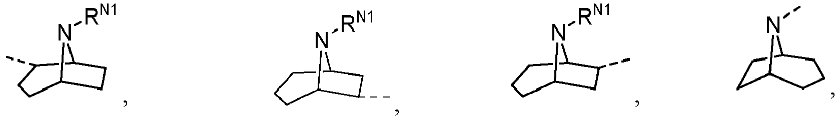

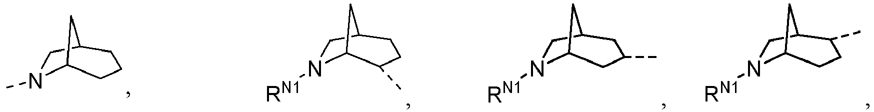

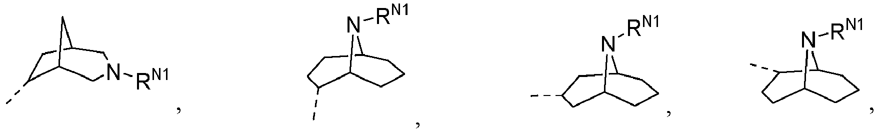

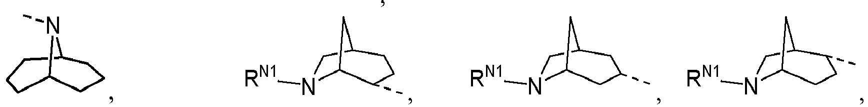

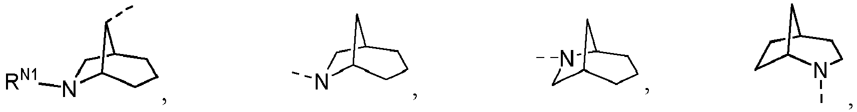

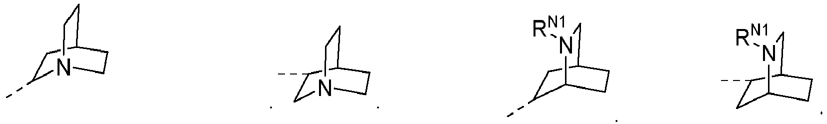

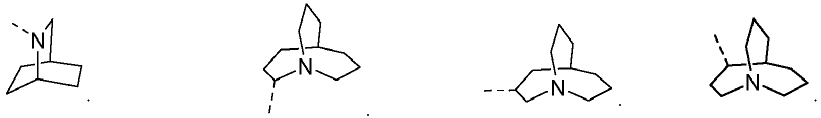

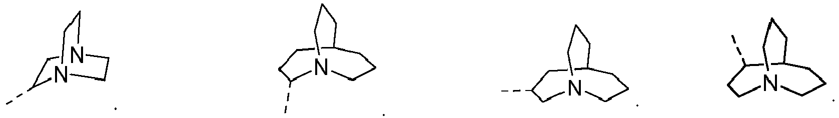

As used herein, **C₇-C₁₆-spiroalkyl** may be represented by generalaized formula **(b3)** 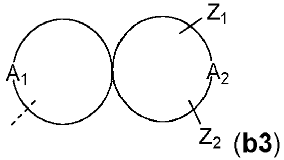
wherein, A₁, and A₂, represent independently C₂-C₇ alkylene; and
C₇-C₁₆ spiroaklyl may be optionally substituted with 1 to 5 substituents selected from Z¹ to Z¹², preferably substituted by Z⁵ - Z⁷ and have 0 - 2 double bonds.

As used herein, the term **"C₇-C₁₆-spiroalkyl"** refers to spirocarbocyclic residues, wherein these spirocarbocyclic residues can be substituted with 1 to 3 substituents selected from Z⁵, Z⁶ and Z⁷. However it is clear to a skilled person that the term "can be substituted" refers to the replacement of a hydrogen atom by one of the substituents Z⁵, Z⁶ and Z⁷. It is also possible that two of the substituents Z⁵, Z⁶ and Z⁷ represent together an oxygen atom and form together with the carbon atom of the spiroalkyl residue to which they are both attached a carbonyl moiety. The carbon atom number of **C₇-C₁₆** refers only to the carbon atoms of the spiro ring system and does not include the carbon atoms of the substituents. Thus a spiro[4,5]decyl residue is counted as a C₁₀-spiroalkyl regardless if this spiro residue carries five pentyl substituents.

Examples of preferred **C₇-C₁₆-spiroalkyl** groups and substituted **C₇-C₁₆-spiroalkyl** groups are 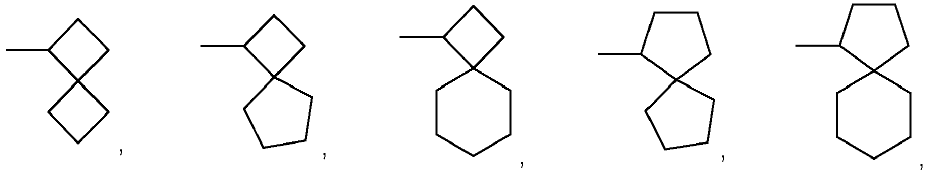 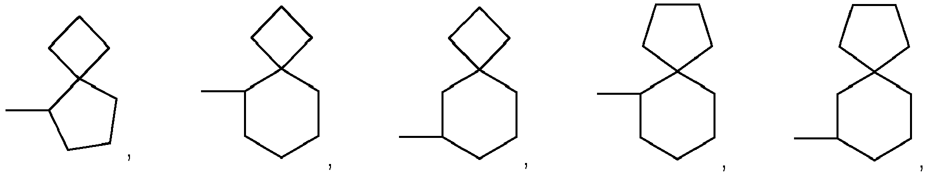 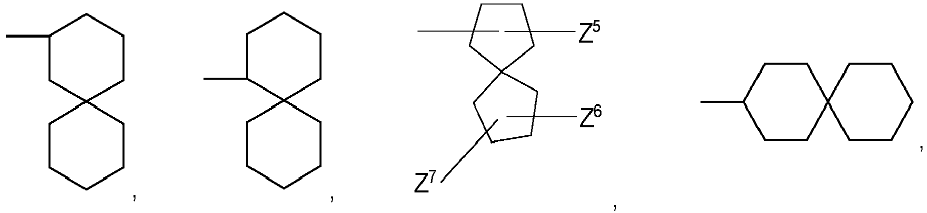 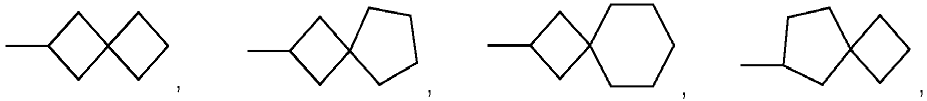 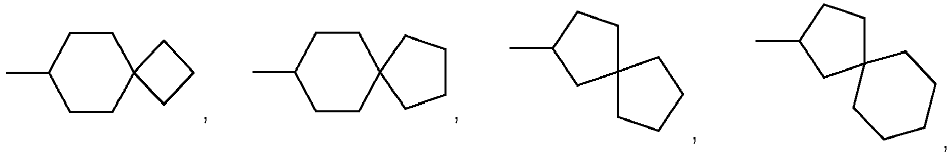 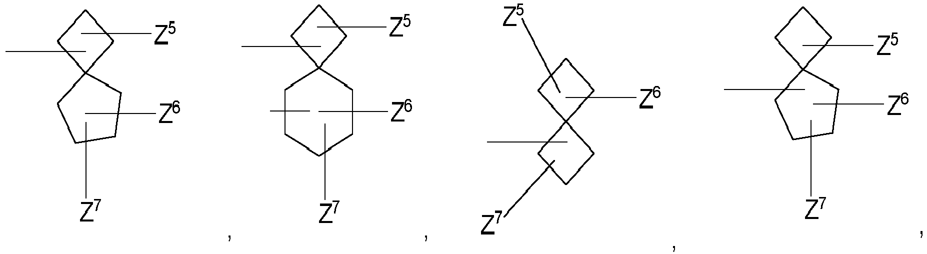 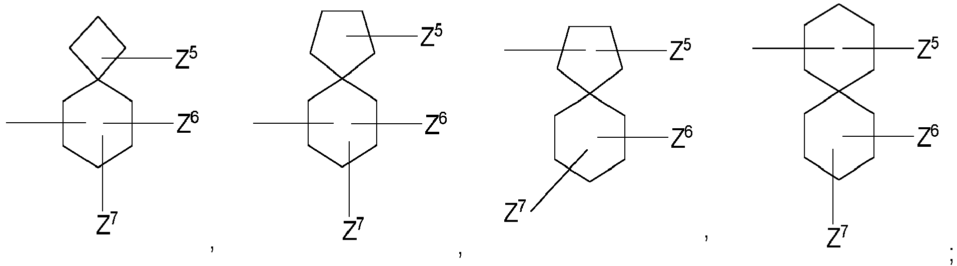 preferred substituents Z⁵, Z⁶ and Z⁷ are -H, -CH₃, -C₂H₅, -C₃H₇, -CH(CH₃)₂, -OH, -OCH₃, -OC₂H₅, -OC₃H₇, -NH₂, -N(CH₃)₂, -F, -CI, -Br, -I, -CN, -CH₂F, -CHF₂, -CF₃, -OCHF₂, -OCF₃, more preferably, at least one of Z⁵, Z⁶ and Z⁷ is not -H.

As used herein, the term **"C₅-C₁₄-spiroheterocyclyl"** may be represented by generalized formula **(b4)** 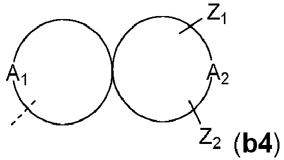
wherein, A₁, and A₂, represent independently C₂-C₆ alkylene and at least one carbon atom of said C₂-C₆ alkylene is replaced with heteroatoms selected from O, N, and S; and
C₅-C₁₄ spiroheterocyclyl ring may be optionally substituted with 1 to 5 substituents selected from Z¹ to Z¹², R^{N1} and R^{N2}, preferably substituted by R^{N1}, Z¹ and Z² and have 0 - 2 double bonds.

As used herein, the term **"Cₛ-C₁₄-spiroheterocyclyl"** refers to spiro residues with one, two or three heteroatoms such as O, S, N in the spiro ring system, wherein these spiroheterocyclic residues can be be optionally substituted with 1 to 5 substituents selected from Z¹ to Z¹², R^{N1} and R^{N2}, preferably substituted with 1 to 3 substituents selected from R^{N1}, Z⁵, Z⁶ and Z⁷. However it is clear to a skilled person that the term "can be substituted" refers to the replacement of a hydrogen atom by one of the substituents R^{N1}, Z⁵, Z⁶ and Z⁷. The carbon atom number of **C₅-C₁₄** refers only to the carbon atoms of the spiro ring system and does not include the carbon atoms of the substituents. Thus a azaspiro[4,5]decyl residue is counted as a C₉-spiroalkyl regardless if this azaspiro[4,5]decyl residue carries five isopropyl substituents.

Examples of preferred C₅-C₁₄-spiroheterocyclyl groups and substituted C₅-C₁₄-spiroheterocyclyl groups are 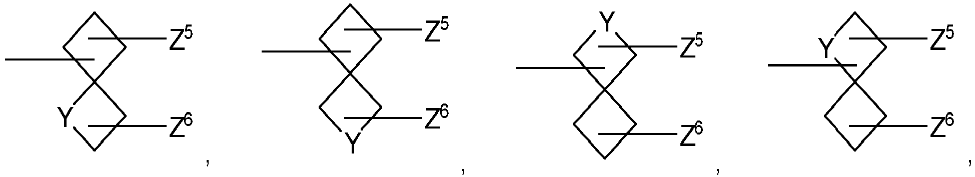 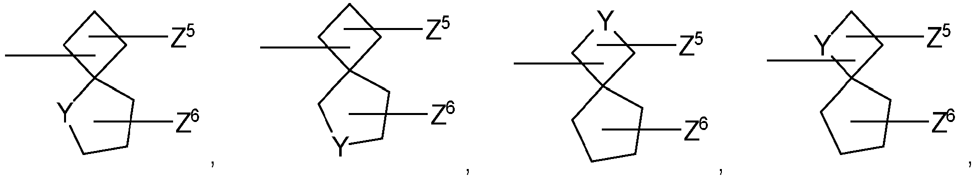 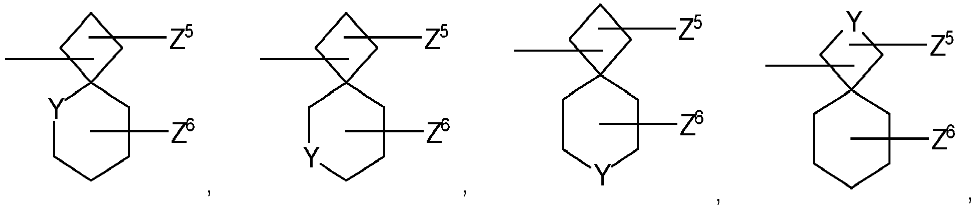 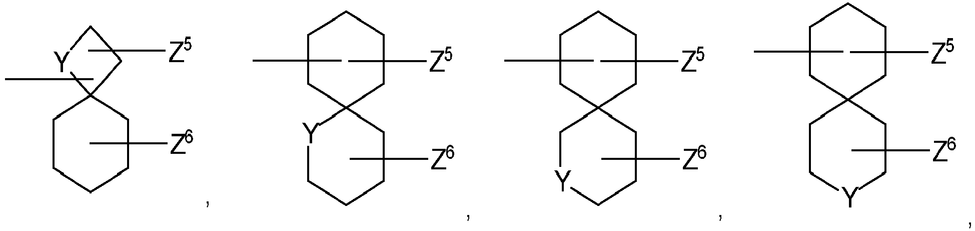 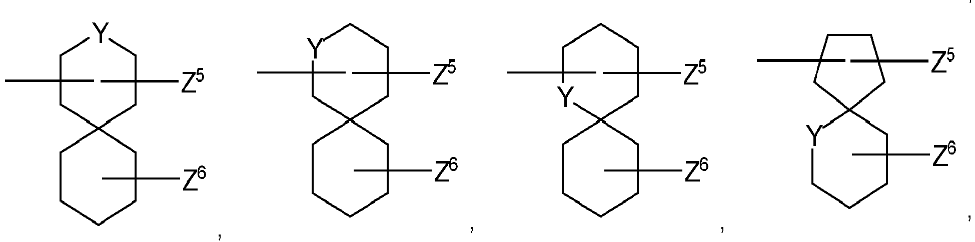 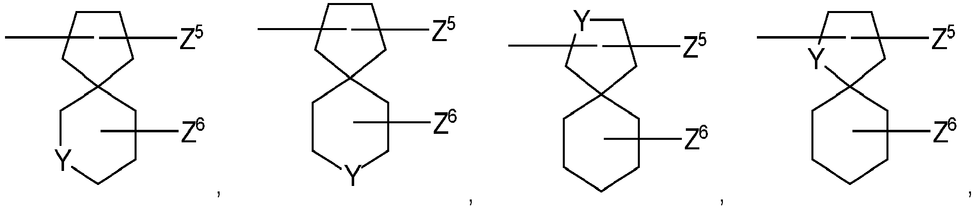 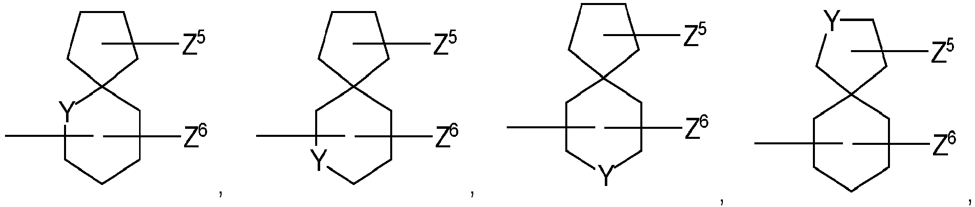 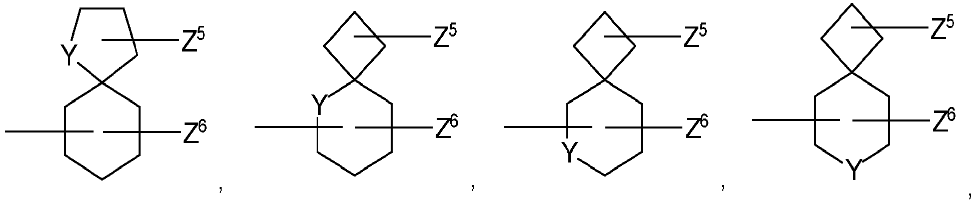 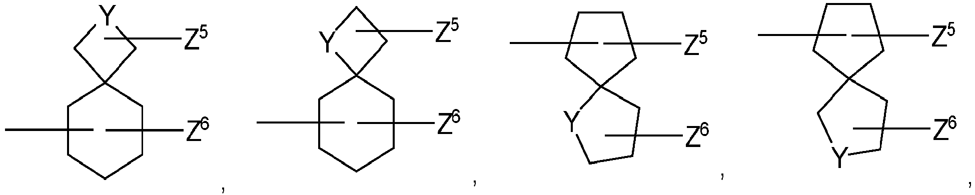 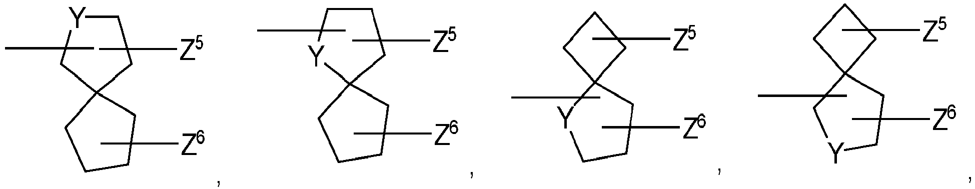 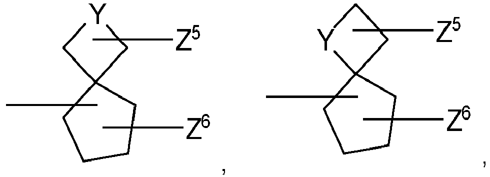 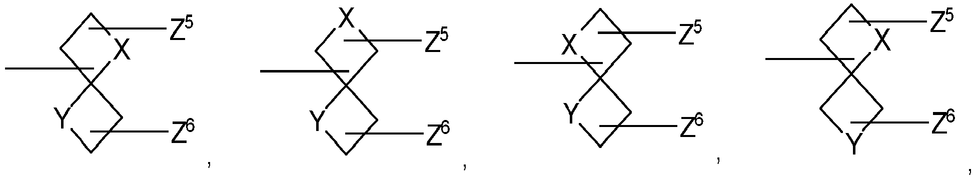 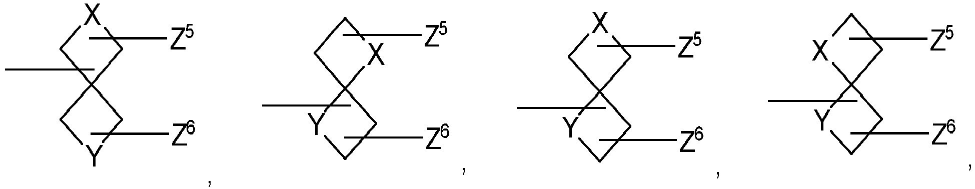 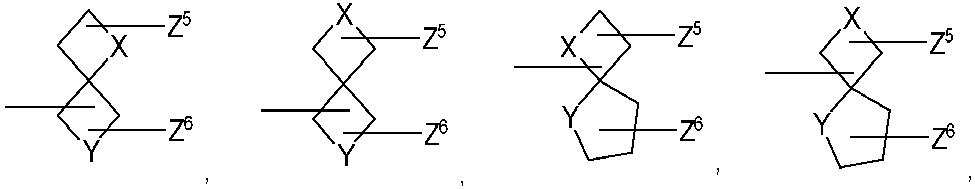 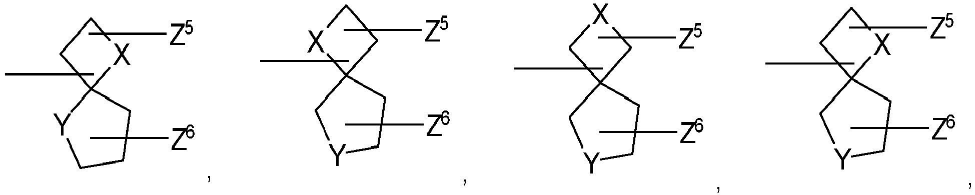 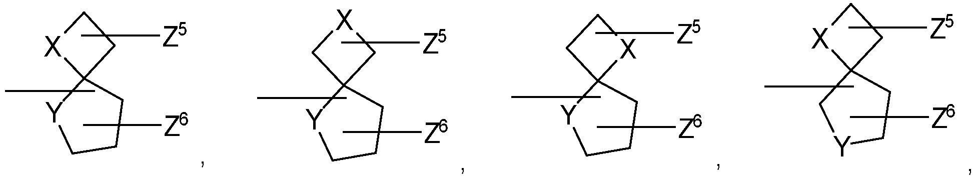 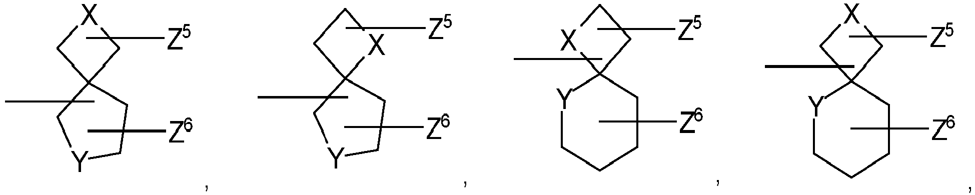 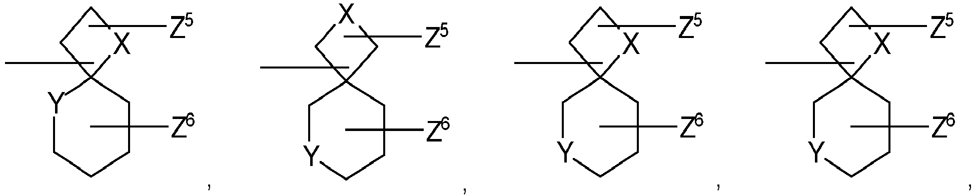 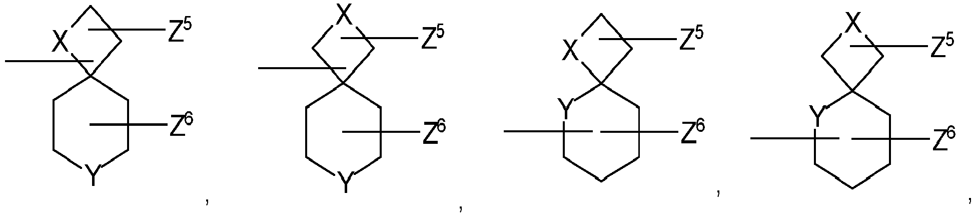 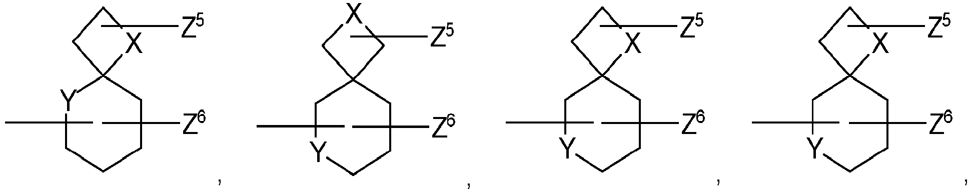 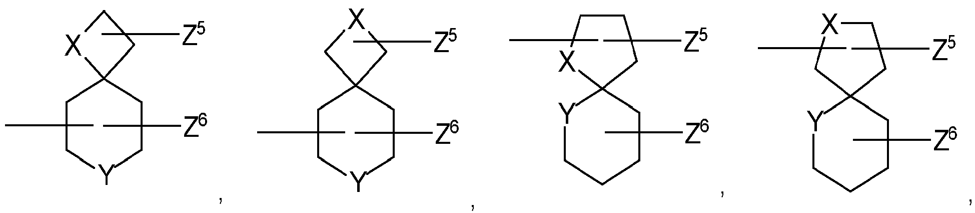 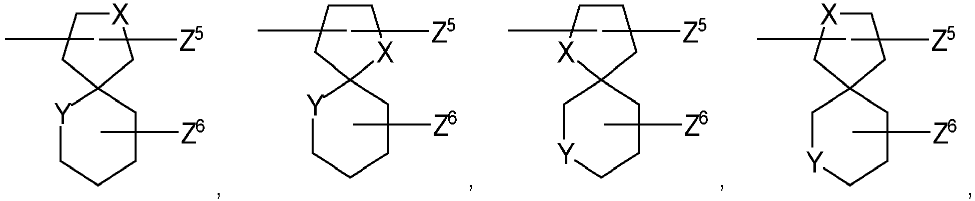 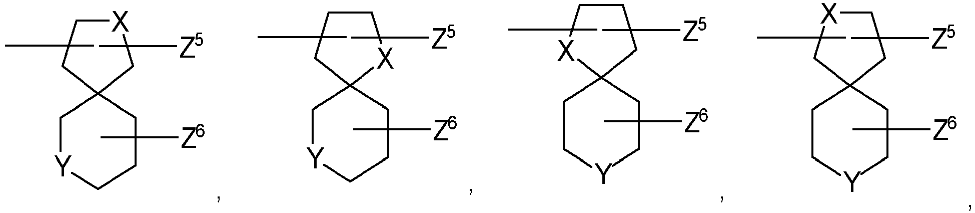 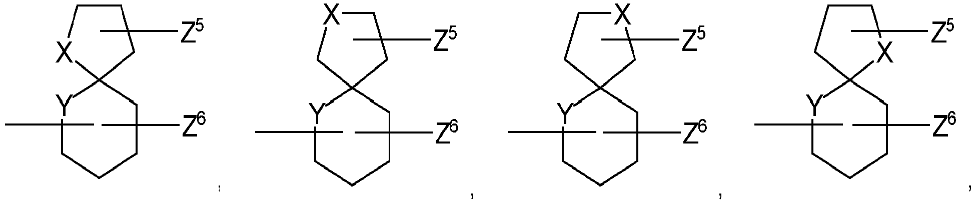 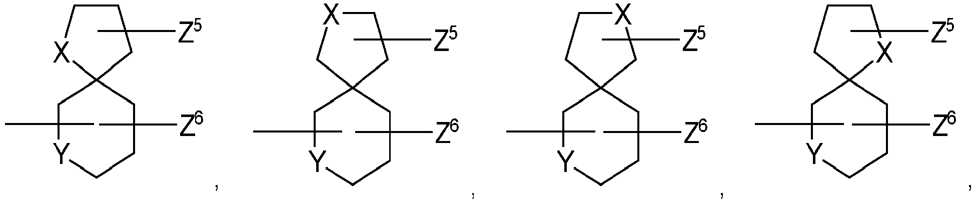 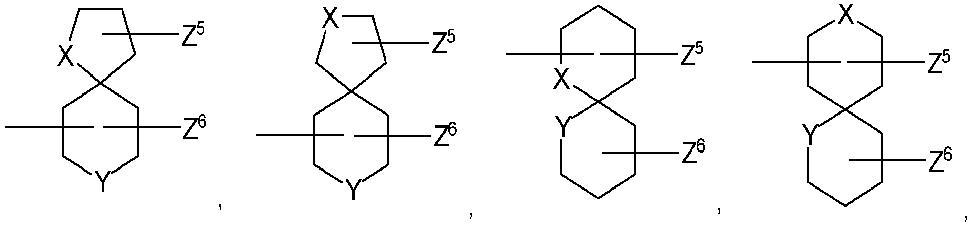 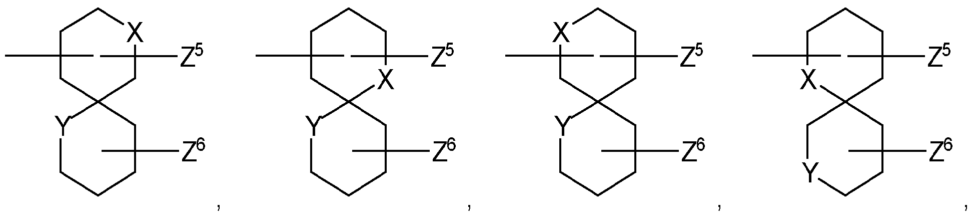 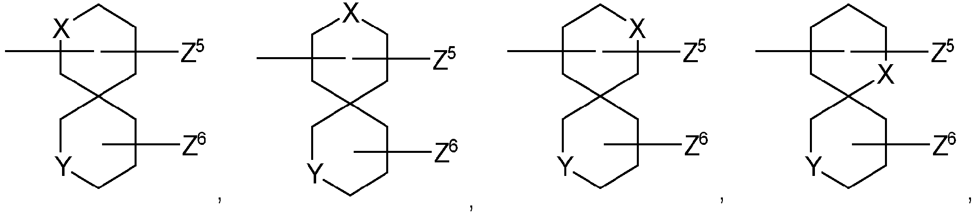 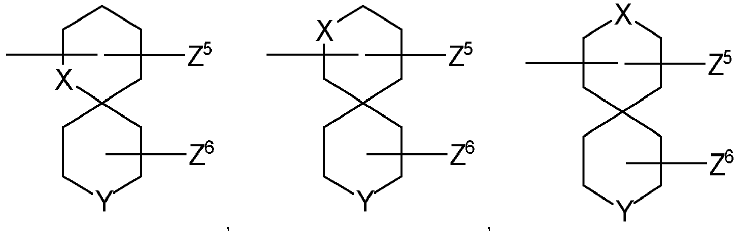 wherein Y and X represent independently of each other -O-, -NH-, -NR^{N1}-, -SO-, or -SO₂-, preferably -NH- and -NR^{N1}- and Z⁵ and Z⁶ represent independently of each other -H, -CH₃, -C₂H₅, -C₃H₇, -CH(CH₃)₂, -OH, -OCH₃, - OC₂H₅, -OC₃H₇, -NH₂, -N(CH₃)₂, -F, -CI, -Br, -I, -CN, -CH₂F, -CHF₂, -CF₃, -OCHF₂, or -OCF₃, more preferably, at least one of Z⁵, and Z⁶ is not -H.

Examples of preferred 4-membered heterocyclic groups and substituted 4-membered heterocyclic groups for **R⁸** or **R⁹** are

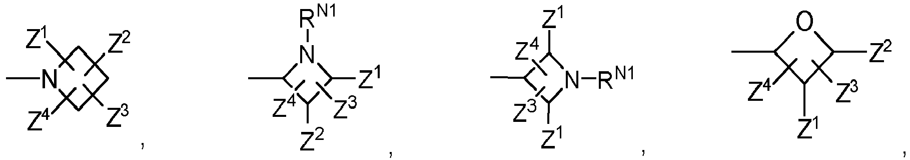

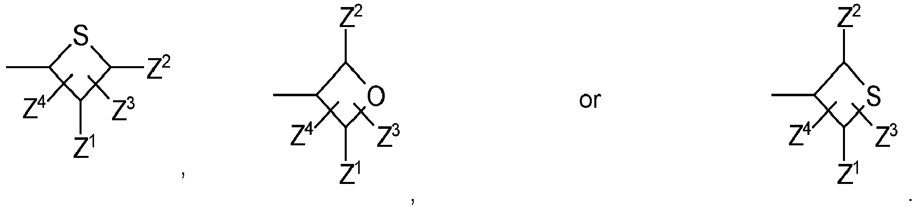

Preferably Z¹ to Z⁴ represent independently of each other -H, -CH₃, -C₂H₅, -C₃H₇, -CH(CH₃)₂, -OH, -OCH₃, -OC₂H₅, -OC₃H₇, -NH₂, -N(CH₃)₂, -F, -CI, -Br, -I, -CN, -CH₂F, -CHF₂, -CF₃, -OCHF₂, and -OCF₃, more preferably, at least one of Z¹ to Z⁴ is not -H.

Preferred substituents for R^{N1} are **-R¹⁵,** -H, -CH₃, -C₂H₅, -C₃H₇, -CH(CH₃)₂, -CHF₂, -CF₃, 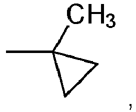 cyclo-C₃H₅, cyclo-C₄H₇, cyclo-C₅H₉, cyclo-C₆H₁₁, cyclo-C₇H₁₃, -C₄H₉, -CH₂₋CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -Ph, -CH₂₋Ph, -CH₂-CH₂-Ph, -C₂H₄-CH=CH₂, -CH₂-CH=CH-CH₃, -CH₂-C(CH₃)=CH₂, -CH(CH₃)-CH=CH₂, -CH₂-CH=C(CH₃)₂, -CH₂-C≡CH, -C₂H₄-C≡CH, -CH₂-C≡C-CH₃, -COCH₃, -COC₂H₅, -COC₃H₇, -CO-cyclo-C₃H₅, -COCH(CH₃)₂, -COC(CH₃)₃, -COOH, -COOCH₃, -COOC₂H₅, -COOC₃H₇, -COO-cyclo-C₃H₅, -COOCH(CH₃)₂, -COOC(CH₃)₃, -COOCH₂Ph, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, or -SO₃H.

Preferred 4-membered heterocyclyl groups are

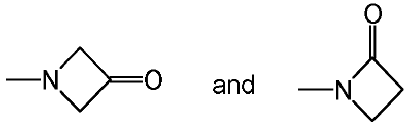

Examples of preferred 5-membered heterocyclyl groups and substituted 5-membered heterocyclyl groups for **R⁸** or **R⁹** are substituted or non substituted ring systems of five atoms including at least one heteroatom such as O, S, SO, SO₂, N, NO, wherein these 5-membered heterocyclic residues can be substituted with 1 to 4 substituents selected from R^{N1}, R^{N2}, Z¹, Z², Z³ and Z⁴. It is also possible that two of the substituents Z¹, Z², Z³ and Z⁴ represent together an oxygen atom and form together with the ring carbon atom of the heterocyclic ring to which they are both attached a carbonyl moiety or a sulfoxide moiety together with the ring sulphur atom to which they are attached or both Z substituents represent oxygen and form a sulfone moiety together with the ring sulphur atom to which they are attached. If the 5-membered heterocyclic residue contains a nitrogen atom which is substituted by R^{N1}. If the 5-membered heterocyclic residue contains two nitrogen atoms which are both substituted by one of the substituents R^{N1}, R^{N2}, the first Z substituent represents R^{N1}, and the second Z substituent represents R^{N2},. The same definition applies for the substituent R⁹ with the only difference that the optional substituents of the 5-membered heterocyclyl residue are Z⁵ to Z⁷ instead of Z¹ to Z⁴. Thus for R⁹ the optional substituent Z¹ is replaced by Z⁵, Z² is replaced by Z⁶, Z³ is replaced by Z⁷, and Z⁴ is hydrogen.

Examples of preferred 5-membered heterocyclic groups and substituted 5-membered heterocyclic groups for R⁸ or R⁹ are 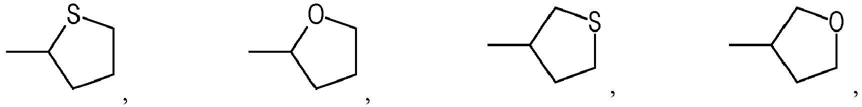 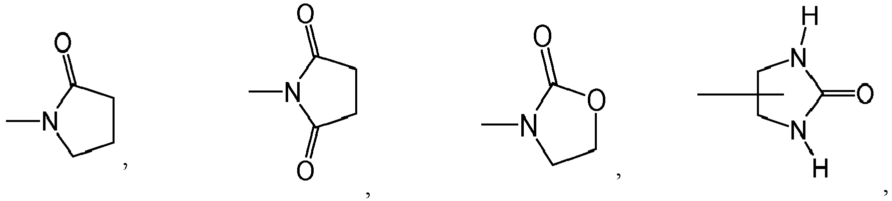 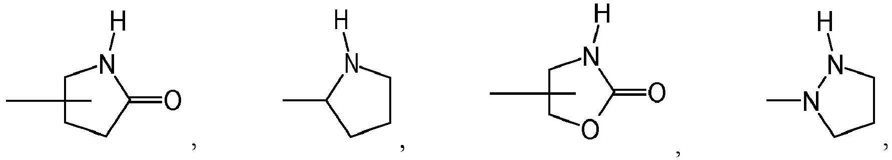 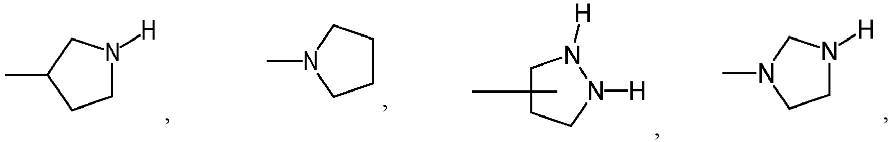 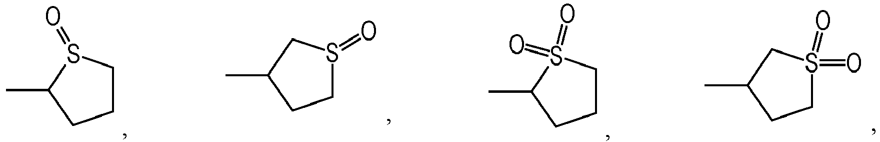 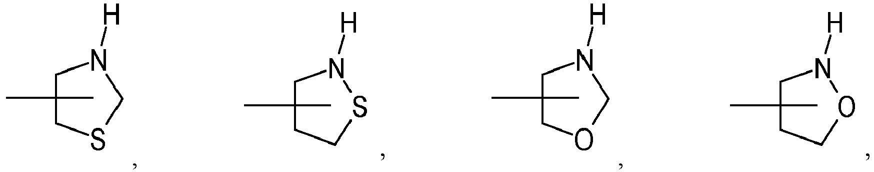 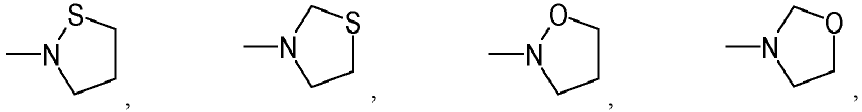 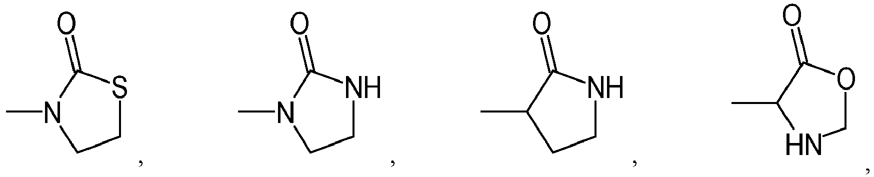 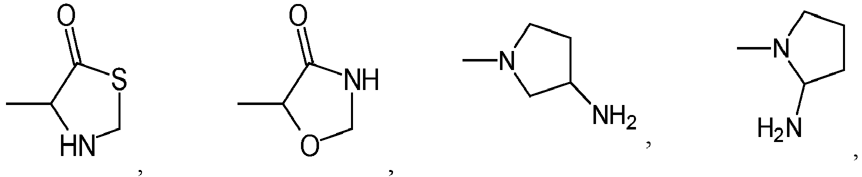 wherein the afore-mentioned 5-membered heterocyclic groups can be substituted with 1 to 4 substituents selected from R^{N1},Z¹, Z², Z³ and Z⁴.

Examples of preferred 6-membered heterocyclic groups and substituted 6-membered heterocyclic groups for R⁸ or R⁹ are 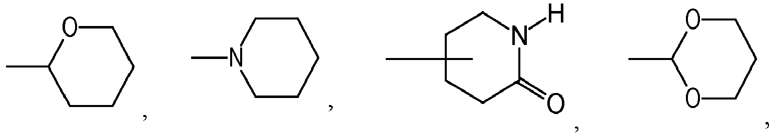 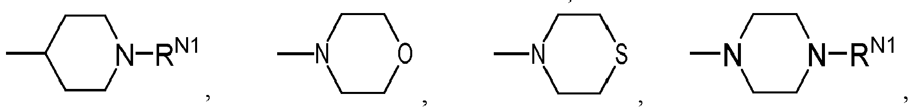 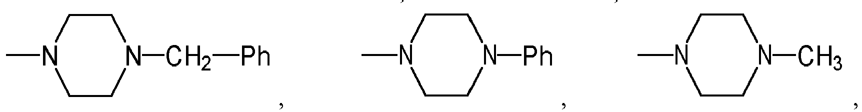 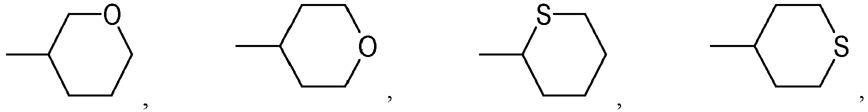 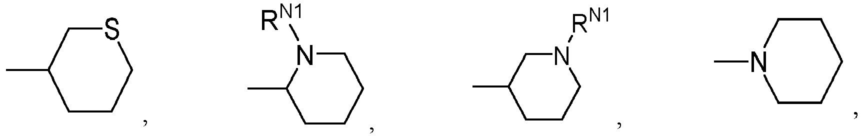 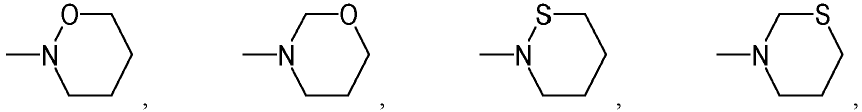 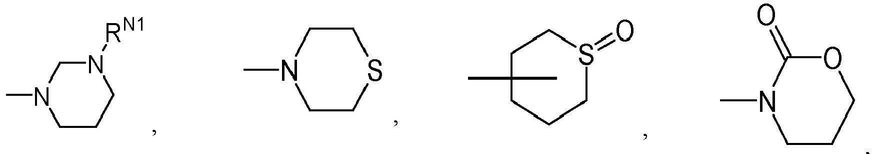 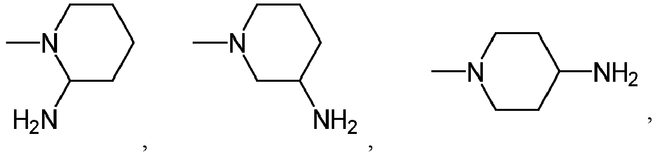 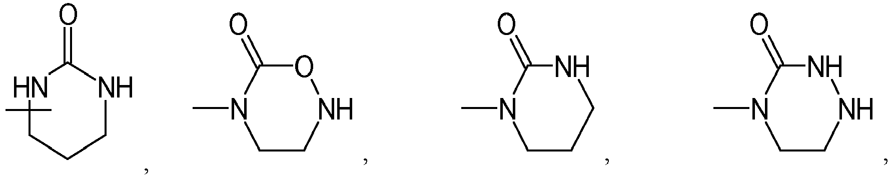 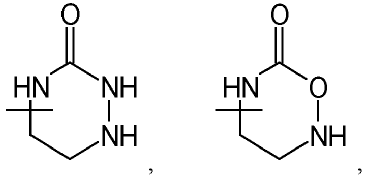 wherein the afore-mentioned 6-membered heterocyclic groups can be substituted with 1 to 4 substituents selected from Z¹, Z², Z³ and Z⁴. Preferred residues for the substituents Z¹ to Z⁴ are disclosed above.

Examples of preferred "monounsaturated 4-membered heterocyclyl" groups and substituted "monounsaturated 4-membered heterocyclyl" groups for **R⁸** or **R⁹** are substituted or non substituted ring systems of four atoms including at least one heteroatom such as O, S, SO, SO₂, N, NO, and one double bond, wherein these monounsaturated 4-membered heterocyclic residues can be substituted with 1 to 4 substituents selected from R^{N1}, R^{N2}, Z¹, Z², Z³ and Z⁴. However it is clear to a skilled person that the term "can be substituted" refers to the replacement of a hydrogen atom by one of the substituents R^{N1}, R^{N2}, Z¹, Z², Z³ or Z⁴. Moreover it is clear to a skilled person that only these hydrogen atoms which are present in the monounsaturated 4-membered heterocyclic residue can be replaced by the substituents R^{N1}, R^{N2}, Z¹, Z², Z³ and Z⁴. It is also possible that two of the substituents Z¹, Z², Z³ and Z⁴ represent together an oxygen atom and form together with the ring carbon atom of the heterocyclic ring to which they are both attached a carbonyl moiety or a sulfoxide moiety together with the ring sulphur atom to which they are attached or both Z substituents represent oxygen and form a sulfone moiety together with the ring sulphur atom to which they are attached. If the monounsaturated 4-membered heterocyclic residue contains a nitrogen atom, which is substituted by R^{N1}. If the monounsaturated 4-membered heterocyclic residue contains two nitrogen atoms which are both substituted by R^{N1}, R^{N2}, the first substituent represents R^{N1}, and the second substituent represents R^{N2}.

Examples of preferred monounsaturated 4-membered heterocyclic groups and substituted 4-membered heterocyclic groups for R⁸ or R⁹ are

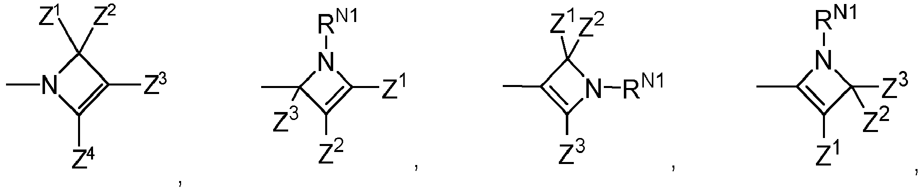

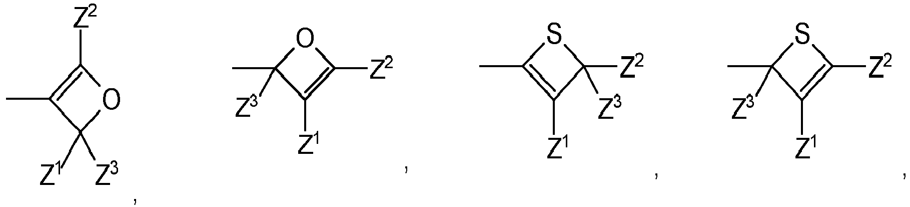

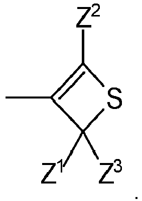

Preferably Z¹ to Z⁴ represent independently of each other -H, -CH₃, -C₂H₅, -C₃H₇, -CH(CH₃)₂, -OH, -OCH₃, -OC₂H₅, -OC₃H₇, -NH₂, -N(CH₃)₂, -F, -CI, -Br, -l, -CN, -CH₂F, -CHF₂, -CF₃, -OCHF₂, and -OCF₃.

Preferred substituents for R^{N1} are -**R¹⁵**, -H, -CH₃, -C₂H₅, -C₃H₇, -CH(CH₃)₂, -CHF₂, -CF₃, 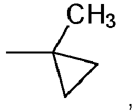 cyclo-C₃H₅, cyclo-C₄H₇, cyclo-C₅H₉, cyclo-C₆H₁₁, cyclo-C₇H₁₃, -C₄H₉, -CH₂-CH(CH₃)₂, -CH(CH₃)-C₂H₅, -C(CH₃)₃, -C₅H₁₁, -CH(CH₃)-C₃H₇, -CH₂₋CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -Ph, -CH₂-Ph, -CH₂-CH₂-Ph, -C₂H₄-CH=CH₂, -CH₂-CH=CH-CH₃, -CH₂-C(CH₃)=CH₂, -CH(CH₃)-CH=CH₂, -CH₂-CH=C(CH₃)₂, -CH₂-C≡CH, -C₂H₄-C≡CH, -CH₂-C≡C-CH₃, -COCH₃, -COC₂H₅, -COC₃H₇, -CO-cyclo-C₃H₅, -COCH(CH₃)₂, -COC(CH₃)₃, -COOH, -COOCH₃, -COOC₂H₅, -COOC₃H₇, -COO-cyclo-C₃H₅, -COOCH(CH₃)₂, -COOC(CH₃)₃, -COOCH₂Ph, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, or -SO₃H.

Examples of preferred "monounsaturated 5-membered heterocyclyl" groups and substituted "monounsaturated 5-membered heterocyclyl" groups for **R⁸** or **R⁹** refers to substituted or non substituted ring systems of five atoms including at least one heteroatom such as O, S, SO, SO₂, N, NO, and one double bond, wherein these monounsaturated 5-membered heterocyclic residues can be substituted with 1 to 4 substituents selected from R^{N1}, R^{N2}, Z¹, Z², Z³ and Z⁴. It is also possible that two of the substituents R^{N1}, R^{N2}, Z¹, Z², Z³ and Z⁴ represent together an oxygen atom and form together with the ring carbon atom of the heterocyclic ring to which they are both attached a carbonyl moiety or a sulfoxide moiety together with the ring sulphur atom to which they are attached or both Z substituents represent oxygen and form a sulfone moiety together with the ring sulphur atom to which they are attached. If the monounsaturated 5-membered heterocyclic residue contains a nitrogen atom which is substituted by R^{N1}. If the monounsaturated 5-membered heterocyclic residue contains two nitrogen atoms which are both substituted by R^{N1}, R^{N2}, the first substituent represents R^{N1} and the second substituent represents R^{N2}.

Examples of preferred monounsaturated 5-membered heterocyclic groups and substituted 5-membered heterocyclic groups for R³ are 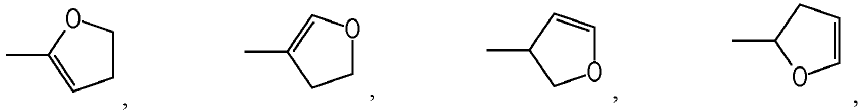 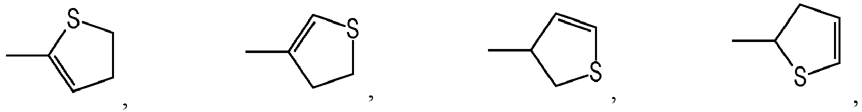 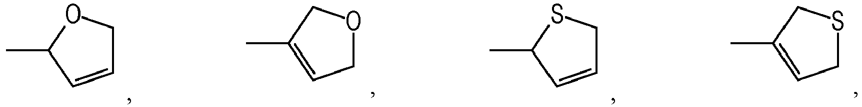 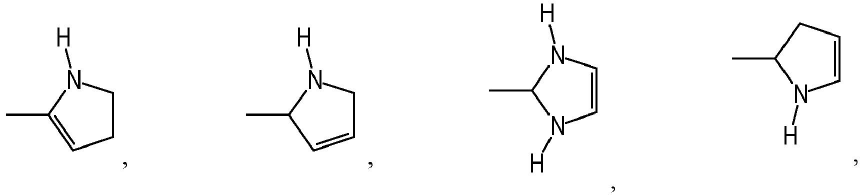 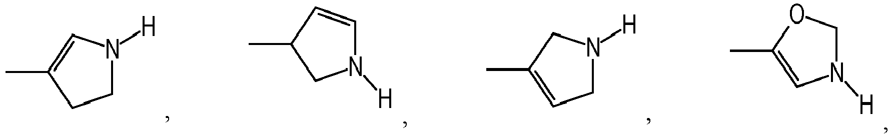 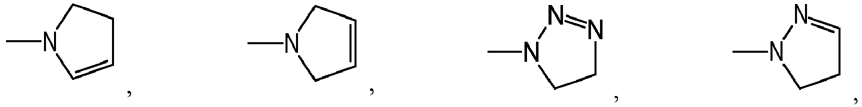 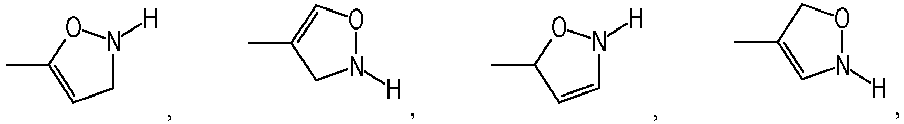 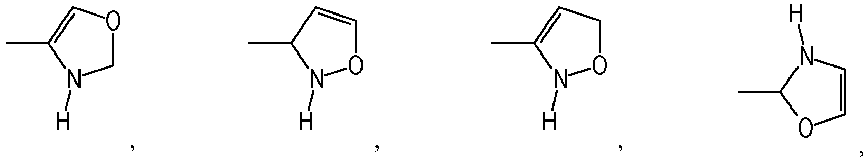 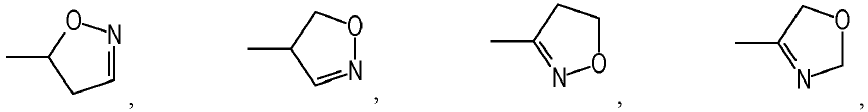 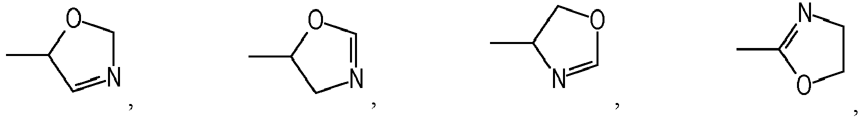 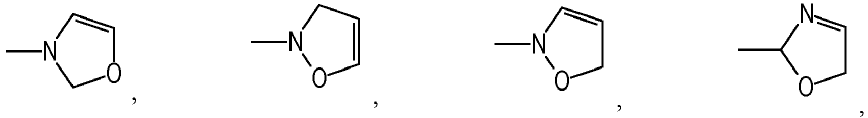 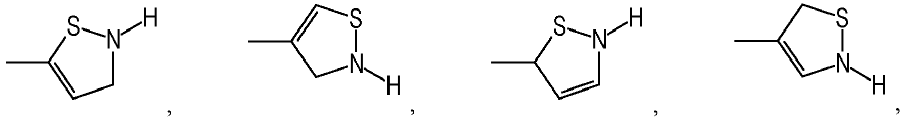 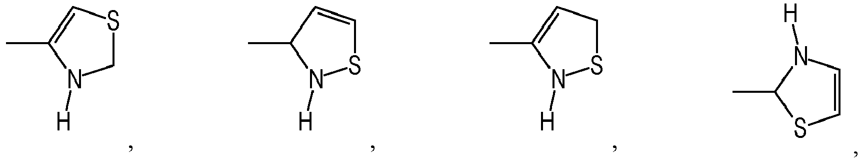 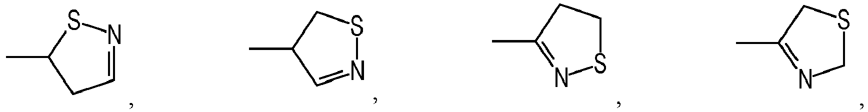 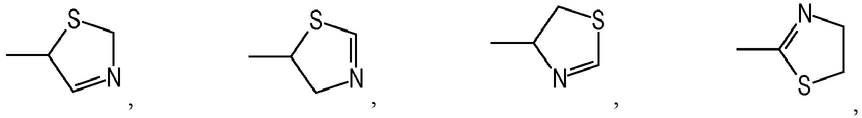 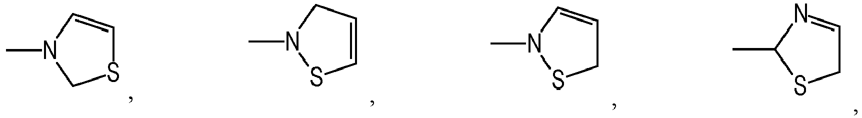 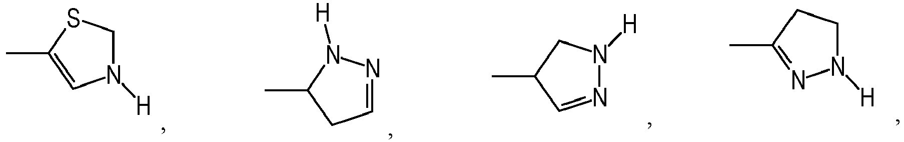 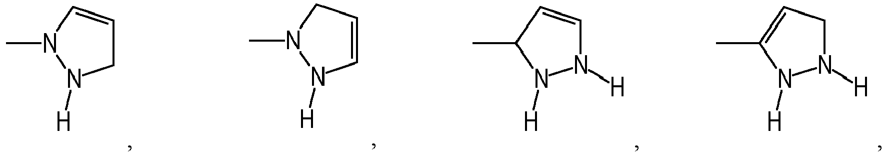 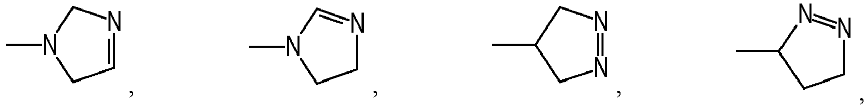 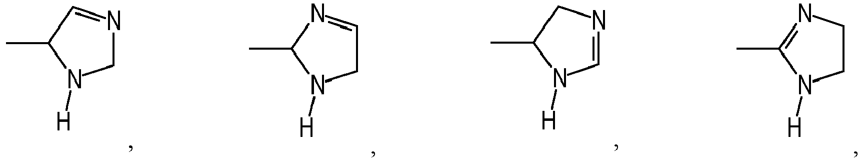 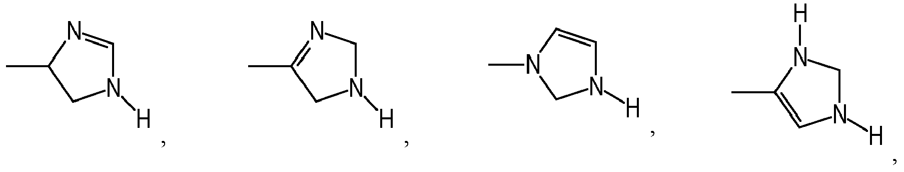 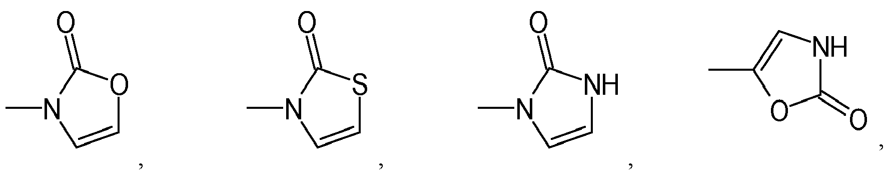 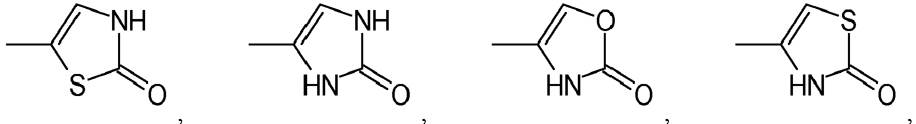 wherein the afore-mentioned monounsaturated 5-membered heterocyclic groups can be substituted with 1 to 4 substituents selected from R^{N1}, R^{N2}, Z¹, Z², Z³ and Z⁴.

R⁸, or R⁹ represents preferably the following spiroheterocyclyl or C₅-C₁₄/N₀-N₂/O₀- O₂/S₀-S₁-spiroheterocyclyl residues: spiro[2,3]heterohexyl, spiro[2,4]heteroheptyl, spiro[2,5]heterooctyl, spiro[2,7]heterononyl, spiro[3,3]heteroheptyl, spiro[3,4]heterooctyl, spiro[3,5]heterononyl, spiro[3,6]heterodecyl, spiro[4,4]heterononyl, spiro[4,5]heterodecyl, spiro[4,6]heteroundecyl, spiro[5,5]heteroundecyl, spiro[5,6]heterododecyl, spiro[6,6]heterotridecyl, wherein the afore-mentioned spiroheterocyclyl or C₅-C₁₄/N₀-N₂/O₀-O₂/S₀-S₁-spiroheterocyclyl residues are linked through a ring carbon atom to the rest of the molecule and wherein the afore-mentioned spiroheterocyclyl or C₅-C₁₄/N₀-N₂/O₀-O₂/S₀-S₁-spiroheterocyclyl residues are optionally substituted with one to three substituents selected from R^{N1}, R^{N2}, Z⁵, Z⁶ and Z⁷. The heteroatom in the afore-mentioned spiroheterocyclyl or C₅-C₁₄/N₀- N₂/O₀-O₂/S₀-S₁-spiroheterocyclyl residues is preferably selected from -O-, -NH-, -NR^{N1}-, -NR^{N2}-, -SO-, and -SO₂-.

More preferably R⁸, or R⁹ represents preferably the following spiroheterocyclyl or C₅- C₁₄/N₀-N₂/O₀-O₂/S₀-S₁-spiroheterocyclyl residues: azaspiro[3,3]heptyl, azaspiro[3,4]octyl, azaspiro[3,5]nonyl, azaspiro[3,6]decyl, azaspiro[4,4]nonyl, azaspiro[4,5]decyl, azaspiro[4,6]undecyl, azaspiro[5,5]undecyl, azaspiro[5,6]dodecyl, azaspiro[6,6]tridecyl, diazaspiro[3,3]heptyl, diazaspiro[3,4]octyl, diazaspiro[3,5]nonyl, diazaspiro[3,6]decyl, diazaspiro[4,4]nonyl, diazaspiro[4,5]decyl, diazaspiro[4,6]undecyl, diazaspiro[5,5]undecyl, diazaspiro[5,6]dodecyl, diazaspiro[6,6]tridecyl, triazaspiro[3,5]nonyl, triazaspiro[3,6]decyl, triazaspiro[4,5]decyl, triazaspiro[4,6]undecyl, triazaspiro[5,5]undecyl, triazaspiro[5,6]dodecyl, triazaspiro[6,6]tridecyl, oxazaspiro[3,3]heptyl, oxazaspiro[3,4]octyl, oxazaspiro[3,5]nonyl, oxazaspiro[3,6]decyl, oxazaspiro[4,4]nonyl, oxazaspiro[4,5]decyl, oxazaspiro[4,6]undecyl, oxazaspiro[5,5]undecyl, oxazaspiro[5,6]dodecyl, oxazaspiro[6,6]tridecyl, oxadiazaspiro[3,5]nonyl, oxadiazaspiro[3,6]decyl, oxadiazaspiro[4,5]decyl, oxadiazaspiro[4,6]undecyl, oxadiazaspiro[5,5]undecyl, oxadiazaspiro[5,6]dodecyl, oxadiazaspiro[6,6]tridecyl, wherein the afore-mentioned spiroheterocyclyl or C₅-C₁₄/N₀-N₂/O₀-O₂/S₀-S₁- spiroheterocyclyl residues are linked through a ring carbon atom to the rest of the molecule and wherein the afore-mentioned spiroheterocyclyl or C₅-C₁₄/N₀-N₂/O₀- O₂/S₀-S₁-spiroheterocyclyl residues are optionally substituted with one to three substituents selected from R^{N1}, R^{N2}, Z⁵, Z⁶ and Z⁷.

Also, R⁸ or R⁹ represents more preferably the following residues: substituted or unsubstituted 4-membered carbocyclyl, substituted or unsubstituted 5-membered carbocyclyl, substituted or unsubstituted 6-membered carbocyclyl, 4-membered heterocyclyl, 5-membered heterocyclyl, 6-membered heterocyclyl, substituted 4-membered heterocyclyl, substituted 5-membered heterocyclyl, substituted 6-membered heterocyclyl, 4-membered nitrogenheterocyclyl, 5-membered nitrogenheterocyclyl, 6-membered nitrogenheterocyclyl, substituted 4-membered nitrogenheterocyclyl, substituted 5-membered nitrogenheterocyclyl, substituted 6-membered nitrogenheterocyclyl, spiro[2,3]heterohexyl, spiro[2,4]heteroheptyl, spiro[2,5]heterooctyl, spiro[2,7]heterononyl, spiro[3,3]heteroheptyl, spiro[3,4]heterooctyl, spiro[3,5]heterononyl, spiro[3,6]heterodecyl, spiro[4,4]heterononyl, spiro[4,5]heterodecyl, spiro[4,6]heteroundecyl, spiro[5,5]heteroundecyl, spiro[5,6]heterododecyl, spiro[6,6]heterotridecyl, substituted spiro[2,3]heterohexyl, substituted spiro[2,4]heteroheptyl, substituted spiro[2,5]heterooctyl, substituted spiro[2,7]heterononyl, substituted spiro[3,3]heteroheptyl, substituted spiro[3,4]heterooctyl, substituted spiro[3,5]heterononyl, substituted spiro[3,6]heterodecyl, substituted spiro[4,4]heterononyl, substituted spiro[4,5]heterodecyl, substituted spiro[4,6]heteroundecyl, substituted spiro[5,5]heteroundecyl, substituted spiro[5,6]heterododecyl, substituted spiro[6,6]heterotridecyl, azaspiro[3,3]heptyl, azaspiro[3,4]octyl, azaspiro[3,5]nonyl, azaspiro[3,6]decyl, azaspiro[4,4]nonyl, azaspiro[4,5]decyl, azaspiro[4,6]undecyl, azaspiro[5,5]undecyl, azaspiro[5,6]dodecyl, azaspiro[6,6]tridecyl, substituted azaspiro[3,3]heptyl, substituted azaspiro[3,4]octyl, substituted azaspiro[3,5]nonyl, substituted azaspiro[3,6]decyl, substituted azaspiro[4,4]nonyl, substituted azaspiro[4,5]decyl, substituted azaspiro[4,6]undecyl, substituted azaspiro[5,5]undecyl, substituted azaspiro[5,6]dodecyl, substituted azaspiro[6,6]tridecyl, diazaspiro[3,3]heptyl, diazaspiro[3,4]octyl, diazaspiro[3,5]nonyl, diazaspiro[3,6]decyl, diazaspiro[4,4]nonyl, diazaspiro[4,5]decyl, diazaspiro[4,6]undecyl, diazaspiro[5,5]undecyl, diazaspiro[5,6]dodecyl, diazaspiro[6,6]tridecyl, substituted diazaspiro[3,3]heptyl, substituted diazaspiro[3,4]octyl, substituted diazaspiro[3,5]nonyl, substituted diazaspiro[3,6]decyl, substituted diazaspiro[4,4]nonyl, substituted diazaspiro[4,5]decyl, substituted diazaspiro[4,6]undecyl, substituted diazaspiro[5,5]undecyl, substituted diazaspiro[5,6]dodecyl, substituted diazaspiro[6,6]tridecyl, triazaspiro[3,5]nonyl, triazaspiro[3,6]decyl, triazaspiro[4,5]decyl, triazaspiro[4,6]undecyl, triazaspiro[5,5]undecyl, triazaspiro[5,6]dodecyl, triazaspiro[6,6]tridecyl, substituted triazaspiro[3,5]nonyl, substituted triazaspiro[3,6]decyl, substituted triazaspiro[4,5]decyl, substituted triazaspiro[4,6]undecyl, substituted triazaspiro[5,5]undecyl, substituted triazaspiro[5,6]dodecyl, or substituted triazaspiro[6,6]tridecyl, oxazaspiro[3,3]heptyl, oxazaspiro[3,4]octyl, oxazaspiro[3,5]nonyl, oxazaspiro[3,6]decyl, oxazaspiro[4,4]nonyl, oxazaspiro[4,5]decyl, oxazaspiro[4,6]undecyl, oxazaspiro[5,5]undecyl, oxazaspiro[5,6]dodecyl, oxazaspiro[6,6]tridecyl, substituted oxazaspiro[3,3]heptyl, substituted oxazaspiro[3,4]octyl, substituted oxazaspiro[3,5]nonyl, substituted oxazaspiro[3,6]decyl, substituted oxazaspiro[4,4]nonyl, substituted oxazaspiro[4,5]decyl, substituted oxazaspiro[4,6]undecyl, substituted oxazaspiro[5,5]undecyl, substituted oxazaspiro[5,6]dodecyl, substituted oxazaspiro[6,6]tridecyl, oxadiazaspiro[3,5]nonyl, oxadiazaspiro[3,6]decyl, oxadiazaspiro[4,5]decyl, oxadiazaspiro[4,6]undecyl, oxadiazaspiro[5,5]undecyl, oxadiazaspiro[5,6]dodecyl, oxadiazaspiro[6,6]tridecyl, substituted oxadiazaspiro[3,5]nonyl, substituted oxadiazaspiro[3,6]decyl, substituted oxadiazaspiro[4,5]decyl, substituted oxadiazaspiro[4,6]undecyl, substituted oxadiazaspiro[5,5]undecyl, substituted oxadiazaspiro[5,6]dodecyl, or substituted oxadiazaspiro[6,6]tridecyl, wherein the afore-mentioned substituted or non-substituted spiroheterocyclyl or C₅- C₁₄/N₀-N₂/O₀-O₂/S₀-S₁-spiroheterocyclyl residues are linked through a ring carbon atom to the rest of the molecule and wherein the afore-mentioned substituted or non-substituted spiroheterocyclyl or C₅-C₁₄/N₀-N₂/O₀-O₂/S₀-S₁-spiroheterocyclyl residues are optionally substituted with one to three substituents selected from Z⁵, Z⁶ and Z⁷. The heteroatom in the afore-mentioned substituted or non-substituted spiroheterocyclyl or C₅-C₁₄/N₀-N₂/O₀-O₂/S₀-S₁-spiroheterocyclyl residues is preferably selected from -O-, -NH-, -NR^{N1}-, -SO-, and -SO₂-.

Preferably Z⁵, Z⁶ and Z⁷ represent independently of each other -H, -CH₃, -C₂H₅, -C₃H₇, -CH(CH₃)₂, -OH, -OCH₃, -OC₂H₅, -OC₃H₇, -NH₂, -N(CH₃)₂, -F, -Cl, -Br, -I, -CN, -CH₂F, -CHF₂, -CF₃, -OCHF₂, and -OCF₃.

If present, **R^{N1}** is preferably selected from: -**R¹⁵**, -H, -CH₃, -C₂H₅, -C₃H₇, -CH(CH₃)₂, -CHF₂, -CF₃, 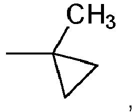 cyclo-C₃H₅, cyclo-C₄H₇, cyclo-C₅H₉, cyclo-C₆H₁₁, cyclo-C₇H₁₃, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -Ph, -CH₂-Ph, -CH₂-CH₂-.Ph, -C₂H₄-CH=CH₂, -CH₂-CH=CH-CH₃, -CH₂-C(CH₃)=CH₂, -CH(CH₃)-CH=CH₂, -CH₂-CH=C(CH₃)₂, -CH₂-C≡CH, -C₂H₄-C≡CH, -CH₂-C≡C-CH₃, -COCH₃, -COC₂H₅, -COC₃H₇, -CO-cyclo-C₃H₅, -COCH(CH₃)₂, -COC(CH₃)₃, -COOH, -COOCH₃, -COOC₂H₅, -COOC₃H₇, -COO-cyclo-C₃H₅, -COOCH(CH₃)₂, -COOC(CH₃)₃, -COOCH₂Ph, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, or -SO₃H.

The term "4-membered nitrogenheterocyclyl" refers to the residue "4-membered heterocyclyl" as defined above, wherein at least one heteroatom is a nitrogen atom and the residue is linked through the at least one nitrogen ring atom to the rest of the molecule and wherein Z¹ is replaced by Z⁵, Z² is replaced by Z⁶, Z³ is replaced by Z⁷, and Z⁴ is hydrogen.

The term "5-membered nitrogenheterocyclyl" refers to the residue "5-membered heterocyclyl" as defined above, wherein at least one heteroatom is a nitrogen atom and the residue is linked through the at least one nitrogen ring atom to the rest of the molecule and wherein Z¹ is replaced by Z⁵, Z² is replaced by Z⁶, Z³ is replaced by Z⁷, and Z⁴ is hydrogen.

The term "6-membered nitrogenheterocyclyl" refers to the residue "6-membered heterocyclyl" as defined above, wherein at least one heteroatom is a nitrogen atom and the residue is linked through the at least one nitrogen ring atom to the rest of the molecule and wherein Z¹ is replaced by Z⁵, Z² is replaced by Z⁶, Z³ is replaced by Z⁷, and Z⁴ is hydrogen.

Still more preferably R⁸ or R⁹ is selected from the following residues: 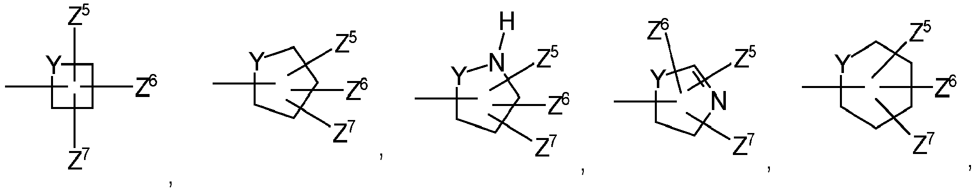 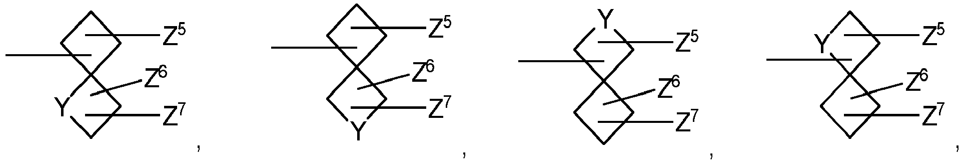 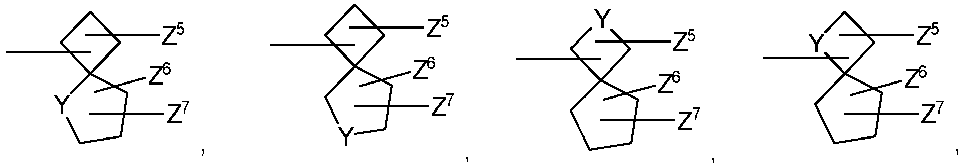 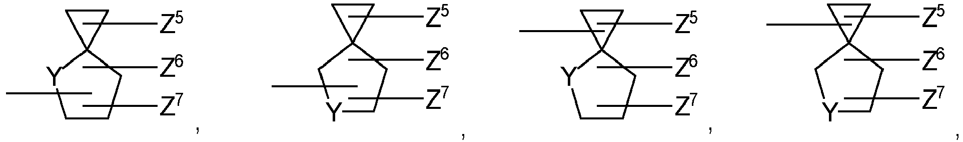 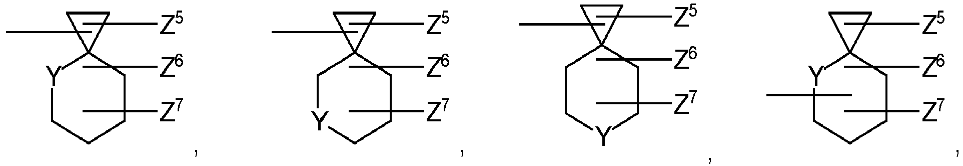 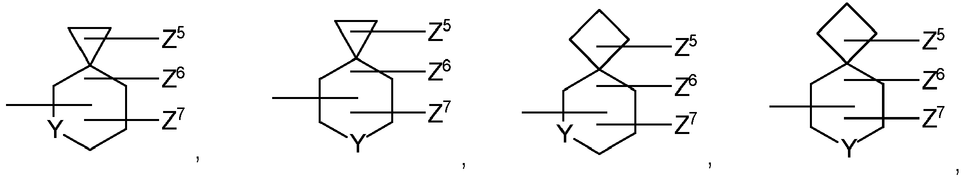 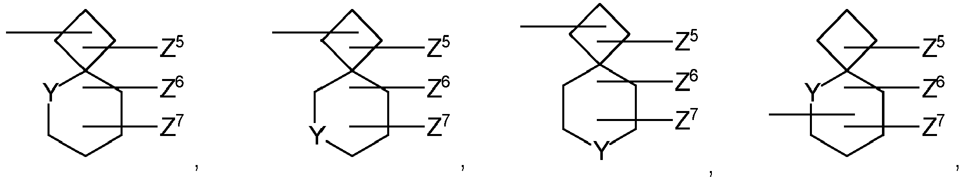 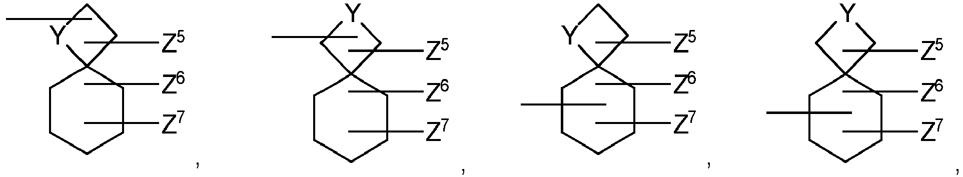 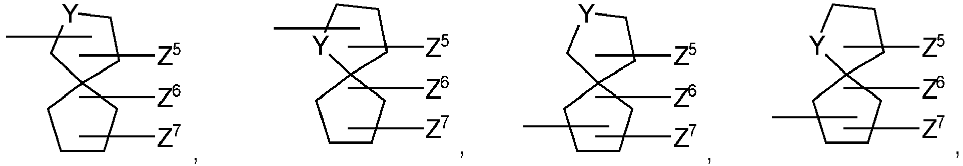 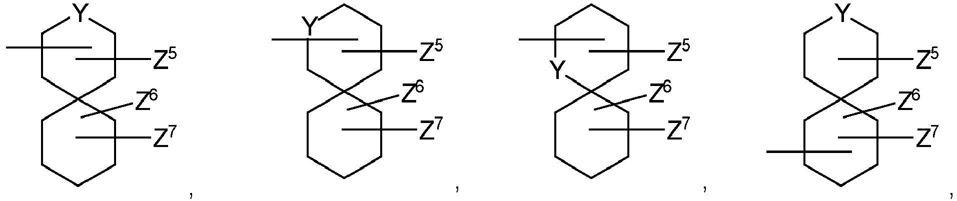 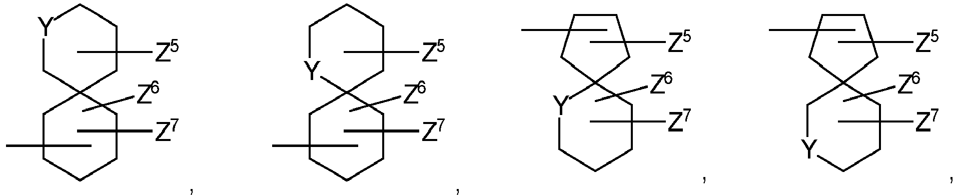 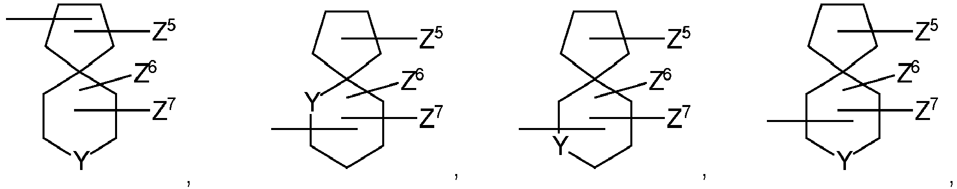 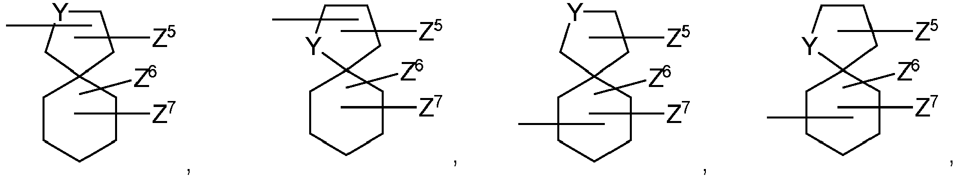 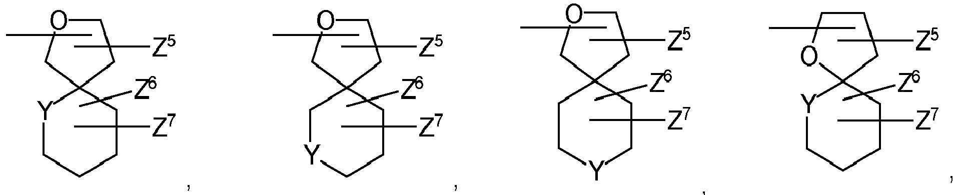 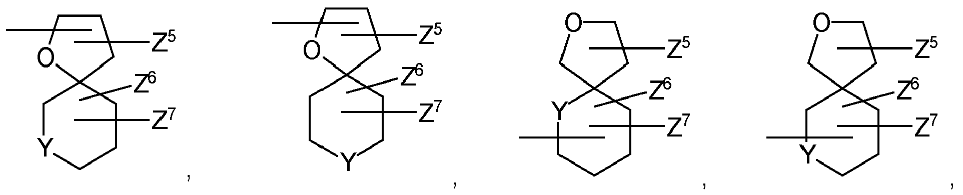 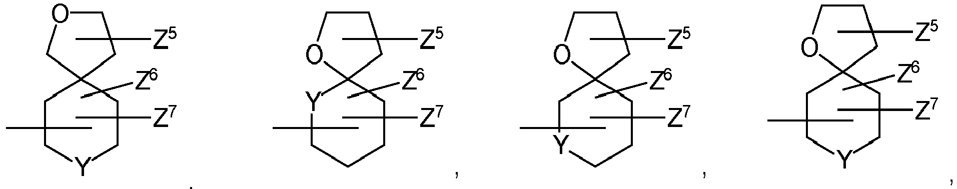 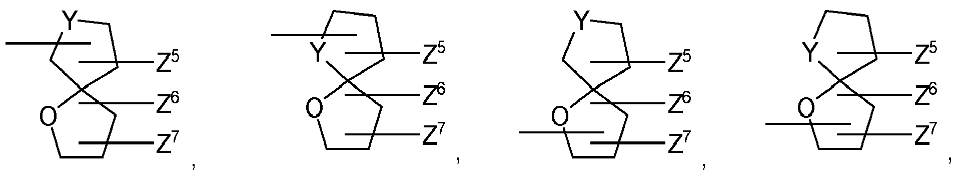 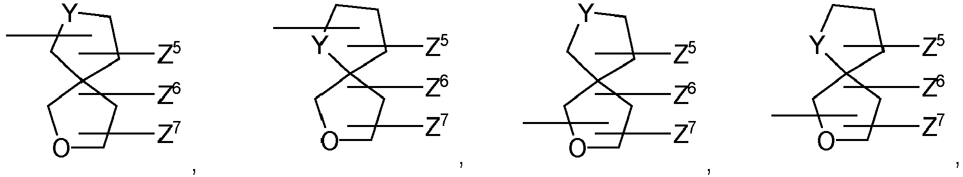 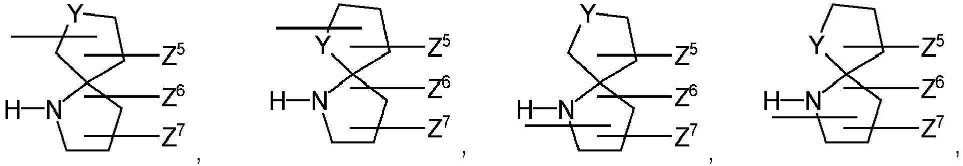 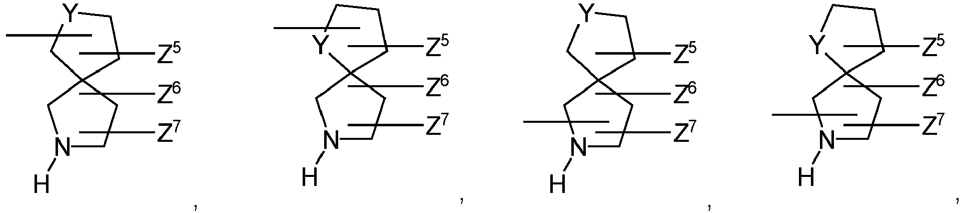 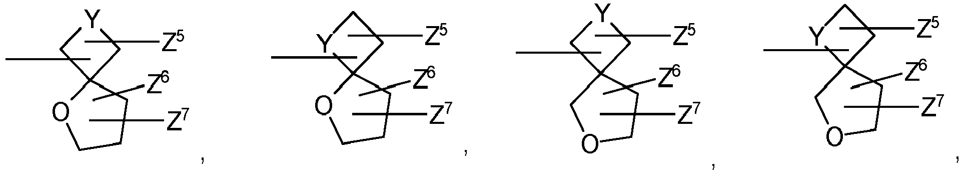 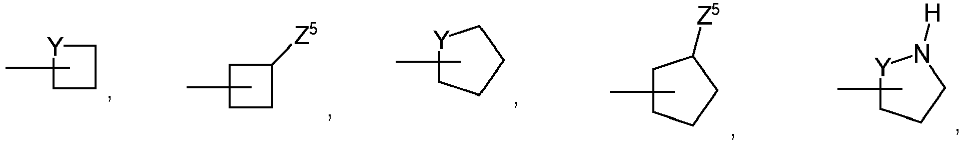 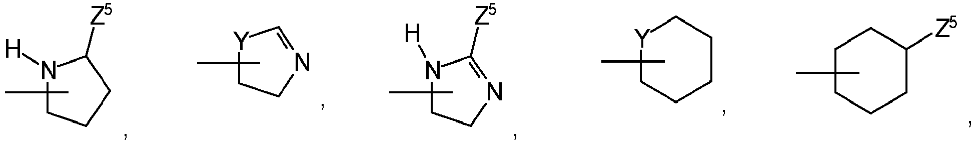 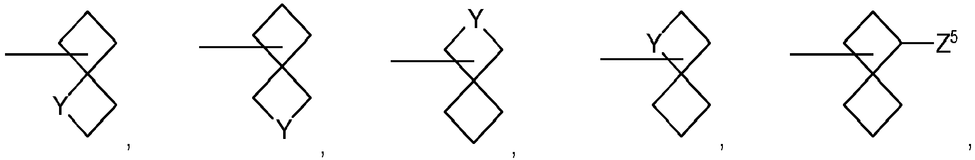 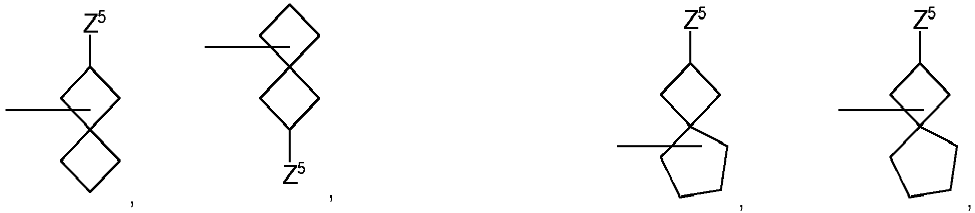 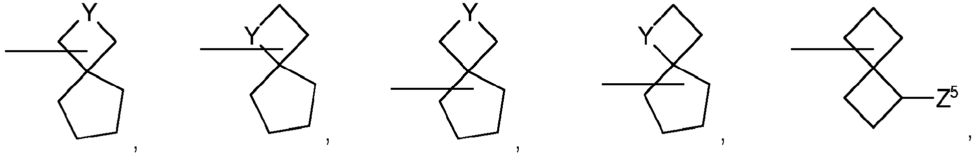 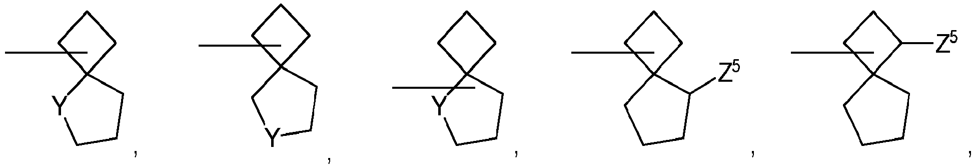 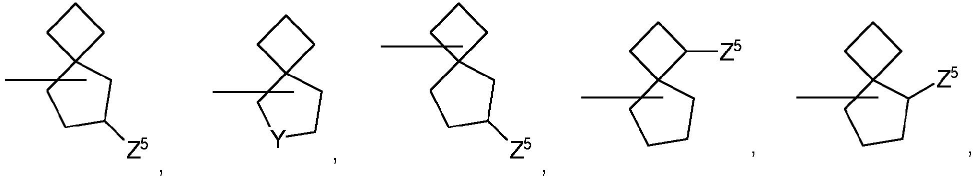 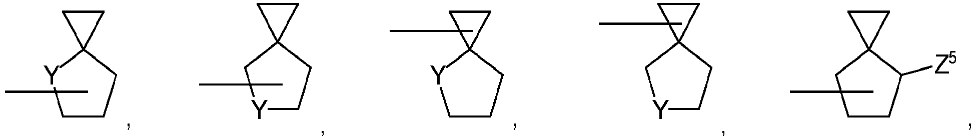 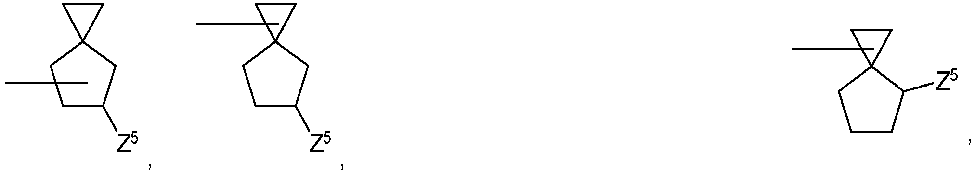 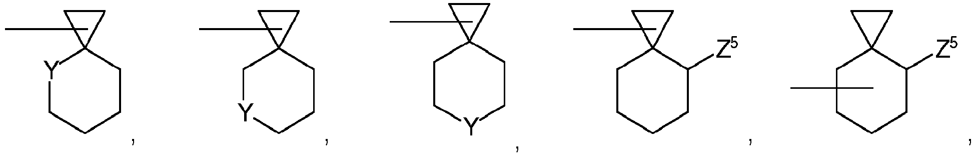 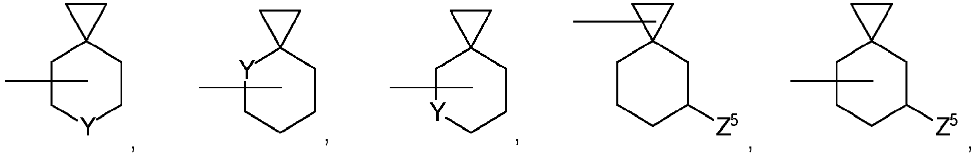 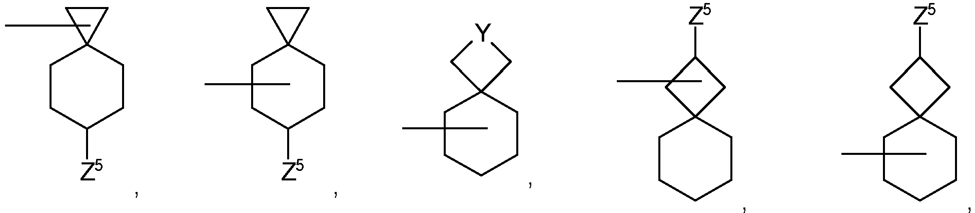 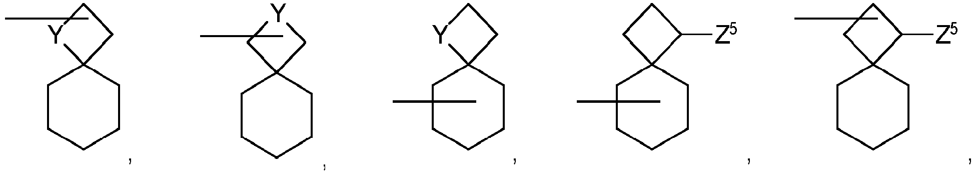 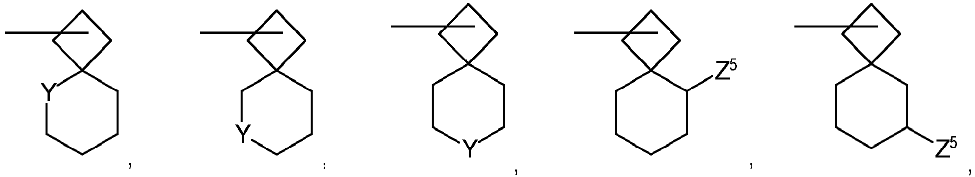 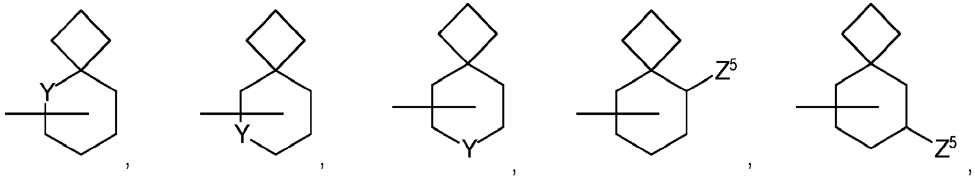 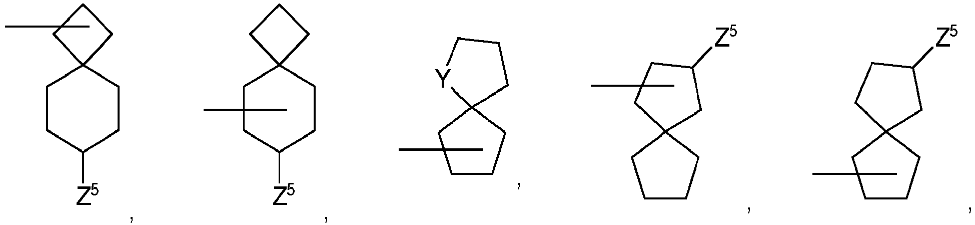 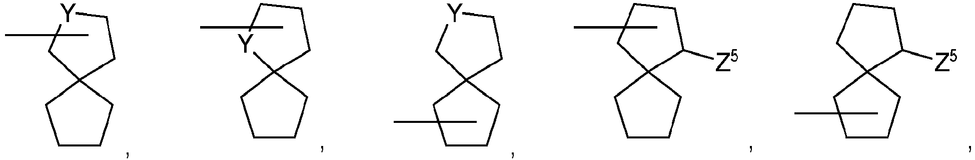 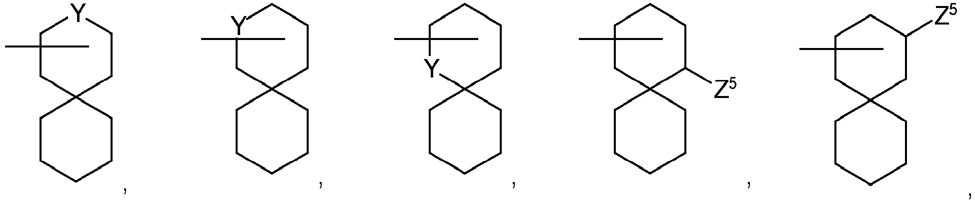 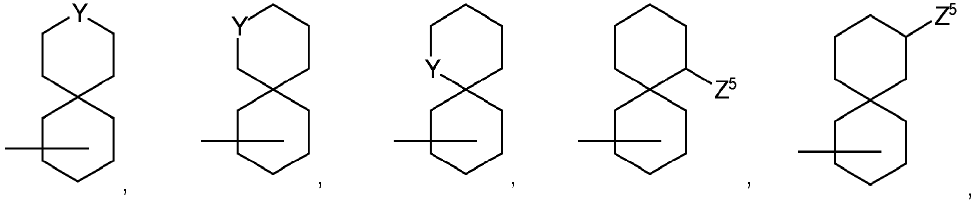 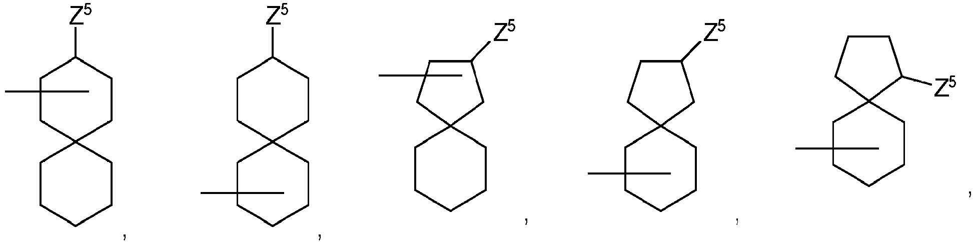 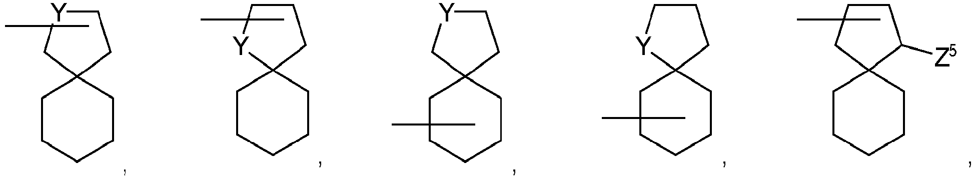 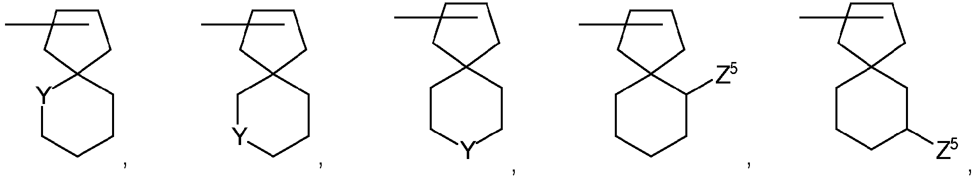 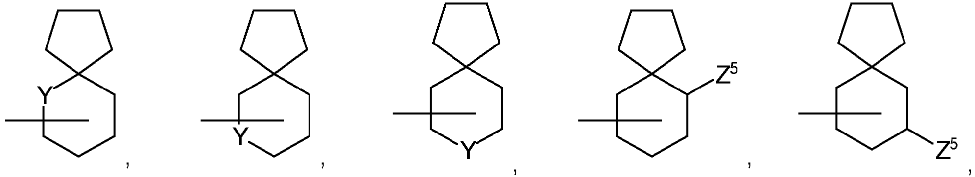 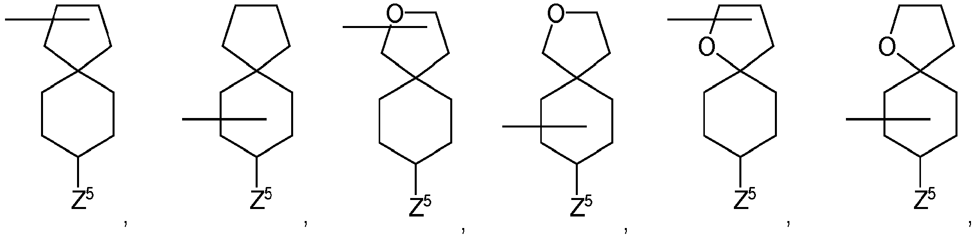 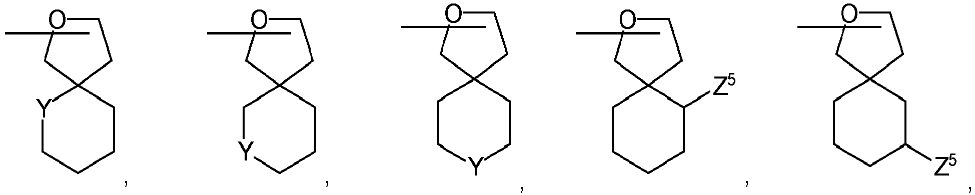 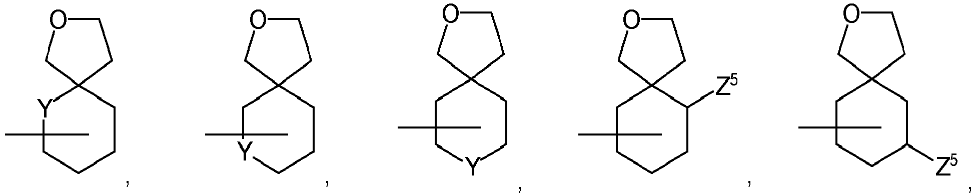 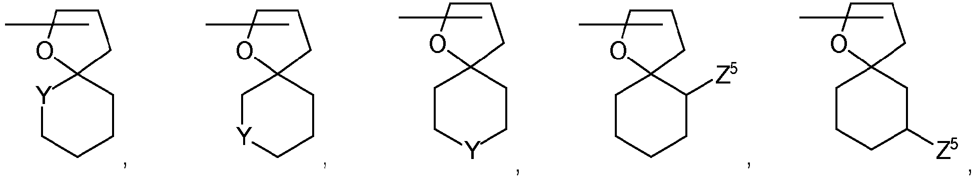 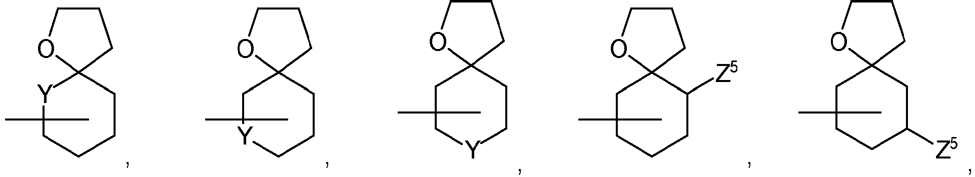 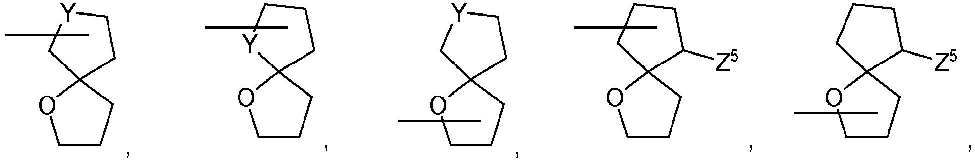 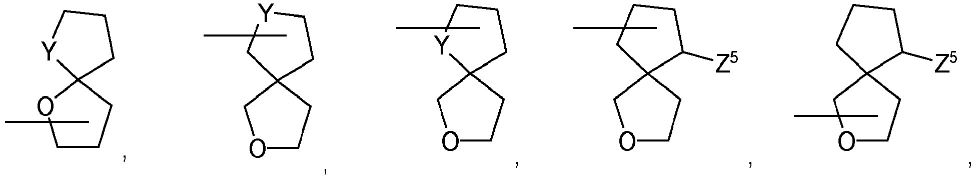 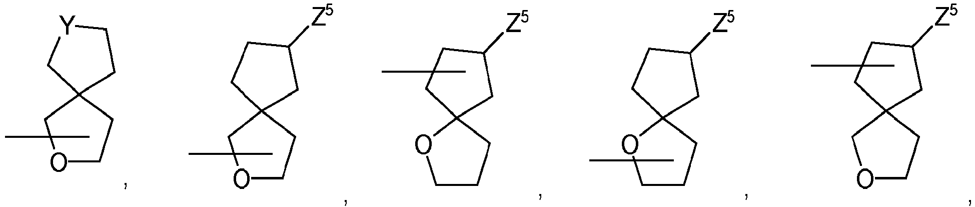 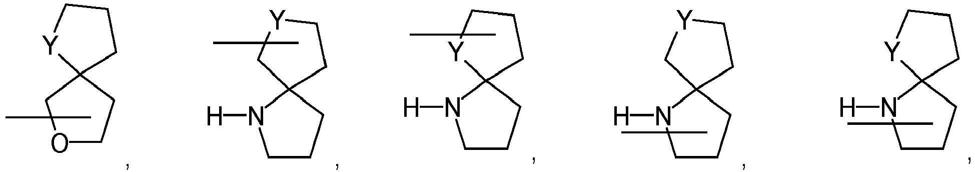 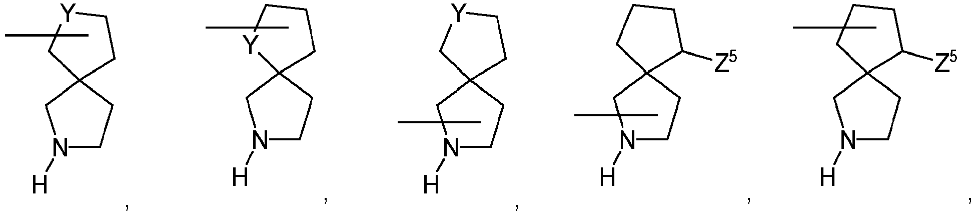 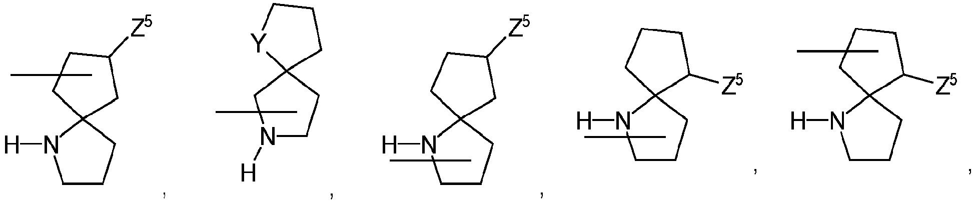 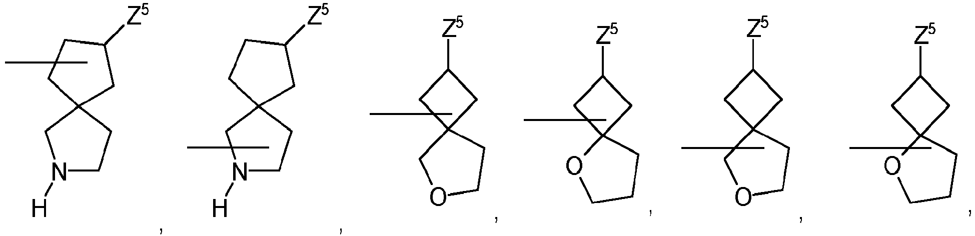 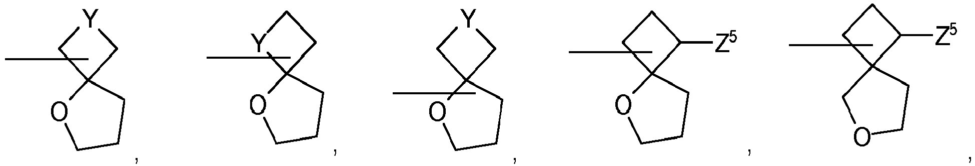 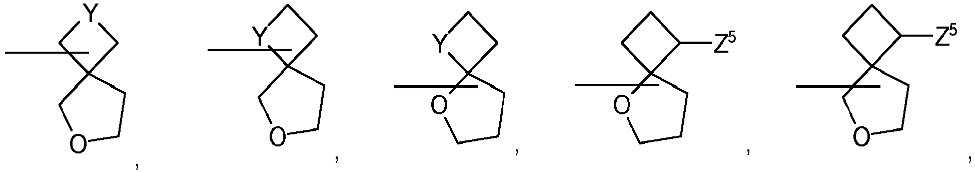 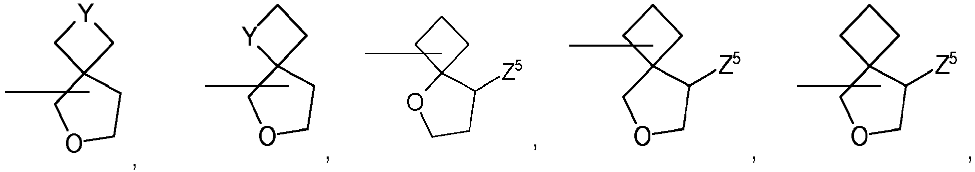 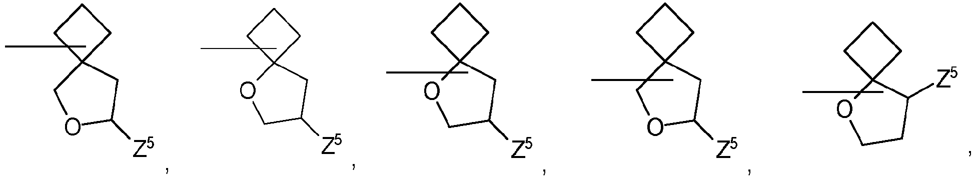 wherein Y represents -O-, -NH-, -NR^{N1}-, -NR^{N2}-, -SO-, or -SO₂-, preferably -NH- and -NR^{N1}- and wherein the substituents Z⁵, Z⁶ and Z⁷ have the meanings as defined herein.

As used herein, the term "**spironitrogencyclyl**" refers to the **C₅-C₁₄/N₁-N₃- spironitrogencyclyl** residues comprising or including the **C₅-C₁₄-spiroheterocyclyl** residues as disclosed above, wherein the heteroatom is nitrogen, i.e. Y is NH, NR^{N1} or NR^{N2}. The term "C₅-C₁₄/N₁-N₃" means that the spiro ring system consists of 5 to 14 carbon atoms and 1 to 3 nitrogen atoms. Moreover the **spironitrogencyclyl** residues can be substituted with 1 to 3 substituents selected from R^{N1}, R^{N2}, Z⁵, Z⁶ and Z⁷. However it is clear to a skilled person that the term "can be substituted" refers to the replacement of a hydrogen atom by one of the substituents R^{N1,} R^{N2}, Z⁵, Z⁶ or Z⁷. It is also possible that two of the substituents Z⁵, Z⁶ and Z⁷ represent together an oxygen atom and form together with the carbon atom of the spironitrogencyclyl residue to which they are both attached a carbonyl moiety. Moreover the **C₅-C₁₄/N₁-N₃-spironitrogencyclyl** residues are characterized in that the spironitrogencyclyl residue is linked through a nitrogen atom of the spiro ring system and not through a carbon atom of the spiro ring system. This means in regard to the above-mentioned C₅-C₁₄-spiroheterocycly residue that the heteroatom Y is nitrogen and that this C₅-C₁₄-spiroheterocycly residue is linked to the rest of the molecule through this nitrogen atom (which is Y). If the **C₅-C₁₄/N₁-N₃- spironitrogencyclyl** residue contains a second nitrogen atom which is substituted by one of the substituents R^{N1}, R^{N2}. Thus the indication "N₂" refers to a first nitrogen atom through which the **spironitrogencyclyl** residue is linked and to the group 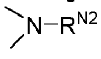 of the spiro ring system. If the spironitrogencyclyl residue contains a third nitrogen atom and both nitrogen atoms are substituted by one of the substituents Z⁵, Z⁶ and Z⁷, the first Z substituent on the second nitrogen atom represents R^{N2} and the second Z substituent on the third nitrogen atom represents R^{N1}. Thus the indication "N₃" refers to a first nitrogen atom through which the **spironitrogencyclyl** residue is linked and to the groups 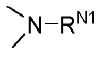 and 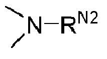 of the spiro ring system. Thus the **C₅-C₁₄/N₁-N₃- spironitrogencyclyl** residue can contain one, two or three nitrogen atoms in the spiro ring system. The numbers of atoms "**C₅-C₁₄/N₁-N₂**" do not include C and N atoms from the substituents Z⁵ to Z⁷.

As used herein, the term **"nitrogenheterocyclyl"** refers to **C₅-C₁₄/N₁-N₃/O₀-O₂/S₀-S₁- nitrogenheterocyclyl** residues comprising or including the **C₅-C₁₄-spiroheterocyclyl** residues as disclosed above, wherein the heteroatom is nitrogen, i.e. Y is NH, NR^{N1} or NR^{N2}. The term "**C₅-C₁₄/N₁-N₃/O₀-O₂/S₀-S₁**" means that the spiro ring system consists of 5 to 14 carbon atoms and 1 to 3 nitrogen atoms, 0 to 2 oxygen atoms and 0 or 1 sulfur atom. Moreover the **nitrogenheterocyclyl** residues can be substituted with 1 to 3 substituents selected from R^{N1}, R^{N2}, Z⁵, Z⁶ and Z⁷. However it is clear to a skilled person that the term "can be substituted" refers to the replacement of a hydrogen atom by one of the substituents Z⁵, Z⁶ or Z⁷. It is also possible that two of the substituents Z⁵, Z⁶ and Z⁷ represent together an oxygen atom and form together with the carbon atom of the **nitrogenheterocyclyl** residue to which they are both attached a carbonyl moiety. Moreover the **C₅-C₁₄/N₁-N₃/O₀-O₂/S₀-S₁-nitrogenheterocyclyl** residues are characterized by that the **nitrogenheterocyclyl** residue is linked through a nitrogen atom of the spiro ring system and not through a carbon atom of the spiro ring system. This means in regard to the above-mentioned C₅-C₁₄-spiroheterocycly residue that the heteroatom Y is nitrogen and that this C₅-C₁₄-spiroheterocycly residue is linked to the rest of the molecule through this nitrogen atom (which is Y). If the C₅-C₁₄/N₁-N₃/O₀- O₂/S₀-S₁-nitrogenheterocyclyl residue contains a second nitrogen atom which is substituted by one of the substituents Z⁵, Z⁶ and Z⁷, said Z substituent represents R^{N2}. Thus, the indication "N₂" refers to a first nitrogen atom through which the nitrogenheterocyclyl residue is linked and to the group 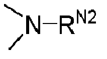 of the spiro ring system. If the **n**itrogenheterocyclyl residue contains a third nitrogen atom and both nitrogen atoms are substituted by one of the substituents Z⁵, Z⁶ and Z⁷, the first Z substituent on the second nitrogen atom represents R^{N2} and the second Z substituent on the third nitrogen atom represents R^{N1}. Thus, the indication "N₃" refers to a first nitrogen atom through which the nitrogenheterocyclyl residue is linked and to the groups 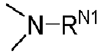 and 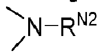 of the spiro ring system. The indication "S₁" refers to the group -S- or -SO- or -SO₂- of the spiro ring system. The indication "S₀" means that no sulfur is present in the nitrogenheterocyclyl residue. The indication "O₁" refers to the group -O- and the indication "O₂" to two groups -O- which are not directly linked to each other, while "O₀" indicates that no oxygen is present in the spiro ring system. Thus, the C₅-C₁₄/N₁- N₃/O₀-O₂/S₀--S₁-nitrogenheterocyclyl residue can contain in total 6 hetero atoms while in total not more than 3 hetero atoms should be present in the spiro ring system. Moreover it is preferred that the heteroatoms in the spiro ring system are not directly bond to each other. The numbers of atoms "**C₅-C₁₄/N₁-N₃/O₀-O₂/S₀-S₁**" do not include C, O, S and N atoms from the substituents R^{N1}, R^{N2}, Z⁵ to Z⁷.

Preferred is the presence of one nitrogen atom or two nitrogen atoms or one nitrogen atom and one sulfur atom or one nitrogen atom and one sulfoxide moiety or one nitrogen atom and one sulphone moiety or one nitrogen atom and one oxygen atom or one nitrogen atom and two oxygen atoms or one oxygen atom and two nitrogen atoms in the spiro ring system.

R⁸ or R⁹ represents preferably the following spironitrogencyclyl, nitrogenheterocyclyl, C₅-C₁₄/N₁-N₃-spironitrogencyclyl or C₅-C₁₄/N₁-N₃/O₀-O₂/S₀-S₁-nitrogenheterocyclyl residues: 4-membered nitrogenheterocyclyl, 5-membered nitrogenheterocyclyl, 6-membered nitrogenheterocyclyl, 5-membered dinitrogenheterocyclyl, 6-membered dinitrogenheterocyclyl, spiro[2,3]heterohexyl, spiro[2,4]heteroheptyl, spiro[2,5]heterooctyl, spiro[2,7]heterononyl, spiro[3,3]heteroheptyl, spiro[3,4]heterooctyl, spiro[3,5]heterononyl, spiro[3,6]heterodecyl, spiro[4,4]heterononyl, spiro[4,5]heterodecyl, spiro[4,6]heteroundecyl, spiro[5,5]heteroundecyl, spiro[5,6]heterododecyl, spiro[6,6]heterotridecyl, wherein the afore-mentioned spironitrogencyclyl, nitrogenheterocyclyl, C₅-C₁₄/N₁-N₃- spironitrogencyclyl or C₅-C₁₄/N₁-N₃/O₀-O₂/S₀-S₁-nitrogenheterocyclyl residues are linked through a ring nitrogen atom to the rest of the molecule and wherein the afore-mentioned spironitrogencyclyl, nitrogenheterocyclyl, C₅-C₁₄/N₁-N₃-spironitrogencyclyl or C₅-C₁₄/N₁-N₃/O₀-O₂/S₀-S₁-nitrogenheterocyclyl residues are optionally substituted with one to three substituents selected from R^{N1}, R^{N2}, Z⁵, Z⁶ and Z⁷.

The term "5-membered dinitrogenheterocyclyl" refers to the residue "5-membered heterocyclyl" as defined above, wherein two heteroatoms are nitrogen atoms and the residue is linked through a nitrogen ring atom to the rest of the molecule and wherein Z¹ is replaced by Z⁵, Z² is replaced by Z⁶, Z³ is replaced by Z⁷, and Z⁴ is hydrogen.

The term "6-membered dinitrogenheterocyclyl" refers to the residue "6-membered heterocyclyl" as defined above, wherein two heteroatoms are nitrogen atoms and the residue is linked through a nitrogen ring atom to the rest of the molecule and wherein Z¹ is replaced by Z⁵, Z² is replaced by Z⁶, Z³ is replaced by Z⁷, and Z⁴ is hydrogen.

Moreover the afore-mentioned spironitrogencyclyl, nitrogenheterocyclyl, C₅-C₁₄/N₁-N₃- spironitrogencyclyl or C₅-C₁₄/N₁-N₃/O₀-O₂/S₀-S₁-nitrogenheterocyclyl residues contain at least one nitrogen atom through which these residues are linked to the rest of the molecule and may contain one or two further moieties selected from oxygen (-O-), sulfoxide (-SO-), sulfone (-SO₂-), carbonyl (-CO-) and nitrogen (-NR^{N1}-).

Preferable substituents Z⁵, Z⁶ and Z⁷ represent independently of each other -H, -CH₃, -C₂H₅, -C₃H₇, -CH(CH₃)₂, -OH, -OCH₃, -OC₂H₅, -OC₃H₇, -NH₂, -NHCH₃, -N(CH₃)₂, -F, -CI, -Br, -l, -CN, -CH₂F, -CHF₂, -CF₃, -OCHF₂, and -OCF₃. **R^{N1},** and **R^{N2}** are preferably selected independently of each other from: -**R¹⁵**, -H, -CH₃, -C₂H₅, -C₃H₇, -CH(CH₃)₂, -CHF₂, -CF₃, 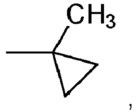 cyclo-C₃H₅, cyclo-C₄H₇, cyclo-C₅H₉, cyclo-C₆H₁₁, cyclo-C₇H₁₃, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -Ph, -CH₂-Ph, -CH₂-CH₂-Ph, -C₂H₄-CH=CH₂, -CH₂-CH=CH-CH₃, -CH₂-C(CH₃)=CH₂, -CH(CH₃)-CH=CH₂, -CH₂-CH=C(CH₃)₂, -CH₂-C≡CH, -C₂H₄-C≡CH, -CH₂-C≡C-CH₃, -COCH₃, -COC₂H₅, -COC₃H₇, -CO-cyclo-C₃H₅, -COCH(CH₃)₂, -COC(CH₃)₃, -COOH, -COOCH₃, -COOC₂H₅, -COOC₃H₇, -COO-cyclo-C₃H₅, -COOCH(CH₃)₂, -COOC(CH₃)₃, -COOCH₂Ph, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, or -SO₃H.

More preferably R⁸ or R⁹ represents the following spironitrogencyclyl, nitrogenheterocyclyl, C₅-C₁₄/N₁-N₃-spironitrogencyclyl or C₅-C₁₄/N₁-N₃/O₀-O₂/S₀-S₁- nitrogenheterocyclyl residues: 4-membered nitrogenheterocyclyl linked through the nitrogen atom to the rest of the molecule, 5-membered nitrogenheterocyclyl linked through the nitrogen atom to the rest of the molecule, 6-membered nitrogenheterocyclyl linked through the nitrogen atom, substituted 4-membered nitrogenheterocyclyl linked through the nitrogen atom, substituted 5-membered nitrogenheterocyclyl linked through the nitrogen atom, substituted 6-membered nitrogenheterocyclyl linked through the nitrogen atom, 5-membered dinitrogenheterocyclyl linked through a nitrogen atom, 6-membered dinitrogenheterocyclyl linked through a nitrogen atom, substituted 5-membered dinitrogenheterocyclyl linked through a nitrogen atom, substituted 6-membered dinitrogenheterocyclyl linked through a nitrogen atom to the rest of the molecule, wherein the afore-mentioned spironitrogencyclyl, nitrogenheterocyclyl, C₅- C₁₄/N₁-N₃-spironitrogencyclyl or C₅-C₁₄/N₁-N₃/O₀-O₂/S₀-S₁-nitrogenheterocyclyl residues are optionally substituted with one to three substituents selected from R^{N1}, R^{N2}, Z⁵, Z⁶ and Z⁷.

Still more preferably R⁸ or R⁹ represents the following spironitrogencyclyl, nitrogenheterocyclyl, C₅-C₁₄/N₁-N₃-spironitrogencyclyl or C₅-C₁₄/N₁-N₃/O₀-O₂/S₀-S₁- nitrogenheterocyclyl residues:
azaspiro[3,3]heptyl linked through the nitrogen atom, azaspiro[3,4]octyl linked through the nitrogen atom, azaspiro[3,5]nonyl linked through the nitrogen atom, azaspiro[3,6]decyl linked through the nitrogen atom, azaspiro[4,4]nonyl linked through the nitrogen atom, azaspiro[4,5]decyl linked through the nitrogen atom, azaspiro[4,6]undecyl linked through the nitrogen atom, azaspiro[5,5]undecyl linked through the nitrogen atom, azaspiro[5,6]dodecyl linked through the nitrogen atom, azaspiro[6,6]tridecyl linked through the nitrogen atom,
substituted azaspiro[3,3]heptyl linked through the nitrogen atom, substituted azaspiro[3,4]octyl linked through the nitrogen atom, substituted azaspiro[3,5]nonyl linked through the nitrogen atom, substituted azaspiro[3,6]decyl linked through the nitrogen atom, substituted azaspiro[4,4]nonyl linked through the nitrogen atom, substituted azaspiro[4,5]decyl linked through the nitrogen atom, substituted azaspiro[4,6]undecyl linked through the nitrogen atom, substituted azaspiro[5,5]undecyl linked through the nitrogen atom, substituted azaspiro[5,6]dodecyl linked through the nitrogen atom, substituted azaspiro[6,6]tridecyl linked through the nitrogen atom,
diazaspiro[3,3]heptyl linked through a nitrogen atom, diazaspiro[3,4]octyl linked through a nitrogen atom, diazaspiro[3,5]nonyl linked through a nitrogen atom, diazaspiro[3,6]decyl linked through a nitrogen atom, diazaspiro[4,4]nonyl linked through a nitrogen atom, diazaspiro[4,5]decyl linked through a nitrogen atom, diazaspiro[4,6]undecyl linked through a nitrogen atom, diazaspiro[5,5]undecyl linked through a nitrogen atom, diazaspiro[5,6]dodecyl linked through a nitrogen atom, diazaspiro[6,6]tridecyl linked through a nitrogen atom,
substituted diazaspiro[3,3]heptyl linked through a nitrogen atom, substituted diazaspiro[3,4]octyl linked through a nitrogen atom, substituted diazaspiro[3,5]nonyl linked through a nitrogen atom, substituted diazaspiro[3,6]decyl linked through a nitrogen atom, substituted diazaspiro[4,4]nonyl linked through a nitrogen atom, substituted diazaspiro[4,5]decyl linked through a nitrogen atom, substituted diazaspiro[4,6]undecyl linked through a nitrogen atom, substituted diazaspiro[5,5]undecyl linked through a nitrogen atom, substituted diazaspiro[5,6]dodecyl linked through a nitrogen atom, substituted diazaspiro[6,6]tridecyl linked through a nitrogen atom,
triazaspiro[3,5]nonyl linked through a nitrogen atom, triazaspiro[3,6]decyl linked through a nitrogen atom, triazaspiro[4,5]decyl linked through a nitrogen atom, triazaspiro[4,6]undecyl linked through a nitrogen atom, triazaspiro[5,5]undecyl linked through a nitrogen atom, triazaspiro[5,6]dodecyl linked through a nitrogen atom, triazaspiro[6,6]tridecyl linked through a nitrogen atom,
substituted triazaspiro[3,5]nonyl linked through a nitrogen atom, substituted triazaspiro[3,6]decyl linked through a nitrogen atom, substituted triazaspiro[4,5]decyl linked through a nitrogen atom, substituted triazaspiro[4,6]undecyl linked through a nitrogen atom, substituted triazaspiro[5,5]undecyl linked through a nitrogen atom, substituted triazaspiro[5,6]dodecyl linked through a nitrogen atom, substituted triazaspiro[6,6]tridecyl linked through a nitrogen atom,
oxazaspiro[3,3]heptyl linked through a nitrogen atom, oxazaspiro[3,4]octyl linked through a nitrogen atom, oxazaspiro[3,5]nonyl linked through a nitrogen atom, oxazaspiro[3,6]decyl linked through a nitrogen atom, oxazaspiro[4,4]nonyl linked through a nitrogen atom, oxazaspiro[4,5]decyl linked through a nitrogen atom, oxazaspiro[4,6]undecyl linked through a nitrogen atom, oxazaspiro[5,5]undecyl linked through a nitrogen atom, oxazaspiro[5,6]dodecyl linked through a nitrogen atom, oxazaspiro[6,6]tridecyl linked through a nitrogen atom,
substituted oxazaspiro[3,3]heptyl linked through a nitrogen atom, substituted oxazaspiro[3,4]octyl linked through a nitrogen atom, substituted oxazaspiro[3,5]nonyl linked through a nitrogen atom, substituted oxazaspiro[3,6]decyl linked through a nitrogen atom, substituted oxazaspiro[4,4]nonyl linked through a nitrogen atom, substituted oxazaspiro[4,5]decyl linked through a nitrogen atom, substituted oxazaspiro[4,6]undecyl linked through a nitrogen atom, substituted oxazaspiro[5,5]undecyl linked through a nitrogen atom, substituted oxazaspiro[5,6]dodecyl linked through a nitrogen atom, substituted oxazaspiro[6,6]tridecyl linked through a nitrogen atom,
oxadiazaspiro[3,5]nonyl linked through a nitrogen atom, oxadiazaspiro[3,6]decyl linked through a nitrogen atom, oxadiazaspiro[4,5]decyl linked through a nitrogen atom, oxadiazaspiro[4,6]undecyl linked through a nitrogen atom, oxadiazaspiro[5,5]undecyl linked through a nitrogen atom, oxadiazaspiro[5,6]dodecyl linked through a nitrogen atom, oxadiazaspiro[6,6]tridecyl linked through a nitrogen atom,
substituted oxadiazaspiro[3,5]nonyl linked through a nitrogen atom, substituted oxadiazaspiro[3,6]decyl linked through a nitrogen atom, substituted oxadiazaspiro[4,5]decyl linked through a nitrogen atom, substituted oxadiazaspiro[4,6]undecyl linked through a nitrogen atom, substituted oxadiazaspiro[5,5]undecyl linked through a nitrogen atom, substituted oxadiazaspiro[5,6]dodecyl linked through a nitrogen atom, substituted oxadiazaspiro[6,6]tridecyl linked through a nitrogen atom, wherein the afore-mentioned substituted spironitrogencyclyl, substituted nitrogenheterocyclyl, substituted C₅-C₁₄/N₁- N₃-spironitrogencyclyl or substituted C₅-C₁₄/N₁-N₃/O₀-O₂/S₀-S₁-nitrogenheterocyclyl residues are optionally substituted with one to three substituents selected from R^{N1}, R^{N2}, Z⁵, Z⁶ and Z⁷. Moreover the afore-mentioned substituted or non-substituted spironitrogencyclyl, substituted or non-substituted nitrogenheterocyclyl, substituted or non-substituted C₅-C₁₄/N₁-N₃-spironitrogencyclyl or substituted or non-substituted C₅- C₁₄/N₁-N₃/O₀-O₂/S₀-S₁-nitrogenheterocyclyl residues contain at least one nitrogen atom through which these residues are linked through the rest of the molecule and may contain one or two further moieties selected from oxygen (-O-), sulfoxide (-SO-), sulfone (-SO₂-), carbonyl (-CO-) and nitrogen (-NR^{N2}-).

Still more preferably R⁸ or R⁹ represents the following spironitrogencyclyl, nitrogenheterocyclyl, C₅-C₁₄/N₁-N₃-spironitrogencyclyl or C₅-C₁₄/N₁-N₃/O₀-O₂/S₀-S₁- nitrogenheterocyclyl residues: 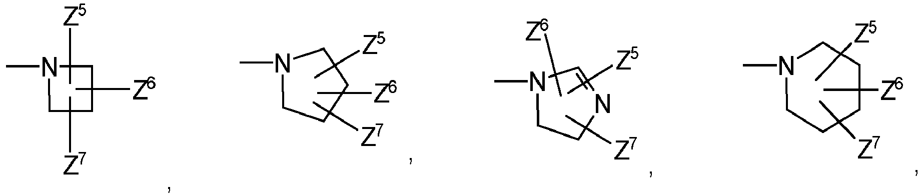 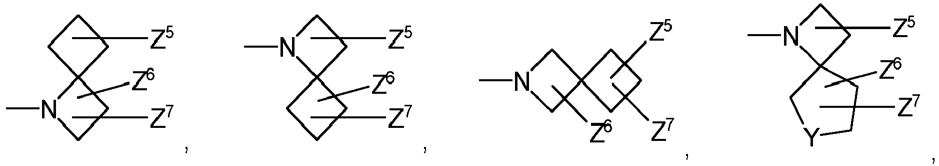 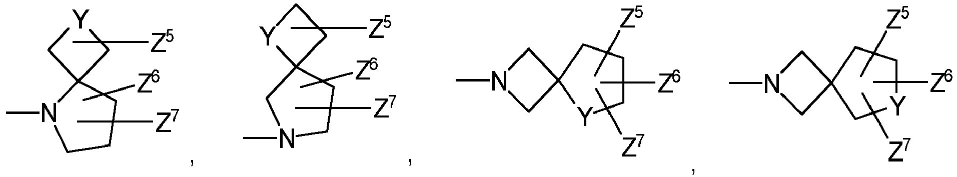 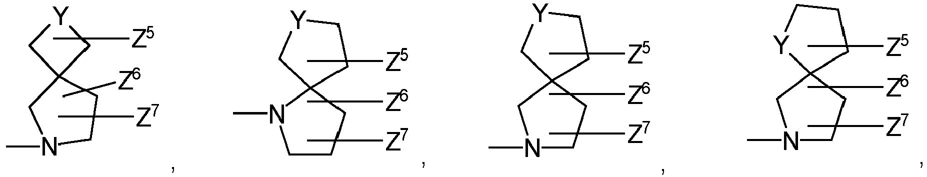 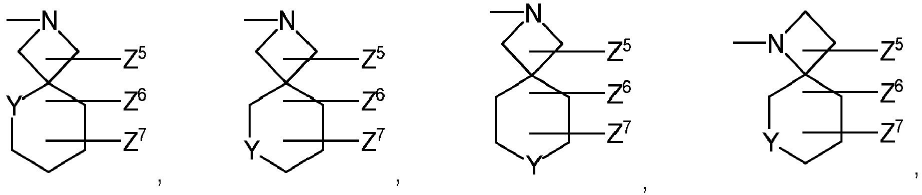 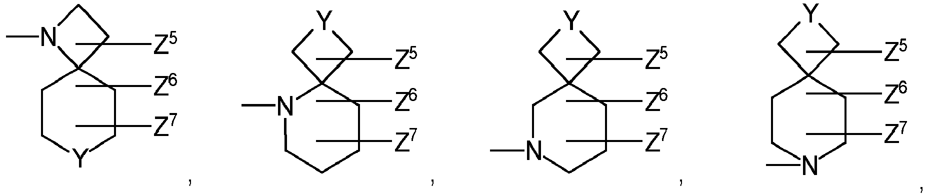 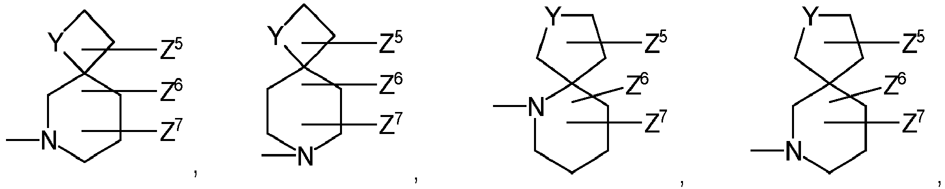 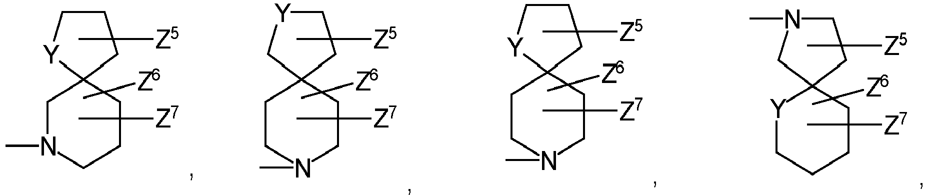 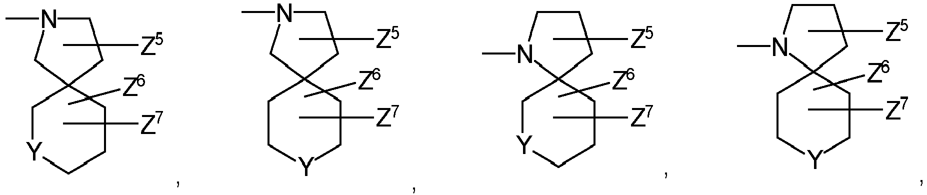 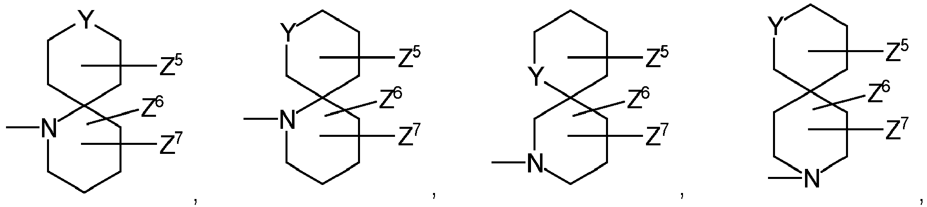 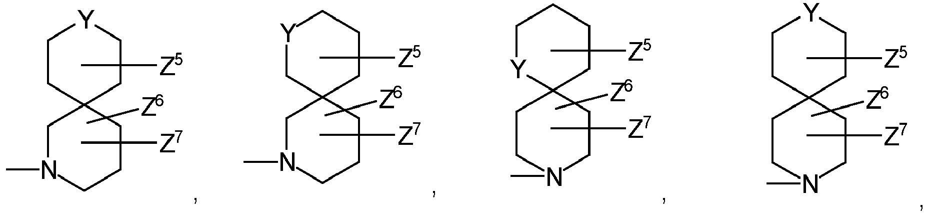 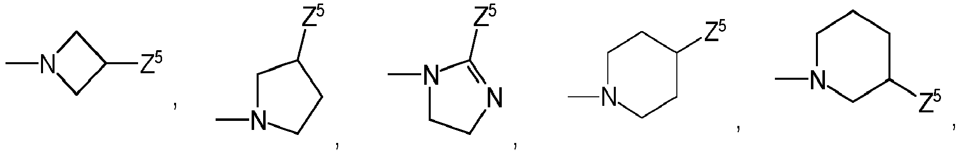 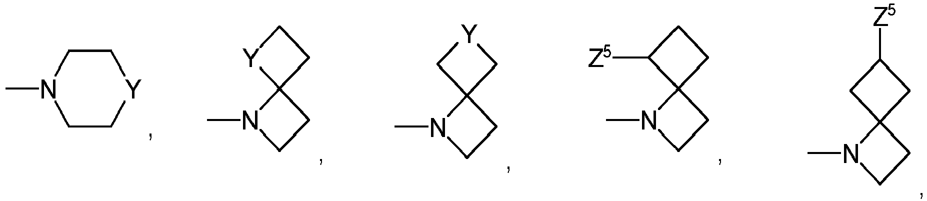 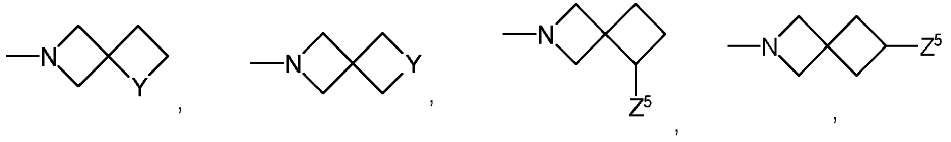 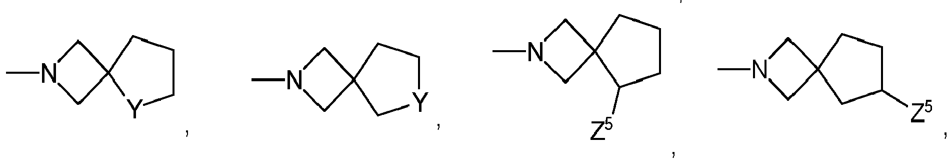 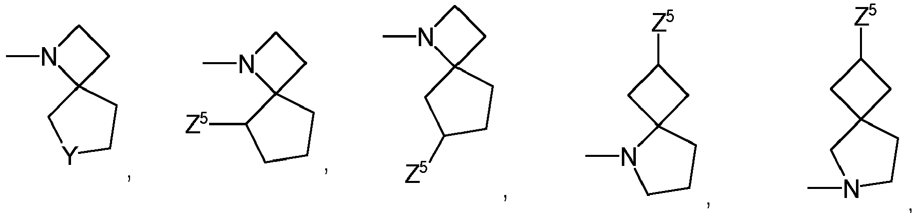 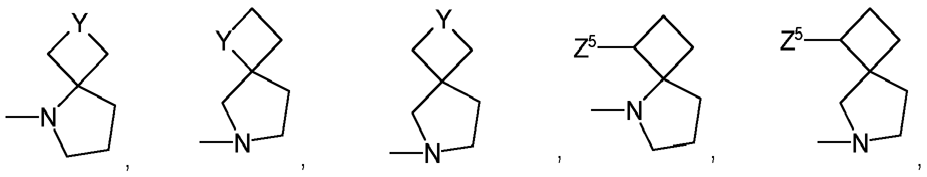 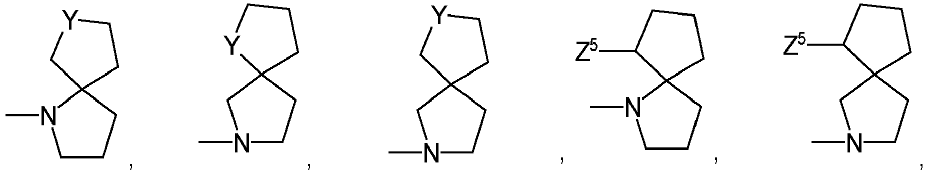 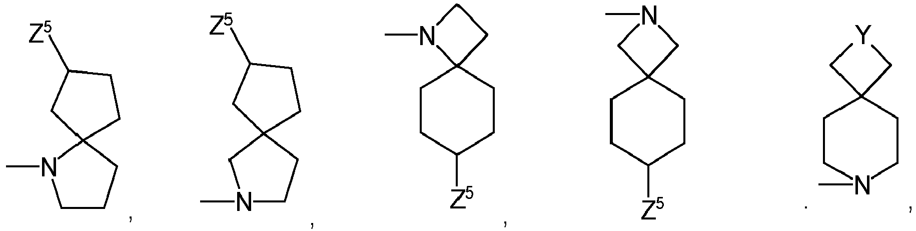 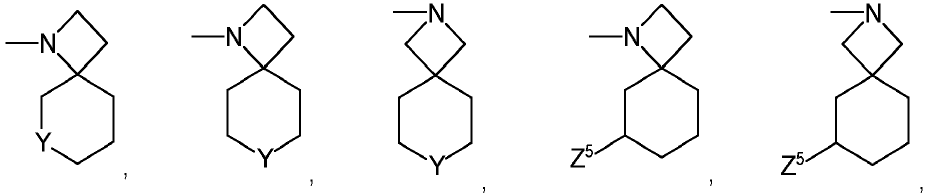 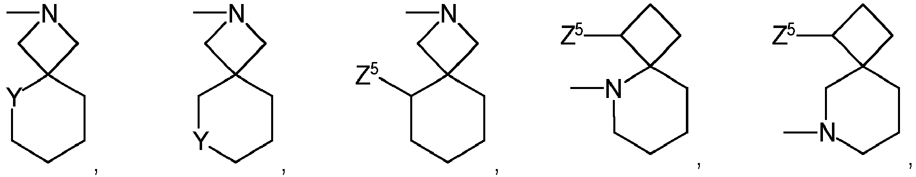 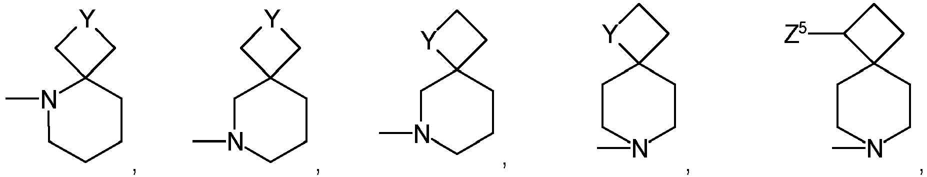 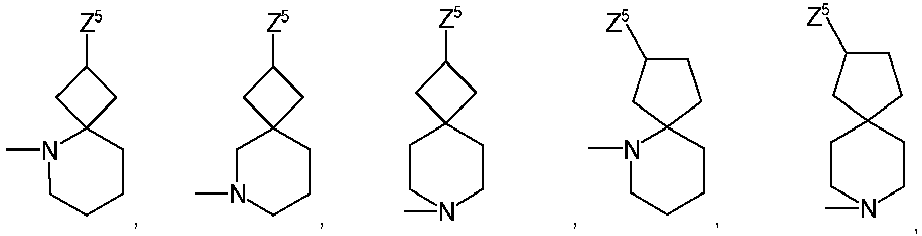 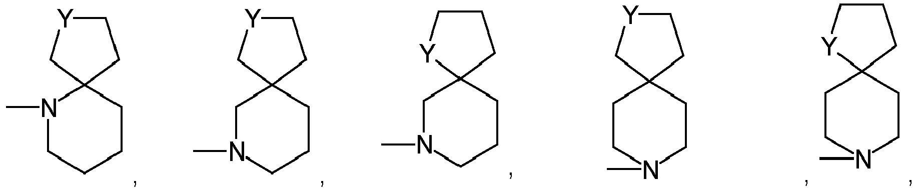 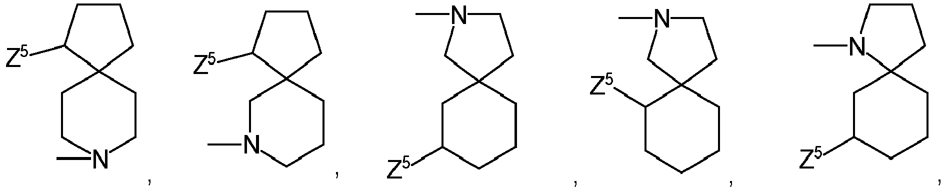 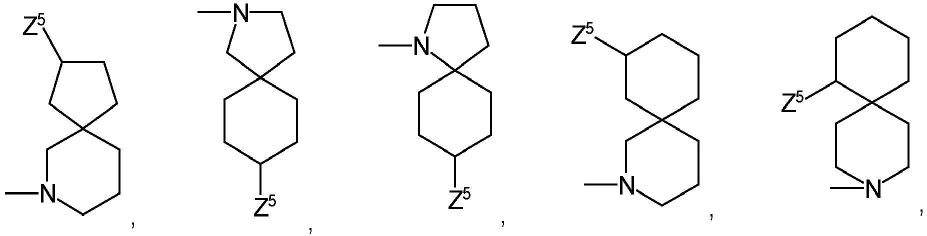 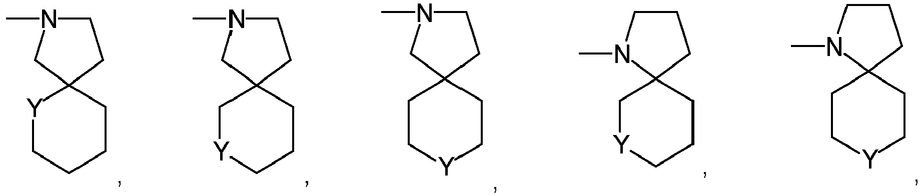 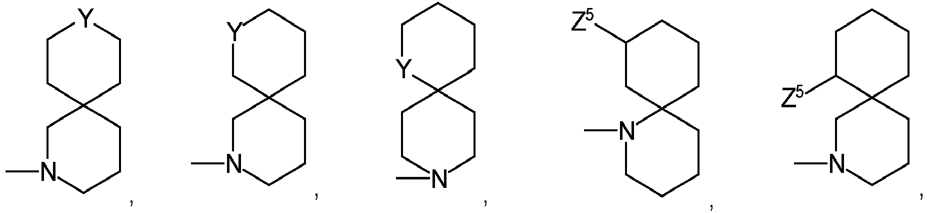 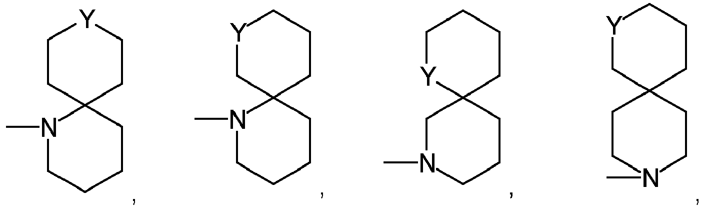 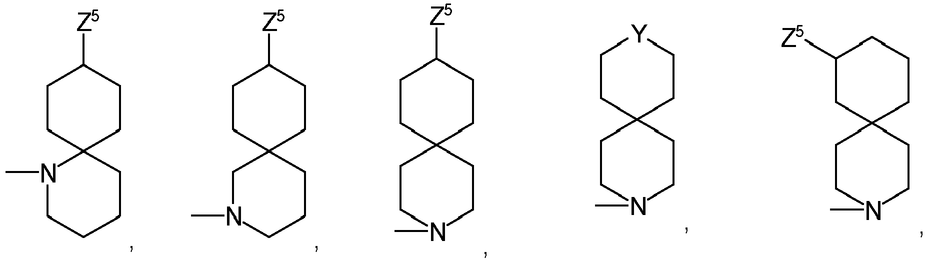 wherein Y represents -O-, -NH-, -NR^{N1}-, -NR^{N2}-, -SO-, or -SO₂-, preferably -NH- and -NR^{N1}- and wherein the substituents Z⁵, Z⁶ and Z⁷ have the meanings as defined herein.

Preferably Z⁵, Z⁶ and Z⁷ represent independently of each other -H, -CH₃, -C₂H₅, -C₃H₇, -CH(CH₃)₂, -OH, -OCH₃, -OC₂H₅, -OC₃H₇, -NH₂, -NH(CH₃), -N(CH₃)₂, -F, -Cl, -Br, -l, -CN, -CH₂F, -CHF₂, -CF₃, -OCHF₂, and -OCF₃, more preferably -NH₂, -NH(CH₃), -N(CH₃)₂.

Preferred, is the compound of formula (**I**), wherein **R⁸** and **R⁹** represent independently of each other:

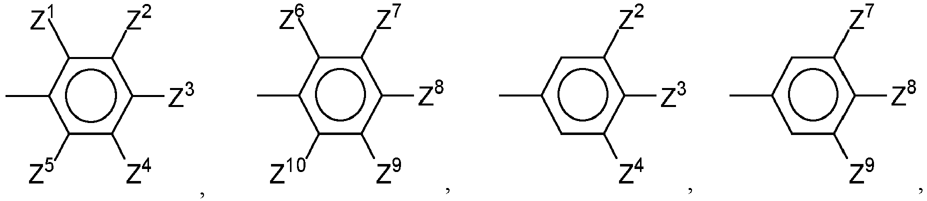

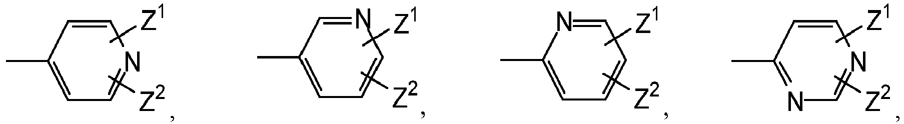

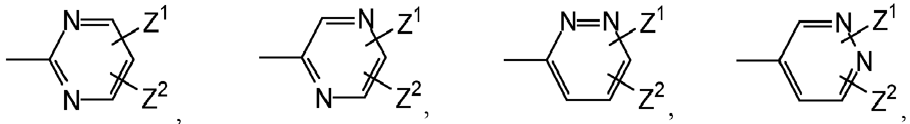

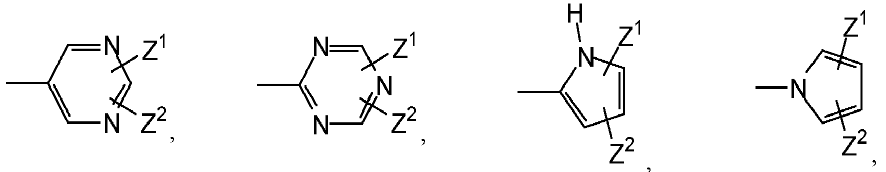

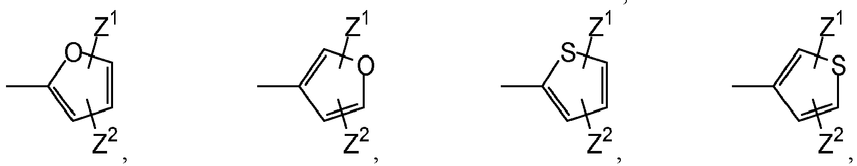

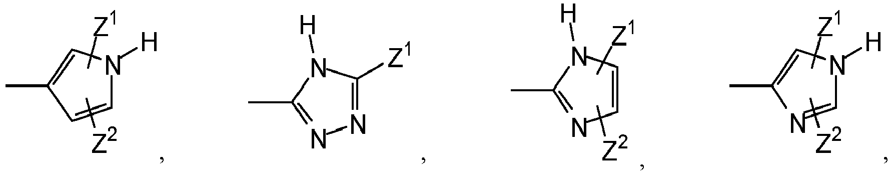

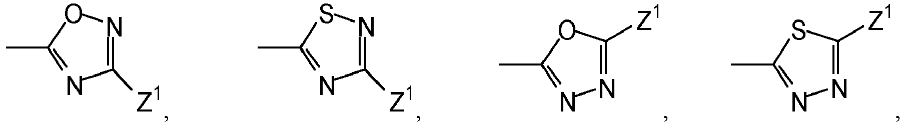

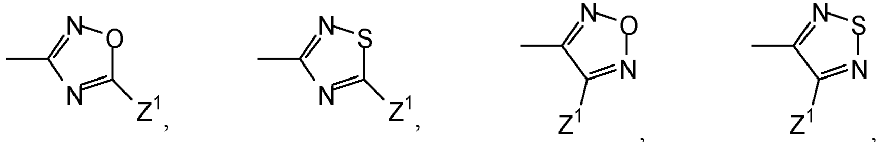

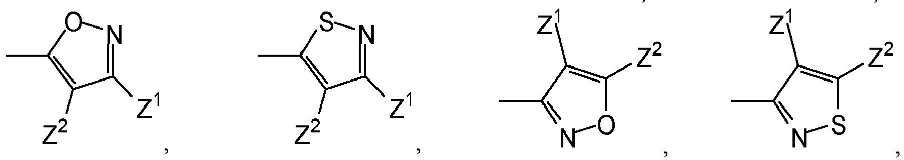

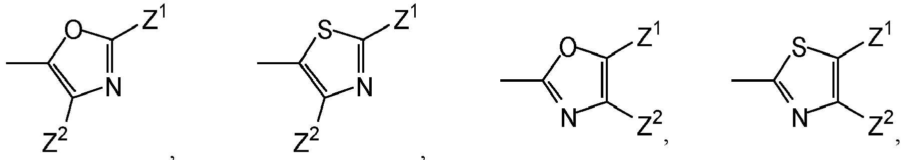

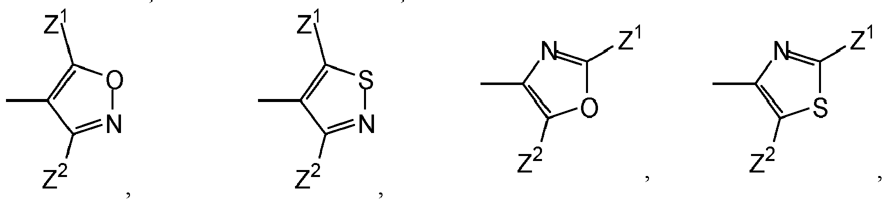

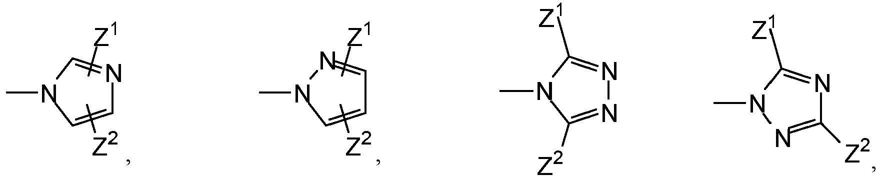

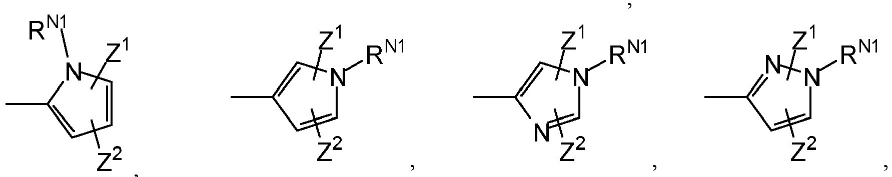

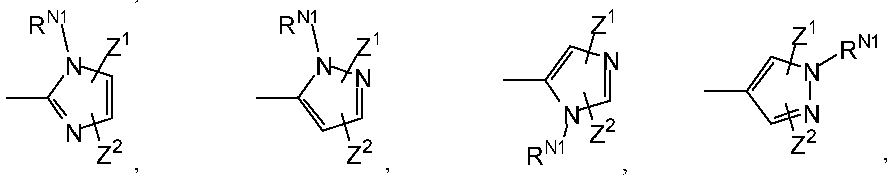

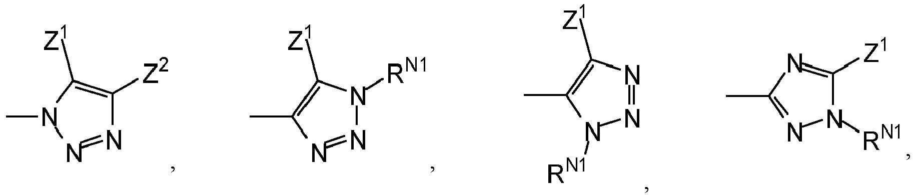

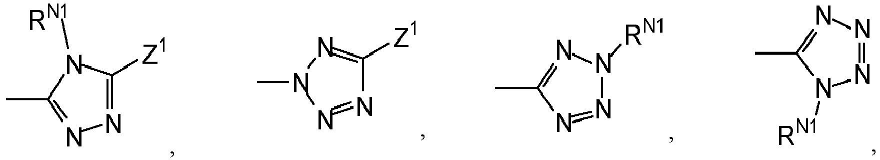

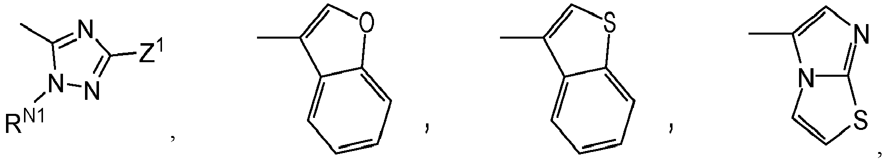

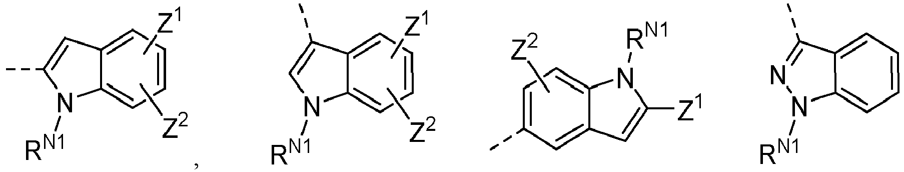

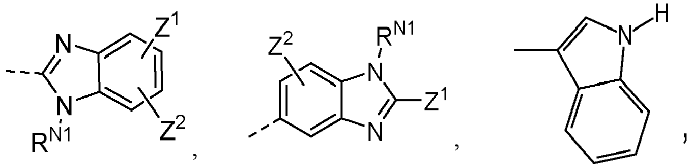

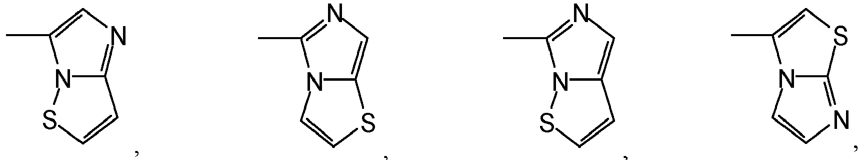

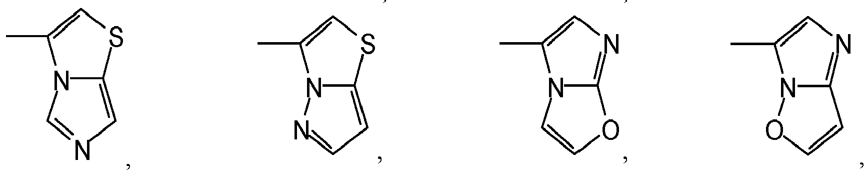

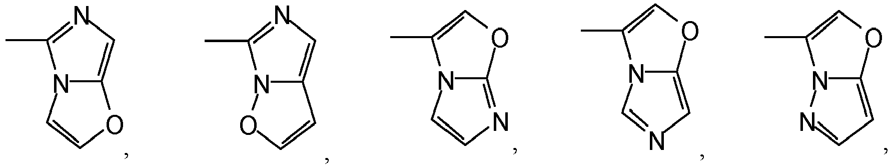

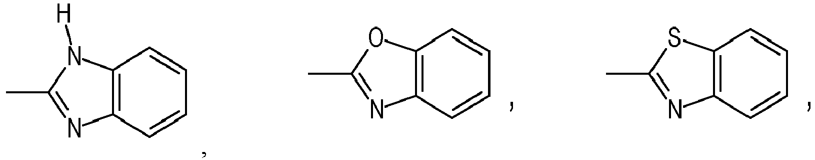

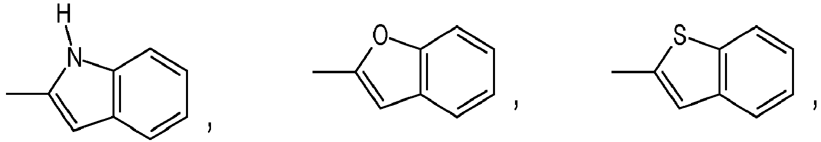

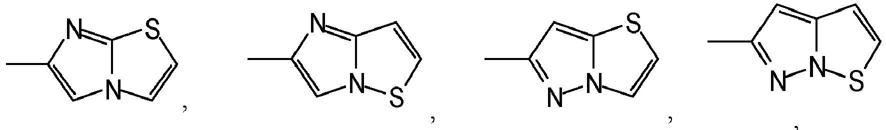

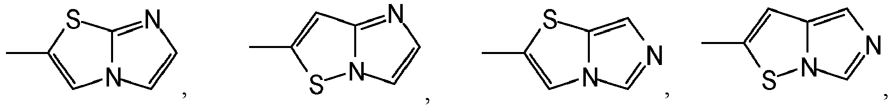

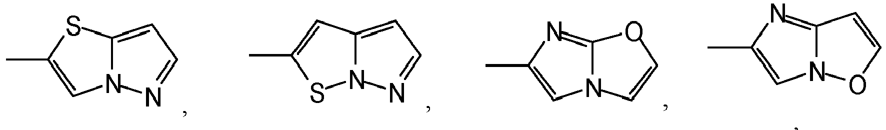

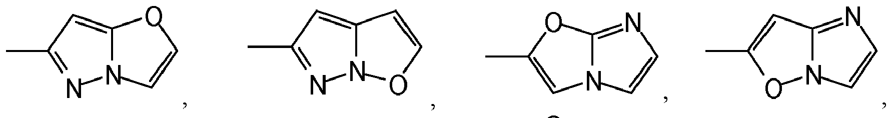

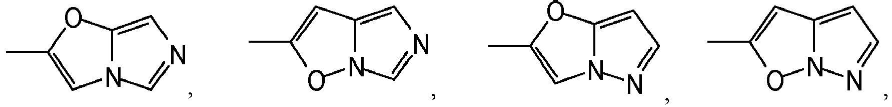

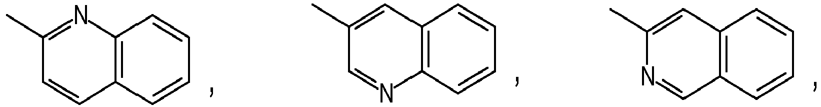

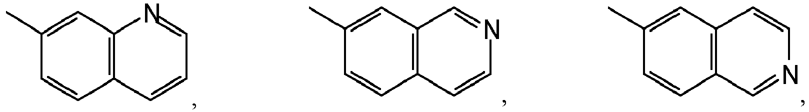

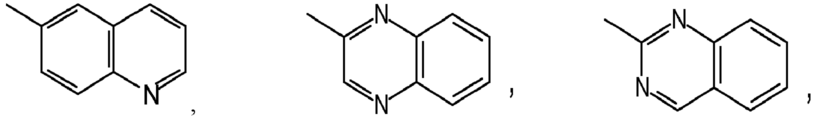

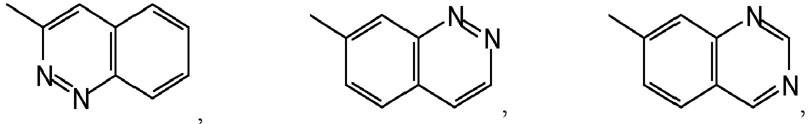

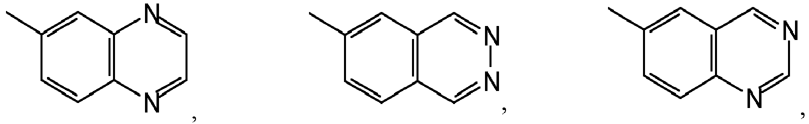

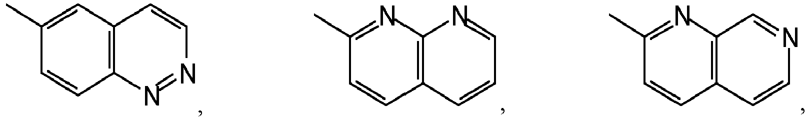

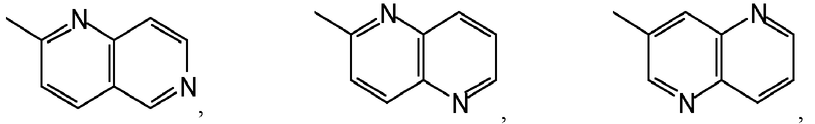

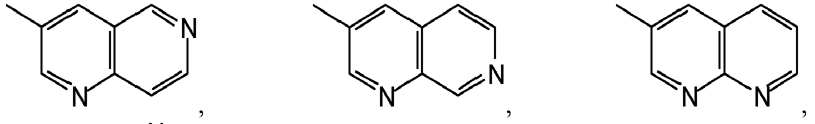

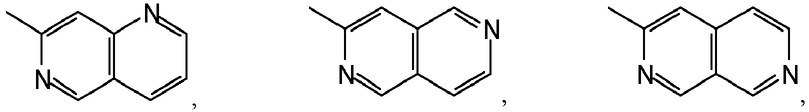

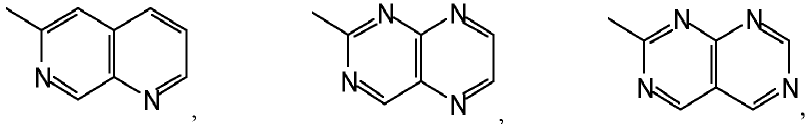

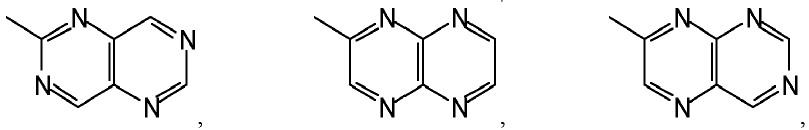

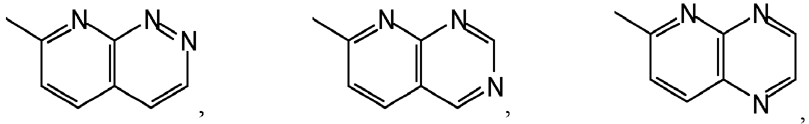

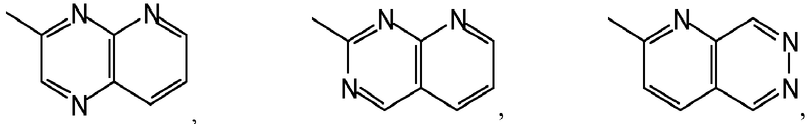

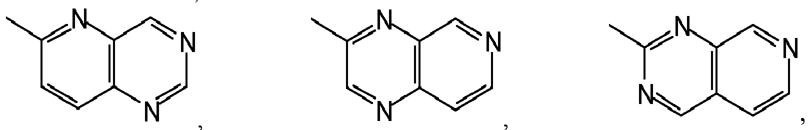

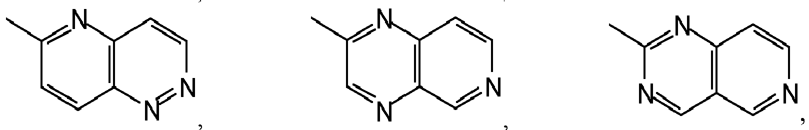

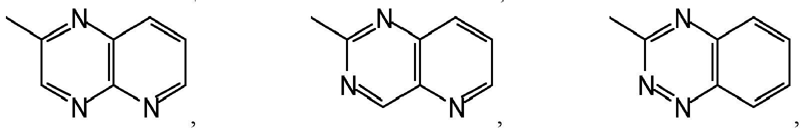

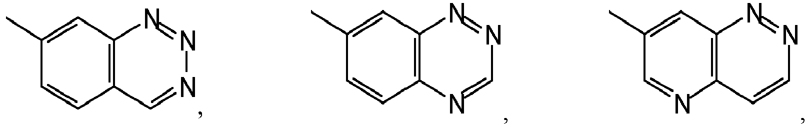

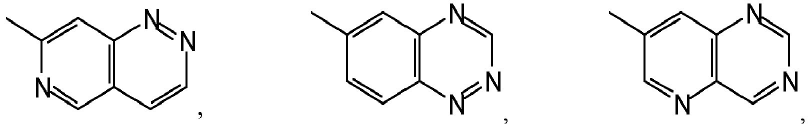

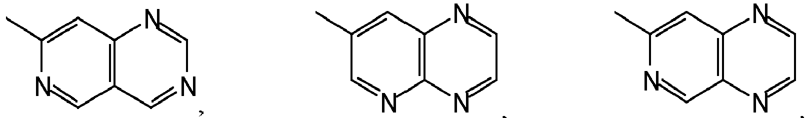

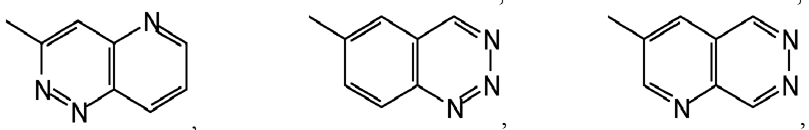

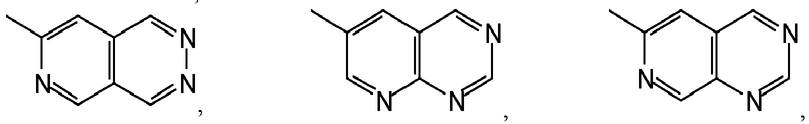

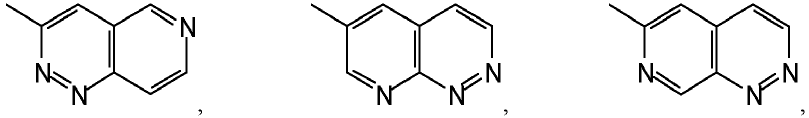

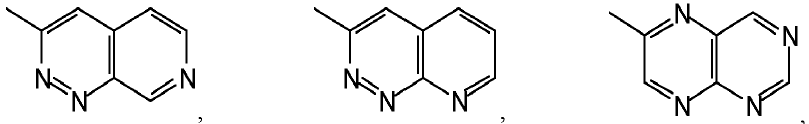

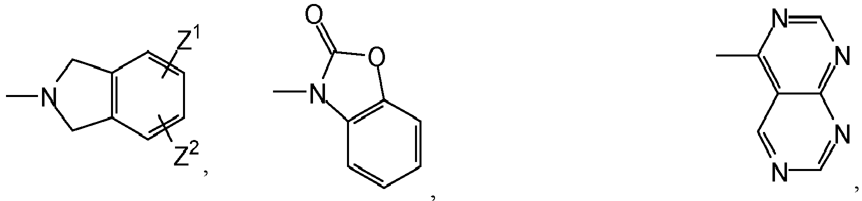

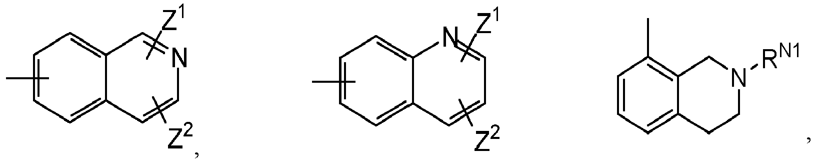

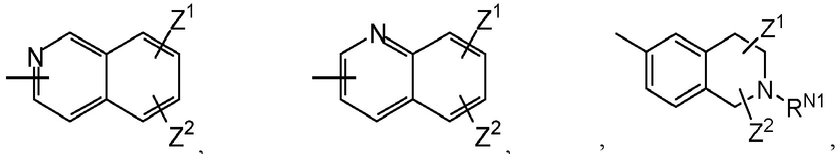

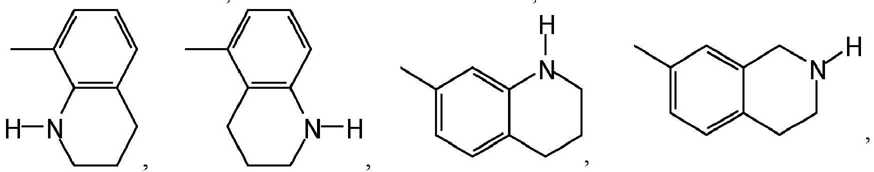

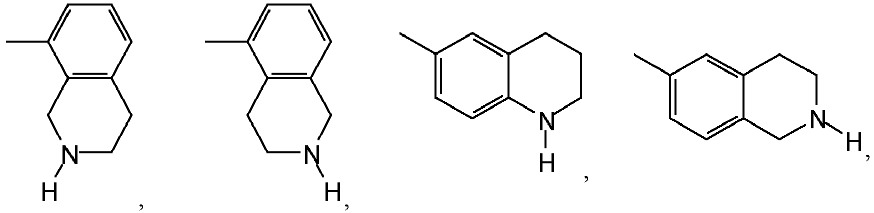

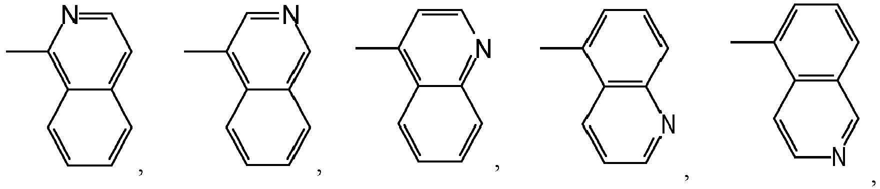

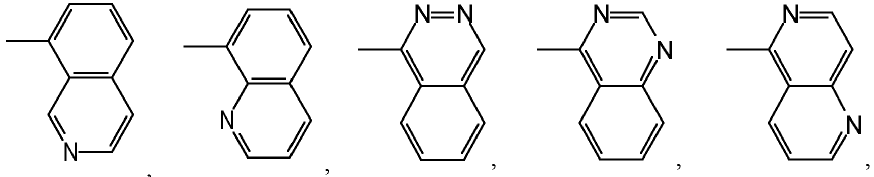

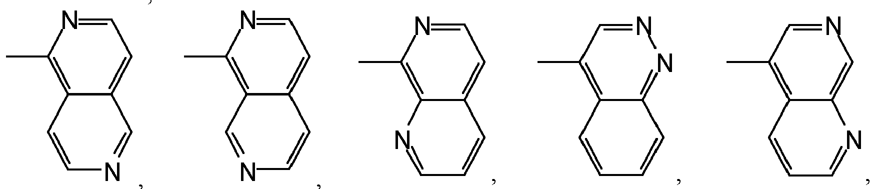

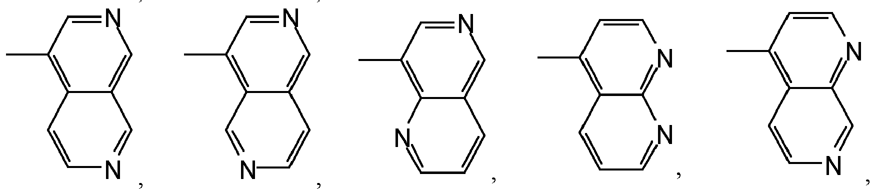

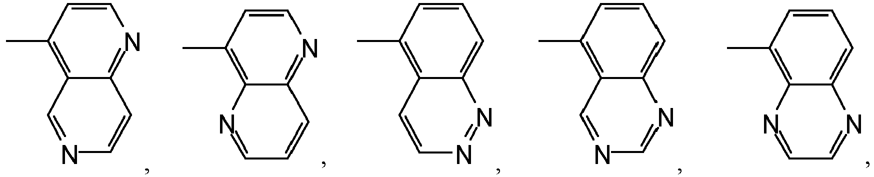

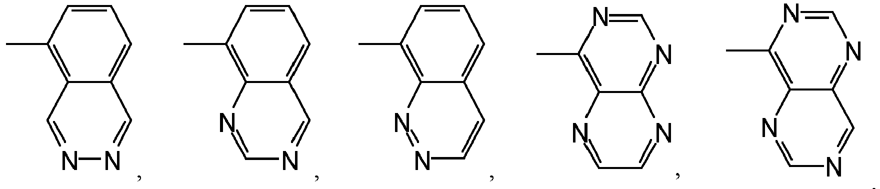

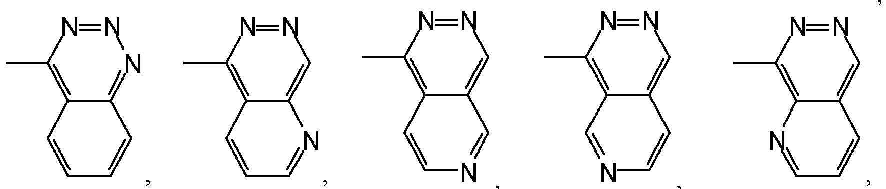

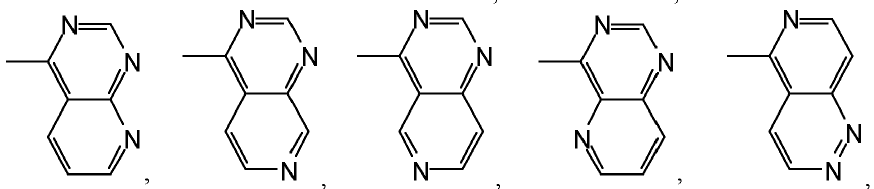

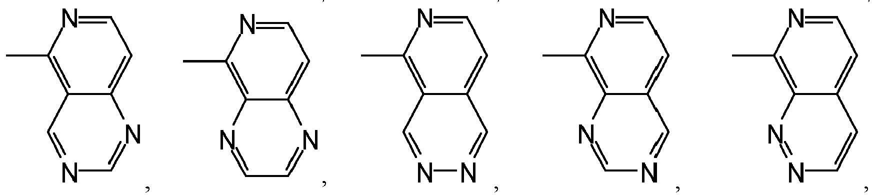

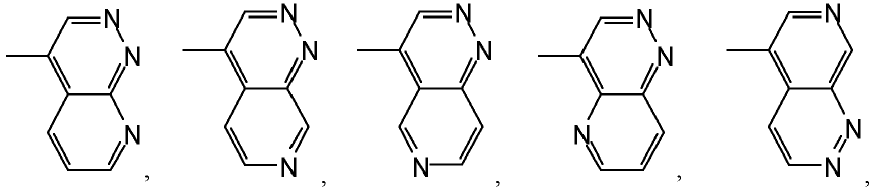

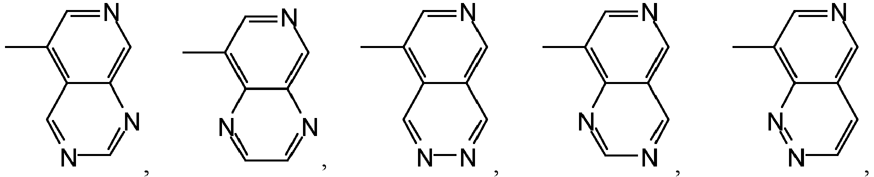

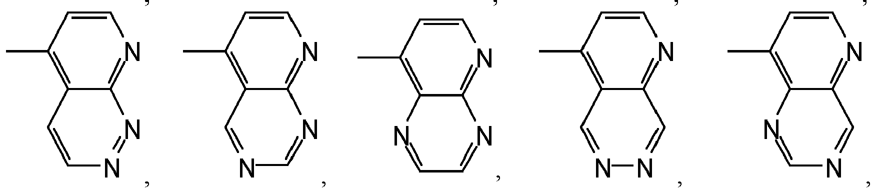

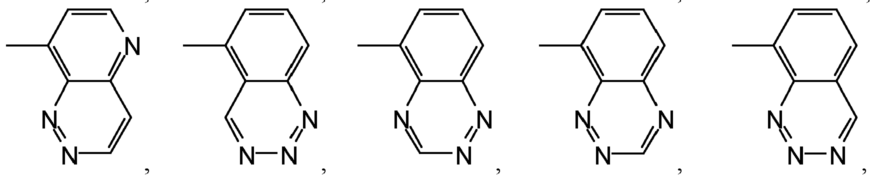

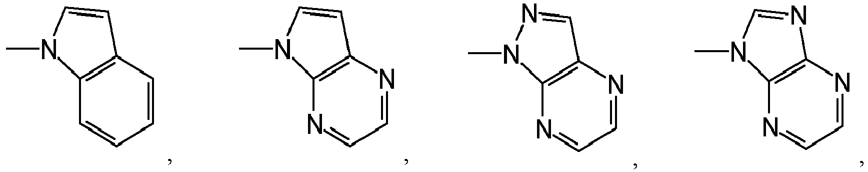

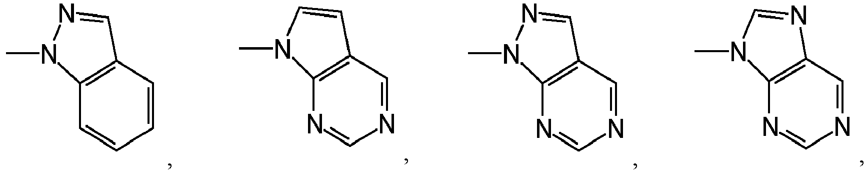

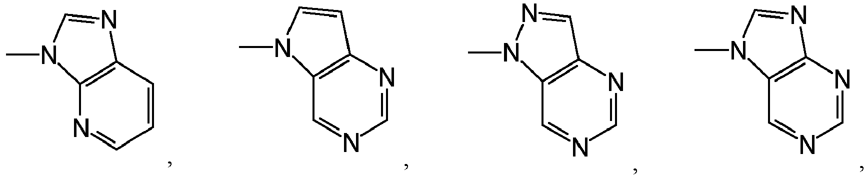

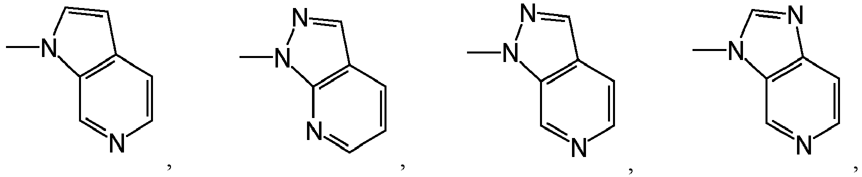

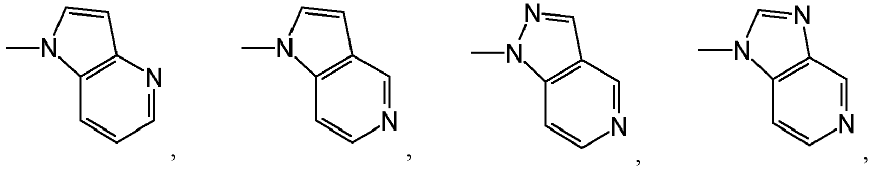

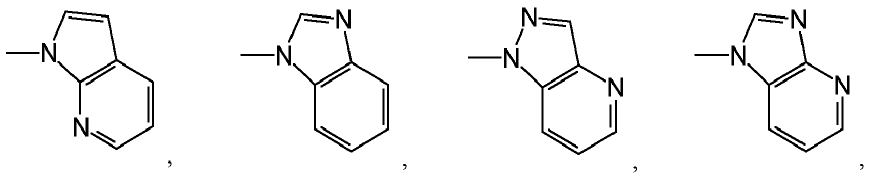

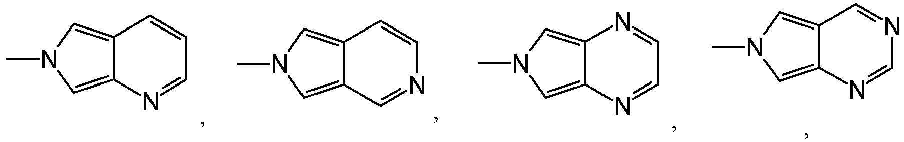

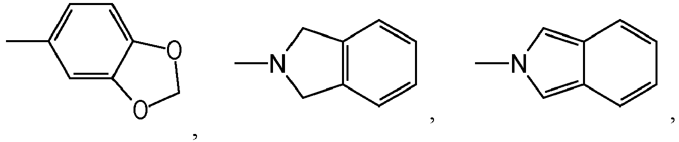

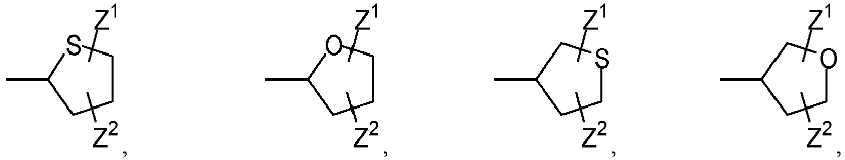

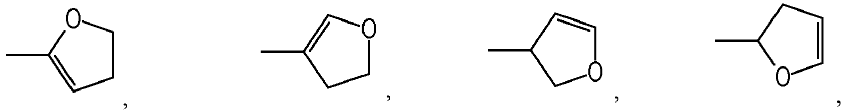

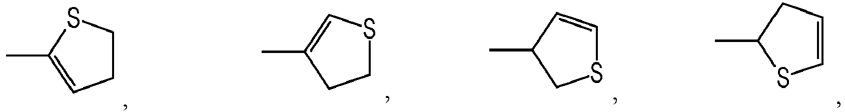

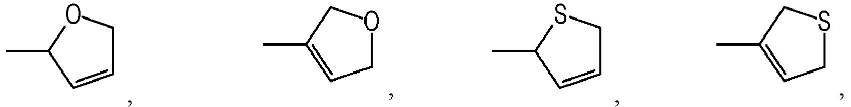

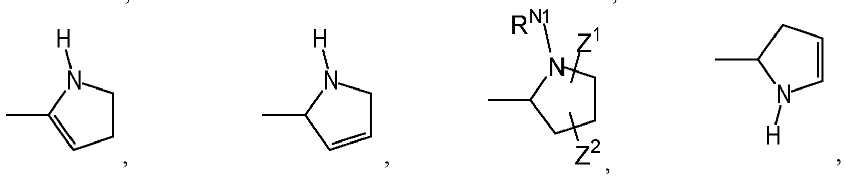

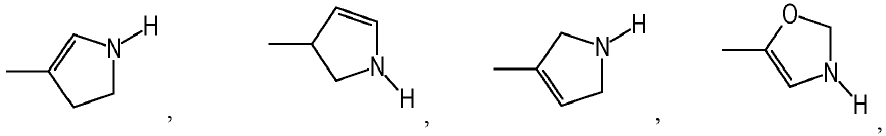

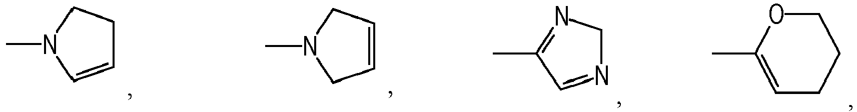

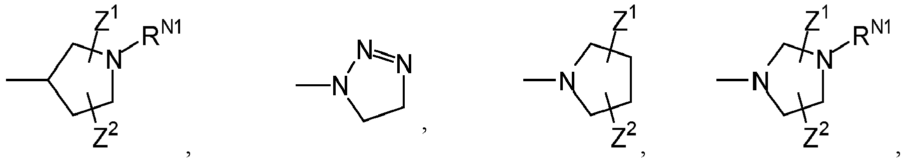

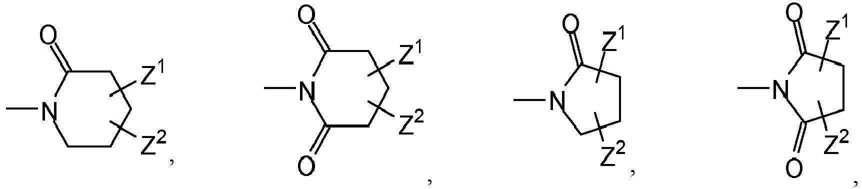

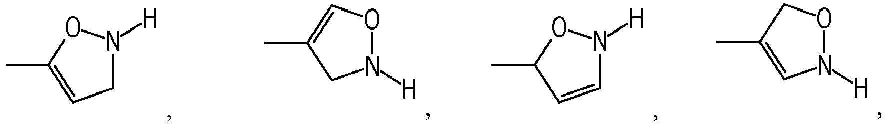

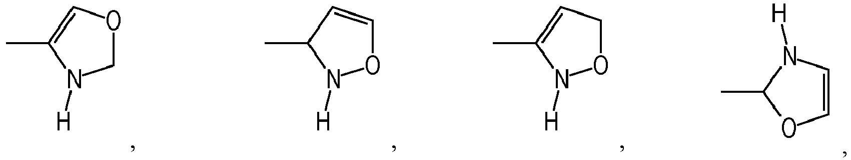

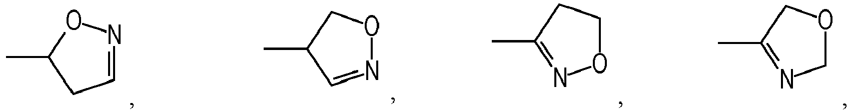

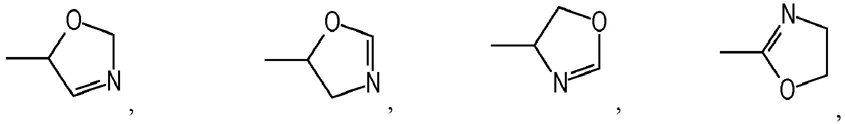

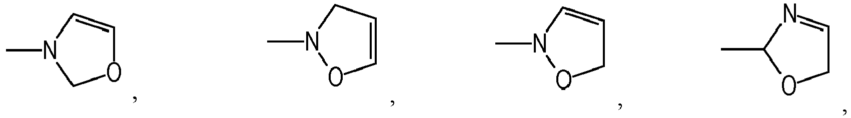

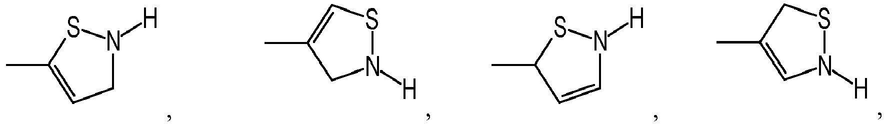

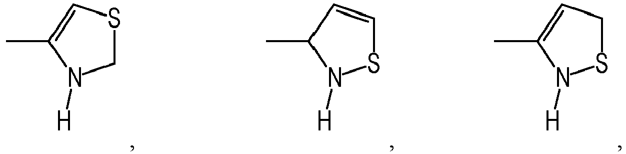

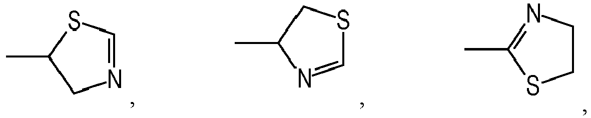

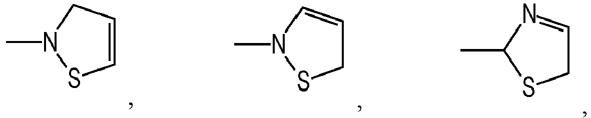

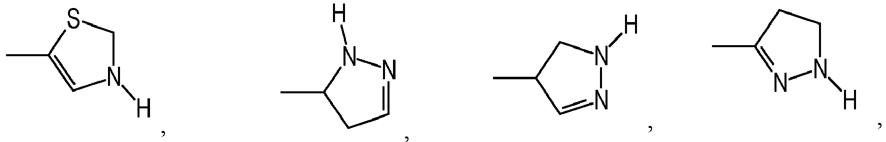

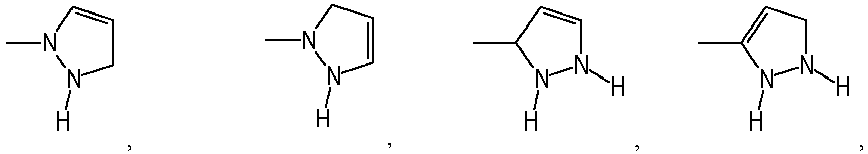

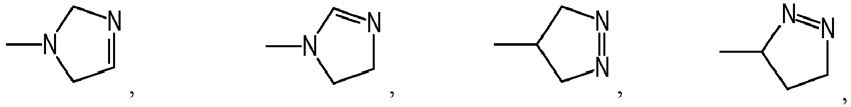

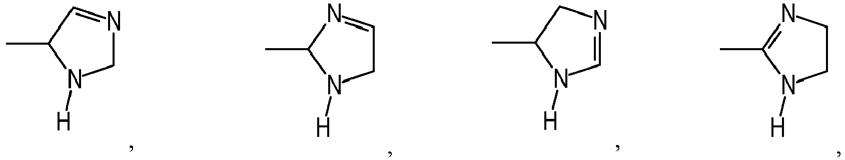

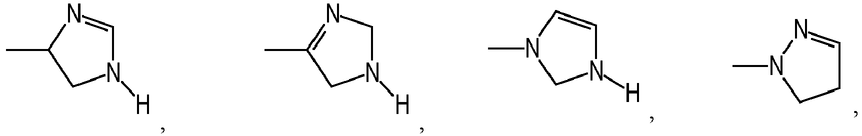

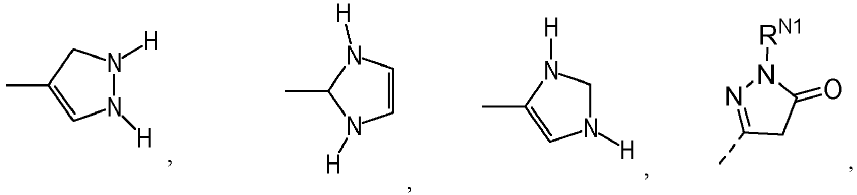

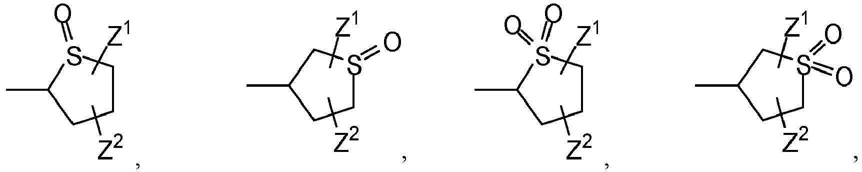

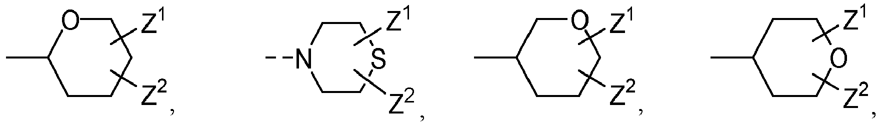

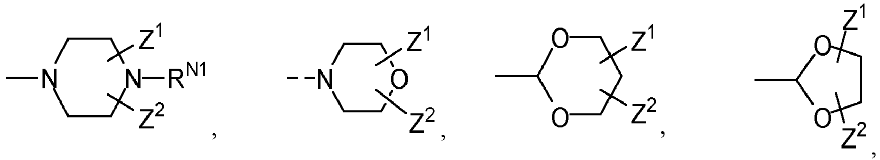

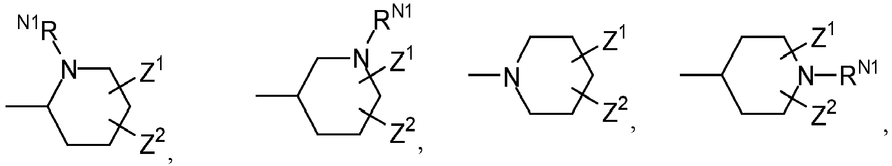

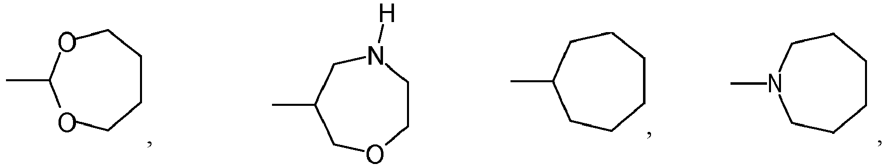

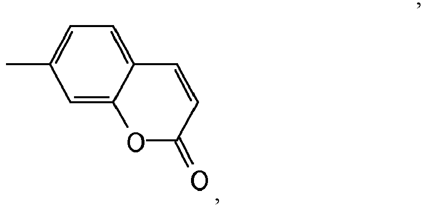

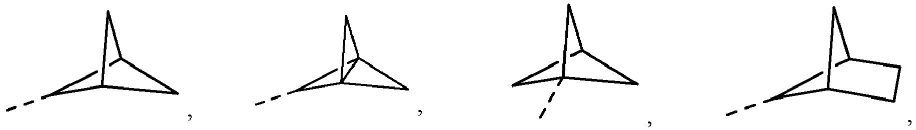

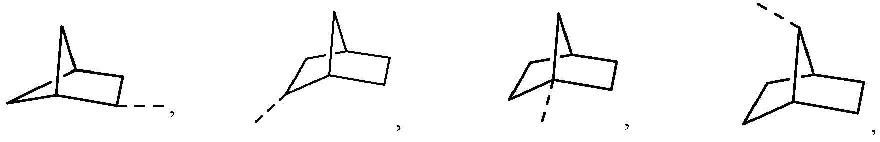

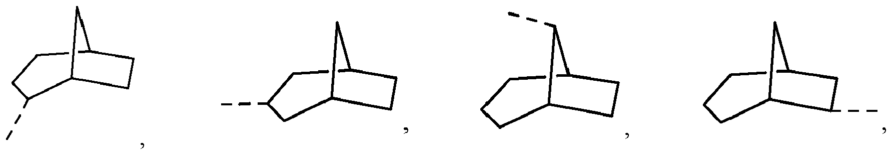

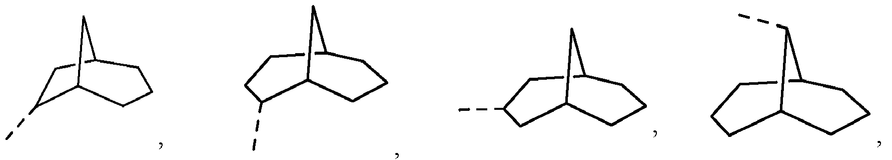

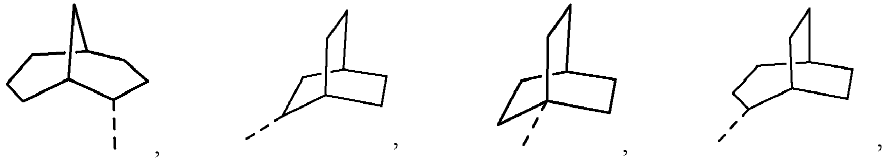

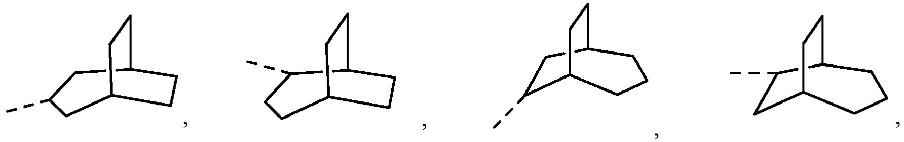

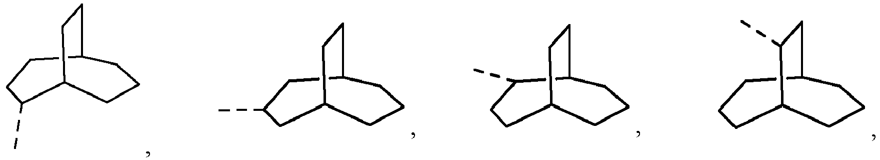

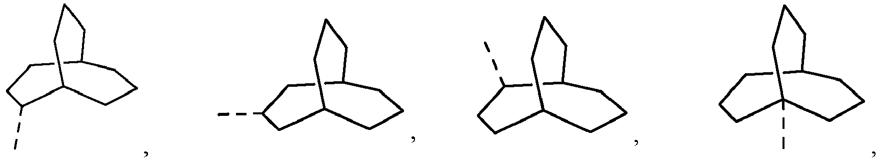

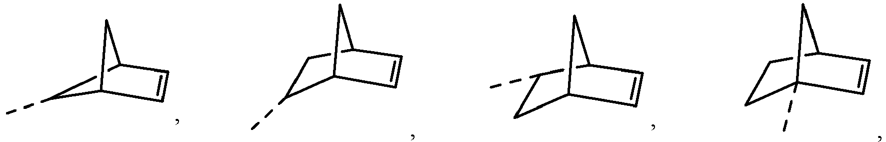

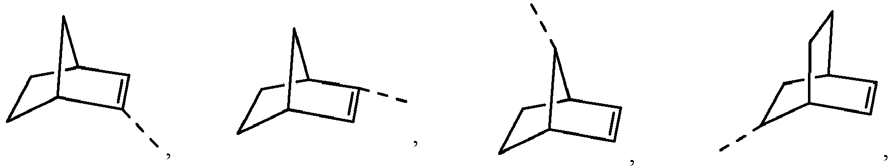

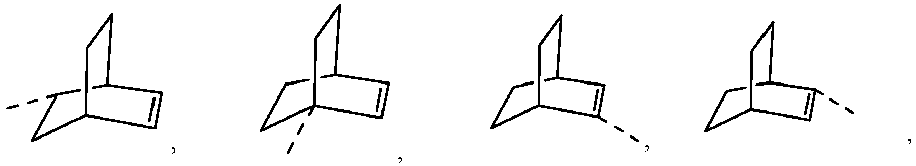

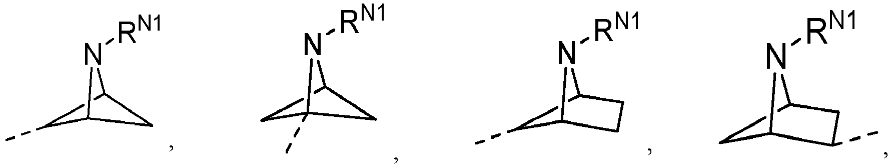

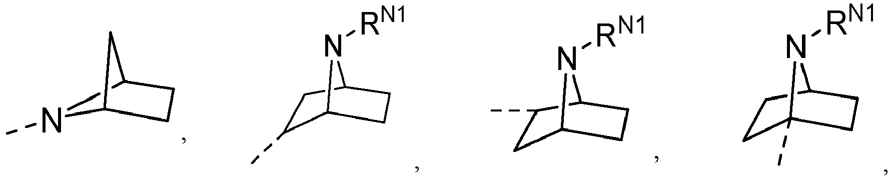

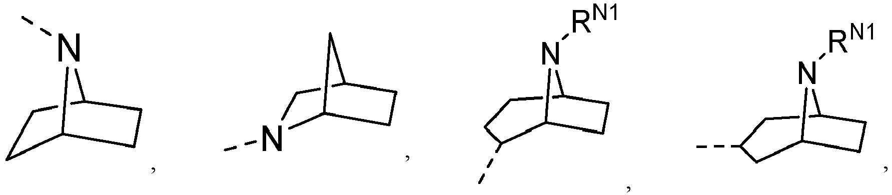

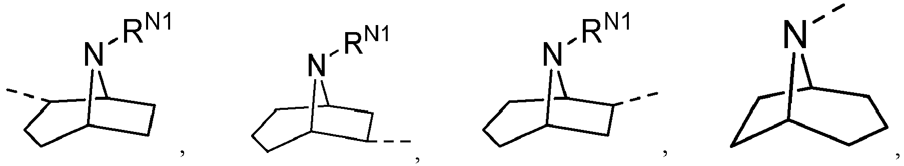

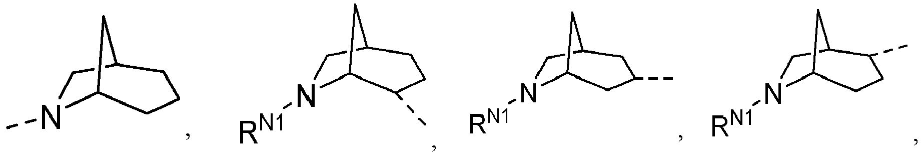

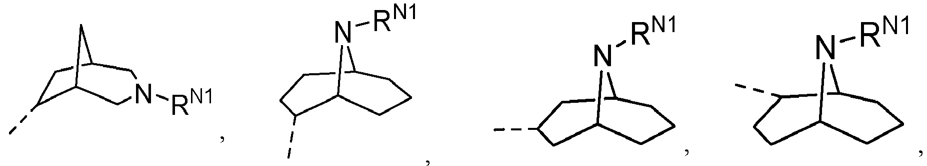

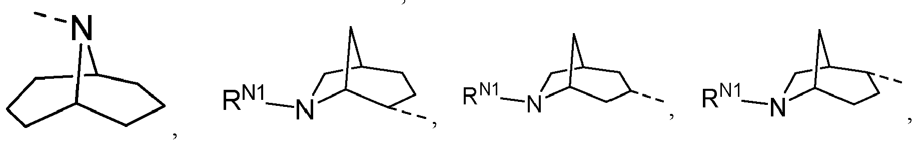

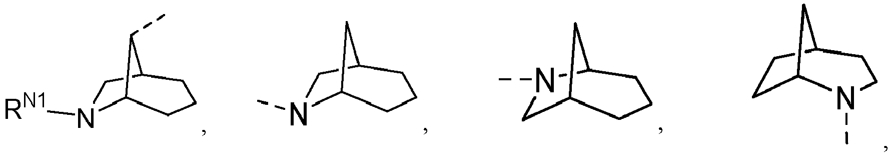

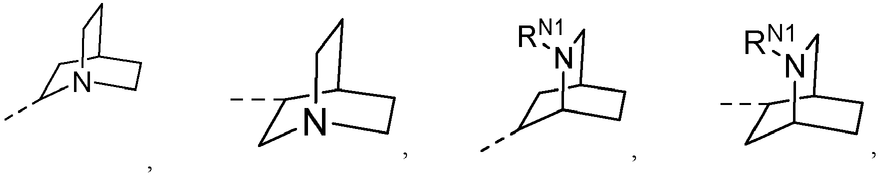

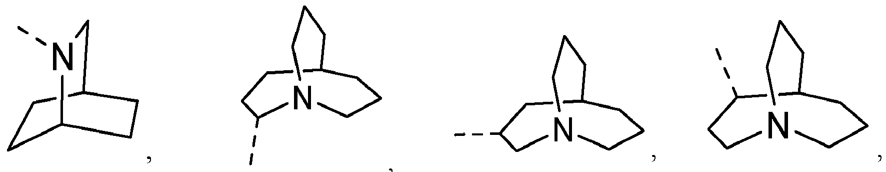

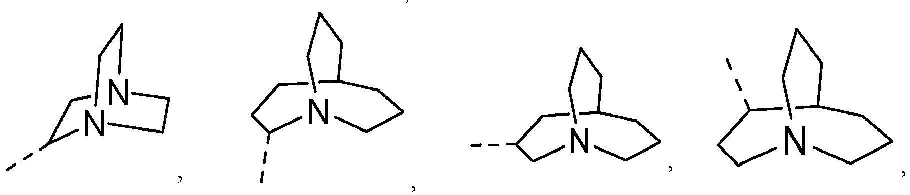

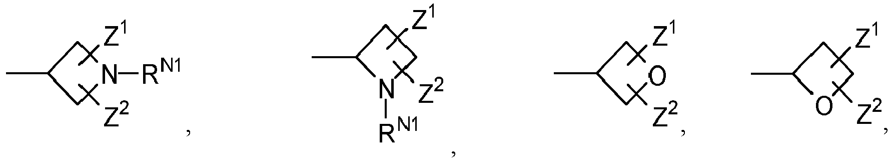

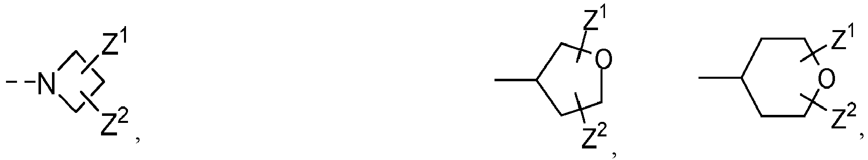

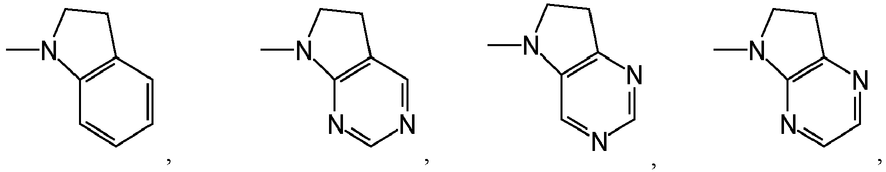

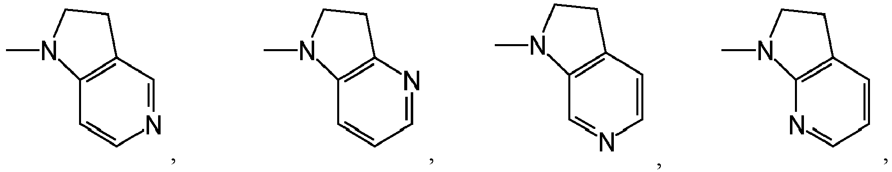

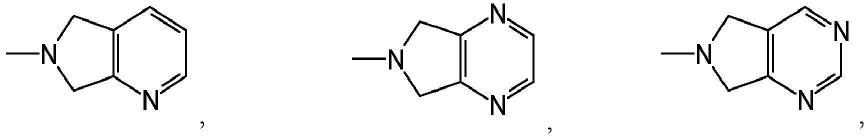

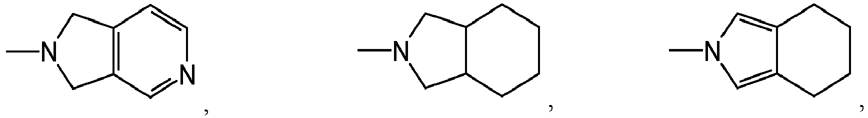

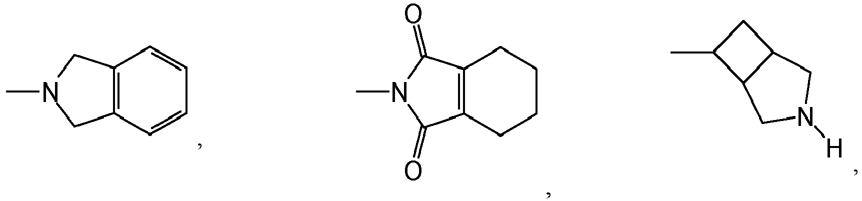

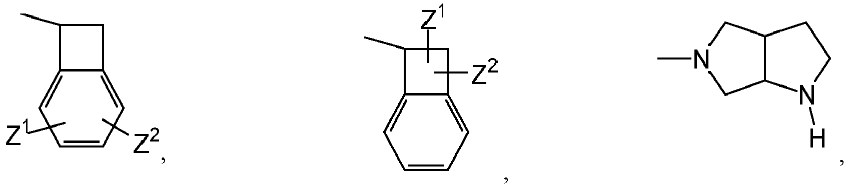

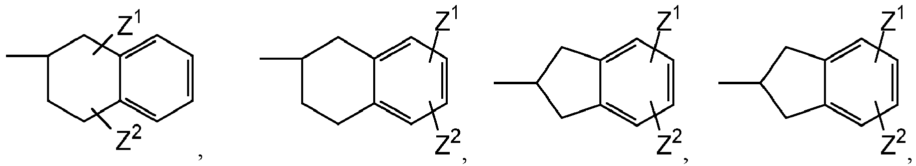

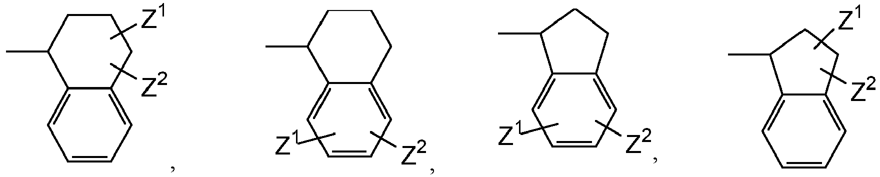

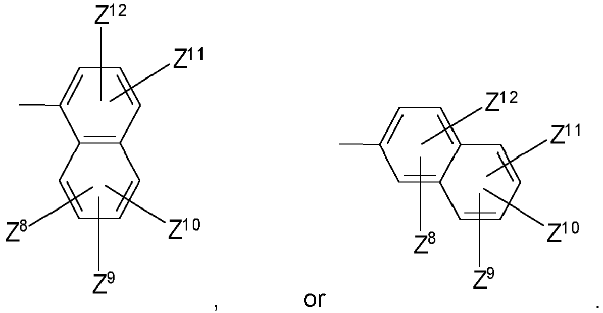

More preferred, is the compound of formula (**I**), wherein **R⁸** and **R⁹** represent independently of each other:

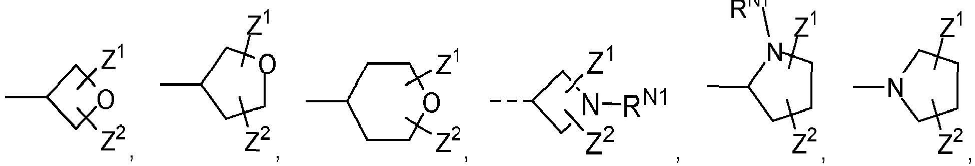

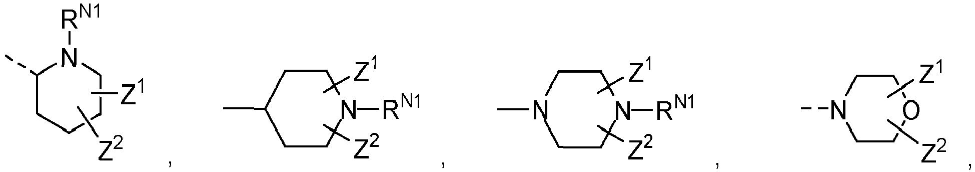

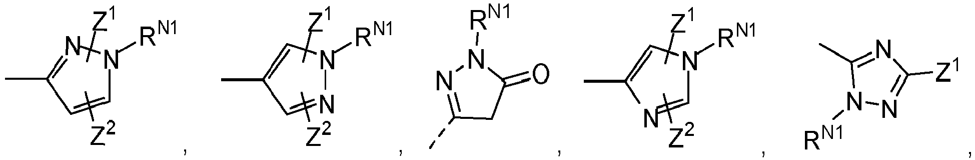

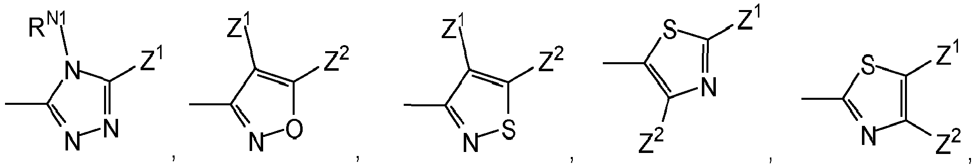

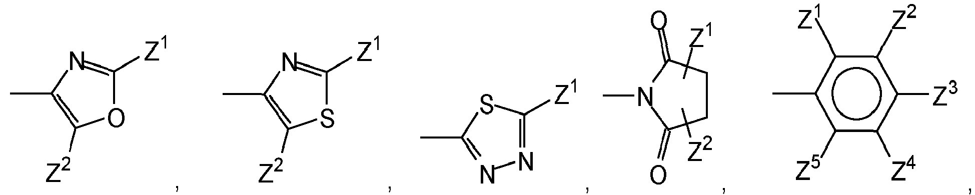

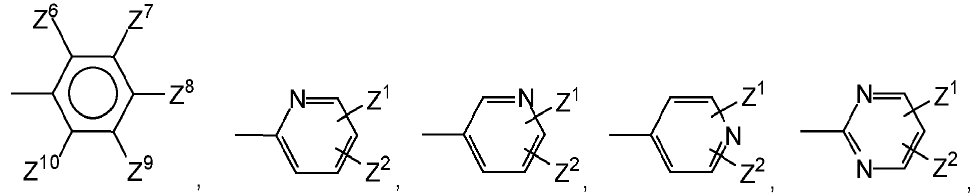

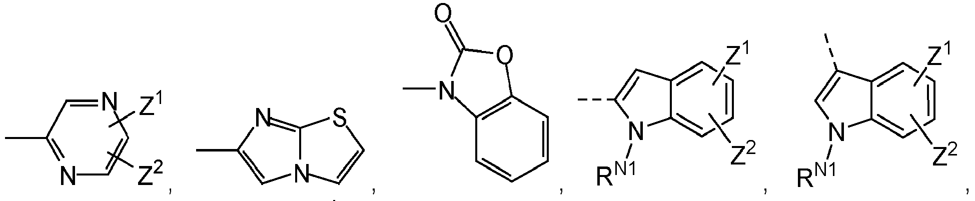

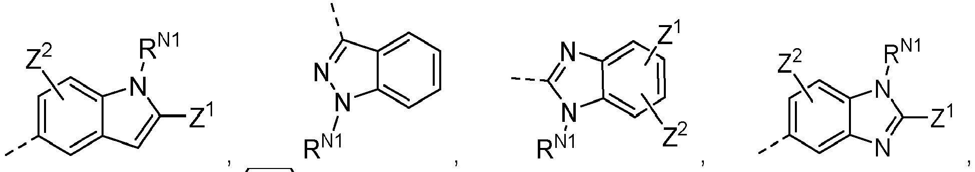

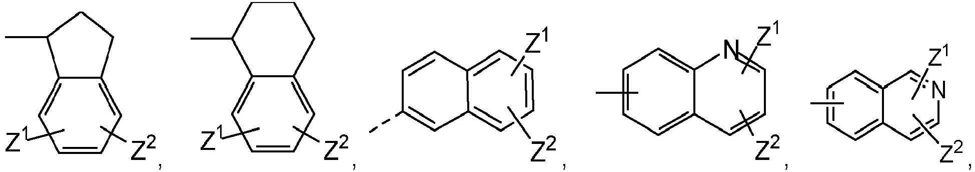

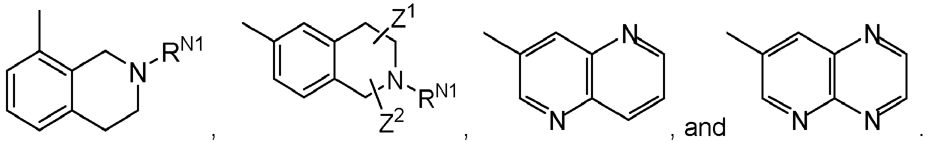

Still more preferred, is the compound of formula (**I**), wherein **R⁸** represents

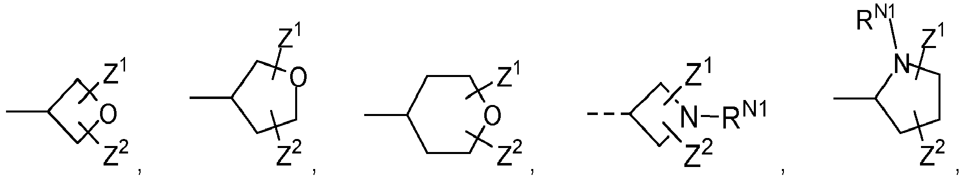

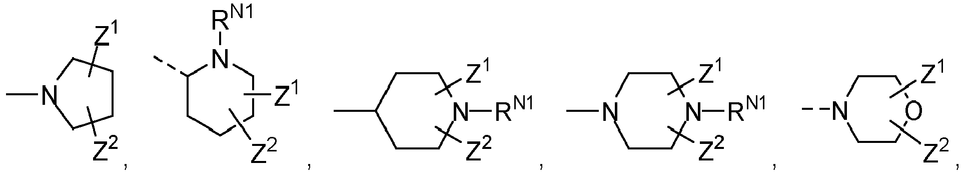

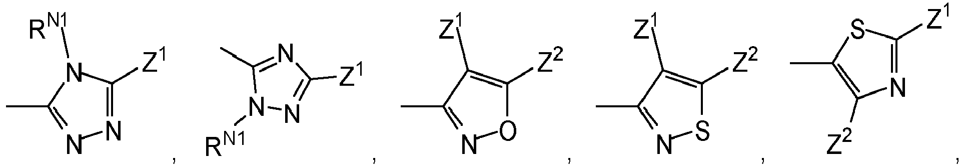

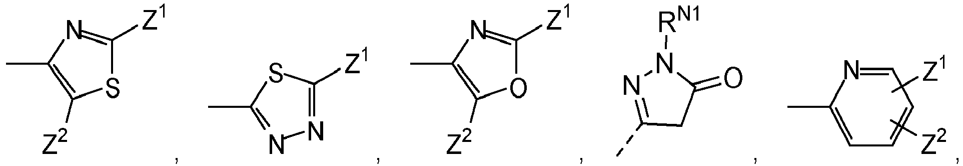

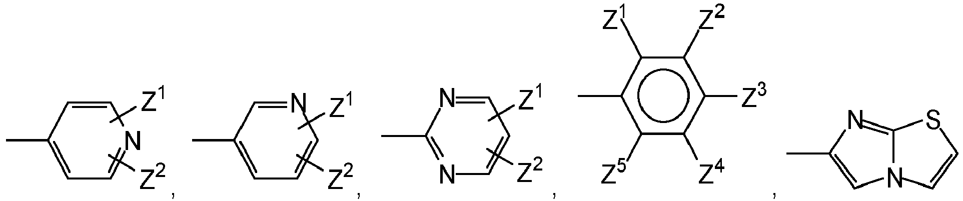

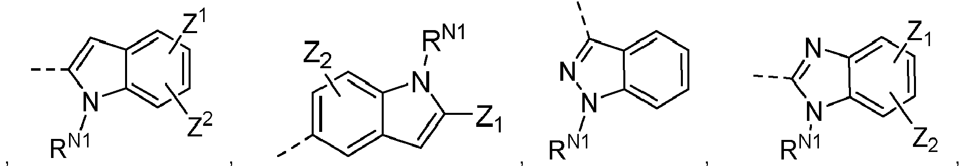

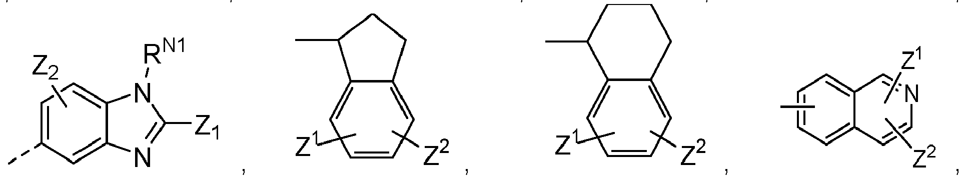

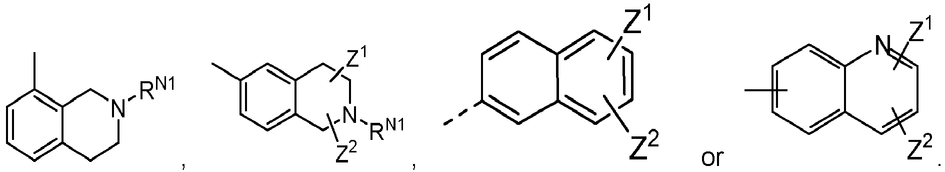

Still preferred, is the compound of the formula (**I**), wherein **R⁹** represents

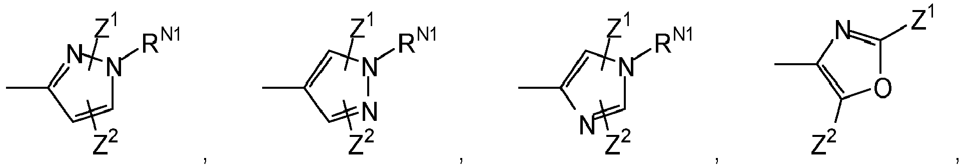

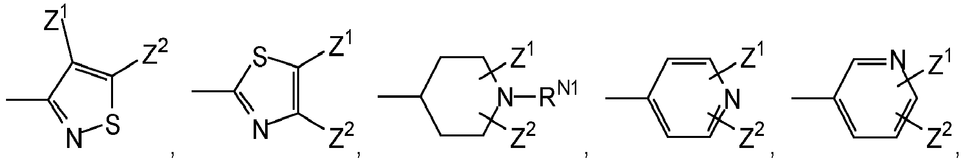

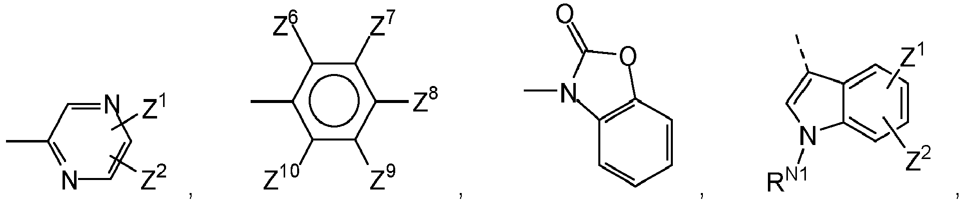

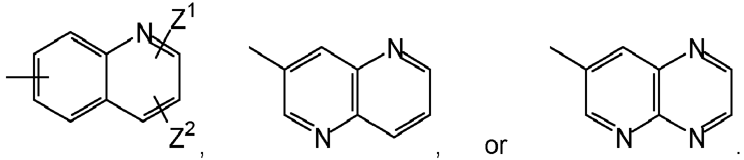

Preferably, **R¹⁵** represents -CH₂-OH, -CH₂CH₂-OH, -CH₂CH₂CH₂-OH, -CH₂CH₂CH₂CH₂-OH, -CH₂CH₂CH₂CH₂CH₂-OH, -CH₂CH₂CH₂CH₂CH₂-OH, -CH₂-CO₂H, -CH₂CH₂-CO₂H, -CHCH₂CH₂-CO₂H, -CH₂CH₂CH₂CH₂-CO₂H, -CH₂CH₂CH₂CH₂CH₂-CO₂H, -CH₂CH₂CH₂CH₂CH₂-CO₂H, -CH₂-SH, -CH₂CH₂-SH, -CH₂CH₂CH₂-SH, -CH₂CH₂CH₂CH₂-SH, -CH₂CH₂CH₂CH₂CH₂-SH, -CH₂CH₂CH₂CH₂CH₂-SH, -CH₂-SO₃H, -CH₂CH₂-SO₃H, -CH₂CH₂CH₂-SO₃H, -CH₂CH₂CH₂CH₂-SO₃H, -CH₂CH₂CH₂CH₂CH₂-SO₃H, -CH₂CH₂CH₂CH₂CH₂-SO₃H, -CH₂-NH₂, -CH₂CH₂-NH₂, -CH₂CH₂CH₂-NH₂, -CH₂CH₂CH₂CH₂-NH₂, -CH₂CH₂CH₂CH₂CH₂-NH₂, -CH₂CH₂CH₂CH₂CH₂CH₂-NH₂, -CH₂CH₂-O-CH₂-CH₂-OH, -(CH₂CH₂-O)₂-CH₂-CH₂-OH, -(CH₂CH₂-O)₃-CH₂-CH₂-OH, -CH₂CH₂-O-CH₂-CH₂-CO₂H, -(CH₂CH₂-O)₂-CH₂-CH₂-CO₂H, -(CH₂CH₂-O)₃-CH₂-CH₂-CO₂H, -CH₂CH₂-O-CH₂-CH₂-SH, -(CH₂CH₂-O)₂-CH₂-CH₂-SH, -(CH₂CH₂-O)₃-CH₂-CH₂-SH, -CH₂CH₂-O-CH₂-CH₂-SO₃H, -(CH₂CH₂-O)₂-CH₂-CH₂-SO₃H, -(CH₂CH₂-O)₃-CH₂-CH₂-SO₃H, -CH₂CH₂-O-CH₂-CH₂- NH₂, -(CH₂CH₂-O)₂-CH₂-CH₂-NH₂, or -(CH₂CH₂-O)₃-CH₂-CH₂-NH₂.

More preferably, **R¹⁵** represents -CH₂-NH₂, -CH₂CH₂-NH₂, -CH₂CH₂CH₂-NH₂, -CH₂-SO₃H, -CH₂CH₂-SO₃H, -CH₂CH₂CH₂-SO₃H, -CH₂CH₂-O-CH₂-CH₂-NH₂, -(CH₂CH₂-O)₂-CH₂-CH₂-NH₂, or -(CH₂CH₂-O)₃-CH₂-CH₂-NH₂.

Preferably, **R¹⁶** represents -CH₂-OH, -CH₂CH₂-OH, -CH₂CH₂CH₂-OH, -CH₂CH₂CH₂CH₂-OH, -CH₂CH₂CH₂CH₂CH₂-OH, -CH₂CH₂CH₂CH₂CH₂-OH, -CH₂-CO₂H, -CH₂CH₂-CO₂H, -CH₂CH₂CH₂-CO₂H, -CH₂CH₂CH₂CH₂-CO₂H, -CH₂CH₂CH₂CH₂CH₂-CO₂H, -CH₂CH₂CH₂CH₂CH₂-CO₂H, -CH₂-SH, -CH₂CH₂-SH, -CH₂CH₂CH₂-SH, -CH₂CH₂CH₂CH₂-SH, -CH₂CH₂CH₂CH₂CH₂-SH, -CH₂CH₂CH₂CH₂CH₂- SH, -CH₂-SO₃H, -CH₂CH₂-SO₃H, -CH₂CH₂CH₂-SO₃H, -CH₂CH₂CH₂CH₂-SO₃H, -CH₂CH₂CH₂CH₂CH₂-SO₃H, -CH₂CH₂CH₂CH₂CH₂-SO₃H, -CH₂-NH₂, -CH₂CH₂-NH₂, -CH₂CH₂CH₂-NH₂, -CH₂CH₂CH₂CH₂-NH₂, -CH₂CH₂CH₂CH₂CH₂-NH₂, -CH₂CH₂CH₂CH₂CH₂CH₂-NH₂, -CH₂CH₂-O-CH₂-CH₂-OH, -(CH₂CH₂-O)₂-CH₂-CH₂-OH, -(CH₂CH₂-O)₃-CH₂-CH₂-OH, -CH₂CH₂-O-CH₂-CH₂-CO₂H, -(CH₂CH₂-O)₂-CH₂-CH₂-CO₂H, -(CH₂CH₂-O)₃-CH₂-CH₂-CO₂H, -CH₂CH₂-O-CH₂-CH₂-SH, -(CH₂CH₂-O)₂-CH₂-CH₂-SH, -(CH₂CH₂-O)₃-CH₂-CH₂-SH, -CH₂CH₂-O-CH₂-CH₂-SO₃H, -(CH₂CH₂-O)₂-CH₂-CH₂-SO₃H, -(CH₂CH₂-O)₃-CH₂-CH₂-SO₃H, -CH₂CH₂-O-CH₂-CH₂-NH₂, -(CH₂CH₂-O)₂-CH₂-CH₂-NH₂, -(CH₂CH₂-O)₃-CH₂-CH₂-NH₂ , -OCH₂-OH, -OCH₂CH₂-OH, -OCH₂CH₂CH₂-OH, -OCH₂CH₂CH₂CH₂-OH, -OCH₂CH₂CH₂CH₂CH₂-OH, -OCH₂CH₂CH₂CH₂CH₂-OH, -OCH₂-CO₂H, -OCH₂CH₂-CO₂H, -OCH₂CH₂CH₂-CO₂H, -OCH₂CH₂CH₂CH₂-CO₂H, -OCH₂CH₂CH₂CH₂CH₂-CO₂H, -OCH₂CH₂CH₂CH₂CH₂-CO₂H, -OCH₂-SH, -OCH₂CH₂-SH, -OCH₂CH₂CH₂-SH, -OCH₂CH₂CH₂CH₂-SH, -OCH₂CH₂CH₂CH₂CH₂-SH, -OCH₂CH₂CH₂CH₂CH₂-SH, -OCH₂-SO₃H, -OCH₂CH₂-SO₃H, -OCH₂CH₂CH₂-SO₃H, -OCH₂CH₂CH₂CH₂-SO₃H, -OCH₂CH₂CH₂CH₂CH₂-SO₃H, -OCH₂CH₂CH₂CH₂CH₂-SO₃H, -OCH₂-NH₂, -OCH₂CH₂-NH₂, -OCH₂CH₂CH₂-NH₂, -OCH₂CH₂CH₂CH₂-NH₂, -OCH₂CH₂CH₂CH₂CH₂-NH₂, -OCH₂CH₂CH₂CH₂CH₂CH₂-NH₂, -OCH₂CH₂-O-CH₂-CH₂-OH, -O(CH₂CH₂-O)₂-CH₂-CH₂-OH, -O(CH₂CH₂-O)₃-CH₂-CH₂-OH, -OCH₂CH₂-O-CH₂-CH₂-CO₂H, -O(CH₂CH₂-O)₂-CH₂-CH₂-CO₂H, -O(CH₂CH₂-O)₃-CH₂-CH₂-CO₂H, -OCH₂CH₂-O-CH₂-CH₂-SH, -O(CH₂CH₂-O)₂-CH₂-CH₂-SH, -O(CH₂CH₂-O)₃-CH₂-CH₂-SH, -OCH₂CH₂-O-CH₂-CH₂-SO₃H, -O(CH₂CH₂-O)₂-CH₂-CH₂-SO₃H, -O(CH₂CH₂-O)₃-CH₂-CH₂-SO₃H, -OCH₂CH₂-O-CH₂-CH₂-NH₂, -O(CH₂CH₂-O)₂-CH₂-CH₂-NH₂, -O(CH₂CH₂-O)₃-CH₂-CH₂-NH₂, -SCH₂-OH, -SCH₂CH₂-OH, -SCH₂CH₂CH₂-OH, -SCH₂CH₂CH₂CH₂-OH, -SCH₂CH₂CH₂CH₂CH₂-OH, -SCH₂CH₂CH₂CH₂CH₂-OH, -SCH₂-CO₂H, -SCH₂CH₂-CO₂H, -SCH₂CH₂CH₂-CO₂H, -SCH₂CH₂CH₂CH₂-CO₂H, -SCH₂CH₂CH₂CH₂CH₂-CO₂H, -SCH₂CH₂CH₂CH₂CH₂-CO₂H, -SCH₂-SH, -SCH₂CH₂-SH, -SCH₂CH₂CH₂-SH, -SCH₂CH₂CH₂CH₂-SH, -SCH₂CH₂CH₂CH₂CH₂-SH, -SCH₂CH₂CH₂CH₂CH₂-SH, -SCH₂-SO₃H, -SCH₂CH₂-SO₃H, -SCH₂CH₂CH₂-SO₃H, -SCH₂CH₂CH₂CH₂-SO₃H, -SCH₂CH₂CH₂CH₂CH₂-SO₃H, -SCH₂CH₂CH₂CH₂CH₂-SO₃H, -SCH₂-NH₂, -SCH₂CH₂-NH₂, -SCH₂CH₂CH₂-NH₂, -SCH₂CH₂CH₂CH₂-NH₂, -SCH₂CH₂CH₂CH₂CH₂-NH₂, -SCH₂CH₂CH₂CH₂CH₂CH₂-NH₂, -SCH₂CH₂-O-CH₂-CH₂-OH, -S(CH₂CH₂-O)₂-CH₂-CH₂-OH, -S(CH₂CH₂-O)₃-CH₂-CH₂-OH, -SCH₂CH₂-O-CH₂-CH₂-CO₂H, -S(CH₂CH₂-O)₂-CH₂-CH₂-CO₂H, -S(CH₂CH₂-O)₃-CH₂-CH₂-CO₂H, -SCH₂CH₂-O-CH₂-CH₂-SH, -S(CH₂CH₂-O)₂-CH₂-CH₂-SH, -S(CH₂CH₂-O)₃-CH₂-CH₂-SH, -SCH₂CH₂-O-CH₂-CH₂-SO₃H, -S(CH₂CH₂-O)₂-CH₂-CH₂-SO₃H, -S(CH₂CH₂-O)₃-CH₂-CH₂-SO₃H, -SCH₂CH₂-O-CH₂-CH₂-NH₂, -S(CH₂CH₂-O)₂-CH₂-CH₂-NH₂, -S(CH₂CH₂-O)₃-CH₂-CH₂-NH₂, -NHCH₂-OH, -NHCH₂CH₂-OH, -NHCH₂CH₂CH₂-OH, -NHCH₂CH₂CH₂CH₂-OH, -NHCH₂CH₂CH₂CH₂CH₂-OH, -NHCH₂CH₂CH₂CH₂CH₂-OH, -NHCH₂-CO₂H, -NHCH₂CH₂-CO₂H, -NHCH₂CH₂CH₂-CO₂H, -NHCH₂CH₂CH₂CH₂-CO₂H, -NHCH₂CH₂CH₂CH₂CH₂-CO₂H, -NHCH₂CH₂CH₂CH₂CH₂-CO₂H, -NHCH₂-SH, -NHCH₂CH₂-SH, -NHCH₂CH₂CH₂-SH, -NHCH₂CH₂CH₂CH₂-SH, -NHCH₂CH₂CH₂CH₂CH₂-SH, -NHCH₂CH₂CH₂CH₂CH₂-SH, -NHCH₂-SO₃H, -NHCH₂CH₂-SO₃H, -NHCH₂CH₂CH₂-SO₃H, -NHCH₂CH₂CH₂CH₂-SO₃H, -NHCH₂CH₂CH₂CH₂CH₂-SO₃H, -NHCH₂CH₂CH₂CH₂CH₂-SO₃H, -NHCH₂-NH₂, -NHCH₂CH₂-NH₂, -NHCH₂CH₂CH₂-NH₂, -NHCH₂CH₂CH₂CH₂-NH₂, -NHCH₂CH₂CH₂CH₂CH₂-NH₂, -NHCH₂CH₂CH₂CH₂CH₂CH₂-NH₂, -NHCH₂CH₂-O-CH₂-CH₂-OH, -NH(CH₂CH₂-O)₂-CH₂-CH₂-OH, -NH(CH₂CH₂-O)₃-CH₂-CH₂-OH, -NHCH₂CH₂-O-CH₂-CH₂-CO₂H, -NH(CH₂CH₂-O)₂-CH₂-CH₂-CO₂H, -NH(CH₂CH₂-O)₃-CH₂-CH₂-CO₂H, -NHCH₂CH₂-O-CH₂-CH₂-SH, -NH(CH₂CH₂-O)₂-CH₂-CH₂-SH, -NH(CH₂CH₂-O)₃-CH₂-CH₂-SH, -NHCH₂CH₂-O-CH₂-CH₂-SO₃H, -NH(CH₂CH₂-O)₂-CH₂-CH₂-SO₃H, -NH(CH₂CH₂-O)₃-CH₂-CH₂-SO₃H, -NHCH₂CH₂-O-CH₂-CH₂-NH₂, -NH(CH₂CH₂-O)₂-CH₂-CH₂-NH₂, or -NH(CH₂CH₂-O)₃-CH₂-CH₂-NH₂.

More preferably, **R¹⁶** represents -CH₂-NH₂, -CH₂CH₂-NH₂, -CH₂CH₂CH₂-NH₂, -CH₂-SO₃H, -CH₂CH₂-SO₃H, -CH₂CH₂CH₂-SO₃H, -CH₂CH₂-O-CH₂-CH₂-NH₂, -(CH₂CH₂-O)₂-CH₂-CH₂-NH₂, or -(CH₂CH₂-O)₃-CH₂-CH₂-NH₂.

Also preferred is the compound of formula (**I**), wherein **A** is -CO-NH-, -CO-NCH₃-, 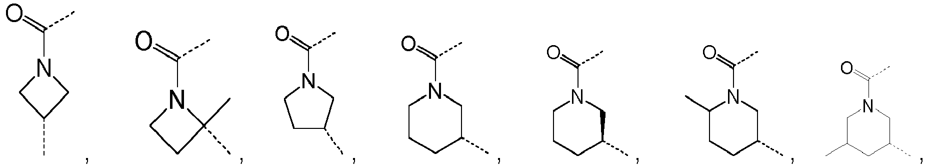 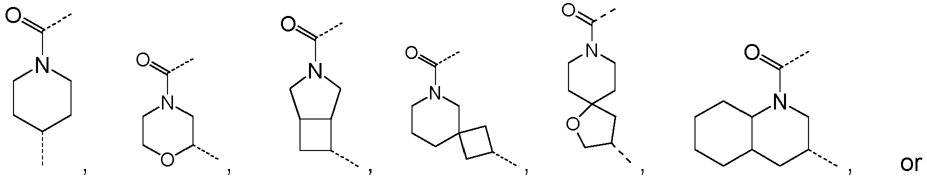 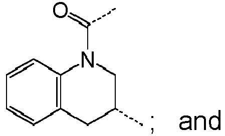
**X₂** represents a bond, -CH₂-, -CH₂CH₂-, or -CH₂CH₂CH₂-; and
**X₁** represents a bond, -CH₂-, -CH₂CH₂-, -CH₂CH₂CH₂-, or -CH₂CH₂CH₂CH₂-;
more preferred are compounds wherein **-X₂-A-X₁-** represents

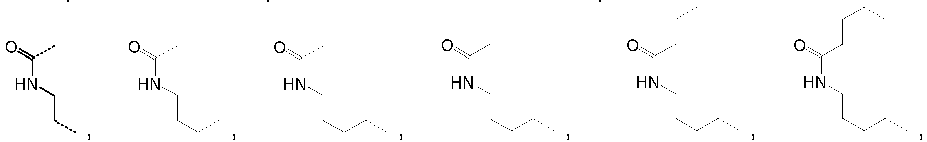

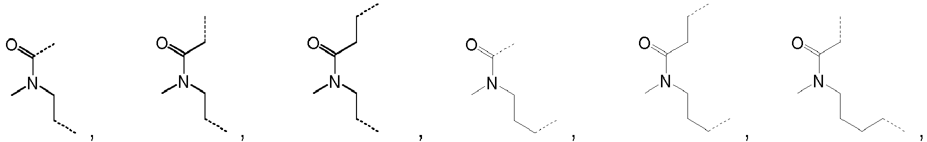

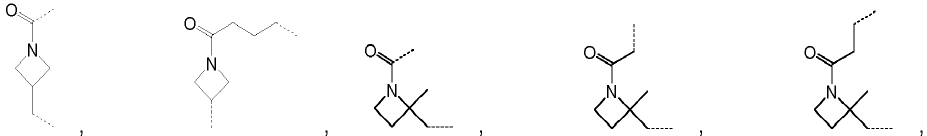

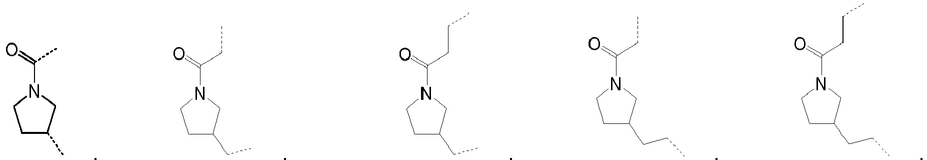

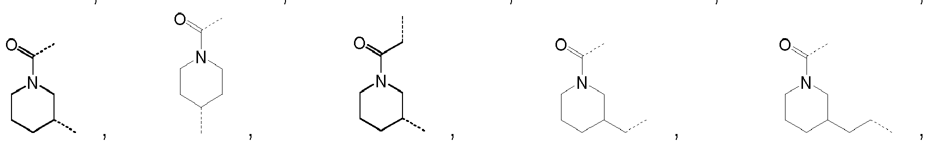

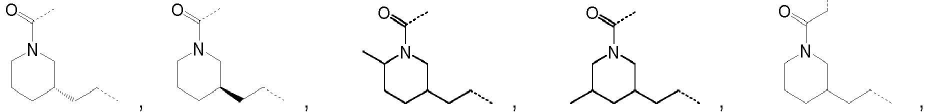

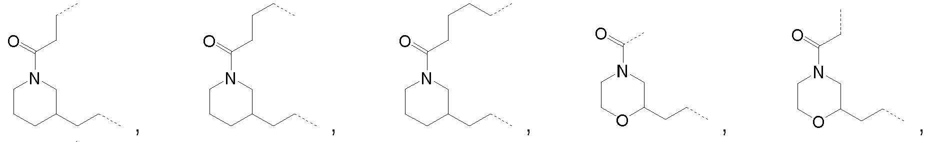

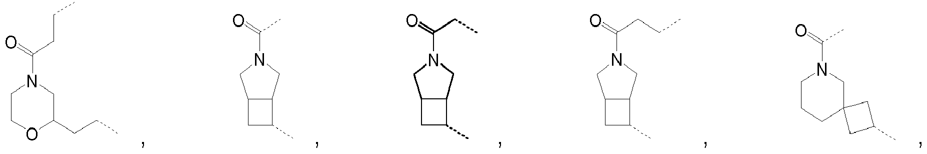

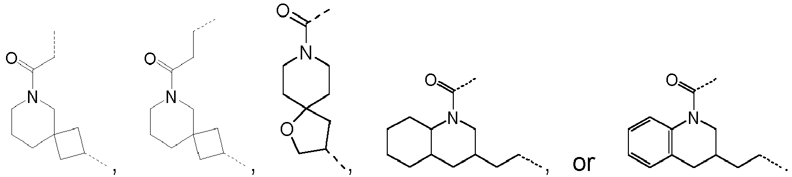

Also preferred, is the compound of general formula (**I**), wherein
**B** represents -H, -NH₂, -NHCOCH₃, -NHCOC(CH₃)₃, -NHCOC(CN)(CH₃)₂, -NHCOCH=CH₂, -NHCOCH₂C(CH₃)₃, -NHCOPh, -NHCOCH₂Ph, -NHCOCH₂-NH(CH₃), -NHCOCH₂-N(CH₃)CO₂tBu, -NHCOC(CH₃)₂CH₂OH, -NHCOC(CH₃)₂CH₂SH, -NHCOC(CH₂CH₃)₂CH₂OH, -NHCOC(CH₃)(CH₂OH)₂, -NHCOCH₂OCH₂CH₂-NH(CH₃), -NHCOCH_{2O}CH₂CH₂-N(CH₃)CO₂tBu, -NHCO₂CH₂CH₃, -NHCO₂(CH₂CH₂O)₃CH₃, -NHCO₂(CH₂CH₂O)₅CH₃, -NHCO₂(CH₂CH₂O)₇CH₃, -NHCO₂CH₂Ph -NHCONHCH₂CH₃, -NHSO₂CH₃, -NHSO₂CH=CH₂, -NHSO₂CH₂Ph, -NHSO₂CH₂CH₂Ph, -NHSO₂CH₂CH₂CH₂Ph, 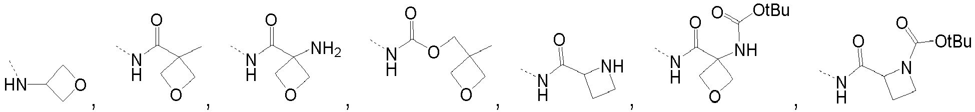 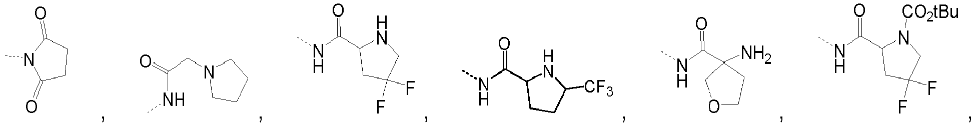 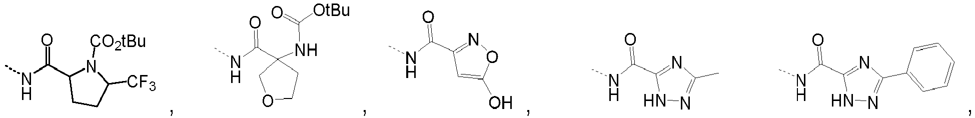 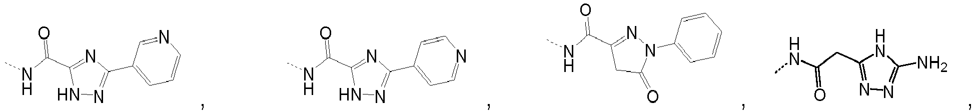 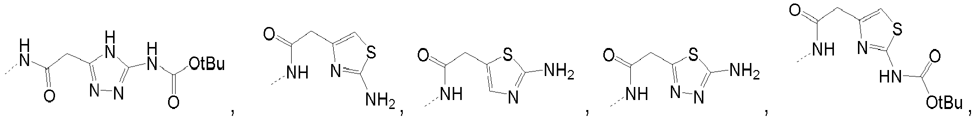 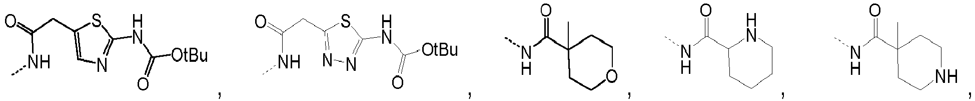 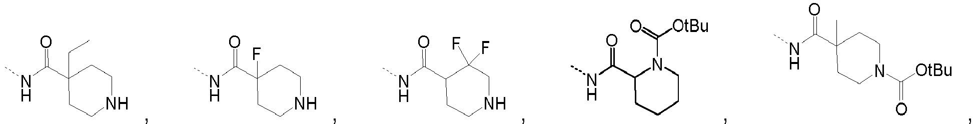 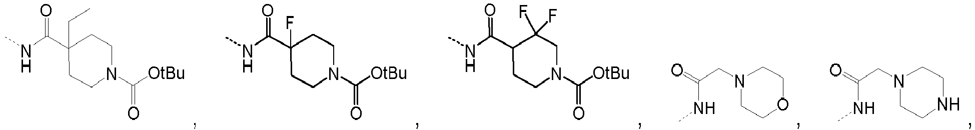 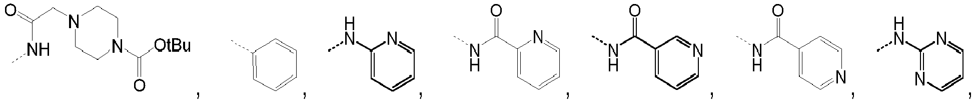 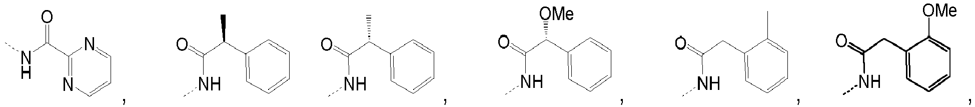 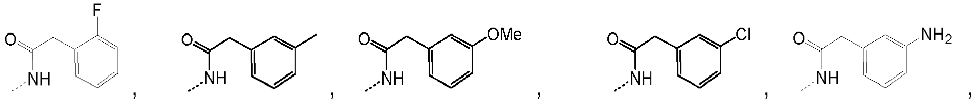 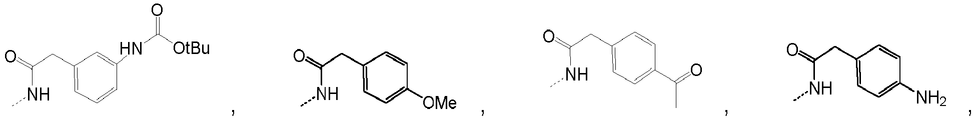 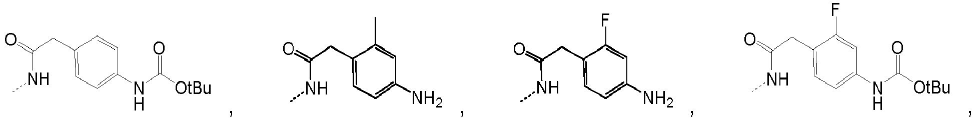 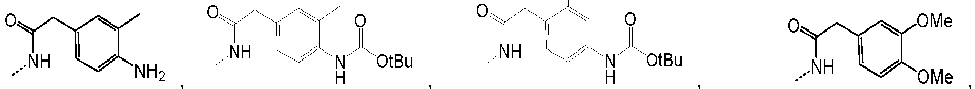 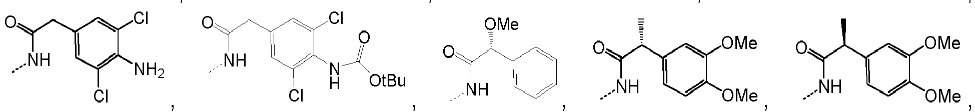 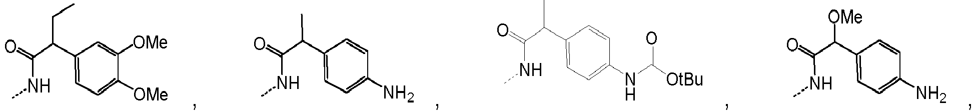 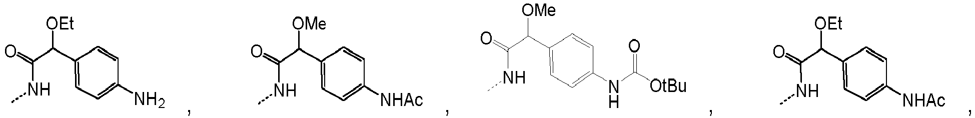 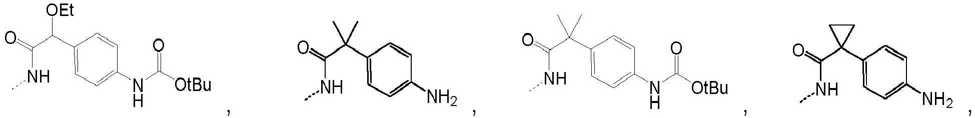 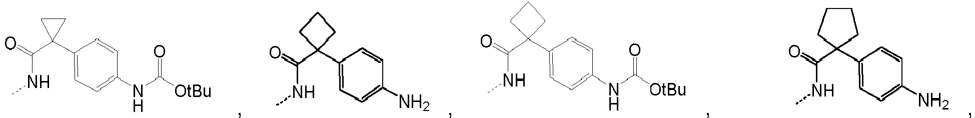 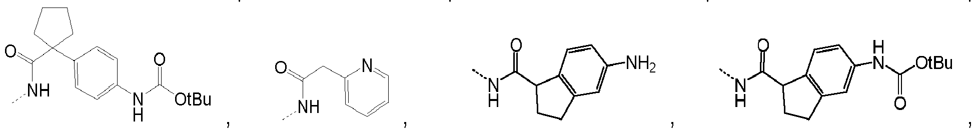 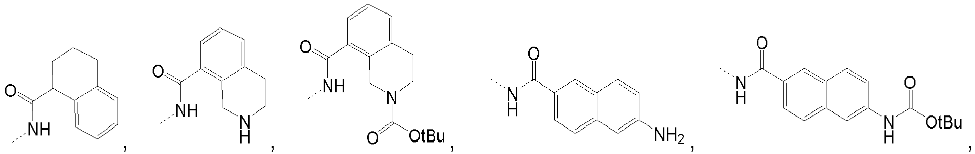 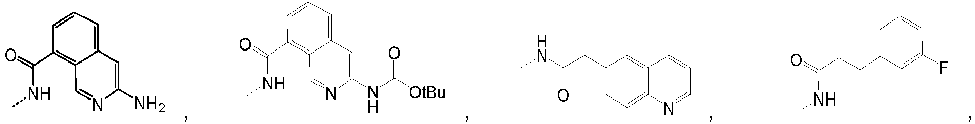 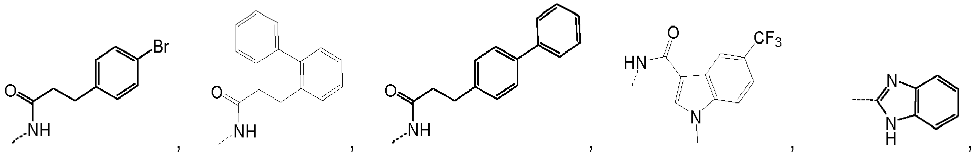 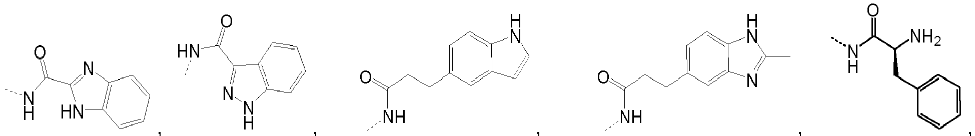 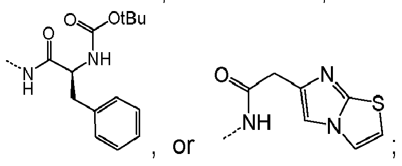 and
**R¹** represents -CH₃,

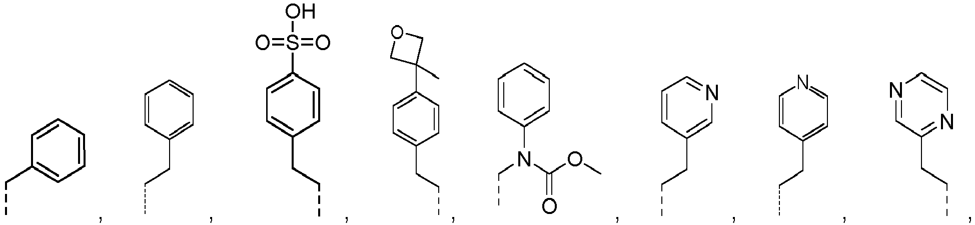

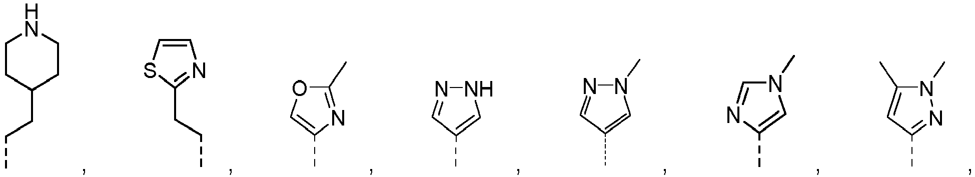

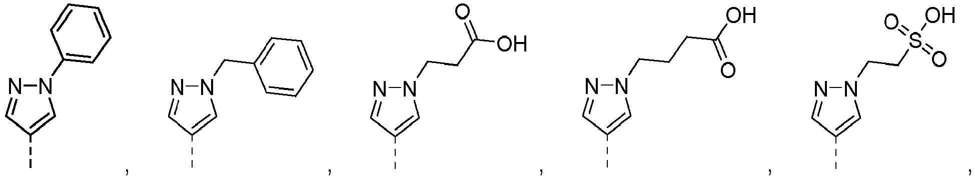

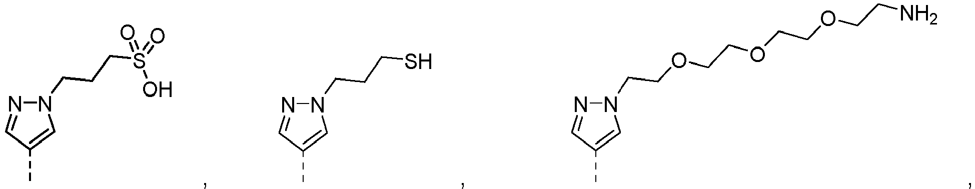

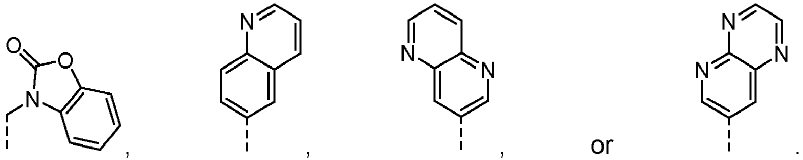

More preferred, the present invention relates to the compound of the formula (I), 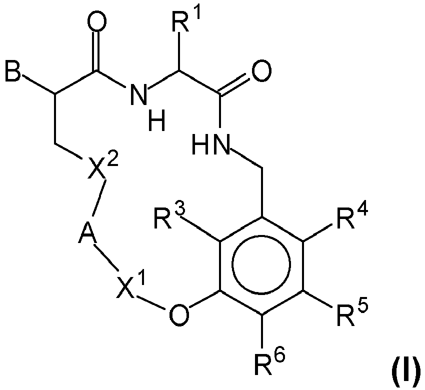 wherein
**A** represents -CO-NH-, -CO-NCH₃- 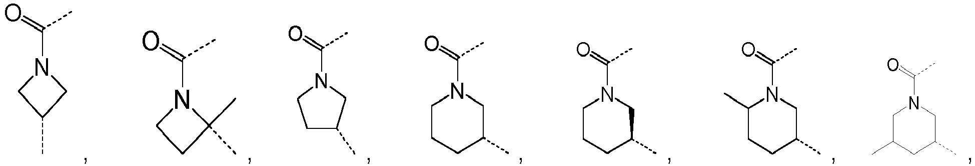 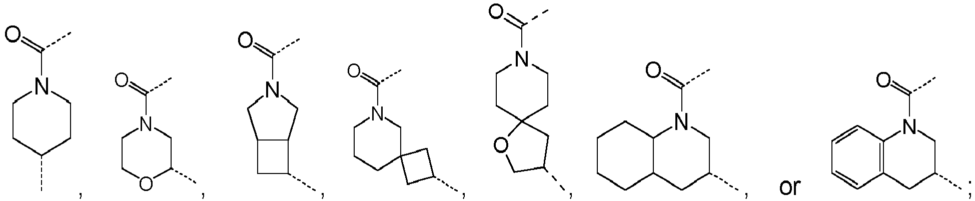
**X₂** represents a bond, -CH₂-, -CH₂CH₂-, or -CH₂CH₂CH₂-;
**X₁** represents a bond, -CH₂-, -CH₂CH₂-, -CH₂CH₂CH₂-, or -CH₂CH₂CH₂CH₂-;
**B** represents -H, -NH₂, -NHCOCH₃, -NHCOC(CH₃)₃, -NHCOC(CN)(CH₃)₂, -NHCOCH=CH₂, -NHCOCH₂C(CH₃)₃, -NHCOPh, -NHCOCH₂Ph, -NHCOCH₂-NH(CH₃), -NHCOCH₂-N(CH₃)CO₂tBu, -NHCOC(CH₃)₂CH₂OH, -NHCOC(CH₃)₂CH₂SH, -NHCOC(CH₂CH₃)₂CH₂OH, -NHCOC(CH₃)(CH₂OH)₂, -NHCOCH₂OCH₂CH₂-NH(CH₃), -NHCOCH_{2O}CH₂CH₂-N(CH₃)CO₂tBu, -NHCO₂CH₂CH₃,-NHCO₂(CH₂CH₂O)₃CH₃, -NHCO₂(CH₂CH₂O)₅CH₃, -NHCO₂(CH₂CH₂O)₇CH₃, -NHCO₂CH₂Ph-NHCONHCH₂CH₃, -NHSO₂CH₃, -NHSO₂CH=CH₂, -NHSO₂CH₂Ph,-NHSO₂CH₂CH₂Ph, -NHSO₂CH₂CH₂CH₂Ph, 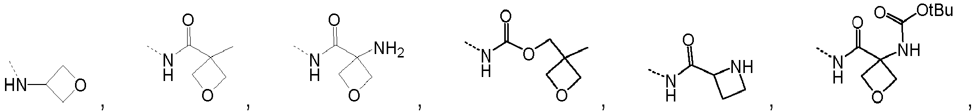 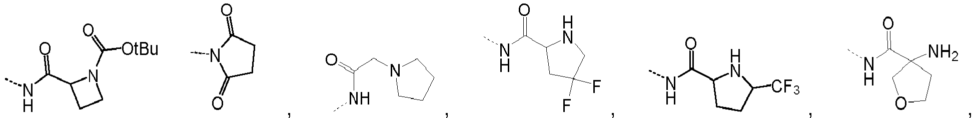 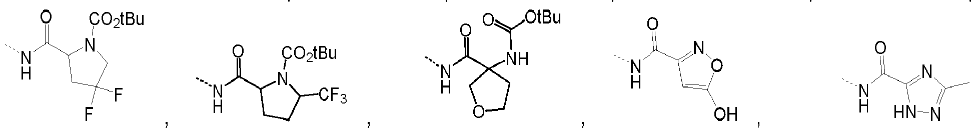 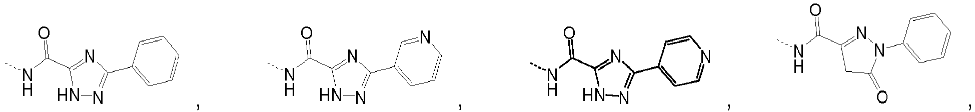 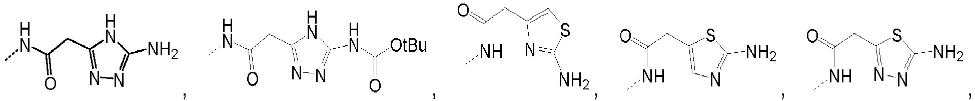 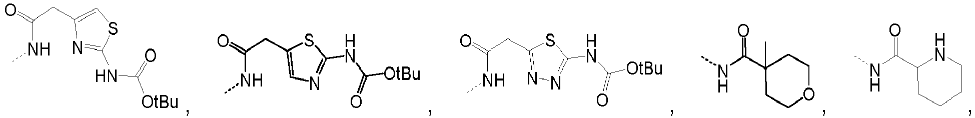 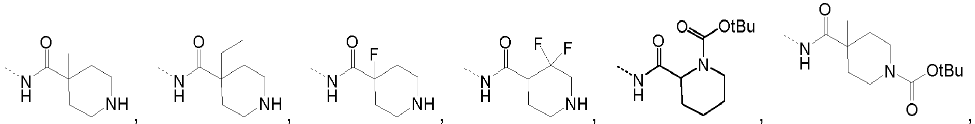 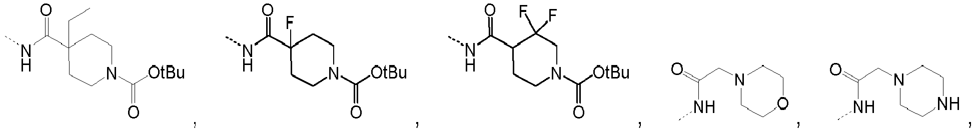 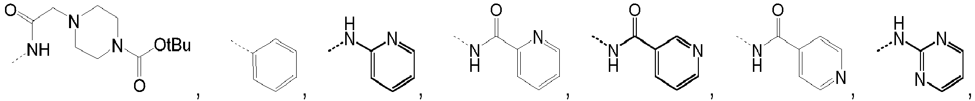 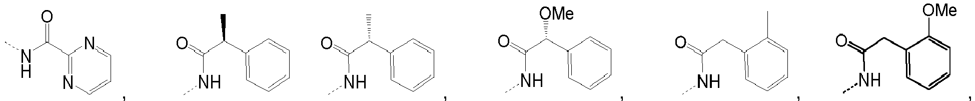 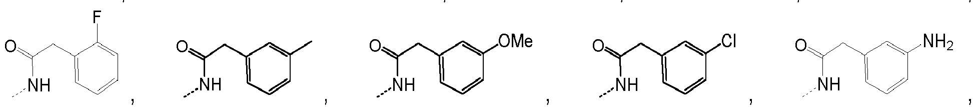 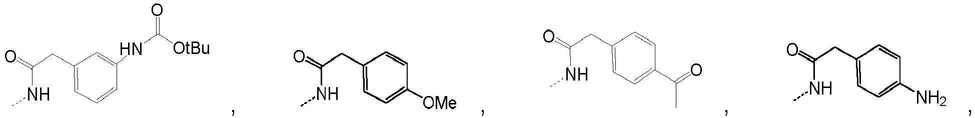 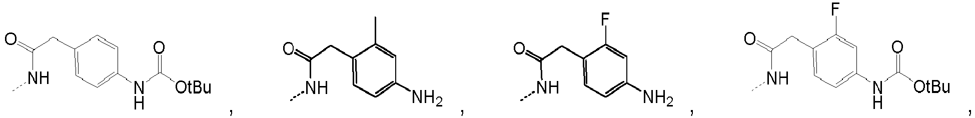 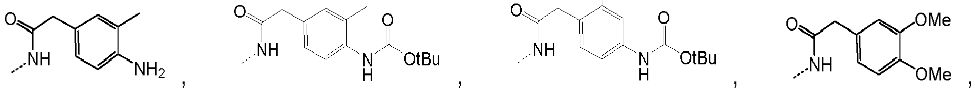 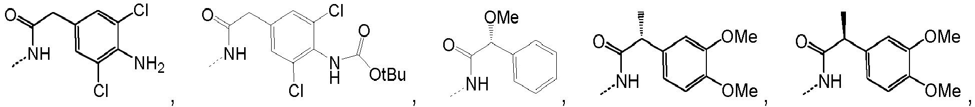 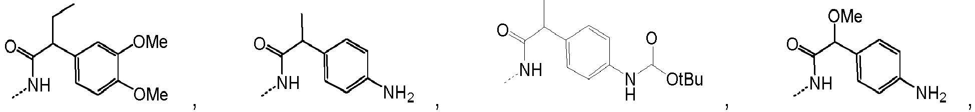 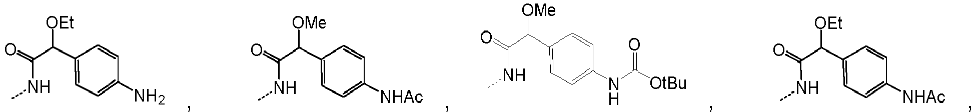 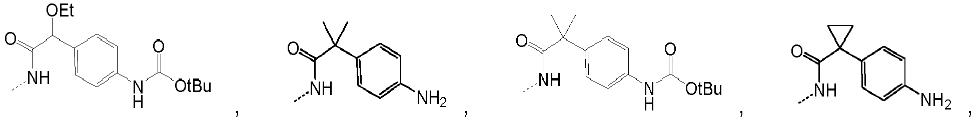 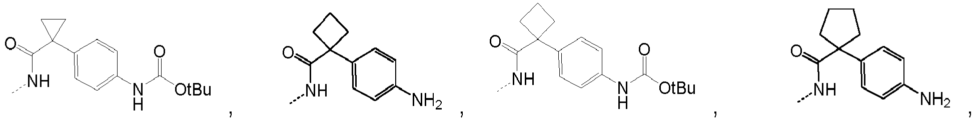 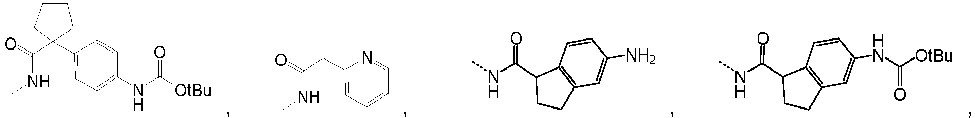 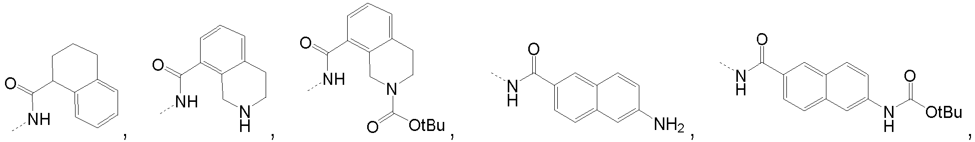 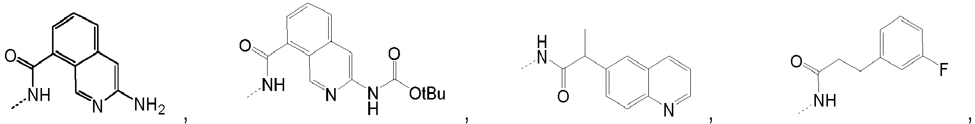 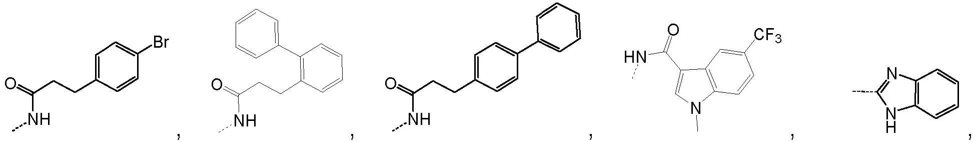 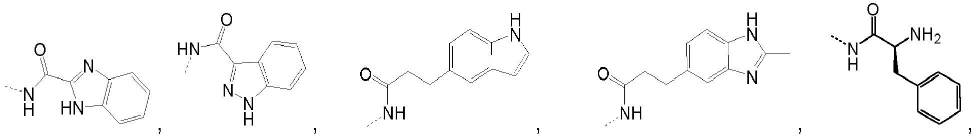 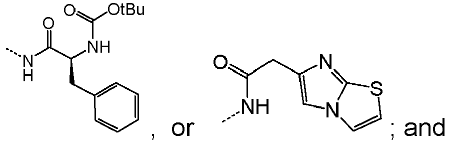
**R¹** represents -CH₃, 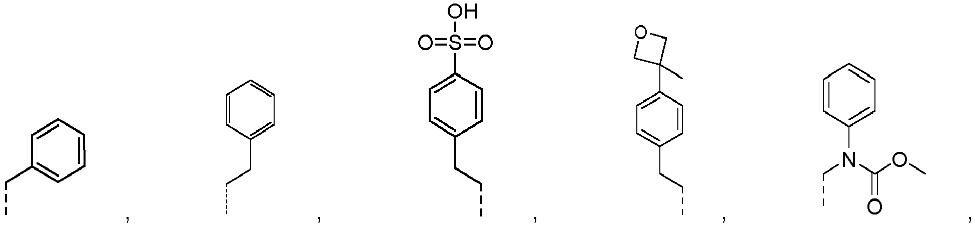 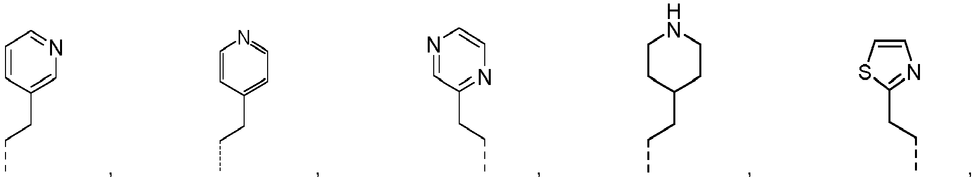 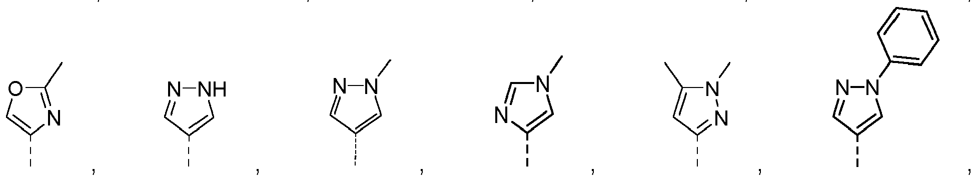 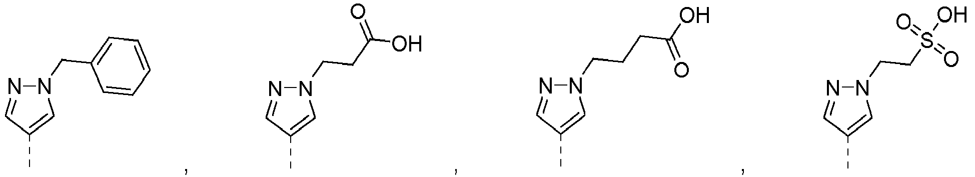 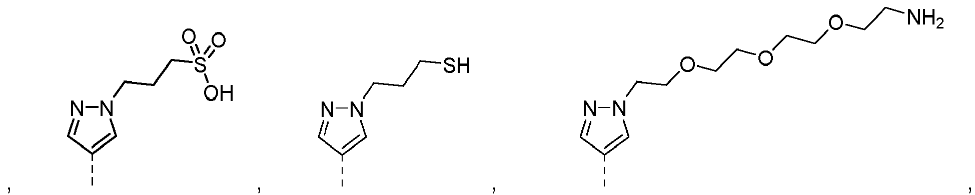 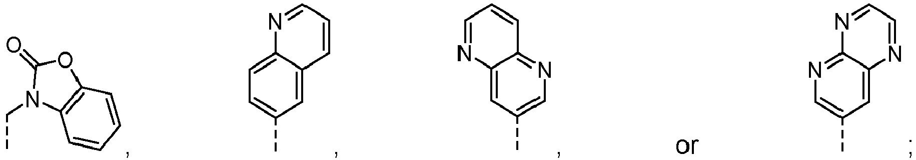
**R³ -R⁶** represent independently of each other -H, -CH₃, -OCH₃, -F, or -CI; or **R ⁵** and **R⁶** form

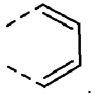

More preferred is the compound of the formula (I) 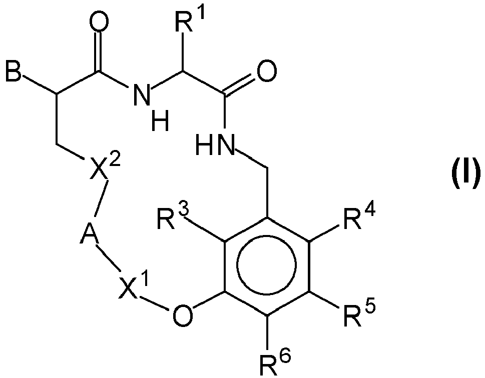 wherein
**-X₂-A-X₁-** represents 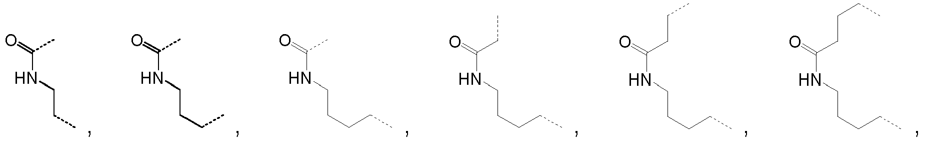 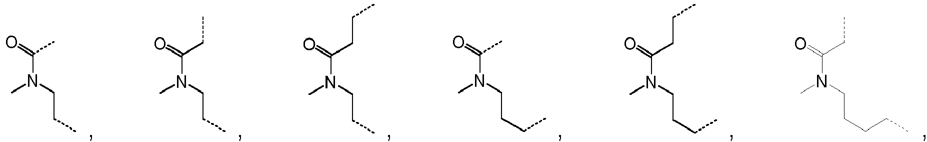 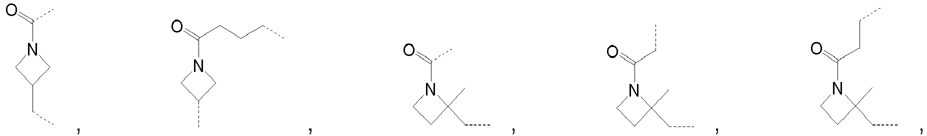 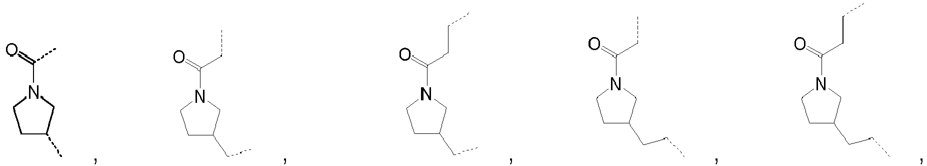 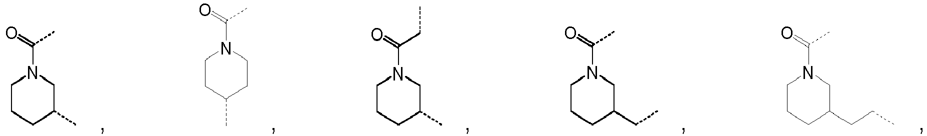 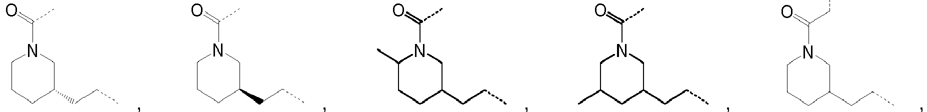 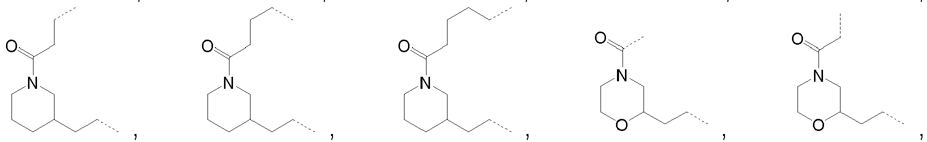 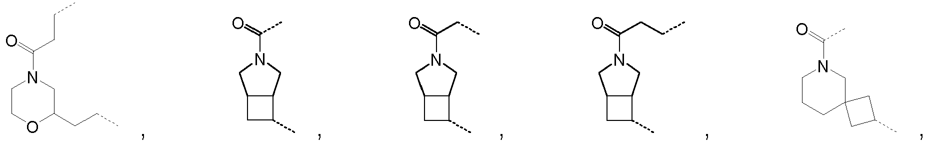 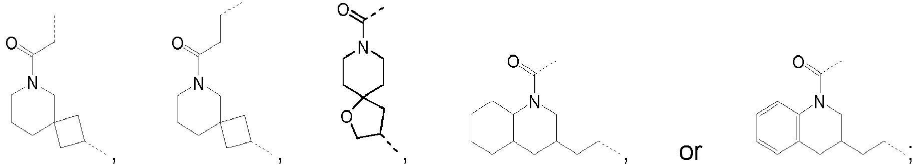
**B** represents -H, -NH₂, -NHCOCH₃, -NHCOC(CH₃)₃, -NHCOC(CN)(CH₃)₂, -NHCOCH=CH₂, -NHCOCH₂C(CH₃)₃, -NHCOPh, -NHCOCH₂Ph, -NHCOCH₂-NH(CH₃), -NHCOCH₂₋N(CH₃)CO₂tBu, -NHCOC(CH₃)₂CH₂OH, -NHCOC(CH₃)₂CH₂SH, -NHCOC(CH₂CH₃)₂CH₂OH, -NHCOC(CH₃)(CH₂OH)₂, - NHCOCH₂OCH₂CH₂-NH(CH₃), -NHCOCH_{2O}CH₂CH₂-N(CH₃)CO₂tBu, -NHCO₂CH₂CH₃, -NHCO₂(CH₂CH₂O)₃CH₃, -NHCO₂(CH₂CH₂O)₅CH₃, -NHCO₂(CH₂CH₂O)₇CH₃, -NHCO₂CH₂Ph -NHCONHCH₂CH₃, -NHSO₂CH₃, -NHSO₂CH=CH₂, -NHSO₂CH₂Ph, -NHSO₂CH₂CH₂Ph, -NHSO₂CH₂CH₂CH₂Ph, 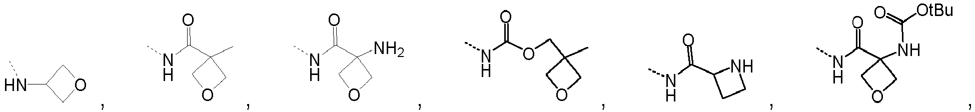 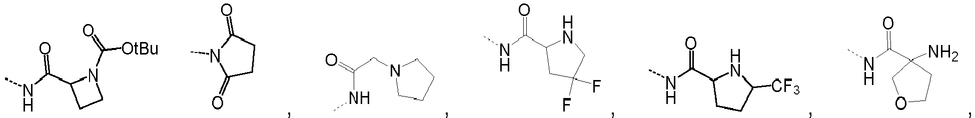 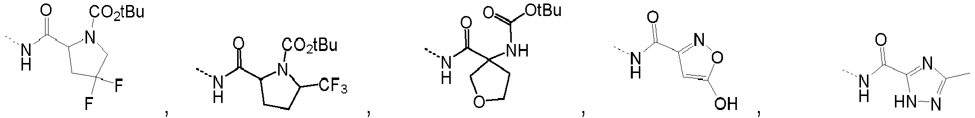 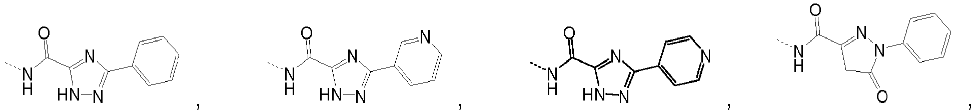 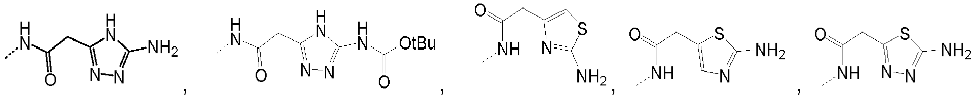 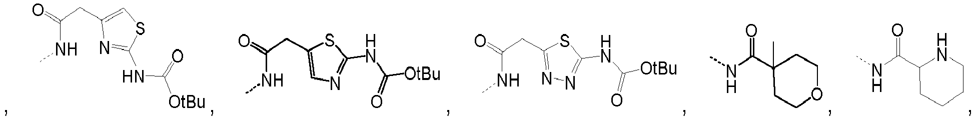 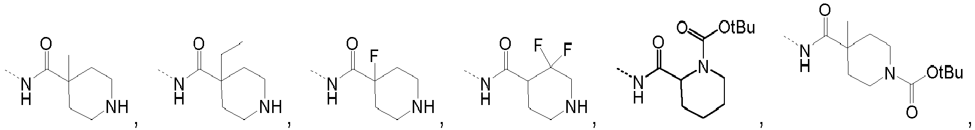 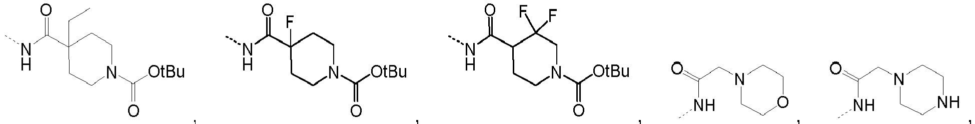 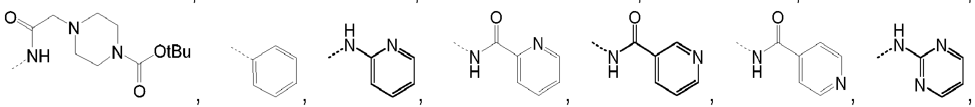 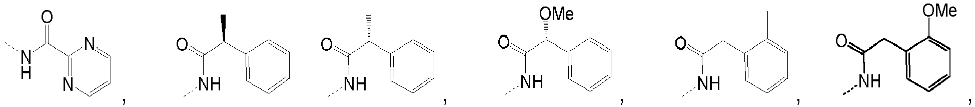 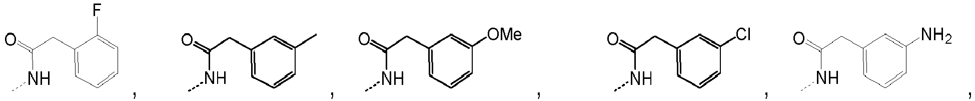 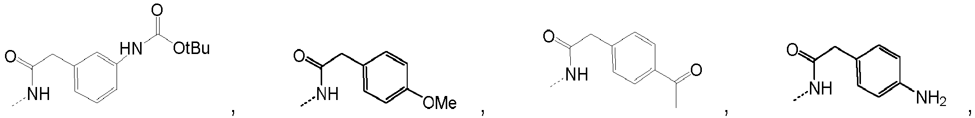 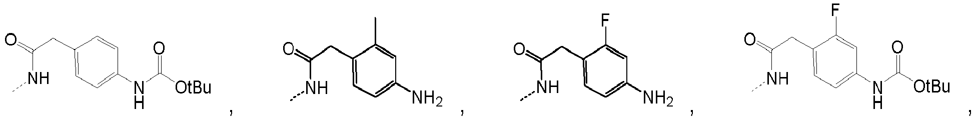 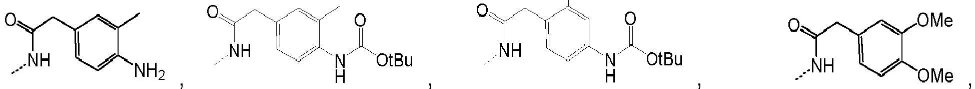 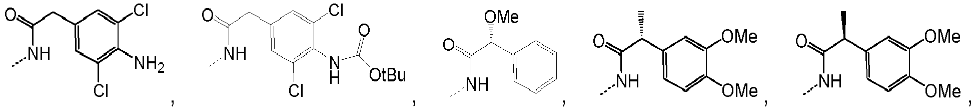 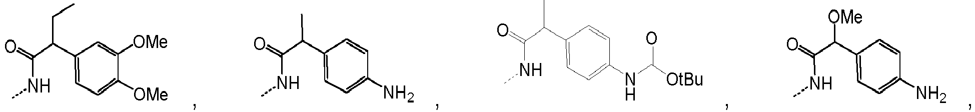 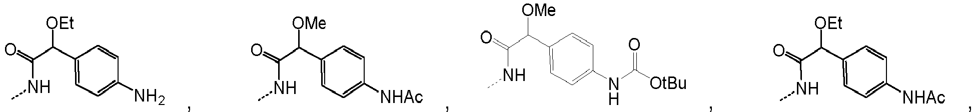 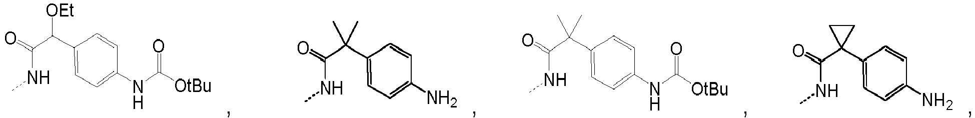 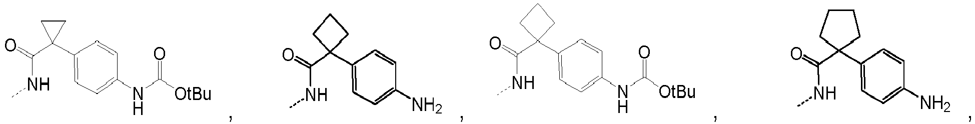 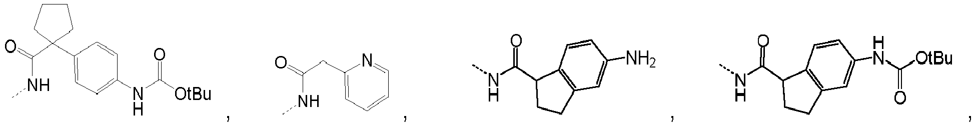 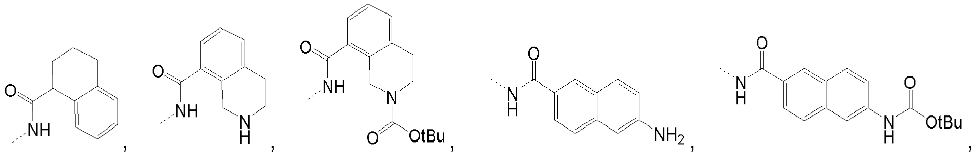 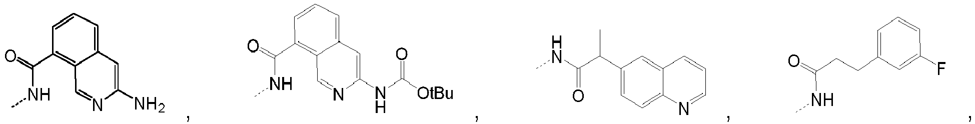 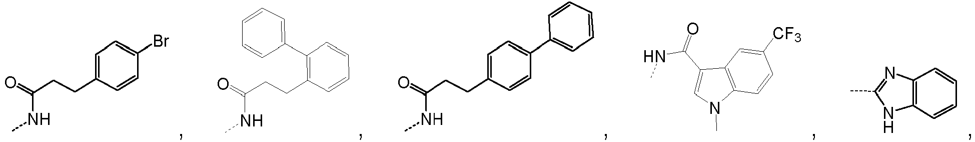 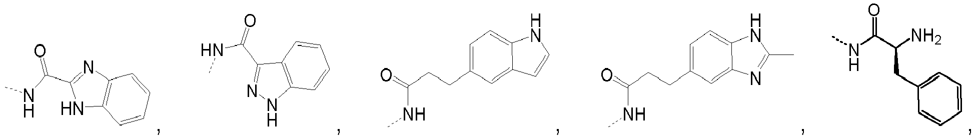 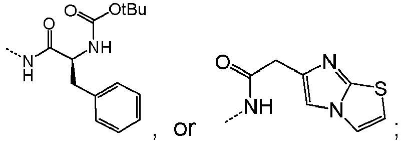 and
**R¹** represents -CH₃, 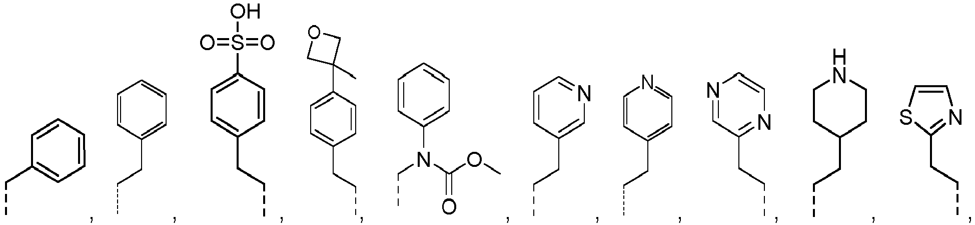 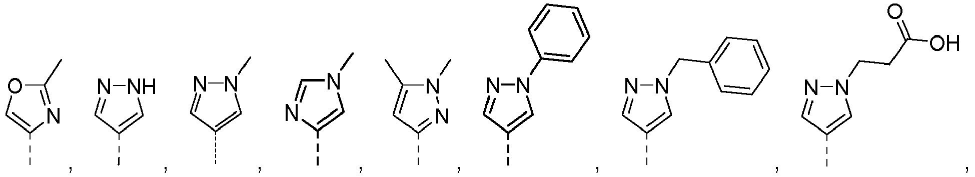 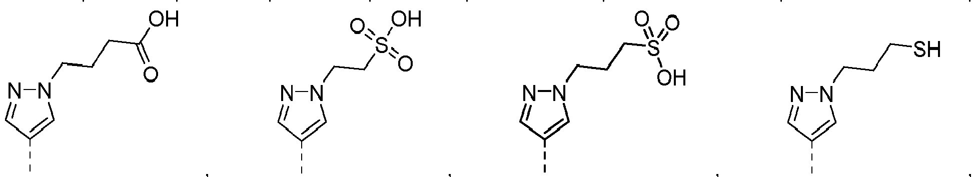 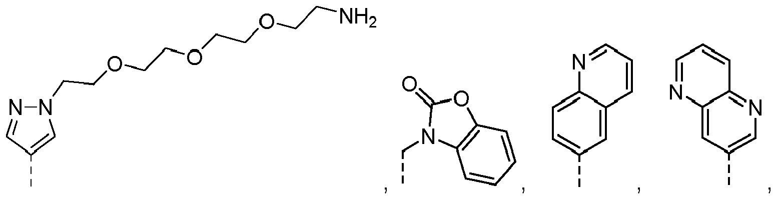 or 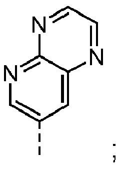
**R³ - R⁶** represent independently of each other -H, -CH₃, -OCH₃, -F, or -CI; or **R ⁵** and **R⁶** form

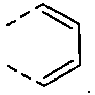

In some embodiments, the present invention relates to the compound having any one of the formulae (**II-1**) - (**II-16**): 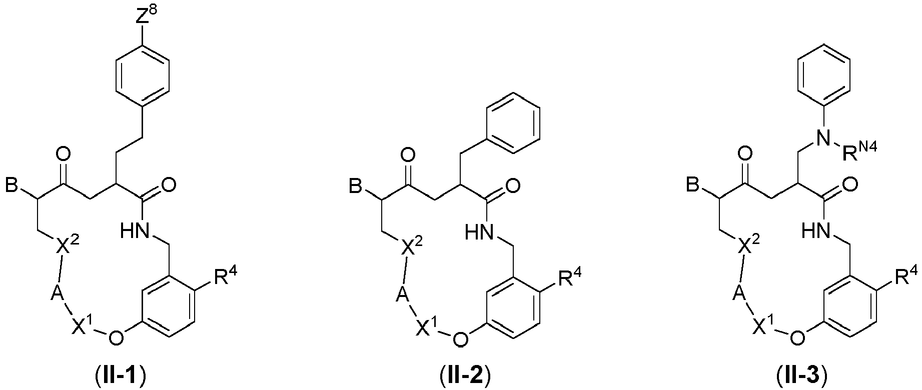 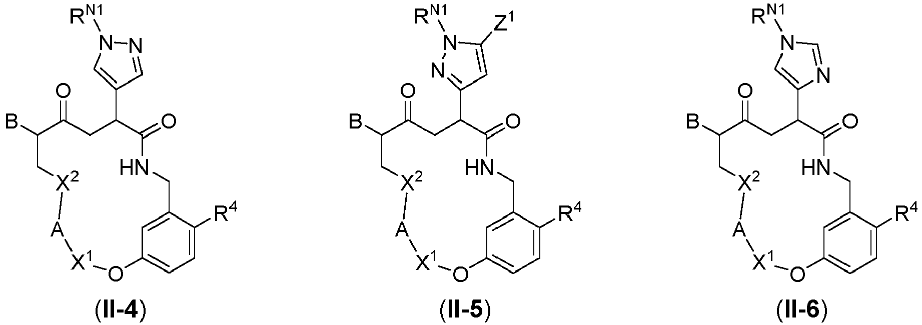 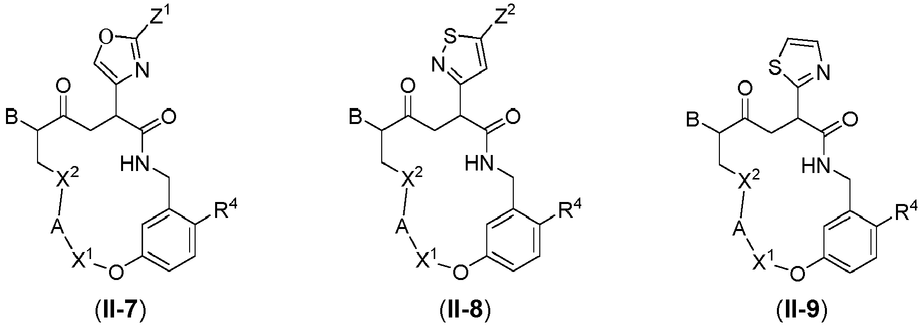 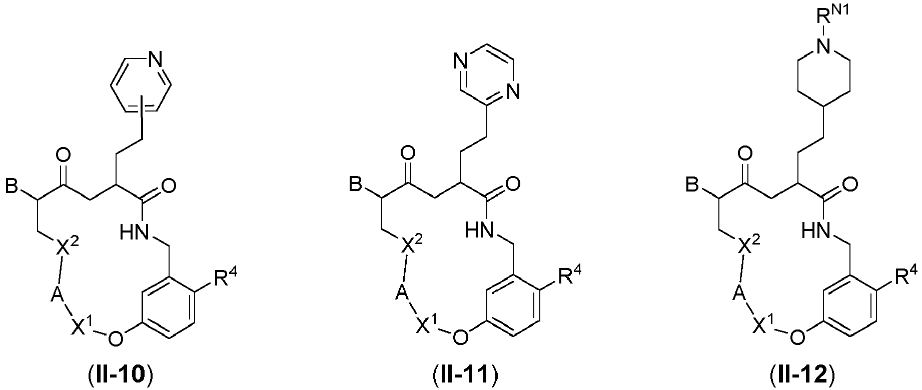 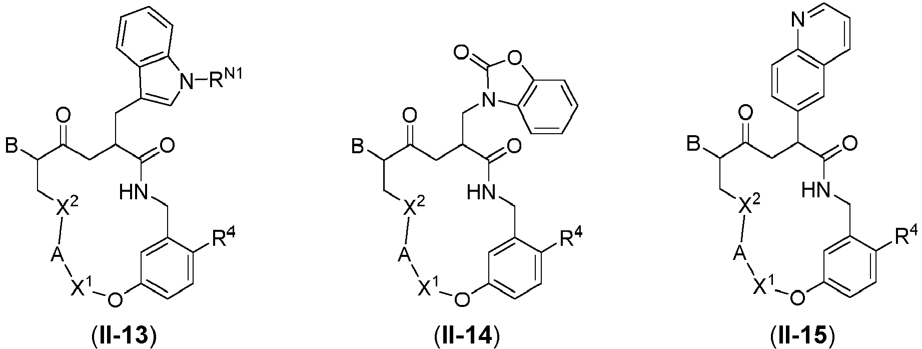 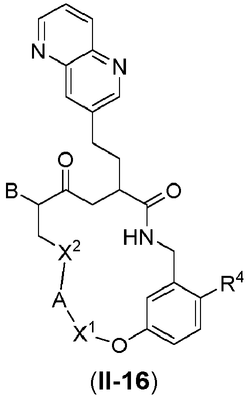 wherein A, B, R⁴, R^{N1}, R^{N4}, X¹, X², Z¹, Z², and Z⁸ have the same meanings as defined above.

In some embodiments, the present invention relates to the compound having any one of the formulae (**III-1**) - (**III-8**)**:** 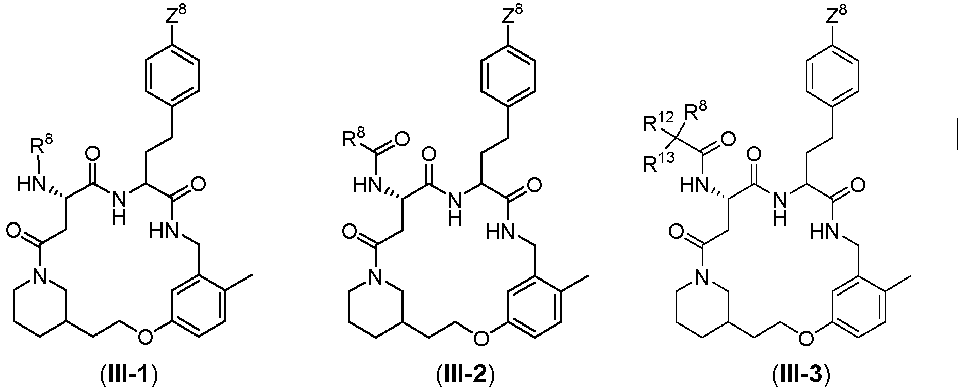 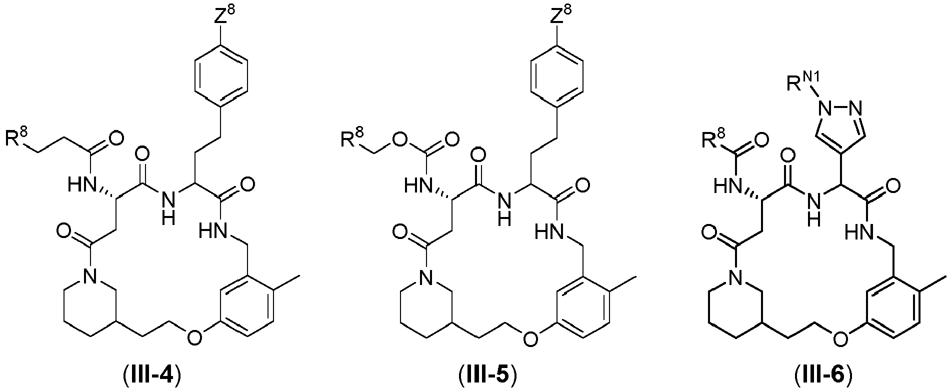 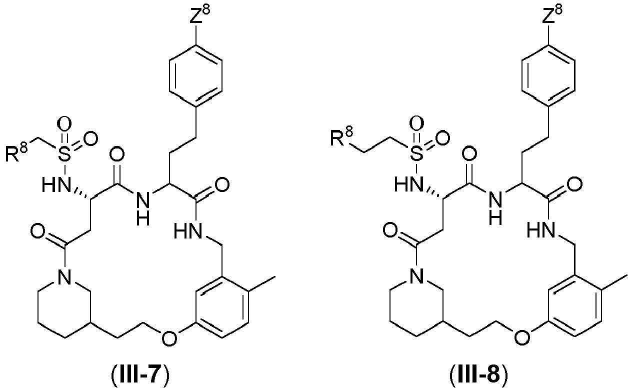 wherein R⁸, R¹², R¹³, R^{N1}, and Z⁸ have the same meanings as defined above.

Preferred are the compounds of any one of the formulae (**III-1**) to (**III-8**), wherein **R⁸** is

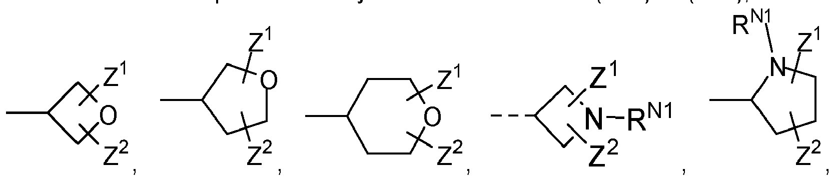

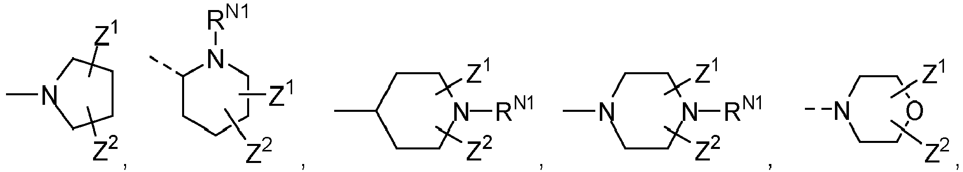

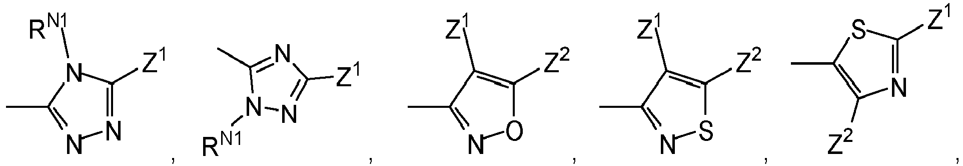

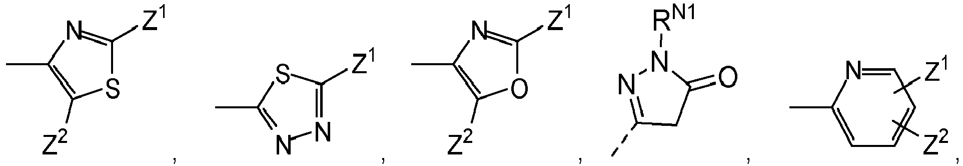

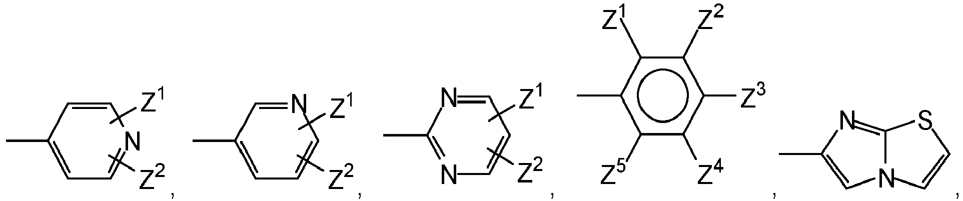

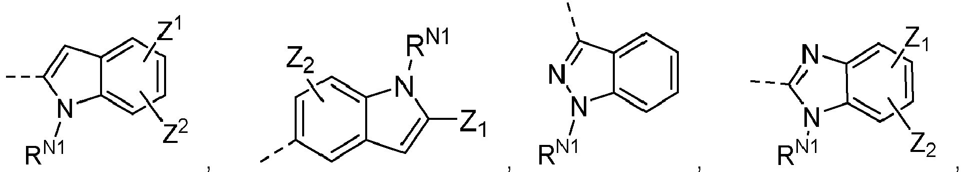

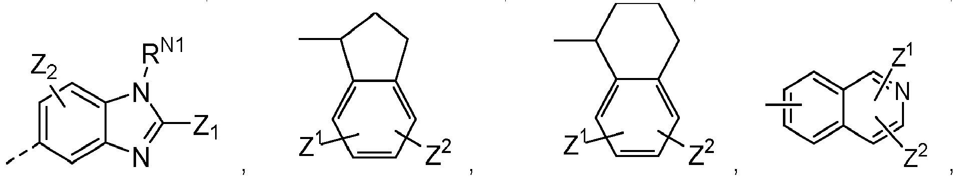

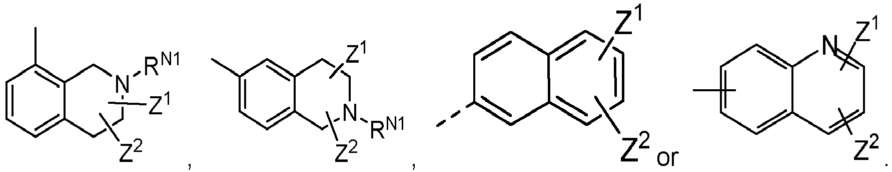

In some embodiments, the present invention relates to the compound having any one of the formulae (**IV-1**) - (**IV-4**): 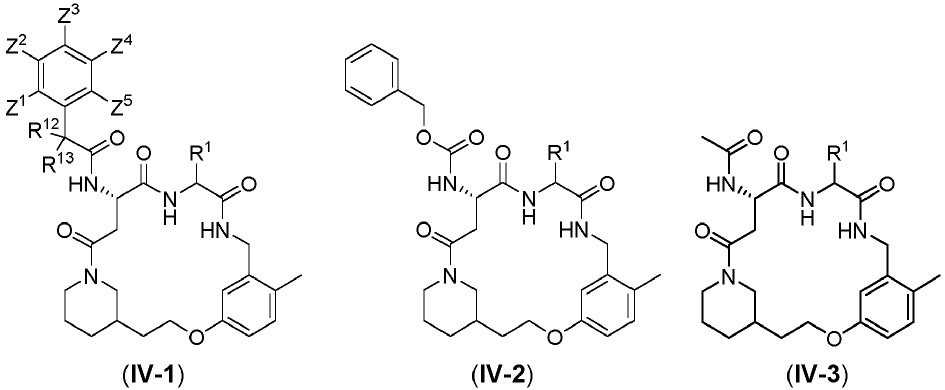 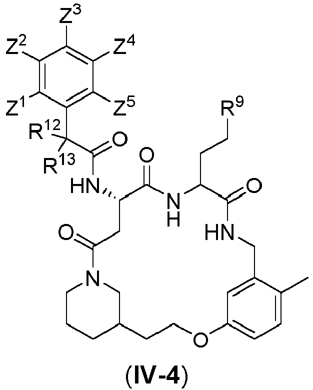 wherein R¹, R⁹, R¹², R¹³, Z¹, Z², Z³, Z⁴, and Z⁵ have the same meanings as defined above.

Preferred are the compounds of any one of the formulae (**IV-1**) to (**IV-3**), wherein
**R¹** represents -(CH₂)ₚ-**R⁹**, or -(CH₂)ₚ-**NR^{N4}-R⁹;**
p is an integer selected from 0, 1, 2, or 3;
and **R⁹** and **R^{N4}** have the same meaning as defined in the formula (**I**).

Mor preferred are the compounds of any one of the formulae (**IV-1**) to (**IV-4**), wherein **R⁹** is 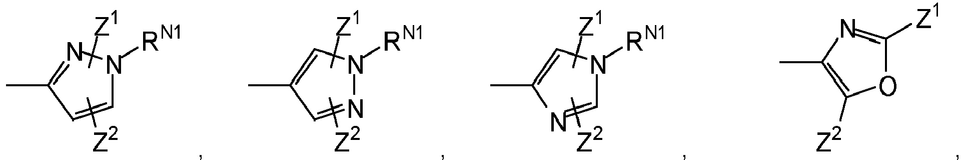 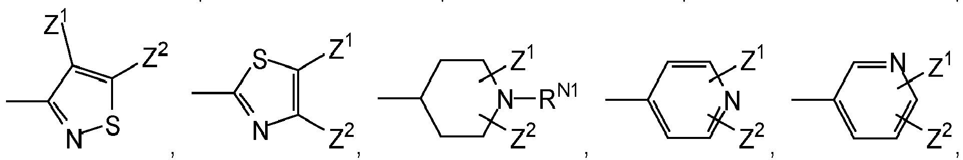 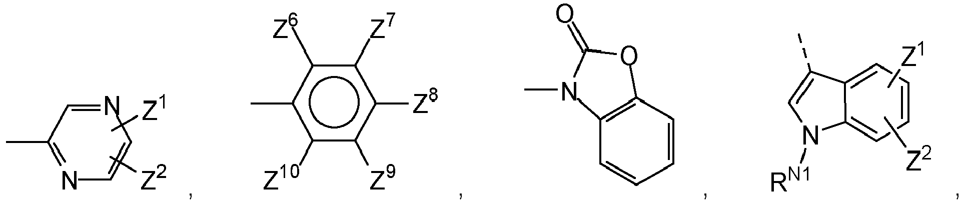 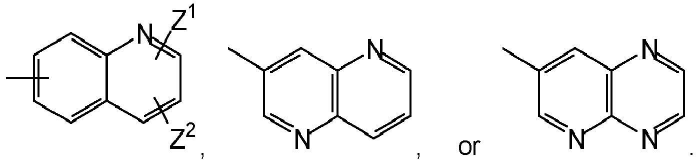 and **Z¹, Z², Z⁶, Z⁷, Z⁸, Z⁹, Z¹⁰** and **R^{N1}** have the same meaning as defined in the formula (**I**).

In some embodiments, the present invention relates to the compound having any one of the formulae (**V-1**) - (**V-9**): 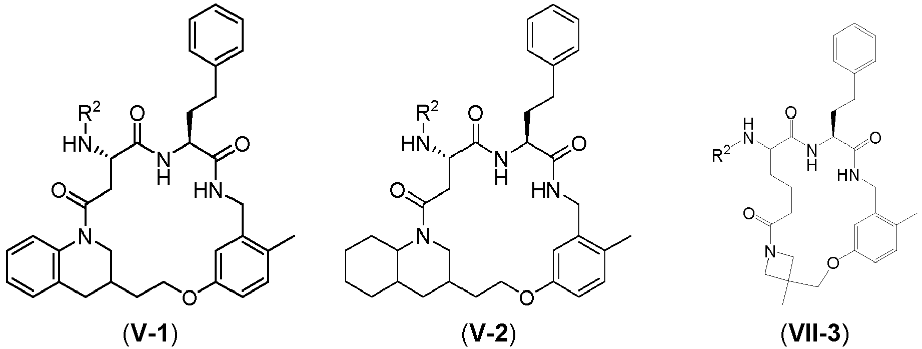 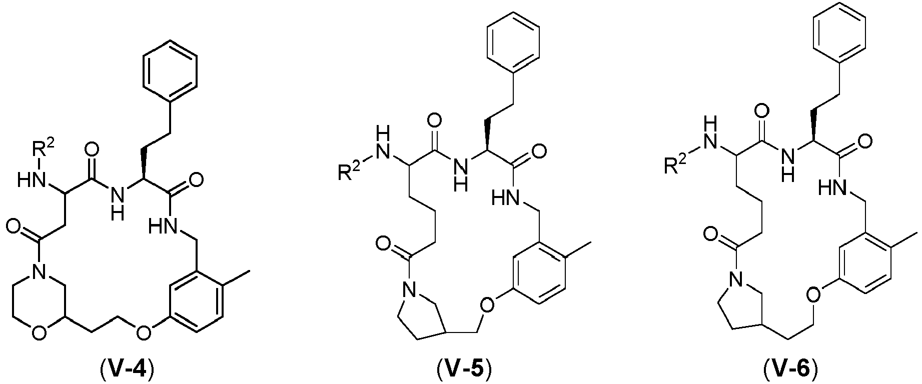 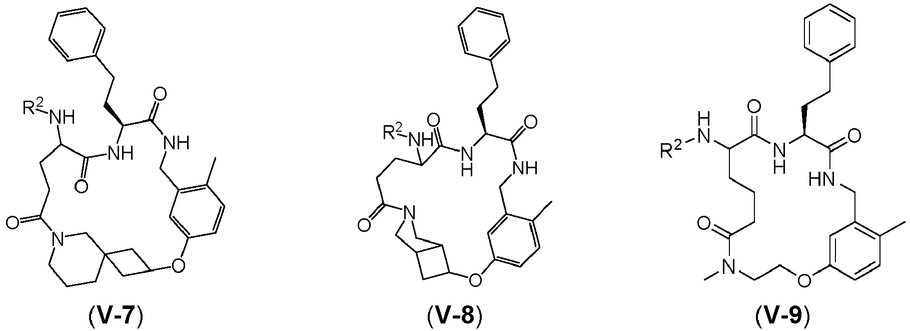 wherein R² has the same meaning as defined above.

Preferred are the compounds of any one of the formulae (**V-1**) to (**V-9**), wherein
**R²** represents -H, **-R⁸**, -CO-C(**R¹²**)(**R¹³**)-**R⁸**, or -CO-C(**R¹²**)(**R¹³**)-(CH₂)-**R⁸;**
**R⁸** represents 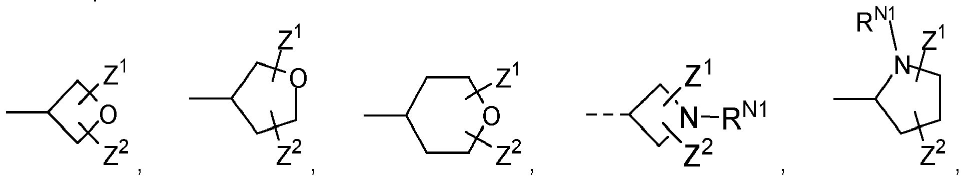 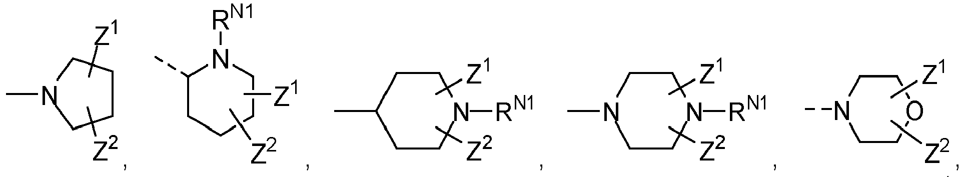 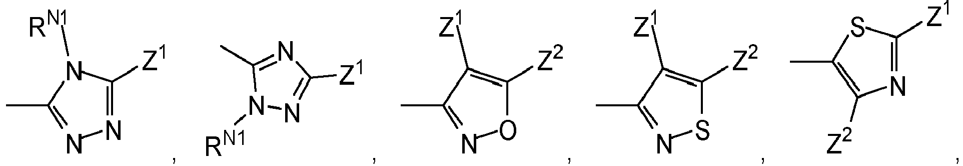 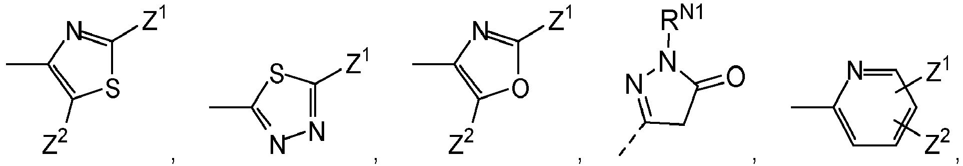 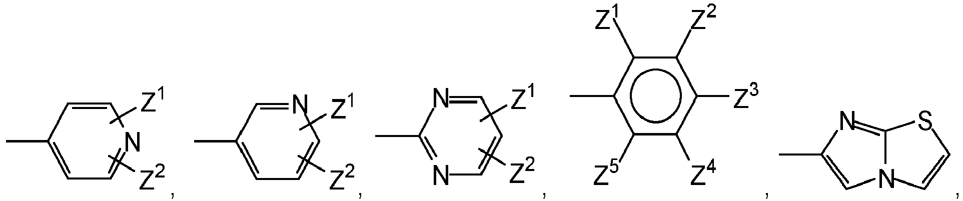 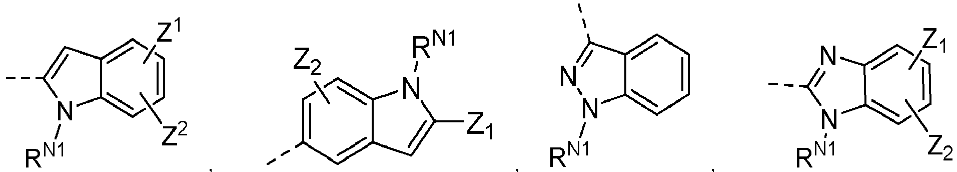 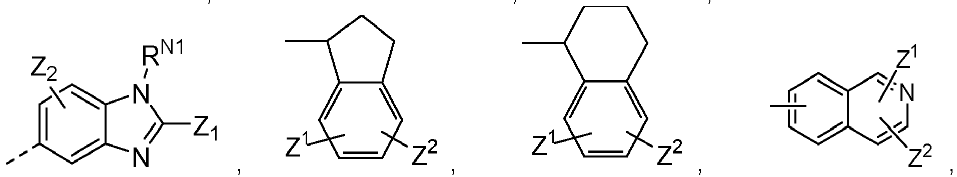 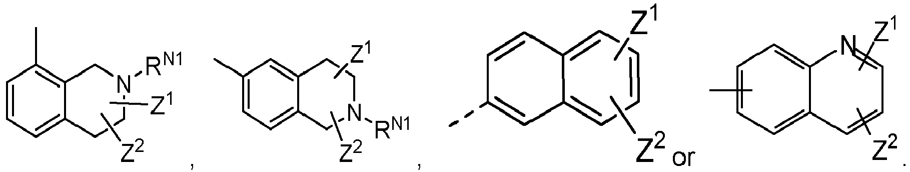
and **R¹²** and **R¹³** have the same meanings as defined above.

Preferred are the compound of formual (**I**), (**II-1**) - (**II-16**), wherein **A** represents -CO-NH-, -CO-N(CH₃)-,

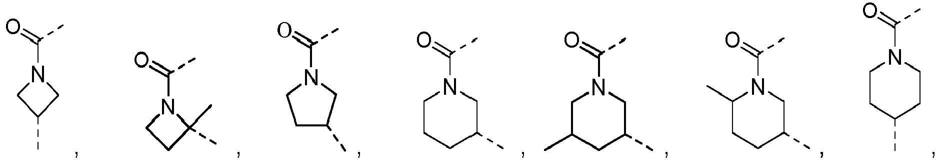

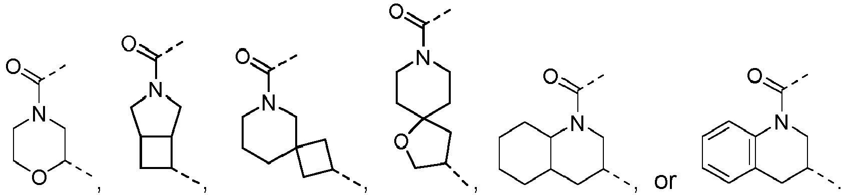

The compounds of any one of the formulae **(III-1)** - (**III-8**), (**IV-1**) - (**IV-4**), and (**V-1**) - (**IV-9**) contain also these moieties as **A**.

Especially preferred compounds according to the present invention include compounds presented by Table 1.

**Table 1:**

| **Cpd** | **structure** | **IUPAC Name** |
|---|---|---|
| **7** | 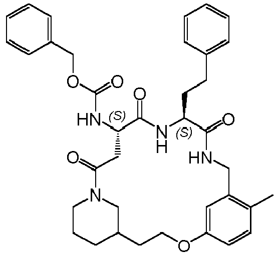 | benzyl ((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamate |
| **8** | 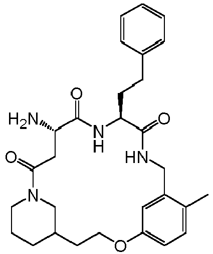 | (9S,12S)-12-amino-5⁴-methyl-9-phenethyl-4-oxa-7,10-diaza-1(3,1)-piperidina-5(1,3)-benzenacyclotetradecaphane-8,11,14-trione |
| **13** | 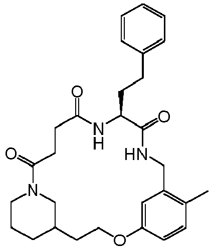 | (9S)-5⁴-methyl-9-phenethyl-4-oxa-7,10-diaza-1(3,1)-piperidina-5(1,3)-benzenacyclotetradecaphane-8,11,14-trione |
| **16** | 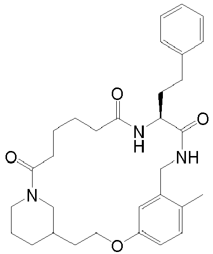 | (9S)-5⁴-methyl-9-phenethyl-4-oxa-7,10-diaza-1(3,1)-piperidina-5(1,3)-benzenacyclohexadecaphane-8,11,16-trione |
| **19** | 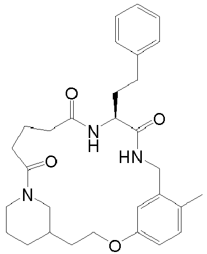 | (9S)-5⁴-methyl-9-phenethyl-4-oxa-7,10-diaza-1(3,1)-piperidina-5(1,3)-benzenacyclopentadecaphane-8,11,15-trione |
| **22** | 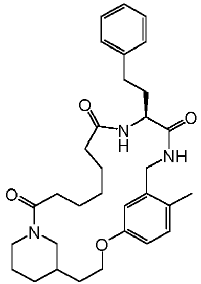 | (9S)-5⁴-methyl-9-phenethyl-4-oxa-7,10-diaza-1(3,1)-piperidina-5(1,3)-benzenacycloheptadecaphane-8,11,17-trione |
| **25** | 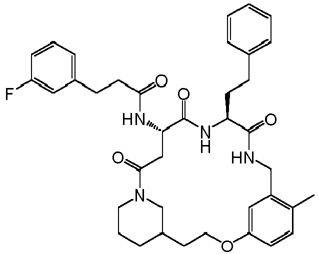 | 3-(3-fluorophenyl)-N-((9S,12S)-5 ⁴-methyl-8,11,14-trioxo-9-phenethyl-4-oxa-7,10-diaza-1(3,1)-piperidina-5(1,3)-benzenacyclotetradecaphane-12-yl)propanamide |
| **26** | 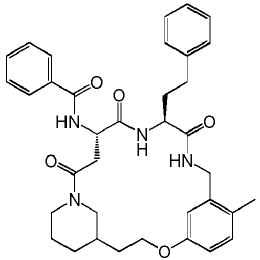 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)benzamide |
| **27** | 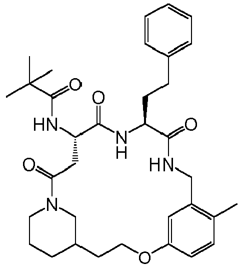 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)pivalamide |
| **28** | 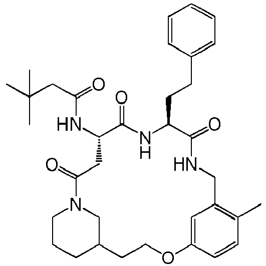 | 3,3-dimethyl-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)butanamide |
| **29** | 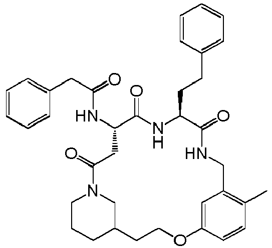 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-phenylacetamide |
| **30** | 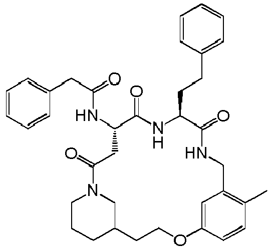 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-phenylacetamide |
| **31** | 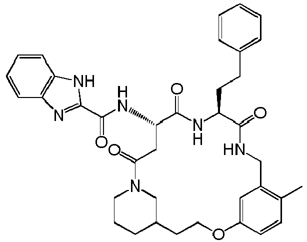 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-1H-benzo[d]imidazole-2-carboxamide |
| **32** | 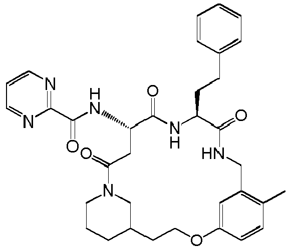 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)pyrimidine-2-carboxamide |
| **33** | 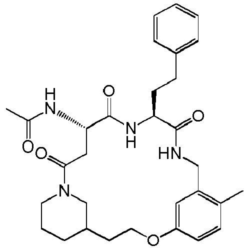 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **34** | 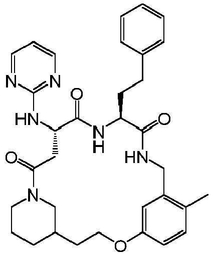 | (9S,12S)-5⁴-methyl-9-phenethyl-12-(pyrimidin-2-ylamino)-4-oxa-7,10-diaza-1(3,1)-piperidina-5(1,3)-benzenacyclotetradecaphane-8,11,14-trione |
| **35** | 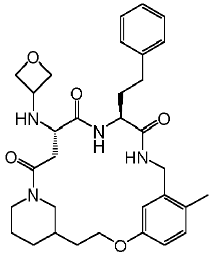 | (9S,12S)-5⁴-methyl-12-(oxetan-3-ylamino)-9-phenethyl-4-oxa-7,10-diaza-1(3,1)-piperidina-5(1,3)-benzenacyclotetradecaphane-8,11,14-trione |
| **36** | 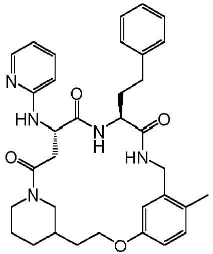 | (9S,12S)-5⁴-methyl-9-phenethyl-12-(pyridin-2-ylamino)-4-oxa-7,10-diaza-1(3,1)-piperidina-5(1,3)-benzenacyclotetradecaphane-8,11,14-trione |
| **37** | 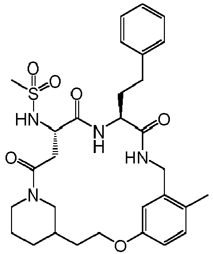 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)methanesulfonamide |
| **38** | 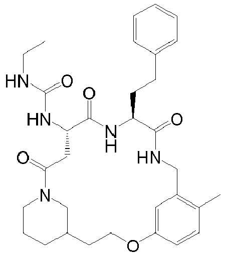 | 1-ethyl-3-((9S,12S)-5⁴-methyl-8,11,14-trioxo-9-phenethyl-4-oxa-7,10-diaza-1(3,1)-piperidina-5(1,3)-benzenacyclotetradecaphane-12-yl)urea |
| **39** | 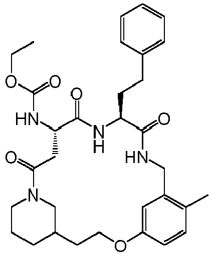 | ethyl ((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamate |
| **40** | 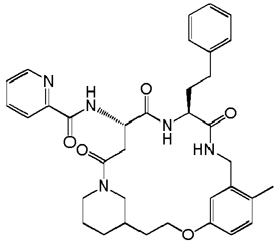 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)picolinamide |
| **41** | 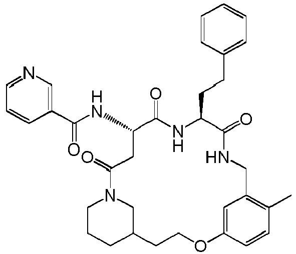 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)nicotinamide |
| **42** | 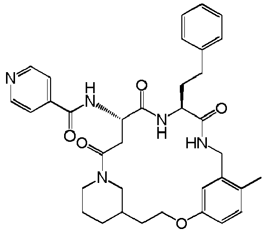 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)isonicotinamide |
| **43** | 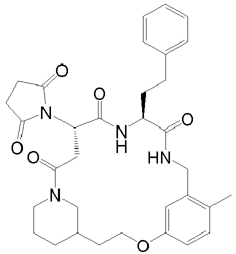 | (9S,12S)-12-(2,5-dioxopyrrolidin-1-yl)-5⁴-methyl-9-phenethyl-4-oxa-7,10-diaza-1(3,1)-piperidina-5(1,3)-benzenacyclotetradecaphane-8,11,14-trione |
| **44** | 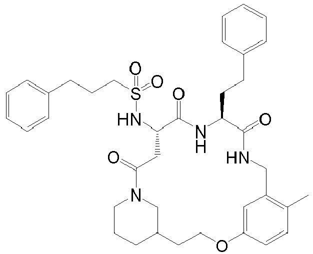 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-3-phenylpropane-1-sulfonamide |
| **45** | 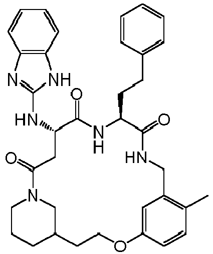 | (9S,12S)-12-((1H-benzo[d]imidazol-2-yl)amino)-5⁴-methyl-9-phenethyl-4-oxa-7,10-diaza-1(3,1)-piperidina-5(1,3)-benzenacyclotetradecaphane-8,11,14-trione |
| **51** | 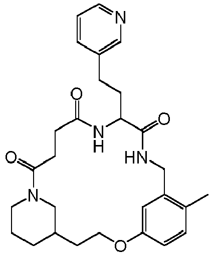 | 5⁴-methyl-9-(2-(pyridin-3-yl)ethyl)-4-oxa-7,10-diaza-1(3,1)-piperidina-5(1,3)-benzenacyclotetradecaphane-8,11,14-trione |
| **59** | 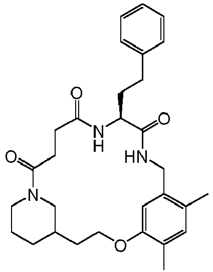 | (9R)-5⁴,5⁶-dimethyl-9-phenethyl-4-oxa-7,10-diaza-1(3,1)-piperidina-5(1,3)-benzenacyclotetradecaphane-8,11,14-trione |
| **60** | 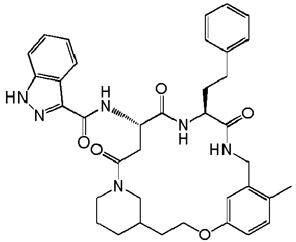 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-1H-indazole-3-carboxam ide |
| **61** | 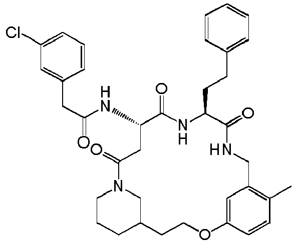 | 2-(3-chlorophenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **62** | 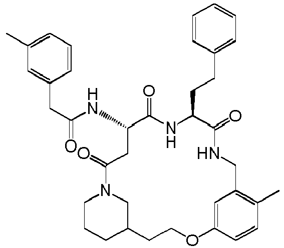 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-(m-tolyl)acetamide |
| **63** | 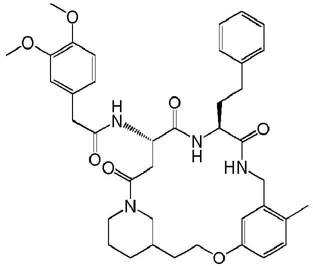 | 2-(3,4-dimethoxyphenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **64** | 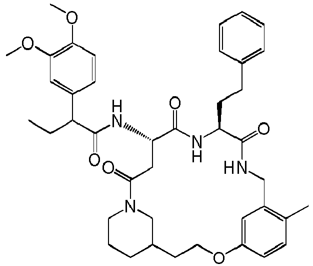 | 2-(3,4-dimethoxyphenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)butanamide |
| **77** | 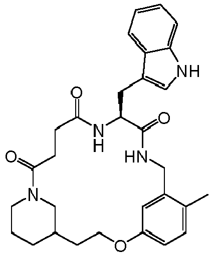 | (9S)-9-((1H-indol-3-yl)methyl)-5⁴-methyl-4-oxa-7,10-diaza-1(3,1)-piperidina-5(1,3)-benzenacyclotetradecaphane-8,11,14-trione |
| **78** | 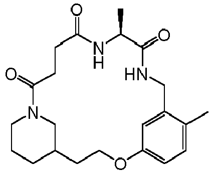 | (9S)-5⁴,9-dimethyl-4-oxa-7,10-diaza-1(3,1)-piperidina-5(1,3)-benzenacyclotetradecaphane-8,11,14-trione |
| **79** | 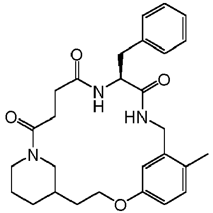 | (9S)-9-benzyl-5⁴-methyl-4-oxa-7,10-diaza-1(3,1)-piperidina-5(1,3)-benzenacyclotetradecaphane-8,11,14-trione |
| **80** | 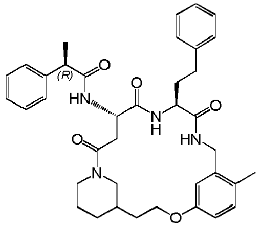 | (2R)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-phenylpropanamide |
| **81** | 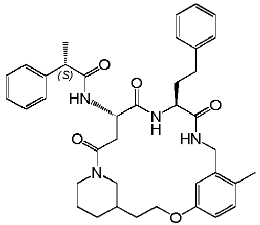 | (2S)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-phenylpropanamide |
| **82** | 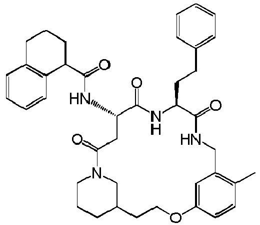 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-1,2,3,4-tetrahydronaphthalene-1-carboxam ide |
| **83** | 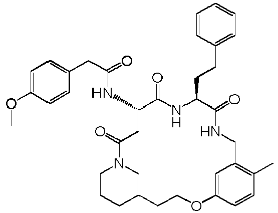 | 2-(4-methoxyphenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **84** | 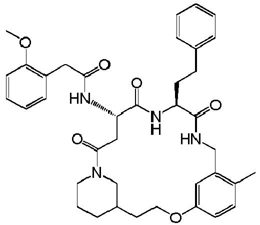 | 2-(2-methoxyphenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **85** | 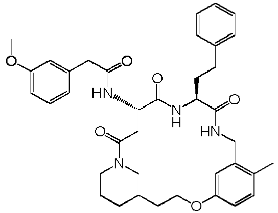 | 2-(3-methoxyphenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **86** | 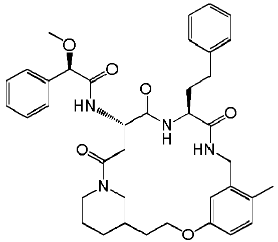 | (2R)-2-methoxy-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-phenylacetamide |
| **87** | 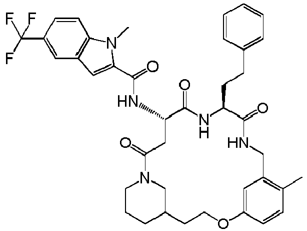 | 1-methyl-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-5-(trifluoromethyl)-1H-indole-2-carboxam ide |
| **88** | 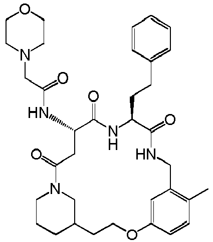 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-morpholinoacetamide |
| **89** | 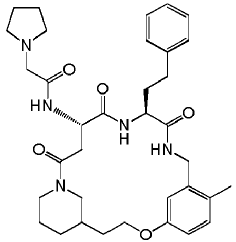 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-(pyrrolidin-1-yl)acetamide |
| **95** | 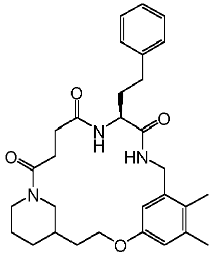 | (9S)-5⁴,5⁵-dimethyl-9-phenethyl-4-oxa-7,10-diaza-1(3,1)-piperidina-5(1,3)-benzenacyclotetradecaphane-8,11,14-trione (95) |
| **105** | 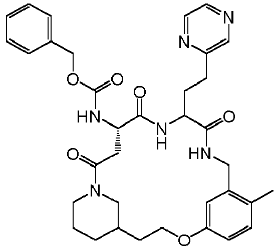 | benzyl ((12S)-5⁴-methyl-8,11, 14-trioxo-9-(2-(pyrazin-2-yl)ethyl)-4-oxa-7,10-diaza-1(3,1)-piperidina-5(1,3)-benzenacyclotetradecaphane-12-yl)carbamate |
| **106** | 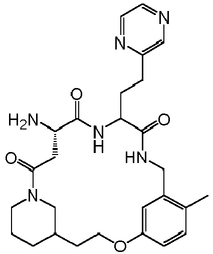 | (12S)-amino-5⁴-methyl-8,11,14-trioxo-9-(2-(pyrazin-2-yl)ethyl)-4-oxa-7,10-diaza-1(3,1)-piperidina-5(1,3)-benzenacyclotetradecaphane |
| **107** | 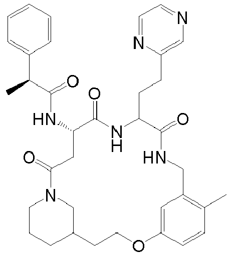 | (2S)-N-((12S)-5⁴-methyl-8,11,14-trioxo-9-(2-(pyrazin-2-yl)ethyl)-4-oxa-7,10-diaza-1(3,1)-piperidina-5(1,3)-benzenacyclotetradecaphane-12-yl)-2-phenylpropanamide |
| **116** | 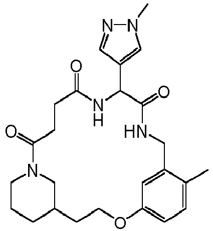 | 5⁴-methyl-9-(1-methyl-1H-pyrazol-4-yl)-4-oxa-7,10-diaza-1(3,1)-piperidina-5(1,3)-benzenacyclotetradecaphane-8,11,14-trione |
| **120** | 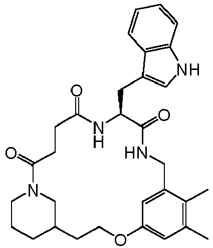 | (9S)-9-((1H-indol-3-yl)methyl)-5⁴,5⁵-dimethyl-4-oxa-7,10-diaza-1(3,1)-piperidina-5(1,3)-benzenacyclotetradecaphane-8,11,14-trione |
| **121** | 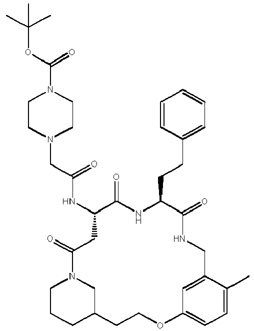 | tert-butyl 4-(2-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)amino)-2-oxoethyl)piperazine-1-carboxylate |
| **122** | 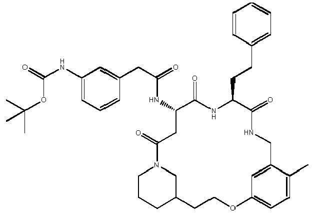 | tert-butyl (3-(2-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)amino)-2-oxoethyl)phenyl)carbamate |
| **123** | 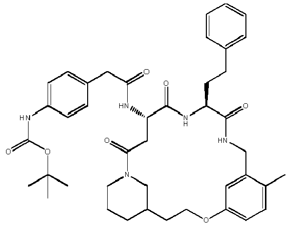 | tert-butyl (4-(2-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)amino)-2-oxoethyl)phenyl)carbamate |
| **124** | 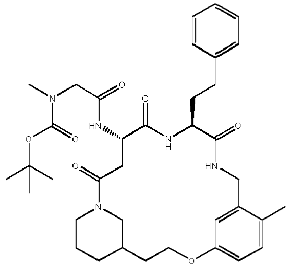 | tert-butyl methyl(2-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)amino)-2-oxoethyl)carbamate |
| **125** | 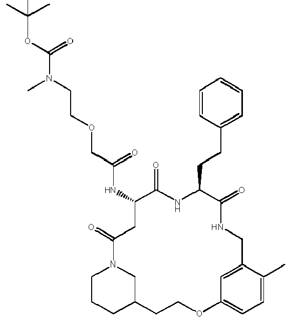 | tert-butyl methyl(2-(2-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)amino)-2-oxoethoxy)ethyl)carbamate |
| **126** | 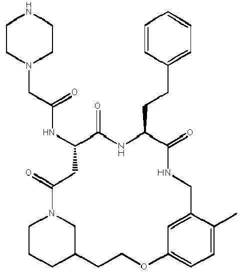 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-(piperazin-1-yl)acetamide |
| **127** | 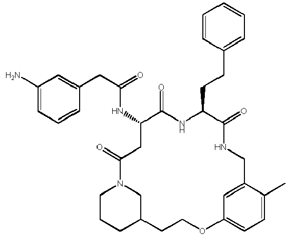 | 2-(3-aminophenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **128** | 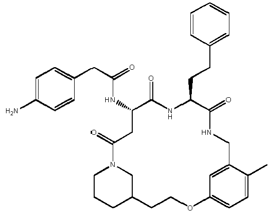 | 2-(4-aminophenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **129** | 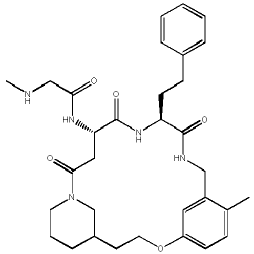 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-(methylamino)acetamide |
| **130** | 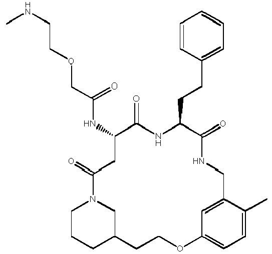 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-(2-(methylamino)ethoxy)acetamide |
| **131** | 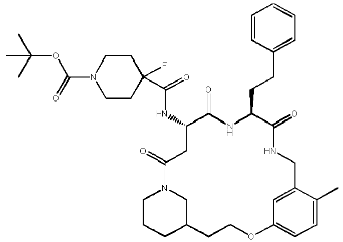 | tert-butyl 4-fluoro-4-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)piperidine-1-carboxylate |
| **132** | 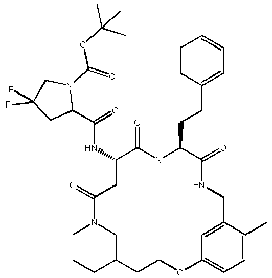 | tert-butyl 4,4-difluoro-2-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)pyrrolidine-1-carboxylate |
| **133** | 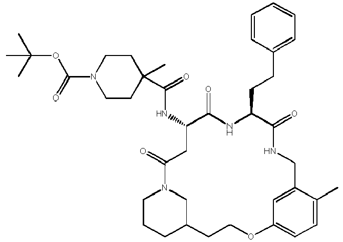 | tert-butyl 4-methyl-4-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)piperidine-1-carboxylate |
| **134** | 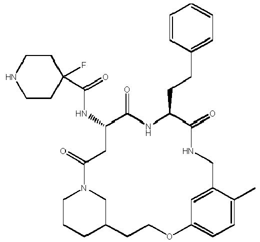 | 4-fluoro-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)piperidine-4-carboxamide |
| **135** | 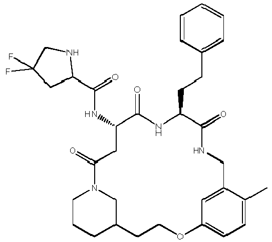 | (2S)-4,4-difluoro-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)pyrrolidine-2-carboxamide |
| **136** | 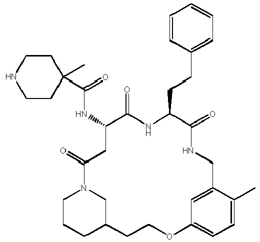 | 4-methyl-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)piperidine-4-carboxamide |
| **143** | 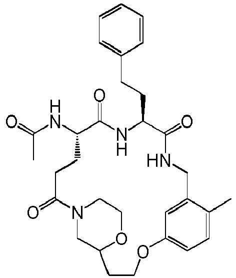 | N-((9S,12S)-5⁴-methyl-8,11,15-trioxo-9-phenethyl-4-oxa-7,10-diaza-1(2,4)-morpholina-5(1,3)-benzenacyclopentadecaphane-12-yl)acetamide |
| **148** | 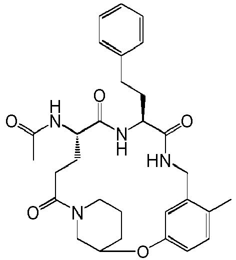 | N-((7S,10S)-3⁴-methyl-6,9,13-trioxo-7-phenethyl-2-oxa-5,8-diaza-1(3,1)-piperidina-3(1,3)-benzenacyclotridecaphane-10-yl)acetamide |
| **149** | 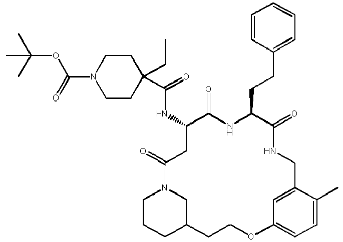 | tert-butyl 4-ethyl-4-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)piperidine-1-carboxylate |
| **150** | 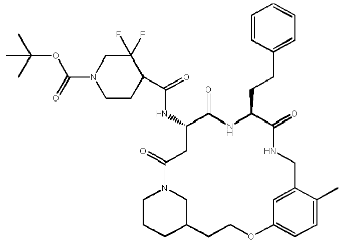 | tert-butyl 3,3-difluoro-4-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)piperidine-1-carboxylate |
| **151** | 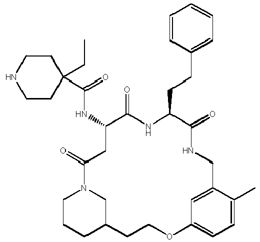 | 4-ethyl-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)piperidine-4-carboxamide |
| **152** | 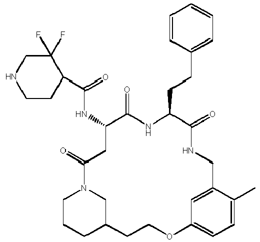 | 3,3-difluoro-N-((9S,12S)-5 ⁴-methyl-8,11,14-trioxo-9-phenethyl-4-oxa-7,10-diaza-1(3,1)-piperidina-5(1,3)-benzenacyclotetradecaphane-12-yl)piperidine-4-carboxamide |
| **153** | 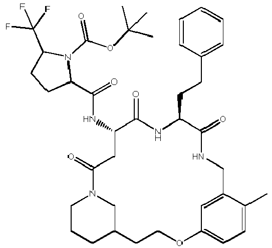 | tert-butyl 2-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)-5-(trifluoromethyl)pyrrolidine-1-carboxylate |
| **154** | 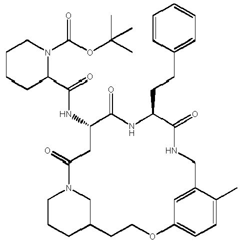 | tert-butyl 2-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)piperidine-1-carboxylate |
| **155** | 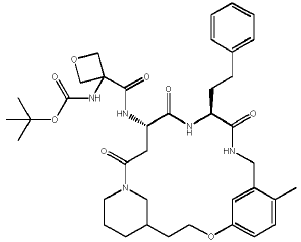 | tert-butyl (3-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)oxetan-3-yl)carbamate |
| **156** | 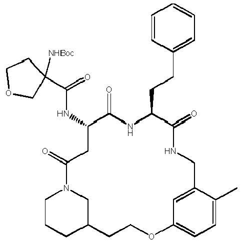 | tert-butyl (3-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)tetrahydrofuran-3-yl)carbamate |
| **157** | 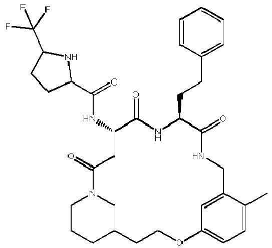 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-5-(trifluoromethyl)pyrrolidine-2-carboxamide |
| **158** | 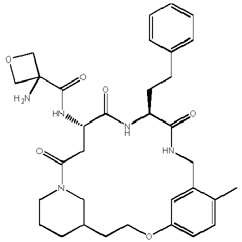 | 3-amino-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)oxetane-3-carboxamide |
| **159** | 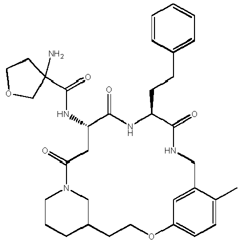 | 3-amino-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)tetrahydrofuran-3-carboxam ide |
| **160** | 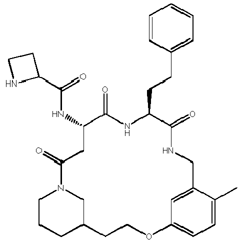 | (2R)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)azetidine-2-carboxamide |
| **161** | 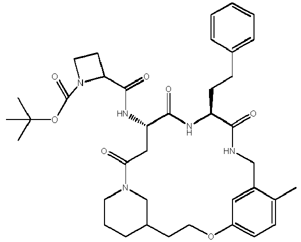 | tert-butyl 2-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)azetidine-1-carboxylate |
| **168** | 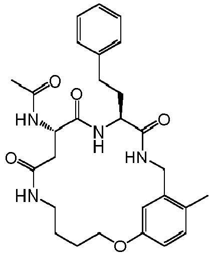 | N-((10S,13S)-1⁴-methyl-8,11,14-trioxo-13-phenethyl-2-oxa-7,12,15-triaza-1(1,3)-benzenacyclohexadecaphane-10-yl)acetamide |
| **171** | 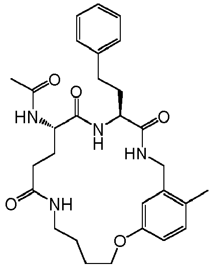 | N-((11S,14S)-1⁴-methyl-8,12,15-trioxo-14-phenethyl-2-oxa-7,13,16-triaza-1(1,3)-benzenacycloheptadecaphane-11-yl)acetamide |
| **172** | 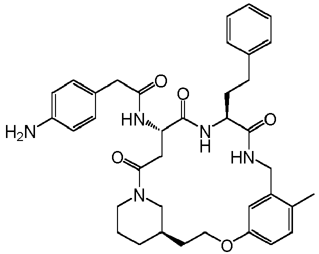 | 2-(4-aminophenyl)-N-((13R,9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **173** | 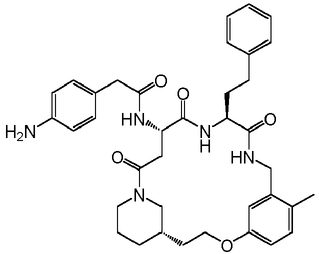 | 2-(4-aminophenyl)-N-((13S,9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **174** | 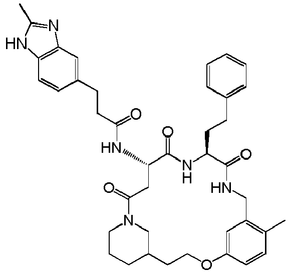 | 3-(2-methyl-1H-benzo[d]imidazol-6-yl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)propanamide |
| **175** | 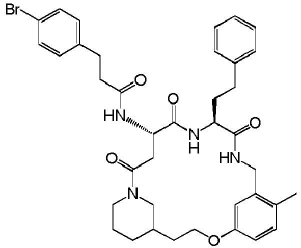 | 3-(4-bromophenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)propanamide |
| **176** | 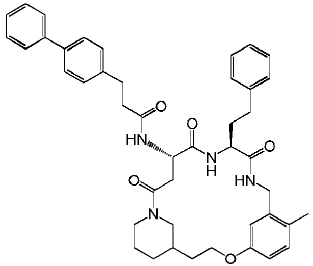 | 3-([1,1'-biphenyl]-4-yl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)propanamide |
| **177** | 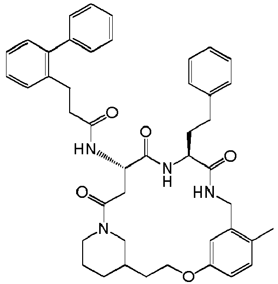 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-3-(2-phenoxyphenyl)propanam ide |
| **178** | 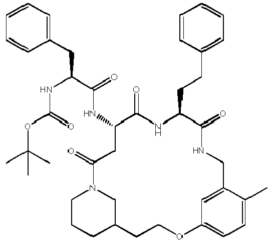 | tert-butyl ((2S)-1-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)amino)-1-oxo-3-phenylpropan-2-yl)carbamate |
| **179** | 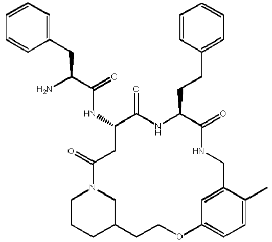 | (2R)-2-amino-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-3-phenylpropanamide |
| **180** | 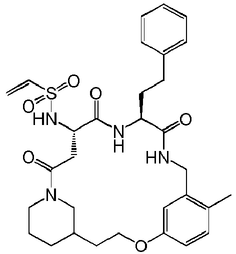 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)ethenesulfonamide |
| **181** | 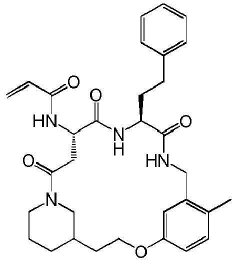 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acrylamide |
| **182** | 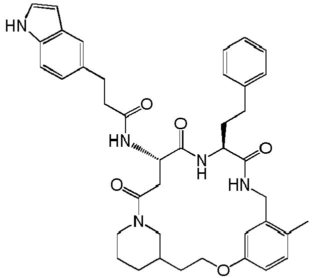 | 3-(1H-indol-5-yl)-N-((9S,12S)-5 ⁴-methyl-8,11,14-trioxo-9-phenethyl-4-oxa-7,10-diaza-1(3,1)-piperidina-5(1,3)-benzenacyclotetradecaphane-12-yl)propanamide |
| **186** | 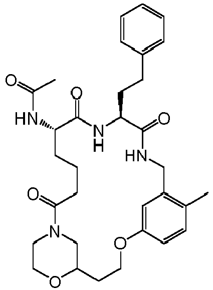 | N-((9S,12S)-5⁴-methyl-8,11,16-trioxo-9-phenethyl-4-oxa-7,10-diaza-1(2,4)-morpholina-5(1,3)-benzenacyclohexadecaphane-12-yl)acetamide |
| **191** | 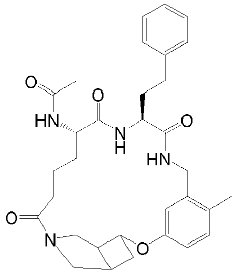 | N-((11R,15S,7S,10S)-3⁴-methyl-6,9,14-trioxo-7-phenethyl-2-oxa-13,5,8-triaza-1(7,3)-bicyclo[3.2.0]heptana-3(1,3)-benzenacyclotetradecaphane-10-yl)acetamide |
| **195** | 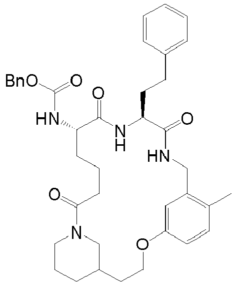 | benzyl ((9S,12S)-5⁴-methyl-8,11,16-trioxo-9-phenethyl-4-oxa-7,10-diaza-1(3,1)-piperidina-5(1,3)-benzenacyclohexadecaphane-12-yl)carbamate |
| **199** | 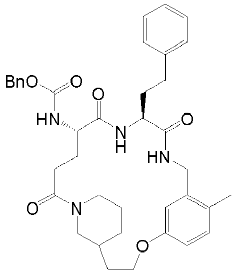 | benzyl ((9S,12S)-5⁴-methyl-8,11,15-trioxo-9-phenethyl-4-oxa-7,10-diaza-1(3,1)-piperidina-5(1,3)-benzenacyclopentadecaphane-12-yl)carbamate |
| **205** | 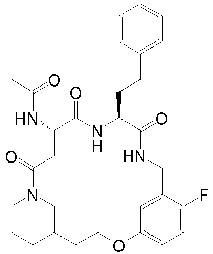 | N-((9S,12S)-5⁴-fluoro-8,11,14-trioxo-9-phenethyl-4-oxa-7,10-diaza-1(3,1)-piperidina-5(1,3)-benzenacyclotetradecaphane-12-yl)acetamide |
| **211** | 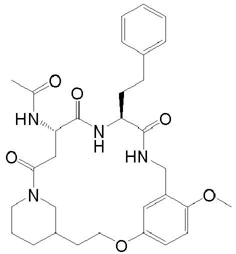 | N-((9S,12S)-5⁴-methoxy-8,11,14-trioxo-9-phenethyl-4-oxa-7,10-diaza-1(3,1)-piperidina-5(1,3)-benzenacyclotetradecaphane-12-yl)acetamide |
| **212** | 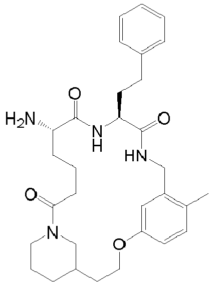 | (9S,12S)-5⁴-methyl-12-amino-8,11,16-trioxo-9-phenethyl-4-oxa-7,10-diaza-1(3,1)-piperidina-5(1,3)-benzenacyclohexadecaphane |
| **213** | 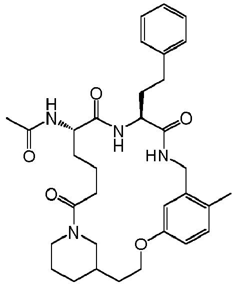 | N-((9S,12S)-5⁴-methyl-8,11,16-trioxo-9-phenethyl-4-oxa-7,10-diaza-1(3,1)-piperidina-5(1,3)-benzenacyclohexadecaphane-12-yl)acetamide |
| **214** | 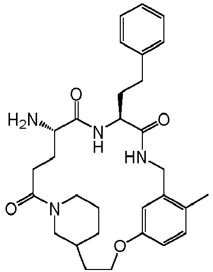 | (9S,12S)-12-amino-5⁴-methyl-8,11,15-trioxo-9-phenethyl-4-oxa-7,10-diaza-1(3,1)-piperidina-5(1,3)-benzenacyclopentadecaphane) |
| **215** | 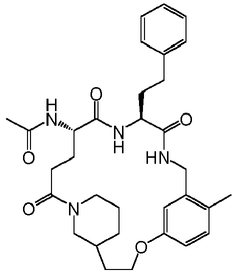 | N-((9S,12S)-5⁴-methyl-8,11,15-trioxo-9-phenethyl-4-oxa-7,10-diaza-1(3,1)-piperidina-5(1,3)-benzenacyclopentadecaphane-12-yl)acetamide |
| **216** | 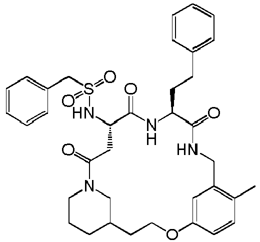 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-1-phenylmethanesulfonamide |
| **217** | 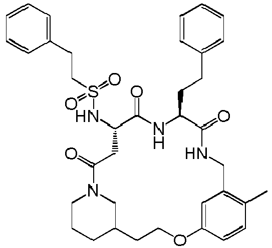 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-phenylethane-1-sulfonam ide |
| **218** | 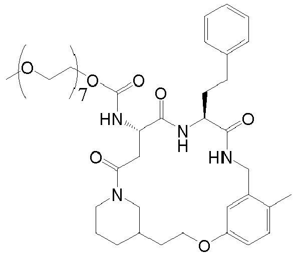 | 2,5,8,11,14,17,20-heptaoxadocosan-22-yl ((9R,12R)-54-methyl-8,11,14-trioxo-9-phenethyl-4-oxa-7,10-diaza-1(3,1)-piperidina-5(1,3)-benzenacyclotetradecaphane-12-yl)carbamate |
| **219** | 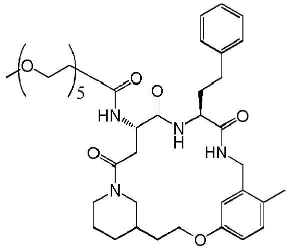 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2,5,8,11,14-pentaoxaheptadecan-17-amide |
| **220** | 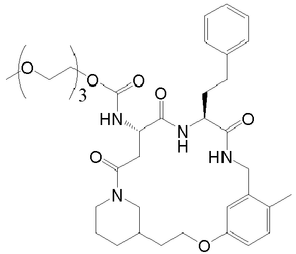 | ((9R,12R)-5⁴-methyl-8,11,14-trioxo-9-phenethyl-4-oxa-7,10-diaza-1(3,1)-piperidina-5(1,3)-benzenacyclotetradecaphane-12-yl)carbamate |
| **252** | 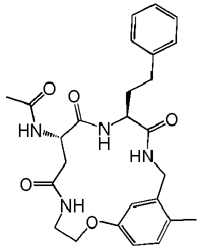 | N-((8S,11S)-1⁴-methyl-6,9,12-trioxo-11-phenethyl-2-oxa-5,10,13-triaza-1(1,3)-benzenacyclotetradecaphane-8-yl)acetamide |
| **253** | 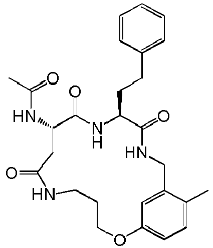 | N-((9S,12S)-1⁴-methyl-7,10,13-trioxo-12-phenethyl-2-oxa-6,11,14-triaza-1(1,3)-benzenacyclopentadecaphane-9-yl)acetamide |
| **254** | 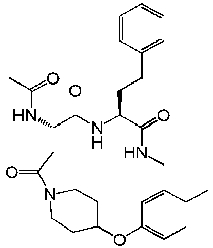 | N-((7S,10S)-3⁴-methyl-6,9,12-trioxo-7-phenethyl-2-oxa-5,8-diaza-1(4,1)-piperidina-3(1,3)-benzenacyclododecaphane-10-yl)acetamide |
| **255** | 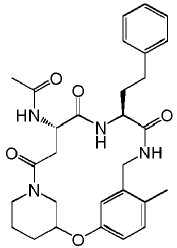 | N-((7S,10S)-3⁴-methyl-6,9,12-trioxo-7-phenethyl-2-oxa-5,8-diaza-1(3,1)-piperidina-3(1,3)-benzenacyclododecaphane-10-yl)acetamide |
| **256** | 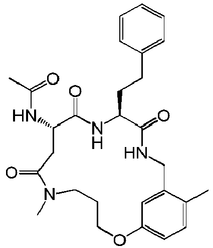 | N-((9S,12S)-1⁴,6-dimethyl-7,10,13-trioxo-12-phenethyl-2-oxa-6,11,14-triaza-1(1,3)-benzenacyclopentadecaphane-9-yl)acetamide |
| **257** | 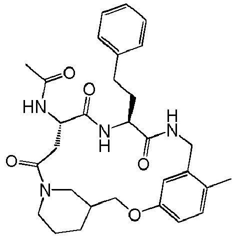 | N-((8S,11S)-4⁴-methyl-7,10,13-trioxo-8-phenethyl-3-oxa-6,9-diaza-1(3,1)-piperidina-4(1,3)-benzenacyclotridecaphane-11-yl)acetamide |
| **258** | 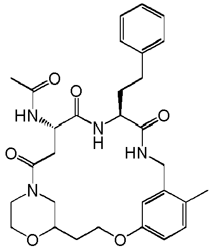 | N-((9S,12S)-5⁴-methyl-8,11,14-trioxo-9-phenethyl-4-oxa-7,10-diaza-1(2,4)-morpholina-5(1,3)-benzenacyclotetradecaphane-12-yl)acetamide |
| **259** | 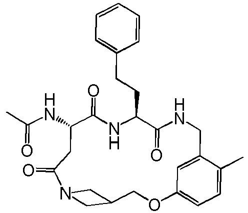 | N-((8S,11S)-4⁴-methyl-7,10,13-trioxo-8-phenethyl-3-oxa-6,9-diaza-1(3,1)-azetidina-4(1,3)-benzenacyclotridecaphane-11-yl)acetamide |
| **260** | 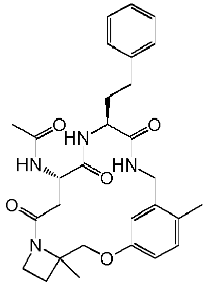 | N-((8S,11S)-1²,4⁴-dimethyl-7,10,13-trioxo-8-phenethyl-3-oxa-6,9-diaza-1(2,1)-azetidina-4(1,3)-benzenacyclotridecaphane-11-yl)acetamide |
| **261** | 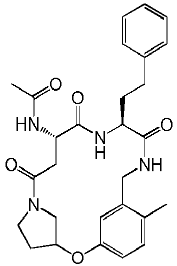 | N-((7S,10S)-3⁴-methyl-6,9,12-trioxo-7-phenethyl-2-oxa-5,8-diaza-1(3,1)-pyrrolidina-3(1,3)-benzenacyclododecaphane-10-yl)acetamide |
| **265** | 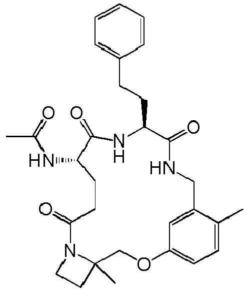 | N-((8S,11S)-1²,4⁴-dimethyl-7,10,14-trioxo-8-phenethyl-3-oxa-6,9-diaza-1(2,1)-azetidina-4(1,3)-benzenacyclotetradecaphane-11-yl)acetamide |
| **269** | 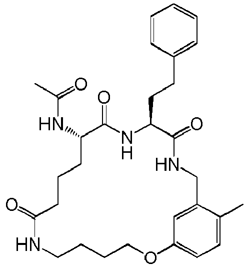 | N-((12S,15S)-1⁴-methyl-8,13,16-trioxo-15-phenethyl-2-oxa-7,14,17-triaza-1 (1,3)-benzenacyclooctadecaphane-12-yl)acetamide |
| **280** | 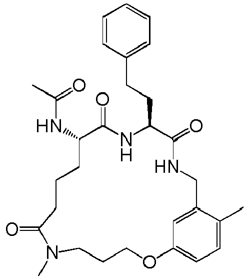 | N-((11S,14S)-1⁴,6-dimethyl-7,12,15-trioxo-14-phenethyl-2-oxa-6,13,16-triaza-1(1,3)-benzenacycloheptadecaphane-11-yl)acetamide |
| **281** | 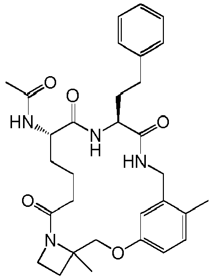 | N-((8S,11S)-1²,4⁴-dimethyl-7,10,15-trioxo-8-phenethyl-3-oxa-6,9-diaza-1(2,1)-azetidina-4(1,3)-benzenacyclopentadecaphane-11-yl)acetamide |
| **282** | 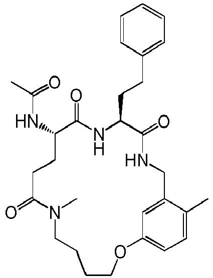 | N-((11S,14S)-1⁴,7 -dimethyl-8,12,15-trioxo-14-phenethyl-2-oxa-7,13,16-triaza-1(1,3)-benzenacycloheptadecaphane-11-yl)acetamide |
| **283** | 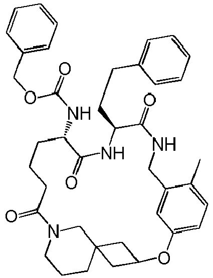 | benzyl ((7S,10S)-3⁴-methyl-6,9,14-trioxo-7-phenethyl-2-oxa-5,8-diaza-15(3,1)-piperidina-3(1,3)-benzena-1(1,3)-cyclobutanapentadecaphane-10-yl)carbamate |
| **284** | 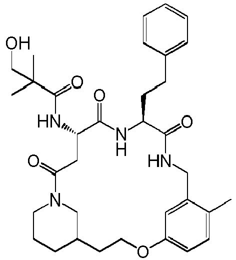 | 3-hydroxy-2,2-dimethyl-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)propanamide |
| **285** | 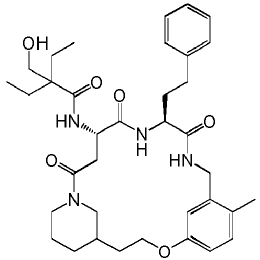 | 2-ethyl-2-(hydroxymethyl)-N-((9S, 12S)-5⁴-methyl-8,11,14-trioxo-9-phenethyl-4-oxa-7,10-diaza-1(3,1)-piperidina-5(1,3)-benzenacyclotetradecaphane-12-yl)butanamide |
| **286** | 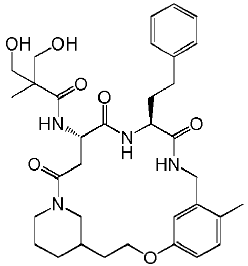 | 3-hydroxy-2-(hydroxymethyl)-2-methyl-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)propanamide |
| **287** | 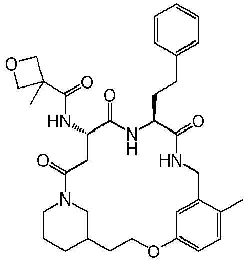 | 3-methyl-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)oxetane-3-carboxamide |
| **288** | 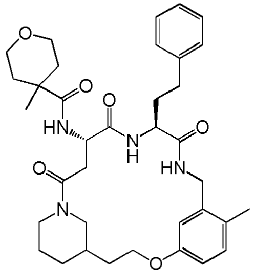 | 4-methyl-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)tetrahydro-2H-pyran-4-carboxamide |
| **289** | 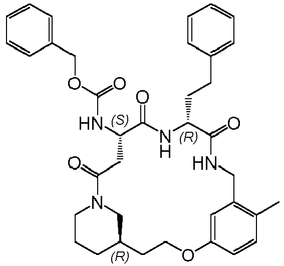 | benzyl ((13R,9R,12S)-5⁴-methyl-8,11,14-trioxo-9-phenethyl-4-oxa-7,10-diaza-1(3,1)-piperidina-5(1,3)-benzenacyclotetradecaphane-12-yl)carbamate |
| **290** | 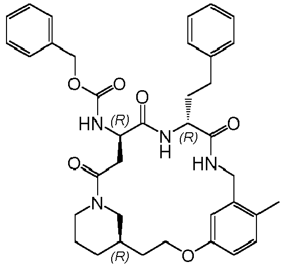 | benzyl ((13R,9R,12R)-5⁴-methyl-8,11,14-trioxo-9-phenethyl-4-oxa-7,10-diaza-1(3,1)-piperidina-5(1,3)-benzenacyclotetradecaphane-12-yl)carbamate |
| **291** | 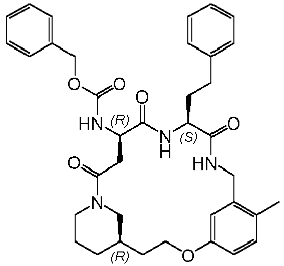 | benzyl ((13R,9S,12R)-5⁴⁻methyl-8,11,14-trioxo-9-phenethyl-4-oxa-7,10-diaza-1(3,1)-piperidina-5(1,3)-benzenacyclotetradecaphane-12-yl)carbamate |
| **292** | 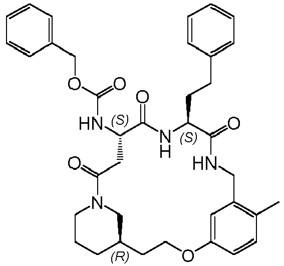 | benzyl ((13R,9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamate |
| **293** | 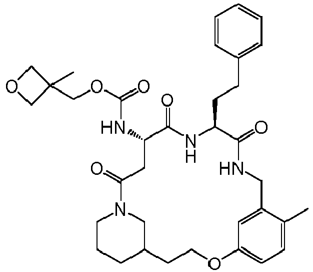 | (3-methyloxetan-3-yl)methyl ((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamate |
| **294** | 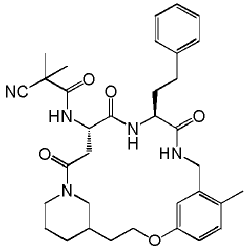 | 2-cyano-2-methyl-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)propanamide |
| **295** | 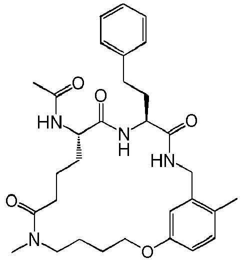 | N-(1⁴,7-dimethyl-8,13,16-trioxo-15-phenethyl-2-oxa-7,14,17-triaza-1(1,3)-benzenacyclooctadecaphane-12-yl)acetamide |
| **296** | 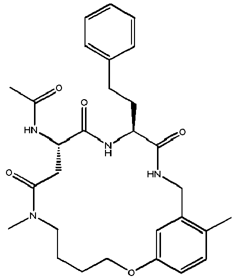 | N-(1⁴,7-dimethyl-8,11,14-trioxo-13-phenethyl-2-oxa-7,12,15-triaza-1(1,3)-benzenacyclohexadecaphane-10-yl)acetamide |
| **298** | 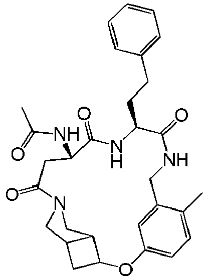 | N-(3⁴-methyl-6,9,12-trioxo-7-phenethyl-2-oxa-13,5,8-triaza-1(6,3)-bicyclo[3.2.0]heptana-3(1,3)-benzenacyclododecaphane-10-yl)acetamide |
| **299** | 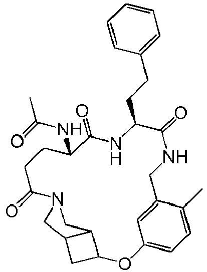 | N-(3⁴-methyl-6,9,13-trioxo-7-phenethyl-2-oxa-13,5,8-triaza-1(6,3)-bicyclo[3.2.0]heptana-3(1,3)-benzenacyclotridecaphane-10-yl)acetamide |
| **300** | 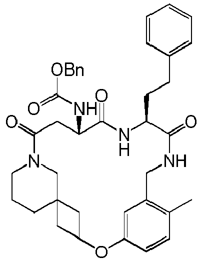 | benzyl ((7S,10S)-3⁴-methyl-6,9,12-trioxo-7-phenethyl-2-oxa-5,8-diaza-13(3,1)-piperidina-3(1,3)-benzena-1(1,3)-cyclobutanatridecaphane-10-yl)carbamate |
| **301** | 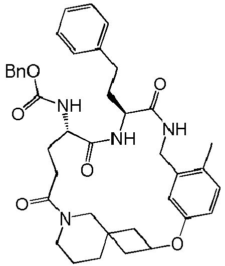 | benzyl ((7S,10S)-3⁴-methyl-6,9,13-trioxo-7-phenethyl-2-oxa-5,8-diaza-14(3,1)-piperidina-3(1,3)-benzena-1(1,3)-cyclobutanatetradecaphane-10-yl)carbamate |
| **302** | 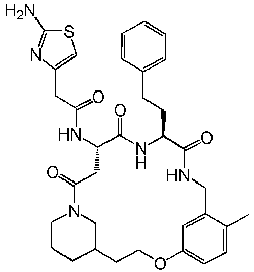 | 2-(2-aminothiazol-4-yl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **303** | 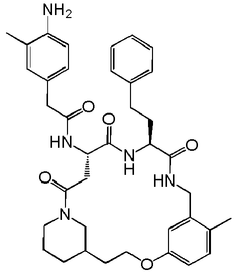 | 2-(4-amino-3-methylphenyl)-N-((9S, 12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **304** | 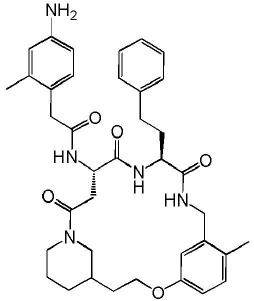 | 2-(4-amino-2-methylphenyl)-N-((9S, 12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **305** | 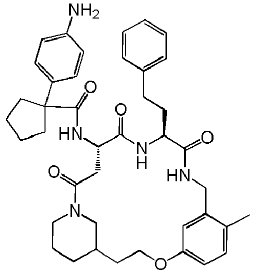 | 1-(4-aminophenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)cyclopentane-1-carboxamide |
| **306** | 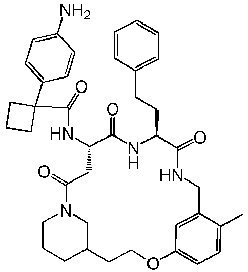 | 1-(4-aminophenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)cyclobutane-1-carboxamide |
| **307** | 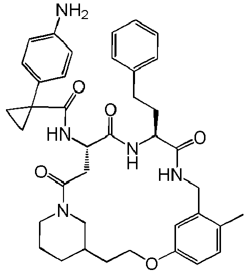 | 1-(4-aminophenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)cyclopropane-1-carboxamide |
| **308** | 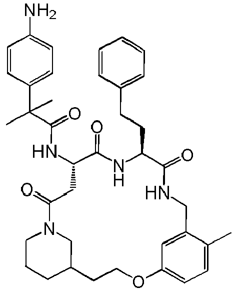 | 2-(4-aminophenyl)-2-methyl-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)propanamide |
| **309** | 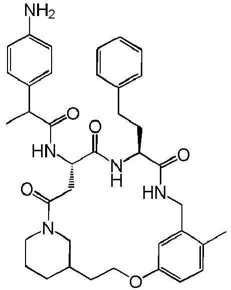 | 2-(4-aminophenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)propanamide |
| **310** | 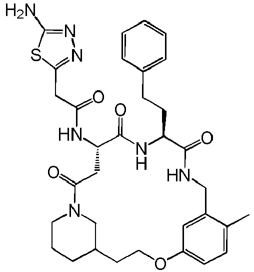 | 2-(5-amino-1,3,4-thiadiazol-2-yl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **311** | 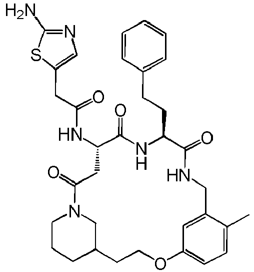 | 2-(2-aminothiazol-5-yl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **312** | 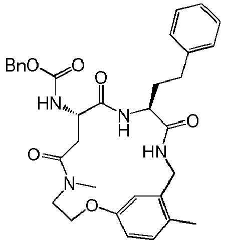 | benzyl ((8S,11S)-1⁴,5-dimethyl-6,9,12-trioxo-11-phenethyl-2-oxa-5,10,13-triaza-1(1,3)-benzenacyclotetradecaphane-8-yl)carbamate |
| **313** | 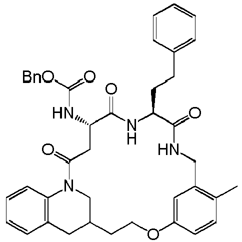 | benzyl ((9S, 12S)-5⁴-methyl-8,11,14-trioxo-9-phenethyl-11,12,13,14-tetrahydro-4-oxa-7,10-diaza-1(3,1)-quinolina-5(1,3)-benzenacyclotetradecaphane-12-yl)carbamate |
| **314** | 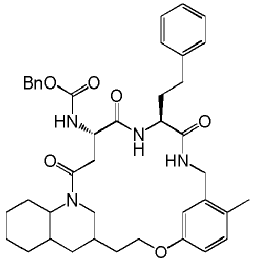 | benzyl ((9S, 12S)-5⁴-methyl-8,11,14-trioxo-9-phenethyl-11,12,13,14,14a,15,16,17,18,18a-decahydro-4-oxa-7,10-diaza-1(3,1)-quinolina-5(1,3)-benzenacyclotetradecaphane-12-yl)carbamate |
| **315** | 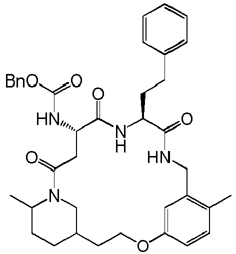 | benzyl ((9S, 12S)-1⁶,5⁴-dimethyl-8,11,14-trioxo-9-phenethyl-4-oxa-7,10-diaza-1(3,1)-piperidina-5(1,3)-benzenacyclotetradecaphane-12-yl)carbamate |
| **316** | 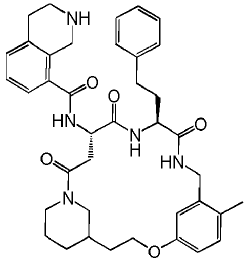 | N-((9S, 12S)-5⁴-methyl-8,11,14-trioxo-9-phenethyl-4-oxa-7,10-diaza-1(3,1)-piperidina-5(1,3)-benzenacyclotetradecaphane-12-yl)-1,2,3,4-tetrahydroisoquinoline-5-carboxam ide |
| **317** | 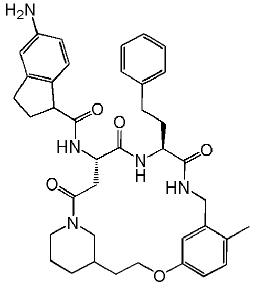 | 5-amino-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2,3-dihydro-1H-indene-1-carboxamide |
| **318** | 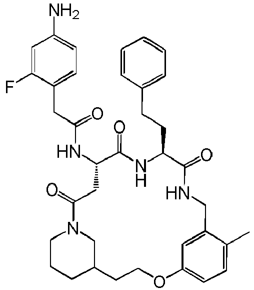 | 2-(4-amino-2-fluorophenyl)-N-((9S, 12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **319** | 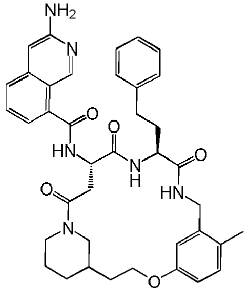 | 3-amino-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)isoquinoline-8-carboxamide |
| **320** | 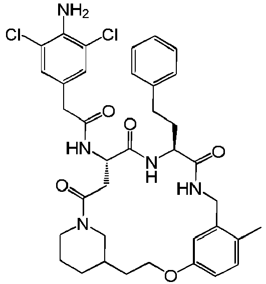 | 2-(4-amino-3,5-dichlorophenyl)-N-((9S, 12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **321** | 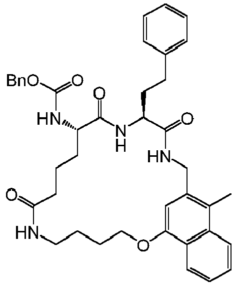 | benzyl ((9S, 12S)-5⁴-methyl-8,11,14-trioxo-9-phenethyl-4-oxa-7,10-diaza-1(3,1)-piperidina-5(1,3)-naphthalenacyclotetradecaphane-12-yl)carbamate |
| **322** | 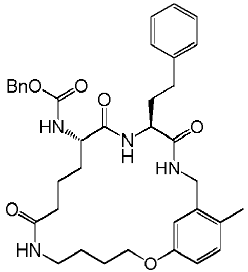 | benzyl ((12S, 15S)-1⁴-methyl-8,13,16-trioxo-15-phenethyl-2-oxa-7,14,17-triaza-1(1,3)-benzenacyclooctadecaphane-12-yl)carbamate |
| **323** | 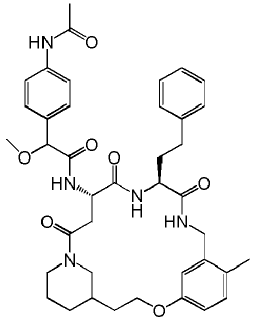 | 2-(4-acetamidophenyl)-2-methoxy-N-((9S, 12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **324** | 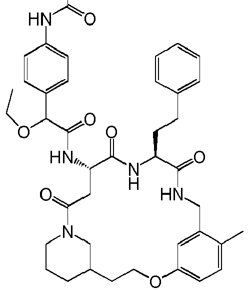 | 2-(4-acetamidophenyl)-2-ethoxy-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **325** | 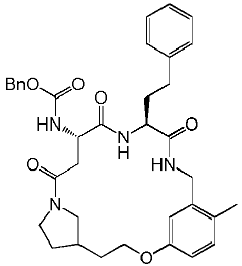 | benzyl ((9S,12S)-5⁴-methyl-8,11,14-trioxo-9-phenethyl-4-oxa-7,10-diaza-1(3,1)-pyrrolidina-5(1,3)-benzenacyclotetradecaphane-12-yl)carbamate |
| **326** | 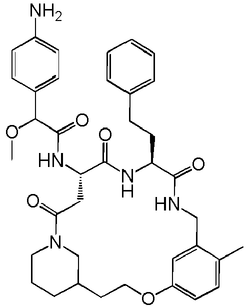 | 2-(4-aminophenyl)-2-methoxy-N-((9S, 12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **327** | 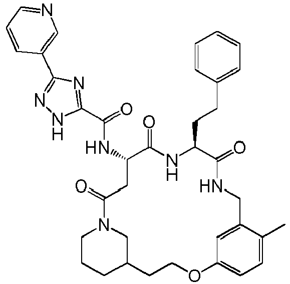 | N-((9S,12R)-5⁴-methyl-8,11,14-trioxo-9-phenethyl-4-oxa-7,10-diaza-1(3,1)-piperidina-5(1,3)-benzenacyclotetradecaphane-12-yl)-2-(3-(pyridin-3-yl)-1H-1,2,4-triazol-5-yl)acetamide |
| **328** | 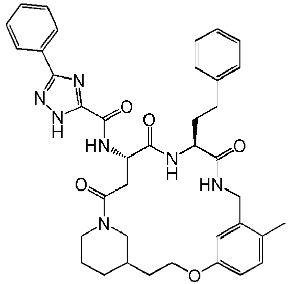 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-(3-phenyl-1H-1,2,4-triazol-5-yl)acetamide |
| **329** | 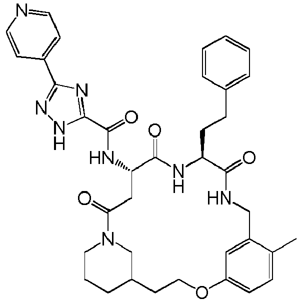 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-(3-(pyridin-4-yl)-1H-1,2,4-triazol-5-yl)acetamide |
| **330** | 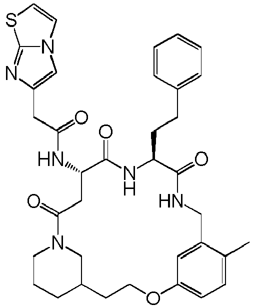 | 2-(imidazo[2,1-b]thiazol-6-yl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **331** | 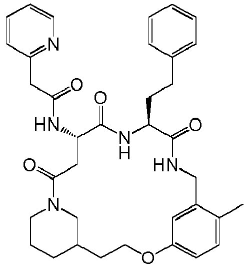 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-(pyridin-2-yl)acetamide |
| **332** | 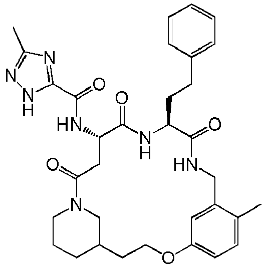 | 2-(3-methyl-1H-1,2,4-triazol-5-yl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **333** | 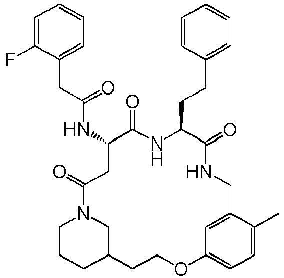 | 2-(2-fluorophenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **334** | 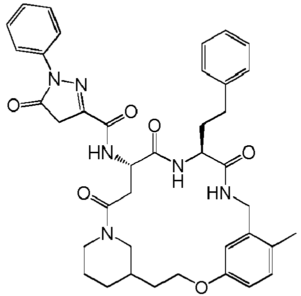 | N-((9S, 12S)-5⁴-methyl-8,11,14-trioxo-9-phenethyl-4-oxa-7,10-diaza-1(3,1)-piperidina-5(1,3)-benzenacyclotetradecaphane-12-yl)-2-(5-oxo-1-phenyl-4,5-dihydro-1H-pyrazol-3-yl)acetamide |
| **335** | 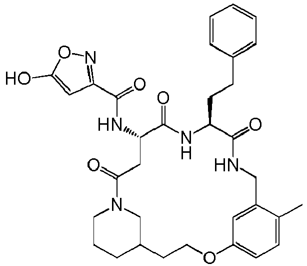 | 2-(5-hydroxyisoxazol-3-yl)-N-((9S, 12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **336** | 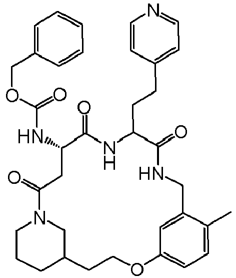 | benzyl ((12S)-5⁴-methyl-8,11, 14-trioxo-9-(2-(pyridin-4-yl)ethyl)-4-oxa-7,10-diaza-1(3,1)-piperidina-5(1,3)-benzenacyclotetradecaphane-12-yl)carbamate |
| **337** | 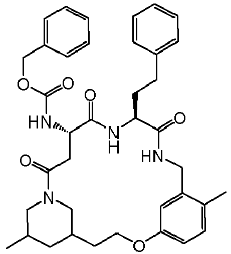 | benzyl ((9S,12S)-1⁵,5⁴-dimethyl-8,11,14-trioxo-9-phenethyl-4-oxa-7,10-diaza-1(3,1)-piperidina-5(1,3)-benzenacyclotetradecaphane-12-yl)carbamate |
| **338** | 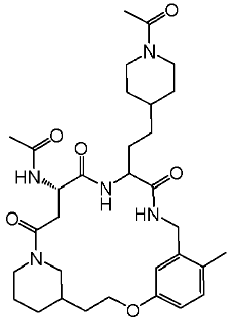 | N-((12S)-9-(2-(1-acetylpiperidin-4-yl)ethyl)-5⁴-methyl-8,11,14-trioxo-4-oxa-7,10-diaza-1(3,1)-piperidina-5(1,3)-benzenacyclotetradecaphane-12-yl)acetamide |
| **339** | 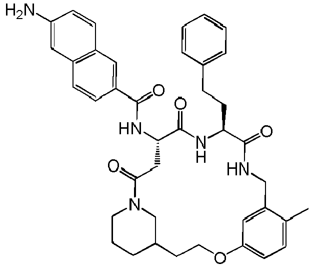 | 6-amino-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-naphthamide |
| **340** | 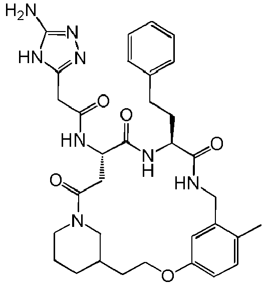 | 2-(5-amino-1H-1,2,4-triazol-3-yl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **341** | 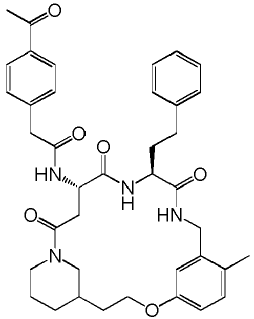 | 2-(4-acetylphenyl)-N-((9S, 12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **342** | 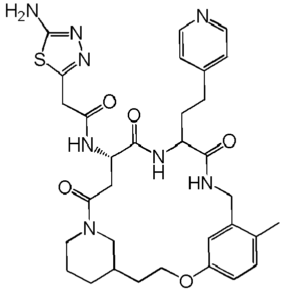 | 2-(5-amino-1,3,4-thiadiazol-2-yl)-N-((12S)-5⁴-methyl-8,11,14-trioxo-9-(2-(pyridin-4-yl)ethyl)-4-oxa-7,10-diaza-1(3,1)-piperidina-5(1,3)-benzenacyclotetradecaphane-12-yl)acetamide |
| **343** | 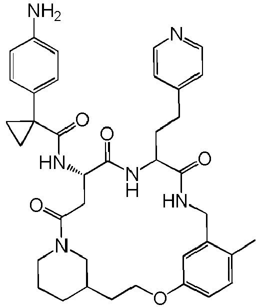 | 1-(4-aminophenyl)-N-((12S)-5⁴-methyl-8,11,14-trioxo-9-(2-(pyridin-4-yl)ethyl)-4-oxa-7,10-diaza-1(3,1)-piperidina-5(1,3)-benzenacyclotetradecaphane-12-yl)cyclopropane-1-carboxamide |
| **344** | 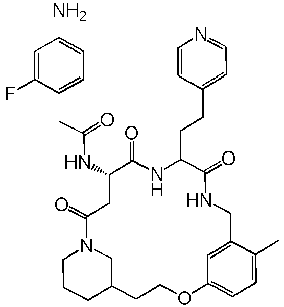 | 2-(4-amino-2-fluorophenyl)-N-((12S)-5⁴-methyl-8,11,14-trioxo-9-(2-(pyridin-4-yl)ethyl)-4-oxa-7,10-diaza-1(3,1)-piperidina-5(1,3)-benzenacyclotetradecaphane-12-yl)acetamide |
| **345** | 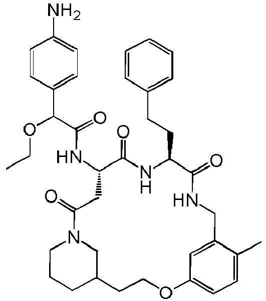 | 2-(4-aminophenyl)-2-ethoxy-N-((9S, 12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **346** | 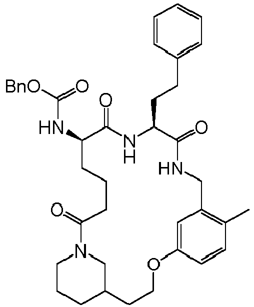 | benzyl ((9S,12R)-5⁴-methyl-8,11,16-trioxo-9-phenethyl-4-oxa-7,10-diaza-1(3,1)-piperidina-5(1,3)-benzenacyclohexadecaphane-12-yl)carbamate |
| **347** | 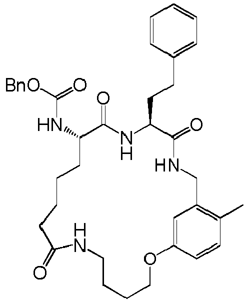 | benzyl ((13S,16S)-1⁴-methyl-8,14,17-trioxo-16-phenethyl-2-oxa-7,15,18-triaza-1(1,3)-benzenacyclononadecaphane-13-yl)carbamate |
| **348** | 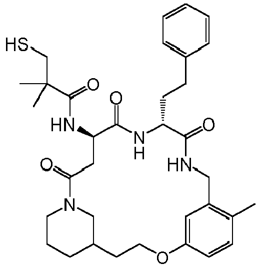 | 3-mercapto-2,2-dimethyl-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)propanamide |
| **349** | 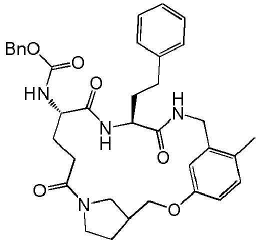 | benzyl ((8S,11S)-4⁴-methyl-7,10,14-trioxo-8-phenethyl-3-oxa-6,9-diaza-1(3,1)-pyrrolidina-4(1,3)-benzenacyclotetradecaphane-11-yl)carbamate |
| **350** | 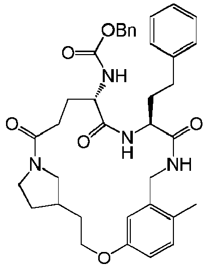 | benzyl ((9S, 12S)-5⁴-methyl-8, 11,15-trioxo-9-phenethyl-4-oxa-7,10-diaza-1(3,1)-pyrrolidina-5(1,3)-benzenacyclopentadecaphane-12-yl)carbamate |
| **351** | 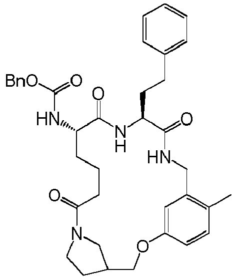 | benzyl ((8S,11S)-4⁴-methyl-7,10,15-trioxo-8-phenethyl-3-oxa-6,9-diaza-1(3,1)-pyrrolidina-4(1,3)-benzenacyclopentadecaphane-11-yl)carbamate |
| **352** | 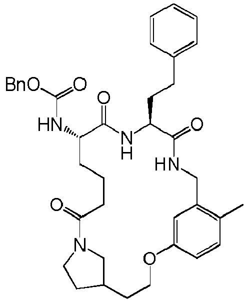 | benzyl ((9S,12S)-5⁴-methyl-8,11,16-trioxo-9-phenethyl-4-oxa-7,10-diaza-1(3,1)-pyrrolidina-5(1,3)-benzenacyclohexadecaphane-12-yl)carbam ate |
| **353** | 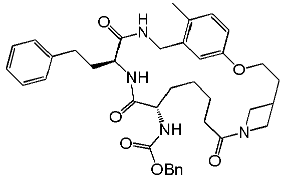 | benzyl ((9S,12S)-5⁴-methyl-8,11,17-trioxo-9-phenethyl-4-oxa-7,10-diaza-1(3,1)-azetidina-5(1,3)-benzenacycloheptadecaphane-12-yl)carbamate |
| **354** | 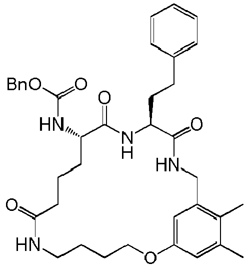 | benzyl ((12S,15S)-1⁴,1⁵-dimethyl-8,13,16-trioxo-15-phenethyl-2-oxa-7,14,17-triaza-1(1,3)-benzenacyclooctadecaphane-12-yl)carbamate |
| **355** | 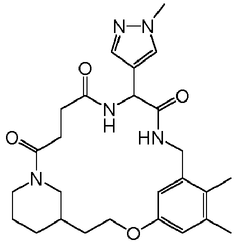 | 5⁴,5⁵-dimethyl-9-(1-methyl-1H-pyrazol-4-yl)-4-oxa-7,10-diaza-1(3,1)-piperidina-5(1,3)-benzenacyclotetradecaphane-8,11,14-trione |
| **356** | 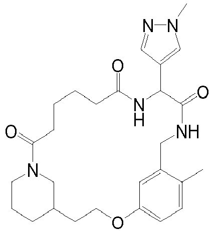 | 5⁴-methyl-9-(1-methyl-1H-pyrazol-4-yl)-4-oxa-7,10-diaza-1(3,1)-piperidina-5(1,3)-benzenacyclohexadecaphane-8,11,16-trione |
| **358** | 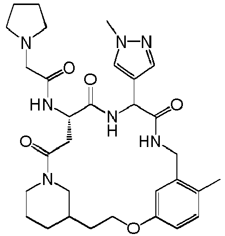 | N-((12S)-5⁴-methyl-9-(1-methyl-1H-pyrazol-4-yl)-8,11,14-trioxo-4-oxa-7,10-diaza-1(3,1)-piperidina-5(1,3)-benzenacyclotetradecaphane-12-yl)-2-(pyrrolidin-1-yl)acetamide |
| **359** | 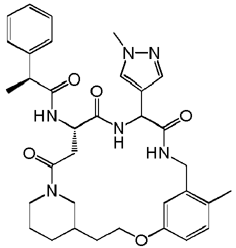 | (2S)-N-((12S)-5⁴-methyl-9-(1-methyl-1H-pyrazol-4-yl)-8,11,14-trioxo-4-oxa-7,10-diaza-1(3,1)-piperidina-5(1,3)-benzenacyclotetradecaphane-12-yl)-2-phenylpropanamide |
| **360** | 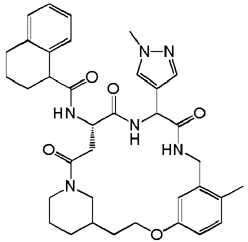 | N-((12S)-5⁴-methyl-9-(1-methyl-1H-pyrazol-4-yl)-8,11,14-trioxo-4-oxa-7,10-diaza-1(3,1)-piperidina-5(1,3)-benzenacyclotetradecaphane-12-yl)-1,2,3,4-tetrahydronaphthalene-1 - carboxam ide |
| **361** | 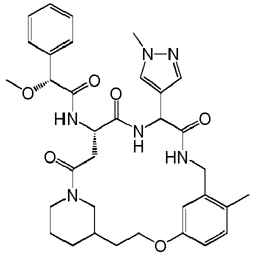 | (2R)-2-methoxy-N-((12S)-5⁴-methyl-9-(1-methyl-1H-pyrazol-4-yl)-8,11,14-trioxo-4-oxa-7,10-diaza-1(3,1)-piperidina-5(1,3)-benzenacyclotetradecaphane-12-yl)-2-phenylacetamide |
| **362** | 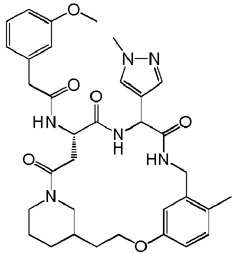 | 2-(3-methoxyphenyl)-N-((12S)-5⁴-methyl-9-(1-methyl-1H-pyrazol-4-yl)-8,11,14-trioxo-4-oxa-7,10-diaza-1(3,1)-piperidina-5(1,3)-benzenacyclotetradecaphane-12-yl)acetamide |
| **363** | 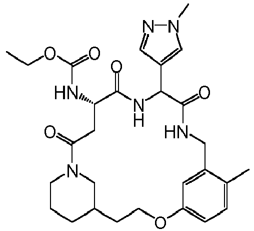 | Ethyl ((12S)-5⁴-methyl-9-(1-methyl-1H-pyrazol-4-yl)-8,11,14-trioxo-4-oxa-7,10-diaza-1(3,1)-piperidina-5(1,3)-benzenacyclotetradecaphane-12-yl)carbamate |
| **364** | 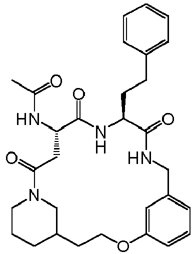 | N-((9S,12S)-8,11,14-trioxo-9-phenethyl-4-oxa-7,10-diaza-1(3,1)-piperidina-5(1,3)-benzenacyclotetradecaphane-12-yl)acetamide |
| **369** | 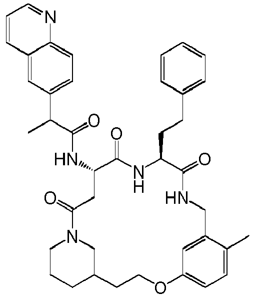 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-(quinolin-6-yl)propanamide |
| **370** | 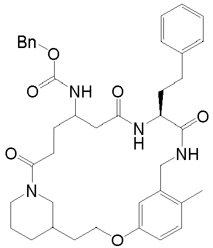 | benzyl ((9S)-5⁴-methyl-8,11,16-trioxo-9-phenethyl-4-oxa-7,10-diaza-1(3,1)-piperidina-5(1,3)-benzenacyclohexadecaphane-13-yl)carbamate |
| **371** | 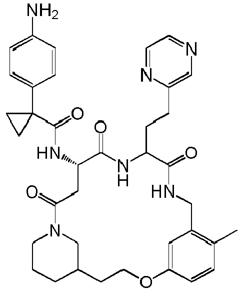 | 1-(4-aminophenyl)-N-((9S,12S)-5⁴-methyl-8,11,14-trioxo-9-(2-(pyrazin-2-yl)ethyl)-4-oxa-7,10-diaza-1(3,1)-piperidina-5(1,3)-benzenacyclotetradecaphane-12-yl)cyclopropane-1-carboxamide |
| **372** | 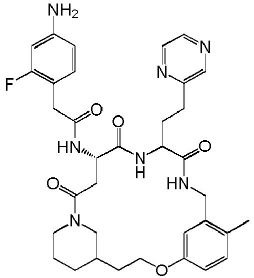 | 2-(4-amino-2-fluorophenyl)-N-((9S,12S)-5⁴-methyl-8,11,14-trioxo-9-(2-(pyrazin-2-yl)ethyl)-4-oxa-7,10-diaza-1(3,1)-piperidina-5(1,3)-benzenacyclotetradecaphane-12-yl)acetamide |
| **373** | 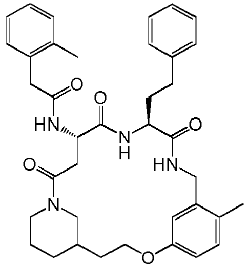 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-(o-tolyl)acetamide |
| **387** | 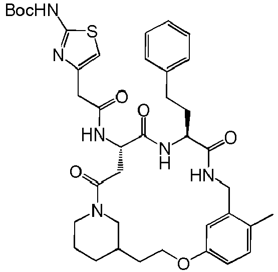 | tert-butyl (4-(2-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)amino)-2-oxoethyl)thiazol-2-yl)carbamate |
| **388** | 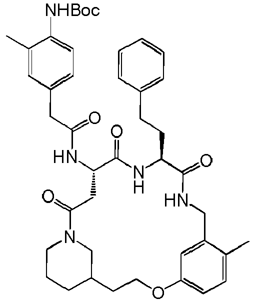 | tert-butyl (2-methyl-4-(2-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)amino)-2-oxoethyl)phenyl)carbamate |
| **389** | 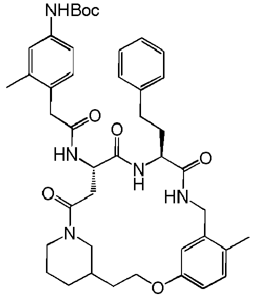 | tert-butyl (3-methyl-4-(2-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)amino)-2-oxoethyl)phenyl)carbamate |
| **390** | 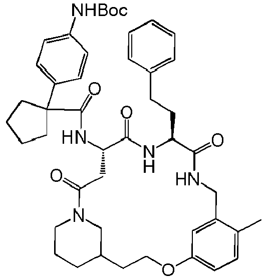 | tert-butyl (4-(1-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)cyclopentyl)phenyl)carbamate |
| **391** | 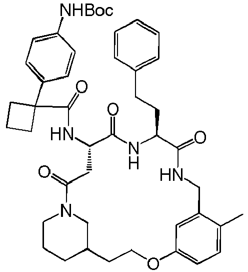 | tert-butyl (4-(1-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)cyclobutyl)phenyl)carbamate |
| **392** | 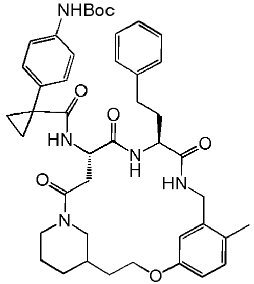 | tert-butyl (4-(1-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)cyclopropyl)phenyl)carbamate |
| **393** | 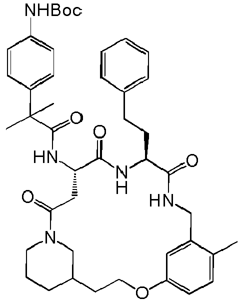 | tert-butyl (4-(2-methyl-1-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)amino)-1-oxopropan-2-yl)phenyl)carbamate |
| **394** | 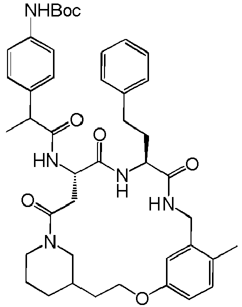 | 2-(4-aminophenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)propanamide |
| **395** | 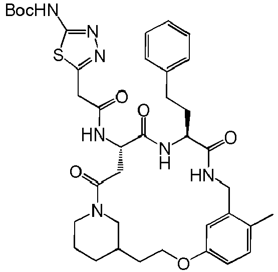 | tert-butyl (5-(2-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)amino)-2-oxoethyl)-1,3,4-thiadiazol-2-yl)carbamate |
| **396** | 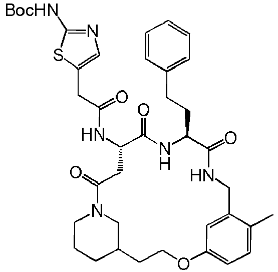 | tert-butyl (5-(2-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)amino)-2-oxoethyl)thiazol-2-yl)carbamate |
| **402** | 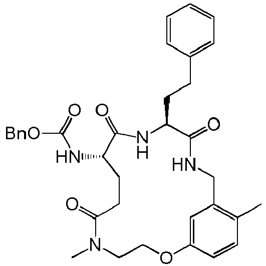 | benzyl ((9S,12S)-1⁴,5-dimethyl-6,10,13-trioxo-12-phenethyl-2-oxa-5,11,14-triaza-1(1,3)-benzenacyclopentadecaphane-9-yl)carbamate |
| **405** | 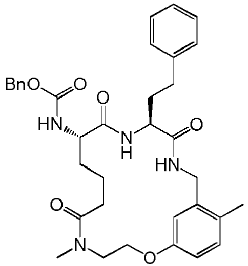 | benzyl ((10S,13S)-1⁴,5-dimethyl-6,11,14-trioxo-13-phenethyl-2-oxa-5, 12, 15-triaza-1 (1,3)-benzenacyclohexadecaphane-10-yl)carbamate |
| **419** | 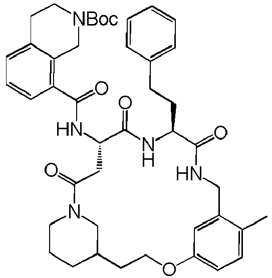 | tert-butyl 8-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)-3,4-dihydroisoquinoline-2(1H)-carboxylat |
| **420** | 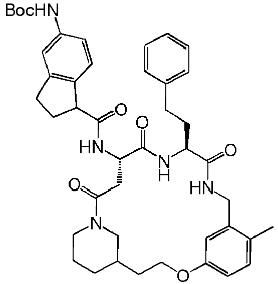 | tert-butyl 8-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)-3,4-dihydroisoquinoline-2(1H)-carboxylat |
| **421** | 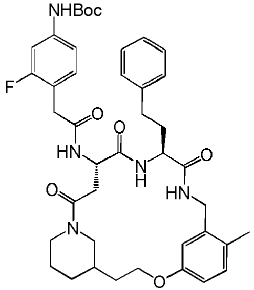 | tert-butyl 8-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)-3,4-dihydroisoquinoline-2(1H)-carboxylat |
| **422** | 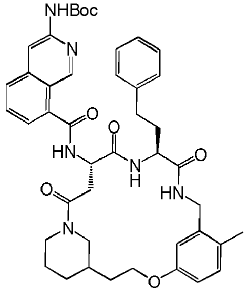 | tert-butyl 8-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)-3,4-dihydroisoquinoline-2(1H)-carboxylat |
| **423** | 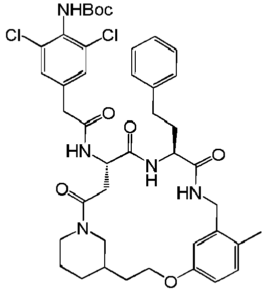 | tert-butyl (2,6-dichloro-4-(2-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)amino)-2-oxoethyl)phenyl)carbamate |
| **429** | 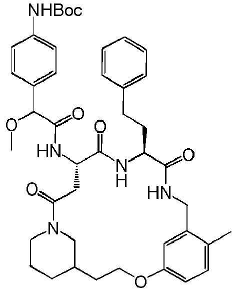 | tert-butyl (4-(1-methoxy-2-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)amino)-2-oxoethyl)phenyl)carbamate |
| **437** | 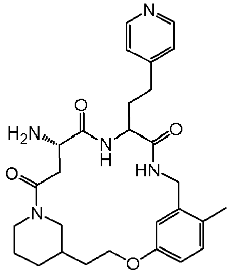 | ((12S)-12-amino-5⁴-methyl-8,11,14-trioxo-9-(2-(pyridin-4-yl)ethyl)-4-oxa-7,10-diaza-1(3,1)-piperidina-5(1,3)-benzenacyclotetradecaphane- |
| **438** | 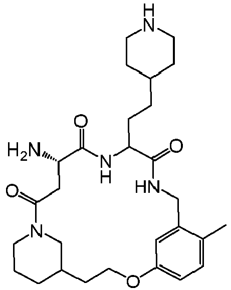 | N-((12S)-12-amino-9-(2-(1-acetylpiperidin-4-yl)ethyl)-54-methyl-8,11,14-trioxo-4-oxa-7,10-diaza-1(3,1)-piperidina-5(1,3)-benzenacyclotetradecaphane |
| **439** | 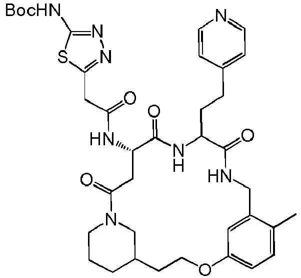 | tert-butyl (5-(2-(((12S)-5⁴-methyl-8,11,14-trioxo-9-(2-(pyridin-4-yl)ethyl)-4-oxa-7,10-diaza-1(3,1)-piperidina-5(1,3)-benzenacyclotetradecaphane-12-yl)amino)-2-oxoethyl)-1,3,4-thiadiazol-2-yl)carbamate |
| **440** | 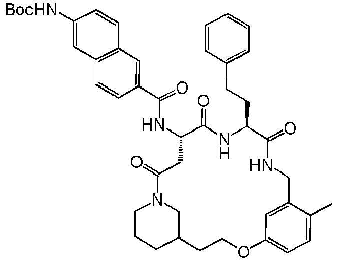 | tert-butyl (6-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)naphthalen-2-yl)carbamate |
| **441** | 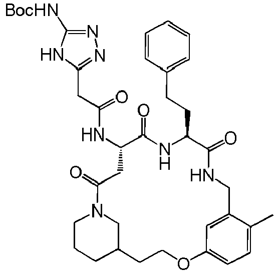 | tert-butyl (5-(2-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)amino)-2-oxoethyl)-4H-1,2,4-triazol-3-yl)carbamate |
| **442** | 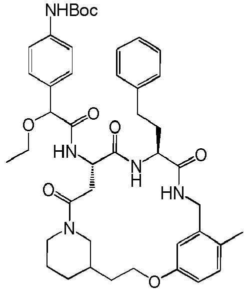 | tert-butyl (4-(1-ethoxy-2-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)amino)-2-oxoethyl)phenyl)carbamate |
| **443** | 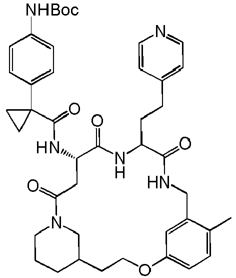 | tert-butyl (4-(1-(((12S)-5⁴-methyl-8,11,14-trioxo-9-(2-(pyridin-4-yl)ethyl)-4-oxa-7,10-diaza-1(3,1)-piperidina-5(1,3)-benzenacyclotetradecaphane-12-yl)carbamoyl)cyclopropyl)phenyl)carbama te |
| **444** | 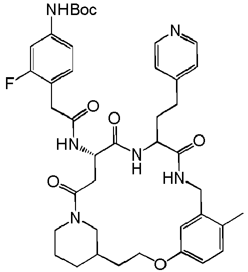 | tert-butyl (3-fluoro-4-(2-(((12S)-5⁴-methyl-8,11,14-trioxo-9-(2-(pyridin-4-yl)ethyl)-4-oxa-7,10-diaza-1(3,1)-piperidina-5(1,3)-benzenacyclotetradecaphane-12-yl)amino)-2-oxoethyl)phenyl)carbamate |
| **479** | 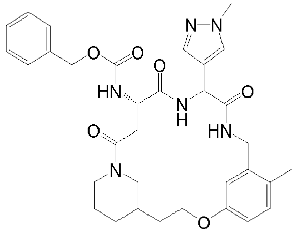 | Benzyl ((12S)-5⁴-methyl-9-(1-methyl-1H-pyrazol-4-yl)-8,11,14-trioxo-4-oxa-7,10-diaza-1(3,1)-piperidina-5(1,3)-benzenacyclotetradecaphane-12-yl)carbamate |
| **480** | 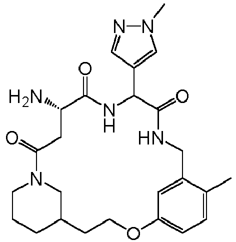 | (12S)-12-amino-5⁴-methyl-9-(1-methyl-1H-pyrazol-4-yl)-4-oxa-7,10-diaza-1(3,1)-piperidina-5(1,3)-benzenacyclotetradecaphane-8,11,14-trione |
| **495** | 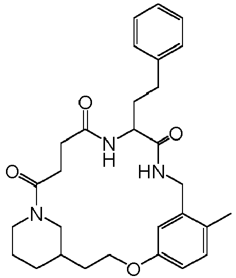 | 5⁴-methyl-9-phenethyl-4-oxa-7,10-diaza-1(3,1)-piperidina-5(1,3)-benzenacyclotetradecaphane-8,11,14-trione |
| **503** | 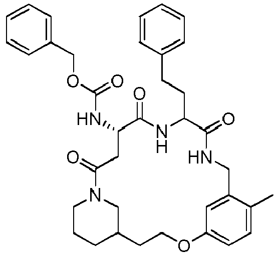 | benzyl ((12S)-9-(2-(1,5-naphthyridin-3-yl)ethyl)-5⁴-methyl-8,11,14-trioxo-4-oxa-7,10-diaza-1(3,1)-piperidina-5(1,3)-benzenacyclotetradecaphane-12-yl)carbamate |
| **505** | 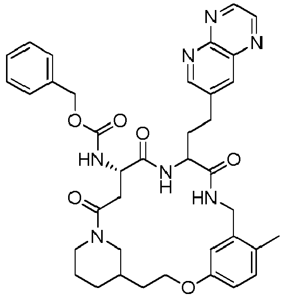 | benzyl ((12S)-5⁴-methyl-8,11,14-trioxo-9-(2-(pyrido[2,3-b]pyrazin-7-yl)ethyl)-4-oxa-7,10-diaza-1(3,1)-piperidina-5(1,3)-benzenacyclotetradecaphane-12-yl)carbamate |
| **509** | 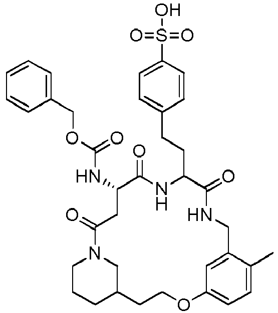 | 4-(2-((12S)-12-(((benzyloxy)carbonyl)amino)-5⁴-methyl-8,11,14-trioxo-4-oxa-7,10-diaza-1(3,1)-piperidina-5(1,3)-benzenacyclotetradecaphane-9-yl)ethyl)benzenesulfonic acid |
| **510** | 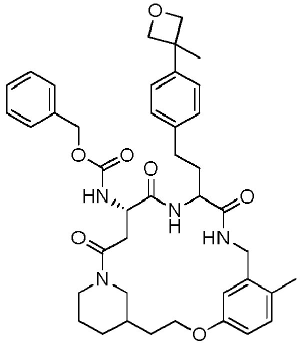 | benzyl ((12S)-5⁴-methyl-9-(4-(3-methyloxetan-3-yl)phenethyl)-8,11,14-trioxo-4-oxa-7,10-diaza-1(3,1)-piperidina-5(1,3)-benzenacyclotetradecaphane-12-yl)carbamate |
| **511** | 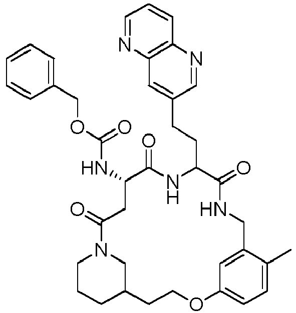 | benzyl ((12S)-9-(2-(1,5-naphthyridin-3-yl)ethyl)-5⁴-methyl-8,11,14-trioxo-4-oxa-7,10-diaza-1(3,1)-piperidina-5(1,3)-benzenacyclotetradecaphane-12-yl)carbamate |
| **512** | 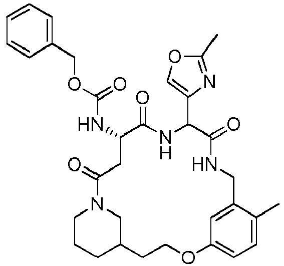 | benzyl ((12S)-5⁴-methyl-9-(2-methyloxazol-4-yl)-8,11,14-trioxo-4-oxa-7,10-diaza-1(3,1)-piperidina-5(1,3)-benzenacyclotetradecaphane-12-yl)carbamate |
| **513** | 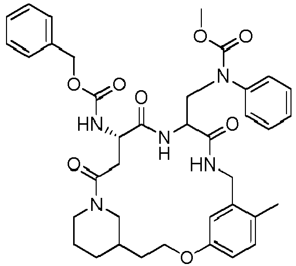 | methyl (((12R)-12-(((benzyloxy)carbonyl)amino)-5⁴-methyl-8,11,14-trioxo-4-oxa-7,10-diaza-1(3,1)-piperidina-5(1,3)-benzenacyclotetradecaphane-9-yl)methyl)(phenyl)carbamate |
| **549** | 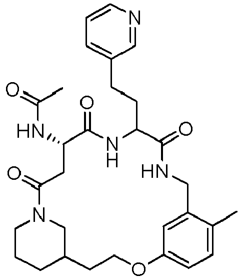 | N-((12S)-5⁴-methyl-8,11,14-trioxo-9-(2-(pyridin-3-yl)ethyl)-4-oxa-7,10-diaza-1(3,1)-piperidina-5(1,3)-benzenacyclotetradecaphane-12-yl)acetamide |
| **550** | 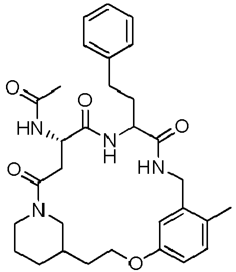 | N-((12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **551** | 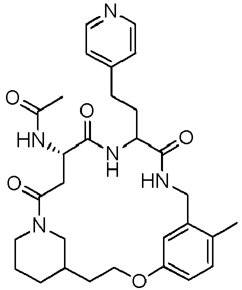 | N-((12S)-5⁴-methyl-8,11,14-trioxo-9-(2-(pyridin-4-yl)ethyl)-4-oxa-7,10-diaza-1(3,1)-piperidina-5(1,3)-benzenacyclotetradecaphane-12-yl)acetamide |
| **552** | 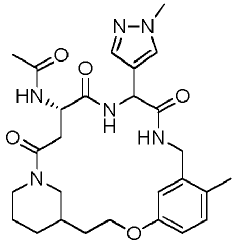 | N-((12S)-5⁴-methyl-9-(1-methyl-1H-pyrazol-4-yl)-8,11,14-trioxo-4-oxa-7,10-diaza-1(3,1)-piperidina-5(1,3)-benzenacyclotetradecaphane-12-yl)acetamide |
| **553** | 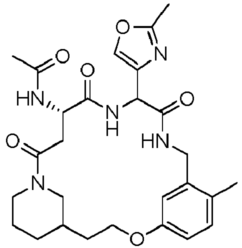 | N-((12S)-5⁴-methyl-9-(2-methyloxazol-4-yl)-8,11,14-trioxo-4-oxa-7,10-diaza-1(3,1)-piperidina-5(1,3)-benzenacyclotetradecaphane-12-yl)acetamide |
| **554** | 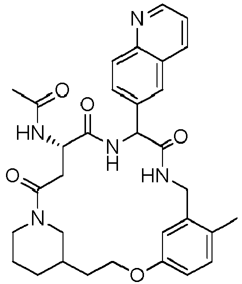 | N-((12S)-5⁴-methyl-8,11,14-trioxo-9-(quinolin-6-yl)-4-oxa-7,10-diaza-1(3,1)-piperidina-5(1,3)-benzenacyclotetradecaphane-12-yl)acetamide |
| **555** | 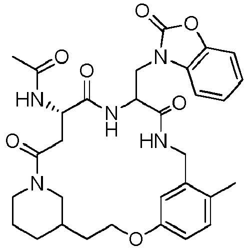 | N-((12S)-5⁴-methyl-8,11,14-trioxo-9-((2-oxobenzo[d]oxazol-3(2H)-yl)methyl)-4-oxa-7,10-diaza-1(3,1)-piperidina-5(1,3)-benzenacyclotetradecaphane-12-yl)acetamide |
| **556** | 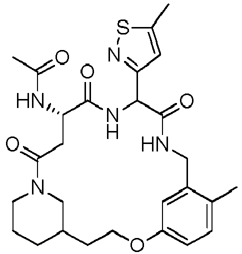 | N-((12S)-5⁴-methyl-9-(5-methylisothiazol-3-yl)-8,11,14-trioxo-4-oxa-7,10-diaza-1(3,1)-piperidina-5(1,3)-benzenacyclotetradecaphane-12-yl)acetamide |
| **557** | 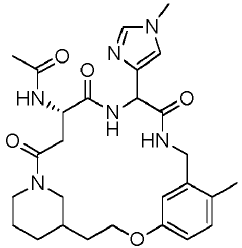 | N-((12S)-5⁴-methyl-9-(1-methyl-1H-imidazol-4-yl)-8,11,14-trioxo-4-oxa-7,10-diaza-1(3,1)-piperidina-5(1,3)-benzenacyclotetradecaphane-12-yl)acetamide |
| **558** | 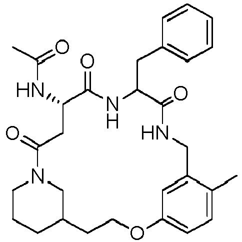 | N-((12S)-9-benzyl-5⁴-methyl-8,11,14-trioxo-4-oxa-7,10-diaza-1(3,1)-piperidina-5(1,3)-benzenacyclotetradecaphane-12-yl)acetamide |
| **559** | 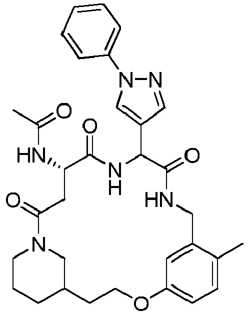 | N-((12S)-5⁴-methyl-8,11,14-trioxo-9-(1-phenyl-1H-pyrazol-4-yl)-4-oxa-7,10-diaza-1(3,1)-piperidina-5(1,3)-benzenacyclotetradecaphane-12-yl)acetamide |
| **560** | 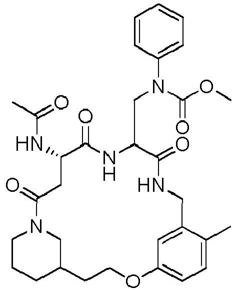 | methyl (((12S)-12-acetamido-5⁴-methyl-8,11,14-trioxo-4-oxa-7,10-diaza-1(3,1)-piperidina-5(1,3)-benzenacyclotetradecaphane-9-yl)methyl)(phenyl)carbam ate |
| **561** | 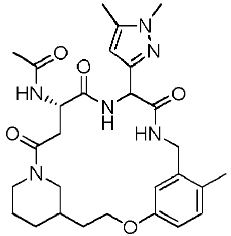 | N-((12S)-9-(1,5-dimethyl-1H-pyrazol-3-yl)-5⁴-methyl-8,11,14-trioxo-4-oxa-7, 10-diaza-1(3,1)-piperidina-5(1,3)-benzenacyclotetradecaphane-12-yl)acetamide |
| **562** | 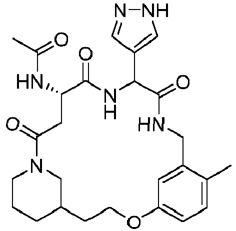 | N-((12S)-5⁴-methyl-8,11,14-trioxo-9-(1H-pyrazol-4-yl)-4-oxa-7,10-diaza-1(3,1)-piperidina-5(1,3)-benzenacyclotetradecaphane-12-yl)acetamide |
| **563** | 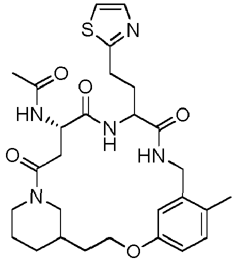 | N-((12S)-5⁴-methyl-8,11,14-trioxo-9-(2-(thiazol-2-yl)ethyl)-4-oxa-7,10-diaza-1(3,1)-piperidina-5(1,3)-benzenacyclotetradecaphane-12-yl)acetamide |
| **564** | 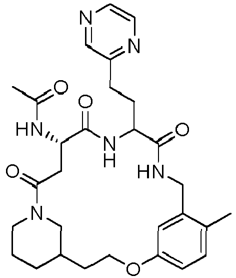 | N-((12S)-5⁴-methyl-8,11,14-trioxo-9-(2-(pyrazin-2-yl)ethyl)-4-oxa-7,10-diaza-1(3,1)-piperidina-5(1,3)-benzenacyclotetradecaphane-12-yl)acetamide |
| **565** | 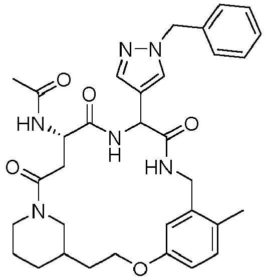 | N-((12S)-9-(1-benzyl-1H-pyrazol-4-yl)-5⁴-methyl-8,11,14-trioxo-4-oxa-7,10-diaza-1(3,1)-piperidina-5(1,3)-benzenacyclotetradecaphane-12-yl)acetamide |
| **566** | 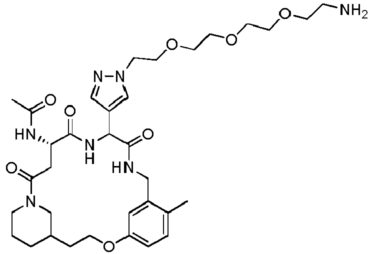 | N-((12S)-9-(1-(2-(2-(2-(2-aminoethoxy)ethoxy)ethoxy)ethyl)-1H-pyrazol-4-yl)-5⁴-methyl-8,11,14-trioxo-4-oxa-7,10-diaza-1(3,1)-piperidina-5(1,3)-benzenacyclotetradecaphane-12-yl)acetamide |
| **567** | 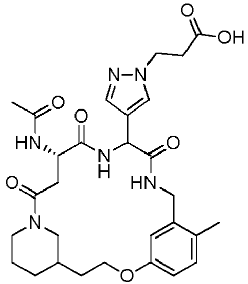 | 3-(4-((12S)-12-acetamido-5⁴-methyl-8,11,14-trioxo-4-oxa-7,10-diaza-1(3,1)-piperidina-5(1,3)-benzenacyclotetradecaphane-9-yl)-1H-pyrazol-1-yl)propanoic acid |
| **568** | 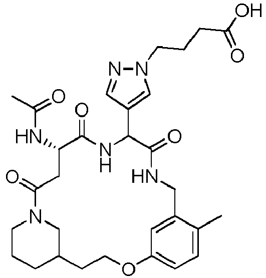 | 4-(4-((12S)-12-acetamido-5⁴-methyl-8,11,14-trioxo-4-oxa-7,10-diaza-1(3,1)-piperidina-5(1,3)-benzenacyclotetradecaphane-9-yl)-1H-pyrazol-1-yl)butanoic acid |
| **570** | 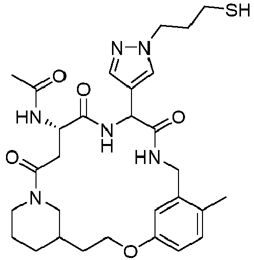 | N-((12S)-9-(1-(3-mercaptopropyl)-1H-pyrazol-4-yl)-5⁴-methyl-8,11,14-trioxo-4-oxa-7,10-diaza-1(3,1)-piperidina-5(1,3)-benzenacyclotetradecaphane-12-yl)acetamide |
| **571** | 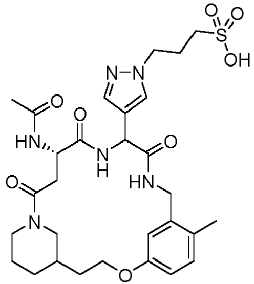 | 3-(4-((12S)-12-acetamido-5⁴-methyl-8,11,14-trioxo-4-oxa-7,10-diaza-1(3,1)-piperidina-5(1,3)-benzenacyclotetradecaphane-9-yl)-1H-pyrazol-1-yl)propane-1-sulfonic acid |
| **572** | 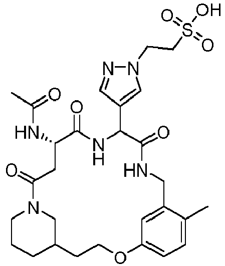 | N-(4-((12S)-12-acetamido-5⁴-methyl-8,11,14-trioxo-4-oxa-7,10-diaza-1(3,1)-piperidina-5(1,3)-benzenacyclotetradecaphane-9-yl)-1H-pyrazol-1-yl)ethane-1-sulfonic acid |

or an enantiomer, a stereoisomeric form, a mixture of enantiomers, a diastereomer, a mixture of diastereomers, a hydrate, a solvate, an acid salt form, a tautomer, a racemate of the above mentioned compounds, or a pharmaceutically acceptable salts thereof.

### Syntheses of compounds

The compound of formula (**I**) is prepared by reference to the methods illustrated in the following schemes 1-3 by suitable selection of reagents with appropriate substitutions. Solvents, temperatures, pressures, and other reaction conditions may readily be selected by one of ordinary skill in the art. Starting materials are commercially available or readily prepared by one of ordinary skill in the art.

Another aspect of the present invention is directed to a method for producing the compound of the formula (**I**) comprising:
**Step 1A:** providing an intermediate compound (**I-1***): 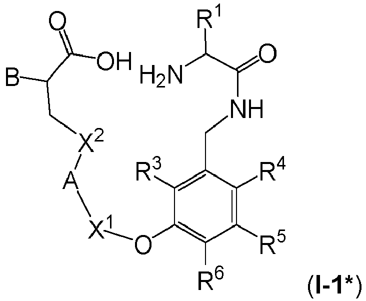 wherein
   **A**, **B**, **R¹**, **R³**, **R⁴**, **R⁵**, **R⁶** , **X¹**, and **X²** have the same meanings as defined in the formula (**I**);
**Step 2A**: performing an intramolecular amide coupling reaction between a carboxylic acid group and an amine group of the intermediate compound (**l-1***) to obtain the compound of the formula (**I**)

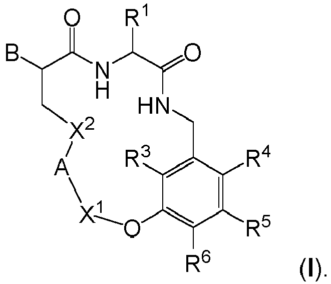

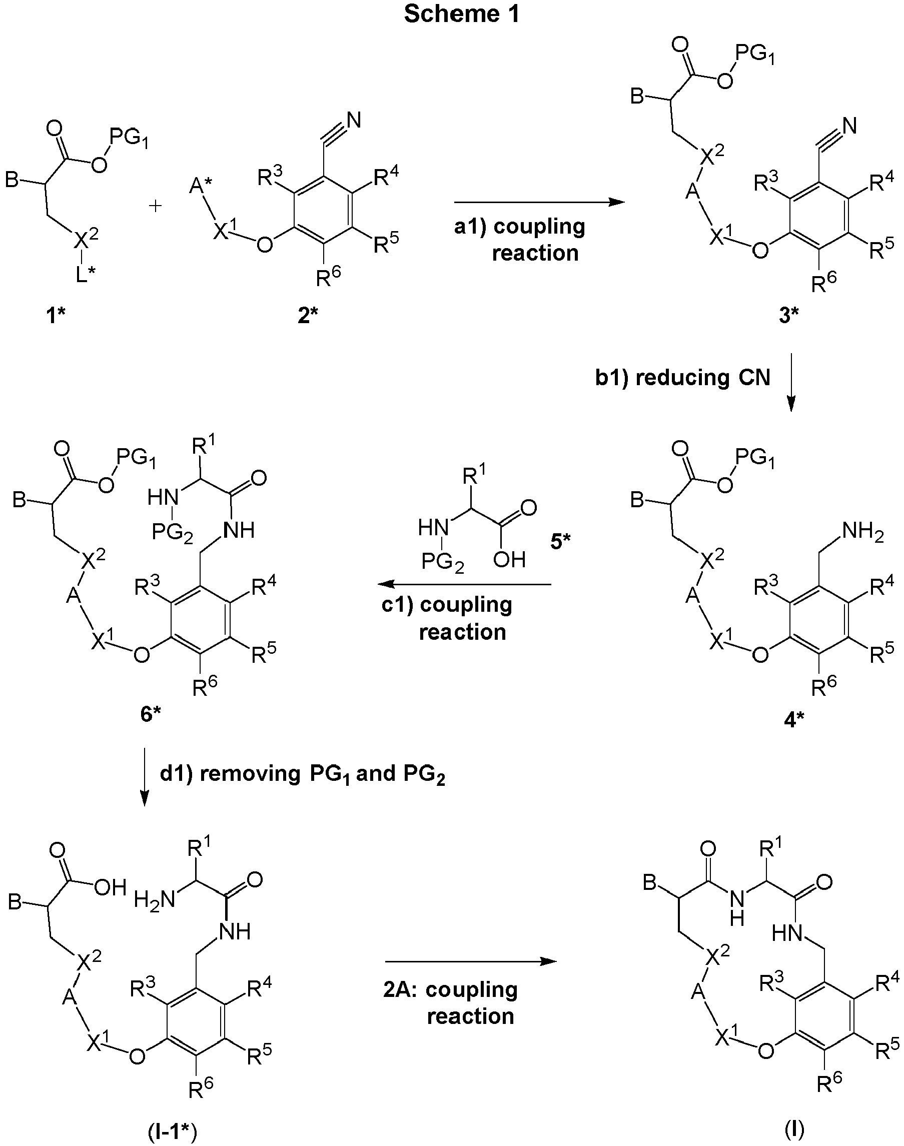

Optionally, the intermediate compound (**l-1***) of **Step 1A** is prepared by **Step 1A'**. **Step1A'** comprises the following steps **a1)** to **d1)**:
**a1**) performing a coupling reaction between a compound **1*** and a compound **2*** 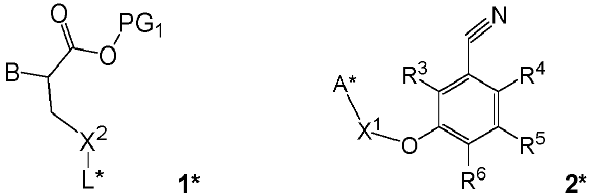 to obtain a compound **3*** 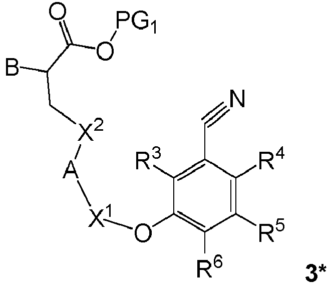
**b1)** reducing a nitril (-CN) group of the compound **3*** to an aminomethyl (-CH₂NH₂) group to obtain a compound **4*** 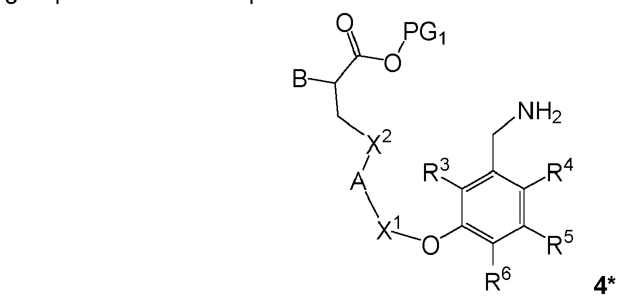
**c1)** performing a coupling reaction between the compound **4*** and a compound **5*** 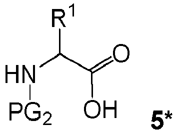 to obtain a compound **6*** 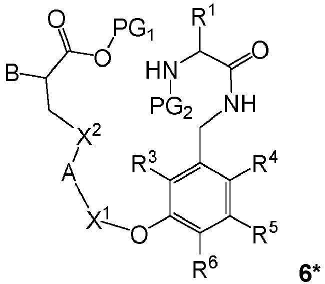
**d1)** removing a carboxyl protecting group PG₁ and an amine protecting group PG₂ to obtain a compound (**l-1***) 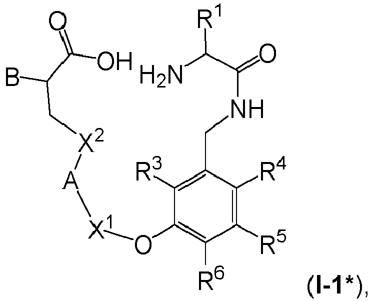 wherein
   **A*** represents -**NH(R^{N6})**-, 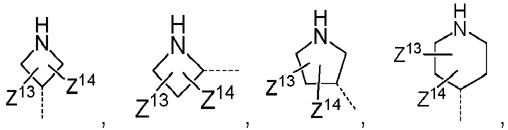 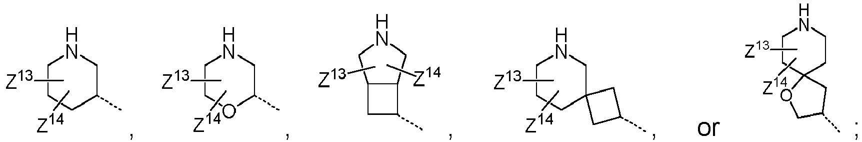
   **L*** represents -CO₂H;
   **PG₁** represents a carboxyl protecting group;
   **PG₂** represents an amine protecting group;
   and **A**, **B**, **R¹**, **R³**, **R⁴**, **R⁵**, **R⁶**, **R^{N6}**, **X¹**, **X²**, **Z¹³**, and **Z¹⁴** have the same meanings as defined in the formula (**I**).

Thus, the method for producing the compound of the formula (I) may comprise **Step 1A'**, **Step 1A**, and **Step 2A**.

Alternatively, the compound of the formula (I) is produced by the following method and thus the present invention refers to a method for producing the compound of the formula (I) comprising:
**Step 1B:** providing an intermediate compound (**I-2***): 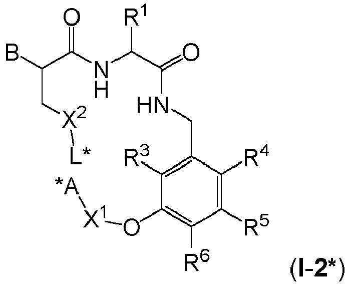 wherein
   **A*** represents -**NH(R^{N6})**-, 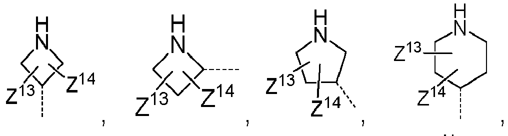 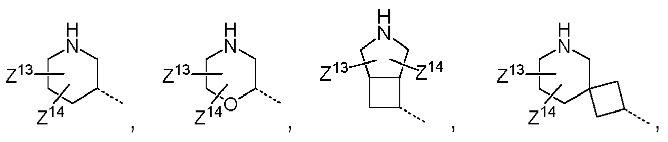 or 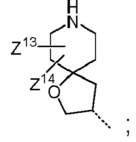
   **L*** represents -CO₂H,
   and **B**, **R¹**, **R²**, **R³**, **R⁴**, **R⁵**, **R⁶**, **R^{N6}**, **X¹**, **X²**, **Z¹³**, and **Z¹⁴** have the same meanings as defined in the formula (**I**);
**Step 2B**: perform an intramolecular amide coupling reaction between the **L*** and an amino group of **A*** moiety of the intermediated compound (**I-2***) to obtain the compound of the formula (**I**).

Optionally, the intermediate compound (**I-2***) of **Step 1B** is prepared by **Step 1B'**. **Step1B'** comprises the following steps **a2)** to **c2)**:
**a2**) performing a coupling reaction between a compound **5*** and a compound **7*** 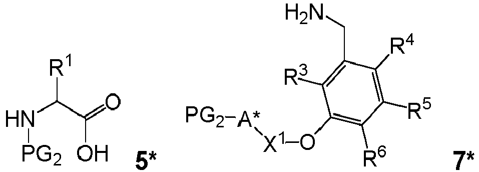 to obtain a compound **8*** 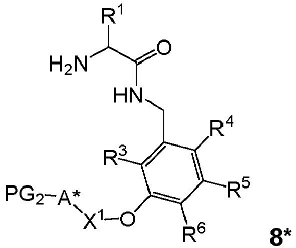
**b2**) perfoming a coupling reaction between the compound **8*** and a compound **1a*** 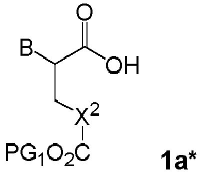 to obtain a compound **12*** 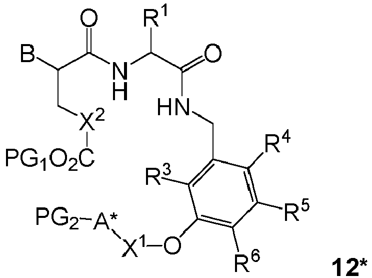
**c2**) removing a carboxyl protecting group PG₁ and an amine protecting group PG₂ of the compound **12*** to obtain a compound (**I-2***) 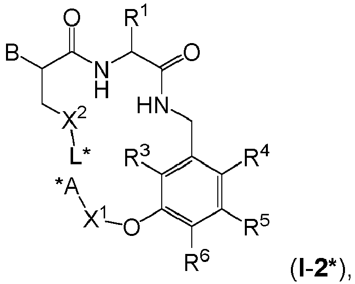 wherein
   **A*** represents -**NH(R^{N6})**- 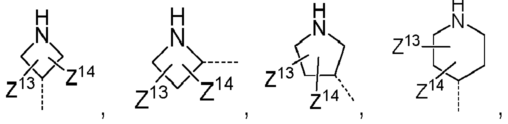 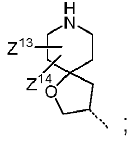 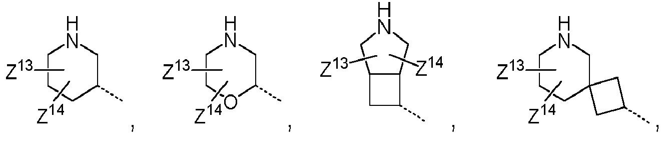 or
   **L*** represents -CO₂H,
   **PG₁** represents a carboxyl protecting group;
   **PG₂** represents an amine protecting group;
   and **B**, **R¹**, **R²**, **R³**, **R⁴**, **R⁵**, **R⁶**, **R^{N6}**, **X¹**, **X²**, **Z¹³**, and **Z¹⁴** have the same meanings as defined in the formula (**I**).

Thus, the method for producing the compound of the formula (**I**) may comprise **Step 1B'**, **Step 1B**, and **Step 2B**.

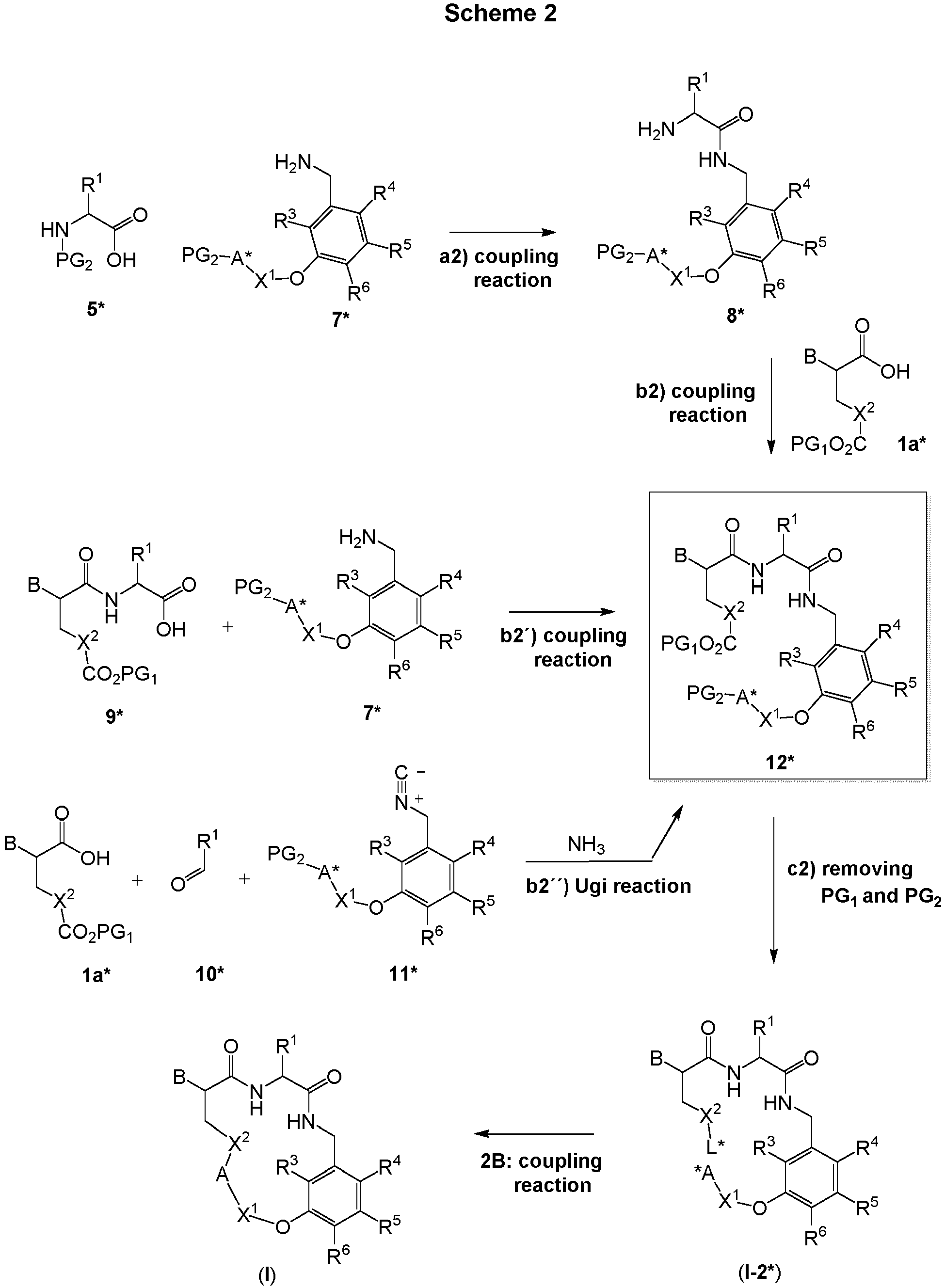

Alternatively, the intermediate compound (**I-2***) of **Step 1B** is prepared by **Step 1Bʺ**. **Step1Bʺ** comprises the following steps **b2')** and **c2)**:
**b2'**) performing a coupling reaction between the compound **7*** and a compound **9*** 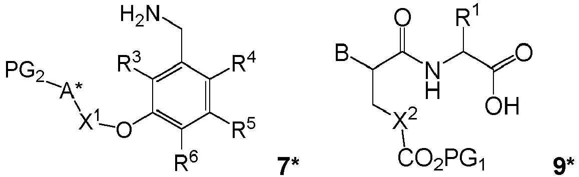 to obtain a compound **12*** 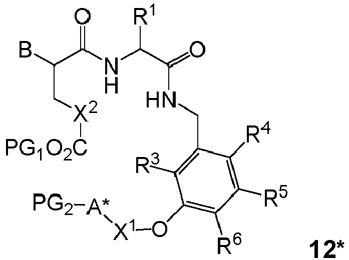
c2) removing a carboxyl protecting group PG₁ and an amine protecting group PG₂ of the compound **12*** to obtain a compound (**I-2***) 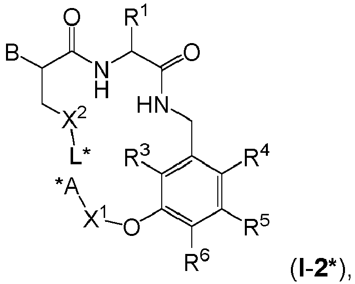 wherein
   **A*** represents -**NH(R^{N6})**- 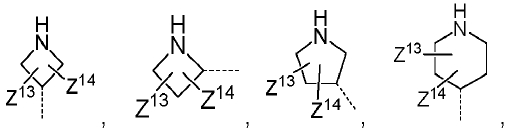 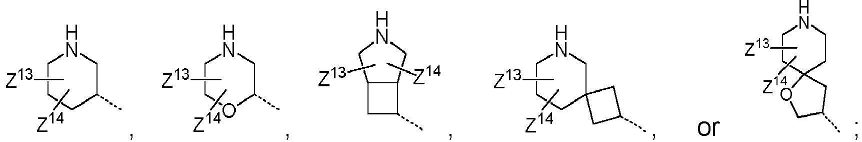
   **L*** represents -CO₂H,
   **PG₁** represents a carboxyl protecting group;
   **PG₂** represents an amine protecting group;
   and **B**, **R¹**, **R², R³, R⁴, R⁵, R⁶, R^{N6}, X¹, X², Z¹³**, and **Z¹⁴** have the same meanings as defined in the formula (I).

Thus, the method for producing the compound of the formula (I) may comprise **Step 1B", Step 1B,** and **Step 2B.**

Alternatively, the intermediate compound (**I-2***) of **Step 1B** is prepared by **Step 1B"'**. **Step1B"'**comprises the following steps **b2")** and **c2)**:
**b2")** performing a Ugi reaction of a compound **1a***, a compound **10a*** (R¹-CHO), a compound **11*** and aqueous ammonia (NH₃) 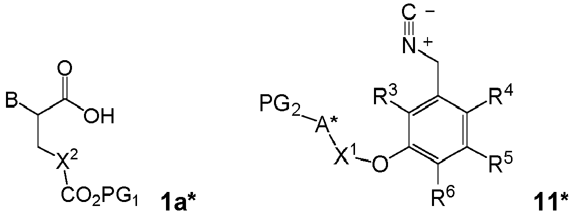 to obtain a compound **12*** 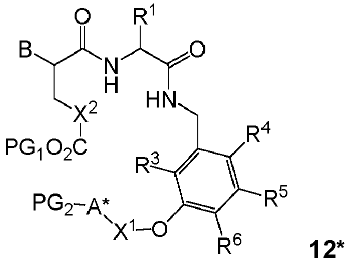
**c2**) removing a carboxyl protecting group PG₁ and an amine protecting group **PG₂** of the compound **12*** to obtain a compound (**I-2***) 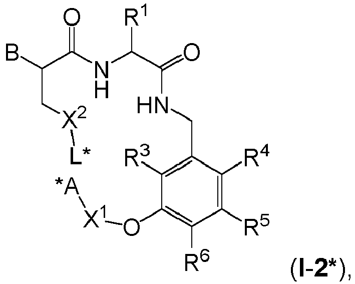 wherein
   **A*** represents -**NH(R^{N6})**-, 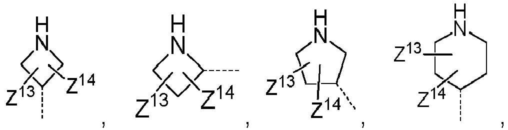 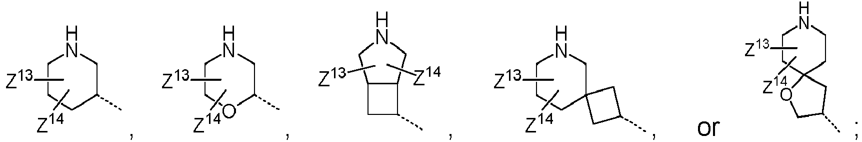
   **L*** represents -CO₂H,
   **PG₁** represents a carboxyl protecting group;
   **PG₂** represents an amine protecting group;
   and **B**, **R¹**, **R², R³, R⁴**, **R⁵**, **R⁶, R^{N6}, X¹**, **X²**, **Z¹³**, and **Z¹⁴** have the same meanings as defined in the formula (I).

Thus, the method for producing the compound of the formula (**I**) may comprise **Step 1B‴, Step 1B,** and **Step 2B.**

Preferably, the method for producing the compound of the formula (I) may comprise **any one of Steps 1B', 1B", and 1B"':**
**Step 1B',** comprising steps **a2), b2),** and **c2)**;
**Step 1B",** comprising steps **b2')** and **c2)**;
**Step 1B"',** comprising steps **b2")** and **c2)**;

**Step 1B,** and
**Step 2B.**

Alternatively, the compound of the formula (I) is produced by the following method and thus the present invention refers to a method for producing the compound of the formula (I) comprising:
**Step 1C:** providing an intermediate compound (**I-3***): 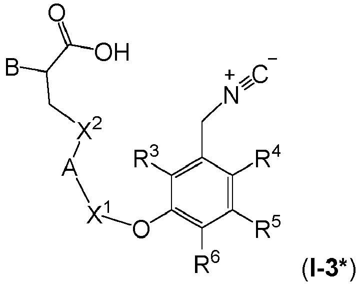 wherein
   **A, B, R¹, R³**, **R⁴**, **R⁵**, **R⁶**, **X¹**, and **X²** have the same meanings as defined in the formula (**I**);
**Step 2C**: perform an intramolecular Ugi reaction of the intermediate compound (**I-3***) with **R¹**-CHO **10*** and aqueous ammonia (NH₃) to obtain the compound of the formula (**I**).

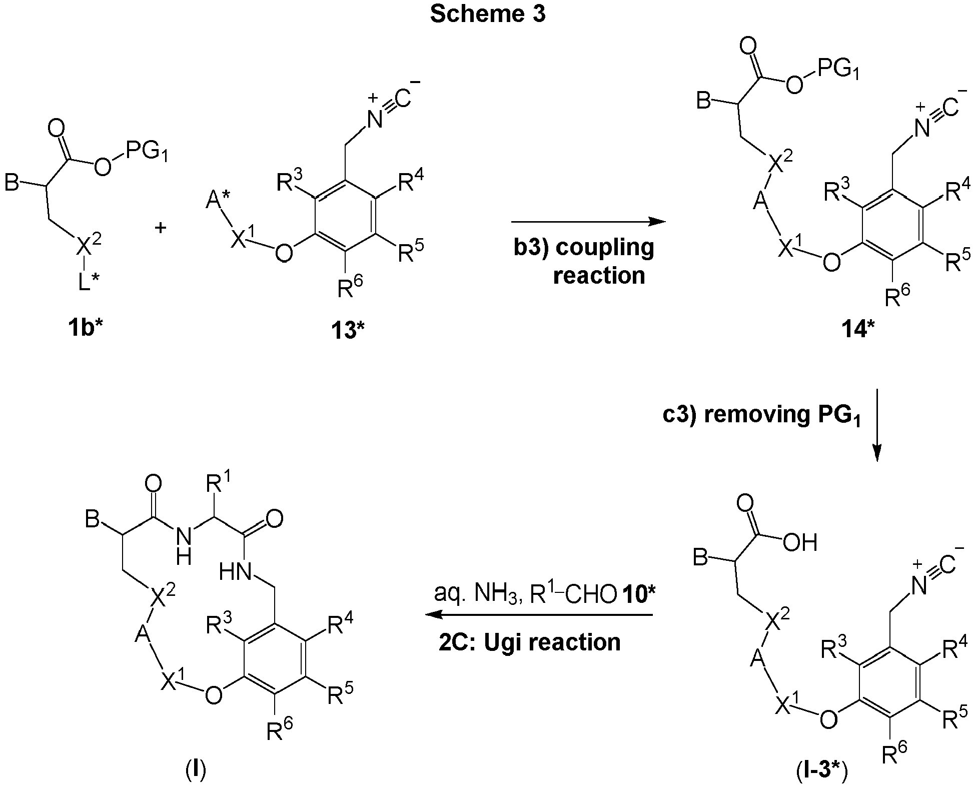

Optionally, the intermediate compound (**I-3***) of **Step 1C** is prepared by **Step 1C′**. **Step1C′** comprises the following steps **b3)** and **c3)**:
**b3)** performing a coupling reaction between the compound **1b*** and a compound **13*** 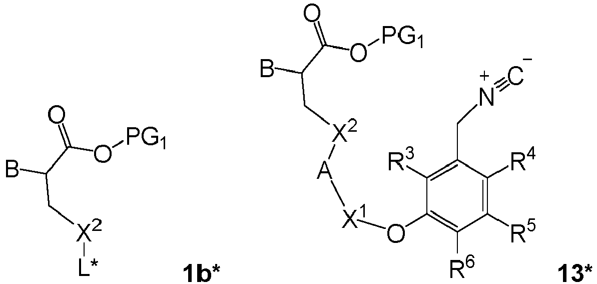 to obtain a compound **14*** 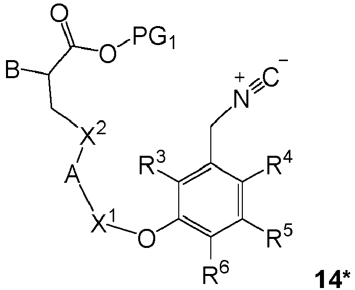
**c3**) removing a carboxyl protecting group PG₁ of the compound **14*** to obtain a compound (**I-3***) 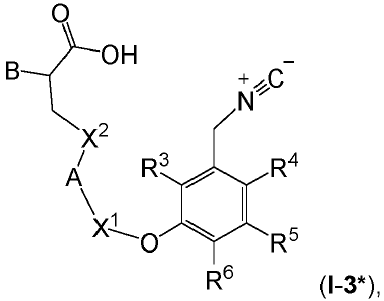 wherein
   **A*** represents -**NH(R^{N6})**-, 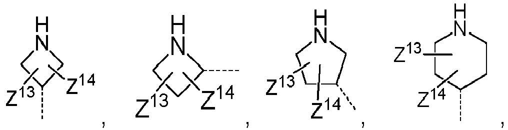 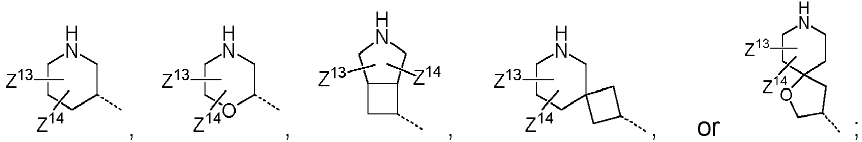
   **L*** represents -CO₂H,
   **PG₁** represents a carboxyl protecting group;
   and **B**, **R¹**, **R², R³**, **R⁴**, **R⁵**, **R⁶, R^{N6}, X¹**, **X²**, **Z¹³**, and **Z¹⁴** have the same meanings as defined in the formula (**I**).

Thus, the method for producing the compound of the formula (I) may comprise **Step 1C′, Step 1C,** and **Step 2C.**

In **Steps 2A,** and **2B** to promote the intramolecular amide coupling reaction between a carboxylic acid group and an amino group of intermediate compound (**I-1***) or (**I-2***), activating reagents are commonly used for activating carboxylic acid. The activation may be introduced separate reaction or *in situ* reaction. Preferably, any of the following coupling reagent can be used to activate carobxylic acid group: BOP (Benzotriazole-1-yl-oxy-tris-(dimethylamino)-phosphonium hexafluorophosphate), PyBOP (Benzotriazole-1-yl-oxy-tris-pyrrolidino-phosphonium hexafluorophosphate), AOP (7-(Azabenzotriazol-1-yl)oxy tris(dimethylamino)phosphonium hexafluorophosphate), PyAOP ((7-Azabenzotriazol-1-yloxy)trispyrrolidinophosphonium hexafluorophosphate), TBTU (2-(1H-Benzotriazole-1-yl)-1,1,3,3-tetramethylaminium tetrafluoroborate), EEDQ (*N*-Ethoxycarbonyl-2-ethoxy-1,2-dihydroquinoline), Polyphosphoric Acid (PPA), DPPA (Diphenyl phosphoryl azide), HATU (*N*-[(Dimethylamino)-1*H*-1,2,3-triazolo-[4,5-*b*]pyridin-1-ylmethylene]-*N*-methyl methanaminium hexafluorophosphate *N*-oxide), HBTU (*N*,*N*,*N'*,*N'*-Tetramethyl-*O*-(1*H-*benzotriazol-1-yl)uronium hexafluorophosphate), HOBt (1-Hydroxybenzotriazole), HOAt (1-Hydroxy-7-azabenzotriazole), DCC (*N*,*N'*-Dicyclohexylcarbodiimide), EDCI (*N*-Ethyl-*N'-*(3-dimethylaminopropyl)carbodiimide), BOP-Cl (Bis(2-oxo-3-oxazolidinyl)phosphinic chloride), TFFH (Tetramethylfluoroformamidinium hexafluorophosphate), Brop (Bromo tris(dimethylamino) phosphonium hexafluorophosphate), PyBrop (Bromo-tris-pyrrolidino-phosphonium hexafluorophosphate) and CIP (2-Chloro-1,3-dimethylimidazolidinium hexafluorophosphate) or a mixture thereof.

Preferably, the intramolecular amide coupling reaction between a carboxylic acid group and an amino group of intermediate compound (**I-1***) or (**I-2***) is performed in the presence of HATU as a coupling reagent and DIPEA as a base.

Another aspect of the present invention is directed to the intermediate compounds **7***, **8***, **11***, **12***, **13***, **14***, **I-1***, **I-2***, and **I-3***:

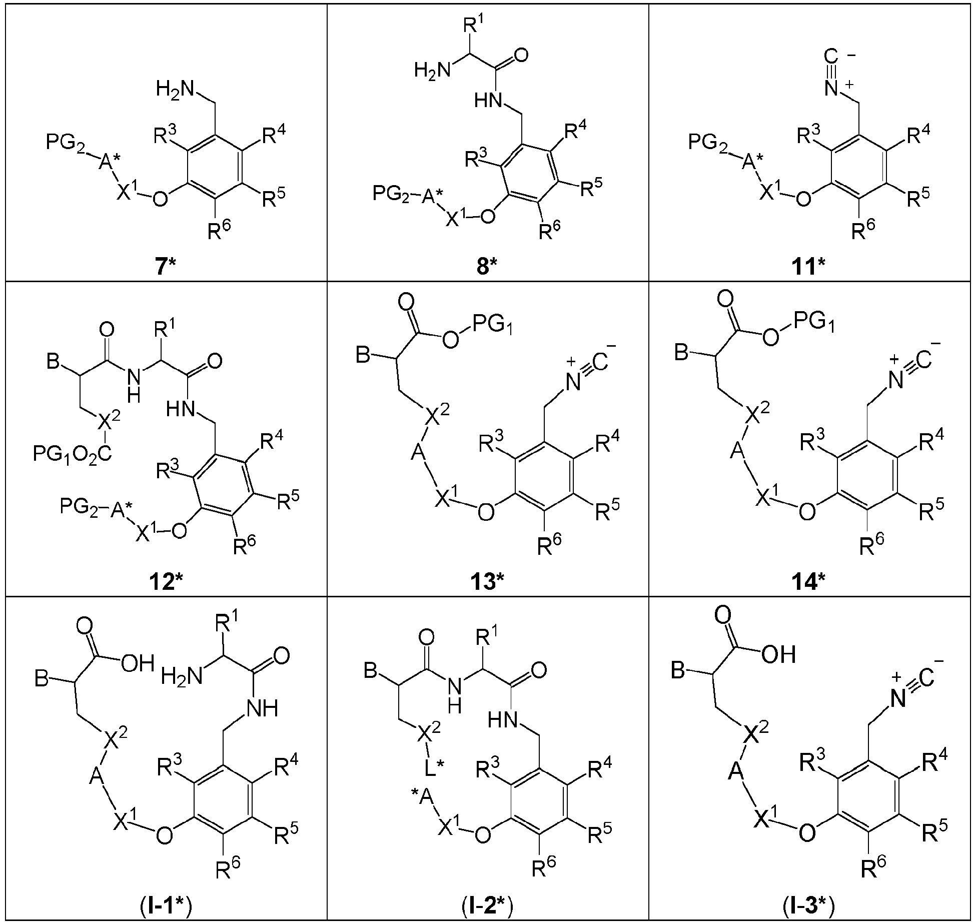

wherein
**A*** represents -NH(**R^{N6}**)-, 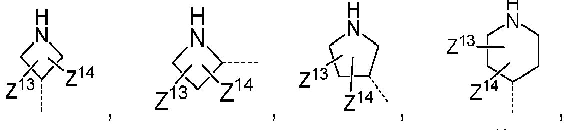 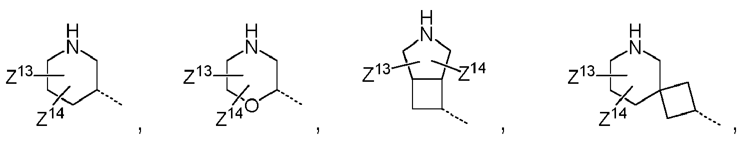 or 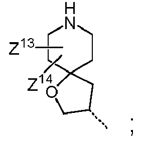
**L*** represents -CO₂H,
**PG₁** represents a carboxyl protecting group;
**PG₂** represents an amine protecting group;
and **B**, **R¹**, **R²**, **R³**, **R⁴**, **R⁵**, **R⁶, R^{N6}**, **X¹**, **X²**, **Z¹³**, and **Z¹⁴** have the same meanings as defined in the formula (I).

### Indication

In a further aspect of the present invention, the novel compounds according to the general formula (I) are used as pharmaceutically active agent.

Surprisingly it was found that the above-mentioned compounds of general formula (I), as well as the pharmaceutical compositions thereof are acting as proteasome inhibitors, and more specifically as inhibitors of proteasome subunit beta type-5.

In the present application, the inhibitory activity assays were performed to determine IC₅₀ values of compounds of general formula (I) against proteasome subunit beta type-5.

Table 2 show activity data (indicated as IC₅₀ values) in the biochemical assays. It is proven that the inventive compounds inhibit proteasome subunit beta type-5 of both the constitutive and immunoproteasome very effectively. In addition it is shown that the inventive compounds also inhibit the subunit beta type-5 of both the constitutive and immunoproteasome in the cell based proteasome glo assays very effectively. Finally it is shown that proteasome inhibition of these compounds translates into inhibition of proliferation of tumor cells (HT29) very effectively.

The pharmaceutical compositions according to the present invention comprise at least one compound according to the present invention as an active ingredient together with at least one pharmaceutically acceptable (i.e. non-toxic) carrier, excipient and/or diluent.

As used herein, proteasome "inhibitor", especially proteasome subunit beta type-5 "inhibitor" refers to any compound capable of downregulating, decreasing, suppressing or otherwise regulating the amount and/or activity of a proteasome can be achieved by any of a variety of mechanisms known in the art, including, but not limited to binding directly to said proteasome subunit beta type-5.

As used herein the term "inhibiting" or "inhibition" refers to the ability of a compound to downregulate, decrease, reduce, suppress, inactivate, or inhibit at least partially the activity of an enzyme, or the expression of an enzyme or protein.

Proteasome subunit beta type-5 is a protein that in humans is encoded by the *PSMB5* gene in case of the constitutive proteasome and by LMP7 in case of the immunoproteasome. Proteasome subunit beta type-5, along with other beta subunits, assembles into two heptameric rings and subsequently a proteolytic chamber for substrate degradation. This protein contains "chymotrypsin-like" activity and is capable of cleaving after large hydrophobic residues of peptide. The eukaryotic proteasome recognized degradable proteins, including damaged proteins for protein quality control purpose or key regulatory protein components for dynamic biological processes. An essential function of a modified proteasome, the immunoproteasome, is the processing of class I MHC peptides.

Further aspects of the present invention relate to the compound of the general formula (I), or the above-mentioned pharmaceutical composition, for use in prophylaxis and/or treatment of diseases caused by or associated with proteasome, or immunoproteasome, in particular, proteasome subunit beta type-5, selected from a cancer, an infectious disease, an inflammatory disease, autoimmune disease and transplant rejection.

In a further aspect of the present invention, methods for preventing and/or treating diseases caused by or associated with proteasome, or immunoproteasome, in particular, proteasome subunit beta type-5 in a mammal, especially in a human, are provided, which methods comprise administering to the mammal an amount of at least one compound according to the general formula (I) and/or pharmaceutically acceptable salts thereof, effective to prevent and/or treat the diseases caused by or associated with proteasome, or immunoproteasome, in particular, proteasome subunit beta type-5 selected from a cancer, a neurodegenerative disease, an infectious disease, an inflammatory disease, autoimmune disease and to supress allograft rejection during transplantation.

The term "effective amount" means an amount of compound that, when administered to a patient in need of such treatment, is sufficient to
(i) treat or prevent a particular disease, condition, or disorder which can be treated with a proteasome inhibitor;
(ii) attenuate, ameliorate, or eliminate one or more symptoms of the particular disease, condition, or disorder; or
(iii) prevent or delay the onset of one or more symptoms of the particular disease, condition, or disorder described herein.

The amount of a compound of general formula (I) that will correspond to such an amount will vary depending upon factors such as the particular compound, disease condition and its severity, the identity (e.g., weight) of the patient in need of treatment, but can nevertheless be routinely determined by one skilled in the art.

### Cancer

The compounds of the present application or the pharmaceutical composition thereof are useful for the treatment and/or prophylaxis of cancer, wherein the cancer is selected from the group consisting of: adenocarcinoma, choroidal melanoma, acute leukemia, acoustic neurinoma, ampullary carcinoma, anal carcinoma, astrocytoma, basal cell carcinoma, pancreatic cancer, desmoid tumour, bladder cancer, bronchial carcinoma, non-small cell lung cancer (NSCLC), breast cancer, Burkitt's lymphoma, corpus cancer, CUP-syndrome (carcinoma of unknown primary), colorectal cancer, small intestine cancer, small intestinal tumours, ovarian cancer, endometrial carcinoma, ependymoma, epithelial cancer types, Ewing's tumours, gastrointestinal tumours, gastric cancer, gallbladder cancer, gall bladder carcinomas, uterine cancer, cervical cancer, cervix, glioblastomas, gynecologic tumours, ear, nose and throat tumours, hematologic neoplasias, hairy cell leukemia, urethral cancer, skin cancer, skin testis cancer, brain tumours (gliomas), brain metastases, testicle cancer, hypophysis tumour, carcinoids, Kaposi's sarcoma, laryngeal cancer, germ cell tumour, bone cancer, colorectal carcinoma, head and neck tumours (tumours of the ear, nose and throat area), colon carcinoma, craniopharyngiomas, oral cancer (cancer in the mouth area and on lips), cancer of the central nervous system, liver cancer, liver metastases, leukemia, eyelid tumor, lung cancer, lymph node cancer (Hodgkin's/Non-Hodgkin's), lymphomas, stomach cancer, malignant melanoma, malignant neoplasia, malignant tumours gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, esophageal carcinoma, osteolytic carcinomas and osteoplastic carcinomas, osteosarcomas, ovarial carcinoma, pancreatic carcinoma, penile cancer, plasmocytoma, squamous cell carcinoma of the head and neck (SCCHN), prostate cancer, pharyngeal cancer, rectal carcinoma, retinoblastoma, vaginal cancer, thyroid carcinoma, Schneeberger disease, esophageal cancer, spinalioms, T-cell lymphoma (mycosis fungoides), thymoma, tube carcinoma, eye tumours, urethral cancer, urologic tumours, urothelial carcinoma, vulva cancer, wart appearance, soft tissue tumours, soft tissue sarcoma, Wilm's tumour, cervical carcinoma and tongue cancer. Particularly suitable for treatment are, for example, astrocytomas, glioblastomas, pancreatic cancer, bronchial cancer, breast cancer, colorectal cancer, ovarian cancer, gastric cancer, laryngeal cancer, malignant melanoma, oesophageal cancer, cervical cancer, liver cancer, bladder cancer, cholangiocarcinoma, and renal cell cancer.

Preferrably, the present application or the pharmaceutical composition thereof are useful for the treatment and/or prophylaxis of cancer, wherein the cancer is leukemia, multiple myeloma, mantle-cell lymphoma (MCL), breast cancer, colorectal cancer, non-small cell lung cancer or ovarian cancer.

Optionally, the compound of the present invention is used for treatment / prophylaxis of said cancer in combination with a thalidomide or a derivative thereof.

The derivative of thalidomide is selected from lenalidomide, pomalidomide, avadomide, and iberdomide and CC-885.

### Infectious disease

The compounds of the present application or the pharmaceutical composition thereof are useful for the treatment and/or prophylaxis of the infectious disease, wherein the infectious disease is selected from the group consisting of: HIV, Echinococcosis, Amebiasis (Entamoeba histolytica Infection), Angiostrongylus Infection, Anisakiasis, Anthrax, Babesiosis (Babesia Infection), Balantidium Infection (Balantidiasis), Baylisascaris Infection (Raccoon Roundworm), Bilharzia (Schistosomiasis), Blastocystis hominis Infection (Blastomycosis), Borreliosis, Botulism, Brainerd Diarrhea, Brucellosis, BSE (Bovine Spongiform Encephalopathy), Candidiasis, Capillariasis (Capillaria Infection), CFS (Chronic Fatigue Syndrome), Chagas Disease (American Trypanosomiasis), Chickenpox (Varicella-Zoster virus), Chlamydia pneumoniae Infection, Cholera, CJD (Creutzfeldt-Jakob Disease), Clonorchiasis (Clonorchis Infection), CLM (Cutaneous Larva Migrans, Hookworm Infection), Coccidioidomycosis, Conjunctivitis, Coxsackievirus A16 (Hand, Foot and Mouth Disease), Cryptococcosis, Cryptosporidium Infection (Cryptosporidiosis), Culex mosquito (Vector of West Nile Virus), Cyclosporiasis (Cyclospora Infection), Cysticercosis (Neurocysticercosis), Cytomegalovirus Infection, Dengue / Dengue Fever, Dipylidium Infection (Dog and Cat Flea Tapeworm), Ebola Virus Hemorrhagic Fever, Echinococcosis (Alveolar Hydatid Disease), Encephalitis, Entamoeba coli Infection, Entamoeba dispar Infection, Entamoeba hartmanni Infection, Entamoeba histolytica Infection (Amebiasis), Entamoeba polecki Infection, Enterobiasis (Pinworm Infection), Enterovirus Infection (non-polio), Epstein-Barr Virus Infection, Escherichia coli Infection, Foodborne Infection, Foot and mouth Disease, Fungal Dermatitis, Gastroenteritis, Group A streptococcal Disease, Group B streptococcal Disease, Hansen's Disease (Leprosy), Hantavirus Pulmonary Syndrome, Head Lice Infestation (Pediculosis), Helicobacter pylori Infection, Hematologic Disease, Hendra Virus Infection, Hepatitis (HCV, HBV), Herpes Zoster (Shingles), Human Ehrlichiosis, Human Parainfluenza Virus Infection, Influenza, Isosporiasis (Isospora Infection), Lassa Fever, Leishmaniasis, Kala-azar (Kala-azar, Leishmania Infection), Leprosy, Lice (Body lice, Head lice, Pubic lice), Lyme Disease, Malaria, Marburg Hemorrhagic Fever, Measles, Meningitis, Mosquito-borne Diseases, Mycobacterium avium Complex (MAC) Infection, Naegleria Infection, Nosocomial Infections, Nonpathogenic Intestinal Amebae Infection, Onchocerciasis (River Blindness), Opisthorciasis (Opisthorcis Infection), Parvovirus Infection, Plague, PCP (Pneumocystis carinii Pneumonia), Polio, Q Fever, Rabies, Respiratory Syncytial Virus (RSV) Infection, Rheumatic Fever, Rift Valley Fever, Rotavirus Infection, Roundworms Infection, Salmonellosis, Salmonella Enteritidis, Scabies, Shigellosis, Shingles, Sleeping Sickness, Smallpox, Streptococcal Infection, Tapeworm Infection (Taenia Infection), Tetanus, Toxic Shock Syndrome, Tuberculosis, Ulcers (Peptic Ulcer Disease), Valley Fever, Vibrio parahaemolyticus Infection, Vibrio vulnificus Infection, Viral Hemorrhagic Fever, Warts, Waterborne infectious Diseases, West Nile Virus Infection (West Nile Encephalitis), Whooping Cough, Yellow Fever.

### Inflammatory disease

Inflammatory disease is caused by .cytokines, TNF-α, IL-1ß, GM-CSF, IL-6/IL-8, adhesion molecules (ICAM-1, VCAM-1, P-selectin) and prostaglandins and/or nitric oxide (NO).

### Autoimmune disease

Furthermore, the compounds of the present application or the pharmaceutical composition thereof are useful for the treatment and/or prophylaxis of autoimmune disease. The autoimmune disease is selected from the group consisting of:
Achalasia, Addison's disease, Adult Still's disease, Agammaglobulinemia, Alopecia areata, Amyloidosis, Ankylosing spondylitis, Anti-GBM/Anti-TBM nephritis, Antiphospholipid syndrome, Autoimmune angioedema, Autoimmune dysautonomia, Autoimmune encephalomyelitis, Autoimmune hepatitis, Autoimmune inner ear disease (AIED), Autoimmune myocarditis, Autoimmune oophoritis, Autoimmune orchitis, Autoimmune pancreatitis, Autoimmune retinopathy, Autoimmune urticaria, Axonal & neuronal neuropathy (AMAN), Baló disease, Behcet's disease, Benign mucosal pemphigoid, Bullous pemphigoid, Castleman disease (CD), Celiac disease, Chagas disease, Chronic inflammatory demyelinating polyneuropathy (CIDP), Chronic recurrent multifocal osteomyelitis (CRMO), Churg-Strauss Syndrome (CSS), Eosinophilic Granulomatosis (EGPA), Cicatricial pemphigoid, Cogan's syndrome, Cold agglutinin disease, Congenital heart block, Coxsackie myocarditis, CREST syndrome, Crohn's disease, Dermatitis herpetiformis, Dermatomyositis, Devic's disease (neuromyelitis optica), Discoid lupus, Dressler's syndrome, Endometriosis, Eosinophilic esophagitis (EoE), Eosinophilic fasciitis, Erythema nodosum, Essential mixed cryoglobulinemia, Evans syndrome, Fibromyalgia, Fibrosing alveolitis, Giant cell arteritis (temporal arteritis), Giant cell myocarditis, Glomerulonephritis, Goodpasture's syndrome, Granulomatosis with Polyangiitis, Graves' disease, Guillain-Barre syndrome, Hashimoto's thyroiditis, Hemolytic anemia, Henoch-Schonlein purpura (HSP), Herpes gestationis or pemphigoid gestationis (PG), Hidradenitis Suppurativa (HS) (Acne Inversa), Hypogammalglobulinemia, IgA Nephropathy, IgG4-related sclerosing disease, Immune thrombocytopenic purpura (ITP), Inclusion body myositis (IBM), Interstitial cystitis (IC), Juvenile arthritis, Juvenile diabetes (Type 1 diabetes), Juvenile myositis (JM), Kawasaki disease, Lambert-Eaton syndrome, Leukocytoclastic vasculitis, Lichen planus, Lichen sclerosus, Ligneous conjunctivitis, Linear IgA disease (LAD), Lupus, Lyme disease chronic, Meniere's disease, Microscopic polyangiitis (MPA), Mixed connective tissue disease (MCTD), Mooren's ulcer, Mucha-Habermann disease, Multifocal Motor Neuropathy (MMN) or MMNCB, Multiple sclerosis, Myasthenia gravis, Myelin Oligodendrocyte Glycoprotein Antibody Disorder, Myositis, Narcolepsy, Neonatal Lupus, Neuromyelitis optica, Neutropenia, Ocular cicatricial pemphigoid, Optic neuritis, Palindromic rheumatism (PR), PANDAS, Paraneoplastic cerebellar degeneration (PCD), Paroxysmal nocturnal hemoglobinuria (PNH), Parry Romberg syndrome, Pars planitis (peripheral uveitis), Parsonage-Turner syndrome, Pemphigus, Peripheral neuropathy, Perivenous encephalomyelitis, Pernicious anemia (PA), POEMS syndrome, Polyarteritis nodosa, Polyglandular syndromes type I, II, III, Polymyalgia rheumatic, Polymyositis, Postmyocardial infarction syndrome, Postpericardiotomy syndrome, Primary Biliary Cholangitis, Primary sclerosing cholangitis, Progesterone dermatitis, Psoriasis, Psoriatic arthritis, Pure red cell aplasia (PRCA), Pyoderma gangrenosum, Raynaud's phenomenon, Reactive Arthritis, Reflex sympathetic dystrophy, Relapsing polychondritis, Restless legs syndrome (RLS), Retroperitoneal fibrosis, Rheumatic fever, Rheumatoid arthritis, Sarcoidosis, Schmidt syndrome, Scleritis, Scleroderma, Sjögren's syndrome, Sperm & testicular autoimmunity, Stiff person syndrome (SPS), Subacute bacterial endocarditis (SBE), Susac's syndrome, Sympathetic ophthalmia (SO), Takayasu's arteritis, Temporal arteritis/Giant cell arteritis, Thrombocytopenic purpura (TTP), Thyroid eye disease (TED), Tolosa-Hunt syndrome (THS), Transverse myelitis, Type 1 diabetes, Ulcerative colitis (UC), Undifferentiated connective tissue disease (UCTD), Uveitis, Vasculitis, Vitiligo, Vogt-Koyanagi-Harada Disease.

Preferred, the autoimmune disease is selected from Lupus nephritis, lupus, systemic lupus erythematosus, myasthenia gravis, multiple sclerosis, polyarthritis, rheumatoid arthritis, irritant sensitivity, psoriasis, asthma, and colitis.

**Transplant rejection** occurs when transplanted tissue is rejected by the recipient's immune system, which destroys the transplanted tissue. Transplant rejection can be lessened by determining the molecular similitude between donor and recipient and by use of immunosuppressant drugs after transplant. The compound of the present invention may be used as immunosuppressant drugs.

The compounds enlisted explicitly in Table 1 are preferred to be used within the methods or indications disclosed herein.

The pharmaceutical compositions of the present invention can be prepared in a conventional solid or liquid carrier or diluent and a conventional pharmaceutically-made adjuvant at suitable dosage level in a known way. The preferred preparations are adapted for oral application. These administration forms include, for example, pills, tablets, film tablets, coated tablets, capsules, powders and deposits.

Furthermore, the present invention also includes pharmaceutical preparations for parenteral application, including dermal, intradermal, intragastral, intracutan, intravasal, intravenous, intramuscular, intraperitoneal, intranasal, intravaginal, intrabuccal, percutan, rectal, subcutaneous, sublingual, topical, or transdermal application, which preparations in addition to typical vehicles and/or diluents contain at least one compound according to the present invention and/or a pharmaceutical acceptable salt thereof as active ingredient.

The pharmaceutical compositions according to the present invention containing at least one compound according to the present invention and/or a pharmaceutical acceptable salt thereof as active ingredient will typically be administered together with suitable carrier materials selected with respect to the intended form of administration, i.e. for oral administration in the form of tablets, capsules (either solid filled, semi-solid filled or liquid filled), powders for constitution, gels, elixirs, dispersable granules, syrups, suspensions, and the like, and consistent with conventional pharmaceutical practices. For example, for oral administration in the form of tablets or capsules, the active drug component may be combined with any oral non-toxic pharmaceutically acceptable carrier, preferably with an inert carrier like lactose, starch, sucrose, cellulose, magnesium stearate, dicalcium phosphate, calcium sulfate, talc, mannitol, ethyl alcohol (liquid filled capsules) and the like. Moreover, suitable binders, lubricants, disintegrating agents and coloring agents may also be incorporated into the tablet or capsule. Powders and tablets may contain about 5 to about 95-weight % of the compounds of the formula (I) or the respective pharmaceutically active salt as active ingredient.

Suitable binders include starch, gelatin, natural sugars, corn sweeteners, natural and synthetic gums such as acacia, sodium alginate, carboxymethylcellulose, polyethylene glycol and waxes. Among suitable lubricants there may be mentioned boric acid, sodium benzoate, sodium acetate, sodium chloride, and the like. Suitable disintegrants include starch, methylcellulose, guar gum, and the like. Sweetening and flavoring agents as well as preservatives may also be included, where appropriate. The disintegrants, diluents, lubricants, binders etc. are discussed in more detail below.

Moreover, the pharmaceutical compositions of the present invention may be formulated in sustained release form to provide the rate controlled release of any one or more of the components or active ingredients to optimise the therapeutic effect(s), e.g. antihistaminic activity and the like. Suitable dosage forms for sustained release include tablets having layers of varying disintegration rates or controlled release polymeric matrices impregnated with the active components and shaped in tablet form or capsules containing such impregnated or encapsulated porous polymeric matrices.

Liquid form preparations include solutions, suspensions, and emulsions. As an example, there may be mentioned water or water/propylene glycol solutions for parenteral injections or addition of sweeteners and opacifiers for oral solutions, suspensions, and emulsions. Liquid form preparations may also include solutions for intranasal administration.

Aerosol preparations suitable for inhalation may include solutions and solids in powder form, which may be present in combination with a pharmaceutically acceptable carrier such as an inert, compressed gas, e.g. nitrogen.

For preparing suppositories, a low melting wax, such as a mixture of fatty acid glycerides like cocoa butter is melted first, and the active ingredient is then dispersed homogeneously therein e.g. by stirring. The molten, homogeneous mixture is then poured into conveniently sized moulds, allowed to cool, and thereby solidified.
Also included are solid form preparations, which are intended to be converted, shortly before use, to liquid form preparations for either oral or parenteral administration. Such liquid forms include solutions, suspensions, and emulsions.

The compounds according to the present invention may also be delivered transdermally. The transdermal compositions may have the form of a cream, a lotion, an aerosol and/or an emulsion and may be included in a transdermal patch of the matrix or reservoir type as is known in the art for this purpose.

The term capsule as recited herein refers to a specific container or enclosure made e.g. of methylcellulose, polyvinyl alcohols, or denatured gelatins or starch for holding or containing compositions comprising the active ingredient(s). Capsules with hard shells are typically made of blended of relatively high gel strength gelatins from bones or pork skin. The capsule itself may contain small amounts of dyes, opaquing agents, plasticisers and/or preservatives.

Under tablet a compressed or moulded solid dosage form is understood which comprises the active ingredients with suitable diluents. The tablet may be prepared by compression of mixtures or granulations obtained by wet granulation, dry granulation, or by compaction well known to a person of ordinary skill in the art.
Oral gels refer to the active ingredients dispersed or solubilised in a hydrophilic semi-solid matrix.
Powders for constitution refers to powder blends containing the active ingredients and suitable diluents which can be suspended e.g. in water or in juice.

Suitable diluents are substances that usually make up the major portion of the composition or dosage form. Suitable diluents include sugars such as lactose, sucrose, mannitol, and sorbitol, starches derived from wheat, corn rice, and potato, and celluloses such as microcrystalline cellulose. The amount of diluent in the composition can range from about 5 to about 95 % by weight of the total composition, preferably from about 25 to about 75 weight %, and more preferably from about 30 to about 60 weight %.

The term disintegrants refers to materials added to the composition to support break apart (disintegrate) and release the pharmaceutically active ingredients of a medicament. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses, and cross-linked microcrystalline celluloses such as sodium croscaramellose, alginates such as alginic acid and sodium alginate, clays such as bentonites, and effervescent mixtures. The amount of disintegrant in the composition may range from about 2 to about 20 weight % of the composition, more preferably from about 5 to ca. 10 weight %.

Binders are substances which bind or "glue" together powder particles and make them cohesive by forming granules, thus serving as the "adhesive" in the formulation. Binders add cohesive strength already available in the diluent or bulking agent. Suitable binders include sugars such as sucrose, starches derived from wheat corn rice and potato, natural gums such as acacia, gelatin and tragacanth, derivatives of seaweed such as alginic acid, sodium alginate and ammonium calcium alginate, cellulose materials such as methylcellulose, sodium carboxymethylcellulose and hydroxypropylmethylcellulose, polyvinylpyrrolidone, and inorganic compounds such as magnesium aluminum silicate. The amount of binder in the composition may range from about 2 to about 20 weight % of the composition, preferably from about 3 to about 10 weight %, and more preferably from about 3 to about 6 weight %.

Lubricants refer to a class of substances which are added to the dosage form to enable the tablet granules etc. after being compressed to release from the mould or die by reducing friction or wear. Suitable lubricants include metallic stearates such as magnesium stearate, calcium stearate, or potassium stearate, stearic acid, high melting point waxes, and other water soluble lubricants such as sodium chloride, sodium benzoate, sodium acetate, sodium oleate, polyethylene glycols and D,L-leucine. Lubricants are usually added at the very last step before compression, since they must be present at the surface of the granules. The amount of lubricant in the composition may range from about 0.2 to about 5 weight % of the composition, preferably from about 0.5 to about 2 weight %, and more preferably from about 0.3 to about 1.5 weight % of the composition.

Glidents are materials that prevent caking of the components of the pharmaceutical composition and improve the flow characteristics of granulate so that flow is smooth and uniform. Suitable glidents include silicon dioxide and talc. The amount of glident in the composition may range from about 0.1 to about 5 weight % of the final composition, preferably from about 0.5 to about 2 weight %.

Coloring agents are excipients that provide coloration to the composition or the dosage form. Such excipients can include food grade dyes adsorbed onto a suitable adsorbent such as clay or aluminum oxide. The amount of the coloring agent may vary from about 0.1 to about 5 weight % of the composition, preferably from about 0.1 to about 1 weight %.

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

### Examples

### Preparation of compounds:

### General Information:

All reactions involving air- or moisture-sensitive reagents or intermediates were carried out in flame-dried glassware under an argon atmosphere. Dry solvents (THF, toluene, MeOH, DMF, DCM) were used as commercially available. ¹H-NMR and ¹³C-NMR were recorded on a Bruker DRX400 (400 MHz). Multiplicities are indicated as: br s (broadened singlet), s (singlet), d (doublet), t (triplet), q (quartet), quin (quintet), m (multiplet); and coupling constants (J) are given in Hertz (Hz). HPLC - electrospray mass spectra (HPLC ES-MS) were obtained using Waters Acquity Performance Liquid Chromatography (UPLC) equipped SQ 3100 Mass detector spectrometer. Column: Acquity UPLC BEH C18 1.7um, 2.1x50mm. Flow: 0.5ml/min. Eluents: A: H₂O with 0.05% formic acid and B: ACN with 0.05% TFA. All chemicals and solvents were purchased from commercial sources like Sigma-Aldrich, Fluka, TCI, Acros Organics, ABCR, Alfa Aesar, Enamine, VWR, Combi-Blocks, Apollo Scientific, Aquilla Pharmatech, Ark Pharm, D-L Chiral Chemicals, ChemBridge, Renno Tech, Accela, KeyOrganics, Pharmablock and Chem Impex. Unless otherwise noted, all commercially available compounds were used as received without further purifications.

### Abbreviations used in the description of the chemistry and in the Examples that follow are:

ACN or MeCN (acetonitrile); Asp (aspartic acid), br (broad); BOC (tet-butyloxycarbonyl), Cbz (benzyloxycarbonyl), CDCl₃ (deuterated chloroform); cHex (cyclohexane); DBU (1,8-diazabicyclo[5.4.0]undec-7-ene), DCM (dichloromethane); DIAD (diisopropyl azodicarboxylate); DIPEA (di-iso-propylethylamine); DMF (dimethylformamide); DMSO (dimethyl sulfoxide); eq. (equivalent); ES (electrospray); EtOAc (ethyl acetate); EtOH (ethanol); Glu (glutamic acid), HATU (*O*-(7-azabenzotriazol-1-yl)-*N*,*N*,*N'*,*N'-*tetramethyluronium hexafluorophosphate); HCI (hydrochloric acid); hPhe (homophenylalanine), MeOH (methanol); MS (mass spectrometry); Mwt (molecular weight); NMR (nuclear magnetic resonance); RP (reversed-phase); RT/r.t. (room temperature); sat. aq. (saturated aqueous); SiO₂ (silica gel); tBu (tert-butyl), TBME (tert-butyl methyl ether), TFA (trifluoroacetic acid); THF (tetrahydrofurane); TIS (triisopropylsilane).

### Preparative Examples

### General procedure A

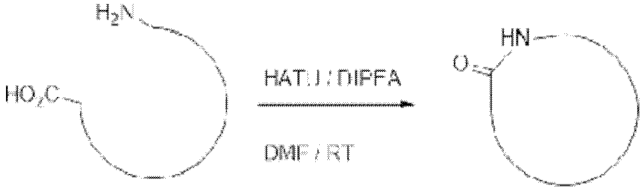

1.2 eq. HATU and 2 eq. DIPEA are dissolved in 1.5 ml/mmol DMF. 1 eq. amino acid in 2 ml/mmol DMF are added dropwise at room temperature or elevated temperature. 1 h after complete addition the reaction mixture was distributed between ethyl acetate and 2 N NaOH solution. The organic phase was separated, washed with brine, dried over sodium sulfate and solvents were evaporated. Depending on scale the raw product was purified by crystallization, flash chromatography or HPLC.

### General procedure B

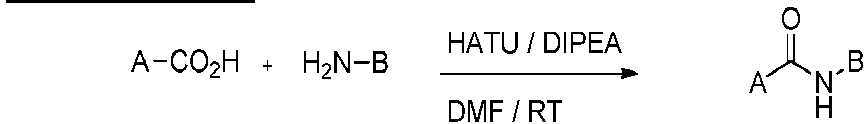

Carboxylic acid (1.5 eq.), HATU (1.1 eq.) and DIPEA (6 eq.) are dissolved in DMF and amine is added. After reaction was completed the reaction mixture can be diluted with ethyl acetate and then washed by sodium hydroxide solution and brine. After drying the organic phase over sodium sulfate and removing the solvents under reduced pressure the raw product was purified by crystallization, flash chromatography or HPLC.

### General procedure C

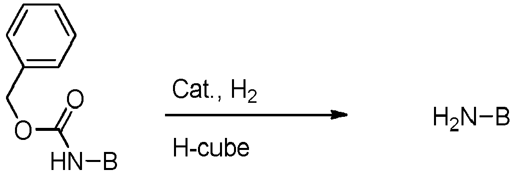

Cbz-protected amine is dissolved in a solvent (e.g. EtOH, ethyl acetate) or solvent mixtures at concentrations of e.g. 10 mg/ml depending on solubility. Hydrogenation by H-Cube^{®} using a Pd/C or Raney-Ni catalyst catridge at 50°C. Typically hydrogen pressure can be set from normal pressure to 50 bar and flow rates of 1 ml/min. If necessary hydrogenation is repeated until complete. Solvent is removed under reduced pressure to give a product usually pure enough to be used in the next reaction. If necessary the product can be purified by normal or revered phase flash chromatography.

### General procedure D

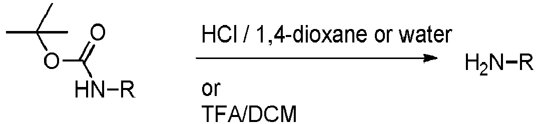

or

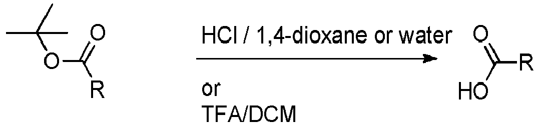

Boc-protected amine or tert-butyl carboxylate is dissolved in e.g. 4 N HCI in 1,4-dioxane, concentrated HCI in water or TFA in DCM (e.g. 20 %) at room temperature or elevated temperature (e.g 60 °C) and stirred at room temperature. After reaction is found to be complete volatiles are removed under reduce pressure. The remaining crude is coevaporated with e.g. acetonitrile or THF to remove excess HCI to give the related ammonium slat or carboxylic acid pure enough to be used in the next reaction.

### General procedure E

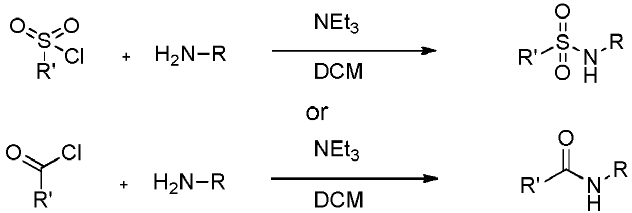

1 eq. amine and triethylamine (3 eq.) are dissolved in DCM (10-15 ml/mmol) and cooled to 0 °C. Sulfonyl chloride or carboxylic acid chloride (1.3 eq.) is added and the mixture is stirred until the reaction was completed. For HPLC purification the reaction mixture was diluted with some methanol. Otherwise the reaction was diluted with ethyl acetate and washed with HCI aq. or Na₂CO₃ aq. and brine. After drying over Na₂SO₄ and removing the solvent under reduced pressure the residue was purified by normal or revered phase flash chromatography.

### General procedure F

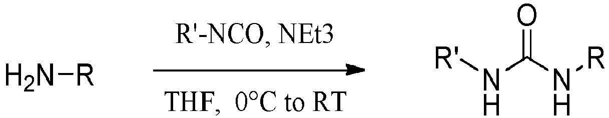

1 eq. amine and triethylamine (3 eq.) are dissolved in THF (10-15 ml/mmol) at RT. The related isocyanate (1.3 eq.) is added and the mixture is stirred until completion. The reaction mixture was diluted with some methanol and directly purified by HPLC.

### General procedure G

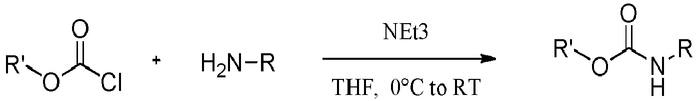

1 eq. amine and triethylamine (3 eq.) are dissolved in THF (10-15 ml/mmol) at 0°C. The related chloroformate (1.5 eq.) is added and the reaction was allowed to warm to room temperature. After complete reaction the reaction mixture was diluted with some methanol and directly purified by HPLC.

### General procedure H

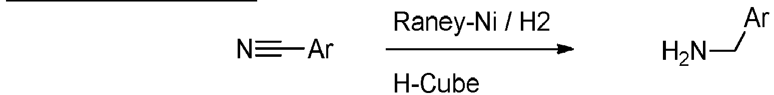

Aromatic nitrile in dissolved in ethanol, 2 M NH₃ in MeOH or other solvents or mixtures at concentrations of e.g. 10 mg/ml depending on solubility. Hydrogenation is achieved by H-Cube^{®} using a Raney-Ni catalyst cartridge at elevated temperatures, e.g. 50-80 °C. Typically hydrogen pressure can be set from 20-50 bar at flow rates of 1 ml/min. If necessary hydrogenation is repeated until complete. Solvent is removed under reduced pressure to give a product usually pure enough to be used in the next reaction. If necessary the product can be purified by normal or revered phase flash chromatography.

### Example A-1: Preparation of compound 8

### (S)-tert-butyl 3-(((benzyloxy)carbonyl)amino)-4-(((S)-1-ethoxy-1-oxo-4-phenylbutan-2-yl)amino)-4-oxobutanoate (1)

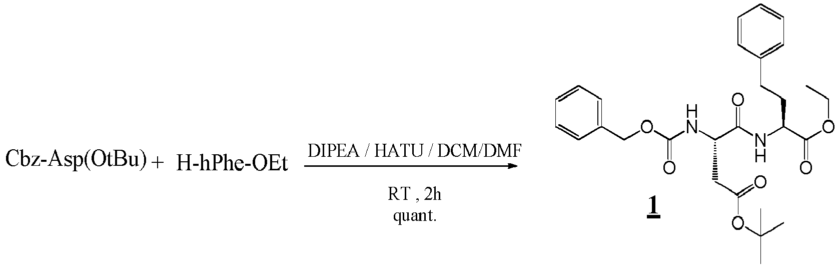

To a solution of Z-L-aspartic acid tert-butyl ester monohydrate (35 g, 102.5 mmol, 1.0 eq.) in DCM (1.2 L) and dry DMF (0.6 L) was added ethyl (S)-2-amino-4-phenylbutanoate hydrochloride (25 g, 102.5 mmol, 1.0 eq.) and HATU (57.3 g, 150.8 mmol, 1.5 eq.). Once purged under N₂, it was added dropwise and in 10 minutes DIPEA (44.4 g, 56.8 mL, 343.8 mmol, 3.4 equiv., previously filtered through a plug of Aloxbasic). After two hours at room temperature, LC-MS monitoring showed none of the starting materials, so, after evaporation of the DCM under reduced pressure, the resulting residue was diluted with EtOAc/TBME 1:1 and then washed two times with saturated NaHCO₃ solution, five times with water and once with brine. The organic phase was dried over MgSO₄, filtered and the solvents evaporated under reduced pressure to obtain 60.7 g of crude **1**. This crude was used in the next step without purification.
Formula: C₂₈H₃₆N₂O₇, exact mass: 512.3, found: 513.4 [M+H]⁺

### Preparation of (S)-2-((S)-2-(((benzyloxy)carbonyl)amino)-4-(tert-butoxy)-4-oxobutanamido)-4-phenylbutanoic acid (2)

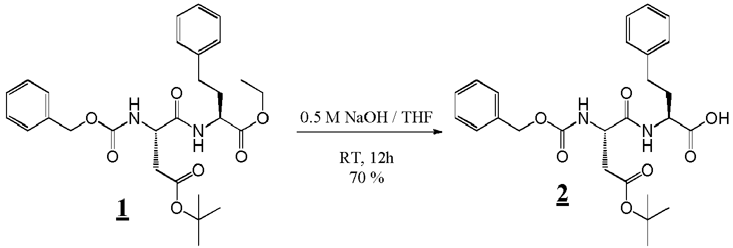

To a suspension of **1** (52 g, 101 mmol, 1.0 eq.) in THF (0.7 L) was added NaOH (0.5M, 202 mL, 101 mmol, 1.0 eq.) dropwise. The resulting mixture was stirred at room temperature for 12 hours before evaporation of the THF under reduced pressure. The resulting residue was diluted with water and TBME, and after decantation, the aqueous phase was further extracted two times with TBME. The resulting basic aqueous phase (pH ~8) was acidified to pH 2 by addition of 10% HCI before extraction three times with DCM. The combined organic phase was dried over MgSO4, filtered and evaporated to obtain 37 g of crude. This crude was purified in normal phase using Grace Reveleris and CHCl3/1% AcOH in MeOH as solvents to obtain 34.6 g of expected compound 2
Formula: C₂₆H₃₂N₂O₇, exact mass: 484.2, found: 485.3 [M+H]⁺

### tert-butyl 3-(2-(3-cyano-4-methylphenoxy)ethyl)piperidine-1-carboxylate (3)

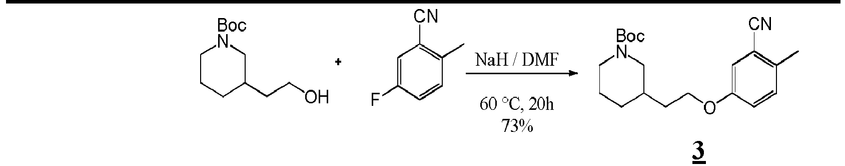

To a solution of N-Boc-3-(2-hydroxyethyl)piperidine (21 g, 91.7 mmol, 1.0 eq.) in dry DMF (180 mL) was added NaH (3.7 g, 60%, 91.7 mmol, 1.0 eq.). The resulting mixture was stirred for 30 minutes before the addition dropwise of a solution of 5-Fluoro-2-methylbenzonitrile (12.4 g, 91.7 mmol, 1.0 eq.) in dry DMF (40 mL). Once added, the mixture was heated at 60°C. After 20 hours of heating, LC-MS monitoring showed 78% conversion, so, after cooling to room temperature, additional NaH was added (3.7 g, 60%, 91.7 mmol, 1.0 eq.). The mixture was stirred again at room temperature for 30 minutes before heating at 60°C for 7 hours. Once cooled, the reaction mixture was quenched carefully by addition of saturated NH₄Cl solution, and extracted three times with TBME. The combined organic phase was washed once with saturated NH₄Cl solution, once with saturated NaHCO₃ solution, three times with water and once with brine. Once dried over MgSO₄ and filtered, the solvent was evaporated under reduced pressure to obtain 33 g of crude. This crude was purified on normal phase using Grace Reveleris and DCM/CyH as solvents to obtain 26.9 g of expected compound 3.
Formula: C₂₀H₂₈N₂O₃, exact mass: 344.2, found: 345.1 [M+H]⁺

### tert-butyl 3-(2-(3-(aminomethyl)-4-methylphenoxy)ethyl)piperidine-1-carboxlate (4)

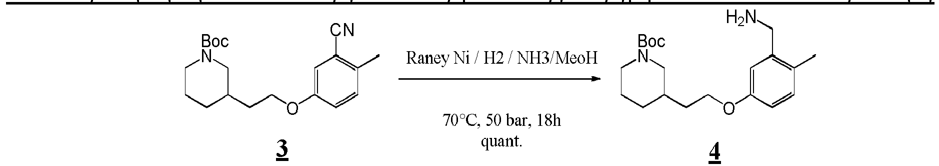

To a solution of **3** (22 g, 63.9 mmol; 1.0 eq.) in 7N ammonia in MeOH (1.2 L) was added Raney-Nickel (22 g, which was washed previously two times with MeOH). The resulting mixture was stirred at 70°C under 50 bar of H₂ for 18 hours. Once cooled, TLC monitoring showed no starting material, so, reaction mixture was filtered and the solid washed with MeOH. The combined filtrate was evaporated under reduced pressure to obtain 25 g of crude **4** as green oil that was used in the next step without purification.

### tert-butyl 3-(2-(3-((5S,8S)-5-(2-(tert-butoxy)-2-oxoethyl)-3,6,9-trioxo-8-phenethyl-1-phenyl-2-oxa-4,7,10-triazaundecan-11-yl)-4-methylphenoxy)ethyl)piperidine-1-carboxylate (5)

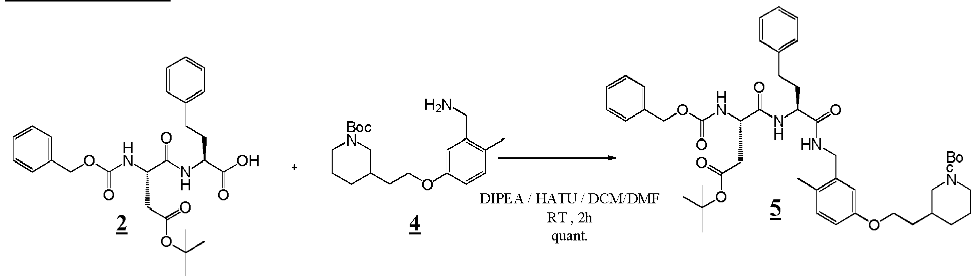

To a solution of **2** (34 g, 70.2 mmol, 1.0 eq.), **4** (24.5 g, 70.2 mmol, 1.0 eq.) and HATU (39.2 g, 103.2 mmol, 1.5 eq.) in DCM (0.86 L) and dry DMF (0.4 L) was added dropwise and in 20 minutes DIPEA (30.4 g, 38.9 mL, 242 mmol, 3.4 eq.). The reaction mixture was stirred at room temperature for 24 hours before evaporation of the DCM under reduced pressure. The resulting solution was added slowly over water (1.2 L) and then stirred at room temperature. The resulted upper layer was decanted and stirred again three times with more water. After final decantation, the slurry was dissolved with ACN and evaporated under reduced pressure. The resulted solid containing water was further co-evaporated three times once dissolved with ACN to obtain 62 g of crude. This crude was purified in normal phase using Grace Reveleris and DCM/CyH as solvents to obtain 43 g of expected compound **5**.
Formula: C₄₆H₆₂N₄O₉, exact mass: 814.5, found: 815.6 [M+H]⁺

### (3S)-3-(((benzyloxy)carbonyl)amino)-4-(((2S)-1-((2-methyl-5-(2-(piperidin-3-yl)ethoxy)benzyl)amino)-1-oxo-4-phenylbutan-2-yl)amino)-4-oxobutanoic acid (6)

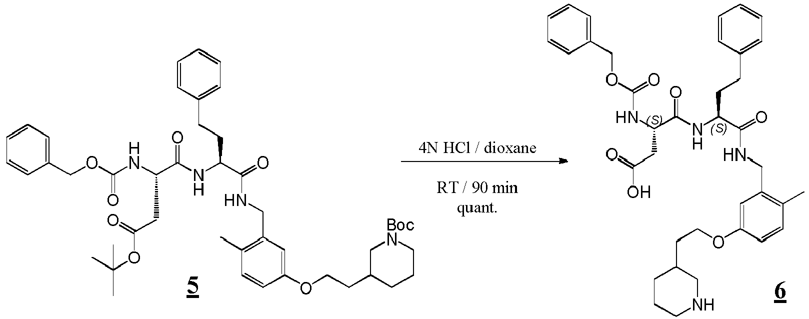

39.84 g (48.9 mmol) starting material 5 were dissolved in 40 ml 4 M HCI in dioxane and stirred at room temperature for 90 min. The solution was concentrated and stirred with additional 20 ml of 4 M HCI in dioxane at 60 °C for 1 h. Volatiles were removed under reduced pressure. Coevaporation with acetonitrile. Crude product 6 was used in the next step without further purification.
Formula: C₃₇H₄gN₄O₇, exact mass: 658.3, found: 659.6 [M+H]⁺

### benzyl ((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamate (7)

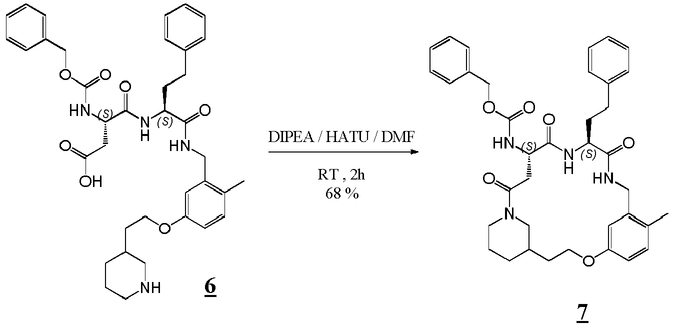

22.3 g (59 mmol) HATU were dissolved in 80 ml DMF. 48.9 mmol amino acid 6 in 114 ml DMF and 51.2 ml DIPEA were added dropwise at room temperature. The reaction mixture was distributed between ethyl acetate and 2 N NaOH solution. Some product precipitated and was collected and washed with MeOH. The organic phase was dried and evaporated. The remaining was triturated with methanol. Combined products summed up to 21.6 g of **7**.
Formula: C₃₇H₄₄N₄O₆, exact mass: 640.3, found:641.4 [M+H]⁺

In analogy to compound 7 the following diastereomers **289, 290, 291** and **292** were synthesized utilizing enantiomerically pure and commercially available building blocks as depicted in the related structures:

### benzyl ((13R,9R,12S)-5⁴-methyl-8,11,14-trioxo-9-phenethyl-4-oxa-7,10-diaza-1(3,1)-piperidina-5(1,3)-benzenacyclotetradecaphane-12-yl)carbamate (289)

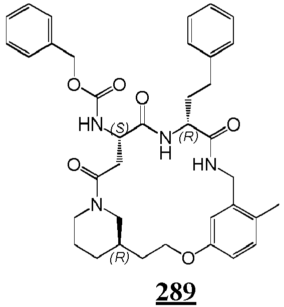

Formula: C₃₇H₄₄N₄O₆, exact mass: 640.3, found: 641.5 [M+H]⁺

### benzyl ((13R,9R,12R)-5⁴-methyl-8,11,14-trioxo-9-phenethyl-4-oxa-7,10-diaza-1(3,1)-piperidina-5(1,3)-benzenacyclotetradecaphane-12-yl)carbamate (290)

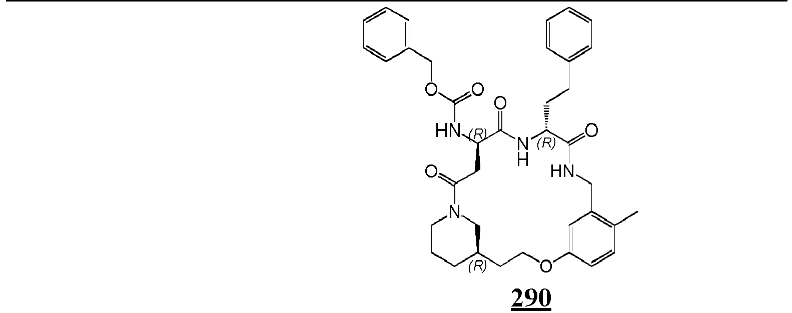

Formula: C₃₇H₄₄N₄O₆, exact mass: 640.3, found: 641.5 [M+H]⁺

### benzyl ((13R,9S, 12R)-5⁴⁻methyl-8, 11, 14-trioxo-9-phenethyl-4-oxa-7, 10-diaza-1(3,1)-piperidina-5(1,3)-benzenacyclotetradecaphane-12-yl)carbamate (291)

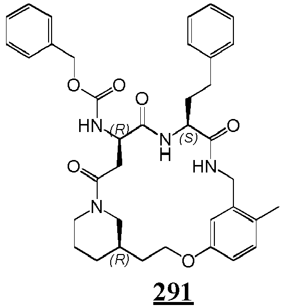

Formula: C₃₇H₄₄N₄O₆, exact mass: 640.3, found: 641.5 [M+H]⁺

### benzyl ((13R,9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamate (292)

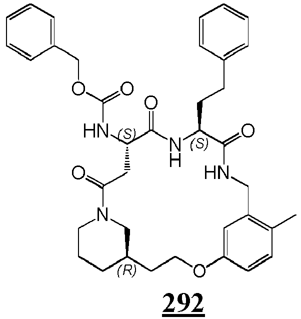

Formula: C₃₇H₄₄N₄O₆, exact mass: 640.3, found: 641.4 [M+H]⁺

### (9S,12S)-12-amino-5⁴-methyl-9-phenethyl-4-oxa-7,10-diaza-1(3,1)-piperidina-5(1,3)-benzenacyclotetradecaphane-8,11,14-trione (8)

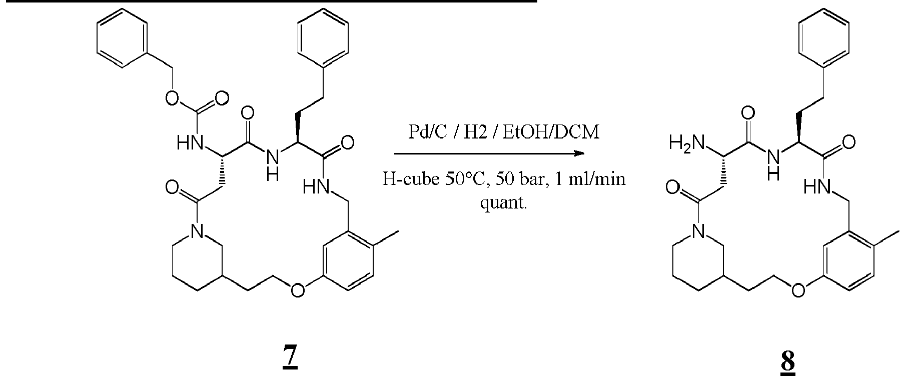

6.8 g benzyl carbamate **7** were dissolved EtOH/DCM (5:1) and hydrogenated with an H-cube (ThalesNano), 10 % Pd/C at 50°C under 50 bar H2 and a flow rate of 1 ml/min. 4 cycles were necessary to completely remove the protecting group. Quantitative yield after evaporation. Formula: C₂₉H₃₈N₄O₄, exact mass: 506.3, found: 507.3 [M+H]⁺

### Example A-2: Preparation of macrocyclic compounds 13, 16, 19 and 22

### tert-butyl 3-(2-(3-(((S)-2-(((benzyloxy)carbonyl)amino)-4-phenylbutanamido)methyl)-4-methylphenoxy)ethyl)piperidine-1-carboxylate (9)

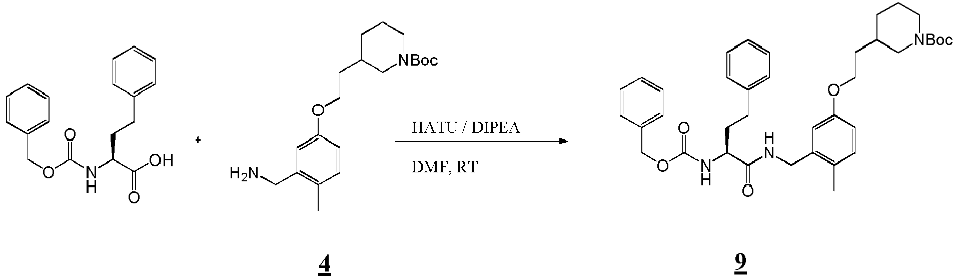

Synthesis according to general procedure B with 473 mg amine 4 and 350 mg Cbz-hPhe. Purification was achieved by normal phase flash chromatography.
Formula: C₃₈H₄₉N₃O₆, exact mass: 643.4, found: 644.4 [M+H]⁺

### tert-butyl 3-(2-(3-(((S)-2-amino-4-phenylbutanamido)methyl)-4-methylphenoxy)ethyl) piperidine-1-carboxylate (10)

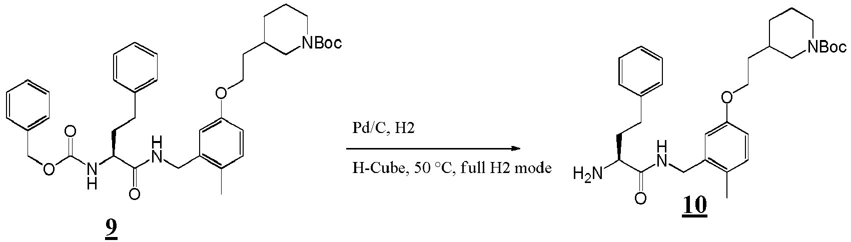

Following general procedure C using 260 mg Cbz-protected amine dissolved in 100 ml ethyl acetate / ethanol (1:1). H-Cube conditions: 1 ml/min, 50°C, full H2 mode. After removing of volatiles under reduced pressure the product was used without further purification.

### Amide derivatives of amine 10

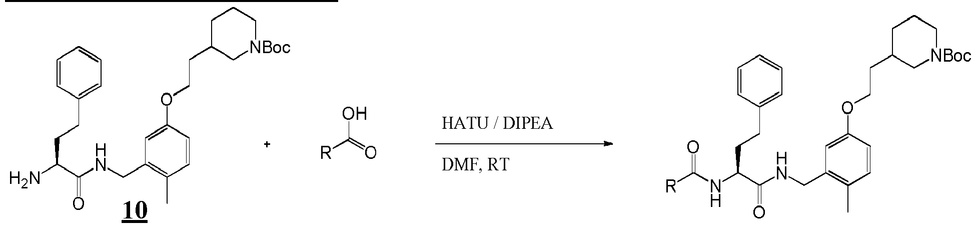

According to general procedure B with 75 mg amine 10. Purification was achieved by normal phase flash chromatography

**Table A-1**

| **Cpd No** | **Carboxylic acid** | **structure** | **name** | **exact mass** | **[M+H]⁺ found** |
|---|---|---|---|---|---|
| **11** | 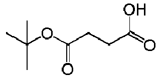 | 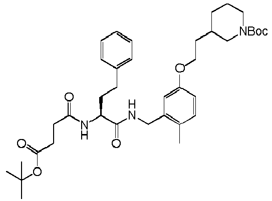 | tert-butyl 3-(2-(3-(((S)-2-(4-(tert-butoxy)-4-oxobutanamido)-4-phenylbutanamido)methyl )-4-methylphenoxy)ethyl)piperidine-1-carboxylate | 665.4 | 666.6 |
| | | | | C₃₈H₅₅ | |
| | | | | N₃O₇ | |
| **17** | 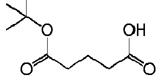 | 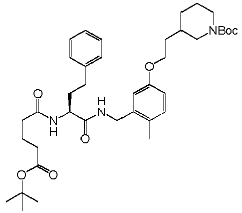 | tert-butyl 3-(2-(3-(((S)-2-(5-(tert-butoxy)-5-oxopentanamido)-4-phenylbutanamido)methyl )-4-methylphenoxy)ethyl)piperidine-1-carboxylate | 679.4 | 680.6 |
| | | | | C₃₉H₅₇ | |
| | | | | N₃O₇ | |
| **20** | 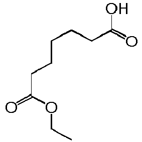 | 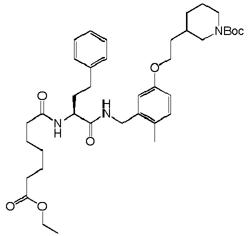 | tert-butyl 3-(2-(3-(((S)-2-(7-ethoxy-7-oxoheptanamido)-4-phenylbutanamido)methyl )-4-methylphenoxy)ethyl)piperidine-1-carboxylate | 679.4 | 680.7 |
| | | | | C₃₉H₅₇ | |
| | | | | N₃O₇ | |
| **14** | 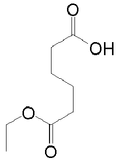 | 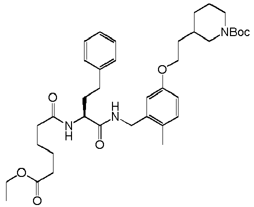 | tert-butyl 3-(2-(3-(((S)-2-(6-ethoxy-6-oxohexanamido)-4-phenylbutanamido)methyl)-4-methylphenoxy)ethyl)piperidine-1-carboxylate | 665.4 | 666.8 |
| | | | | C₃₈H₅₅ | |
| | | | | N₃O₇ | |

### Removal of protecting groups from compounds 11, 14, 17 and 20

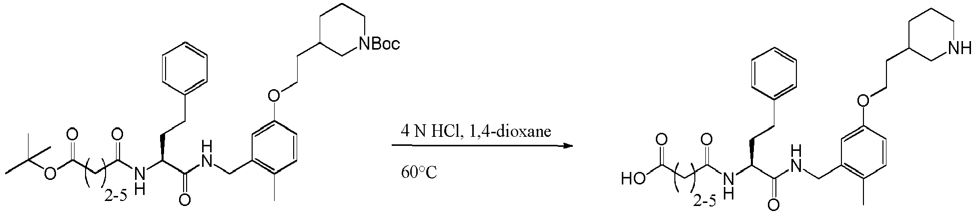

Protecting groups were cleaved according to general procedure D stirring compounds in 4 N HCl / 1,4-dioxane at 60 °C for about 30 min.

**Table A-2**

| **Cpd no** | starting material | product structure | name | exact mass | [M+H]⁺ found |
|---|---|---|---|---|---|
| **12** | **11** | 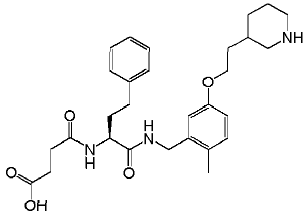 | 4-(((2S)-1-((2-methyl-5-(2-(piperidin-3-yl)ethoxy)benzyl)amino)-1-oxo-4-phenylbutan-2-yl)amino)-4-oxobutanoic acid | 509.3 | 510.2 |
| | | | | C₂₉H₃₉ | |
| | | | | N₃O₅ | |
| **18** | **17** | 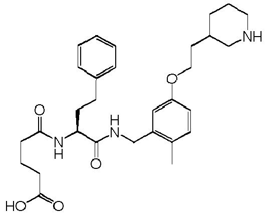 | 5-(((2S)-1-((2-methyl-5-(2-(piperidin-3-yl)ethoxy)benzyl)amino)-1-oxo-4-phenylbutan-2-yl)amino)-5-oxopentanoic acid | 523.3 | 524.3 |
| | | | | C₃₀H₄₁ | |
| | | | | N₃O₅ | |
| **21** | **20** | 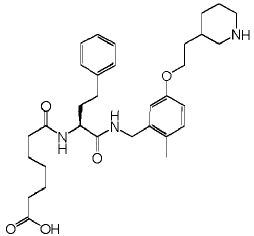 | 7-(((2S)-1-((2-methyl-5-(2-(piperidin-3-yl)ethoxy)benzyl)amino)-1-oxo-4-phenylbutan-2-yl)amino)-7-oxoheptanoic acid | 551.3 | 552.3 |
| | | | | C₃₂H₄₅ | |
| | | | | N₃O₅ | |
| **15** | **14** | 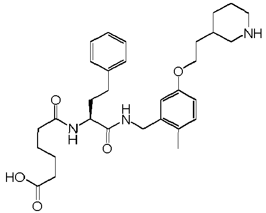 | 6-(((2S)-1-((2-methyl-5-(2-(piperidin-3-yl)ethoxy)benzyl)amino)-1-oxo-4-phenylbutan-2-yl)amino)-6-oxohexanoic acid | 537.3 | 538.7 |
| | | | | C₃₁H₄₃ | |
| | | | | N₃O₅ | |

### Macrocyclization of amino acids 12, 15, 18 and 21

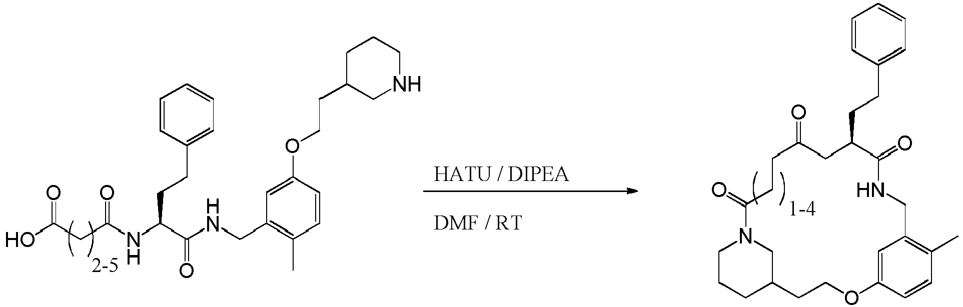

Macrocyclizations according to general procedure A. reaction mixtures were diluted with some methanol and purified via HPLC.

**Table A-3**

| Starting material | | Product | | | | |
|---|---|---|---|---|---|---|
| **Cpd** | Amount [mmol] | structure | name | Cpd no | exact mass | [M+H]⁺ found |
| **12** | 0.09 | 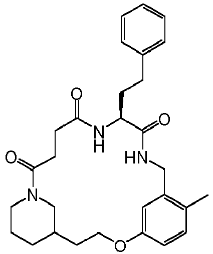 | (9S)-5⁴-methyl-9-phenethyl-4-oxa-7,10-diaza-1(3,1)-piperidina-5(1,3)-be nzenacyclotetradecaphane-8, 11,14-trione | **13** | 491.3 | 492.5 |
| | | | | | C₂₉H₃₇ | |
| | | | | | N₃O₄ | |
| **18** | 0.171 | 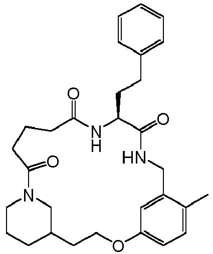 | (9S)-5⁴-methyl-9-phenethyl-4-oxa-7,10-diaza-1(3,1)-piperidina-5(1,3)-benzenacyclopentadecaphane-8,11,15-trione | **19** | 505.3 | 506.5 |
| | | | | | C₃₀H₃₉ | |
| | | | | | N₃O₄ | |
| **21** | 0.196 | 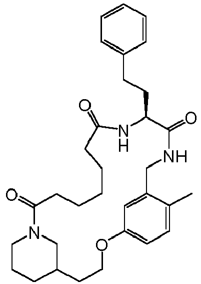 | (9S)-5⁴-methyl-9-phenethyl-4-oxa-7,10-diaza-1(3,1)-piperidina-5(1,3)-benzenacycloheptadecaphane-8,11,17-trione | **22** | 533.3 | 534.5 |
| | | | | | C₃₂H₄₃ | |
| | | | | | N₃O₄ | |
| **15** | 0.138 | 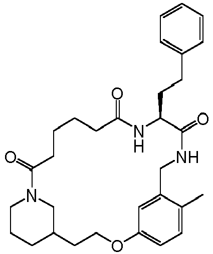 | (9S)-5⁴-methyl-9-phenethyl-4-oxa-7,10-diaza-1(3,1)-piperidina-5(1,3)-benzenacyclohexadecaphane-8,11,16-trione | **16** | 519.3 | 520.5 |
| | | | | | C₃₁H₄₁ | |
| | | | | | N₃O₄ | |

### Example A-3: Preparation of Macrocyclic compounds 25, 26, 27, 28, 29, 30, 31, 32, 33, 40, 41, 42, 60, 61, 62, 63, 64, 80, 81, 82, 83, 84, 85, 86, 87, 88, 89, 174, 175, 176, 177, 182, 219, 284, 285, 286, 287, 288, 323, 324, 327, 328, 329, 330, 331, 332, 333, 334, 335, 341, 369, 373,

### tert-butyl 3-(2-(3-(((S)-2-((S)-4-ethoxy-2-(3-(3-fluorophenyl)propanamido)-4-oxobutanamido)-4-phenylbutanamido)methyl)-4-methylphenoxy)ethyl)piperidine-1-carboxylate (23)

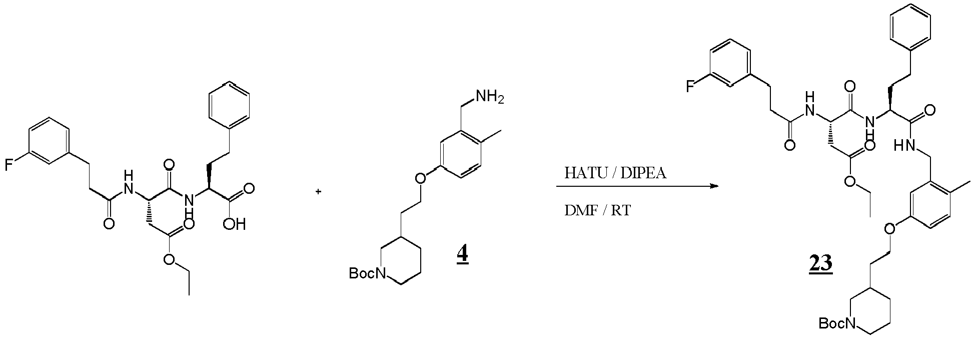

Amide coupling according to general procedure B with 113 mg carboxylic acid (prepared in analogy to compound **2)** and 108 mg benzyl amine **4**. Purification was achieved by normal phase flash chromatography applying a cyclohexane/ ethyl acetate gradient. Formula: C₄₅H₅₉FN₄O₈, exact mass: 802.4, found: 703.6 [M+H-Boc]+

### (3S)-3-(3-(3-fluorophenyl)propanamido)-4-(((2S)-1-((2-methyl-5-(2-(piperidin-3-yl)ethoxy)benzyl)amino)-1-oxo-4-phenylbutan-2-yl)amino)-4-oxobutanoic acid (24)

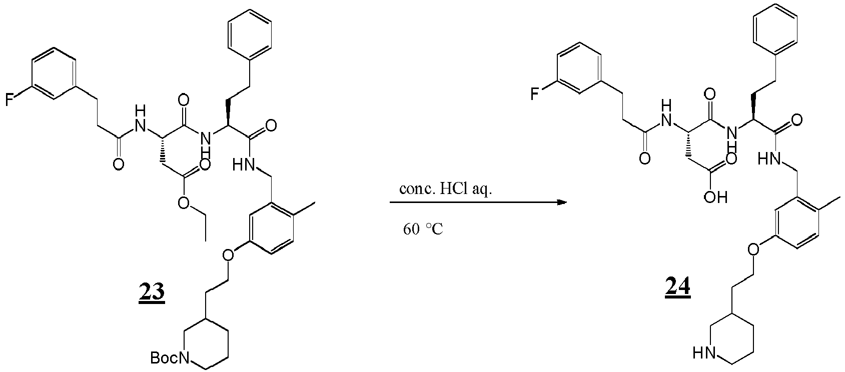

Both protecting groups were cleaved according to general procedure D in concentrated HCI in water at 60 °C for 90 min.
Formula: C₃₈H₄₇FN₄O₆, exact mass: 674.3, found: 675.2 [M+H]+

### 3-(3-fluorophenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)propanamide (25)

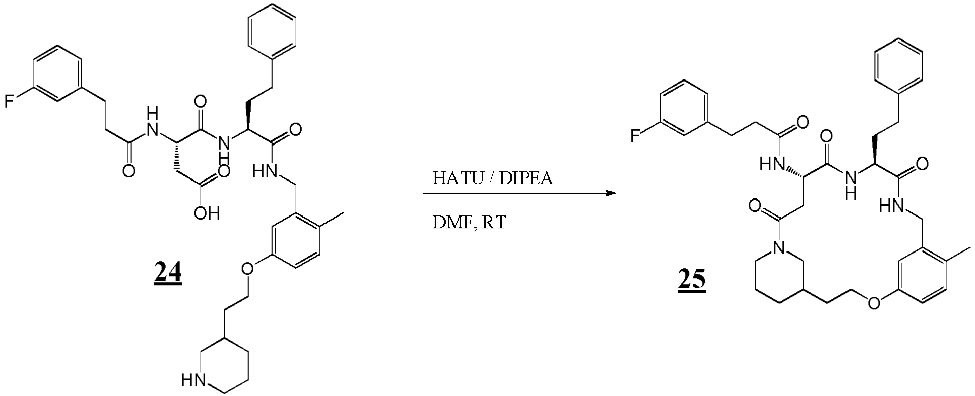

0.146 mmol amino acid **24** were cyclized according to general procedure A. Purification via RP18 flash chromatography.
Formula: C₃₈H₄₅FN₄O₅, exact mass: 656.3, found: 657.3 [M+H]+

### Amide derivatizations of compound 8 according to general procedure B

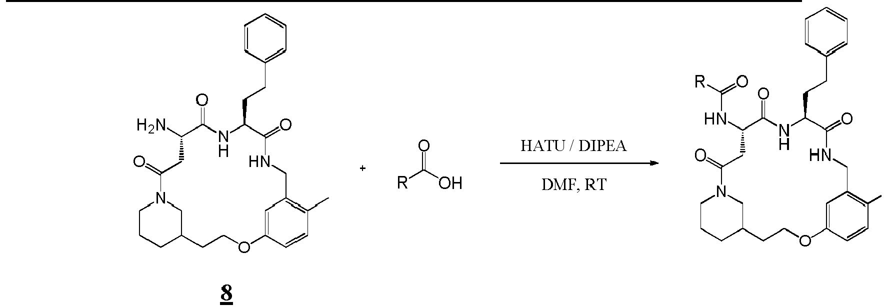

Amine **8** (e.g. 20 mg) was coupled with carboxylic acids according to general procedure B to give amides disclosed in table below. Purifications were achieved by HPLC or reversed phase flash chromatography.

**Table A-4**

| **Cpd No** | **structure** | **name** | **Exact mass** | **[M+H]⁺ found** |
|---|---|---|---|---|
| **26** | 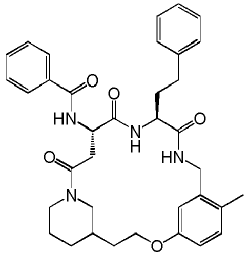 | N-((9S, 12S)-5⁴-methyl-8, 11, 14-trioxo-9-phenethyl-4-oxa-7,10-diaza-1(3,1)-piperidina-5(1,3)-benzenacyclotetradecaphane-12-yl)benzamide | 610.3 | 611.4 |
| | | | C₃₆H₄₂ | |
| | | | N₄O₅ | |
| **27** | 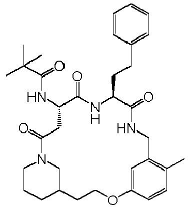 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)pivalamide | 590.3 | 591.4 |
| | | | C₃₄H₄₆ | |
| | | | N₄O₅ | |
| **28** | 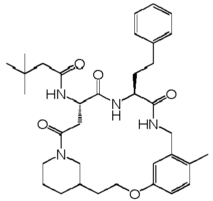 | 3,3-dimethyl-N-((9S, 12S)-5⁴-methyl-8,11,14-trioxo-9-phenethyl-4-oxa-7,10-diaza-1(3,1)-piperidina-5(1,3)-benzenacyclotetradecaphane-12-yl)butanamide | 604.4 | 605.4 |
| | | | C₃₅H₄₈ | |
| | | | N₄O₅ | |
| **29** | 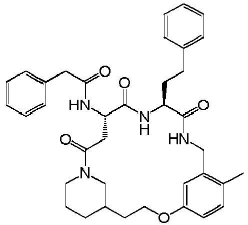 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-phenylacetamide | 624.3 | 625.5 |
| | | | C₃₇H₄₄ | |
| | | | N₄O₅ | |
| **30** | 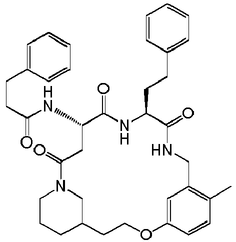 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-3-phenylpropanamide | 638.3 | 639.5 |
| | | | C₃₈H₄₆ | |
| | | | N₄O₅ | |
| **3** | 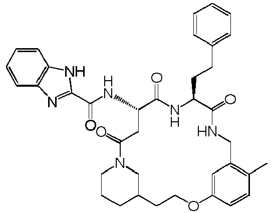 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-1H-benzo[d]imidazole-2-carboxamide | 650.3 | 651.1 |
| | | | C₃₇H₄₂ | |
| | | | N₆O₅ | |
| **32** | 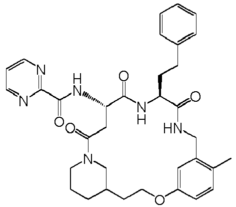 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)pyrimidine-2-carboxamide | 612.3 | 613.4 |
| | | | C₃₄H₄₀ | |
| | | | N₆O₅ | |
| **33** | 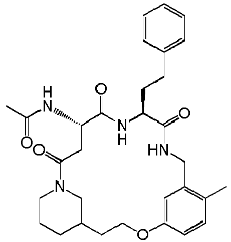 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide | 548.3 | 549.3 |
| | | | C₃₁ H₄₀ | |
| | | | N₄O₅ | |
| **40** | 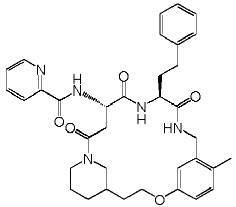 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)picolinamide | 611.3 | 612.4 |
| | | | C₃₅H₄₁ | |
| | | | N₅O₅ | |
| **41** | 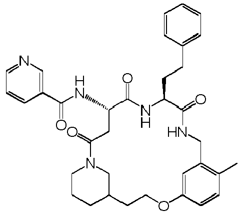 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)nicotinamide | 611.3 | 612.4 |
| | | | C₃₅H₄₁ | |
| | | | N₅O₅ | |
| **42** | 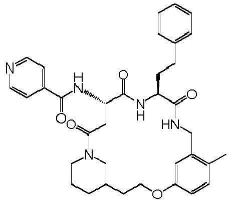 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)isonicotinamide | 611.3 | 612.3 |
| | | | C₃₅H₄₁ | |
| | | | N₅O₅ | |
| **60** | 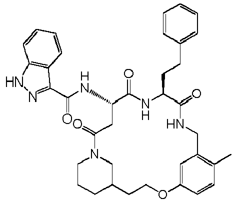 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-1H-indazole-3-carboxamide | 650.3 | 651.5 |
| | | | C₃₇H₄₂ | |
| | | | N₆O₅ | |
| **61** | 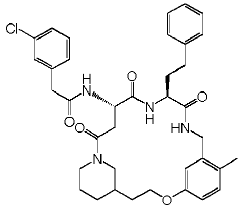 | 2-(3-chlorophenyl)-N-((9S,12S)-54-methyl-8,11,14-trioxo-9-phenethyl-4-oxa-7,10-diaza-1(3,1)-piperidina-5(1,3)-benzenacyclotetradecaphane-12-yl)acetamide | 658.3 | 659.3 |
| | | | C₃₇H₄₃ | |
| | | | CIN₄ | |
| | | | O₅ | |
| **62** | 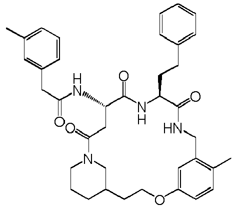 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-(m-tolyl)acetamide | 638.3 | 639.4 |
| | | | C₃₈H₄₆ | |
| | | | N₄O₅ | |
| **63** | 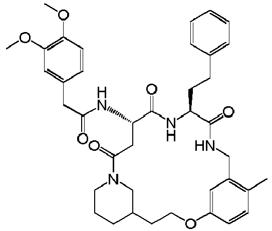 | 2-(3,4-dimethoxyphenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide | 684.4 | 685.4 |
| | | | C₃₉H₄₈ | |
| | | | N₄O₇ | |
| **64** | 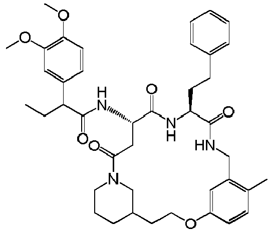 | 2-(3,4-dimethoxyphenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)butanamide | 712.4 | 713.4 |
| | | | C₄₁H₅₂ | |
| | | | N₄O₇ | |
| **80** | 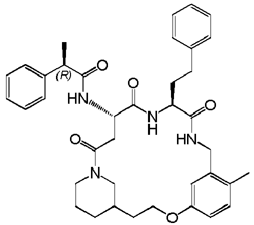 | (2R)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-phenylpropanamide | 638.3 | 639.4 |
| | | | C₃₈H₄₆ | |
| | | | N₄O₅ | |
| **81** | 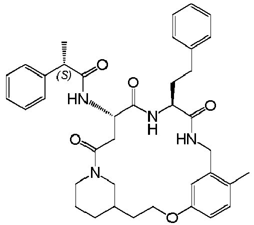 | (2S)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-phenylpropanamide | 638.3 | 639.4 |
| | | | C₃₈H₄₆ | |
| | | | N₄O₅ | |
| **82** | 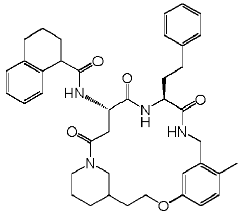 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-1,2,3,4-tetrahydronaphthalene-1-carboxamide | 664.4 | 665.4 |
| | | | C₄₀H₄₈ | |
| | | | N₄O₅ | |
| **83** | 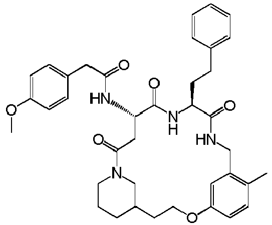 | 2-(4-methoxyphenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide | 654.3 | 655.4 |
| | | | C₃₈H₄₆ | |
| | | | N₄O₆ | |
| **84** | 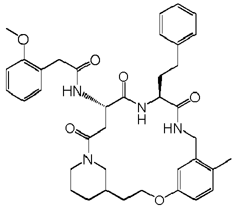 | 2-(2-methoxyphenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide | 654.3 | 655.3 |
| | | | C₃₈H₄₆ | |
| | | | N₄O₆ | |
| **85** | 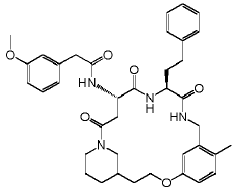 | 2-(3-methoxyphenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide | 654.3 | 655.4 |
| | | | C₃₈H₄₆ | |
| | | | N₄O₆ | |
| **86** | 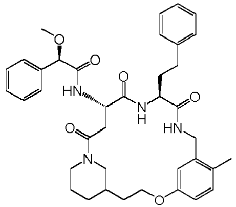 | (2R)-2-methoxy-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-phenylacetamide | 654.3 | 655.4 |
| | | | C₃₈H₄₆ | |
| | | | N₄O₆ | |
| **87** | 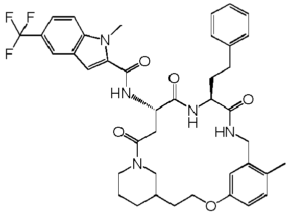 | 1-methyl-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-5-(trifluoromethyl)-1H-indole-2-carboxamide | 731.3 | 732.4 |
| | | | C₄₀H₄₄ | |
| | | | F₃N₅ | |
| | | | O₅ | |
| **88** | 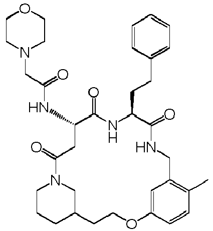 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-morpholinoacetamide | 633.4 | 634.4 |
| | | | C₃₅H₄₇ | |
| | | | N₅O₆ | |
| **89** | 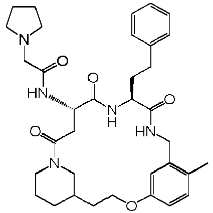 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-(pyrrolidin-1-yl)acetamide | 617.4 | 618.4 |
| | | | C₃₅H₄₇ | |
| | | | N₅O₅ | |
| **174** | 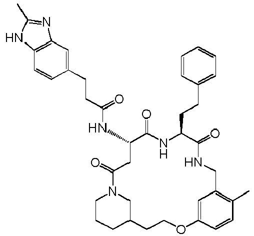 | 3-(2-methyl-1H-benzo[d]imidazol-6-yl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)propanamide | 692.4 | 693.4 |
| | | | C₄₀H₄₈ | |
| | | | N₆O₅ | |
| **175** | 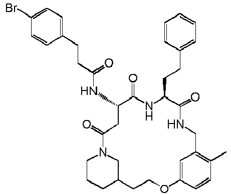 | 3-(4-bromophenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)propanamide | 716.3 | 717.3 |
| | | | C₃₈H₄₅ | |
| | | | BrN₄ | |
| | | | O₅ | |
| **176** | 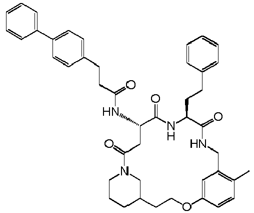 | 3-([1,1'-biphenyl]-4-yl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)propanamide | 714.4 | 715.4 |
| | | | C₄₄H₅₀ | |
| | | | N₄O₅ | |
| **177** | 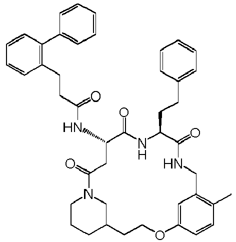 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-3-(2-phenoxyphenyl)propanamide | 714.4 | 715.4 |
| | | | C₄₄H₅₀ | |
| | | | N₄O₅ | |
| **182** | 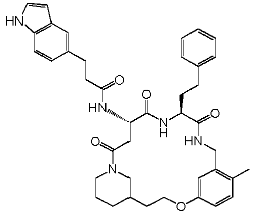 | 3-(1H-indol-5-yl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)propanamide | 677.4 | 678.5 |
| | | | C₄₀H₄₇ | |
| | | | N₅O₅ | |
| **219** | 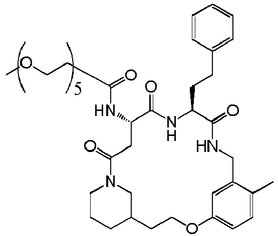 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2,5,8,11,14-pentaoxaheptadecan-17-amide | 768.4 | 769.6 |
| | | | C₄₁H₆₀ | |
| | | | N₄O₁₀ | |
| **284** | 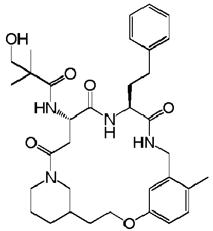 | 3-hydroxy-2,2-dimethyl-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)propanamide | 606.3 | 607.5 |
| | | | C₃₄H₄₆ | |
| | | | N₄O₆ | |
| **285** | 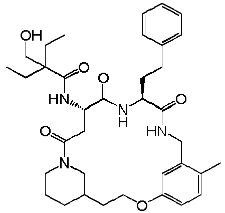 | 2-ethyl-2-(hydroxymethyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)butanamide | 634.4 | 635.5 |
| | | | C₃₆H₅₀ | |
| | | | N₄O₆ | |
| **286** | 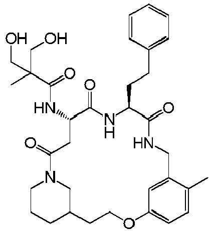 | 3-hydroxy-2-(hydroxymethyl)-2-methyl-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)propanamide | 622.3 | 623.5 |
| | | | C₃₄H₄₆ | |
| | | | N₄O₇ | |
| **287** | 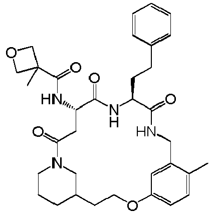 | 3-methyl-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)oxetane-3-carboxamide | 604.3 | 605.7 |
| | | | C₃₄H₄₄ | |
| | | | N₄O₆ | |
| **288** | 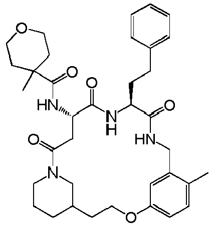 | 4-methyl-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)tetrahydro-2H-pyran-4-carboxamide | 632.4 | 633.5 |
| | | | C₃₆H₄₈ | |
| | | | N₄O₆ | |
| **323** | 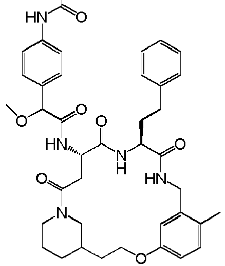 | 2-(4-acetamidophenyl)-2-methoxy-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide | 711.4 | 712.4 |
| | | | C₄₀H₄₉ | |
| | | | N₅O₇ | |
| **324** | 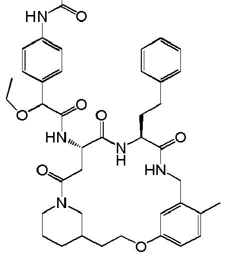 | 2-(4-acetamidophenyl)-2-ethoxy-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide | 725.4 | 726.4 |
| | | | C₄₁H₅₁ | |
| | | | N₅O₇ | |
| **327** | 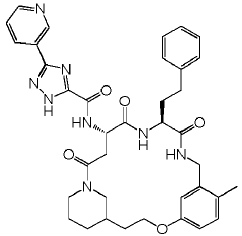 | N-((9S,12R)-5⁴-methyl-8,11,14-trioxo-9-phenethyl-4-oxa-7,10-diaza-1(3,1)-piperidina-5(1,3)-benzenacyclotetradecaphane-12-yl)-2-(3-(pyridin-3-yl)-1H-1,2,4-triazol-5-yl)acetamide | 692.3 | 693.4 |
| | | | C₃₈H₄₄ | |
| | | | N₈O₅ | |
| **328** | 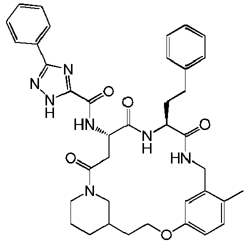 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-(3-phenyl-1H-1,2,4-triazol-5-yl)acetamide | 691.3 | 692.4 |
| | | | C₃₉H₄₅ | |
| | | | N₇O₅ | |
| **329** | 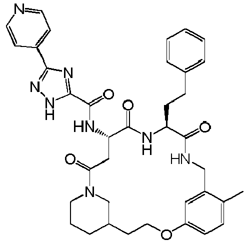 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-(3-(pyridin-4-yl)-1H-1,2,4-triazol-5-yl)acetamide | 692.3 | 693.3 |
| | | | C₃₈H₄₄ | |
| | | | N₈O₅ | |
| **330** | 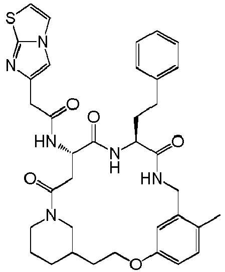 | 2-(imidazo[2,1-b]thiazol-6-yl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide | 670.3 | 671.3 |
| | | | C₃₆H₄₂ | |
| | | | N₆O₅S | |
| **331** | 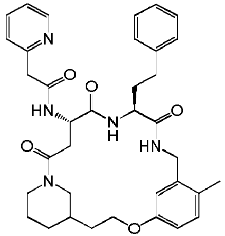 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-(pyridin-2-yl)acetamide | 625.3 | 507.4 |
| | | | C₃₆H₄₃ | |
| | | | N₅O₅ | |
| **332** | 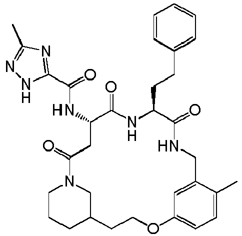 | 2-(3-methyl-1H-1,2,4-triazol-5-yl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide | 629.3 | 630.4 |
| | | | C₃₄H₄₃ | |
| | | | N₇O₅ | |
| **333** | 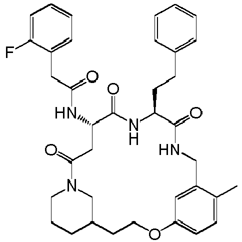 | 2-(2-fluorophenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide | 642.3 | 643.4 |
| | | | C₃₇H₄₃ | |
| | | | FN₄O₅ | |
| **334** | 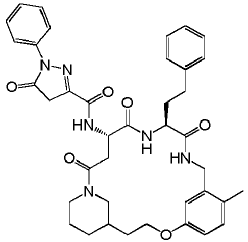 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-(5-oxo-1-phenyl-4,5-dihydro-1H-pyrazol-3-yl)acetamide | 706.3 | 707.4 |
| | | | C₄₀H₄₆ | |
| | | | N₆O₆ | |
| **335** | 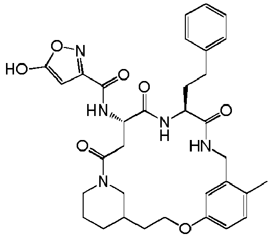 | 2-(5-hydroxyisoxazol-3-yl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide | 631.3 | 632.4 |
| | | | C₃₄H₄₁ | |
| | | | N₅O₇ | |
| **341** | 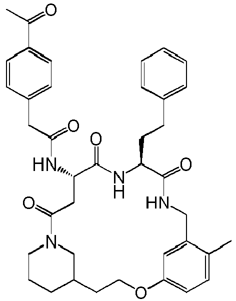 | 2-(4-acetylphenyl)-N-((9S,12S)-54-methyl-8,11,14-trioxo-9-phenethyl-4-oxa-7,10-diaza-1(3,1)-piperidina-5(1,3)-benzenacyclotetradecaphane-12-yl)acetamide | 666.3 | 667.4 |
| | | | C₃₉H₄₆ | |
| | | | N₄O₆ | |
| **369** | 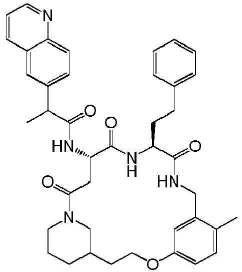 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-(quinolin-6-yl)propanamide | 689.4 | 690.4 |
| | | | C₄₁H₄₇ | |
| | | | N₅O₅ | |
| **373** | 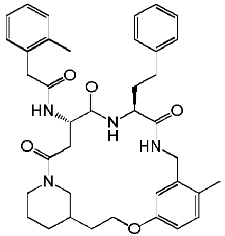 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-(o-tolyl)acetamide | 638.3 | 639.4 |
| | | | C₃₈H₄₆ | |
| | | | N₄O₅ | |

### Example A-4: Preparation of macrocyclic compounds 34, 35, 36, 37, 38, 39, 43, 44, 45, and 364

### (9S, 12S)-5⁴-methyl-9-phenethyl-12-(pyrimidin-2-ylamino)-4-oxa-7,10-diaza-1(3,1)-piperidina-5(1,3)-benzenacyclotetradecaphane-8,11,14-trione (34)

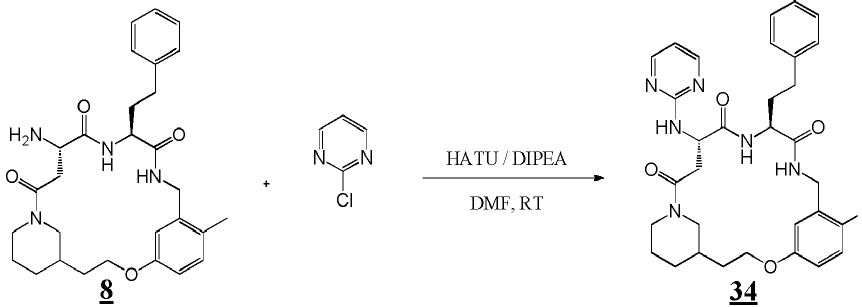

20 mg amine 8, 9 mg 2-chloropyrimidine and 25 mg K₃PO₄ in 0.5 ml DMF were heated to 100 °C over night. Diluted with some methanol, filtered and purified via HPLC. Formula: C₃₃H₄₀N₆O₄, exact mass: 584.3, found: 585.3 [M+H]⁺

### (9S,12S)-5⁴-methyl-12-(oxetan-3-ylamino)-9-phenethyl-4-oxa-7,10-diaza-1(3,1)-piperidina-5(1,3)-benzenacyclotetradecaphane-8,11,14-trione (35)

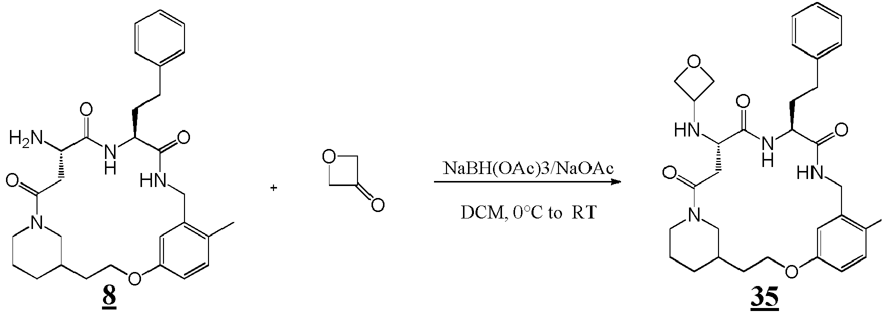

20 mg amine 8 and 4 mg 3-oxetanone in DCM were cooled in an ice bath. Sodium acetate (0.5 eq.) and 3 eq. sodium triacetoxyborohydride were added and the reaction mixture was allowed to warm to room temperature overnight. Additional 4.3 mg ketone and 17 mg borohydride were added and stirring was continued for another 3h when some methanol was added and the reaction mixture was purified via HPLC.
Formula: C₃₂H₄₂N₄O₅, exact mass: 562.3, found: 563.5 [M+H]⁺

### (9S, 12S)-5⁴-methyl-9-phenethyl-12-(pyridin-2-ylamino)-4-oxa-7,10-diaza-1(3,1)-piperidina-5(1,3)-benzenacyclotetradecaphane-8,11,14-trione (36)

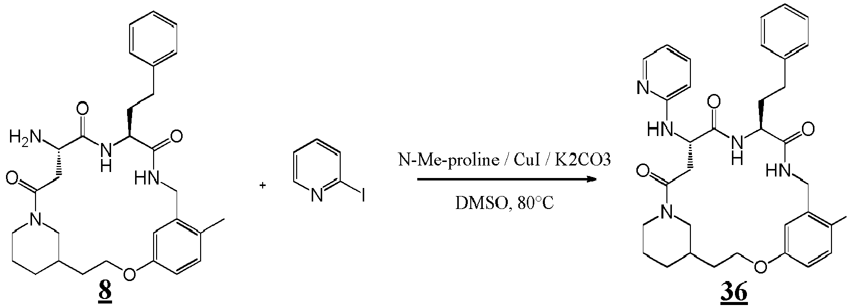

20 mg amine 8, 6.4 mg 2-iodopyridine, 5.2 mg N-Me-proline, 3.8 mg CuI and 11.1 mg K₂CO₃ in 500 µl DMSO were heated to 80 °C for 24 h. The mixture was diluted with some methanol, filtered and purified via HPLC.
Formula: C₃₄H₄₁N₅O₄, exact mass: 583.3, found: 584.3 [M+H]⁺

### N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)methanesulfonamide (37)

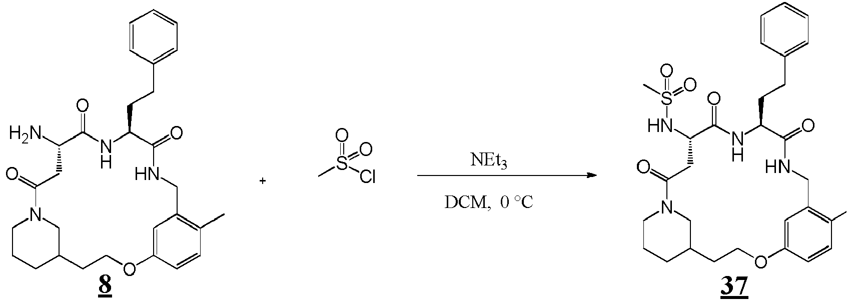

Compound 37 was synthesized according to general procedure E with 20 mg amine 8. Product was purified by HPLC.
Formula: C₃₀H₄₀N₄O₆S, exact mass: 584.3, found: 585.4 [M+H]⁺

### 1-ethyl-3-((9S,12S)-5⁴-methyl-8,11,14-trioxo-9-phenethyl-4-oxa-7,10-diaza-1(3,1)-piperidina-5(1,3)-benzenacyclotetradecaphane-12-yl)urea (38)

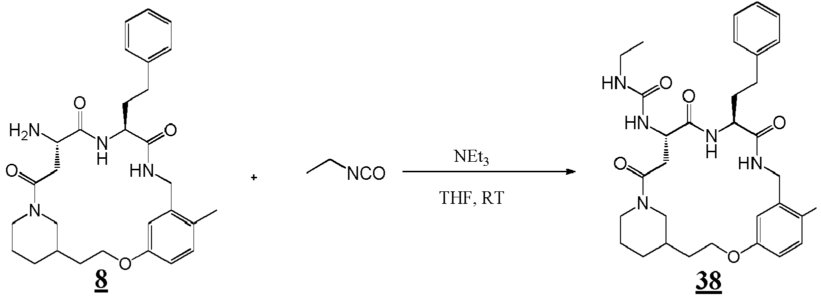

Compound 38 was synthesized according to general procedure F with 20 mg amine 8 in 0.5 ml THF.
Formula: C₃₂H₄₃N₅O₅, exact mass: 577.3, found: 578.4 [M+H]⁺

### ethyl ((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamate (39)

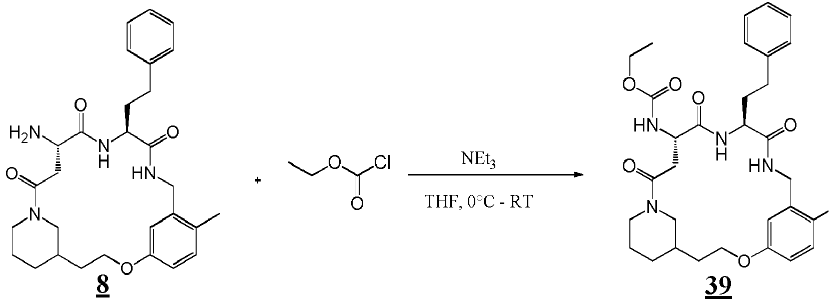

Compound **39** was synthesized according to general procedure G with 20 mg amine 8 in 0.5 ml THF.
Formula: C₃₂H₄₂N₄O₆, exact mass: 578.3, found: 579.4 [M+H]⁺

### (9S,12S)-12-(2,5-dioxopyrrolidin-1-yl)-5⁴-methyl-9-phenethyl-4-oxa-7,10-diaza-1(3,1)-piperidina-5(1,3)-benzenacyclotetradecaphane-8,11,14-trione (43)

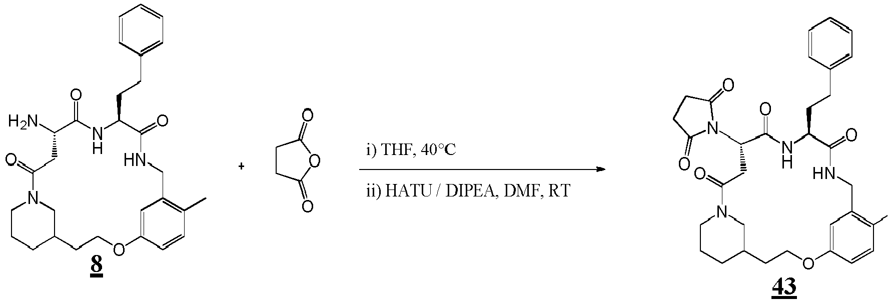

10 mg amine 8 and 2.0 mg succinic anhydride in 0.3 ml THF were stirred at 40 °C overnight. Volatiles were removed under reduced pressure and the residue dissolved in 300 µl DMF. 8.4 mg HATU and 20.9 µl DIPEA in additional 300 µl DMF were added at RT. After complete reaction some methanol was added and purification was achieved by HPLC.
Formula: C₃₃H₄₀N₄O₆, exact mass: 588.3, found: 589.4 [M+H]⁺

### N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-3-phenylpropane-1-sulfonamide (44)

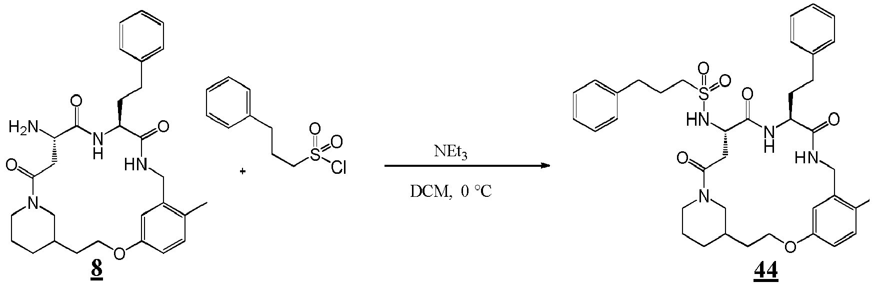

Compound **44** was synthesized according to general procedure E with 20 mg amine 8. Product was purified by HPLC.
Formula: C₃₈H₄₈N₄O₆S, exact mass: 688.3, found: 689.5 [M+H]⁺

### (9S,12S)-12-((1H-benzo[d]imidazol-2-yl)amino)-5⁴-methyl-9-phenethyl-4-oxa-7,10-diaza-1(3,1)-piperidina-5(1,3)-benzenacyclotetradecaphane-8,11,14-trione (45)

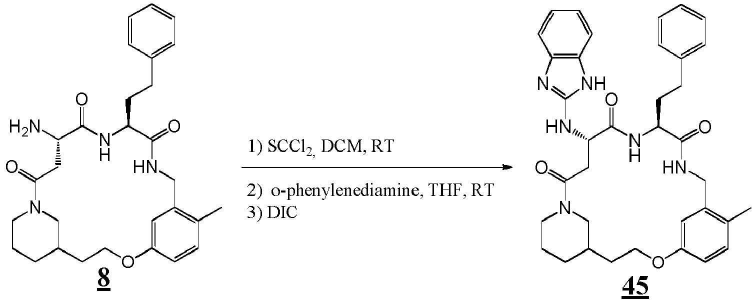

To 20 mg amine 8 in 1 ml DCM 30.5 µl thiophosgene were added. 1 ml saturated NaHCO₃ solution was added and the mixture was stirred for until starting material was consumed. The organic phase was separated, dried and volatiles were removed under reduced pressure. The residue and 5.2 mg o-phenylenediamine were dissolved in 0.5 ml THF and stirred overnight to give the intermediate thiourea. 5.6 mg *N*,*N'-*diisopropylcarbodiimid were added and the mixture was kept at 55 °C until reaction was almost complete. The mixture was diluted with some methanol and purified by HPLC.
Formula: C₃₆H₄₂N₆O₄, exact mass: 622.3, found: 623.5 [M+H]⁺

### N-((9S,12S)-8,11,14-trioxo-9-phenethyl-4-oxa-7, 10-diaza-1(3,1)-piperidina-5(1,3)-benzenacyclotetradecaphane-12-yl)acetamide (364)

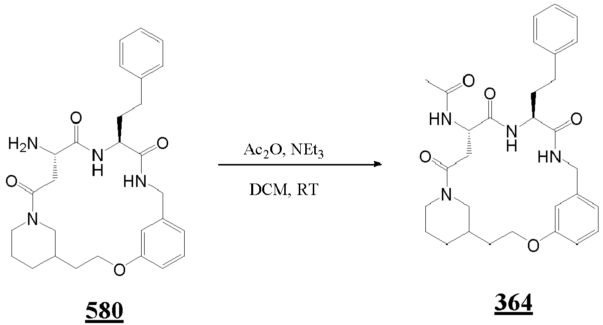

Compound **580** was made in analogy to compound **8** utilizing tert-butyl-3-(2-(3-aminomethyl(phenoxy)ethyl)piperidine-1-carboxylate **579** instead of amine **4**.

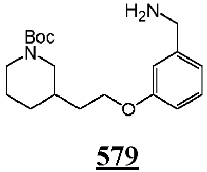

Formula: C₃₀H₃₈N₄O₅, exact mass: 534.3, found: 535.5 [M+H]⁺

### Example A-5: Preparation of macrocyclic compound 51

### 2-methyl-5-(2-(piperidin-3-yl)ethoxy)benzonitrile (46)

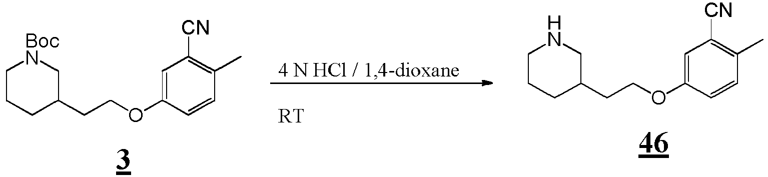

Compound **46** was prepared from 390 mg Boc-protected amine 3 according to general procedure D with 4 N HCI in 1,4-dioxane at RT. Raw product was coevaporated twice with methanol and use without further purification.
Formula: C₁₅H₂₀N₂O, exact mass: 244.2, found: 245.2 [M+H]⁺

### tert-butyl 4-(3-(2-(3-cyano-4-methylphenoxy)ethyl)piperidin-1-yl)-4-oxobutanoate (47)

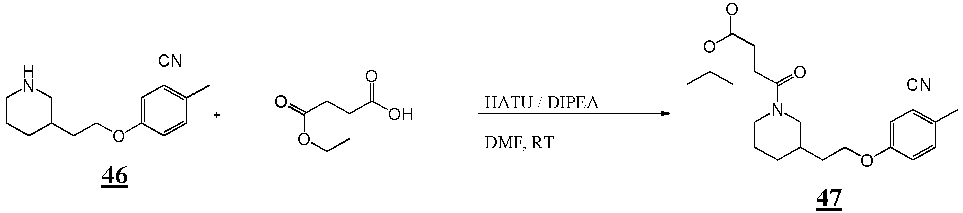

Synthesis according to general procedure B with 1.13 mmol amine **4** and 296 mg mono-tert.-butyl succinate. Purification was achieved by normal phase flash chromatography. Formula: C₂₃H₃₂N₂O₄, exact mass: 400.2, found: 401.4 [M+H]⁺

### tert-butyl 4-(3-(2-(3-(aminomethyl)-4-methylphenoxy)ethyl)piperdin-1-yl)-4-oxobutanoate (48)

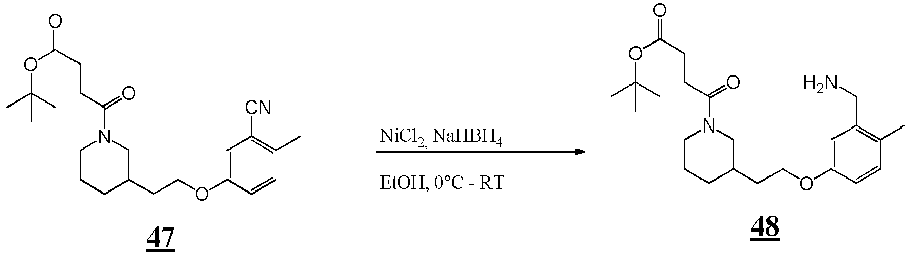

142 mg nitrile **47** and 91 mg NiCl2 were stirred in 7 ml ethanol at 0 °C. 54 mg NaBH₄ were added and the mixture was allowed to come to room temperature. After 1h each same amounts as before of NaBH₄ and NiCl2 were added. After 1h the mixture was filtered over Celite. Filtrate was concentrated under reduced pressure, distributed between water and ethyl acetate. The organic phase was sepatrated and the aqueous phase extracted several times with ethyl acetat. Combined organic phases were dried over MgSO₄ and solvent was removed under reduce pressure. The crude was used without further purification.
Formula: C₂₃H₃₆N₂O₄, exact mass: 404.3, found: 405.1 [M+H]⁺

### tert-butyl 4-(3-(2-(3-((2-((tert-butoxycarbonyl)amino)-4-(pyridin-3-yl)butanamido)methyl)-4-methylphenoxy)ethyl)piperidin-1-yl)-4-oxobutanoate (49)

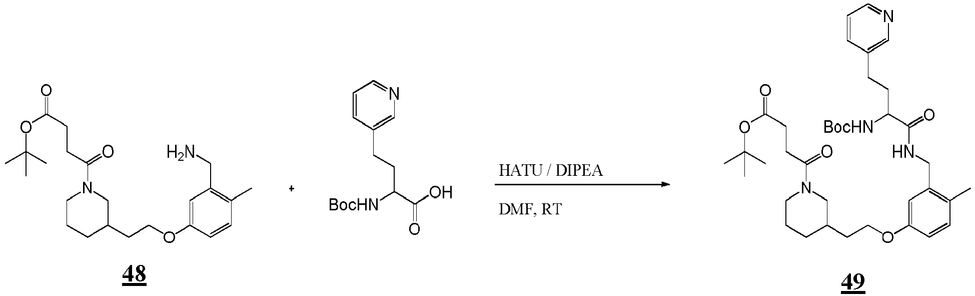

Synthesis according to general procedure B with 94 mg amine **48** and 50 mg 2-((tert-butoxycarbonyl)amino)-4-(pyridin-3-yl)butanoic acid. Purification was achieved by normal phase flash chromatography
Formula: C₃₇H₅₄N₄O₇, exact mass: 666.4, found: 667.4 [M+H]⁺

### 4-(3-(2-(3-((2-amino-4-(pyridin-3-yl)butanamido)methyl)-4-methylphenoxy)ethyl) piperidin-1-yl)-4-oxobutanoic acid (50)

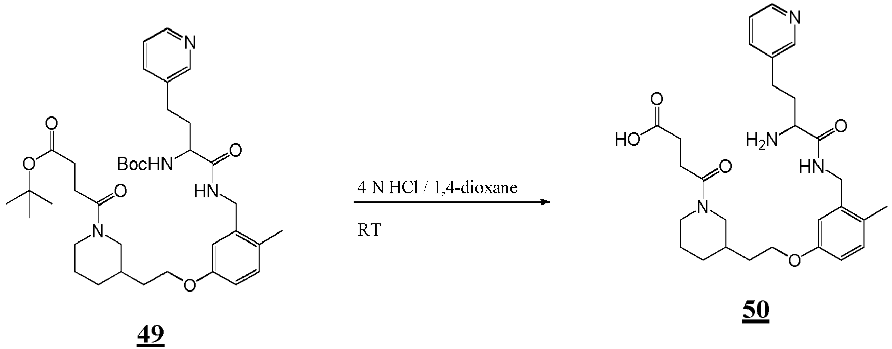

Protecting groups were cleaved according to general procedure D stirring compounds in 4 N HCI / 1,4-dioxane at room temperature. After deprotection was complete solvent was removed and the crude dissolved in acetonitrile and concentrated twice. Product used without further purification.
Formula: C₂₈H₃₈N₄O₅, exact mass: 510.3, found: 511.3 [M+H]⁺

### 5⁴-methyl-9-(2-(pyridin-3-yl)ethyl)-4-oxa-7,10-diaza-1(3,1)-piperidina-5(1,3)-benzenacyclotetradecaphane-8,11,14-trione (51)

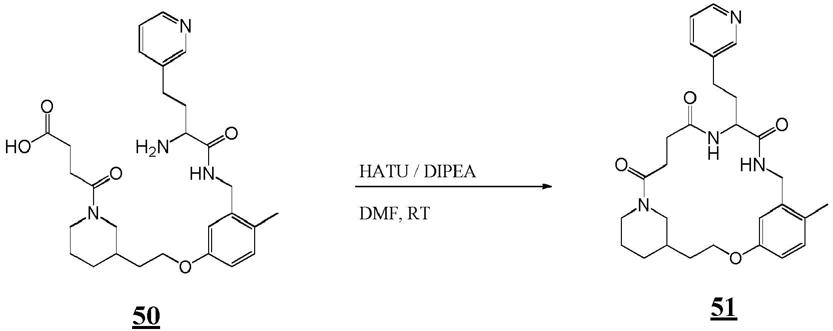

Macrocyclization was achieved according to general procedure A with 0.179 mmol amino acid **50**. Reaction mixtures were diluted with some methanol and purified via HPLC.
Formula: C₂₈H₃₆N₄O₄, exact mass: 492.3, found: 493.3 [M+H]⁺

### Example A-6: Preparation of macrocyclic compound 59

### tert-butyl 3-(2-(5-bromo-2,4-dimethylphenoxy)ethyl)piperidine-1-carboxylate (52)

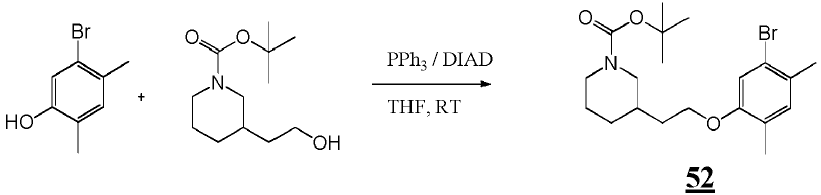

855 mg tert-butyl 3-(2-hydroxyethyl)piperidine-1-carboxylate, 500 mg 5-bromo-2,4-dimethylphenol and 978 mg PPh₃ were dissolved in 12 ml THF. 734 µl diisopropyl azodicarboxylate in 3 ml THF were added dropwise and the reaction was stirred for 2h at room temperature. The reaction was diluted with ethyl acetate and washed with saturated NaHCO₃ solution and brine. After drying the organic phase over MgSO₄ and removing volatiles under reduced pressure the crude was purified by normal phase flash chromatography.
Formula: C₂₀H₃₀BrNO₃, exact mass: 411.1, found: 414.1 [M+H]⁺

### tert-butyl 3-(2-(5-cyano-2,4-dimethylphenoxy)ethyl)piperidine-1-carboxylate (53)

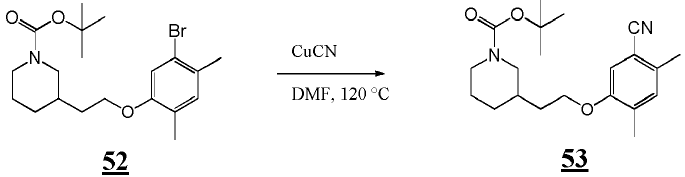

Bromide **52** (780 mg) and 340 mg CuCN in DMF were stirred at 120 °C for 4d. The mixture was diluted with ethyl acetate, washed with saturated NaHCO₃ solution and brine and dried over MgSO₄. Solvent was removed under reduced pressure and the residue was purified by normal phase column chromatography (silica, cyclohexane/ethyl acetate gradient).
Formula: C₂₁H₃₀N₂O₃, exact mass: 358.2, found: 358.3 [M+H]⁺

### tert-butyl 3-(2-(5-(aminomethyl)-2,4-dimethylphenoxy)ethyl)piperidine-1-carboxylate (54)

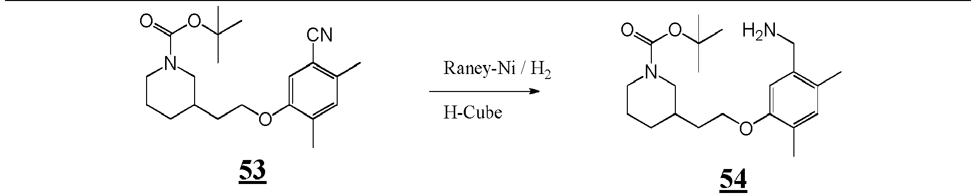

Nitrile **53** (432 mg) in 40 ml EtOH were reduced according to general procedure H in 4 cycles at 50 bar, 70 °C and 0.5 ml/min. After removing volatiles the residue was used without further purification.
Formula: C₂₁H₃₄N₂O₃, exact mass: 362.3, found: 363.3 [M+H]⁺

### tert-butyl 3-(2-(5-(((S)-2-(((benzyloxy)carbonyl)amino)-4-phenylbutanamido)methyl)-2,4-dimethylphenoxy)ethyl)piperidine-1-carboxylate (55)

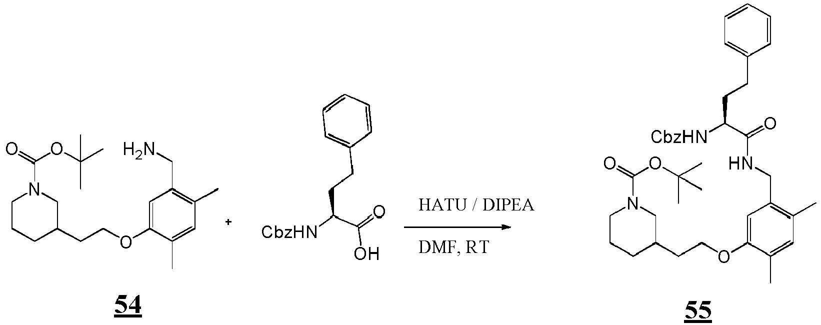

Synthesis according to general procedure B with 1.21 mmol amine **54** and 493 mg (S)-2-(((benzyloxy)carbonyl)amino)-4-phenylbutanoic acid. Purification was achieved by normal phase flash chromatography.
Formula: C₃₉H₅₁N₃O₆, exact mass: 657.4, found: 658.3 [M+H]⁺

### tert-butyl 3-(2-(5-(((S)-2-amino-4-phenylbutanamido)methyl)-2,4-dimethylphenoxy) ethyl)piperidine-1-carboxylate (56)

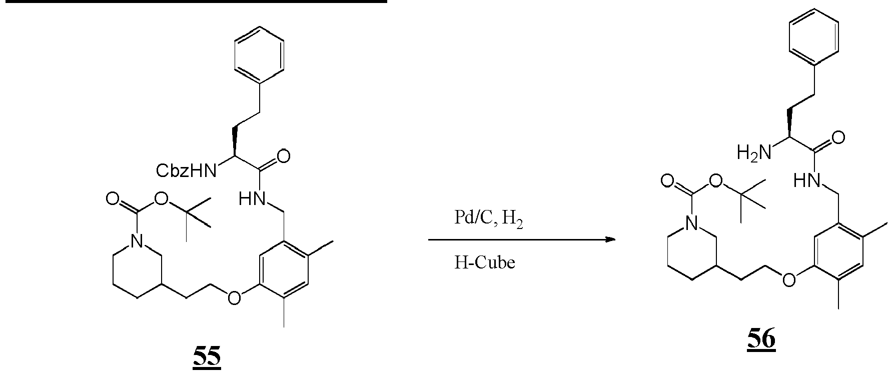

Synthesis according general procedure C with 695 mg protected amine in 80 ml ethanol / ethyl acetate (1:1) with 20 bar, 50°C and 0.5 ml/min. After removing volatiles the crude was used for the next step.
Formula: C₃₁H₄₅N₃O₄, exact mass: 523.3, found: 524.4 [M+H]⁺

### tert-butyl 3-(2-(5-(((S)-2-(4-(tert-butoxy)-4-oxobutanamido)-4-phenylbutanamido)methyl)-2,4-dimethylphenoxy)ethyl)piperidine-1-carboxylate (57)

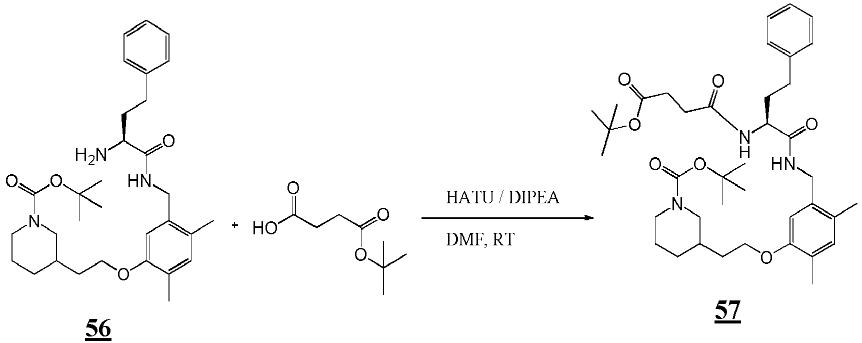

Synthesis according to general procedure B with 200 mg amine **56** and 100 mg mono-tert.-butyl succinate. Purification was achieved by normal phase flash chromatography. Formula: C₃₉H₅₇N₃O₇, exact mass: 679.4, found: 680.4 [M+H]⁺

### 4-(((2S)-1-((2,4-dimethyl-5-(2-(piperidin-3-yl)ethoxy)benzyl)amino)-1-oxo-4-phenylbutan-2-vl)amino)-4-oxobutanoic acid (58)

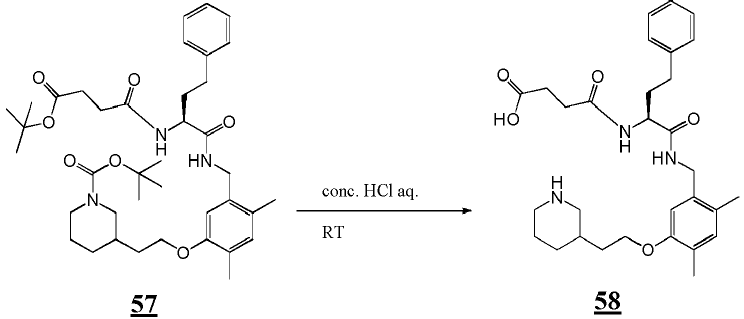

Both protecting groups were cleaved according to general procedure D in concentrated HCI in water at RT for 2h. Crude was coevaporated twice with acetonitrile and used without further purification.
Formula: C₃₀H₄₁N₃O₅, exact mass: 523.3, found: - [M+H]⁺

### (9R)-5⁴,5⁶-dimethyl-9-phenethyl-4-oxa-7,10-diaza-1(3,1)-piperidina-5(1,3)-benzenacyclotetradecaphane-8,11,14-trione (59)

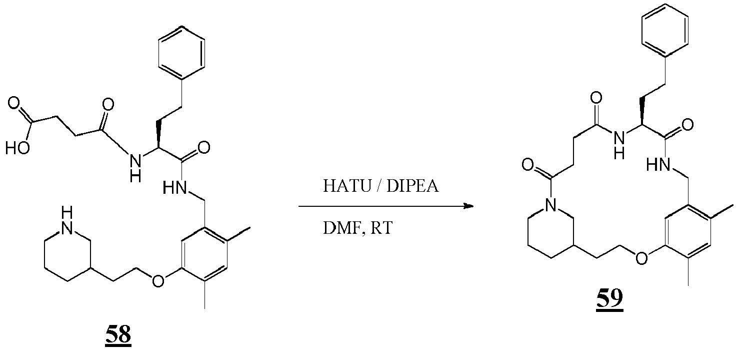

Macrocyclization was achieved according to general procedure A with 0.447 mmol amino acid **58.** Reaction mixtures were diluted with some methanol and purified via HPLC.
Formula: C₃₀H₃₉N₃O₄, exact mass: 505.3, found: 506.3 [M+H]⁺

### Example A-7: Preparation of macrocyclic compounds 77, 78, and 79

### (S)-tert-butyl 4-((1-(benzyloxy)-3-(1H-indol-3-yl)-1-oxopropan-2-yl)amino)-4-oxobutanoate (65)

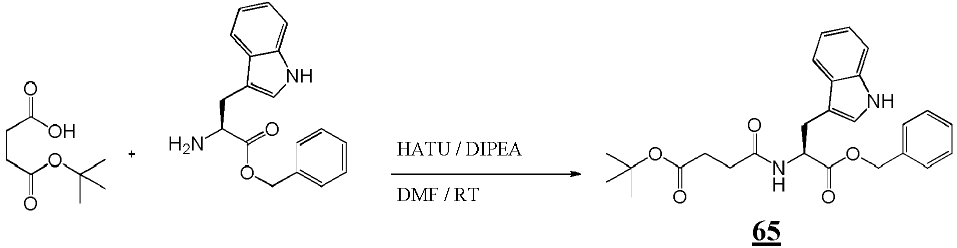

The amide bond was formed according to general procedure B with 1.02 g (S)-benzyl 2-amino-3-(1H-indol-3-yl)propanoate and 400 mg mono-tert.-butyl succinate. Purification was achieved by RP18 reversed phase flash chromatography.
Formula: C₂₆H₃₀N₂O₅, exact mass: 450.2, found: 451.3 [M+H]⁺

### (S)-tert-butyl 4-((1-(benzyloxy)-1-oxopropan-2-yl)amino)-4-oxobutanoate (66)

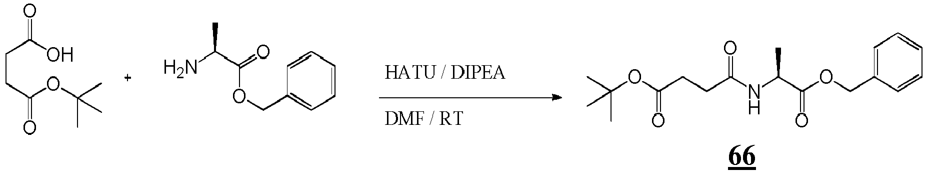

The amide bond was formed according to general procedure B with 744 g (S)-benzyl 2-aminopropanoate and 400 mg mono-tert.-butyl succinate. Purification was achieved by RP18 reversed phase flash chromatography.
Formula: C₁₈H₂₅NO₅, exact mass: 360.2, found: 361.2 [M+H]⁺

### (S)-tert-butyl 4-((1-(benzyloxy)-1-oxo-3-phenylpropan-2-yl)amino)-4-oxobutanoate (67)

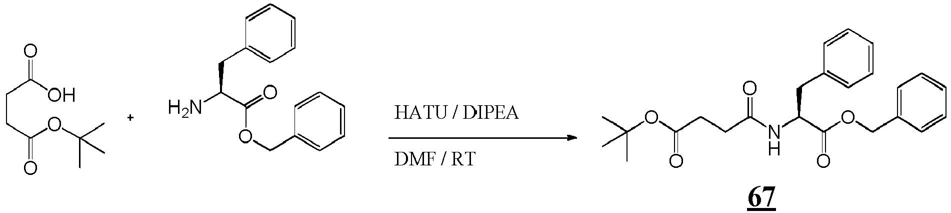

The amide bond was formed according to general procedure B with 1.01 g (S)-benzyl 2-amino-3-phenylpropanoate and 400 mg mono-tert.-butyl succinate. Purification was achieved by RP18 reversed phase flash chromatography.
Formula: C₂₄H₂₉NO₅, exact mass: 411.2, found: 412.3 [M+H]⁺

### (S)-2-(4-(tert-butoxy)-4-oxobutanamido)-3-(1H-indol-3-yl)propanoic acid (68)

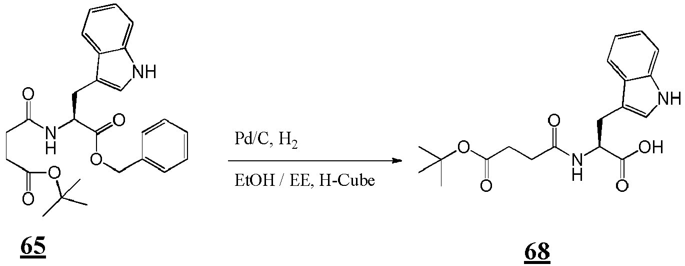

Benzyl ester was cleaved according to general procedure C using Raney-Ni catalyst cartridge, 20 bar at 50 °C in a solvent mixture of ethanol / ethyl acetate (3:1). Product was purified by normal phase column chromatography (silica, cyclohexane/ethyl acetate gradient). Formula: C₁₉H₂₄N₂O₅, exact mass: 360.2, found: 361.2 [M+H]⁺

### (S)-2-(4-(tert-butoxy)-4-oxobutanamido)propanoic acid (69)

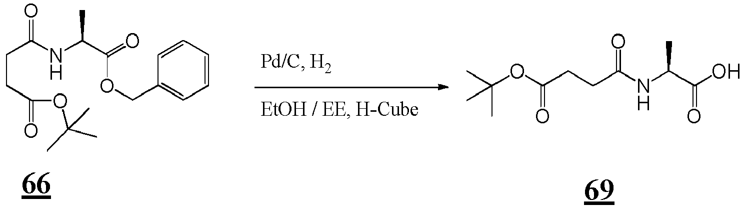

Benzyl ester was cleaved according to general procedure C using Raney-Ni catalyst cartridge, 20 bar at 50 °C in a solvent mixture of ethanol / ethyl acetate (3:1). Product was purified by normal phase column chromatography (silica, cyclohexane/ethyl acetate gradient). Formula: C₁₁H₁₉NO₅, exact mass: 245.1, found: 246.2 [M+H]⁺

### (S)-2-(4-(tert-butoxy)-4-oxobutanamido)-3-phenylpropanoic acid (70)

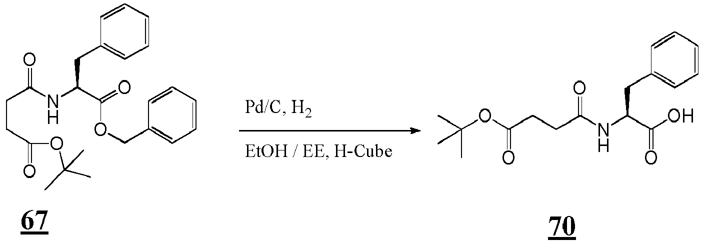

Benzyl ester was cleaved according to general procedure C using Raney-Ni catalyst cartridge, 20 bar at 50 °C in a solvent mixture of ethanol / ethyl acetate (3:1). Product was purified by normal phase column chromatography (silica, cyclohexane/ethyl acetate gradient). Formula: C₁₇H₂₃NO₅, exact mass: 321.2, found: 322.2 [M+H]⁺

### tert-butyl 3-(2-(3-(((S)-2-(4-(tert-butoxy)-4-oxobutanamido)-3-(1H-indol-3-yl)propanamido)methyl)-4-methylphenoxy)ethyl)piperidine-1-carboxylate (71)

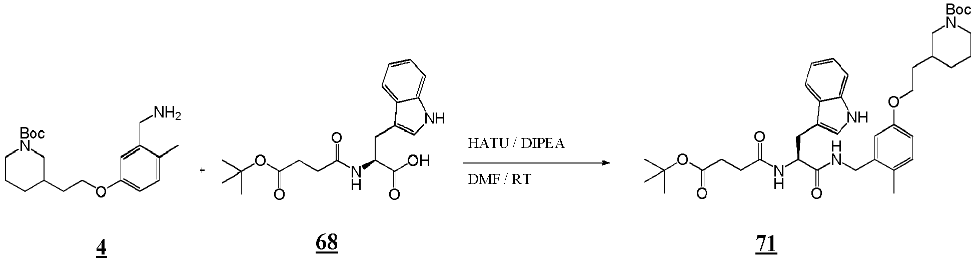

The amide bond was formed according to general procedure B with 100 mg amine **4** and 134 mg carboxylic acid **68.** Purification was achieved by RP18 reversed phase flash chromatography. Formula: C₃₉H₅₄N₄O₇, exact mass: 690.4, found: 691.6 [M+H]⁺

### tert-butyl 3-(2-(3-(((S)-2-(4-(tert-butoxy)-4-oxobutanamido)propanamido)methyl)-4-methylphenoxy)ethyl)piperidine-1-carboxylate (72)

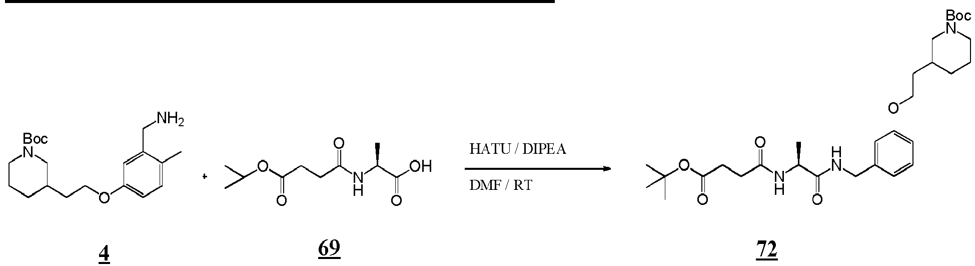

The amide bond was formed according to general procedure B with 100 mg amine **4** and 91 mg carboxylic acid 69. Purification was achieved by RP18 reversed phase flash chromatography. Formula: C₃₁H₄₉N₃O₇, exact mass: 575.4, found: 576.4 [M+H]⁺

### tert-butyl 3-(2-(3-(((S)-2-(4-(tert-butoxy)-4-oxobutanamido)-3-phenylpropanamido) methyl)-4-methylphenoxy)ethyl)piperidine-1-carboxylate (73)

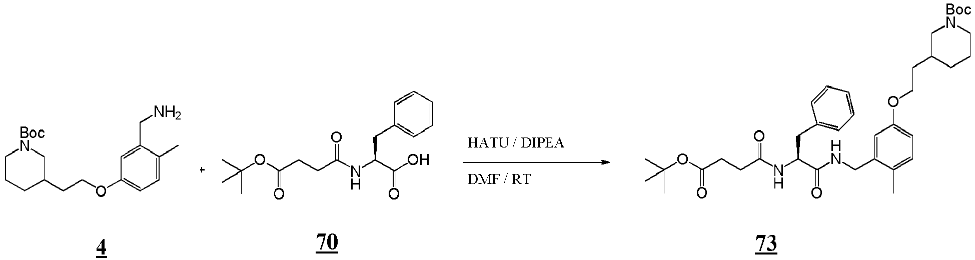

The amide bond was formed according to general procedure B with 100 mg amine **4** and 120 mg carboxylic acid **70.** Purification was achieved by RP18 reversed phase flash chromatography. Formula: C₃₇H₅₃N₃O₇, exact mass: 651.4, found: 652.4 [M+H]⁺

### 4-(((2S)-3-(1H-indol-3-yl)-1-((2-methyl-5-(2-(piperidin-3-yl)ethoxy)benzyl)amino)-1-oxopropan-2-vl)amino)-4-oxobutanoic acid (74)

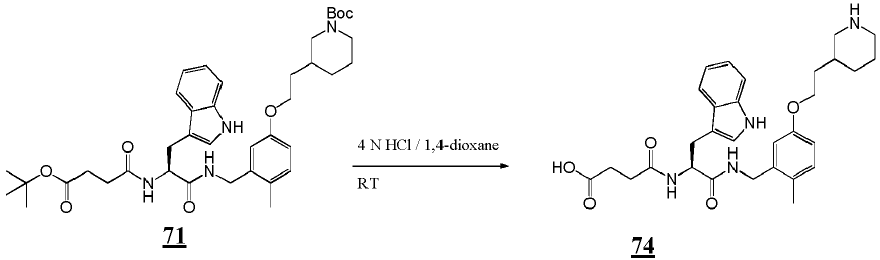

Protecting groups were cleaved according to general procedure D stirring compounds in 4 N HCI / 1,4-dioxane at room temperature. After deprotection was complete solvent was removed and the crude dissolved in acetonitrile and concentrated twice. Product used without further purification.
Formula: C₃₀H₃₈N₄O₅, exact mass: 534.3, found: 536.3 [M+H]⁺

### 4-(((2S)-1-((2-methyl-5-(2-(piperidin-3-yl)ethoxy)benzyl)amino)-1-oxopropan-2-yl)amino)-4-oxobutanoic acid (75)

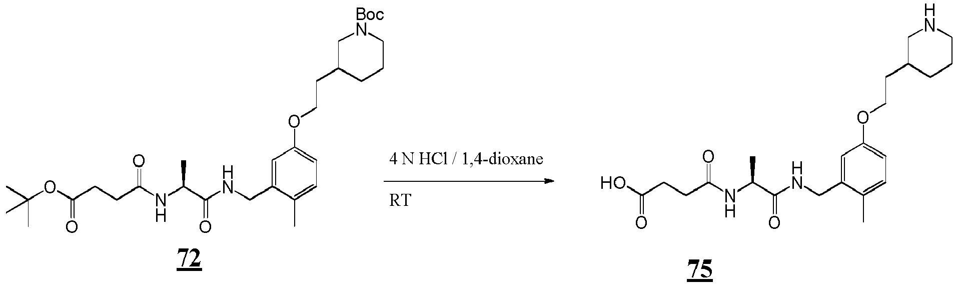

Protecting groups were cleaved according to general procedure D stirring compounds in 4 N HCI / 1,4-dioxane at room temperature. After deprotection was complete solvent was removed and the crude dissolved in acetonitrile and concentrated twice. Product used without further purification.
Formula: C₂₂H₃₃N₃O₅, exact mass: 419.2, found: 420.1 [M+H]⁺

### 4-(((2S)-1-((2-methyl-5-(2-(piperidin-3-yl)ethoxy)benzyl)amino)-1-oxo-3-phenylpropan-2-yl)amino)-4-oxobutanoic acid (76)

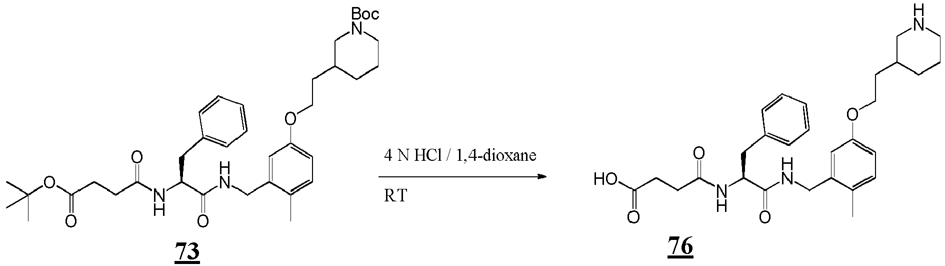

Protecting groups were cleaved according to general procedure D stirring compounds in 4 N HCI / 1,4-dioxane at room temperature. After deprotection was complete solvent was removed and the crude dissolved in acetonitrile and concentrated twice. Product used without further purification.
Formula: C₂₈H₃₇N₃O₅, exact mass: 495.3, found: 496.3 [M+H]⁺

### (9S)-9-((1H-indol-3-yl)methyl)-5⁴-methyl-4-oxa-7,10-diaza-1(3,1)-piperidina-5(1,3)-benzenacyclotetradecaphane-8,11,14-trione (77)

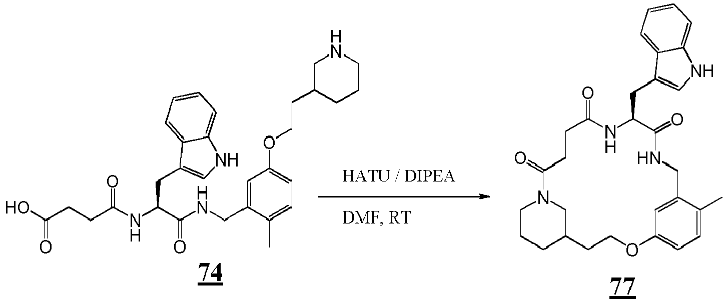

Macrocyclization was achieved according to general procedure A with 0.287 mmol amino acid **74** Reaction mixtures were diluted with some methanol and purified via HPLC. Formula: C₃₀H₃₆N₄O₄, exact mass: 516.3, found: 517.3 [M+H]⁺

### (9S)-5⁴,9-dimethyl-4-oxa-7,10-diaza-1(3,1)-piperidina-5(1,3)-benzenacyclotetradecaphane-8,11,14-trione (78)

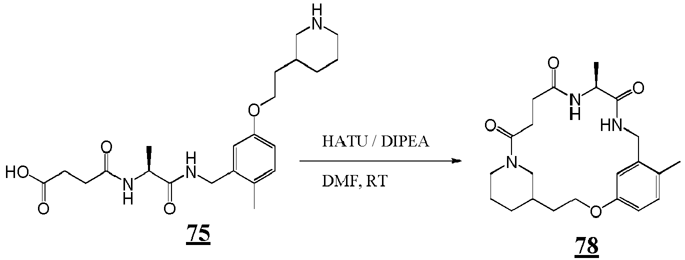

Macrocyclization was achieved according to general procedure A with 0.224 mmol amino acid **75** Reaction mixtures were diluted with some methanol and purified via HPLC. Formula: C₂₂H₃₁N₃O₄, exact mass: 401.2, found: 402.3 [M+H]⁺

### (9S)-9-benzyl-5⁴-methyl-4-oxa-7,10-diaza-1(3,1)-piperidina-5(1,3)-benzenacyclotetradecaphane-8,11,14-trione (79)

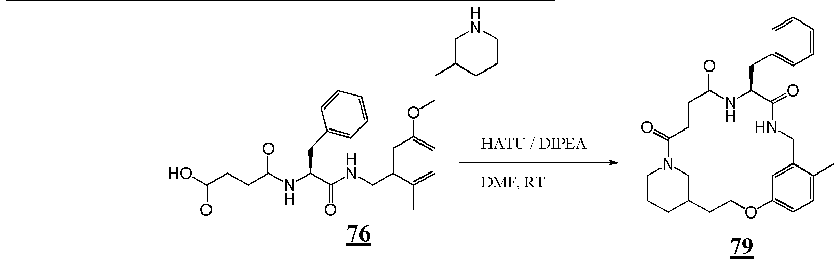

Macrocyclization was achieved according to general procedure A with 0.215 mmol amino acid **76** Reaction mixtures were diluted with some methanol and purified via HPLC. Formula: C₂₈H₃₅N₃O₄, exact mass: 477.3, found: 478.3 [M+H]⁺

### Example A-8: Preparation of macrocyclic compound 95

### tert-butyl 3-(2-(3-(((S)-2-(((benzyloxy)carbonyl)amino)-4-phenylbutanamido)methyl)-4,5-dimethylphenoxy)ethyl)piperidine-1-carboxylate (91)

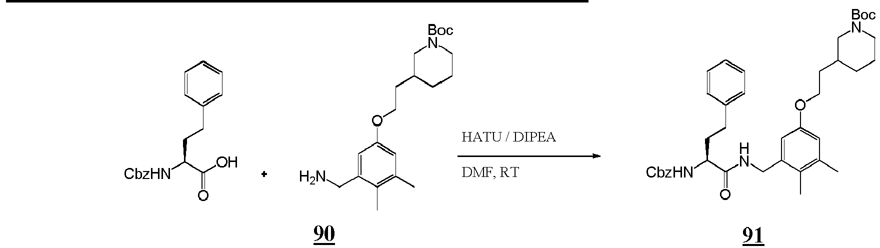

The amide bond was formed according to general procedure B. Purification was achieved normal phase column chromatography (silica, cyclohexane/ethyl acetate gradient). Formula: C₃₉H₅₁N₃O₆, exact mass: 657.4, found: 658.6 [M+H]⁺

### tert-butyl 3-(2-(3-(((S)-2-amino-4-phenylbutanamido)methyl)-4,5-dimethylphenoxy)ethyl) -piperidine-1-carboxylate (92)

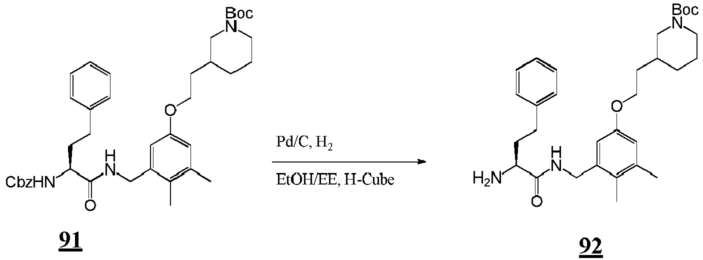

Benzyl carbamate was cleaved according to general procedure C using 10% Pd/C catalyst cartridge, 20 bar at 50 °C in a solvent mixture of ethanol / ethyl acetate (1:1). Dried product was used without further purification.
Formula: C₃₁H₄₅N₃O₄, exact mass: 523.3, found: 524.6 [M+H]⁺

### tert-butyl 3-(2-(3-(((S)-2-(4-(tert-butoxy)-4-oxobutanamido)-4-phenylbutanamido)methyl)-4,5-dimethylphenoxy)ethyl)piperidine-1-carboxylate (93)

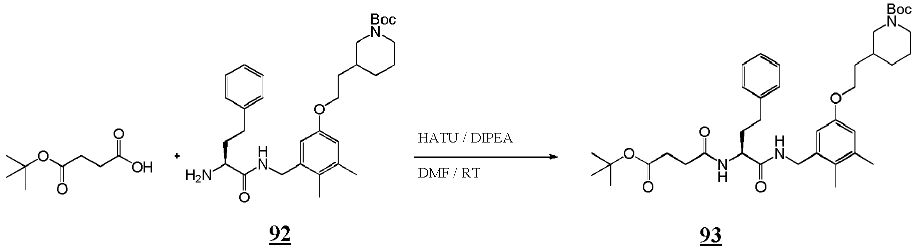

The amide bond was formed according to general procedure B with about 0.181 mmol amine **92** and 47 mg carboxylic acid. Purification was achieved by normal phase column chromatography (silica, cyclohexane/ethyl acetate gradient).
Formula: C₃₉H₅₇N₃O₇, exact mass: 679.4, found: 680.7 [M+H]⁺

### 4-(((2S)-1-((2,3-dimethyl-5-(2-(piperidin-3-yl)ethoxy)benzyl)amino)-1-oxo-4-phenylbutan-2-yl)amino)-4-oxobutanoic acid (94)

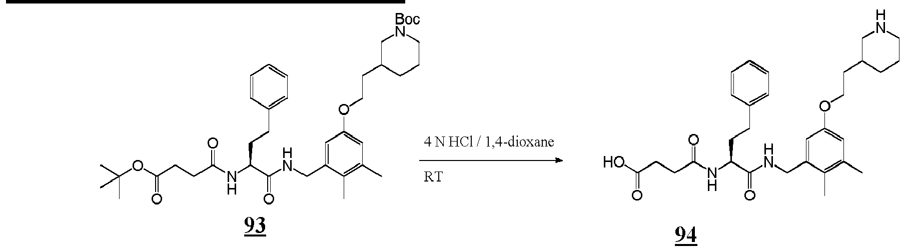

Protecting groups were cleaved according to general procedure D stirring compounds in 4 N HCI / 1,4-dioxane at room temperature. After deprotection was complete solvent was removed and the crude dissolved in acetonitrile and concentrated twice. Product used without further purification.
Formula: C₃₀H₄₁N₃O₅, exact mass: 523.3, found: 524.5 [M+H]⁺

### (9S)-5⁴,5⁵-dimethyl-9-phenethyl-4-oxa-7,10-diaza-1(3,1)-piperidina-5(1,3)-benzenacyclotetradecaphane-8,11,14-trione (95)

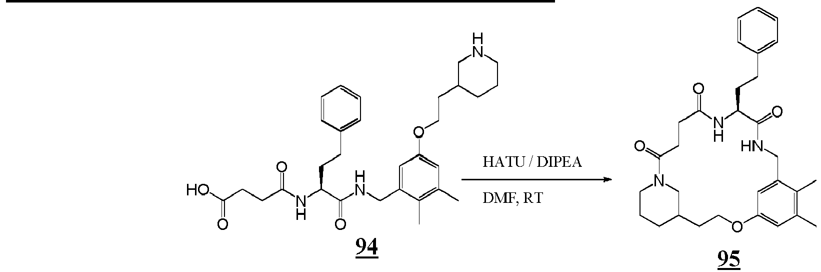

Macrocyclization was achieved according to general procedure A with about 0.125 mmol amino acid **94.** Reaction mixture was diluted with some methanol and purified via HPLC. Formula: C₃₀H₃₉N₃O₄, exact mass: 505.3, found: 506.4 [M+H]⁺

### Example A-9: Preparation of macrocyclic compounds 105, 106, 107, 371, and 372 2-vinylpyrazine (96)

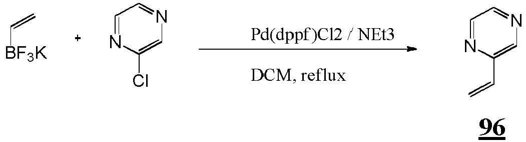

To a solution of 2-chloropyrazine (78 g, 681 mmol) in THF (800 mL) under argon atm. was added potassium vinyltrifluoroborate (137 g, 1022 mmol), triethylamine (247 mL, 2020 mmol), and Pd(dppf)Cl₂ in dichloromethane (5.6 g, 6.9 mmol). The reaction mass was refluxed for 24 h, cooled to r.t., diluted with MTBE (800 mL), and filtered through a pad of Na₂SO₄. The filtrate was evaporated under reduced pressure to obtain crude 2-vinylpyrazine (60 g, 565.5 mmol) which was used in the next step without further purification.

### diethyl 2-acetamido-2-(pyrazin-2-ylmethyl)malonate (97)

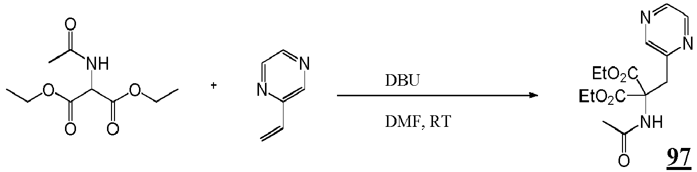

Diethyl acetamidomalonate (184.6 g, 850 mmol) and DBU (127 mL, 850 mmol) were dissolved in DMF (800 mL) and it was stirred 15 min at r.t. After that 2-vinylpyrazine (60 g, 565.5 mmol) was slowly added dropwise into reaction mixture and stirred for 24 h at r.t. Then it was concentrated under reduced pressure diluted with water (800 mL), and extracted with ethyl acetate (2x800 mL). Combined organic layers were washed with brine (3x800 mL), dried over Na₂SO₄, and concentrated to obtained 120 g of diethyl 2-acetamido-2-(pyrazin-2-ylmethyl)malonate (371 mmol, 65.6% yield) which was used in the next step without further purification.

### ethyl 2-amino-4-(pyrazin-2-yl)butanoate (98)

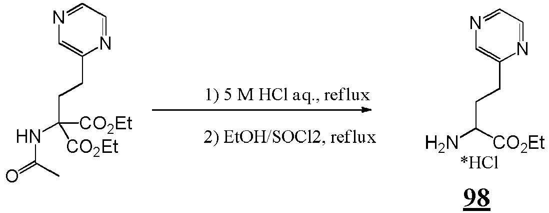

The diethyl 2-acetamido-2-(pyrazin-2-ylmethyl)malonate (120 g, 371 mmol) was dissolved in 5M hydrochloric acid (1000 mL) and refluxed for 14 h. Then the solvent was evaporated under reduced pressure to give 96 g of crude intermediate (441 mmol, 19% yield) which was used in the next step without further purification.

To cooled (5-10°C) solution of crude from step before (441 mmol) in absolute ethanol (500 mL) was slowly added dropwise SOCl2 (14.5 mL, 199 mmol) and stirred for 30 min. Then it was refluxed for 12 h without air access. Then the reaction mixture was concentrated under reduced pressure, and the obtained ethyl 2-amino-4-(pyrazin-2-yl)butanoate (100 g, 407 mmol) was used in the next step without further purification.
Formula: C₁₀H₁₅N₃O₂, exact mass: 209.1, found: 210.2 [M+H]⁺

### ethyl 2-(((benzyloxy)carbonyl)amino)-4-(pyrazin-2-yl)butanoate (99)

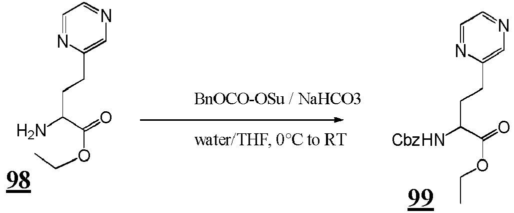

5.0 g amino ester **98** were dissolved in 25 ml water and 68 ml THF and cooled to 0°C. 5.07 g N-(benzyloxycarbonyloxy)succinimide and 8.24 g trimethylamine were added and the mixture was allow to warm to room temperature. After complete consumption of starting material the mixture was distributed between saturated NaHCO₃ solution and ethyl acetate. The organic phase was dried over MgSO₄ and volatiles were removed under reduced pressure. The crude was used without further purification.

### 2-(((benzyloxy)carbonyl)amino)-4-(pyrazin-2-yl)butanoic acid (100)

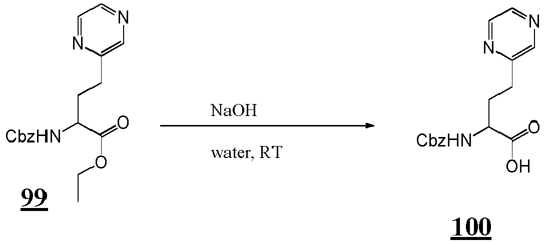

6.45 g ester **99** were dissolved in 47 ml 1,4-dioxane and 20 mL 1 M NaOH solution in water at room temperature. After 3h the mixture was brought to pH 3 with 1 M HCl solution in water. The mixture was extracted with ethyl acetate twice and combined organic phases were dried over MgSO₄. Volatiles were removed under reduced pressure. The crude was pure enough to be used in the following reactions.
Formula: C₁₆H₁₇N₃O₄, exact mass: 315.1, found: 316.1 [M+H]⁺

### tert-butyl 3-(2-(3-((2-(((benzyloxy)carbonyl)amino)-4-(pyrazin-2-yl)butanamido)methyl)-4-methylphenoxy)ethyl)piperidine-1-carboxylate (101)

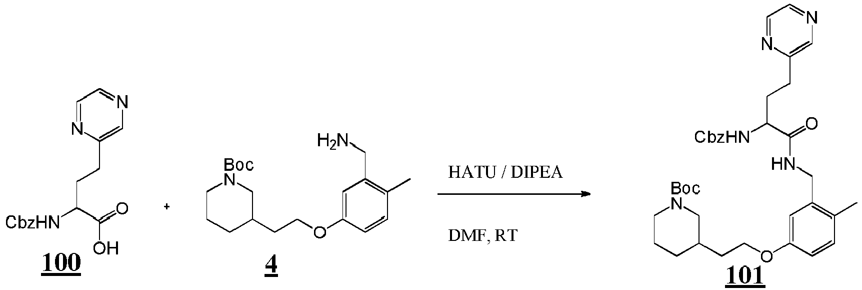

600 mg carboxylic acid **100** was coupled with 663 mg amine **4** according to general procedure B. Purification was achieved by reversed phase column chromatography (RP18, water/acetonitrile gradient).

### tert-butyl 3-(2-(3-((2-amino-4-(pyrazin-2-yl)butanamido)methyl)-4-methylphenoxy)ethyl) piperidine-1-carboxylate (102)

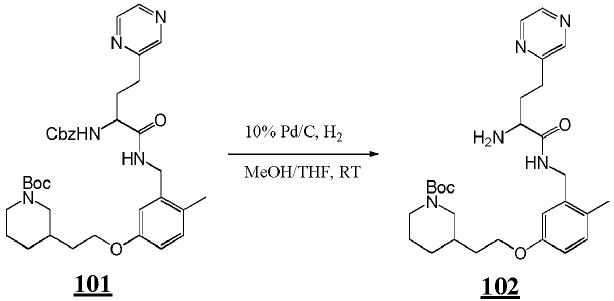

660 mg compound 101 and 70 mg 10% Pd/C in 8 ml methanol and 1.5 ml THF were hydrogenated at room temperature under normal pressure. After complete reaction the mixture was filtrated and volatiles were removed. Purification was achieved by normal phase column chromatography (silica, cyclohexane/ethyl acetate gradient).
Formula: C₂₈H₄₁N₅O₄, exact mass: 511.3, found: 512.3 [M+H]⁺

### tert-butyl 3-(2-(3-((5S)-5-(2-(tert-butoxy)-2-oxoethyl)-3,6,9-trioxo-1-phenyl-8-(2-(pyrazin-2-yl)ethyl)-2-oxa-4,7,10-triazaundecan-11-yl)-4-methylphenoxy)ethyl)piperidine-1-carboxylate (103)

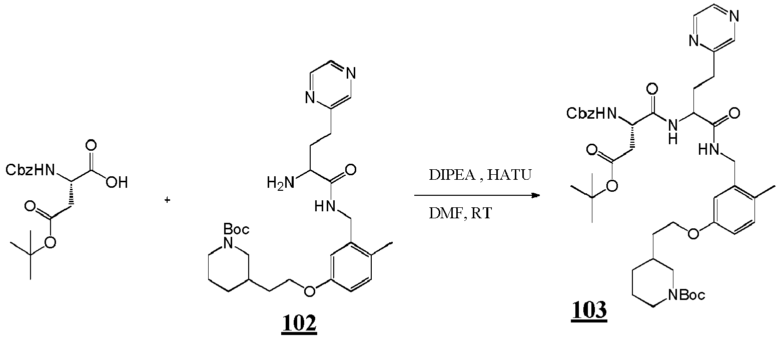

The amide bond was formed according to general procedure B with 243 mg amine **102** and 230 mg Cbz-Asp(OtBu)-OH. Purification was achieved by normal phase column chromatography (silica, cyclohexane/ethyl acetate gradient).
Formula: C₄₄H₆₀N₆O₉, exact mass: 816.4, found: 717.6 [M+H-Boc]⁺

### (3S)-3-(((benzyloxy)carbonyl)amino)-4-((1-((2-methyl-5-(2-(piperidin-3-yl)ethoxy)benzyl)amino)-1-oxo-4-(pyrazin-2-yl)butan-2-yl)amino)-4-oxobutanoic acid (104)

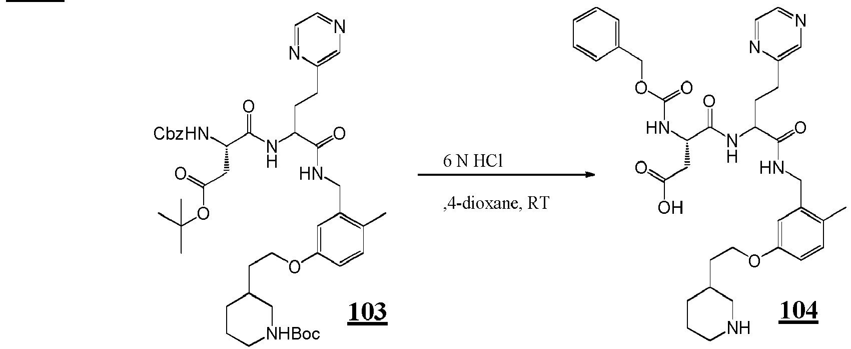

Protecting groups were cleaved from 280 mg compound **103** according to general procedure D stirring compounds in 10 ml 6 N HCl / 1,4-dioxane at room temperature. After deprotection was complete solvent was removed and the crude dissolved in acetonitrile and concentrated twice. Product used without further purification.
Formula: C₃₆H₄₄N₆O₇, exact mass: 660.3, found: 662.3 [M+H]⁺

### benzyl ((12S)-5⁴-methyl-8,11,14-trioxo-9-(2-(pyrazin-2-yl)ethyl)-4-oxa-7,10-diaza-1(3,1)-piperidina-5(1,3)-benzenacyclotetradecaphane-12-yl)carbamate (105)

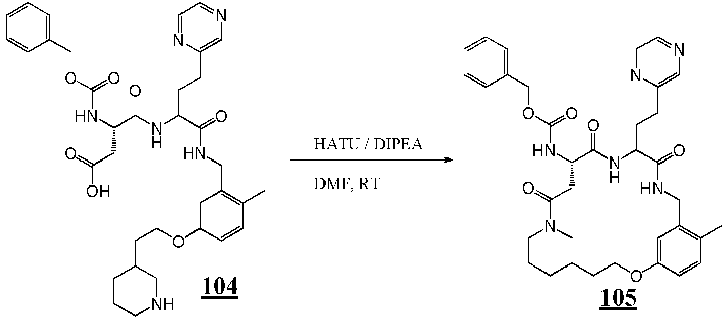

Macrocyclization was achieved according to general procedure A with 0.343 mmol amino acid 58. Reaction mixtures were diluted with some methanol and purified by reversed phase column chromatography (RP18, water/methanol gradient).
Formula: C₃₅H₄₂N₆O₆, exact mass: 642.3, found: 643.5 [M+H]⁺

### (12S)-amino-5⁴-methyl-8,11,14-trioxo-9-(2-(pyrazin-2-yl)ethyl)-4-oxa-7,10-diaza-1(3,1)-piperidina-5(1,3)-benzenacyclotetradecaphane (106)

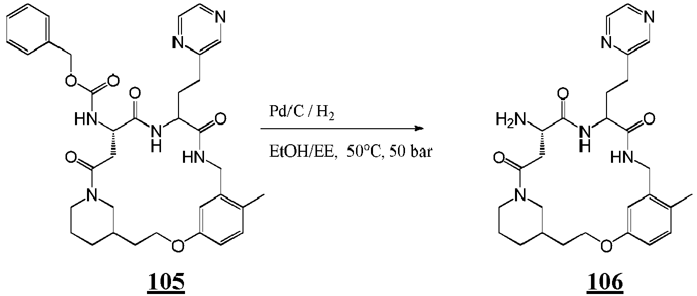

Benzyl carbamate was cleaved according to general procedure C using 10% Pd/C catalyst cartridge, 20 bar at 50 °C in a solvent mixture of ethanol / ethyl acetate (3:1). After removing of volatiles the residue was directly used in the next step.
Formula: C₂₇H₃₆N₆O₄, exact mass: 508.3, found: - [M+H]⁺

### (2S)-N-((12S)-5⁴-methyl-8,11,14-trioxo-9-(2-(pyrazin-2-yl)ethyl)-4-oxa-7,10-diaza-1(3,1)-piperidina-5(1,3)-benzenacyclotetradecaphane-12-yl)-2-phenylpropanamide (107)

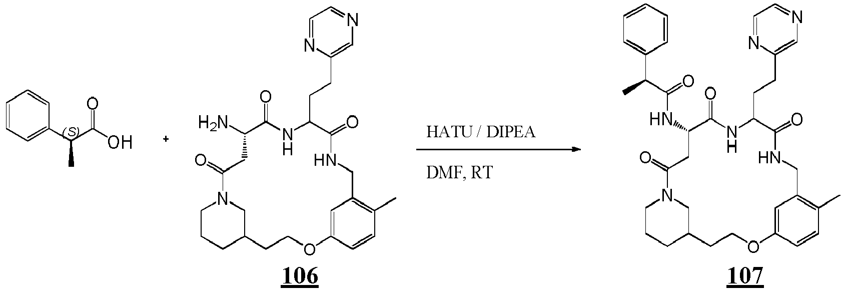

The amide bond was formed according to general procedure B with e.g. 25 mg amine **106** and 11 mg carboxylic acid. Purification was achieved by revered phase HPLC.
Formula: C₃₆H₄₄N₆O₅, exact mass: 640.3, found: 641.4 [M+H]⁺

Additional examples as exemplified by compound **107** are disclosed in the table below. In the cases of compounds **371** and **372** Boc groups were cleaved from the related Boc-protected intermediates with 40% TFA in DCM before final purification via HPLC.

**Table A-5**

| **Cpd no** | **structure** | **name** | Exact mass | [M+H]⁺ found |
|---|---|---|---|---|
| **107** | 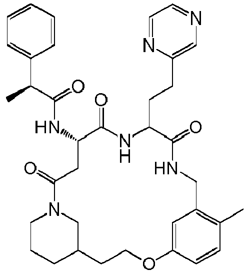 | (2S)-N-((12S)-5⁴-methyl-8,11,14-trioxo-9-(2-(pyrazin-2-yl)ethyl)-4-oxa-7,10-diaza-1(3,1)-piperidina-5(1,3)-benzenacyclotetradecaphane-12-yl)-2-phenylpropanamide | 640.3 | 641.4 |
| | | | C₃₆H₄₄ N₆O₅ | |
| **371** | 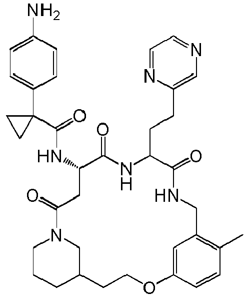 | 1-(4-aminophenyl)-N-((9S,12S)-5⁴-methyl-8,11,14-trioxo-9-(2-(pyrazin-2-yl)ethyl)-4-oxa-7,10-diaza-1 (3,1)-piperidina-5(1,3)-benzenacyclotetradecaphane-12-yl)cyclopropane-1-carboxam ide | 667.3 | 668.5 |
| | | | C₃₇H₄₅ N₇O₅ | |
| **372** | 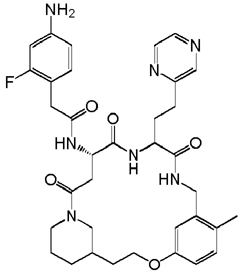 | 2-(4-amino-2-fluorophenyl)-N-((9S,12S)-5⁴-methyl-8,11, 14-trioxo-9-(2-(pyrazin-2-yl)ethyl)-4-oxa-7,10-diaza-1(3,1)-piperidina-5(1,3)-benzenacyclotetradecaphane-12-yl)acetamide | 659.3 | 660.4 |
| | | | C₃₅H₄₂F N₇O₅ | |

### Example A-10: Preparation of compound (116)

### ethyl 4-(3-(2-(3-(aminomethyl)-4-methylphenoxy)ethyl)piperidin-1-yl)-4-oxobutanoate (112)

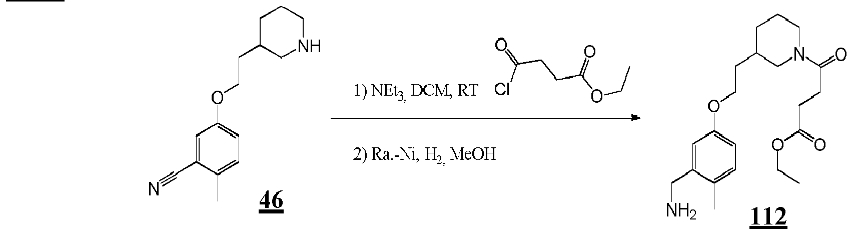

2.48 g amine **46** and 5.64 ml NEt₃ were dissolved in 60 ml DCM and cooled to 0°C. 1.86 g ethyl 4-chloro-4-oxobutanoate were added dropwise and the mixture was allowed to come to room temperature. After complete consumption of the starting amine saturated NaHCO₃ solution was added and the mixture was extracted with DCM. The organic phase was dried and concentrated under reduced pressure. Purification of the residue was achieved by normal phase column chromatography (silica, cyclohexane/ethyl acetate gradient).

2.0 g of the intermediate ester were dissolved in 135 ml methanol and hydrogenated with 7.0 Raney nickel under 50 bar at 70 °C. The mixture was filtered and concentrated under reduced pressure. The residue was pure enough to be used in the following reactions (mixture of methyl and ethyl ester).
Formula: C₂₁H₃₂N₂O₄, exact mass: 376.2, found: 377.3 [M+H]⁺

### ethyl 2-amino-2-(1-methyl-1H-pyrazol-4-yl)acetate (109)

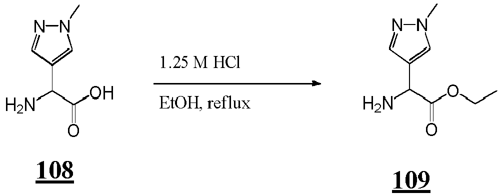

3.75 g 2-amino-2-(1-methyl-1H-pyrazol-4-yl)acetic acid are refluxed in 60 ml 1.25 M HCl in ethanol until esterification was found to be complete. Volatiles were removed under reduced pressure and the residue was coevaporated with acetonitrile twice. The crude was used without further purification.
Formula: C₈H₁₃N₃O₂, exact mass: 183.1, found: 184.3 [M+H]⁺

### ethyl 2-(((benzyloxy)carbonyl)amino)-2-(1-methyl-1H-pyrazol-4-yl)acetate (110)

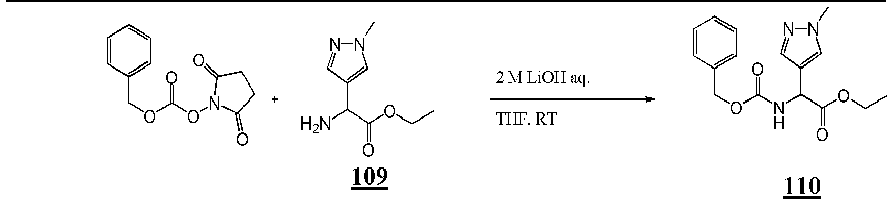

24 mmol amino ester **109** was dissolved in THF/water (3:1) at 0°C. 6.0 g CbzOSu and 13.1 ml NEt₃ were added and the mixtures was allowed to come to room temperature. The mixture was distributed between ethyl acetate and saturated NaHCO₃ solution. The organic phase was washed with 2 N HCl, dried over Na₂SO₄ and concentrated. The crude was purified by normal phase column chromatography (silica, cyclohexane/ethyl acetate gradient) to give 3.54 g ester **110.**
Formula: C₁₆H₁₉N₃O₄, exact mass: 317.1, found: 318.3 [M+H]⁺

### 2-(((benzyloxy)carbonyl)amino)-2-(1-methyl-1H-pyrazol-4-yl)acetic acid (111)

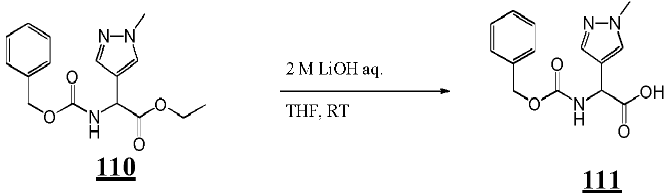

3.4 g ester **110** were dissolved in 12 ml THF and 4 ml 2 M LiOH aq. were added. Some additional water was added to obtain only one phase. After completion of reaction the mixture was washed with ethyl acetate and the aqueous phase was acified to pH 2. Extraction with ethyl acetate and removing the solvent under reduced pressure afforded 2.1 g Cbz protected amino acid **111**.
Formula: C₁₄H₁₅N₃O₄, exact mass: 289.1, found: 290.3 [M+H]⁺

### ethyl 4-(3-(2-(3-((2-(((benzyloxy)carbonyl)amino)-2-(1-methyl-1H-pyrazol-4-yl)acetamido)methyl)-4-methylphenoxy)ethyl)piperidin-1-yl)-4-oxobutanoate (113)

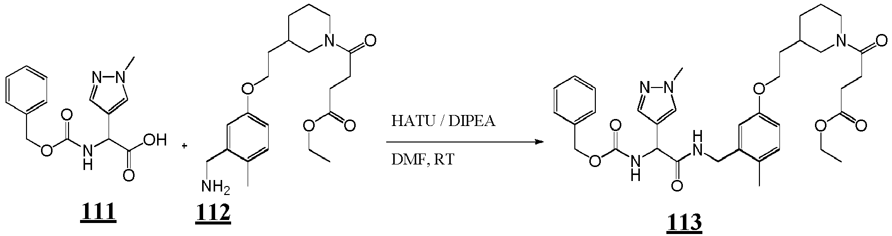

Peptide coupling was done according to general procedure B with 468 mg amine **112** and 300 mg amino acid **111.** Purification was achieved by normal phase column chromatography (silica, cyclohexane/ethyl acetate gradient).
Formula: C₃₅H₄₅N₅O₇, exact mass: 647.3, found: 648.3 [M+H]⁺

### ethyl 4-(3-(2-(3-((2-amino-2-(1-methyl-1H-pyrazol-4-yl)acetamido)methyl)-4-methylphenoxy) ethyl)piperidin-1-yl)-4-oxobutanoate (114)

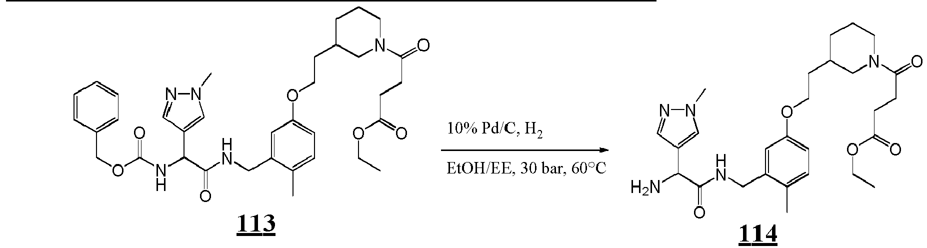

From 647 mg compound **113** the benzyl carbamate was cleaved according to general procedure C using 10% Pd/C catalyst cartridge, 30 bar at 60 °C in a solvent mixture of ethanol / ethyl acetate (1:1). Product was used without further purification after removing the volatiles.
Formula: C₂₇H₃₉N₅O₅, exact mass: 513.3, found: 514.3 [M+H]⁺

### 4-(3-(2-(3-((2-amino-2-(1-methyl-1H-pyrazol-4-yl)acetamido)methyl)-4-methylphenoxy)ethyl)piperidin-1-yl)-4-oxobutanoic acid (115)

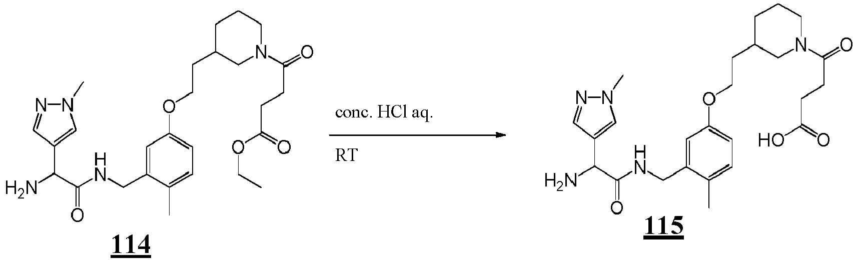

1.04 mmol ester **114** were dissolved in 20 ml concentrated aqueous HCl solution at room temperature. After complete reaction volatiles were removed under reduced pressure and the crude was directly used in the next step.
Formula: C₂₂H₃₅N₅O₅, exact mass: 485.3, found: 486.2 [M+H]⁺

### 5⁴-methyl-9-(1-methyl-1H-pyrazol-4-yl)-4-oxa-7,10-diaza-1(3,1)-piperidina-5(1 ,3)-benzenacyclotetradecaphane-8,11,14-trione (116)

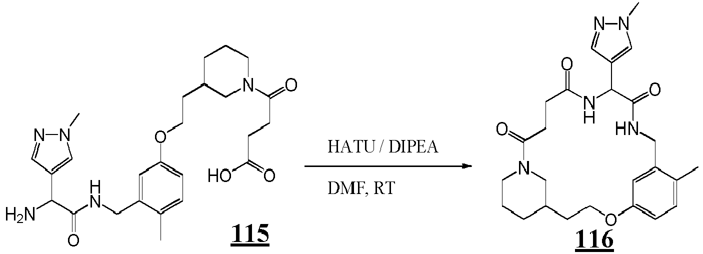

Macrocyclization was achieved according to general procedure A with 1.04 mmol amino acid **115.** Reaction mixtures were diluted with some methanol and purified via HPLC.
Formula: C₂₅H₃₃N₅O₄, exact mass: 467.3, found: 468.3 [M+H]⁺

### Example A-11: Preparation of compound (120)

### 2,3-dimethyl-5-(2-(piperidin-3-yl)ethoxy)benzonitrile (574)

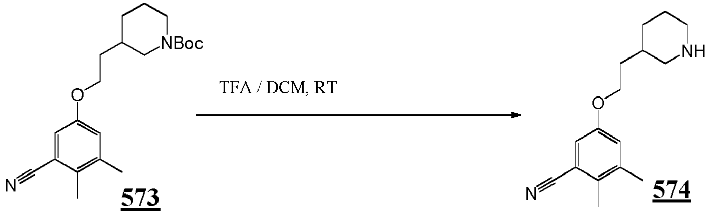

Boc group was removed from 1.77 g of compound **573** according general procedure D using 2.8 g TFA in 49 ml DCM. Volatiles were removed, the residue was dissolved in DCM and washed with 5 % aqueous NaOH solution. The organic phase was dried over MgSO₄, filtered and the solvent was removed under reduced pressure. The crude was used without further purification.

### ethyl 4-(3-(2-(3-cyano-4,5-dimethylphenoxy)ethyl)piperidin-1-yl)-4-oxobutanoate (575)

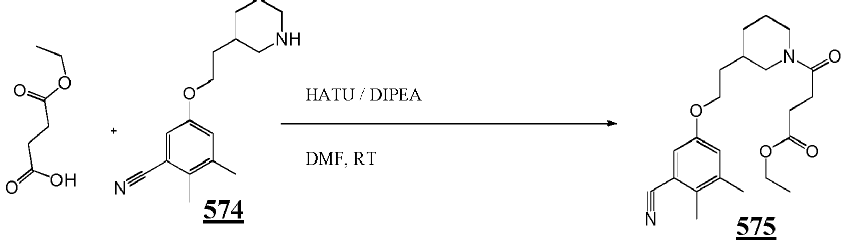

The amide bond was formed according to general procedure B with amine **574.** Purification was achieved by normal phase flash chromatography.

### ethyl 4-(3-(2-(3-(aminomethyl)-4,5-dimethylphenoxy)ethyl)piperidin-1-yl)-4-oxobutanoate (117)

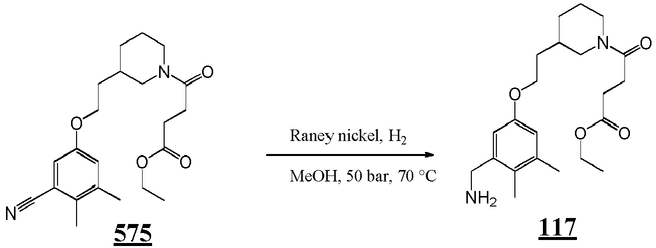

536 mg of the intermediate nitrile **575** were dissolved in 36 ml methanol and hydrogenated with 1.9 g Raney nickel at 70 °C and 50 bar. The mixture was filtered and concentrated under reduced pressure. The residue was pure enough to be used in the following reactions (mixture of methyl and ethyl ester).
Formula: C₂₂H₃₄N₂O₄, exact mass: 390.3, found: 391.3 [M+H]⁺

### tert-butyl 3-((2S)-2-((tert-butoxycarbonyl)amino)-3-((5-(2-(1-(4-ethoxy-4 oxobutanoyl)piperidin-3-yl)ethoxy)-2,3-dimethylbenzyl)amino)-3-oxopropyl)-1H-indole-1-carboxylate (118)

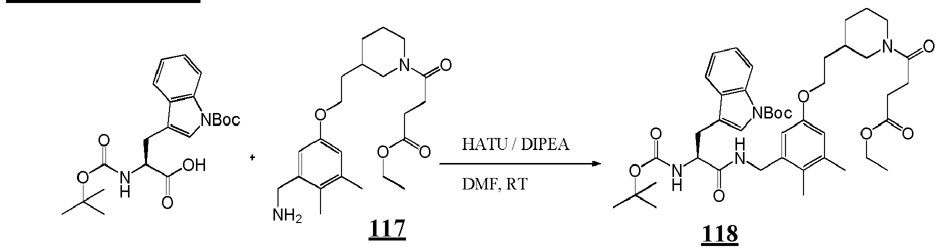

Condensation was achieved according to general procedure B with 232 mg amine **117** and 312 mg Boc protected amino acid. Purification was achieved by normal phase column chromatography (silica, cyclohexane/ethyl acetate gradient).
Formula: C₄₃H₆₀N₄O₉, exact mass: 776.4, found: 777.5 [M+H]⁺

### 4-(3-(2-(3-(((S)-2-amino-3-(1H-indol-3-yl)propanamido)methyl)-4,5 dimethylphenoxy)ethyl)piperidin-1-yl)-4-oxobutanoic acid (119)

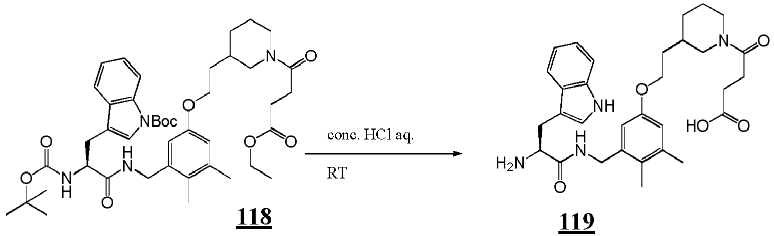

Protecting groups were cleaved from 240 mg compound **118** according to general procedure D stirring compounds in 10 ml concentrated aqueous HCl solution at room temperature. After deprotection was complete solvent was removed and the crude dissolved in acetonitrile and concentrated twice. Product used without further purification.
Formula: C₃₁H₄₀N₄O₅, exact mass: 548.3, found: 549.4 [M+H]⁺

### (9S)-9-((1H-indol-3-yl)methyl)-5⁴,5⁵-dimethyl-4-oxa-7,10-diaza-1(3,1)-piperidina-5(1,3)-benzenacyclotetradecaphane-8,11,14-trione (120)

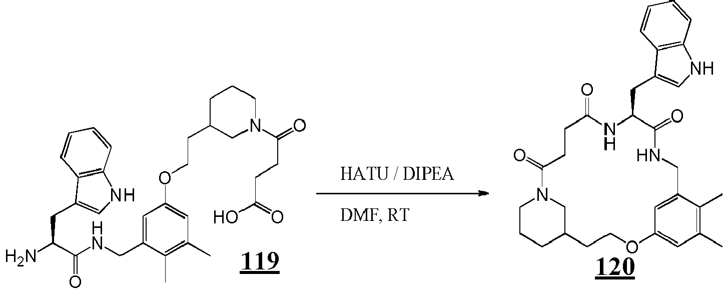

Macrocyclization was achieved according to general procedure A with 0.309 mmol amino acid **119.** Reaction mixture was diluted with some methanol and purified via HPLC.
Formula: C₃₁H₃₈N₄O₄, exact mass: 530.3, found: 531.4 [M+H]⁺

### Example A-12: Derivatizations of compound 8 with Boc-protected amino acids

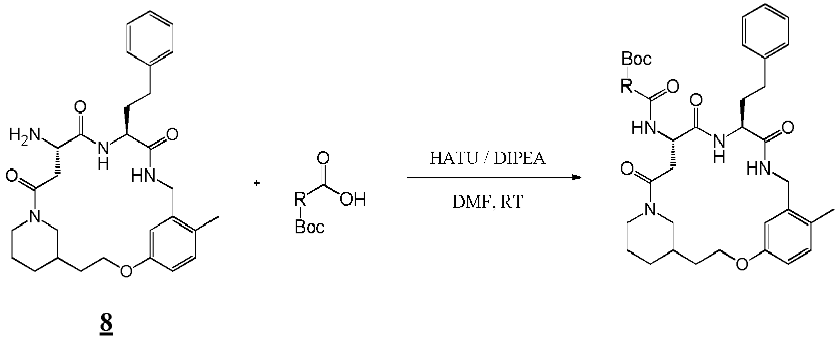

Amine **8** (e.g. 20 to 100 mg) was coupled with carboxylic acids according to general procedure B to give amides disclosed in table below. Purifications were achieved by normal phase column chromatography (silica, cyclohexane/ethyl acetate gradient).

**Table A-6**

| **Cpd no** | **structure** | **name** | Exact mass | [M+H]⁺ found |
|---|---|---|---|---|
| **121** | 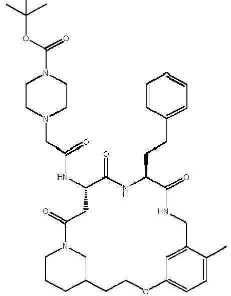 | tert-butyl 4-(2-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)amino)-2-oxoethyl)piperazine-1-carboxylate | 732.4 | 733.5 |
| | | | C₄₀H₅₆ N₆O₇ | |
| **122** | 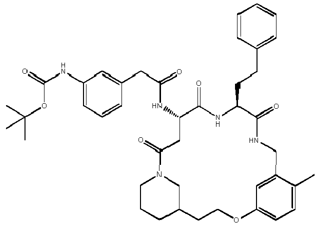 | tert-butyl (3-(2-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)amino)-2-oxoethyl)phenyl)carbamate | 739.4 | 740.6 |
| | | | C₄₂H₅₃ N₅O₇ | |
| **123** | 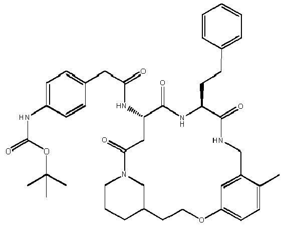 | tert-butyl (4-(2-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)amino)-2-oxoethyl)phenyl)carbamate | 739.4 | 740.7 |
| | | | C₄₂H₅₃ N₅O₇ | |
| **124** | 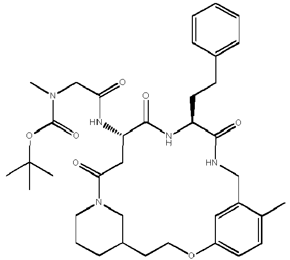 | tert-butyl methyl(2-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)amino)-2-oxoethyl)carbamate | 677.4 | 678.5 |
| | | | C₃₇H₅₁ N₅O₇ | |
| **125** | 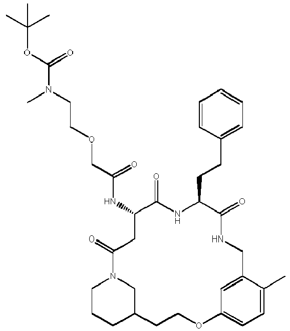 | tert-butyl methyl(2-(2-(((9S, 12S)-5⁴-methyl-8,11,14-trioxo-9-phenethyl-4-oxa-7,10-diaza-1(3,1)-piperidina-5(1,3)-benzenacyclotetradecaphane-12-yl)amino)-2-oxoethoxy)ethyl) carbamate | 721.4 | 722.6 |
| | | | C₃₉H₅₅ N₅O₈ | |
| **131** | 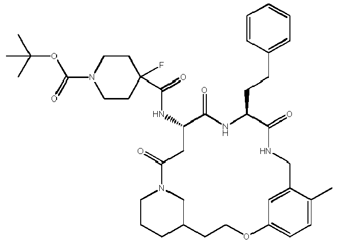 | tert-butyl 4-fluoro-4-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)piperidine-1-carboxylate | 735.4 | 736.7 |
| | | | C₄₀H₅₄ FN₅O₇ | |
| **132** | 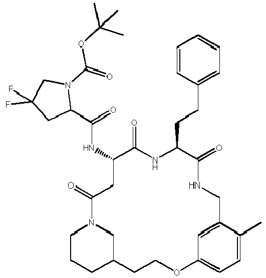 | tert-butyl 4,4-difluoro-2-(((9S,12S)-5⁴-methyl-8, 11,14-trioxo-9-phenethyl-4-oxa-7,10-diaza-1(3,1)-piperidina-5(1,3)-benzenacyclotetradecaphane-12-yl)carbamoyl)pyrrolidine-1-carboxylate | 739.4 | 740.6 |
| | | | C₃₉H₅₁ F₂N₅O₇ | |
| **133** | 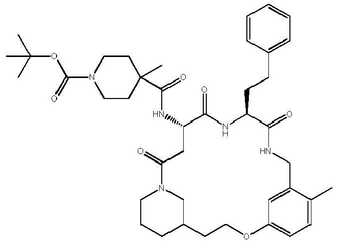 | tert-butyl 4-methyl-4-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)piperidine-1-carboxylate | 731.4 | 732.8 |
| | | | C₄₁H₅₇ N₅O₇ | |
| **149** | 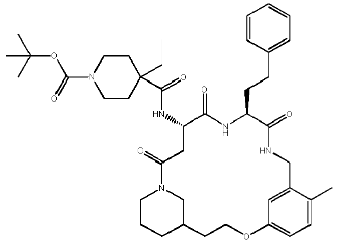 | tert-butyl 4-ethyl-4-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)piperidine-1-carboxylate | 745.4 | 746.7 |
| | | | C₄₂H₅₉ N₅O₇ | |
| **150** | 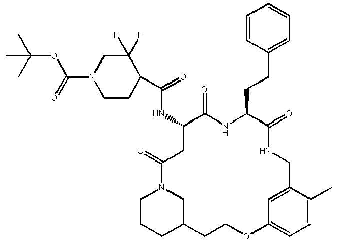 | tert-butyl 3,3-difluoro-4-(((9S,12S)-5⁴-methyl-8, 11,14-trioxo-9-phenethyl-4-oxa-7,10-diaza-1(3,1)-piperidina-5(1,3)-benzenacyclotetradecaphane-12-yl)carbamoyl)piperidine-1-carboxylate | 753.4 | 754.6 |
| | | | C₄₀H₅₃ F₂N₅O₇ | |
| **153** | 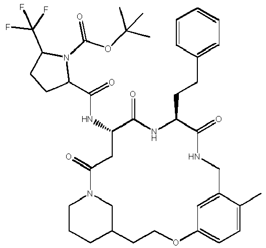 | tert-butyl 2-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)-5-(trifluoromethyl)pyrrolidine-1-carboxylate | 771.4 | 772.5 |
| | | | C₄₀H₅₂ F₃N₅O₇ | |
| **154** | 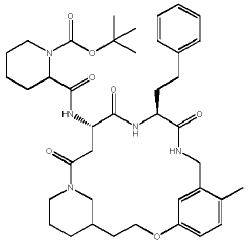 | tert-butyl 2-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)piperidine-1-carboxylate | 717.4 | 718.5 |
| | | | C₄₀H₅₅ N₅O₇ | |
| **155** | 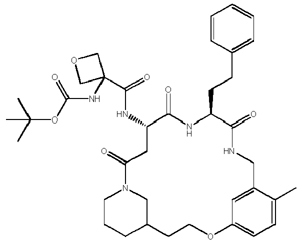 | tert-butyl (3-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)oxetan-3-yl)carbamate | 705.4 | 706.4 |
| | | | C₃₈H₅₁ N₅O₈ | |
| **156** | 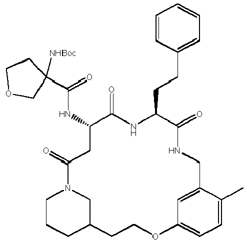 | tert-butyl (3-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)tetrahydrofuran-3-yl)carbamate | 719.4 | 720.5 |
| | | | C₃₉H₅₃ N₅O₈ | |
| **161** | 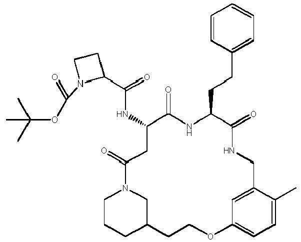 | tert-butyl 2-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)azetidine-1-carboxylate | 689.4 | 690.6 |
| | | | C₃₈H₅₁ N₅O₇ | |
| **178** | 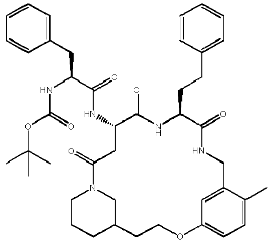 | tert-butyl ((2S)-1-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)amino)-1-oxo-3-phenylpropan-2-yl)carbamate | 753.4 | 654.4 [M+H-Boc]+ |
| | | | C₄₃H₅₅ N₅O₇ | |
| **387** | 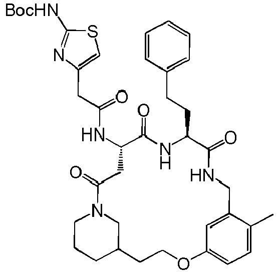 | tert-butyl (4-(2-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)amino)-2-oxoethyl)thiazol-2-yl)carbamate | 746.3 | 747.1 |
| | | | C₃₉H₅₀ N₆O₇S | |
| **388** | 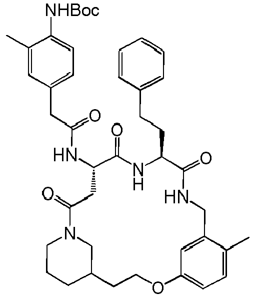 | tert-butyl (2-methyl-4-(2-(((9S,12S)-5⁴-methyl-8, 11,14-trioxo-9-phenethyl-4-oxa-7,10-diaza-1(3,1)-piperidina-5(1,3)-benzenacyclotetradecaphane-12-yl)amino)-2-oxoethyl) phenyl)carbamate | 753.4 | 754.4 |
| | | | C₄₃H₅₅ N₅O₇ | |
| **389** | 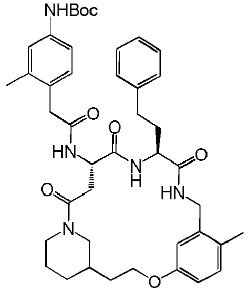 | tert-butyl (3-methyl-4-(2-(((9S,12S)-5⁴-methyl-8, 11,14-trioxo-9-phenethyl-4-oxa-7,10-diaza-1(3,1)-piperidina-5(1,3)-benzenacyclotetradecaphane-12-yl)amino)-2-oxoethyl)phenyl)carbamate | 753.4 | 754.4 |
| | | | C₄₃H₅₅ N₅O₇ | |
| **390** | 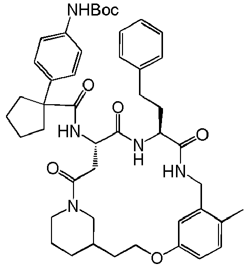 | tert-butyl (4-(1-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)cyclopentyl)phenyl)carbamate | 793.4 | 794.4 |
| | | | C₄₆H₅₉ N₅O₇ | |
| **391** | 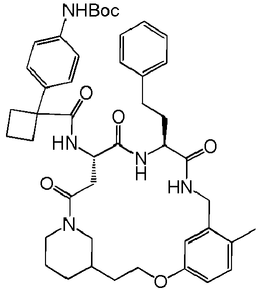 | tert-butyl (4-(1-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)cyclobutyl)phenyl)carbamate | 779.4 | 780.4 |
| | | | C₄₅H₅₇ N₅O₇ | |
| **392** | 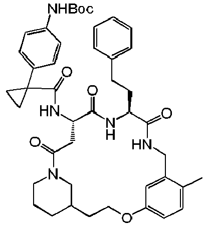 | tert-butyl (4-(1-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)cyclopropyl)phenyl)carbamate | 765.4 | 766.4 |
| | | | C₄₄H₅₅ N₅O₇ | |
| **393** | 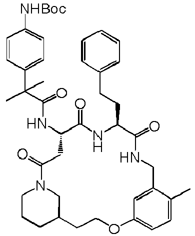 | tert-butyl (4-(2-methyl-1-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)amino)-1-oxopropan-2-yl)phenyl)carbamate | 767.4 | 768.4 |
| | | | C₄₄H₅₇ N₅O₇ | |
| **394** | 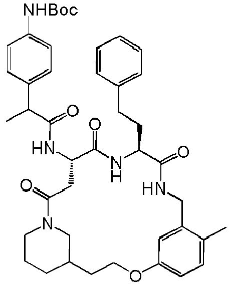 | 2-(4-aminophenyl)-N-((9S, 12S)-5⁴-methyl-8,11,14-trioxo-9-phenethyl-4-oxa-7,10-diaza-1(3,1)-piperidina-5(1,3)-benzenacyclotetradecaphane-12-yl)propanamide | 653.4 | 654.4 |
| | | | C₃₈H₄₇ N₅O₅ | |
| **395** | 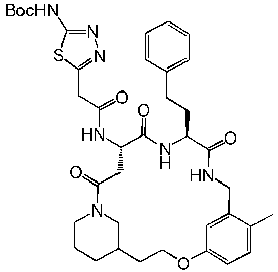 | tert-butyl (5-(2-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)amino)-2-oxoethyl)-1,3,4-thiadiazol-2-yl)carbamate | 747.3 | 748.4 |
| | | | C₃₈H₄₉ N₇O₇S | |
| **396** | 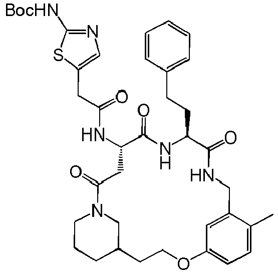 | tert-butyl (5-(2-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)amino)-2-oxoethyl)thiazol-2-yl)carbamate | 746.3 | 747.4 |
| | | | C₃₉H₅₀ N₆O₇S | |
| **419** | 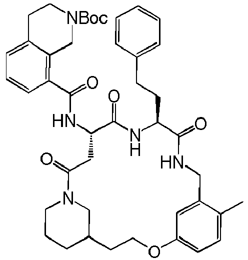 | tert-butyl 8-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)-3,4-dihydroisoquinoline-2(1H)-carboxylate | 765.4 | 766.4 |
| | | | C₄₄H₅₅ N₅O₇ | |
| **420** | 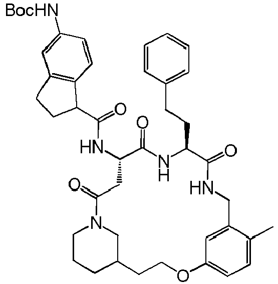 | tert-butyl (1-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)-2,3-dihydro-1H-inden-5-yl)carbamate | 765.4 | 766.3 |
| | | | C₄₄H₅₅ N₅O₇ | |
| **421** | 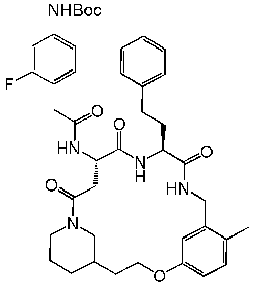 | tert-butyl (3-fluoro-4-(2-(((9S,12S)-5⁴-methyl-8, 11,14-trioxo-9-phenethyl-4-oxa-7,10-diaza-1(3,1)-piperidina-5(1,3)-benzenacyclotetradecaphane-12-yl)amino)-2-oxoethyl) phenyl)carbamate | 757.4 | 758.3 |
| | | | C₄₂H₅₂F N₅O₇ | |
| **422** | 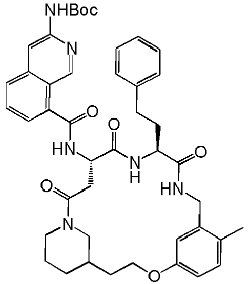 | tert-butyl (8-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)isoquinolin-3-yl)carbamate | 776.4 | 777.3 |
| | | | C₄₄H₅₂ N₆O₇ | |
| **423** | 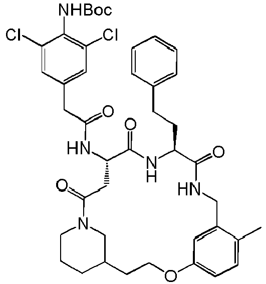 | tert-butyl (2,6-dichloro-4-(2-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)amino)-2-oxoethyl) phenyl)carbamate | 807.3 | 708.3 [M+H-Boc]+ |
| | | | C₄₂H₅₁ Cl₂N₅O₇ | |
| **429** | 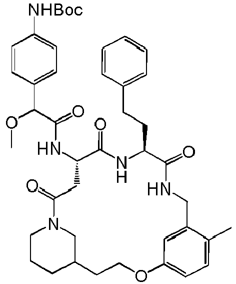 | tert-butyl (4-(1-methoxy-2-(((9S,12S)-5⁴-methyl-8, 11,14-trioxo-9-phenethyl-4-oxa-7,10-diaza-1(3,1)-piperidina-5(1,3)-benzenacyclotetradecaphane-12-yl)amino)-2-oxoethyl) phenyl)carbamate | 769.4 | 770.4 |
| | | | C₄₃H₅₅ N₅O₈ | |
| **440** | 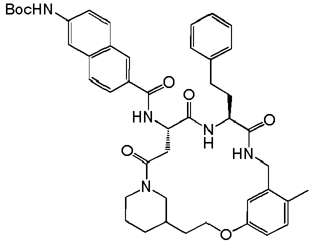 | tert-butyl (6-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)naphthalen-2-yl)carbamate | 775.4 | 775.4 |
| | | | C₄₅H₅₃ N₅O₇ | |
| **441** | 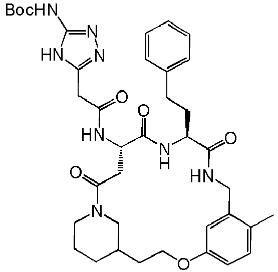 | tert-butyl (5-(2-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)amino)-2-oxoethyl)-4H-1,2,4-triazol-3-yl)carbamate | 730.4 | 731.4 |
| | | | C₃₈H₅₀ N₈O₇ | |
| **442** | 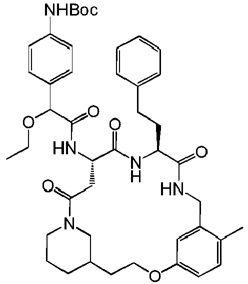 | tert-butyl (4-(1-ethoxy-2-(((9S,12S)-5⁴-methyl-8, 11,14-trioxo-9-phenethyl-4-oxa-7,10-diaza-1(3,1)-piperidina-5(1,3)-benzenacyclotetradecaphane-12-yl)amino)-2-oxoethyl)phenyl)carbamate | 783.4 | 784.5 |
| | | | C₄₄H₅₇ N₅O₈ | |

### Example A-13:_Removal of Boc-protecting groups to give basic macrocycles

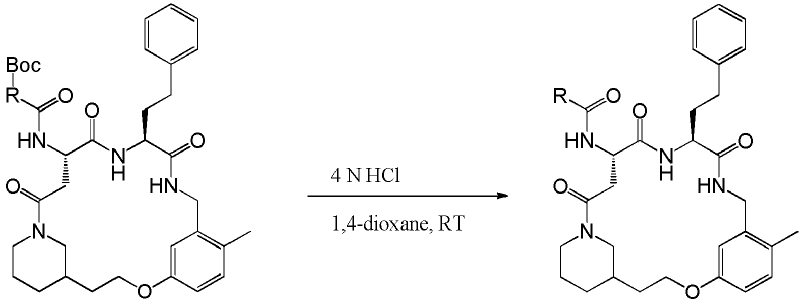

Boc protecting groups were cleaved from the compound according to general procedure D stirring compounds in 4-10 ml 4 N HCl / 1,4-dioxane at room temperature. After deprotection was complete solvent was removed and the crude product was purified by reversed phase HPLC.

**Table A-7**

| **Cpd no** | From cpd | structure | name | Exact mass | [M+H]⁺ found |
|---|---|---|---|---|---|
| **126** | **121** | 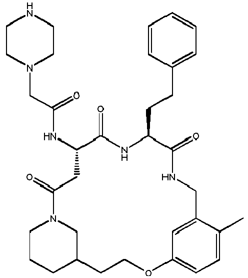 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-(piperazin-1-yl)acetamide | 632.4 | 633.5 |
| | | | | C₃₅H₄₈ N₆O₅ | |
| **127** | **122** | 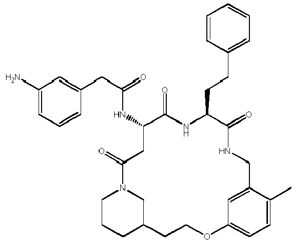 | 2-(3-aminophenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide | 639.3 | 640.5 |
| | | | | C₃₇H₄₅ N₅O₅ | |
| **128** | **123** | 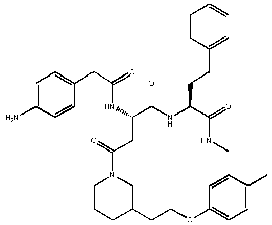 | 2-(4-aminophenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide | 639.3 | 640.5 |
| | | | | C₃₇H₄₅ N₅O₅ | |
| **129** | **124** | 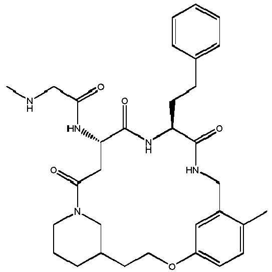 | N-((9S,12S)-5⁴-methyl-8,11,14-trioxo-9-phenethyl-4-oxa-7,10-diaza-1(3,1)-piperidina-5(1,3)-benzenacyclotetradecaphane -12-yl)-2-(methylamino)acetamide | 577.3 | 578.5 |
| | | | | C₃₂H₄₃ N₅O₅ | |
| **130** | **125** | 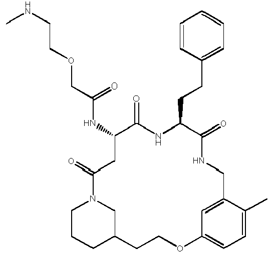 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-(2-(methyl amino)ethoxy)acetamide | 621.4 | 622.6 |
| | | | | C₃₄H₄₇ N₅O₆ | |
| **134** | **131** | 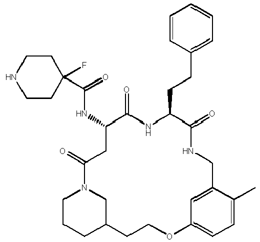 | 4-fluoro-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)piperidine-4-carboxamide | 635.3 | 636.5 |
| | | | | C₃₅H₄₆F N₅O₅ | |
| **135** | **132** | 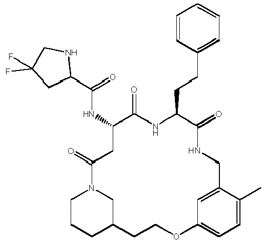 | (2S)-4,4-difluoro-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)pyrrolidine-2-carboxamide | 639.3 | 640.4 |
| | | | | C₃₄H₄₃F ₂N₅O₅ | |
| **136** | **133** | 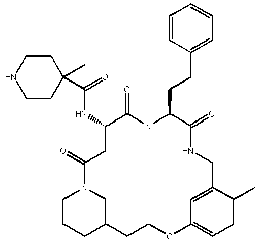 | 4-methyl-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)piperidine-4-carboxamide | 631.4 | 632.5 |
| | | | | C₃₆H₄₉N ₅O₅ | |
| **151** | **149** | 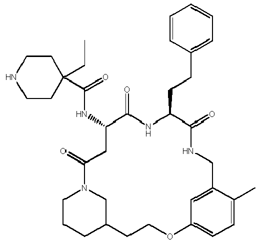 | 4-ethyl-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)piperidine-4-carboxamide | 645.4 | 646.5 |
| | | | | C₃₇H₅₁N ₅O₅ | |
| **152** | **150** | 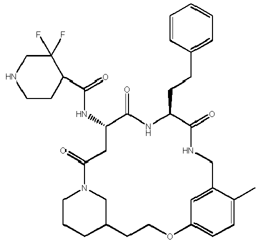 | 3,3-difluoro-N-((9S,12S)-5⁴-methyl-8, 11, 14-trioxo-9-phenethyl-4-oxa-7,10-diaza-1(3,1)-piperidina-5(1,3)-benzenacyclotetradecaphane-12-yl)piperidine-4-carboxamide | 653.3 | 654.4 |
| | | | | C₃₅H₄₅F ₂N₅O₅ | |
| **160** | **161** | 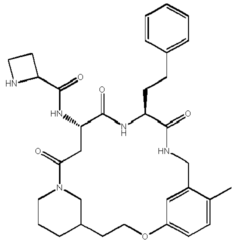 | (2R)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)azetidine-2-carboxamide | 589.74 | - |
| | | | | C₃₃H₄₃ N₅O₅ | |
| **157** | **153** | 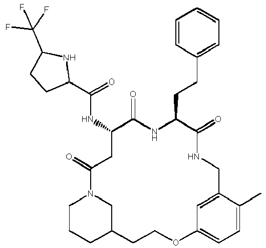 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-5-(trifluoromethyl) pyrrolidine-2-carboxamide | 671.3 | 672.5 |
| | | | | C₃₅H₄₄ F₃N₅O₅ | |
| **158** | **154** | 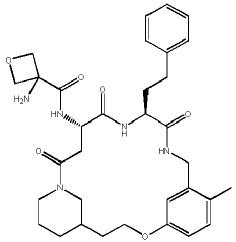 | 3-amino-N-((9S,12S)-5⁴-methyl-8, 11, 14-trioxo-9-phenethyl-4-oxa-7,10-diaza-1(3,1)-piperidina-5(1,3)-benzenacyclotetradecaphane-12-yl)oxetane-3-carboxamide | 605.3 | 606.5 |
| | | | | C₃₃H₄₃ N₅O₆ | |
| **159** | **155** | 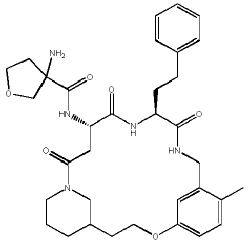 | 3-amino-N-((9S,12S)-5⁴-methyl-8, 11, 14-trioxo-9-phenethyl-4-oxa-7,10-diaza-1(3,1)-piperidina-5(1,3)-benzenacyclotetradecaphane-12-yl)tetrahydrofuran-3-carboxamide | 619.3 | 620.5 |
| | | | | C₃₄H₄₅ N₅O₆ | |
| **179** | **178** | 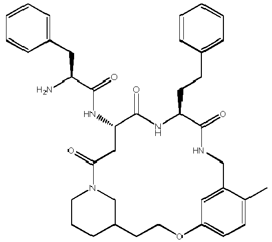 | (2R)-2-amino-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-3-phenylpropanamide | 653.4 | 654.4 |
| | | | | C₃₈H₄₇ N₅O₅ | |
| **302** | **387** | 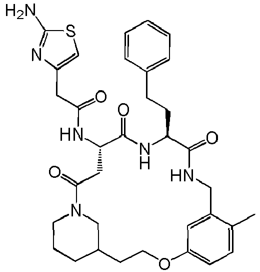 | 2-(2-aminothiazol-4-yl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide | 646.3 | 647.7 |
| | | | | C₃₄H₄₂ N₆O₅S | |
| **303** | **388** | 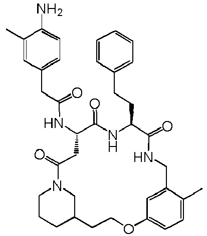 | 2-(4-amino-3-methylphenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide | 653.4 | 654.6 |
| | | | | C₃₈H₄₇ N₅O₅ | |
| **304** | **389** | 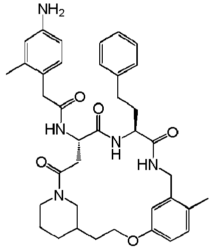 | 2-(4-amino-2-methylphenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide | 653.4 | 654.4 |
| | | | | C₃₈H₄₇ N₅O₅ | |
| **305** | **390** | 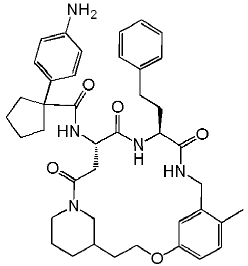 | 1-(4-aminophenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)cyclopentane-1-carboxamide | 693.4 | 694.5 |
| | | | | C₄₁H₅₁ N₅O₅ | |
| **306** | **391** | 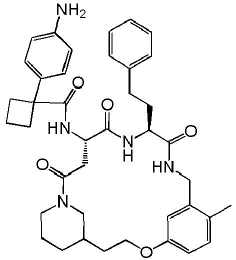 | 1-(4-aminophenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)cyclobutane-1-carboxamide | 679.4 | 680.4 |
| | | | | C₄₀H₄₉ N₅O₅ | |
| **307** | **392** | 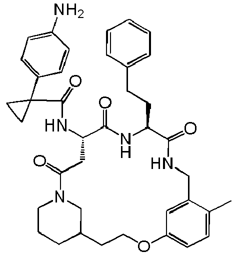 | 1-(4-aminophenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)cyclopropane-1-carboxamide | 665.4 | 666.4 |
| | | | | C₃₉H₄₇ N₅O₅ | |
| **308** | **393** | 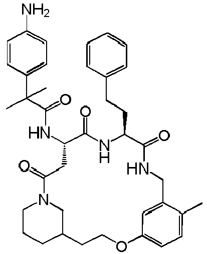 | 2-(4-aminophenyl)-2-methyl-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)propanamide | 667.4 | 668.5 |
| | | | | C₃₉H₄₉ N₅O₅ | |
| **309** | **394** | 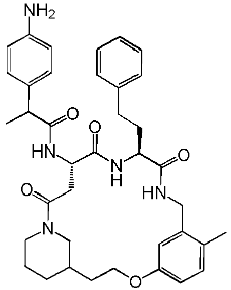 | 2-(4-aminophenyl)-N-((9S,12S)-5⁴-methyl-8, 11,14-trioxo-9-phenethyl-4-oxa-7,10-diaza-1(3,1)-piperidina-5(1,3)-benzenacyclotetradecaphane-12-yl)propanamide | 653.4 | 654.4 |
| | | | | C₃₈H₄₇ N₅O₅ | |
| **310** | **395** | 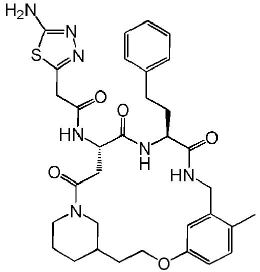 | 2-(5-amino-1,3,4-thiadiazol-2-yl)-N-((9S,12S)-54-methyl-8,11,14-trioxo-9-phenethyl-4-oxa-7,10-diaza-1(3,1)-piperidina-5(1,3)-benzenacyclotetradecaphane-12-yl)acetamide | 647.3 | 648.3 |
| | | | | C₃₃H₄₁ N₇O₅S | |
| **311** | **396** | 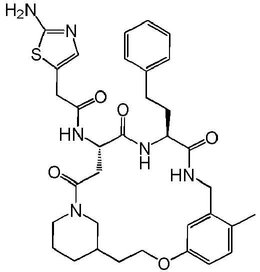 | 2-(2-aminothiazol-5-yl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide | 646.3 | 647.4 |
| | | | | C₃₄H₄₂ N₆O₅S | |
| **316** | **419** | 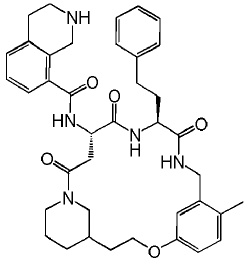 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-1,2,3,4-tetrahydroisoquinoline-5-carboxamide | 665.4 | 666.4 |
| | | | | C₃₉H₄₇ N₅O₅ | |
| **317** | **420** | 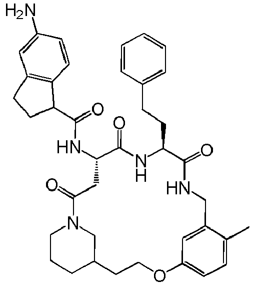 | 5-amino-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2,3-dihydro-1H-indene-1-carboxamide | 665.4 | 666.5 |
| | | | | C₃₉H₄₇ N₅O₅ | |
| **318** | **421** | 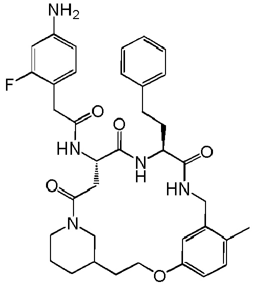 | 2-(4-amino-2-fluorophenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide | 657.3 | 658.4 |
| | | | | C₃₇H₄₄F N₅O₅ | |
| **319** | **422** | 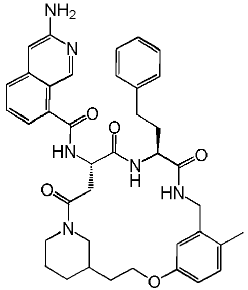 | 3-amino-N-((9S,12S)-5⁴-methyl-8, 11, 14-trioxo-9-phenethyl-4-oxa-7,10-diaza-1(3,1)-piperidina-5(1,3)-benzenacyclotetradecaphane-12-yl)isoquinoline-8-carboxamide | 676.3 | 677.4 |
| | | | | C₃₉H₄₄ N₆O₅ | |
| **320** | **423** | 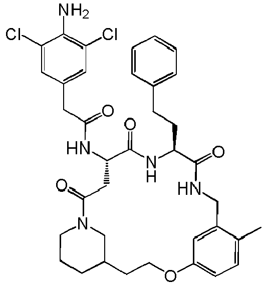 | 2-(4-amino-3,5-dichlorophenyl)-N-((9S, 12S)-5⁴-methyl-8,11,14-trioxo-9-phenethyl-4-oxa-7,10-diaza-1(3,1)-piperidina-5(1,3)-benzenacyclotetradecaphane-12-yl)acetamide | 707.3 | 708.3 |
| | | | | C₃₇H₄₃ Cl₂N₅O₅ | |
| **326** | **429** | 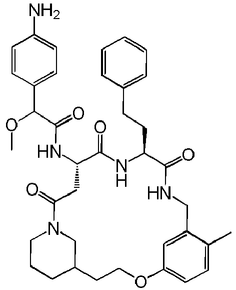 | 2-(4-aminophenyl)-2-methoxy-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide | 669.4 | 670.4 |
| | | | | C₃₈H₄₇ N₅O₆ | |
| **339** | **440** | 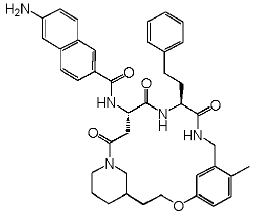 | 6-amino-N-((9S,12S)-5⁴-methyl-8, 11, 14-trioxo-9-phenethyl-4-oxa-7,10-diaza-1(3,1)-piperidina-5(1,3)-benzenacyclotetradecaphane-12-yl)-2-naphthamide | 675.3 | 676.4 |
| | | | | C₄₀H₄₅ N₅O₅ | |
| **340** | **441** | 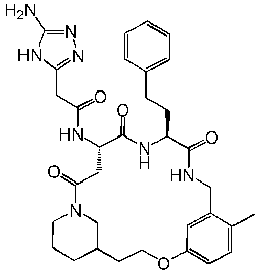 | 2-(5-amino-1H-1,2,4-triazol-3-yl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide | 630.3 | 631.4 |
| | | | | C₃₃H₄₂ N₈O₅ | |
| **345** | **442** | 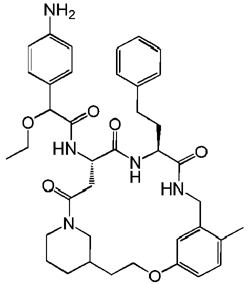 | 2-(4-aminophenyl)-2-ethoxy-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide | 683.4 | 684.4 |
| | | | | C₃₉H₄₉ N₅O₆ | |

### Example A-14: Preparation of intermediate compounds

### (S)-tert-butyl 4-acetamido-5-(((S)-1-ethoxy-1-oxo-4-phenylbutan-2-yl)amino)-5-oxopentanoate (137)

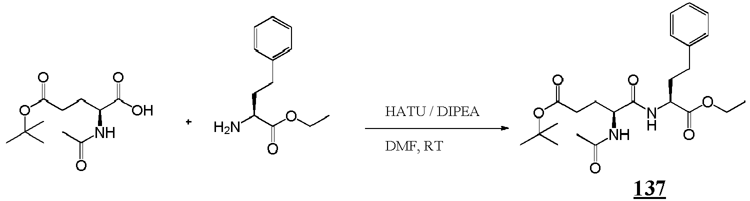

The amide bond can be formed according to general procedure B with Ac-Glu(OtBu)-OH and H-hPhe-OEt. Purification can be achieved by normal phase flash chromatography.
Formula: C₂₃H₃₄N₂O₆, exact mass: 434.2, found: 435.3 [M+H]⁺

### (S)-2-((S)-2-acetamido-5-(tert-butoxy)-5-oxopentanamido)-4-phenylbutanoic acid (138)

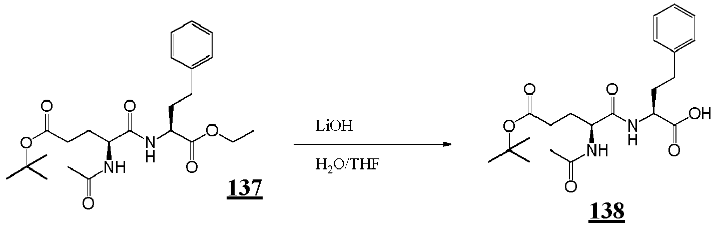

Ester **137** was dissolved in 3 ml THF. 0.5 ml water and 0.5 2 M LiOH aq. were added at room temperature. After complete saponification the mixture was acidified to pH 2 and extracted with ethyl acetate. The organic phase was dried over MgSO₄ and solvents were removed under reduced pressure. The residue was used in the next step without further purification.
Formula: C₂₁H₃₀N₂O₆, exact mass: 406.2, found: 407.5 [M+H]⁺

### tert-butyl 2-2-3-cyano-4-methylphenoxy)ethyl)morpholine-4-carboxylate (140)

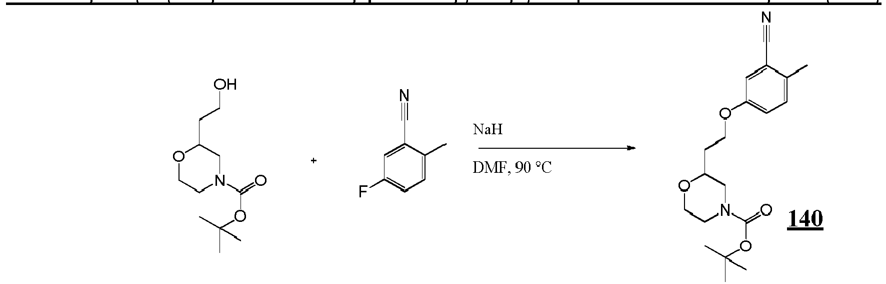

924 mg alcohol was deprotonated in DMF with 153 mg NaH at room temperature. 450 mg 5-fluoro-2-methylbenzonitrile were added and the mixture was heated to 90 °C until reaction was complete. Reaction mixture was diluted with ethyl acetate and washed with saturated NaHCO₃ solution and brine. After drying over MgSO₄ and removing the solvent under reduced pressure the residue was purified by normal phase column chromatography (silica, cyclohexane/ethyl acetate gradient).
Formula: C₁₉H₂₆N₂O₄, exact mass: 346.2, found: 247.3 [M+H]⁺

Building blocks in the following table were synthesized as exemplified by compound **140** utilizing appropriately substituted 3-fluorobenzonitriles and related boc-protected aminoalcoholes.

**Table A-8**

| **Cpd no** | structure | name | Exact mass | [M+H]⁺ found |
|---|---|---|---|---|
| **140** | 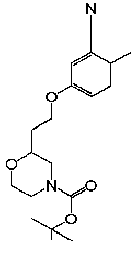 | tert-butyl 2-(2-(3-cyano-4-methylphenoxy)ethyl)morpholine-4-carboxylate | 346.2 | 247.3 [M+H-Boc]+ |
| | | | C₁₉H₂₆ | |
| | | | N₂O₄ | |
| **144** | 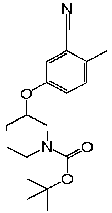 | ert-butyl 3-(3-cyano-4-methylphenoxy)piperidine-1-carboxylate | 316.2 | 217.1 [M+H-Boc]+ |
| | | | C₁₈H₂₄ | |
| | | | N₂O₃ | |
| **187** | 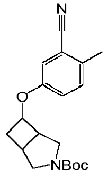 | tert-butyl 6-(3-cyano-4-methylphenoxy)-3-azabicyclo[3.2.0]heptane-3-carboxylate | 328.2 | 229.3 [M+H-Boc]+ |
| | | | C₁₉H₂₄N | |
| | | | ₂O₃ | |
| **229** | 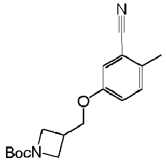 | tert-butyl 3-((3-cyano-4-methyl phenoxy)methyl)azetidine-1-carboxylate | 302.2 | |
| | | | C₁₇H₂₂ | |
| | | | N₂O₃ | |
| **413** | 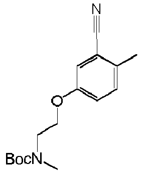 | tert-butyl (2-(3-cyano-4-methylphenoxy)ethyl)(methyl)carbamate | 290.2 | |
| | | | C₁₆H₂₂ | |
| | | | N₂O₃ | |
| **412** | 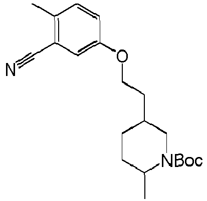 | tert-butyl 5-(2-(3-cyano-4-methylphenoxy)ethyl)-2-methylpiperidine-1-carboxylate | 358.2 | |
| | | | C₂₁H₃₀ | |
| | | | N₂O₃ | |
| **414** | 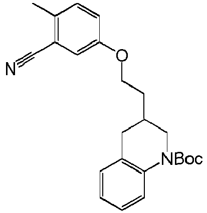 | tert-butyl 3-(2-(3-cyano-4-methylphenoxy)ethyl)-3,4-dihydroquinoline-1(2H)-carboxylate | 392.2 | |
| | | | C₂₄H₂₈ | |
| | | | N₂O₃ | |
| **415** | 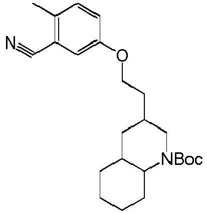 | tert-butyl 3-(2-(3-cyano-4-methyl phenoxy)ethyl)octahydroquinoline-1(2H)-carboxylate | 398.3 | |
| | | | C₂₄H₃₄ | |
| | | | N₂O₃ | |
| **431** | 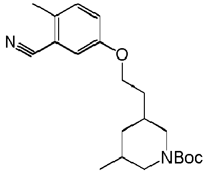 | tert-butyl 3-(2-(3-cyano-4-methylphenoxy)ethyl)-5-methylpiperidine-1-carboxylate | 358.2 | 359.3 |
| | | | C₂₁H₃₀ | |
| | | | N₂O₃ | |
| **454** | 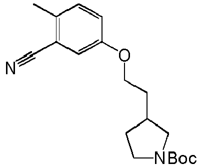 | tert-butyl 3-(2-(3-cyano-4-methylphenoxy)ethyl)pyrrolidine-1-carboxylate | 330.2 | 331.4 |
| | | | C₁₉H₂₆ | |
| | | | N₂O₃ | |
| **453** | 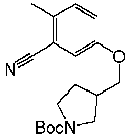 | tert-butyl 3-((3-cyano-4-methylphenoxy)methyl)pyrrolidine-1-carboxylate | 316.2 | 317.4 |
| | | | C₁₈H₂₄ | |
| | | | N₂O₃ | |
| **462** | 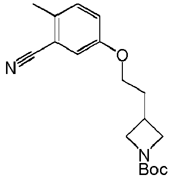 | tert-butyl 3-(2-(3-cyano-4-methylphenoxy)ethyl)azetidine-1-carboxylate | 316.2 | 317.3 |
| | | | C₁₈H₂₄ | |
| | | | N₂O₃ | |
| **466** | 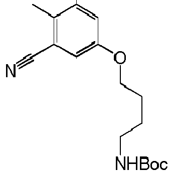 | tert-butyl (4-(3-cyano-4,5-dimethylphenoxy)butyl)carbamate | 318.2 | 319.4 |
| | | | C₁₈H₂₆ | |
| | | | N₂O₃ | |
| **573** | 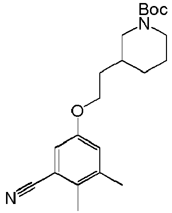 | tert-butyl 3-(2-(3-cyano-4,5-dimethylphenoxy)ethyl)piperidine-1-carboxylate | 358.2 | |
| | | | C₂₁H₃₀ | |
| | | | N₂O₃ | |
| **577** | 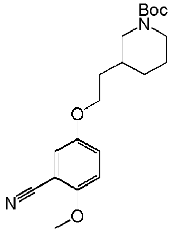 | tert-butyl 3-(2-(3-cyano-4-methoxyphenoxy)ethyl)piperidine-1 -carboxylate | 360.2 | |
| | | | C₂₀H₂₈ | |
| | | | N₂O₄ | |
| **578** | 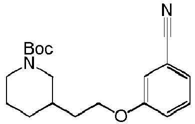 | tert-butyl 3-(2-(3-cyanophenoxy)ethyl)piperidine-1-carboxylate | 330.2 | 331.3 |
| | | | C₁₉H₂₆ | |
| | | | N₂O₃ | |

### tert-butyl 2-(2-(3-(aminomethyl)-4-methylphenoxy)ethyl)morpholine-4-carboxylate (141)

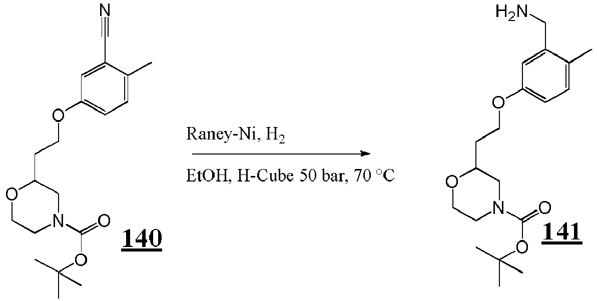

1.15 g nitrile 140 were reduced was cleaved according to general procedure H using Raney-Ni catalyst cartridge, 50 bar at 70 °C in 70 ml ethanol. Volatiles were removed under reduced pressure and the residue was purified by normal phase column chromatography (silica, cyclohexane/ethyl acetate gradient). Building blocks in the following table were synthesized as exemplified by compound **141.**
Formula: C₁₉H₃₀N₂O₄, exact mass: 350.2, found: 351.3 [M+H]⁺

**Table A-9**

| **Cpd no** | From cpd | structure | name | Exact mass | [M+H]⁺ found |
|---|---|---|---|---|---|
| **90** | **573** | 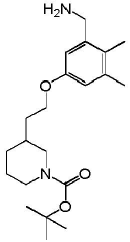 | tert-butyl 3-(2-(3-(aminomethyl)-4,5-dimethylphenoxy)ethyl)piperidine-1-carboxylate | 362.3 | 363.3 |
| | | | | C₂₁H₃₄ | |
| | | | | N₂O₃ | |
| **141** | **140** | 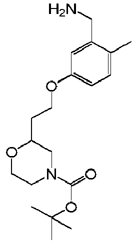 | tert-butyl 2-(2-(3-(aminomethyl)-4-methylphenoxy)ethyl)morpholine-4-carboxylate | 350.2 | 351.3 |
| | | | | C₁₉H₃₀ | |
| | | | | N₂O₄ | |
| **145** | **144** | 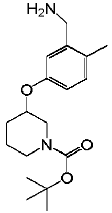 | tert-butyl 3-(3-(aminomethyl)-4-methylphenoxy)piperidine-1-carboxylate | 320.2 | 321.3 |
| | | | | C₁₈H₂₈ | |
| | | | | N₂O₃ | |
| **188** | **187** | 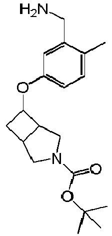 | tert-butyl 6-(3-(aminomethyl)-4-methylphenoxy)-3-azabicyclo[3.2.0]heptane-3-carboxylate | 332.2 | 333.3 |
| | | | | C₁₉H₂₈ | |
| | | | | N₂O₃ | |
| **222** | | 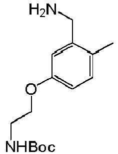 | tert-butyl (2-(3-(aminomethyl)-4-methylphenoxy)ethyl)carbamate | 280.2 | 281.2 |
| | | | | C₁₅H₂₄ | |
| | | | | N₂O₃ | |
| **224** | | 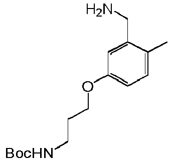 | tert-butyl (3-(3-(aminomethyl)-4-methylphenoxy)propyl)carbamate | 294.2 | 295.2 |
| | | | | C₁₆H₂₆ | |
| | | | | N₂O₃ | |
| **225** | | 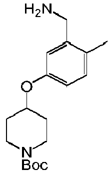 | tert-butyl 4-(3-(aminomethyl)-4-methylphenoxy)piperidine-1-carboxylate | 320.2 | 321.5 |
| | | | | C₁₈H₂₈ | |
| | | | | N₂O₃ | |
| **226** | | 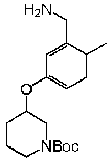 | tert-butyl 3-(3-(aminomethyl)-4-methylphenoxy)piperidine-1-carboxylate | 320.2 | 321.3 |
| | | | | C₁₈H₂₈ | |
| | | | | N₂O₃ | |
| **227** | | 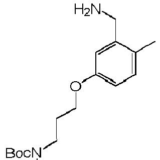 | tert-butyl (3-(3-(aminomethyl)-4-methylphenoxy)propyl)(methyl)carbamate | 308.2 | 309.1 |
| | | | | C₁₇H₂₈ | |
| | | | | N₂O₃ | |
| **228** | | 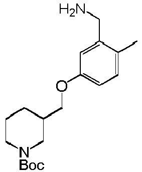 | tert-butyl 3-((3-(aminomethyl)-4-methylphenoxy)methyl)piperidine-1-carboxylate | 334.2 | 335.3 |
| | | | | C₁₉H₃₀ | |
| | | | | N₂O₃ | |
| **230** | **229** | 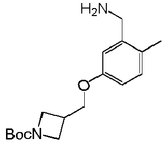 | tert-butyl 3-((3-(aminomethyl)-4-methylphenoxy)methyl)azetidine-1-carboxylate | 306.2 | 307.3 |
| | | | | C₁₇H₂₆ | |
| | | | | N₂O₃ | |
| **231** | | 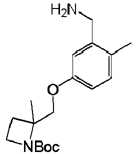 | tert-butyl 2-((3-(aminomethyl)-4-methylphenoxy)methyl)-2-methylazetidine-1-carboxylate | 320.2 | 321.2 |
| | | | | C₁₈H₂₈ | |
| | | | | N₂O₃ | |
| **232** | | 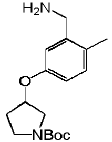 | tert-butyl 3-(3-(aminomethyl)-4-methylphenoxy)pyrrolidine-1-carboxylate | 306.2 | 307.2 |
| | | | | C₁₇H₂₆ | |
| | | | | N₂O₃ | |
| **270** | | 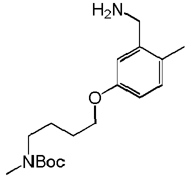 | tert-butyl (4-(3-(aminomethyl)-4-methylphenoxy)butyl)(methyl)carbamate | 322.2 | 323.2 |
| | | | | C₁₈H₃₀ | |
| | | | | N₂O₃ | |
| **271** | | 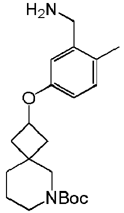 | tert-butyl 2-(3-(aminomethyl)-4-methylphenoxy)-6-azaspiro[3.5]nonane-6-carboxylate | 360.2 | 361.1 |
| | | | | C₂₁H₃₂ | |
| | | | | N₂O₃ | |
| **399** | **413** | 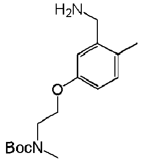 | tert-butyl (2-(3-(aminomethyl)-4-methylphenoxy)ethyl)(methyl)carbamate | 294.2 | - |
| | | | | C₁₆H₂₆ | |
| | | | | N₂O₃ | |
| **411** | **412** | 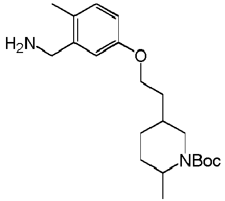 | tert-butyl 5-(2-(3-(aminomethyl)-4-methylphenoxy)ethyl)-2-methylpiperidine-1-carboxylate | 362.3 | 363.3 |
| | | | | C₂₁H₃₄ | |
| | | | | N₂O₃ | |
| **416** | **414** | 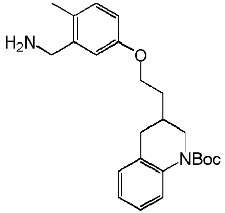 | tert-butyl 3-(2-(3-(aminomethyl)-4-methylphenoxy)ethyl)-3,4-dihydroquinoline-1(2H)-carboxylate | 396.2 | 397.3 |
| | | | | C₂₄H₃₂ | |
| | | | | N₂O₃ | |
| **417** | **415** | 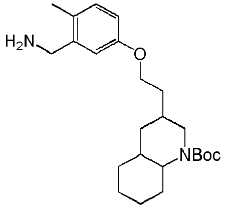 | tert-butyl 3-(2-(3-(aminomethyl)-4-methylphenoxy)ethyl)octahydroquinoline-1(2H)-carboxylate | 402.3 | 403.4 |
| | | | | C₂₄H₃₈ | |
| | | | | N₂O₃ | |
| **426** | **454** | 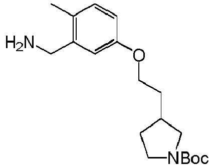 | tert-butyl 3-(2-(3-(aminomethyl)-4-methylphenoxy)ethyl)pyrrolidine-1-carboxylate | 334.2 | 335.4 |
| | | | | C₁₉H₃₀ | |
| | | | | N₂O₃ | |
| **430** | **431** | 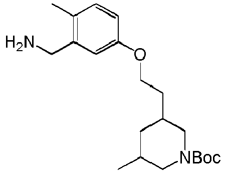 | tert-butyl 3-(2-(3-(aminomethyl)-4-methylphenoxy)ethyl)-5-methylpiperidine-1-carboxylate | 362.3 | 363.4 |
| | | | | C₂₁H₃₄ | |
| | | | | N₂O₃ | |
| **455** | **453** | 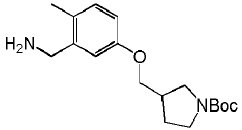 | tert-butyl 3-((3-(aminomethyl)-4-methylphenoxy)methyl)pyrrolidine-1-carboxylate | 320.2 | 321.3 |
| | | | | C₁₈H₂₈ | |
| | | | | N₂O₃ | |
| **463** | **462** | 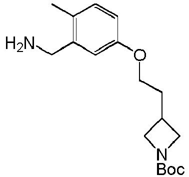 | tert-butyl 3-(2-(3-(aminomethyl)-4-methylphenoxy)ethyl)azetidine-1-carboxylate | 320.2 | 321.3 |
| | | | | C₁₈H₂₈ | |
| | | | | N₂O₃ | |
| **468** | **466** | 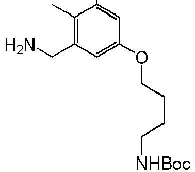 | tert-butyl (4-(3-(aminomethyl)-4,5-dimethylphenoxy) butyl)carbamate | 322.2 | 323.3 |
| | | | | C₁₈H₃₀ | |
| | | | | N₂O₃ | |
| **206** | **577** | 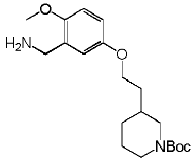 | tert-butyl 3-(2-(3-(aminomethyl)-4-methoxyphenoxy)ethyl)piperidine-1-carboxylate | 364.2 | 365.0 |
| | | | | C₂₀H₃₂ | |
| | | | | N₂O₄ | |
| **579** | **578** | 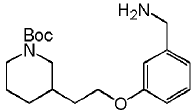 | tert-butyl 3-(2-(3-(aminomethyl)phenoxy)ethyl)piperidine-1-carboxylate | 334.2 | 335.3 |
| | | | | C₁₉H₃₀ | |
| | | | | N₂O₃ | |

### Example A-15: Preparation of macrocyclic compounds 143, and 148

### tert-butyl 2-(2-(3-(((S)-2-((S)-2-acetamido-5-(tert-butoxy)-5-oxopentanamido)-4-phenylbutanamido)methyl)-4-methylphenoxy)ethyl)morpholine-4-carboxylate (139)

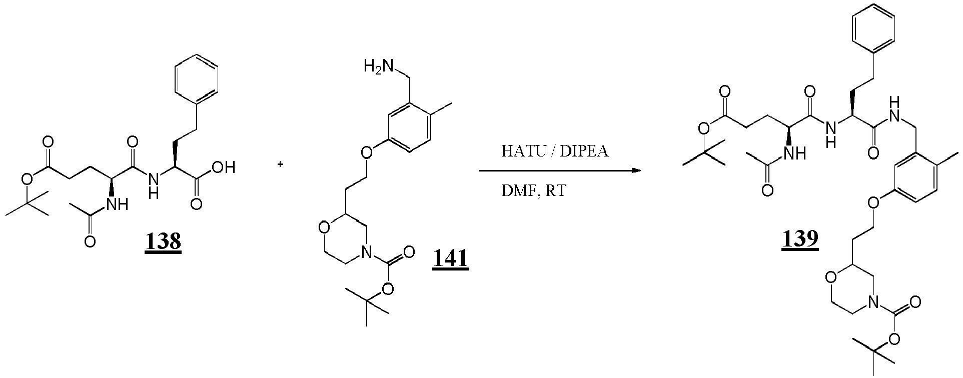

The amide bond was formed according to general procedure B with 96 mg amine **141** and 94 mg protected amino acid **138.** Purification was achieved by normal phase flash chromatography. Building blocks in the following table were synthesized as exemplified by compound **139.**

**Table A-10**

| **Cpd no** | From cpd | structure | name | Exact mass | [M+H]⁺ found |
|---|---|---|---|---|---|
| **139** | **141** | 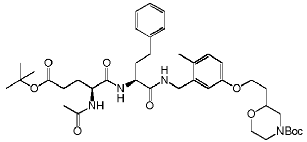 | tert-butyl 2-(2-(3-(((S)-2-((S)-2-acetamido-5-(tert-butoxy)-5-oxopentanamido)-4-phenylbutanamido)methyl)-4-methylphenoxy)ethyl)morpholine-4-carboxylate | 738.4 | 739.5 |
| | | | | C₄₀H₅₈ | |
| | | | | N₄O₉ | |
| **146** | **145** | 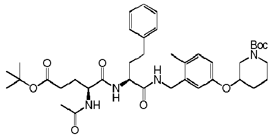 | tert-butyl 3-(3-(((S)-2-((S)-2-acetamido-5-(tert-butoxy)-5-oxopentanamido)-4-phenylbutanamido)methyl)-4-methylphenoxy)piperidine-1-carboxylate | 708.4 | 709.6 |
| | | | | C₃₉H₅₆ | |
| | | | | N₄O₈ | |

### (4S)-4-acetamido-5-(((2S)-1-((2-methyl-5-(2-(morpholin-2-yl)ethoxy)benzyl)amino)-1-oxo-4-phenylbutan-2-yl)amino)-5-oxopentanoic acid (142)

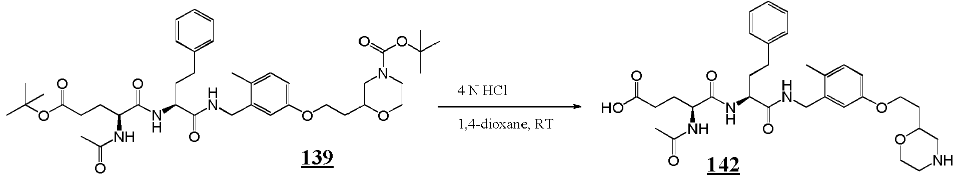

Protecting groups were cleaved from compound **139** according to general procedure D stirring compounds in 10 ml 4 N HCI / 1,4-dioxane at room temperature. After deprotection was complete solvent was removed and the crude dissolved in acetonitrile and concentrated twice. Product used without further purification.

Building blocks in the following table were synthesized as exemplified by compound **142**.

**Table A-11**

| **Cpd no** | From cpd | structure | name | [M+H]⁺ calc. | [M+H]⁺ found |
|---|---|---|---|---|---|
| **142** | **139** | 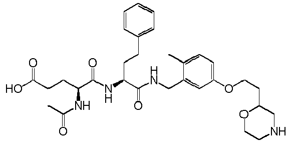 | (4S)-4-acetamido-5-(((2S)-1-((2-methyl-5-(2-(morpholin-2-yl)ethoxy)benzyl)amino)-1-oxo-4-phenylbutan-2-yl)amino)-5-oxopentanoic acid | 582.3 | 583.6 |
| | | | | C₃₁H₄₂ | |
| | | | | N₄O₇ | |
| **147** | **146** | 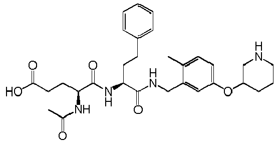 | (4S)-4-acetamido-5-(((2S)-1-((2-methyl-5-(piperidin-3-yloxy)benzyl)amino)-1-oxo-4-phenylbutan-2-yl)amino)-5-oxopentanoic acid | 552.3 | 553.4 |
| | | | | C₃₀H₄₀ | |
| | | | | N₄O₆ | |

### N-((9S,12S)-5⁴-methyl-8,11,15-trioxo-9-phenethyl-4-oxa-7,10-diaza-1(2,4)-morpholina-5(1,3)-benzenacyclopentadecaphane-12-yl)acetamide (143)

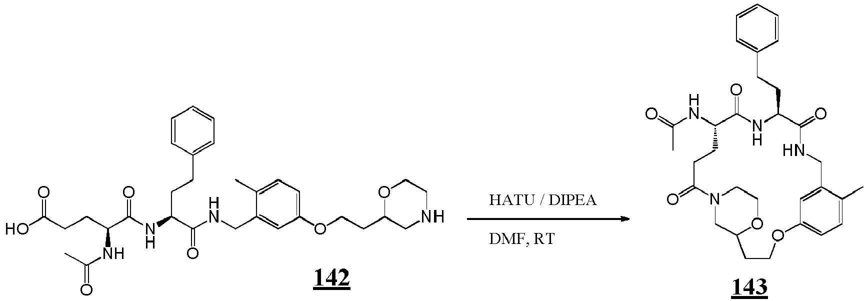

Macrocyclization was achieved according to general procedure A with 0.20 mmol amino acid **142.** Reaction mixture was diluted with some methanol and purified via HPLC. Compounds in the following table were synthesized as exemplified by compound **143.**

**Table A-12**

| Cpd no | From cpd | Scale [nmol] | structure | name | Exact mass | [M+H]⁺ found |
|---|---|---|---|---|---|---|
| **143** | **142** | 200 | 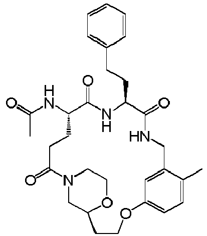 | N-((9S,128)-5⁴-methyl-8,11,15-trioxo-9-phenethyl-4-oxa-7,10-diaza-1(2,4)-morpholina-5(1,3)-benzenacyclopentadecaphane-12-yl)acetamide | 564.3 | 565.4 |
| | | | | | C₃₁H₄₀ N₄O₆ | |
| **148** | **147** | 230 | 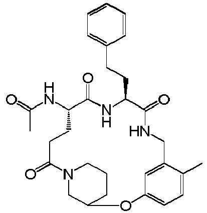 | N-((7S,10S)-3⁴-methyl-6,9,13-trioxo-7-phenethyl-2-oxa-5,8-diaza-1(3,1)-piperidina-3(1,3)-benzenacyclotridecaphane-10-yl)acetamide | 534.3 | 535.4 |
| | | | | | C₃₀H₃₈ N₄O₅ | |

### Example A-16: Preparation of macrocyclic compound 168

### tert-butyl (4-(3-cyano-4-methylphenoxy)butyl)carbamate (162)

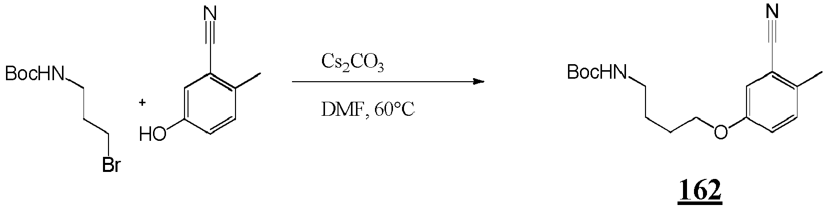

978 mg phenol, 2.5 g bromide and 6.46 g Cs₂CO₃ were stirred in 10 ml DMF at 60 °C until the phenol was consumed. The reaction mixture was diluted with DCM and washed with brine. Solvent was removed and the residue was purified by normal phase column chromatography (silica, cyclohexane/ethyl acetate gradient).
Formula: C₁₇H₂₄N₂O₃, exact mass: 304.2, found: 304 (GC), 205.2 [M+H-Boc]⁺

### tert-butyl (4-(3-(aminomethyl)-4-methylphenoxy)butyl)carbamate (163)

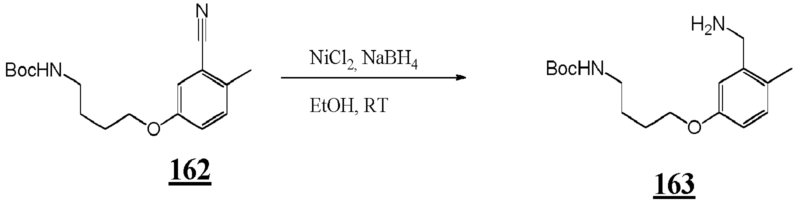

To 1.55 g nitrile **162** an 660 mg NiCl₂ in ethanol 680 mg NaBH₄ were added in portions at room temperature. After complete reaction the mixture was filtered over a pad of Celite. Crude was purified by normal phase column chromatography (silica, DCM/methanol gradient).
Formula: C₁₇H₂₈N₂O₃, exact mass: 308.2, found: 309.2 [M+H]⁺

### (S)-tert-butyl-(4-(3-((2-(((benzyloxy)carbonyl)amino)-4-phenylbutanamido)methyl)-4 methylphenoxy)butyl)carbamate (164)

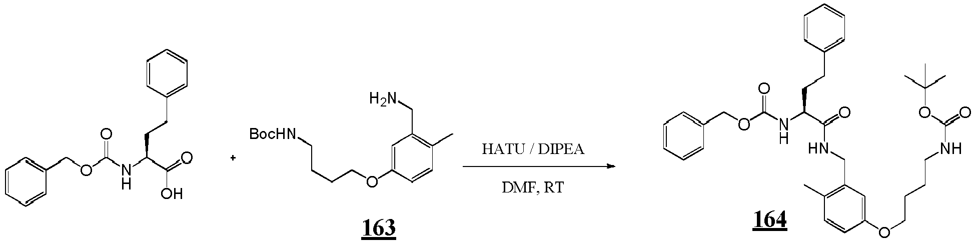

The amide bond was formed according to general procedure B with 346 mg amine 163 and 421 mg Cbz-hPhe-OH. Purification was achieved by normal phase column chromatography (silica, cyclohexane/ethyl acetate gradient).
Formula: C₃₅H₄₅N₃O₆, exact mass: 603.3, found: 604.4 [M+H]⁺

### (S)-tert-butyl (4-(3-((2-amino-4-phenylbutanamido)methyl)-4-methylphenoxy)butyl) carbamate (165)

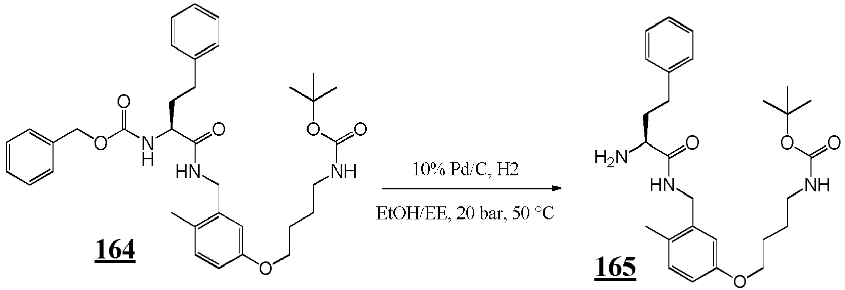

Benzyl ester was cleaved according to general procedure C using 10 % Pd/C catalyst cartridge, 20 bar at 50 °C in a solvent mixture of ethanol / ethyl acetate. Product was directly used after removing of volatiles.
Formula: C₂₇H₃₉N₃O₄, exact mass: 469.3, found: 470.4 [M+H]⁺

### (S)-tert-butyl 3-acetamido-4-(((S)-1-((5-(4-((tert-butoxycarbonyl)amino)butoxy)-2-methylbenzyl)amino)-1-oxo-4-phenylbutan-2-yl)amino)-4-oxobutanoate (166)

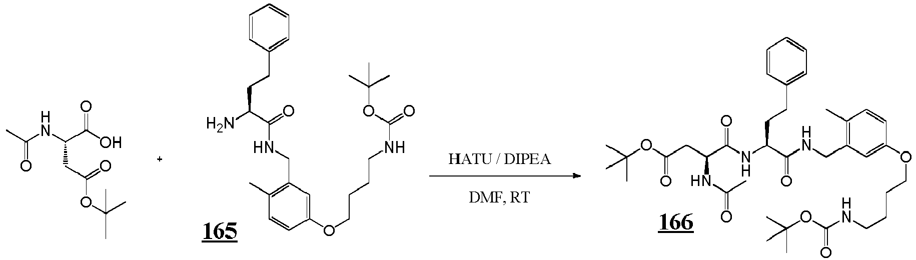

The amide bond was formed according to general procedure B with 190 mg amine 165 and 112 mg Ac-Asp(OtBu)-OH. Purification was achieved by normal phase column chromatography (silica, cyclohexane/ethyl acetate gradient).
Formula: C₃₇H₅₄N₄O₈, exact mass: 682.4, found: 683.4 [M+H]⁺

### (S)-3-acetamido-4-(((S)-1-((5-(4-aminobutoxy)-2-methylbenzyl)amino)-1-oxo-4-phenylbutan-2-yl)amino)-4-oxobutanoic acid (167)

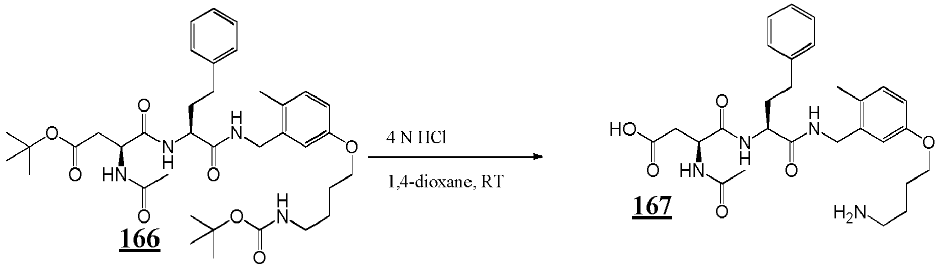

Protecting groups were cleaved from 276 mg compound 166 according to general procedure D stirring compounds in 6 ml 4 N HCl / 1,4-dioxane at room temperature. After deprotection was complete solvent was removed and the crude dissolved in acetonitrile and concentrated twice. Product used without further purification.
Formula: C₂₈H₃₈N₄O₆, exact mass: 526.3, found: 527.3 [M+H]⁺

### N-((10S,13S)-1⁴-methyl-8,11,14-trioxo-13-phenethyl-2-oxa-7,12,15-triaza-1(1,3)-benzenacyclohexadecaphane-10-yl)acetamide (168)

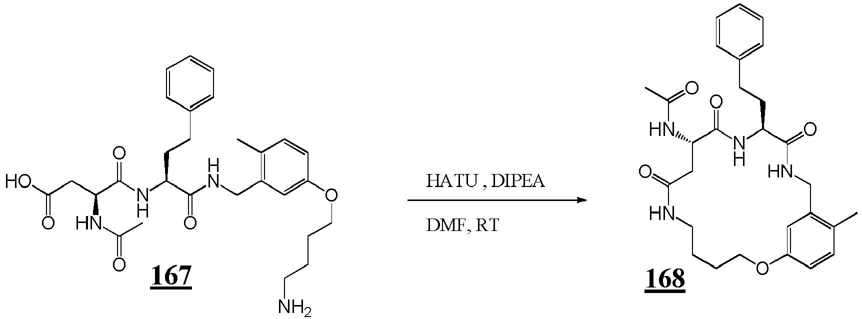

Macrocyclization was achieved according to general procedure A with 0.40 mmol amino acid 167. Reaction mixtures were diluted with some methanol and purified via HPLC.
Formula: C₂₈H₃₆N₄O₅, exact mass: 508.3, found: 509.3 [M+H]⁺

### Example A-17: Preparation of macrocyclic compounds 171-173

### (S)-tert-butyl 4-acetamido-5-(((S)-1-((5-(4-((tert-butoxycarbonyl)amino)butoxy)-2-methylbenzyl)amino)-1-oxo-4-phenylbutan-2-yl)amino)-5-oxopentanoate (169)

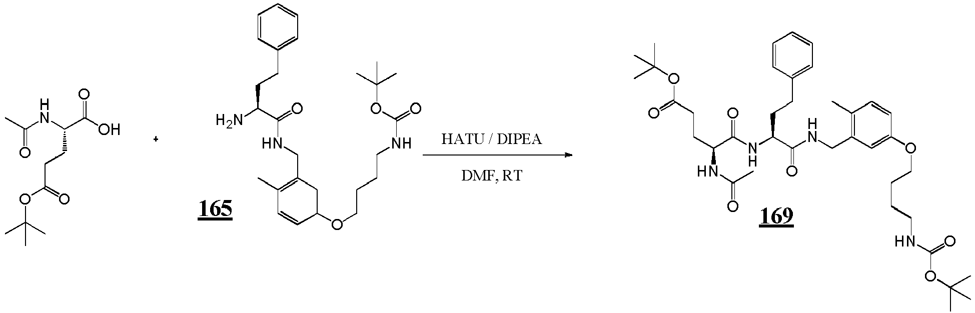

The amide bond was formed according to general procedure B with 190 mg amine **165** and 119 mg Ac-Glut(OtBu)-OH. Purification was achieved by normal phase column chromatography (silica, cyclohexane/ethyl acetate gradient).
Formula: C₃₈H₅₆N₄O₈, exact mass: 696.4, found: 697.5 [M+H]⁺

### (S)-4-acetamido-5-(((S)-1-((5-(4-aminobutoxy)-2-methylbenzyl)amino)-1-oxo-4-phenylbutan-2-yl)amino)-5-oxopentanoic acid (170)

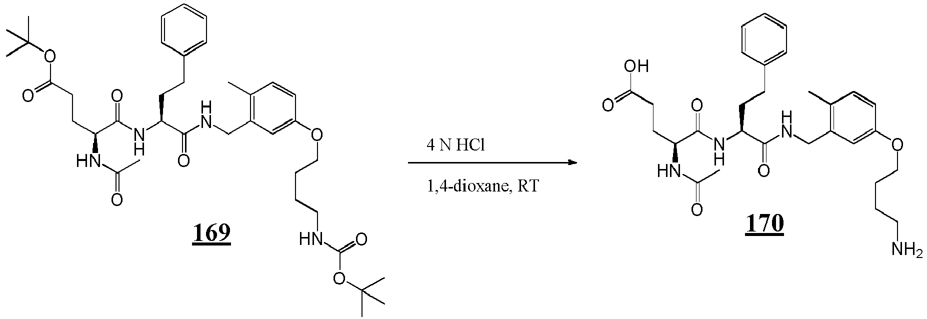

Protecting groups were cleaved from 282 mg compound **169** according to general procedure D stirring compounds in 6 ml 4 N HCl / 1,4-dioxane at room temperature. After deprotection was complete solvent was removed and the crude dissolved in acetonitrile and concentrated twice. Product used without further purification.
Formula: C₂₉H₄₀N₄O₆, exact mass: 540.3, found: 541.3 [M+H]⁺

### N-((11S,14S)-1⁴-methyl-8,12,15-trioxo-14-phenethyl-2-oxa-7,13,16-triaza-1(1,3)-benzenacycloheptadecaphane-11-yl)acetamide (171)

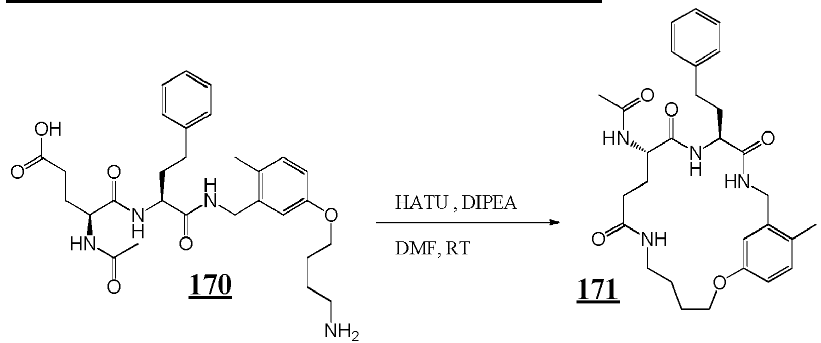

Macrocyclization was achieved according to general procedure A with 0.40 mmol amino acid **170.** Reaction mixtures were diluted with some methanol and purified via HPLC. Formula: C₂₉H₃₈N₄O₆, exact mass: 522.3, found: 523.3 [M+H]⁺

### 2-(4-aminophenyl)-N-((13R,9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide (172)

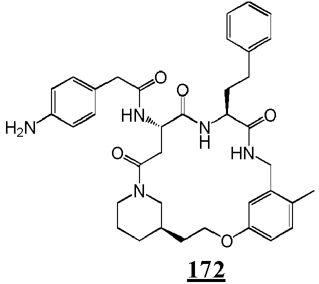

Compound **172** was prepared in analogy to compound 128 with the exception that (R)-tert-butyl 3-(2-hydroxyethyl)piperidine-1-carboxylate was used to synthesize a enantiomerically pure derivative of tert-butyl 3-(2-(3-cyano-4-methylphenoxy)ethyl)piperidine-1-carboxylate **3.**
Formula: C₃₇H₄₅N₅O₅, exact mass: 639.3, found: 640.4 [M+H]⁺

### 2-(4-aminophenyl)-N-((13S,9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide (173)

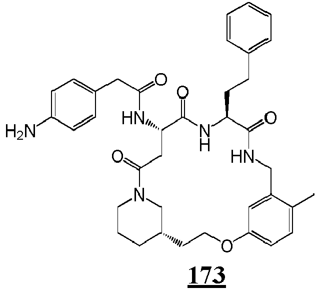

Compound **172** was prepared in analogy to compound **128** with the exception that (S)-tert-butyl 3-(2-hydroxyethyl)piperidine-1-carboxylate was used to synthesize a enantiomerically pure derivative of tert-butyl 3-(2-(3-cyano-4-methylphenoxy)ethyl)piperidine-1-carboxylate **3.**
Formula: C₃₇H₄₅N₅O₅, exact mass: 639.3, found: 640.4 [M+H]⁺

### Example A-18: Preparation of macrocyclic compounds 180, 181, 216, 217, and 294

### Amide derivatizations of compound 8 according to general procedure E

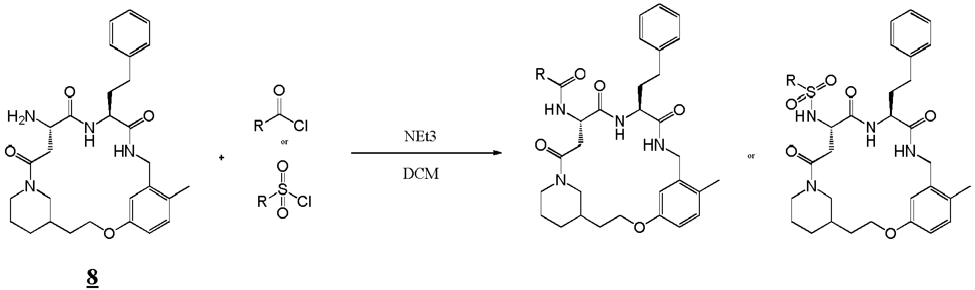

Compounds were synthesized according to general procedure E with e.g. 20 to 100 mg amine **8** and carboxylic acid chlorides or sulfonyl chlorides to give amides or sulfonamides as disclosed in table below. Purifications were achieved by HPLC or reversed phase flash chromatography.

**Table A-13**

| **Cpd no** | structure | name | Exact mass | [M+H]⁺ found |
|---|---|---|---|---|
| **180** | 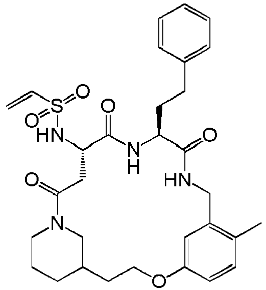 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)ethenesulfonamide | 596.3 | 597.4 |
| | | | C₃₁H₄₀ N₄O₆S | |
| **181** | 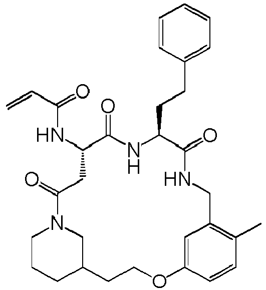 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acrylamide | 560.3 | 561.4 |
| | | | C₃₂H₄₀ N₄O₅ | |
| **216** | 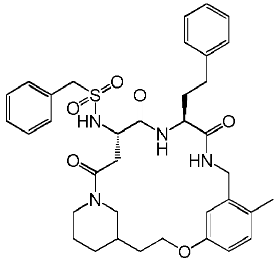 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-1-phenylmethanesulfonamide | 660.3 | 661.7 |
| | | | C₃₆H₄₄ N₄O₆S | |
| **217** | 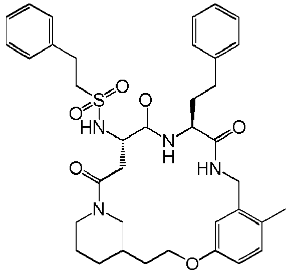 | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-phenylethane-1-sulfonamide | 674.3 | 675.7 |
| | | | C₃₇H₄₆ N₄O₆S | |
| **294** | 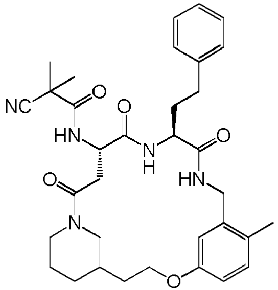 | 2-cyano-2-methyl-N-((9S,12S)-54-methyl-8,11,14-trioxo-9-phenethyl-4-oxa-7,10-diaza-1(3,1)-piperidina-5(1,3)-benzenacyclotetradecaphane-12-yl)propanamide | 601.3 | 602.5 |
| | | | C₃₄H₄₃ N₅O₅ | |

### Example A-19: Preparation of macrocyclic compound 186

### tert-butyl 2-(2-(3-(((S)-2-((S)-2-acetamido-6-(tert-butoxy)-6-oxohexanamido)-4-phenylbutanamido)methyl)-4-methylphenoxy)ethyl)morpholine-4-carboxylate (184)

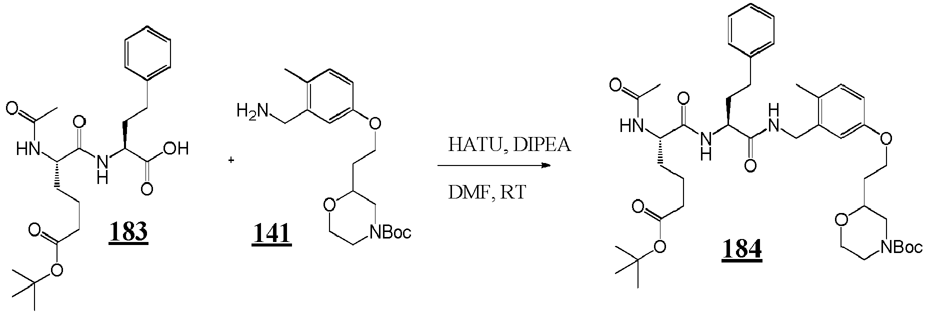

The amide bond was formed according to general procedure B with 140 mg amine **141** and 268 mg acid **183.** Purification was achieved by normal phase column chromatography (silica, cyclohexane/ethyl acetate gradient).
Formula: C₄₁H₆₀N₄O₉, exact mass: 752.4, found: 753.7 [M+H]⁺

### (5S)-5-acetamido-6-(((2S)-1-((2-methyl-5-(2-(morpholin-2-yl)ethoxy)benzyl)amino)-1-oxo-4-phenylbutan-2-yl)amino)-6-oxohexanoic acid (185)

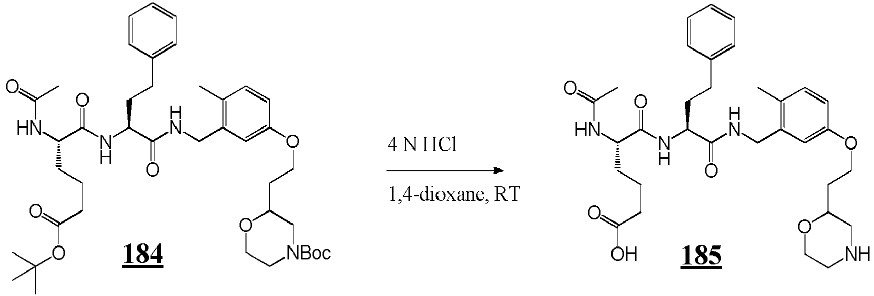

Protecting groups were cleaved from 99 mg compound **184** according to general procedure D stirring compounds in 10 ml 4 N HCl / 1,4-dioxane at room temperature. After deprotection was complete solvent was removed and the crude dissolved in acetonitrile and concentrated twice. Product used without further purification
Formula: C₃₂H₄₄N₄O₇, exact mass: 596.3, found: 597.3 [M+H]⁺

### N-((9S,12S)-5⁴-methyl-8,11,16-trioxo-9-phenethyl-4-oxa-7,10-diaza-1(2,4)-morpholina-5(1,3)-benzenacyclohexadecaphane-12-yl)acetamide (186

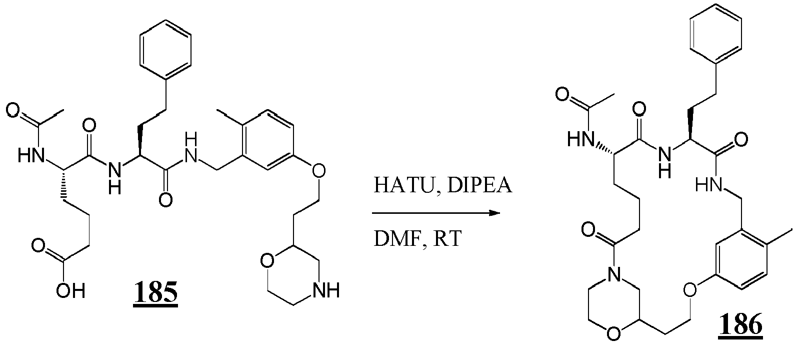

Macrocyclization was achieved according to general procedure A with 0.166 mmol amino acid **185.** Reaction mixtures were diluted with some methanol and purified via HPLC. Formula: C₃₂H₄₂N₄O₆, exact mass: 578.3, found: 579.6 [M+H]⁺

### Example A-20: Preparation of macrocyclic compound 191

### tert-butyl 6-(3-(((S)-2-((S)-2-acetamido-6-(tert-butoxy)-6-oxohexanamido)-4 phenylbutanamido)methyl)-4-methylphenoxy)-3-azabicyclo[3.2.0]heptane-3-carboxylate (189)

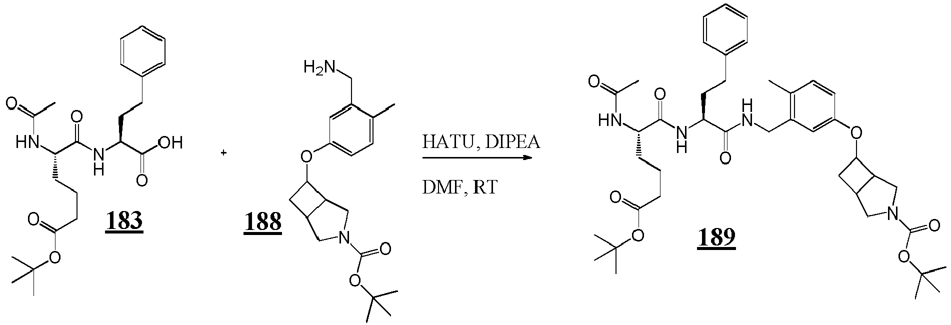

The amide bond was formed according to general procedure B with 134 mg amine **188** and 113 mg acid **183**. Purification was achieved by normal phase column chromatography (silica, cyclohexane/ethyl acetate gradient).
Formula: C₄₁H₅₈N₄O₈, exact mass: 734.4, found: 735.7 [M+H]⁺

### (5S)-6-(((2S)-1-((5-(3-azabicyclo[3.2.0]heptan-6-yloxy)-2-methylbenzyl)amino)-1-oxo-4-phenylbutan-2-yl)amino)-5-acetamido-6-oxohexanoic acid (190)

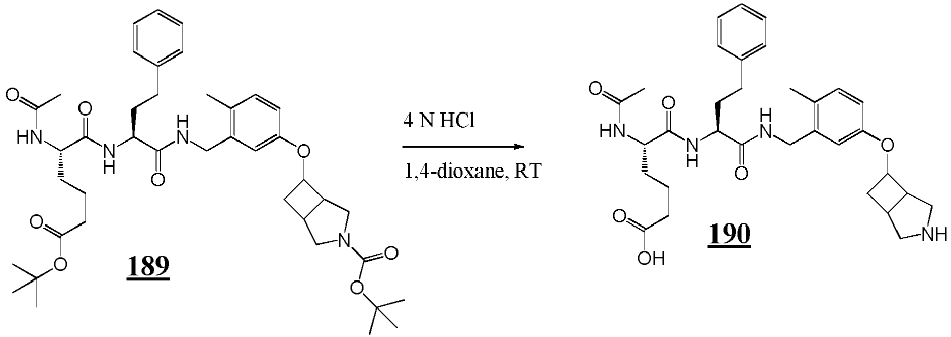

Protecting groups were cleaved from 168 mg compound **189** according to general procedure D stirring compounds in 10 ml 4 N HCl / 1,4-dioxane at room temperature. After deprotection was complete solvent was removed and the crude dissolved in acetonitrile and concentrated twice. Product used without further purification
Formula: C₃₂H₄₂N₄O₆, exact mass: 578.3, found: 579.3 [M+H]⁺

### N-((11R,15S,7S,10S)-3⁴-methyl-6,9,14-trioxo-7-phenethyl-2-oxa-13,5,8-triaza-1y(7,3)-bicyclo[3.2.0]heptana-3(1,3)-benzenacyclotetradecaphane-10-yl)acetamide (191)

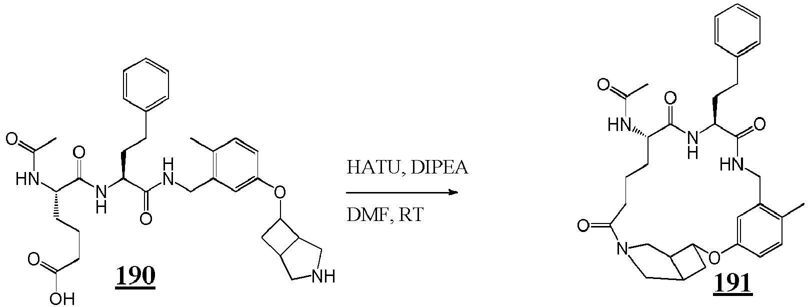

Macrocyclization was achieved according to general procedure A with 0.166 mmol amino acid **190.** Reaction mixtures were diluted with some methanol and purified via HPLC.
Formula: C₃₂H₄₀N₄O₅, exact mass: 560.3, found: 561.5 [M+H]⁺

### Example A-21: Preparation of macrocyclic compounds 347 and 353

### (S)-tert-butyl 6-(((benzyloxy)carbonyl)amino)-7-(((S)-1-((5-(3-((tert-butoxycarbonyl) amino)propoxy)-2-methylbenzyl)amino)-1-oxo-4-phenylbutan-2-yl)amino)-7-oxoheptanoate (446)

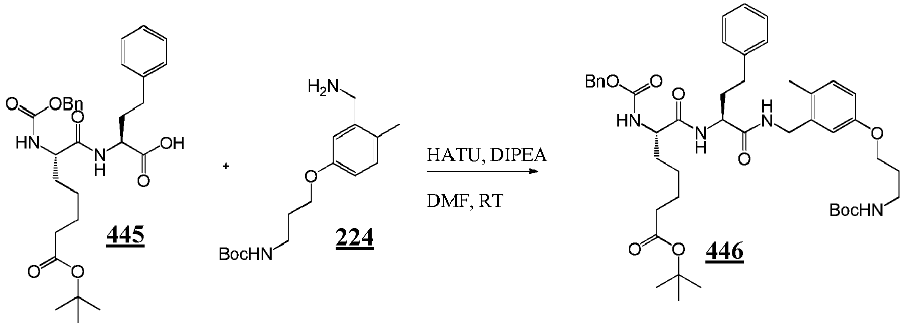

The amide bond was formed according to general procedure B with 187 mg amine **224** and 222 mg acid **445.** Purification was achieved by normal phase column chromatography (silica, cyclohexane/ethyl acetate gradient). Additional examples utilizing other amines than amine 4 are disclosed in the following table.

**Table A-14**

| **Cpd no** | From amine | structure | name | Exact mass | [M+H]⁺ found |
|---|---|---|---|---|---|
| **446** | **224** | 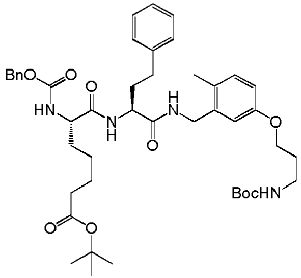 | (S)-tert-butyl 6-(((benzyloxy)carbonyl)amino)-7-(((S)-1-((5-(3-((tert-butoxycarbonyl)amino)propoxy)-2-methylbenzyl)amino)-1-oxo-4-phenylbutan-2-yl)amino)-7-oxoheptanoate | 802.5 | 703.4 |
| | | | | C₄₅H₆₂ N₄O₉ | |
| **447** | **163** | 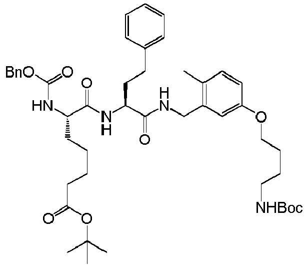 | (S)-tert-butyl 6-(((benzyloxy)carbonyl)amin o)-7-(((S)-1-((5-(4-((tert-butoxycarbonyl)amino)butoxy)-2-methylbenzyl)amino)-1-oxo-4-phenylbutan-2-yl)amino)-7-oxoheptanoate | 816.5 | 717.5 |
| | | | | C₄₆H₆₄ N₄O₉ | [M+H-Boc]+ |
| **464** | | 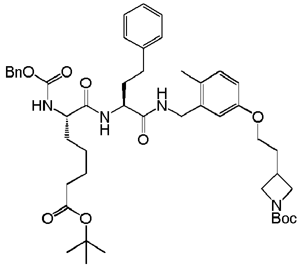 | tert-butyl 3-(2-(3-((5S,8S)-5-(5-(tert-butoxy)-5-oxopentyl)-3,6,9-trioxo-8-phenethyl-1-phenyl-2-oxa-4,7,10-triazaundecan-11-yl)-4-methylphenoxy)ethyl)azetidine-1-carboxylate | 828.5 | 829.7 |
| | | | | C₄₇H₆₄ N₄O₉ | |

### (S)-7-(((S)-1-((5-(3-aminopropoxy)-2-methylbenzyl)amino)-1-oxo-4-phenylbutan-2-yl)amino)-6-(((benzyloxy)carbonyl)amino)-7-oxoheptanoic acid (448)

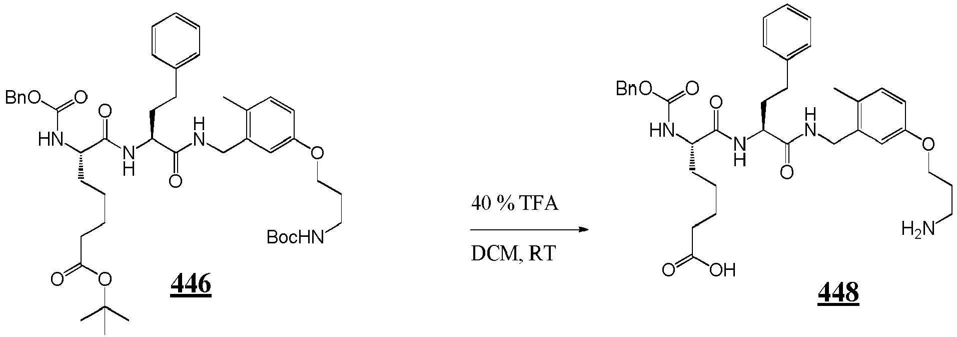

Protecting groups were cleaved from 244 mg compound **446** according to general procedure D stirring compounds in 5 ml 40 % TFA in DCM at room temperature. After deprotection was complete solvent was removed and the crude dissolved in acetonitrile and concentrated twice. Product used without further purification. Additional examples are disclosed in the following table.

**Table A-15**

| **Cpd no** | From cpd | structure | name | Exact mass | [M+H]⁺ found |
|---|---|---|---|---|---|
| **448** | **446** | 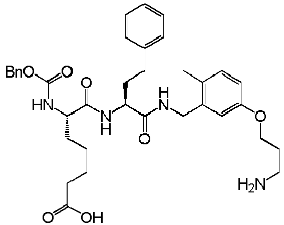 | (S)-7-(((S)-1-((5-(3-aminopropoxy)-2-methylbenzyl)amino)-1-oxo-4-phenylbutan-2-yl)amino)-6-(((benzyloxy)carbonyl)amino)-7-oxoheptanoic acid | 646.3 | 647.4 |
| | | | | C₃₆H₄₆ N₄O₇ | |
| **449** | **447** | 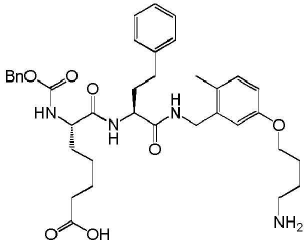 | (S)-7-(((S)-1-((5-(4-aminobutoxy)-2-methylbenzyl)amino)-1-oxo-4-phenylbutan-2-yl)amino)-6-(((benzyloxy)carbonyl)amino)-7-oxoheptanoic acid | 660.4 | 661.4 |
| | | | | C₃₇H₄₈ N₄O₇ | |
| **465** | **464** | 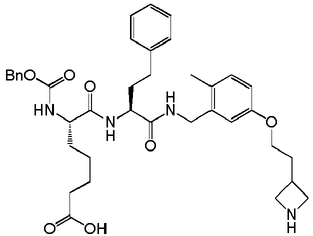 | (S)-7-(((S)-1-((5-(2-(azetidin-3-yl)ethoxy)-2-methylbenzyl)amino)-1-oxo-4-phenylbutan-2-yl)amino)-6-(((benzyloxy)carbonyl)amino)-7-oxoheptanoic acid | 672.4 | 673.4 |
| | | | | C₃₈H₄₈ N₄O₇ | |

### benzyl ((13S,16S)-1⁴-methyl-8,14,17-trioxo-16-phenethyl-2-oxa-7,15,18-triaza-1(1,3)-benzenacyclononadecaphane-13-yl)carbamate (347)

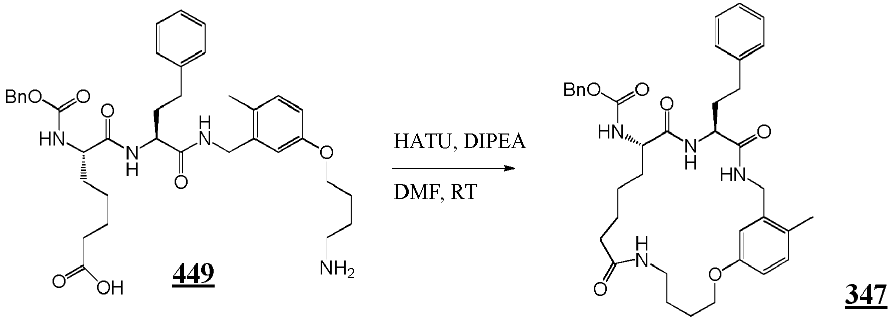

Macrocyclization was achieved according to general procedure A with 0.3 mmol amino acid **449.** Reaction mixtures were diluted with some methanol and purified via HPLC. Additional examples are disclosed in the following table.

**Table A-16**

| **Cpd no** | From cpd | structure | name | Exact mass | [M+H]⁺ found |
|---|---|---|---|---|---|
| **347** | **449** | 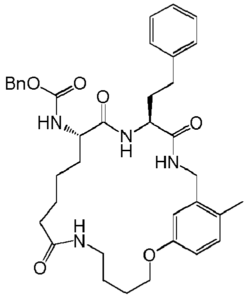 | benzyl ((13S,16S)-1⁴-methyl-8,14,17-trioxo-16-phenethyl-2-oxa-7,15,18-triaza-1(1,3)-benzenacyclononadecaphane-13-yl)carbamate | 642.3 | 643.4 |
| | | | | C₃₇H₄₆ N₄O₆ | |
| **353** | **465** | 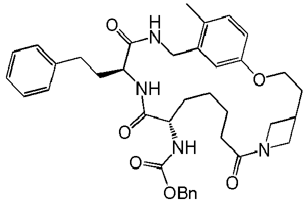 | benzyl ((9S,12S)-5⁴-methyl-8,11,17-trioxo-9-phenethyl-4-oxa-7,10-diaza-1(3,1)-azetidina-5(1,3)-benzenacycloheptadecaphane-12-yl)carbamate | 654.3 | 655.5 |
| | | | | C₃₈H₄₆ N₄O₆ | |

### Example A-22: Preparation of macrocyclic compounds 195, 283, 322, 351, 352, 354, and 405

### tert-butyl 3-(2-(3-((5S,8S)-5-(4-(tert-butoxy)-4-oxobutyl)-3,6,9-trioxo-8-phenethyl-1-phenyl-2-oxa-4,7,10-triazaundecan-11-yl)-4-methylphenoxy)ethyl)piperidine-1-carboxylate (193)

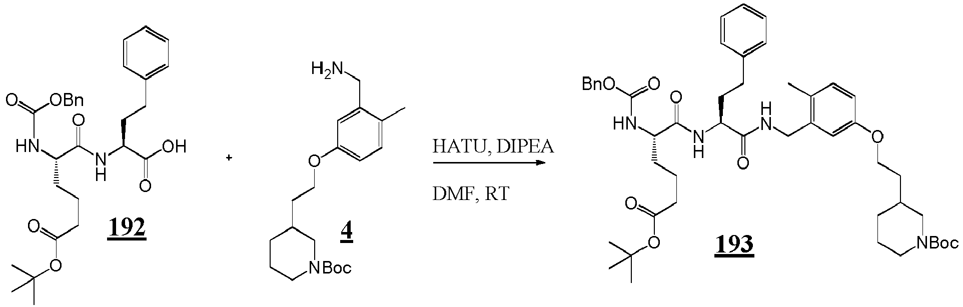

The amide bond was formed according to general procedure B with 615 mg amine 4 and 711 mg acid **192.** Purification was achieved by normal phase column chromatography (silica, cyclohexane/ethyl acetate gradient). Additional examples utilizing other amines than amine 4 are disclosed in the following table.

**Table A-17**

| **Cpd no** | From amine | structure | name | Exact mass | [M+H]⁺ found |
|---|---|---|---|---|---|
| **193** | **4** | 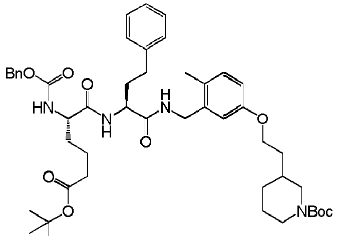 | tert-butyl 3-(2-(3-((5S,8S)-5-(4-(tert-butoxy)-4-oxobutyl)-3,6,9-trioxo-8-phenethyl-1-phenyl-2-oxa-4,7,10-triazaundecan-11-yl)-4-methylphenoxy)ethyl)piperidine-1-carboxylate | 842.5 | 843.8 |
| | | | | C₄₈H₆₆ N₄O₉ | |
| **403** | **399** | 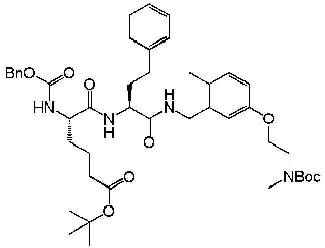 | (S)-tert-butyl 5-(((benzyloxy)carbonyl)amino)-6-(((S)-1-((5-(2-((tert-butoxycarbonyl)(methyl)amino)ethoxy)-2-methylbenzyl)amino)-1-oxo-4-phenylbutan-2-yl)amino)-6-oxohexanoate | 788.4 | 789.4 |
| | | | | C₄₄H₆₀ N₄O₉ | |
| **424** | **224** | 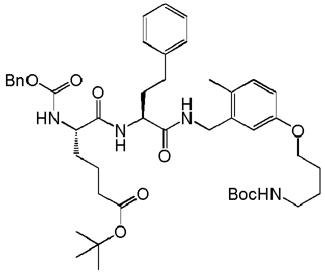 | (S)-tert-butyl 5-(((benzyloxy)carbonyl)amino)-6-(((S)-1-((5-(4-((tert-butoxycarbonyl)amino)butoxy)-2-methylbenzyl)amino)-1-oxo-4-phenylbutan-2-yl)amino)-6-oxohexanoate | 802.5 | 703.4 |
| | | | | C₄₅H₆₂ N₄O₉ | |
| **458** | **426** | 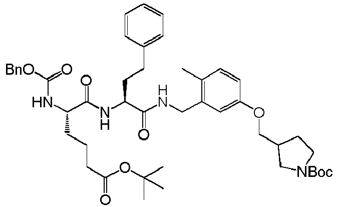 | tert-butyl 3-((3-((5S,8S)-5-(4-(tert-butoxy)-4-oxobutyl)-3,6,9-trioxo-8-phenethyl-1-phenyl-2-oxa-4,7,10-triazaundecan-11-yl)-4-methylphenoxy) methyl)pyrrolidine-1-carboxylate | 814.5 | 815.7 |
| | | | | C₄₆H₆₂ N₄O₉ | |
| **459** | **455** | 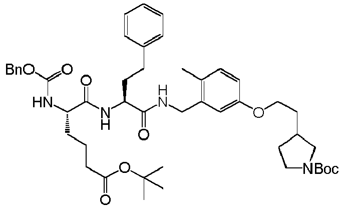 | tert-butyl 3-(2-(3-((5S,8S)-5-(4-(tert-butoxy)-4-oxobutyl)-3,6,9-trioxo-8-phenethyl-1 -phenyl-2-oxa-4,7,10-triazaundecan-11-yl)-4-methylphenoxy)ethyl)pyrrolidine-1-carboxylate | 828.4 | 829.4 |
| | | | | C₄₇H₆₄ N₄O₉ | |
| **467** | **468** | 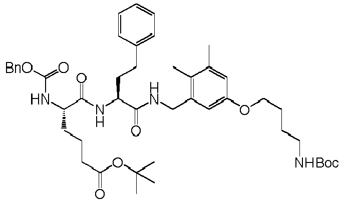 | (S)-tert-butyl 5-(((benzyloxy)carbonyl)amino)-6-(((S)-1-((5-(4-((tert-butoxycarbonyl)amino)butoxy)-2,3-dimethylbenzyl)amino)-1-oxo-4-phenylbutan-2-yl)amino)-6-oxohexanoate | 816.5 | 817.4 |
| | | | | C₄₆H₆₄ N₄O₉ | |
| **275** | **271** | 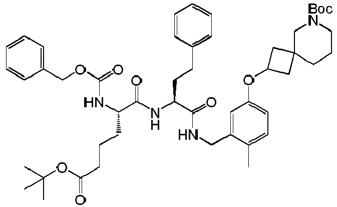 | tert-butyl 2-(3-((5S,8S)-5-(4-(tert-butoxy)-4-oxobutyl)-3,6,9-trioxo-8-phenethyl-1-phenyl-2-oxa-4,7,10-triazaundecan-11-yl)-4-methylphenoxy)-6-azaspiro[3.5]nonane-6-carboxylate | 854.5 | 855.3 |
| | | | | C₄₉H₆₆ N₄O₉ | |

### (5S)-5-(((benzyloxy)carbonyl)amino)-6-(((2S)-1-((2-methyl-5-(2-(piperidin-3-yl)ethoxy)benzyl)amino)-1-oxo-4-phenylbutan-2-yl)amino)-6-oxohexanoic acid (194)

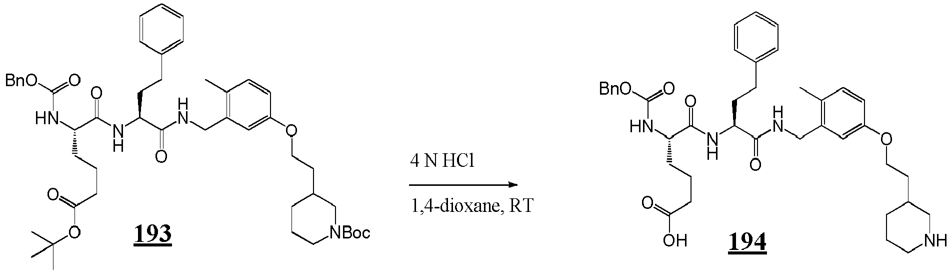

Protecting groups were cleaved from 900 mg compound **193** according to general procedure D stirring compounds in 10 ml 4 N HCl / 1,4-dioxane at room temperature. After deprotection was complete solvent was removed and the crude dissolved in acetonitrile and concentrated twice. Product used without further purification. Additional examples are disclosed in the following table.

**Table A-18**

| **Cpd no** | From cpd | structure | name | Exact mass | [M+H]⁺ found |
|---|---|---|---|---|---|
| **194** | **193** | 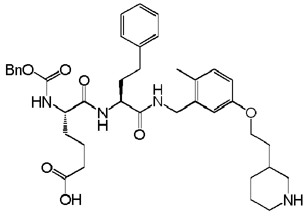 | (5S)-5-(((benzyloxy)carbonyl)amino)-6-(((2S)-1-((2-methyl-5-(2-(piperidin-3-yl)ethoxy)benzyl)amino)-1-oxo-4-phenylbutan-2-yl)amino)-6-oxohexanoic acid | 686.4 | 687.4 |
| | | | | C₃₉H₅₀ N₄O₇ | |
| **404** | **403** | 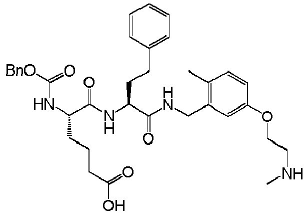 | (S)-5-(((benzyloxy)carbonyl)amino)-6-(((S)-1-((2-methyl-5-(2-(methylamino)ethoxy)benzyl)amino)-1-oxo-4-phenylbutan-2-yl)amino)-6-oxohexanoic acid | 632.3 | - |
| | | | | C₃₅H₄₄ N₄O₇ | |
| **425** | **424** | 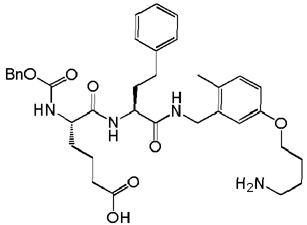 | (S)-6-(((S)-1-((5-(4-aminobutoxy)-2-methylbenzyl)amino)-1-oxo-4-phenylbutan-2-yl)amino)-5-(((benzyloxy)carbonyl)amino)-6-oxohexanoic acid | 646.3 | 647.3 |
| | | | | C₃₆H₄₆ N₄O₇ | |
| **460** | **458** | 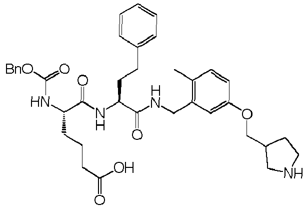 | (5S)-5-(((benzyloxy)carbonyl)amino)-6-(((2S)-1-((2-methyl-5-(pyrrolidin-3-ylmethoxy)benzyl)amino)-1-oxo-4-phenylbutan-2-yl)amino)-6-oxohexanoic acid | 658.3 | 659.6 |
| | | | | C₃₇H₄₆ N₄O₇ | |
| **461** | **459** | 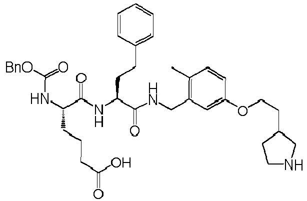 | (5S)-5-(((benzyloxy)carbonyl)amino)-6-(((2S)-1-((2-methyl-5-(2-(pyrrolidin-3-yl)ethoxy)benzyl)amino)-1-oxo-4-phenylbutan-2-yl)amino)-6-oxohexanoic acid | 672.4 | 673.4 |
| | | | | C₃₈H₄₈ N₄O₇ | |
| **469** | **467** | 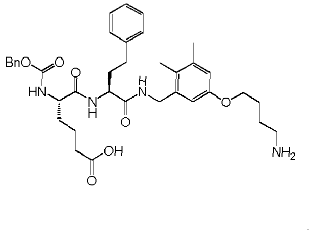 | (S)-6-(((S)-1-((5-(4-aminobutoxy)-2,3-dimethylbenzyl)amino)-1-oxo-4-phenylbutan-2-yl)amino)-5-(((benzyloxy)carbonyl)amino)-6-oxohexanoic acid | 660.4 | 661.5 |
| | | | | C₃₇H₄₈ N₄O₇ | |
| **279** | **275** | 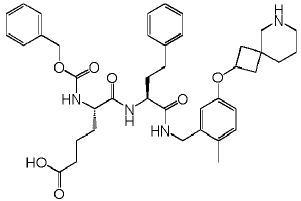 | (S)-6-(((S)-1-((5-(6-azaspiro[3.5]nonan-2-yloxy)-2-methylbenzyl)amino)-1-oxo-4-phenylbutan-2-yl)amino)-5-(((benzyloxy)carbonyl)amino)-6-oxohexanoic acid | 698.4 | 699.4 |
| | | | | C₄₀H₅₀ N₄O₇ | |

### benzyl ((9S,12S)-5⁴-methyl-8,11,16-trioxo-9-phenethyl-4-oxa-7,10-diaza-1(3,1)-piperidina-5(1,3)-benzenacyclohexadecaphane-12-yl)carbamate (195)

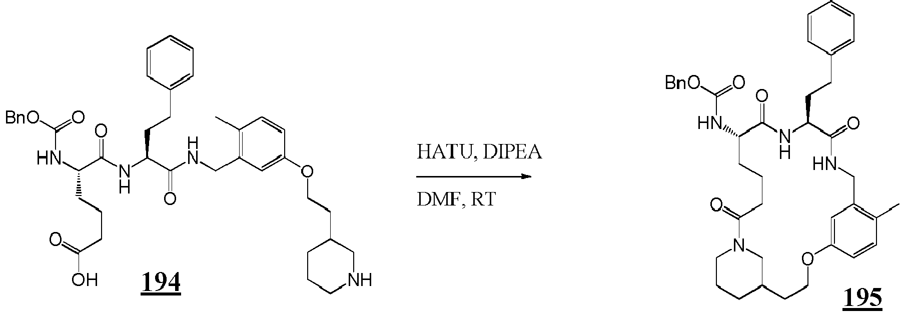

Macrocyclization was achieved according to general procedure A with 1.07 mmol amino acid **194.** Reaction mixtures were diluted with some methanol and purified via HPLC. Additional examples are disclosed in the following table.

**Table A-19**

| **Cpd no** | From cpd | structure | name | Exact mass | [M+H]⁺ found |
|---|---|---|---|---|---|
| **195** | **194** | 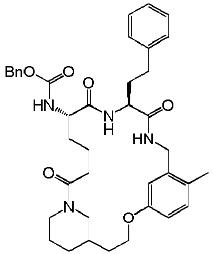 | benzyl ((9S,12S)-5⁴-methyl-8,11,16-trioxo-9-phenethyl-4-oxa-7,10-diaza-1(3,1)-piperidina-5(1,3)-benzenacyclohexadecaphane-12-yl)carbamate | 668.4 | 669.7 |
| | | | | C₃₉H₄₈ N₄O₆ | |
| **405** | **404** | 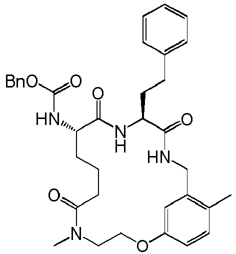 | benzyl ((108,138)-1⁴,5-dimethyl-6,11,14-trioxo-13-phenethyl-2-oxa-5,12,15-triaza-1(1,3)-benzenacyclohexadecaphane-10-yl)carbamate | 614.3 | 615.3 |
| | | | | C₃₅H₄₂ N₄O₆ | |
| **322** | **425** | 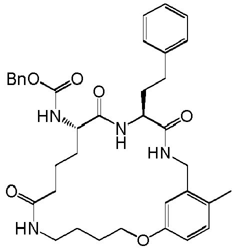 | benzyl ((12S,15S)-1⁴-methyl-8,13,16-trioxo-15-phenethyl-2-oxa-7,14,17-triaza-1(1,3)-benzenacyclooctadecaphane-12-yl)carbamate | 628.3 | 629.4 |
| | | | | C₃₆H₄₄ N₄O₆ | |
| **351** | **460** | 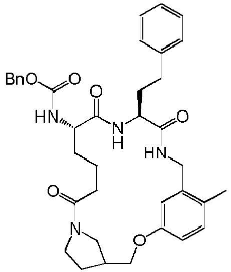 | benzyl ((8S,11S)-4⁴-methyl-7,10,15-trioxo-8-phenethyl-3-oxa-6,9-diaza-1(3,1)-pyrrolidina-4(1,3)-benzenacyclopentadecaphane-11-yl)carbamate | 640.3 | 641.5 |
| | | | | C₃₇H₄₄ N₄O₆ | |
| **352** | **461** | 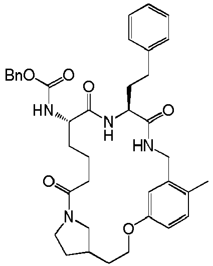 | benzyl ((9S,12S)-5⁴-methyl-8,11,16-trioxo-9-phenethyl-4-oxa-7,10-diaza-1(3,1)-pyrrolidina-5(1,3)-benzenacyclohexadecaphane-12-yl)carbamate | 654.3 | 655.6 |
| | | | | C₃₈H₄₆ N₄O₆ | |
| **354** | **469** | 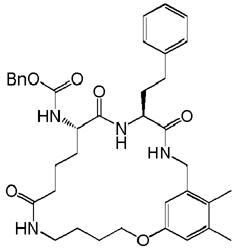 | benzyl ((12S,15S)-14,15-dimethyl-8,13,16-trioxo-15-phenethyl-2-oxa-7,14,17-triaza-1(1,3)-benzenacyclooctadecaphane-12-yl)carbamate | 642.3 | 643.5 |
| | | | | C₃₇H₄₆ N₄O₆ | |
| **283** | **279** | 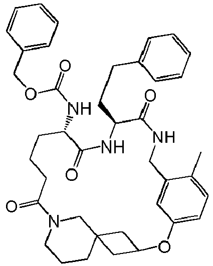 | benzyl ((7S,10S)-34-methyl-6,9,14-trioxo-7-phenethyl-2-oxa-5,8-diaza-15(3,1)-piperidina-3(1,3)-benzena-1(1,3)-cyclobutanapentadecaphane-10-yl)carbamate | 680.4 | 681.5 |
| | | | | C₄₀H₄₈ N₄O₆ | |

### Example A-23: Preparation of macrocyclic compounds 199, 301, 349, 350 and 402

### tert-butyl 3-(2-(3-((5S,8S)-5-(3-(tert-butoxy)-3-oxopropyl)-3,6,9-trioxo-8-phenethyl-1-phenyl-2-oxa-4,7,10-triazaundecan-11-yl)-4-methylphenoxy)ethyl)piperidine-1-carboxylate (197)

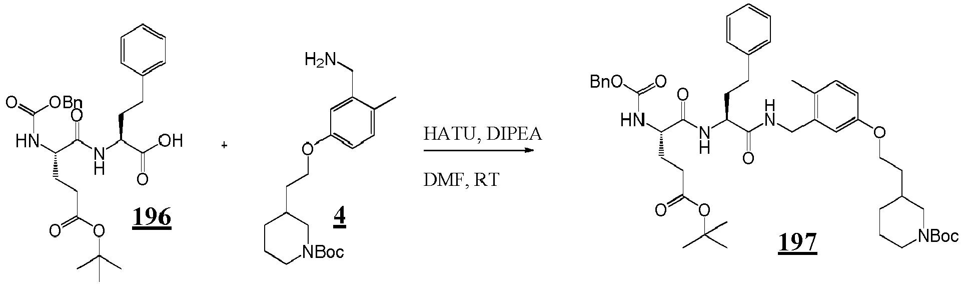

The amide bond was formed according to general procedure B with 615 mg amine **4** and 808 mg acid **196.** Purification was achieved by normal phase column chromatography (silica, cyclohexane/ethyl acetate gradient). Additional examples utilizing other amines than amine 4 are disclosed in the following table.

**Table A-20**

| **Cpd no** | From amine | structure | name | Exact mass | [M+H]⁺ found |
|---|---|---|---|---|---|
| **197** | **4** | 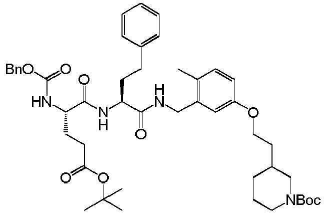 | tert-butyl 3-(2-(3-((5S,8S)-5-(3-(tert-butoxy)-3-oxopropyl)-3,6,9-trioxo-8-phenethyl-1 -phenyl-2-oxa-4,7,10-triazaundecan-11-yl)-4-methyl phenoxy)ethyl)piperidine-1-carboxylate | 828.5 | 830.6 |
| | | | | C₄₇H₆₄ N₄O₉ | |
| **385** | **271** | 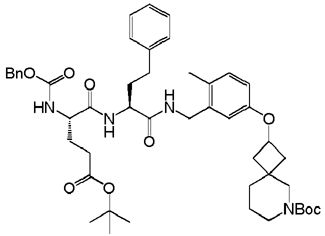 | tert-butyl 2-(3-((5S,8S)-5-(3-(tert-butoxy)-3-oxopropyl)-3,6,9-trioxo-8-phenethyl-1-phenyl-2-oxa-4,7,10-triazaundecan-11-yl)-4-methylphenoxy)-6-azaspiro[3.5]nonane-6-carboxylate | 840.5 | 841.6 |
| | | | | C₄₈H₆₄ N₄O₉ | |
| **400** | **399** | 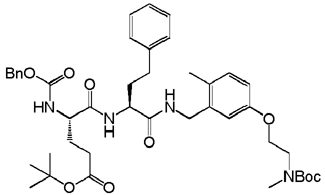 | (S)-tert-butyl 4-(((benzyloxy)carbonyl)amino)-5-(((S)-1-((5-(2-((tert-butoxycarbonyl)(methyl)amino)ethoxy)-2-methylbenzyl)amino)-1-oxo-4-phenylbutan-2-yl)amino)-5-oxopentanoate | 774.4 | - |
| | | | | C₄₃H₅₈ N₄O₉ | |
| **451** | **426** | 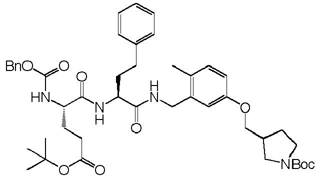 | tert-butyl 3-((3-((5S,8S)-5-(3-(tert-butoxy)-3-oxopropyl)-3,6,9-trioxo-8-phenethyl-1-phenyl-2-oxa-4,7,10-triazaundecan-11-yl)-4-methylphenoxy) methyl)pyrrolidine-1-carboxylate | 800.4 | 801.4 |
| | | | | C₄₅H₆₀ N₄O₉ | |
| **452** | **455** | 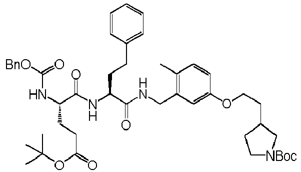 | tert-butyl 3-(2-(3-((5S,8S)-5-(3-(tert-butoxy)-3-oxopropyl)-3,6,9-trioxo-8-phenethyl-1 -phenyl-2-oxa-4,7,10-triazaundecan-11-yl)-4-methylphenoxy)ethyl)pyrrolidine-1-carboxylate | 814.5 | 815.4 |
| | | | | C₄₆H₆₂ N₄O₉ | |

### (4S)-4-(((benzyloxv)carbonyl)amino)-5-(((2S)-1-((2-methyl-5-(2-(piperidin-3-yl)ethoxy)benzyl)amino)-1-oxo-4-phenylbutan-2-yl)amino)-5-oxopentanoic acid (198)

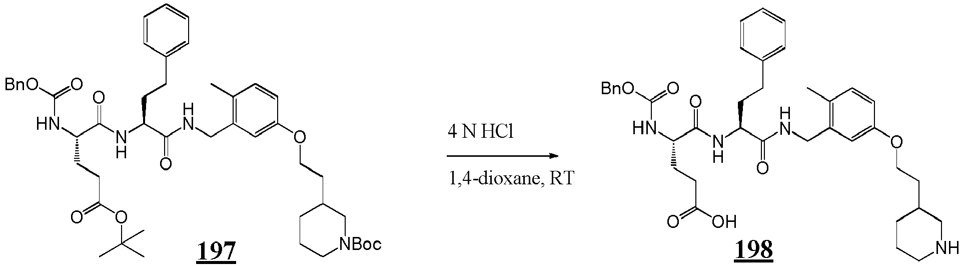

Protecting groups were cleaved from 850 mg compound **197** according to general procedure D stirring compounds in 10 ml 4 N HCl / 1,4-dioxane at room temperature. After deprotection was complete solvent was removed and the crude dissolved in acetonitrile and concentrated twice. Product used without further purification. Additional examples are disclosed in the following table.

**Table A-21**

| **Cpd no** | From cpd | structure | name | Exact mass | [M+H]⁺ found |
|---|---|---|---|---|---|
| **198** | **197** | 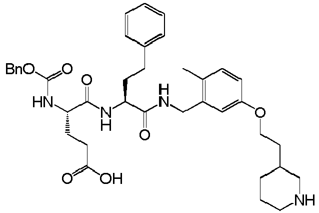 | (4S)-4-(((benzyloxy)carbonyl)amino)-5-(((2S)-1-((2-methyl-5-(2-(piperidin-3-yl)ethoxy)benzyl)amino)-1-oxo-4-phenylbutan-2-yl)amino)-5-oxopentanoic acid | 672.4 | 673.7 |
| | | | | C₃₈H₄₈ N₄O₇ | |
| **386** | **385** | 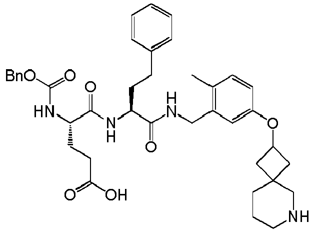 | (S)-5-(((S)-1-((5-(6-azaspiro[3.5]nonan-2-yloxy)-2-methylbenzyl)amino)-1-oxo-4-phenylbutan-2-yl)amino)-4-(((benzyloxy)carbonyl)amino)-5-oxopentanoic acid | 684.4 | 685.4 |
| | | | | C₃₉H₄₈ N₄O₇ | |
| **401** | **400** | 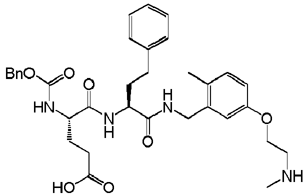 | (S)-4-(((benzyloxy)carbonyl)amino)-5-(((S)-1-((2-methyl-5-(2-(methylamino)ethoxy)benzyl)amino)-1-oxo-4-phenylbutan-2-yl)amino)-5-oxopentanoic acid | 618.3 | - |
| | | | | C₃₄H₄₂ N₄O₇ | |
| **456** | **451** | 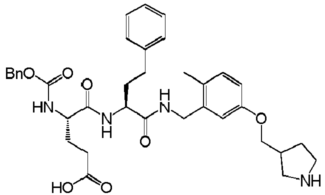 | (4S)-4-(((benzyloxy)carbonyl)amino)-5-(((2S)-1-((2-methyl-5-(pyrrolidin-3-ylmethoxy)benzyl)amino)-1-oxo-4-phenylbutan-2-yl)amino)-5-oxopentanoic acid | 644.3 | 645.5 |
| | | | | C₃₆H₄₄ N₄O₇ | |
| **457** | **452** | 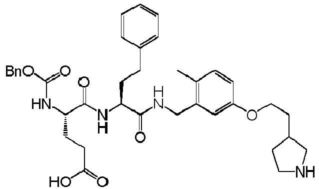 | (4S)-4-(((benzyloxy)carbonyl)amino)-5-(((2S)-1-((2-methyl-5-(2-(pyrrolidin-3-yl)ethoxy)benzyl)amino)-1-oxo-4-phenylbutan-2-yl)amino)-5-oxopentanoic acid | 658.3 | 629.5 |
| | | | | C₃₇H₄₆ N₄O₇ | |

### benzyl ((9S,12S)-5⁴-methyl-8,11,15-trioxo-9-phenethyl-4-oxa-7,10-diaza-1(3,1)-piperidina-5(1,3)-benzenacyclopentadecaphane-12-yl)carbamate (199)

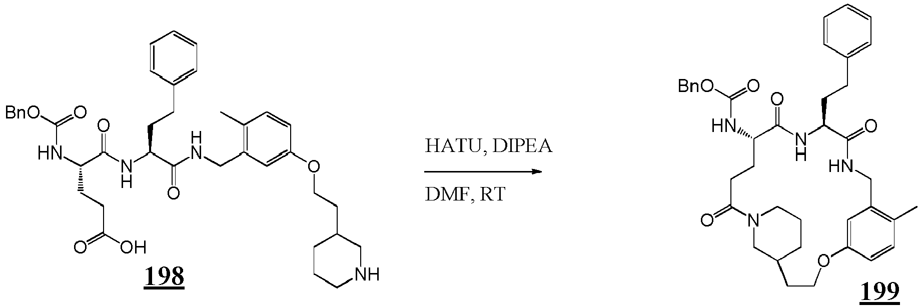

Macrocyclization was achieved according to general procedure A with 1.03 mmol amino acid **198.** Reaction mixtures were diluted with some methanol and purified via HPLC. Additional examples are disclosed in the following table.

**Table A-22**

| **Cpd no** | From cpd | structure | name | Exact mass | [M+H]⁺ found |
|---|---|---|---|---|---|
| **199** | **198** | 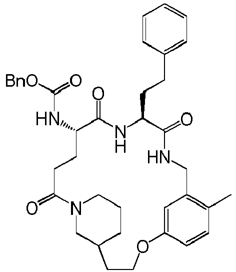 | benzyl ((9S,12S)-5⁴-methyl-8,11,15-trioxo-9-phenethyl-4-oxa-7, 10-diaza-1(3,1)-piperidina-5(1,3)-benzenacyclopentadecaphane-12-yl)carbamate | 654.3 | 655.6 |
| | | | | C₃₈H₄₆ N₄O₆ | |
| **301** | **386** | 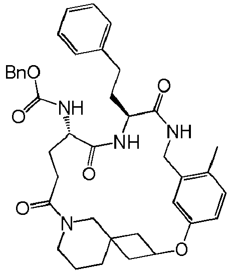 | benzyl ((7S,10S)-3⁴-methyl-6,9,13-trioxo-7-phenethyl-2-oxa-5,8-diaza-14(3,1)-piperidina-3(1,3)-benzena-1(1,3)-cyclobutanatetradecaphane-10-yl)carbamate | 666.3 | 667.4 |
| | | | | C₃₉H₄₆ N₄O₆ | |
| **402** | **401** | 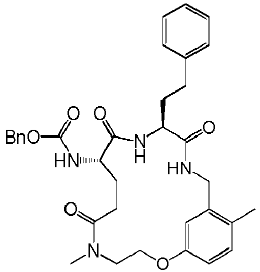 | benzyl ((9S,12S)-1⁴,5-dimethyl-6,10,13-trioxo-12-phenethyl-2-oxa-5,11,14-triaza-1(1,3)-benzenacyclopentadecaphane-9-yl)carbamate | 600.3 | 601.3 |
| | | | | C₃₄H₄₀ N₄O₆ | |
| **349** | **456** | 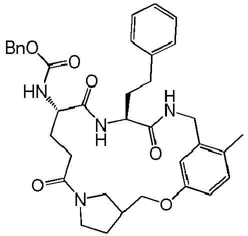 | benzyl ((8S,11S)-4⁴-methyl-7,10,14-trioxo-8-phenethyl-3-oxa-6,9-diaza-1(3,1)-pyrrolidina-4(1,3)-benzenacyclotetradecaphane-11-yl)carbamate | 626.3 | 627.4 |
| | | | | C₃₆H₄₂ N₄O₆ | |
| **350** | **457** | 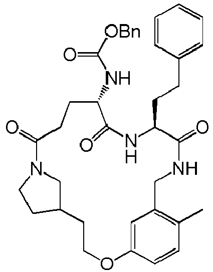 | benzyl ((9S,12S)-5⁴-methyl-8,11,15-trioxo-9-phenethyl-4-oxa-7,10-diaza-1(3,1)-pyrrolidina-5(1,3)-benzenacyclopentadecaphane-12-yl)carbamate | 640.3 | 640.5 |
| | | | | C₃₇H₄₄ N₄O₆ | |

### Example A-24: Preparation of macrocyclic compounds 300, 312, 313, 314, 315, 325, and 337

### tert-butyl 2-(3-((5S,8S)-5-(2-(tert-butoxy)-2-oxoethyl)-3,6,9-trioxo-8-phenethyl-1-phenyl-2-oxa-4,7,10-triazaundecan-11-yl)-4-methylphenoxy)-6-azaspiro[3.5]nonane-6-carboxylate (383)

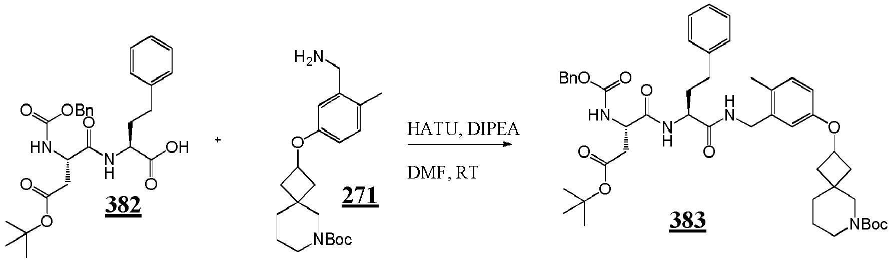

The amide bond was formed according to general procedure B with 100 mg amine **271** and 336 mg acid **382.** Purification was achieved by normal phase column chromatography (silica, cyclohexane/ethyl acetate gradient). Additional examples utilizing other amines than amine **271** are disclosed in the following table.

**Table A-23**

| **Cpd no** | From amine | structure | name | Exact mass | [M+H]⁺ found |
|---|---|---|---|---|---|
| **383** | **271** | 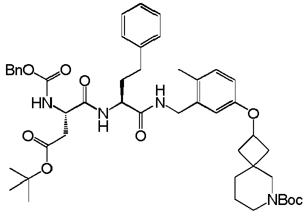 | tert-butyl 2-(3-((5S,8S)-5-(2-(tert-butoxy)-2-oxoethyl)-3,6,9-trioxo-8-phenethyl-1-phenyl-2-oxa-4,7,10-triazaundecan-11-yl)-4-methylphenoxy)-6-azaspiro[3.5]nonane-6-carboxylate | 814.5 | 717.5 [M+H-Boc]+ |
| **397** | **399** | 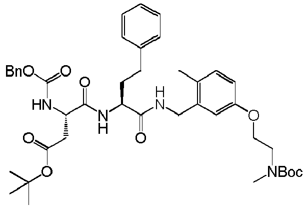 | (S)-tert-butyl 3-(((benzyloxy)carbonyl)amino)-4-(((S)-1-((5-(2-((tert-butoxycarbonyl)(methyl)amino)ethoxy)-2-methylbenzyl)amino)-1-oxo-4-phenylbutan-2-yl)amino)-4-oxobutanoate | 826.5 | 727.5 [M+H-Boc]+ |
| **406** | **416** | 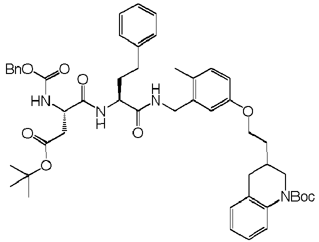 | tert-butyl 3-(2-(3-((5S,8S)-5-(2-(tert-butoxy)-2-oxoethyl)-3,6,9-trioxo-8-phenethyl-1-phenyl-2-oxa-4,7,10-triazaundecan-11-yl)-4-methylphenoxy)ethyl)-3,4-dihydroquinoline-1(2H)-carboxylate | 862.5 | 863.3 |
| **407** | **417** | 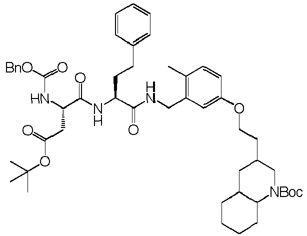 | tert-butyl 3-(2-(3-((5S,8S)-5-(2-(tert-butoxy)-2-oxoethyl)-3,6,9-trioxo-8-phenethyl-1-phenyl-2-oxa-4,7,10-triazaundecan-11-yl)-4-methyl phenoxy)ethyl)octahydroquinoline-1(2H)-carboxylate | 868.5 | 869.3 |
| **410** | **411** | 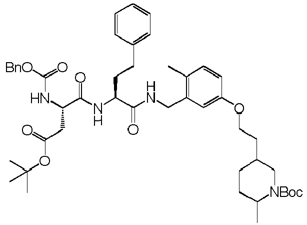 | tert-butyl 5-(2-(3-((5S,8S)-5-(2-(tert-butoxy)-2-oxoethyl)-3,6,9-trioxo-8-phenethyl-1-phenyl-2-oxa-4,7,10-triazaundecan-11-yl)-4-methylphenoxy)ethyl)-2-methylpiperidine-1-carboxylate | 828.5 | 729.6 [M+H-Boc]+ |
| **427** | **426** | 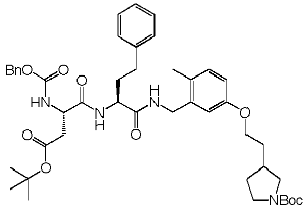 | tert-butyl 3-(2-(3-((5S,8S)-5-(2-(tert-butoxy)-2-oxoethyl)-3,6,9-trioxo-8-phenethyl-1-phenyl-2-oxa-4,7,10-triazaundecan-11-yl)-4-methylphenoxy)ethyl)pyrrolidine-1-carboxylate | 800.4 | 701.4 [M+H-Boc]+ |
| **432** | **430** | 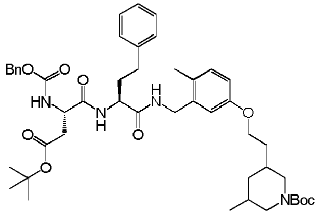 | tert-butyl 3-(2-(3-((5S,8S)-5-(2-(tert-butoxy)-2-oxoethyl)-3,6,9-trioxo-8-phenethyl-1-phenyl-2-oxa-4,7,10-triazaundecan-11-yl)-4-methylphenoxy)ethyl)-5-methylpiperidine-1-carboxylate | 828.5 | 829.4 |

### (S)-4-(((S)-1-((5-(6-azaspiro[3.5]nonan-2-yloxy)-2-methylbenzyl)amino)-1-oxo-4-phenylbutan-2-yl)amino)-3-(((benzyloxy)carbonyl)amino)-4-oxobutanoic acid (384)

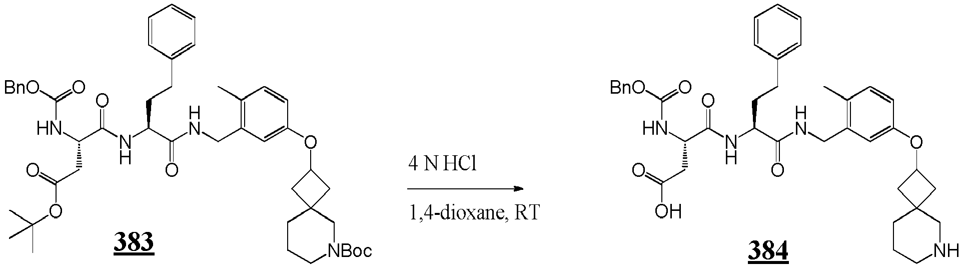

Protecting groups were cleaved from 900 mg compound **383** according to general procedure D stirring compounds in 10 ml 4 N HCl / 1,4-dioxane at room temperature. After deprotection was complete solvent was removed and the crude dissolved in acetonitrile and concentrated twice. Product used without further purification. Additional examples are disclosed in the following table.

**Table A-24**

| **Cpd no** | From cpd | structure | name | Exact mass | [M+H]⁺ found |
|---|---|---|---|---|---|
| **384** | **383** | 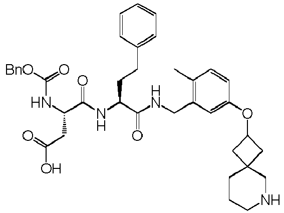 | (S)-4-(((S)-1-((5-(6-azaspiro[3.5]nonan-2-yloxy)-2-methylbenzyl)amino)-1-oxo-4-phenylbutan-2-yl)amino)-3-(((benzyloxy)carbonyl)amino)-4-oxobutanoic acid | 670.3 | 671.4 |
| **398** | **397** | 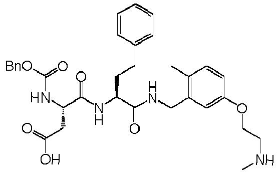 | (S)-3-(((benzyloxy)carbonyl)amino)-4-(((S)-1-((2-methyl-5-(2-(methylamino)ethoxy)benzyl)amino)-1-oxo-4-phenylbutan-2-yl)amino)-4-oxobutanoic acid | 604.3 | |
| **408** | **406** | 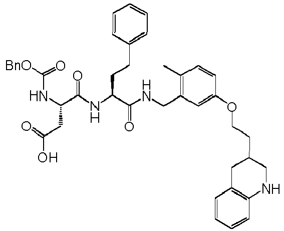 | (3S)-3-(((benzyloxy)carbonyl)amino)-4-(((2S)-1-((2-methyl-5-(2-(1,2,3,4-tetrahydroquinolin-3-yl)ethoxy)benzyl)amino)-1-oxo-4-phenylbutan-2-yl)amino)-4-oxobutanoic acid | 706.3 | 707.3 |
| **409** | **407** | 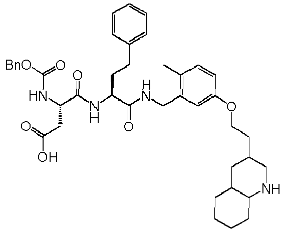 | (3S)-3-(((benzyloxy)carbonyl)amino)-4-(((2S)-1-((5-(2-(decahydroquinolin-3-yl)ethoxy)-2-methylbenzyl)amino)-1-oxo-4-phenylbutan-2-yl)amino)-4-oxobutanoic acid | 712.4 | 713.3 |
| **418** | **410** | 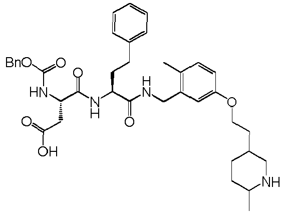 | (3S)-3-(((benzyloxy)carbonyl)amino)-4-(((2S)-1-((2-methyl-5-(2-(6-methylpiperidin-3-yl)ethoxy)benzyl)amino)-1-oxo-4-phenylbutan-2-yl)amino)-4-oxobutanoic acid | 672.4 | 673.3 |
| **428** | **427** | 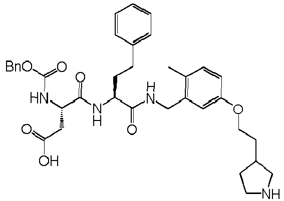 | (3S)-3-(((benzyloxy)carbonyl)amino)-4-(((2S)-1-((2-methyl-5-(2-(pyrrolidin-3-yl)ethoxy)benzyl)amino)-1-oxo-4-phenylbutan-2-yl)amino)-4-oxobutanoic acid | 644.3 | 645.2 |
| **433** | **432** | 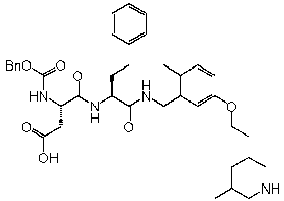 | (3S)-3-(((benzyloxy)carbonyl)amino)-4-(((2S)-1-((2-methyl-5-(2-(5-methylpiperidin-3-yl)ethoxy)benzyl)amino)-1-oxo-4-phenylbutan-2-yl)amino)-4-oxobutanoic acid | 672.4 | 673.4 |

### benzyl ((7S,10S)-3⁴-methyl-6,9,12-trioxo-7-phenethyl-2-oxa-5,8-diaza-13(3,1)-piperidina-3(1,3)-benzena-1(1,3)-cyclobutanatridecaphane-10-yl)carbamate (300)

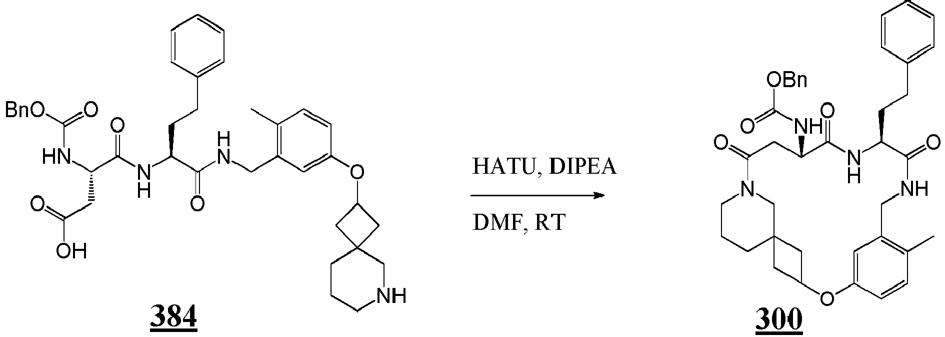

Macrocyclization was achieved according to general procedure A with 93 mg amino acid **384.** Reaction mixtures were diluted with some methanol and purified via HPLC. Additional examples are disclosed in the following table.

**Table A-25**

| **Cpd no** | From cpd | structure | name | Exact mass | [M+H]⁺ found |
|---|---|---|---|---|---|
| **300** | 384 | 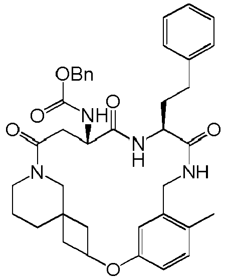 | benzyl ((7S,10S)-3⁴-methyl-6,9,12-trioxo-7-phenethyl-2-oxa-5,8-diaza-13(3,1)-piperidina-3(1,3)-benzena-1(1,3)-cyclobutanatridecaphane-10-yl)carbamate | 652.3 | 653.4 |
| **312** | 398 | 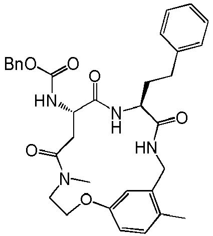 | benzyl ((8S,11S)-1⁴,5-dimethyl-6,9,12-trioxo-11-phenethyl-2-oxa-5,10, 13-triaza-1(1,3)-benzenacyclotetradecaphane-8-yl)carbamate | 586.3 | 587.3 |
| **313** | 408 | 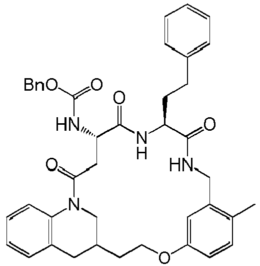 | benzyl ((9S,12S)-5⁴-methyl-8,11,14-trioxo-9-phenethyl-11,12,13,14-tetrahydro-4-oxa-7,10-diaza-1(3,1)-quinolina-5(1,3)-benzenacyclotetradecaphane-12-yl)carbamate | 688.3 | 689.4 |
| **314** | 409 | 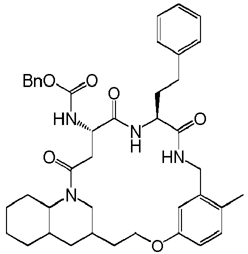 | benzyl ((9S,12S)-5⁴-methyl-8,11,14-trioxo-9-phenethyl-11,12,13,14,14a,15,16,17,1 8,18a-decahydro-4-oxa-7,10-diaza-1(3,1)-quinolina-5(1,3)-benzenacyclotetradecaphane-12-yl)carbamate | 694.4 | 965.4 |
| **315** | 418 | 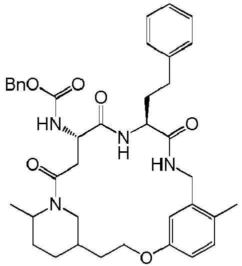 | benzyl ((9S,12S)-1⁶,5⁴-dimethyl-8,11,14-trioxo-9-phenethyl-4-oxa-7,10-diaza-1(3,1)-piperidina-5(1,3)-benzenacyclotetradecaphane-12-yl)carbamate | 654.3 | 655.4 |
| **325** | 428 | 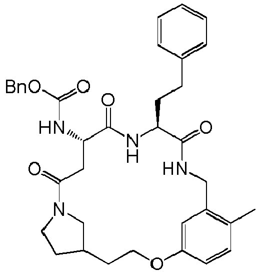 | benzyl ((9S,12S)-5⁴-methyl-8,11,14-trioxo-9-phenethyl-4-oxa-7,10-diaza-1(3,1)-pyrrolidina-5(1,3)-benzenacyclotetradecaphane-12-yl)carbamate | 626.3 | 627.3 |
| **337** | 433 | 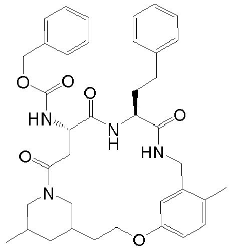 | benzyl ((9S,12S)-1⁵,5⁴-dimethyl-8,11,14-trioxo-9-phenethyl-4-oxa-7,10-diaza-1(3,1)-piperidina-5(1,3)-benzenacyclotetradecaphane-12-yl)carbamate | 654.3 | 655.4 |

### Example A-25: Preparation of macrocyclic compound 211

### tert-butyl 3-(2-(3-cyano-4-fluorophenoxy)ethyl)piperidine-1-carboxylate (576)

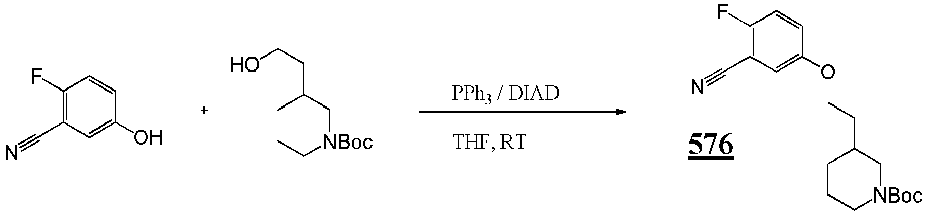

1.03 g tert-butyl 3-(2-hydroxyethyl)piperidine-1-carboxylate, 500mg 2-fluoro-5-hydroxybenzonitrile and 1.17 g PPh₃ were dissolved in THF. 879 µl DIAD in 5 ml THF were added and the mixture was stirred at room temperature for 2h. Saturated NaHCO₃ solution was added and the mixture was extracted with DCM. The organic phase was dried over MgSO₄ and volatiles were removed. Purification was achieved by normal phase column chromatography (silica, cyclohexane/ethyl acetate gradient).

### tert-butyl 3-(2-(3-(aminomethyl)-4-fluorophenoxy)ethyl)piperidine-1-carboxylate (200)

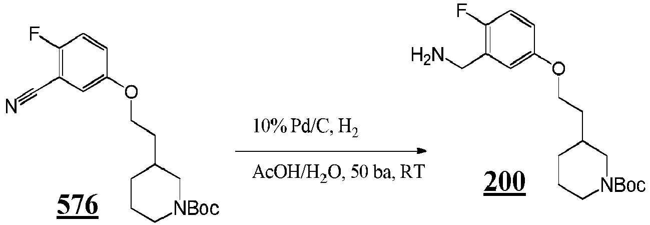

1.02 g nitrile were dissolved in 36 ml acetic acid and 5 ml water. Hydrogenation to amine **200** was achieved with 706 mg 10 % Pd/C under 50 bar hydrogen at room temperature. The mixture was filtered over a pad of celite and washed with MeOH. The solvent was removed under reduced pressure. The residue was dissolved in ethyl acetate and washed with NaHCO₃-solution (3x). Combined organic layers were dried over MgSO₄, filtered off, and concentrated under reduced pressure. The residue was pure enough for the next reactions.
Formula: C₁₉H₂₉FN₂O₃, exact mass: 352.2.3, found: 353.0 [M+H]⁺

### tert-butyl 3-(2-(3-(((S)-2-(((benzvloxv)carbonvl)amino)-4-phenylbutanamido)methyl)-4-fluorophenoxy)ethyl)piperidine-1-carboxylate (201)

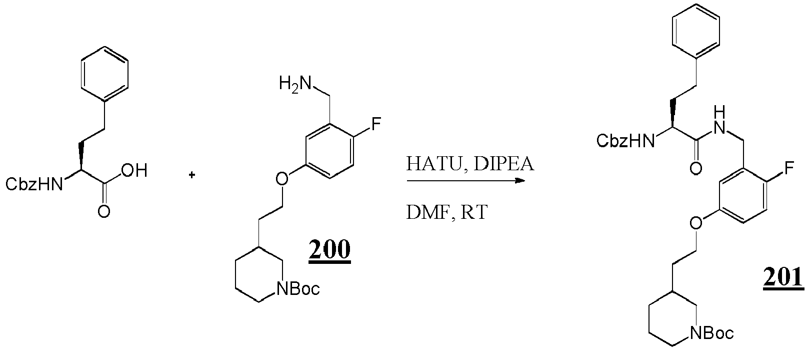

The amide bond was formed according to general procedure B with 615 mg amine **200** and 820 mg Cbz-hPhe-OH. Purification was achieved by normal phase column chromatography (silica, cyclohexane/ethyl acetate gradient).
Formula: C₃₇H₄₆FN₃O₆, exact mass: 647.3, found: 648.6 [M+H]⁺

### tert-butyl 3-(2-(3-(((S)-2-amino-4-phenylbutanamido)methyl)-4-fluorophenoxy)ethyl)piperidine-1-carboxylate (202)

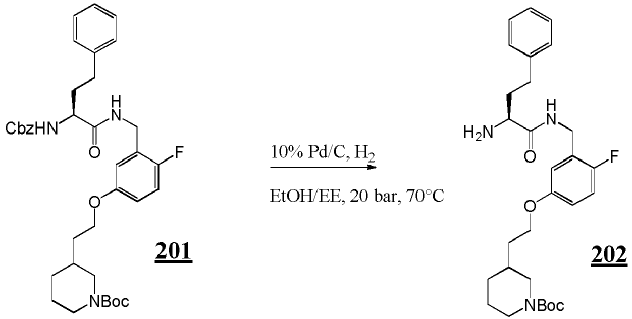

Benzyl carbamate was cleaved according to general procedure C using 10% Pd/C catalyst cartridge, 20 bar at 70 °C in a solvent mixture of ethanol / ethyl acetate. Product was purified by normal phase column chromatography (silica, cyclohexane/ethyl acetate gradient).
Formula: C₂₉H₄₀FN₃O₄, exact mass: 513.3, found: 514.3 [M+H]⁺

### tert-butyl 3-(2-(3-(((S)-2-((S)-2-acetamido-4-(tert-butoxy)-4-oxobutanamido)-4-phenylbutanamido)methyl)-4-fluorophenoxy)ethyl)piperidine-1-carboxylate (203)

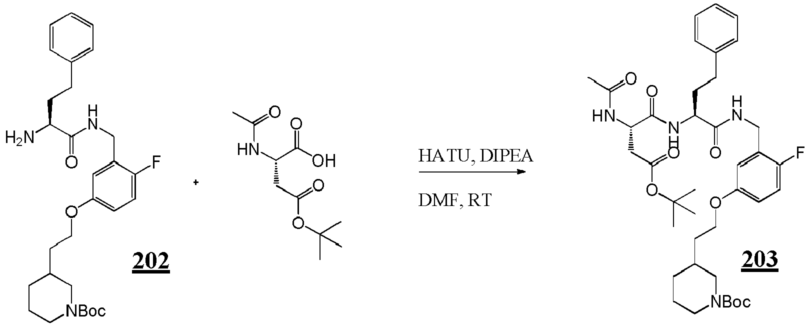

The amide bond was formed according to general procedure B with 150 mg amine **202** and 88 mg Ac-Asp(OtBu)-OH. Purification was achieved by normal phase flash chromatography.
Formula: C₃₉H₅₅FN₄O₈, exact mass: 726.4, found: 727.7 [M+H]⁺

### (3S)-3-acetamido-4-(((2S)-1-((2-fluoro-5-(2-(piperidin-3-yl)ethoxy)benzyl)amino)-1-oxo 4-phenylbutan-2-yl)amino)-4-oxobutanoic acid (204)

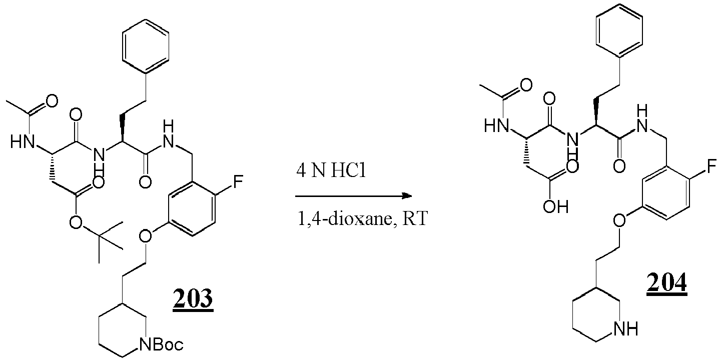

Protecting groups were cleaved from 101 mg compound **203** according to general procedure D stirring compounds in 10 ml 4 N HCl / 1,4-dioxane at room temperature. After deprotection was complete solvent was removed and the crude dissolved in acetonitrile and concentrated twice. Product used without further purification
Formula: C₃₀H₃₉FN₄O₆, exact mass: 570.3, found: 571.4 [M+H]⁺

### N-((9S,12S)-5⁴-fluoro-8,11,14-trioxo-9-phenethyl-4-oxa-7,10-diaza-1(3,1)-piperidina-5(1,3)-benzenacyclotetradecaphane-12-yl)acetamide (205)

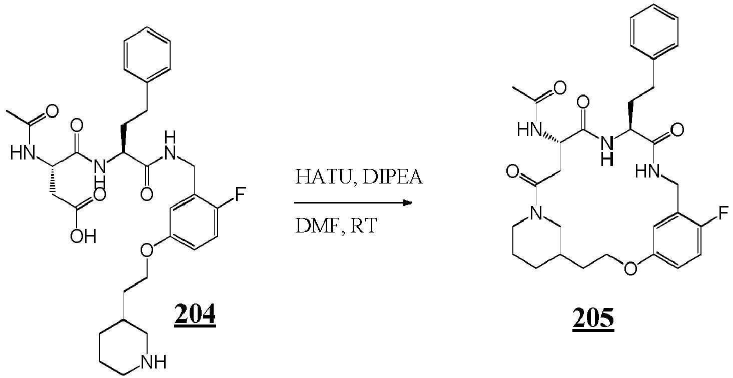

Macrocyclization was achieved according to general procedure A with 1.03 mmol amino acid **204.** Reaction mixtures were diluted with some methanol and purified via HPLC.
Formula: C₃₀H₃₇FN₄O₅, exact mass: 552.3, found: 553.6 [M+H]⁺

### tert-butyl 3-(2-(3-(((S)-2-(((benzyloxy)carbonyl)amino)-4-phenylbutanamido)methyl)-4-methoxhenoxy)ethyl)pieridine-1-carboxylate (207)

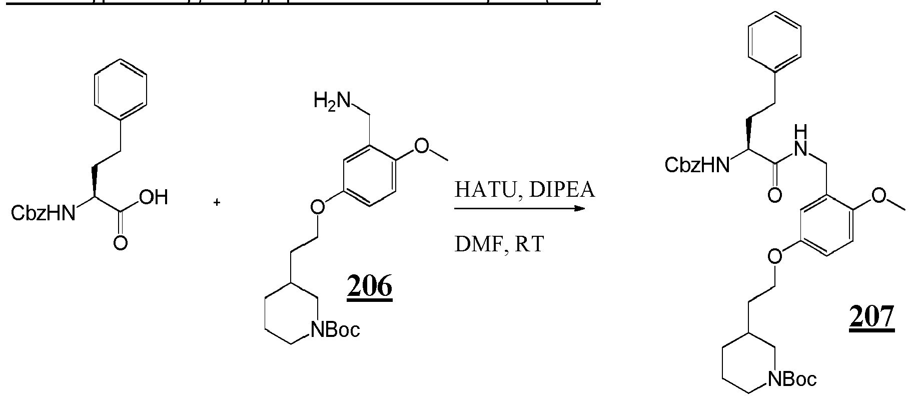

The amide bond was formed according to general procedure B with 648 mg amine **206** and 836 mg Cbz-hPhe-OH. Purification was achieved by normal phase column chromatography (silica, cyclohexane/ethyl acetate gradient).
Formula: C₃₈H₄₉N₃O₇, exact mass: 659.4, found: 660.7 [M+H]⁺

### tert-butyl 3-(2-(3-(((S)-2-amino-4-phenylbutanamido)methyl)-4-methoxyphenoxy)ethyl) piperidine-1-carboxylate (208)

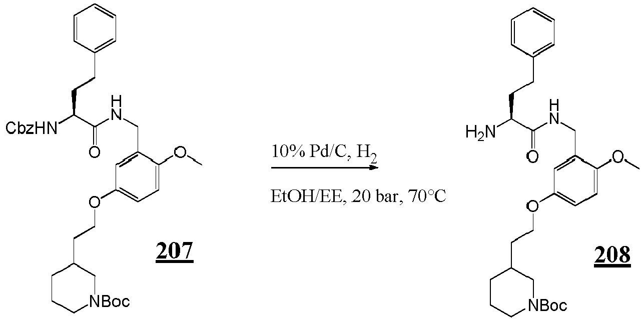

Benzyl carbamate was cleaved according to general procedure C using 10% Pd/C catalyst cartridge, 20 bar at 70 °C in a solvent mixture of ethanol / ethyl acetate. Product was purified by normal phase column chromatography (silica, cyclohexane/ethyl acetate gradient).
Formula: C₃₀H₄₃N₃O₅, exact mass: 525.3, found: 526.5 [M+H]⁺

### tert tert-butyl 3-(2-(3-(((S)-2-((S)-2-acetamido-4-(tert-butoxy)-4-oxobutanamido)-4-phenylbutanamido)methyl)-4-methoxyphenoxy)ethyl)piperidine-1-carboxylate (209)

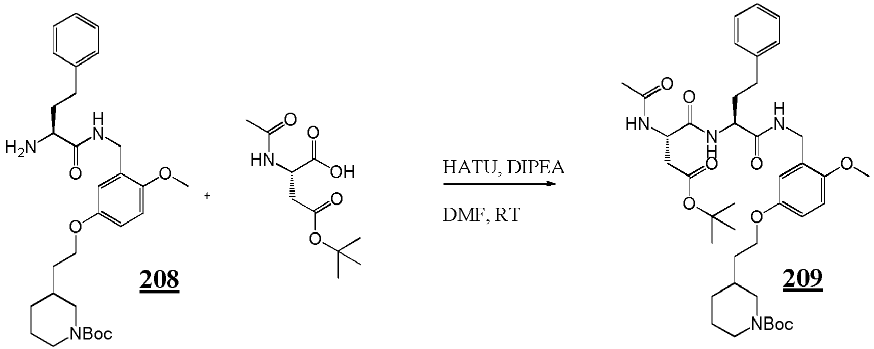

The amide bond was formed according to general procedure B with 150 mg amine **208** and 86 mg Ac-Asp(OtBu)-OH. Purification was achieved by normal phase flash chromatography.
Formula: C₄₀H₅₈N₄O₉, exact mass: 738.4, found: 739.7 [M+H]⁺

### (3S)-3-acetamido-4-(((2S)-1-((2-methoxy-5-(2-(piperidin-3-yl)ethoxy)benzyl)amino)-1-oxo-4-phenylbutan-2-yl)amino)-4-oxobutanoic acid (210)

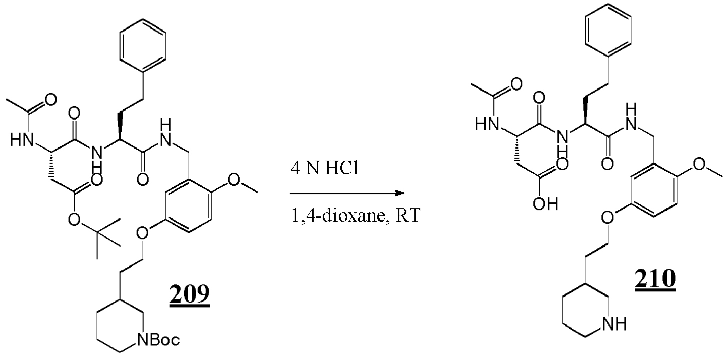

Protecting groups were cleaved from 188 mg compound **209** according to general procedure D stirring compounds in 10 ml 4 N HCl / 1,4-dioxane at room temperature. After deprotection was complete solvent was removed and the crude dissolved in acetonitrile and concentrated twice. Product used without further purification
Formula: C₃₁H₄₂N₄O₇, exact mass: 582.3, found: 583.4 [M+H]⁺

### N-((9S,12S)-5⁴-methoxy-8,11,14-trioxo-9-phenethyl-4-oxa-7,10-diaza-1(3,1)-piperidina-5(1,3)-benzenacyclotetradecaphane-12-yl)acetamide (211)

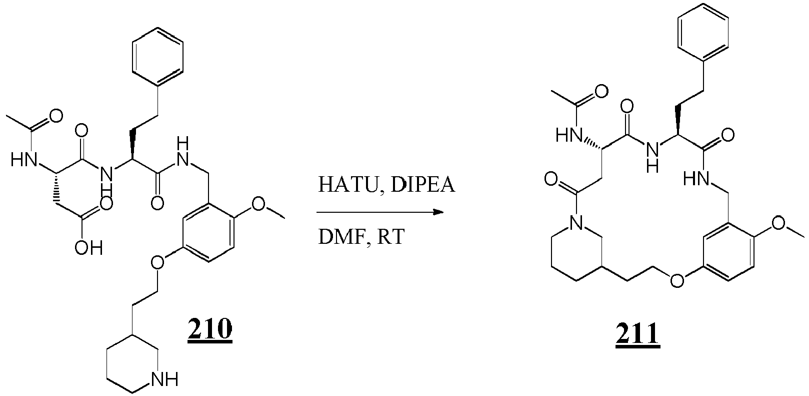

Macrocyclization was achieved according to general procedure A with 0.289 mmol amino acid 210. Reaction mixtures were diluted with some methanol and purified via HPLC.
Formula: C₃₁H₄₀N₄O₆, exact mass: 564.3, found: 565.5 [M+H]⁺

### Example A-26: Preparation of macrocyclic compound 212, 213, 214, and 215

### (9S,12S)-5⁴-methyl-12-amino-8,11,16-trioxo-9-phenethyl-4-oxa-7,10-diaza-1(3,1)-piperidina-5(1,3)-benzenacyclohexadecaphane (212)

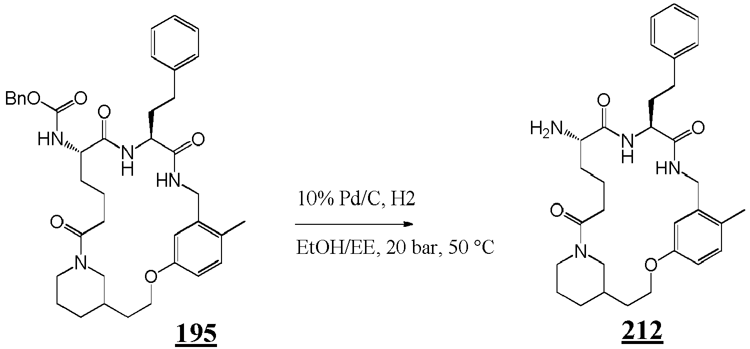

Benzyl carbamate was cleaved according to general procedure C using 10% Pd/C catalyst cartridge, 20 bar at 70 °C in a solvent mixture of ethanol / ethyl acetate. Product was purified by normal phase column chromatography (silica, cyclohexane/ethyl acetate gradient).
Formula: C₃₁H₄₂N₄O₄, exact mass: 534.3, found: 535.6 [M+H]⁺

### N-((9S,12S)-5⁴-methyl-8,11,16-trioxo-9-phenethyl-4-oxa-7,10-diaza-1(3,1)-piperidina-5(1,3)-benzenacyclohexadecaphane-12-yl)acetamide (213)

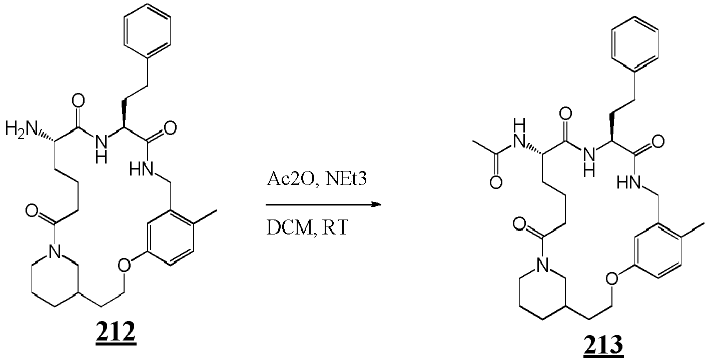

50 mg amine **212** and 54 µL NEt₃ were dissolved DCM and 14 µl acetic anhydride were added. After complete reaction solvent was removed and the residue dissolved in some methanol. Purification via HPLC.
Formula: C₃₃H₄₄N₄O₅, exact mass: 576.3, found: 577.6 [M+H]⁺

### (9S,12S)-12-amino-5⁴-methyl-8,11,15-trioxo-9-phenethyl-4-oxa-7,10-diaza-1(3,1)-piperidina-5(1,3)-benzenacyclopentadecaphane) (214)

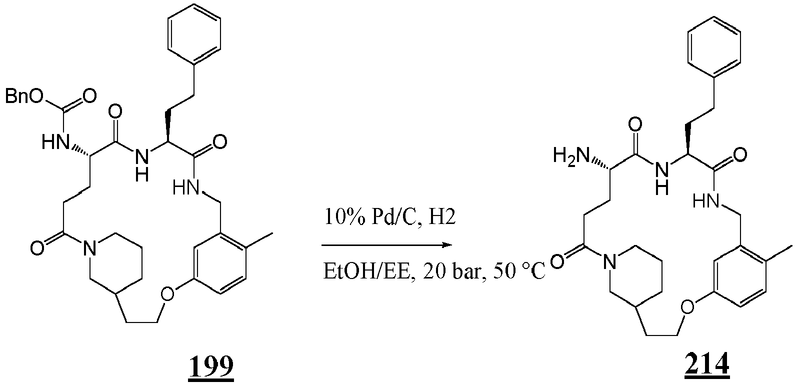

Benzyl carbamate was cleaved from compound **199** according to general procedure C using 10% Pd/C catalyst cartridge, 20 bar at 70 °C in a solvent mixture of ethanol / ethyl acetate. Product was purified by normal phase column chromatography (silica, cyclohexane/ethyl acetate gradient).
Formula: C₃₀H₄₀N₄O₄, exact mass: 520.3, found: 521.5 [M+H]⁺

### N-((9S,12S)-5⁴-methyl-8,11,15-trioxo-9-phenethyl-4-oxa-7,10-diaza-1(3,1)-piperidina-5(1,3)-benzenacyclopentadecaphane-12-yl)acetamide (215)

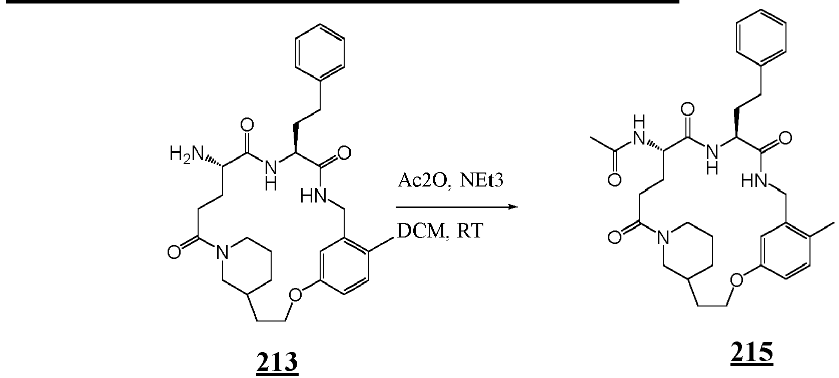

50 mg amine **213** and 54 µL NEt₃ were dissolved DCM and 14 µl acetic anhydride were added. After complete reaction solvent was removed and the residue dissolved in some methanol. Purification via HPLC.
Formula: C₃₂H₄₂N₄O₅, exact mass: 562.3, found: 563.5 [M+H]⁺

### Example A-27: Preparation of macrocyclic compounds 218, 220, and 293

### 2,5,8,11,14,17,20-heptaoxadocosan-22-yl ((9R,12R)-5⁴-methyl-8,11,14-trioxo-9-phenethyl-4-oxa-7,10-diaza-1(3,1)-piperidina-5(1,3)-benzenacyclotetradecaphane-12-yl)carbamate (218)

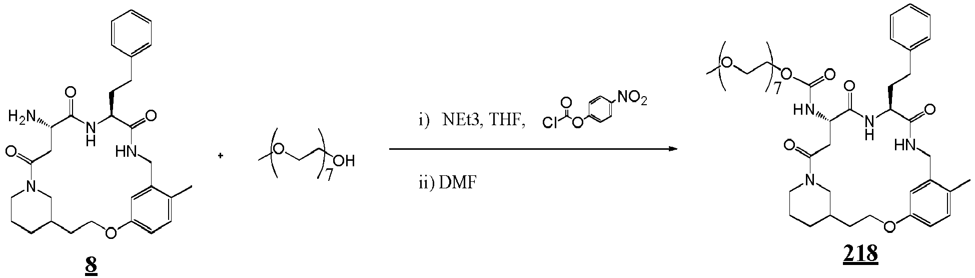

20 mg 2,5,8,11,14,17,20-heptaoxadocosan-22-ol and 24.7 µl trimethylamine in THF were cooled to 0°C. 4-Nitrophenyl chloroformate (14.2 mg) were added and the mixture was allowed to warm to room temperature. Volatiles were removed under reduced pressure and the residue dissolved in 1 ml DMF. 30 mg amine 8 were added together with 62 µl DIPEA at room temperature. After consumption of compound 8 the reaction mixture was purified by reversed phase HPLC.
Formula: C₄₅H₆₈N₄O₁₃, exact mass: 872.5, found: 873.9 [M+H]⁺

### 2-(2-(2-methoxyethoxy)ethoxy)ethyl ((9R,12R)-5⁴-methyl-8,11,14-trioxo-9-phenethyl-4-oxa-7,10-diaza-1(3,1)-piperidina-5(1,3)-benzenacyclotetradecaphane-12-yl)carbamate (220)

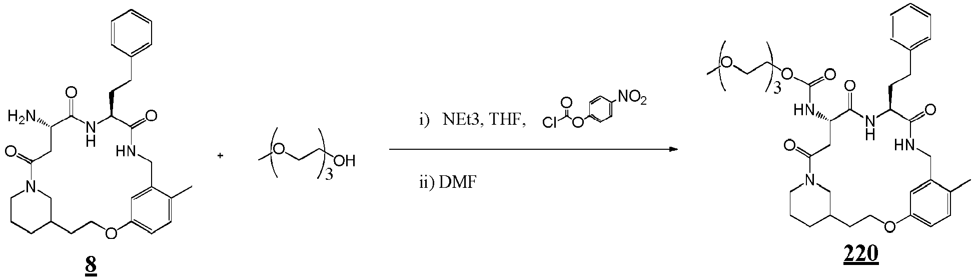

50 mg 2-(2-(2-methoxyethoxy)ethoxy)ethanol and 213 µl trimethylamine in THF were cooled to 0°C. 4-Nitrophenyl chloroformate (92 mg) were added and the mixture was allowed to warm to room temperature. Volatiles were removed under reduced pressure and the residue dissolved in 1 ml DMF. 103 mg amine **8** were added together with 212 µl DIPEA at room temperature. After consumption of compound **8** the reaction mixture was purified by reversed phase HPLC.
Formula: C₃₇H₅₂N₄O₉, exact mass: 696.4, found: 697.5 [M+H]⁺

### (3-methyloxetan-3-vl)methl ((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamate (293)

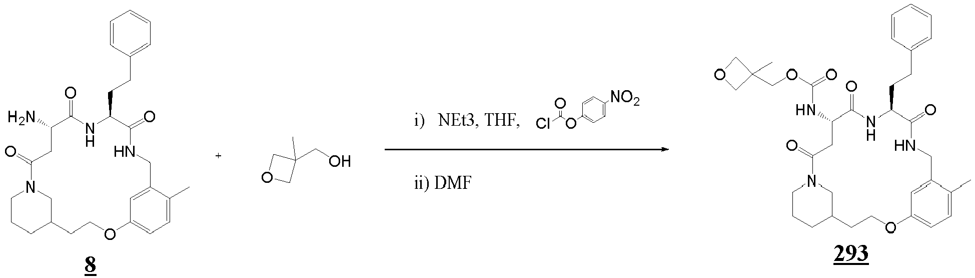

50 (3-methyloxetan-3-yl)methanol and 341 µl trimethylamine in THF were cooled to 0°C. 4-Nitrophenyl chloroformate (148 mg) were added and the mixture was allowed to warm to room temperature. Volatiles were removed under reduced pressure and the residue dissolved in 1 ml DMF. 124 mg amine **8** were added together with 742 µl DIPEA at room temperature. After consumption of compound **8** the reaction mixture was purified by reversed phase HPLC.
Formula: C₃₅H₄₆N₄O₇, exact mass: 634.3, found: 635.4 [M+H]⁺

### Example A-28: Preparation of macrocyclic compounds 252 -261, 296 and 298

### (S)-tert-butyl 3-acetamido-4-(((S)-1-((5-(2-((tert-butoxycarbonyl)amino)ethoxy)-2-methylbenzyl)amino)-1-oxo-4-phenylbutan-2-yl)amino)-4-oxobutanoate (223)

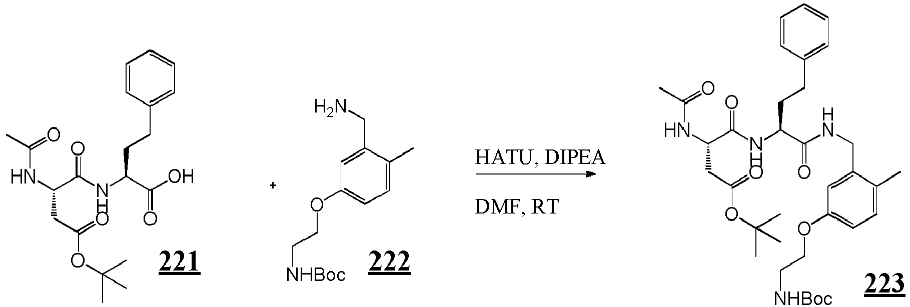

Amide couplings between benzylamine derivatives and dipeptide **221** were accomplished according to general procedure B, typically using 100 mg dipeptide **221.** As an example compound **223** was synthesized from 100 mg dipeptide **221,** 93 mg amine **222,** 116 mg HATU and 266 µl DIPEA in DMF at room temperature. Purification was achieved by normal phase column chromatography (silica, cyclohexane/ethyl acetate gradient). Additional examples are disclosed in the following table.

**Table A-26**

| **Cpd no** | From amine | structure | name | Exact mass | [M+H]⁺ found |
|---|---|---|---|---|---|
| **223** | **222** | 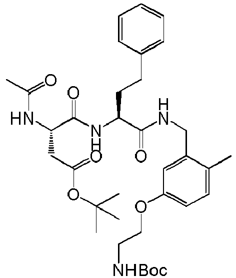 | (S)-tert-butyl 3-acetamido-4-(((S)-1-((5-(2-((tert-butoxycarbonyl)amino)ethoxy )-2-methylbenzyl)amino)-1-oxo-4-phenylbutan-2-yl)amino)-4-oxobutanoate | 654.4 | 655.7 |
| **233** | **224** | 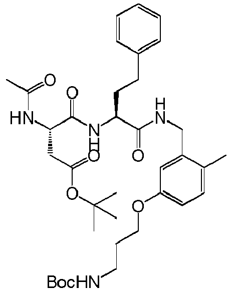 | (S)-tert-butyl 3-acetamido-4-(((S)-1-((5-(3-((tert-butoxycarbonyl)amino)propox y)-2-methylbenzyl)amino)-1-oxo-4-phenylbutan-2-yl)amino)-4-oxobutanoate | 668.4 | 669.7 |
| **234** | **225** | 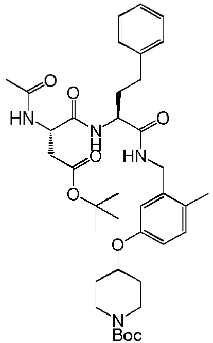 | tert-butyl 4-(3-(((S)-2-((S)-2-acetamido-4-(tert-butoxy)-4-oxobutanamido)-4-phenylbutanamido)methyl)-4-methylphenoxy)piperidine-1-carboxylate | 694.4 | 695.7 |
| **235** | **226** | 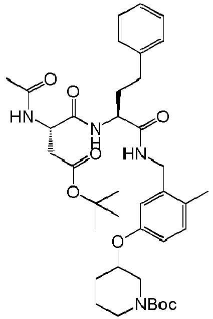 | tert-butyl 3-(3-(((S)-2-((S)-2-acetamido-4-(tert-butoxy)-4-oxobutanamido)-4-phenylbutanamido)methyl)-4-methylphenoxy)piperidine-1-carboxylate | 694.4 | 695.7 |
| **236** | **227** | 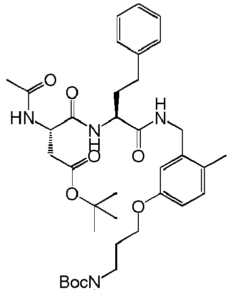 | (S)-tert-butyl 3-acetamido-4-(((S)-1-((5-(3-((tert-butoxycarbonyl)(methyl)amino)propoxy)-2-methylbenzyl)amino)-1-oxo-4-phenylbutan-2-yl)amino)-4-oxobutanoate | 682.4 | 683.5 |
| **237** | **228** | 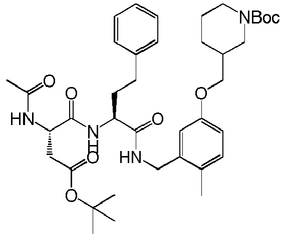 | tert-butyl 3-((3-(((S)-2-((S)-2-acetamido-4-(tert-butoxy)-4-oxobutanamido)-4-phenylbutanamido)methyl)-4-methylphenoxy)methyl)piperidine-1-carboxylate | 708.4 | 709.7 |
| **238** | **141** | 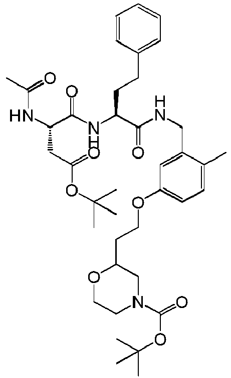 | tert-butyl 2-(2-(3-(((S)-2-((S)-2-acetamido-4-(tert-butoxy)-4-oxobutanamido)-4-phenylbutanamido)methyl)-4-methylphenoxy)ethyl)morpholine-4-carboxylate | 724.4 | 725.7 |
| **239** | **230** | 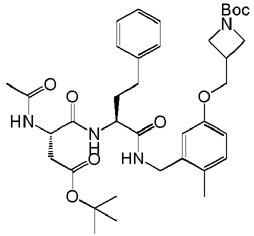 | tert-butyl 3-((3-(((S)-2-((S)-2-acetamido-4-(tert-butoxy)-4-oxobutanamido)-4-phenylbutanamido)methyl)-4-methylphenoxy) methyl)azetid ine-1-carboxylate | 680.4 | 681.7 |
| **240** | **231** | 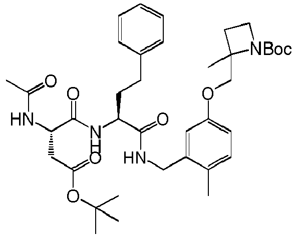 | tert-butyl 2-((3-(((S)-2-((S)-2-acetamido-4-(tert-butoxy)-4-oxobutanamido)-4-phenylbutanamido)methyl)-4-methylphenoxy)methyl)-2-methylazetidine-1-carboxylate | 694.4 | 695.7 |
| **241** | **232** | 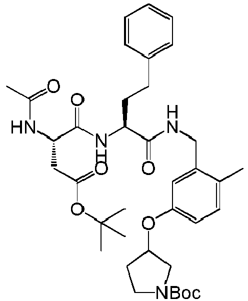 | tert-butyl 3-(3-(((S)-2-((S)-2-acetamido-4-(tert-butoxy)-4-oxobutanamido)-4-phenylbutanamido)methyl)-4-methylphenoxy)pyrrolidine-1-carboxylate | 680.4 | 681.7 |
| **376** | **270** | 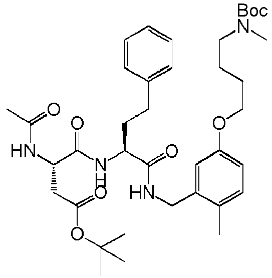 | (S)-tert-butyl 3-acetamido-4-(((S)-1-((5-(4-((tert-butoxycarbonyl)(methyl)amino)butoxy)-2-methylbenzyl)amino)-1-oxo-4-phenylbutan-2-yl)amino)-4-oxobutanoate | 568.3 | 569.4 |
| **378** | **188** | 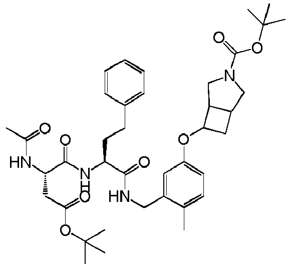 | tert-butyl 6-(3-(((S)-2-((S)-2-acetamido-4-(tert-butoxy)-4-oxobutanamido)-4-phenylbutanamido)methyl)-4-methylphenoxy)-3-azabicyclo[3.2.0]heptane-3-carboxylate | 540.3 | 541.3 |

### (S)-3-acetamido-4-(((S)-1-((5-(2-aminoethoxy)-2-methylbenzyl)amino)-1-oxo-4-phenylbutan-2-yl)amino)-4-oxobutanoic acid (242)

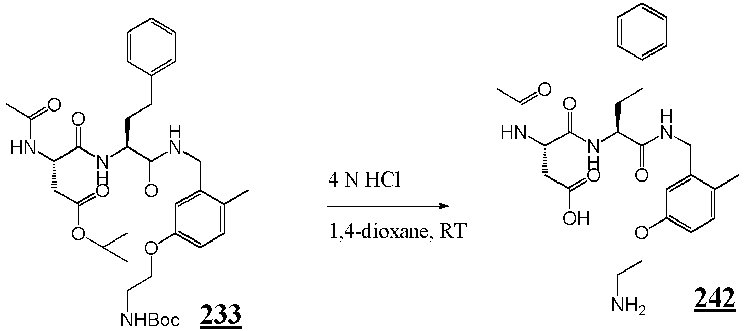

Protecting groups were cleaved from 65 mg compound **233** according to general procedure D stirring compounds in 10 ml 4 N HCl / 1,4-dioxane at room temperature. After deprotection was complete solvent was removed and the crude dissolved in acetonitrile and concentrated twice. Product used without further purification.

As exemplified by compound **242** additional examples disclosed in the following table were prepared.

**Table A-27**

| **Cpd no** | From cpd | structure | name | Exact mass | [M+H]⁺ found |
|---|---|---|---|---|---|
| **242** | **223** | 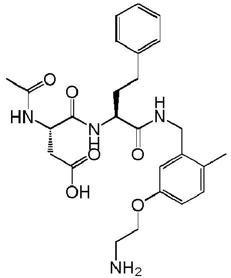 | (S)-3-acetamido-4-(((S)-1-((5-(2-aminoethoxy)-2-methylbenzyl)amino)-1-oxo-4-phenylbutan-2-yl)amino)-4-oxobutanoic acid | 498.2 | 499.2 |
| **243** | **233** | 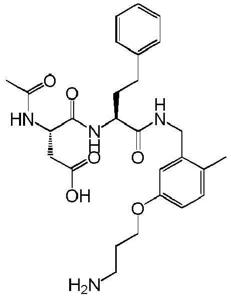 | (S)-3-acetamido-4-(((S)-1-((5-(3-aminopropoxy)-2-methylbenzyl)amino)-1-oxo-4-phenylbutan-2-yl)amino)-4-oxobutanoic acid | 512.3 | 513.4 |
| **244** | **234** | 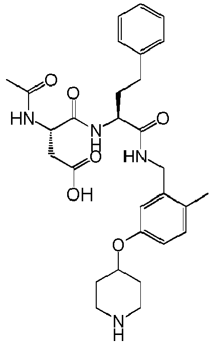 | (S)-3-acetamido-4-(((S)-1-((2-methyl-5-(piperidin-4-yloxy)benzyl)amino)-1-oxo-4-phenylbutan-2-yl)amino)-4-oxobutanoic acid | 538.3 | 539.3 |
| **245** | **235** | 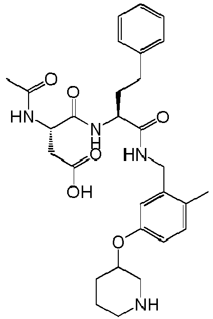 | (3S)-3-acetamido-4-(((2S)-1-((2-methyl-5-(piperidin-3-yloxy)benzyl)amino)-1-oxo-4-phenylbutan-2-yl)amino)-4-oxobutanoic acid | 538.3 | 539.4 |
| **246** | **236** | 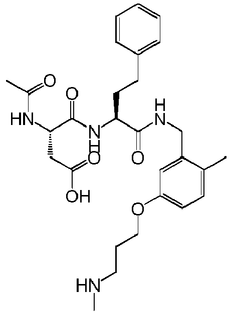 | (S)-3-acetamido-4-(((S)-1-((2-methyl-5-(3-(methylamino)propoxy)benzyl)amino)-1-oxo-4-phenylbutan-2-yl)amino)-4-oxobutanoic acid | 526.3 | 527.4 |
| **247** | **237** | 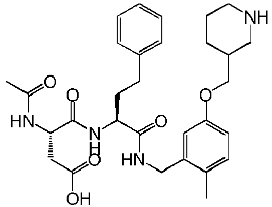 | (3S)-3-acetamido-4-(((2S)-1-((2-methyl-5-(piperidin-3-ylmethoxy)benzyl)amino)-1-oxo-4-phenylbutan-2-yl)amino)-4-oxobutanoic acid | 552.3 | 553.5 |
| **248** | **238** | 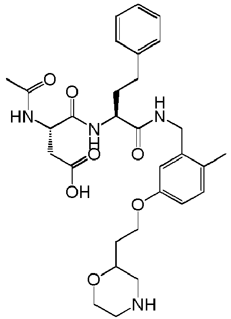 | (3S)-3-acetamido-4-(((2S)-1-((2-methyl-5-(2-(morpholin-2-yl)ethoxy)benzyl)amino)-1-oxo-4-phenylbutan-2-yl)amino)-4-oxobutanoic acid | 568.3 | 569.7 |
| **249** | **239** | 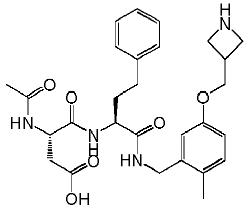 | (S)-3-acetamido-4-(((S)-1-((5-(azetidin-3-ylmethoxy)-2-methylbenzyl)amino)-1-oxo-4-phenylbutan-2-yl)amino)-4-oxobutanoic acid | 524.3 | 525.5 |
| **250** | **240** | 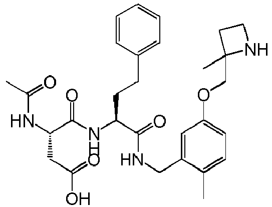 | (3S)-3-acetamido-4-(((2S)-1-((2-methyl-5-((2-methylazetidin-2-yl)methoxy)benzyl)amino)-1-oxo-4-phenylbutan-2-yl)amino)-4-oxobutanoic acid | 538.3 | 539.5 |
| **251** | **241** | 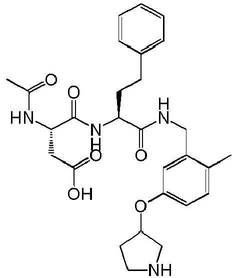 | (3S)-3-acetamido-4-(((2S)-1-((2-methyl-5-(pyrrolidin-3-yloxy)benzyl)amino)-1-oxo-4-phenylbutan-2-yl)amino)-4-oxobutanoic acid | 524.3 | 525.3 |
| **377** | **376** | 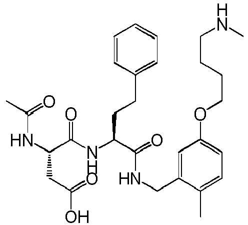 | (S)-3-acetamido-4-(((S)-1-((2-methyl-5-(4-(methylamino)butoxy)benzyl)amino)-1-oxo-4-phenylbutan-2-yl)amino)-4-oxobutanoic acid | 540.3 | 541.3 |
| **379** | **378** | 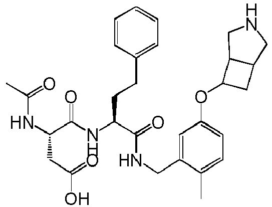 | (3S)-4-(((2S)-1-((5-(3-azabicyclo[3.2.0]heptan-6-yloxy)-2-methylbenzyl)amino)-1-oxo-4-phenylbutan-2-yl)amino)-3-acetamido-4-oxobutanoic acid | 550.3 | - |

### N-((8S,11S)-1⁴-methyl-6,9,12-trioxo-11-phenethyl-2-oxa-5,10,13-triaza-1(1,3)-benzenacyclotetradecaphane-8-yl)acetamide (252)

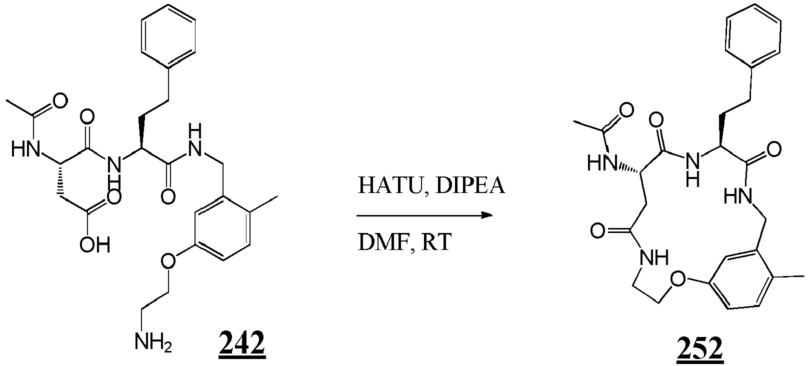

Macrocyclization was achieved according to general procedure A with 0.248 mmol amino acid **242.** Reaction mixtures were diluted with some methanol and purified via HPLC. As exemplified by compound **252** additional examples disclosed in the following table were prepared.

**Table A-28**

| **Cpd no** | From cpd | structure | name | Exact mass | [M+H]⁺ found |
|---|---|---|---|---|---|
| **252** | 242 | 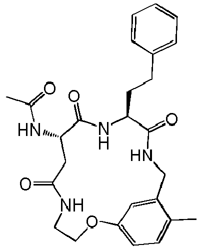 | N-((8S,11S)-1⁴-methyl-6,9, 12-trioxo-11-phenethyl-2-oxa-5,10,13-triaza-1(1,3)-benzenacyclotetradecaphane-8-yl)acetamide | 480.2 | 481.4 |
| | | | | C₂₆H₃₂ N₄O₅ | |
| **253** | 243 | 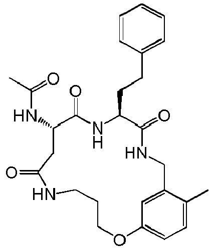 | N-((9S,12S)-1⁴-methyl-7,10,13-trioxo-12-phenethyl-2-oxa-6,11, 14-triaza-1(1,3)-benzenacyclopentadecaphane -9-yl)acetamide | 494.3 | 495.4 |
| | | | | C₂₇H₃₄ N₄O₅ | |
| **254** | 244 | 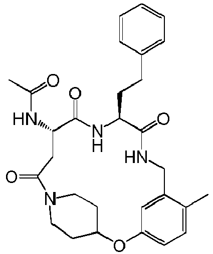 | N-((7S,10S)-3⁴-methyl-6,9,12-trioxo-7-phenethyl-2-oxa-5,8-diaza-1(4,1)-piperidina-3(1,3)-benzenacyclododecaphane-10-yl)acetamide | 520.3 | 521.4 |
| | | | | C₂₉H₃₆ N₄O₅ | |
| **255** | 245 | 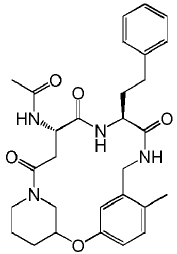 | N-((7S,10S)-3⁴-methyl-6,9,12-trioxo-7-phenethyl-2-oxa-5,8-diaza-1(3,1)-piperidina-3(1,3)-benzenacyclododecaphane-10-yl)acetamide | 520.3 | 521.4 |
| | | | | C₂₉H₃₆ N₄O₅ | |
| **256** | 246 | 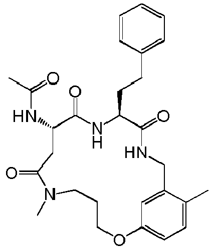 | N-((9S,12S)-1⁴,6-dimethyl-7,10,13-trioxo-12-phenethyl-2-oxa-6,11,14-triaza-1(1,3)-benzenacyclopentadecaphane -9-yl)acetamide | 508.3 | 509.4 |
| | | | | C₂₈H₃₆ N₄O₅ | |
| **257** | 247 | 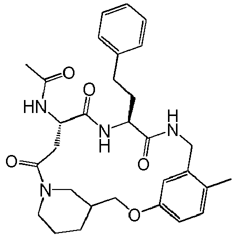 | N-((8S,11S)-4⁴-methyl-7,10,13-trioxo-8-phenethyl-3-oxa-6,9-diaza-1(3,1)-piperidina-4(1,3)-benzenacyclotridecaphane-11-yl)acetamide | 534.3 | 535.4 |
| | | | | C₃₀H₃₈ N₄O₅ | |
| **258** | 248 | 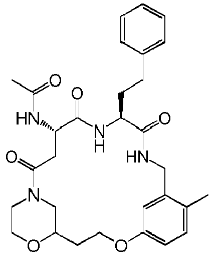 | N-((98,128)-5⁴-methyl-8,11,14-trioxo-9-phenethyl-4-oxa-7,10-diaza-1(2,4)-morpholina-5(1,3)-benzenacyclotetradecaphane-12-yl)acetamide | 550.3 | 551.4 |
| | | | | C₃₀H₃₈ N₄O₆ | |
| **259** | 249 | 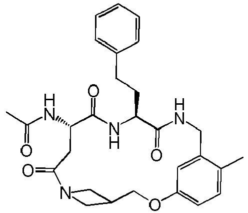 | N-((8S,11S)-4⁴-methyl-7,10,13-trioxo-8-phenethyl-3-oxa-6,9-diaza-1(3,1)-azetidina-4(1,3)-benzenacyclotridecaphane-11-yl)acetamide | 506.3 | 507.4 |
| | | | | C₂₈H₃₄ N₄O₅ | |
| **260** | 250 | 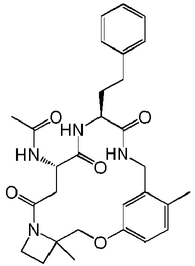 | N-((8S,11S)-1²,4⁴-dimethyl-7,10,13-trioxo-8-phenethyl-3-oxa-6,9-diaza-1(2,1)-azetidina-4(1,3)-benzenacyclotridecaphane-11-yl)acetamide | 520.3 | 521.4 |
| | | | | C₂₉H₃₆ N₄O₅ | |
| **261** | 251 | 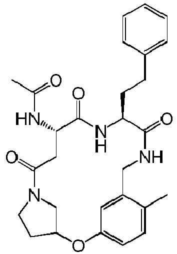 | N-((7S,10S)-3⁴-methyl-6,9,12-trioxo-7-phenethyl-2-oxa-5,8-diaza-1(3,1)-pyrrolidina-3(1,3)-benzenacyclododecaphane-10-yl)acetamide | 506.3 | 507.4 |
| | | | | C₂₈H₃₄ N₄O₅ | |
| **296** | 377 | 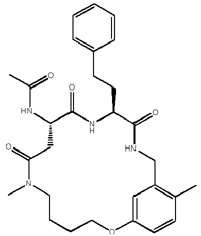 | N-(1⁴,7-dimethyl-8,11,14-trioxo-13-phenethyl-2-oxa-7,12,15-triaza-1(1,3)-benzenacyclohexadecaphane-10-yl)acetamide | 522.3 | 523.4 |
| | | | | C₂₉H₃₈ N₄O₅ | |
| **298** | 379 | 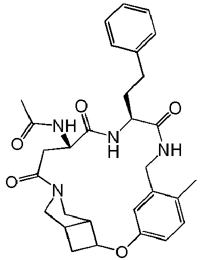 | N-(3⁴-methyl-6,9,12-trioxo-7-phenethyl-2-oxa-13,5,8-triaza-1(6,3)-bicyclo[3.2.0]heptana-3(1,3)-benzenacyclododecaphane-10-yl)acetamide | 532.3 | 533.3 |
| | | | | C₃₀H₃₆ N₄O₅ | |

### Example A-29: Preparation of macrocyclic compounds 265, 282 and 299

### tert-butyl 2-((3-(((S)-2-((S)-2-acetamido-5-(tert-butoxy)-5-oxopentanamido)-4-phenylbutanamido)methyl)-4-methylphenoxy)methyl)-2-methylazetidine-1-carboxylate (263)

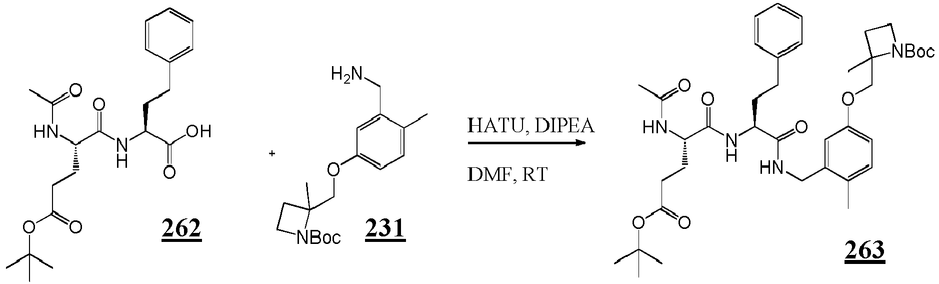

Amide couplings between benzylamine derivatives and dipeptide **262** were accomplished according to general procedure B, typically using 100 mg dipeptide 262. As an example compound **263** was synthesized from 112 mg dipeptide **262,** 115 mg amine **231,** 125 mg HATU and 214 µl DIPEA in DMF at room temperature. Purification was achieved by normal phase column chromatography (silica, cyclohexane/ethyl acetate gradient). Additional examples are disclosed in the following table.

**Table A-29**

| **Cpd no** | From amine | structure | name | Exactm mass. | [M+H]⁺ found |
|---|---|---|---|---|---|
| **263** | **231** | 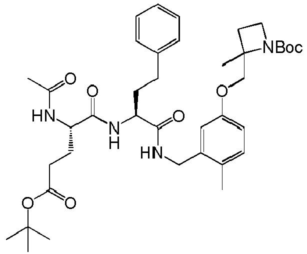 | tert-butyl 2-((3-(((S)-2-((S)-2-acetamido-5-(tert-butoxy)-5-oxopentanamido)-4-phenylbutanamido)methyl)-4-methylphenoxy)methyl)-2-methylazetidine-1-carboxylate | 708.4 | 709.7 |
| **274** | **270** | 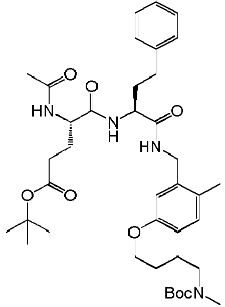 | (S)-tert-butyl 4-acetamido-5-(((S)-1-((5-(4-((tert-butoxycarbonyl)(methyl)amino)butoxy)-2-methylbenzyl)amino)-1-oxo-4-phenylbutan-2-yl)amino)-5-oxopentanoate | 710.4 | 711.5 |
| **380** | **188** | 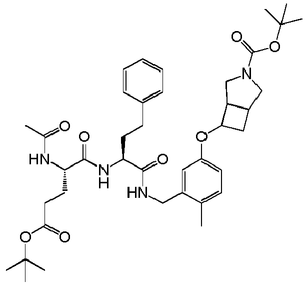 | tert-butyl 6-(3-(((S)-2-((S)-2-acetamido-5-(tert-butoxy)-5-oxopentanamido)-4-phenylbutanamido)methyl)-4-methylphenoxy)-3-azabicyclo[3.2.0]heptane-3-carboxylate | 720.4 | 721.5 |

### (4S)-4-acetamido-5-(((2S)-1-((2-methyl-5-((2-methylazetidin-2-yl)methoxy)benzyl)amino)-1-oxo-4-phenylbutan-2-yl)amino)-5-oxopentanoic acid (264)

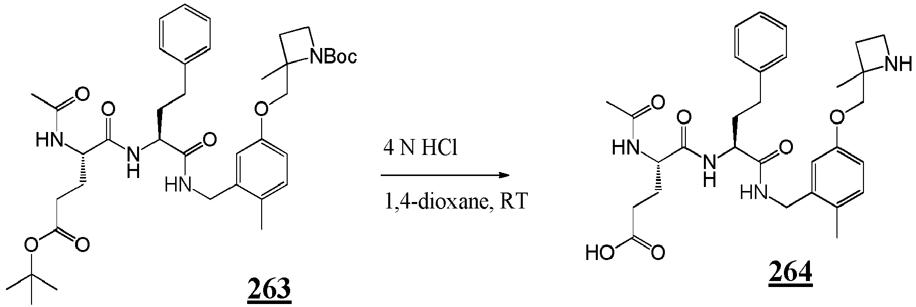

Protecting groups were cleaved from 117 mg compound **263** according to general procedure D stirring compounds in 10 ml 4 N HCl / 1,4-dioxane at room temperature. After deprotection was complete solvent was removed and the crude dissolved in acetonitrile and concentrated twice. Product used without further purification.

As exemplified by compound 264 additional examples disclosed in the following table were prepared.

**Table A-30**

| **Cpd no** | From cpd | structure | name | Exact mass | [M+H]⁺ found |
|---|---|---|---|---|---|
| **264** | **263** | 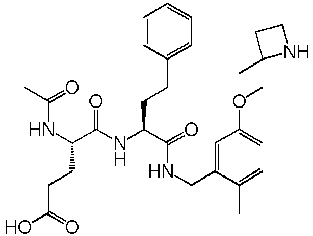 | (4S)-4-acetamido-5-(((2S)-1-((2-methyl-5-((2-methylazetidin-2-yl)methoxy)benzyl)amino)-1-oxo-4-phenylbutan-2-yl)amino)-5-oxopentanoic acid | 552.3 | 553.4 |
| **278** | **274** | 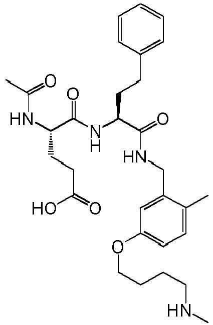 | (S)-4-acetamido-5-(((S)-1-((2-methyl-5-(4-(methylamino)butoxy)benzyl)amino)-1-oxo-4-phenylbutan-2-yl)amino)-5-oxopentanoic acid | 554.3 | 555.4 |
| **381** | **380** | 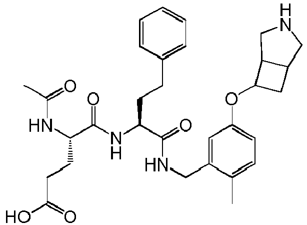 | (4S)-5-(((2S)-1-((5-(3-azabicyclo[3.2.0]heptan-6-yloxy)-2-methylbenzyl)amino)-1-oxo-4-phenylbutan-2-yl)amino)-4-acetamido-5-oxopentanoic acid | 564.3 | 565.4 |

### N-((8S,11S)-1²,4⁴-dimethyl-7,10,14-trioxo-8-phenethyl-3-oxa-6,9-diaza-1(2,1)-azetidina-4(1,3)-benzenacyclotetradecaphane-11-yl)acetamide(265)

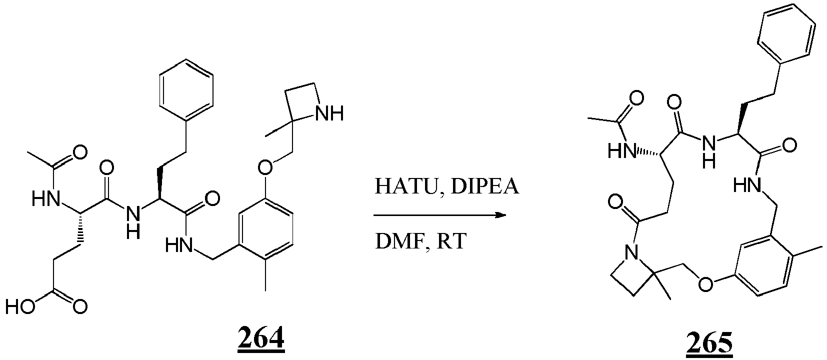

Macrocyclization was achieved according to general procedure A with 0.165 mmol amino acid **264.** Reaction mixtures were diluted with some methanol and purified via HPLC. As exemplified by compound **265** additional examples disclosed in the following table were prepared.

**Table A-31**

| **Cpd no** | From cpd | structure | name | Exact mass | [M+H]⁺ found |
|---|---|---|---|---|---|
| **265** | **264** | 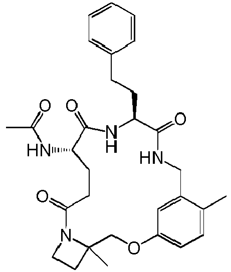 | N-((8S,11S)-1²,4⁴-dimethyl-7,10,14-trioxo-8-phenethyl-3-oxa-6,9-diaza-1(2,1)-azetidina-4(1,3)-benzenacyclotetradecaphane-11-yl)acetamide | 534.3 | 535.4 |
| | | | | C₃₀H₃₈ N₄O₅ | |
| **282** | **278** | 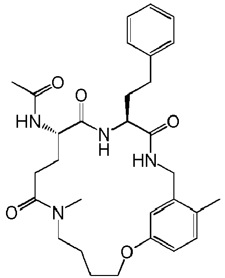 | N-((11S,14S)-1⁴,7-dimethyl-8,12,15-trioxo-14-phenethyl-2-oxa-7,13,16-triaza-1(1,3)-benzenacycloheptadecaphane-11-yl)acetamide | 536.3 | 537.3 |
| | | | | C₃₀H₄₀ N₄O₅ | |
| **299** | **381** | 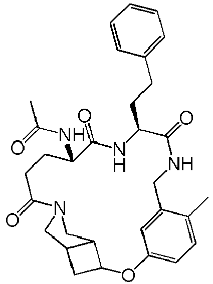 | N-(3⁴-methyl-6,9,13-trioxo-7-phenethyl-2-oxa-13,5,8-triaza-1(6,3)-bicyclo[3.2.0]heptana-3(1,3)-benzenacyclotridecaphane-10-yl)acetamide | 546.3 | 547.3 |
| | | | | C₃₁H₃₈ N₄O₅ | |

### Example A-30: Preparation of macrocyclic compounds 269, 280, 281 and 29

### (S)-tert-butyl 5-acetamido-6-(((S)-1-((5-(4-((tert-butoxycarbonyl)amino)butoxy)-2-methylbenzyl)amino)-1-oxo-4-phenylbutan-2-vl)amino)-6-oxohexanoate (267)

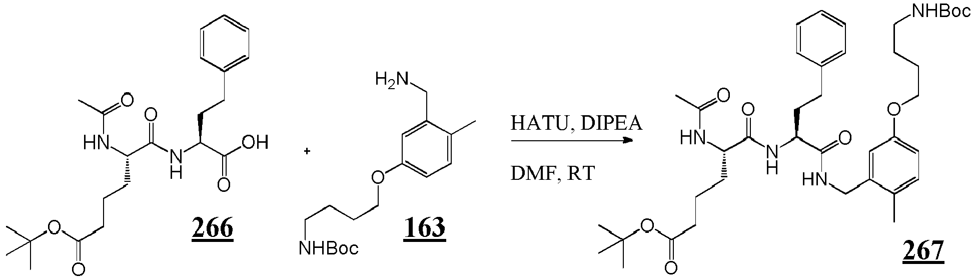

Amide couplings between benzylamine derivatives and dipeptide **266** were accomplished according to general procedure B, typically using 100 mg dipeptide **262.** As an example compound **263** was synthesized from 113 mg dipeptide **266,** 107 mg amine **163,** 122 mg HATU and 280 µl DIPEA in DMF at room temperature. Purification was achieved by normal phase column chromatography (silica, cyclohexane/ethyl acetate gradient). Additional examples are disclosed in the following table.

**Table A-32 n**

| **Cpd no** | From amine | structure | name | Exact mass | [M+H]⁺ found |
|---|---|---|---|---|---|
| **267** | **163** | 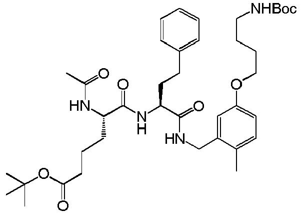 | (S)-tert-butyl 5-acetamido-6-(((S)-1-((5-(4-((tert-butoxycarbonyl)amino)butoxy)-2-methylbenzyl)amino)-1-oxo-4-phenylbutan-2-yl)amino)-6-oxohexanoate | 710.4 | 710.7 |
| **272** | **227** | 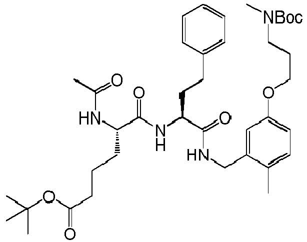 | (S)-tert-butyl 5-acetamido-6-(((S)-1-((5-(3-((tert-butoxycarbonyl)(methyl)amino)propoxy)-2-methylbenzyl)amino)-1-oxo-4-phenylbutan-2-yl)amino)-6-oxohexanoate | 722.4 | 723.8 |
| **273** | **232** | 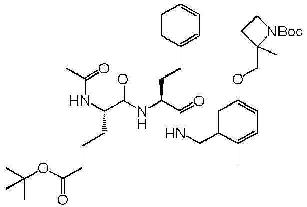 | tert-butyl 2-((3-(((S)-2-((S)-2-acetamido-6-(tert-butoxy)-6-oxohexanamido)-4-phenylbutanamido)methyl)-4-methylphenoxy)methyl)-2-methylazetidine-1-carboxylate | 710.4 | 711.5 |
| **374** | **270** | 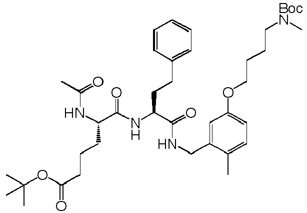 | (S)-tert-butyl 5-acetamido-6-(((S)-1-((5-(4-((tert-butoxycarbonyl)(methyl)amino)butoxy)-2-methylbenzyl)amino)-1-oxo-4-phenylbutan-2-yl)amino)-6-oxohexanoate | 724.4 | 725.6 |

### (S)-5-acetamido-6-(((S)-1-((5-(4-aminobutoxy)-2-methylbenzyl)amino)-1-oxo-4-phenylbutan-2-yl)amino)-6-oxohexanoic acid (268)

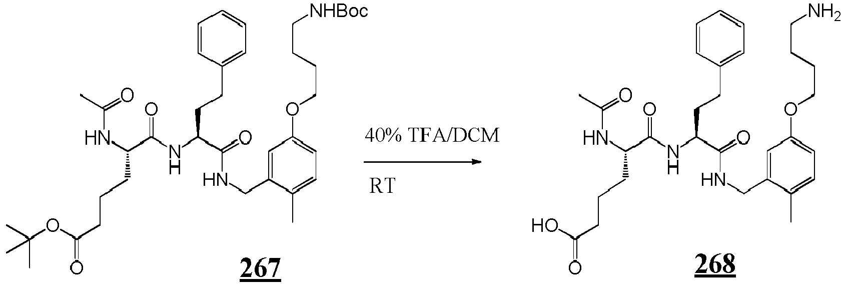

Protecting groups were cleaved from 78 mg compound **267** according to general procedure D stirring compounds in 5 ml 40% TFA/DCM at room temperature. After deprotection was complete solvent was removed and the crude dissolved in acetonitrile and concentrated twice. Product used without further purification.

As exemplified by compound **268** additional examples disclosed in the following table were prepared.

**Table A-33**

| **Cpd no** | From cpd | structure | name | Exact mass | [M+H]⁺ found |
|---|---|---|---|---|---|
| **268** | **267** | 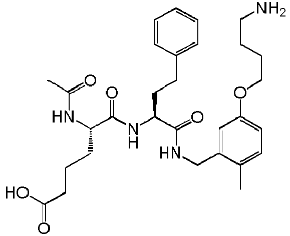 | (S)-5-acetamido-6-(((S)-1-((5-(4-aminobutoxy)-2-methylbenzyl)amino)-1-oxo-4-phenylbutan-2-yl)amino)-6-oxohexanoic acid | 554.3 | 555.4 |
| **276** | **272** | 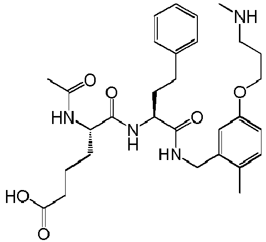 | (S)-5-acetamido-6-(((S)-1-((2-methyl-5-(3-(methylamino)propoxy)benzyl)amino)-1-oxo-4-phenylbutan-2-yl)amino)-6-oxohexanoic acid | 554.3 | 555.4 |
| **277** | **273** | 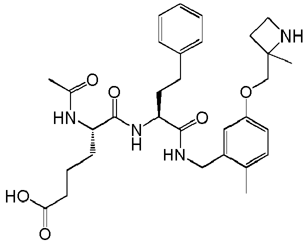 | (5S)-5-acetamido-6-(((2S)-1-((2-methyl-5-((2-methylazetidin-2-yl)methoxy)benzyl)amino)-1-oxo-4-phenylbutan-2-yl)amino)-6-oxohexanoic acid | 566.3 | 567.4 |
| **375** | **374** | 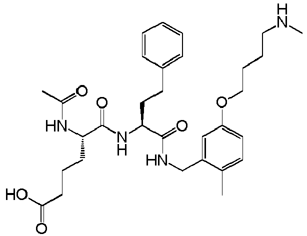 | (S)-5-acetamido-6-(((S)-1-((2-methyl-5-(4-(methylamino)butoxy)benzyl )amino)-1-oxo-4-phenylbutan-2-yl)amino)-6-oxohexanoic acid | 568.3 | 569.4 |

### N-((12S,15S)-1⁴-methyl-8,13,16-trioxo-15-phenethyl-2-oxa-7,14,17-triaza-1(1,3)-benzenacyclooctadecaphane-12-yl)acetamide (269)

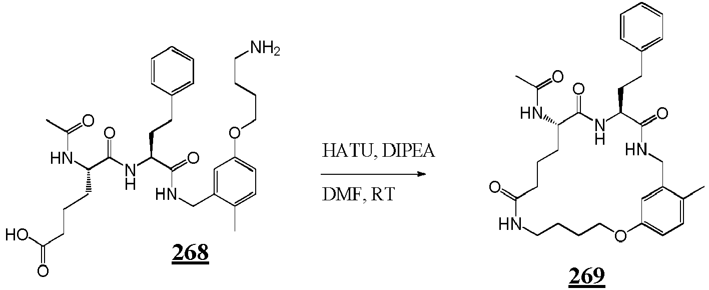

Macrocyclization was achieved according to general procedure A with 0.120 mmol amino acid **268.** Reaction mixtures were diluted with some methanol and purified via HPLC. As exemplified by compound **269** additional examples disclosed in the following table were prepared.

**Table A-34**

| **Cpd no** | From cpd | structure | name | [M+H]⁺ calc. | [M+H]⁺ found |
|---|---|---|---|---|---|
| **269** | **268** | 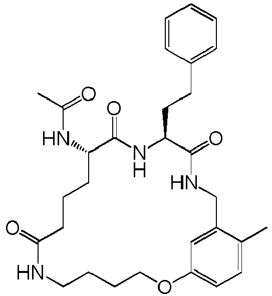 | N-((12S,15S)-1⁴-methyl-8,13,16-trioxo-15-phenethyl-2-oxa-7,14,17-triaza-1(1,3)-benzenacyclooctadecaphane-12-yl)acetamide | 536.3 | 537.4 |
| | | | | C₃₀H₄₀ N₄O₅ | |
| **280** | **276** | 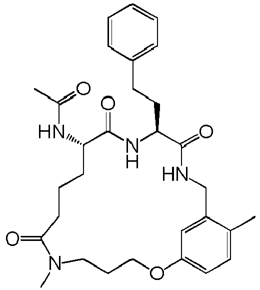 | N-((11S,14S)-1⁴,6-dimethyl-7,12,15-trioxo-14-phenethyl-2-oxa-6,13,16-triaza-1(1,3)-benzenacycloheptadecaphane-11-yl)acetamide | 536.3 | 537.4 |
| | | | | C₃₀H₄₀ N₄O₅ | |
| **281** | 277 | 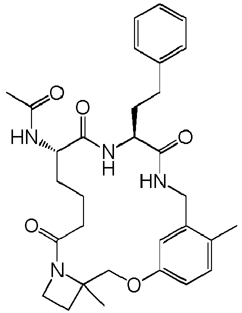 | N-((8S,11S)-1²,4⁴-dimethyl-7,10,15-trioxo-8-phenethyl-3-oxa-6,9-diaza-1(2,1)-azetidina-4(1,3)-benzenacyclopentadecaphane-11-yl)acetamide | 548.3 | 549.5 |
| | | | | C₃₁H₄₂ N₄O₆ | |
| **295** | 375 | 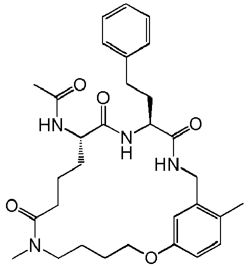 | N-(1⁴,7-dimethyl-8,13,16-trioxo-15-phenethyl-2-oxa-7,14,17-triaza-1(1,3)-benzenacyclooctadecaphane-12-yl)acetamide | 550.3 | 551.4 |
| | | | | C₃₁H₄₂ N₄O₅ | |

### Example A-31: Preparation of macrocyclic compounds 336, and 549 - 564

### tert-butyl 3-(2-(3-((5S)-5-(2-(tert-butoxy)-2-oxoethyl)-3,6,9-trioxo-1-phenyl-8-(2-(pyridin-4-yl)ethyl)-2-oxa-4,7,10-triazaundecan-11-yl)-4-methylphenoxyl)ethyl)piperidine-1-carboxylate(435)

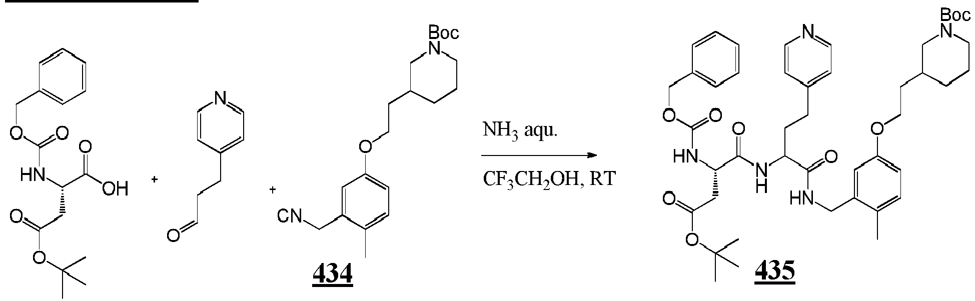

For this Ugi reaction with 400 mg 3-(pyridin-4-yl)propanal and 960 mg Cbz-Asp(OtBu)-OH were dissolved in 3.5 ml 2,2,2-trifluoroethanol. 700 µl ammonia solution (30%, aqueous) and 715 mg isonitrile **434** were added and the mixture was stirred at room temperature overnight. Additional ammonia solution (350 µl) and 250 mg aldehyde in 3.5 ml 2,2,2-trifluoroethanol were added and stirring at room temperature was continued for another day. Saturated NaHCO₃ solution was added and the mixture was extracted with ethyl acetate. After drying the combined organic phases over MgSO₄ and removing volatiles under reduced pressure the product was obtained after normal phase column chromatography (silica, cyclohexane/ethyl acetate gradient).
Formula: C₄₅H₆₁N₅O₉, exact mass: 815.4, found: 716.4 [M+H]⁺

The examples in the following table were prepared according to the procedure described for compound **435** but utilizing Ac-Asp(OtBu)-OH instead of Cbz-Asp(OtBu)-OH and related aldehydes:

**Table A-35**

| **Cpd no** | structure | name | Exact mass | [M+H]⁺ found |
|---|---|---|---|---|
| **541** | 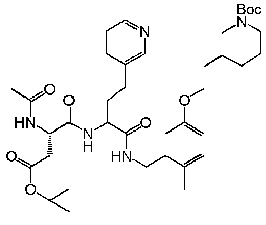 | tert-butyl 3-(2-(3-((2-((S)-2-acetamido-4-(tert-butoxy)-4-oxobutanamido)-4-(pyridin-3-yl)butanamido)methyl)-4-methylphenoxy)ethyl)piperidine-1-carboxylate | 723.4 | 724.8 |
| **542** | 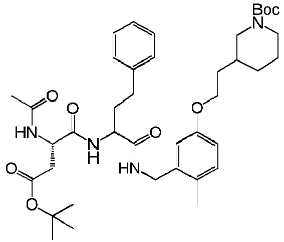 | tert-butyl 3-(2-(3-((2-((S)-2-acetamido-4-(tert-butoxy)-4-oxobutanamido)-4-phenylbutanamido)methyl)-4-methylphenoxy)ethyl)piperidine-1-carboxylate | 722.4 | 723.7 |
| **543** | 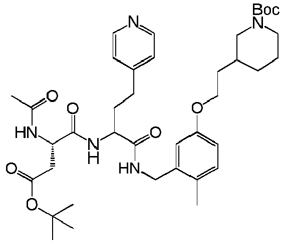 | tert-butyl 3-(2-(3-((2-((S)-2-acetamido-4-(tert-butoxy)-4-oxobutanamido)-4-(pyridin-4-yl)butanamido)methyl)-4-methylphenoxy)ethyl)piperidine-1-carboxylate | 723.4 | 724.5 |
| **544** | 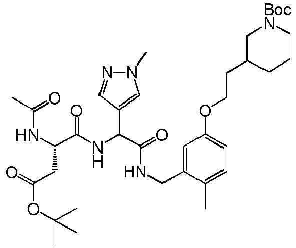 | tert-butyl 3-(2-(3-((2-((S)-2-acetamido-4-(tert-butoxy)-4-oxobutanamido)-2-(1-methyl-1H-pyrazol-4-yl)acetamido)methyl)-4-methylphenoxy)ethyl)piperidine-1-carboxylate | 698.4 | 699.6 |
| **517** | 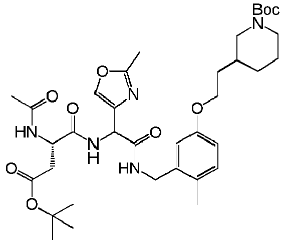 | tert-butyl 3-(2-(3-((2-((S)-2-acetamido-4-(tert-butoxy)-4-oxobutanamido)-2-(2-methyloxazol-4-yl)acetamido)methyl)-4-methylphenoxy)ethyl)piperidine-1-carboxylate | 699.4 | 700.6 |
| **518** | 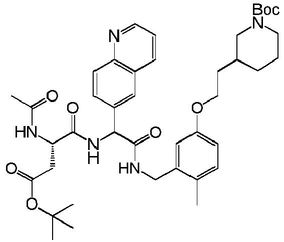 | tert-butyl 3-(2-(3-((2-((S)-2-acetamido-4-(tert-butoxy)-4-oxobutanamido)-2-(quinolin-6-yl)acetamido)methyl)-4-methylphenoxy)ethyl)piperidine-1-carboxylate | 745.4 | 746.7 |
| **519** | 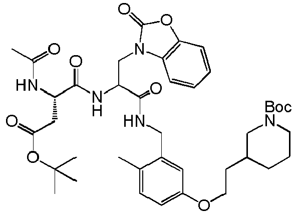 | tert-butyl 3-(2-(3-((2-((S)-2-acetamido-4-(tert-butoxy)-4-oxobutanamido)-3-(2-oxobenzo[d]oxazol-3(2H)-yl)propanamido)methyl)-4-methylphenoxy)ethyl)piperidine-1-carboxylate | 765.4 | 766.7 |
| **520** | 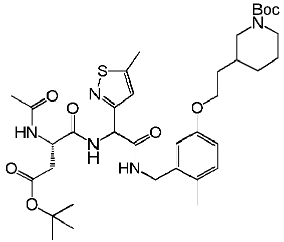 | tert-butyl 3-(2-(3-((2-((S)-2-acetamido-4-(tert-butoxy)-4-oxobutanamido)-2-(5-methylisothiazol-3-yl)acetamido)methyl)-4-methylphenoxy)ethyl)piperidine-1-carboxylate | 715.4 | 716.8 |
| **521** | 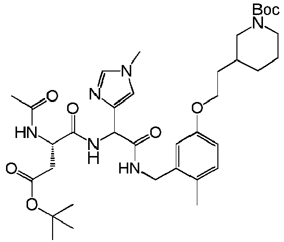 | tert-butyl 3-(2-(3-((2-((S)-2-acetamido-4-(tert-butoxy)-4-oxobutanamido)-2-(1-methyl-1H-imidazol-4-yl)acetamido)methyl)-4-methylphenoxy)ethyl)piperidine-1-carboxylate | 698.4 | 699.7 |
| **522** | 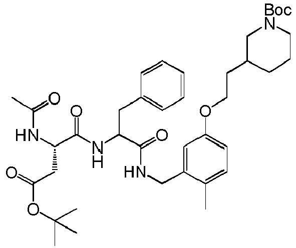 | tert-butyl 3-(2-(3-((2-((S)-2-acetamido-4-(tert-butoxy)-4-oxobutanamido)-3-phenylpropanamido)methyl)-4-methylphenoxy)ethyl)piperidine-1-carboxylate | 708.4 | 709.8 |
| **523** | 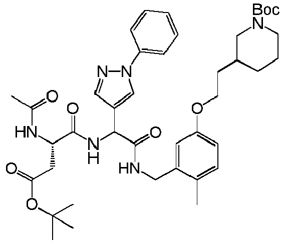 | tert-butyl 3-(2-(3-((2-((S)-2-acetamido-4-(tert-butoxy)-4-oxobutanamido)-2-(1-phenyl-1H-pyrazol-4-yl)acetamido)methyl)-4-methylphenoxy)ethyl)piperidine-1-carboxylate | 760.4 | 761.5 |
| **524** | 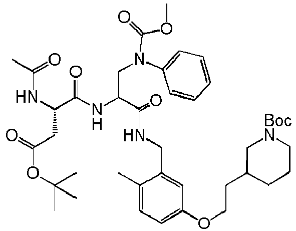 | tert-butyl 3-(2-(3-((2-((S)-2-acetamido-4-(tert-butoxy)-4-oxobutanamido)-3-((methoxycarbonyl)(phenyl)amino)propanamido)methyl)-4-methylphenoxy)ethyl)piperidine-1-carboxylate | 781.4 | 782.8 |
| **525** | 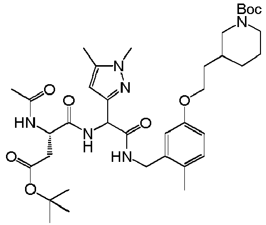 | tert-butyl 3-(2-(3-((2-((S)-2-acetamido-4-(tert-butoxy)-4-oxobutanamido)-2-(1,5-dimethyl-1H-pyrazol-3-yl)acetamido)methyl)-4-methylphenoxy)ethyl)piperidine-1-carboxylate | 712.4 | 713.8 |
| **526** | 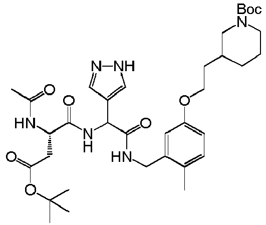 | tert-butyl 3-(2-(3-((2-((S)-2-acetamido-4-(tert-butoxy)-4-oxobutanamido)-2-(1H-pyrazol-3-yl)acetamido)methyl)-4-methylphenoxy)ethyl)piperidine-1-carboxylate | 684.4 | 685.7 |
| **527** | 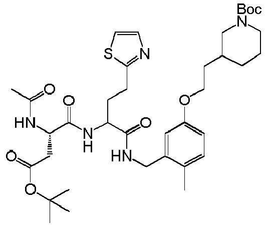 | tert-butyl 3-(2-(3-((2-((S)-2-acetamido-4-(tert-butoxy)-4-oxobutanamido)-4-(thiazol-2-yl)butanamido)methyl)-4-methylphenoxy)ethyl)piperidine-1-carboxylate | 729.4 | 730.7 |
| **528** | 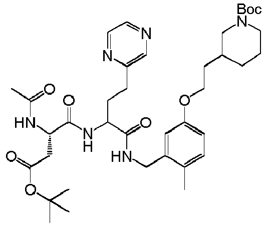 | tert-butyl 3-(2-(3-((2-((S)-2-acetamido-4-(tert-butoxy)-4-oxobutanamido)-4-(pyrazin-2-yl)butanamido)methyl)-4-methylphenoxy)ethyl)piperidine-1-carboxylate | 724.4 | 725.8 |

### (3S)-3-(((benzyloxy)carbonyl)amino)-4-((1-((2-methyl-5-(2-(piperidin-3-yl)ethoxy)benzyl) amino)-1-oxo-4-(pyridin-4-yl)butan-2-yl)amino)-4-oxobutanoic acid (436)

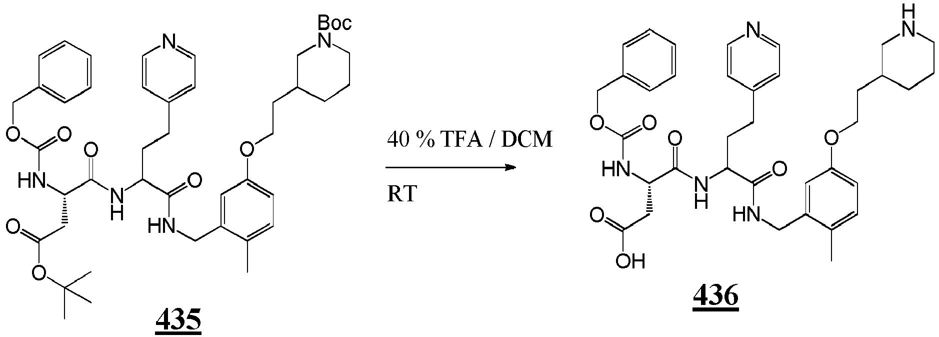

Protecting groups were cleaved from 750 mg compound **435** according to general procedure D stirring compounds in 5 ml 40% TFA/DCM at room temperature. After deprotection was complete solvent was removed and the crude dissolved in acetonitrile and concentrated twice. Product used without further purification.
Formula: C₃₅H₄₅N₅O₇, exact mass: 659.3, found: 660.3 [M+H]⁺

The examples in the following table were prepared similar to the procedure described for compound **436:**

**Table A-36**

| **Cpd no** | From Cpd | structure | name | Exact mass | [M+H]⁺ found |
|---|---|---|---|---|---|
| **545** | **541** | 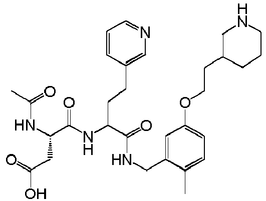 | (3S)-3-acetamido-4-((1-((2-methyl-5-(2-(piperidin-3-yl)ethoxy)benzyl)amino)-1-oxo-4-(pyridin-3-yl)butan-2-yl)amino)-4-oxobutanoic acid | 567.3 | 568.4 |
| **546** | **542** | 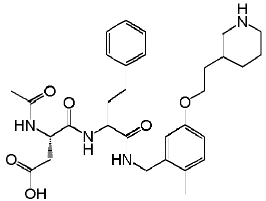 | (3S)-3-acetamido-4-((1-((2-methyl-5-(2-(piperidin-3-yl)ethoxy)benzyl)amino)-1-oxo-4-phenylbutan-2-yl)amino)-4-oxobutanoic acid | 566.3 | 567.4 |
| **547** | **543** | 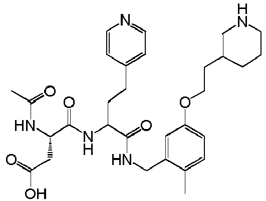 | (3S)-3-acetamido-4-((1 -((2-methyl-5-(2-(piperidin-3-yl)ethoxy)benzyl)amino)-1-oxo-4-(pyridin-4-yl)butan-2-yl)amino)-4-oxobutanoic acid | 567.3 | 568.4 |
| **548** | **544** | 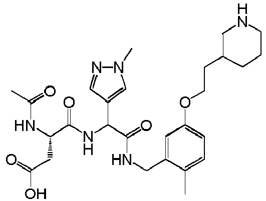 | (3S)-3-acetamido-4-((1-(1-methyl-1H-pyrazol-4-yl)-2-((2-methyl-5-(2-(pi peridin-3-yl)ethoxy)benzyl)amino)-2-oxoethyl)amino)-4-oxobutanoic acid | 542.3 | 543.3 |
| **529** | **517** | 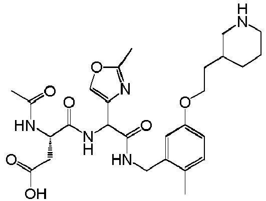 | (3S)-3-acetamido-4-((2-((2-methyl-5-(2-(piperidin-3-yl)ethoxy)benzyl)amino)-1-(2-methyloxazol-4-yl)-2-oxoethyl)amino)-4-oxobutanoic acid | 543.3 | 544.3 |
| **530** | **518** | 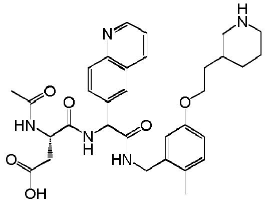 | (3S)-3-acetamido-4-((2-((2-methyl-5-(2-(piperidin-3-yl)ethoxy)benzyl)amino)-2-oxo-1-(quinolin-6-yl)ethyl)amino)-4-oxobutanoic acid | 589.3 | 590.6 |
| **531** | **519** | 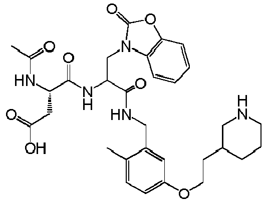 | (3S)-3-acetamido-4-((1-((2-methyl-5-(2-(piperidin-3-yl)ethoxy)benzyl)amino)-1-oxo-3-(2-oxobenzo[d]oxazol-3(2H)-yl)propan-2-yl)amino)-4-oxobutanoicacid | 609.3 | 610.4 |
| **532** | **520** | 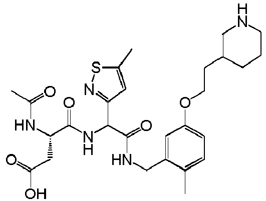 | (3S)-3-acetamido-4-((2-((2-methyl-5-(2-(piperidin-3-yl)ethoxy)benzyl)amino)-1-(5-methylisothiazol-3-yl)-2-oxoethyl)amino)-4-oxobutanoic acid | 559.2 | 560.4 |
| **533** | **521** | 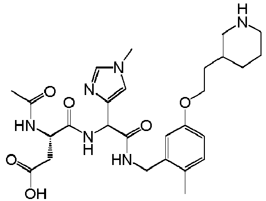 | (3S)-3-acetamido-4-((1-(1-methyl-1H-imidazol-4-yl)-2-((2-methyl-5-(2-(piperidin-3-yl)ethoxy)benzyl)amino)-2-oxoethyl)amino)-4-oxobutanoic acid | 542.3 | 543.4 |
| **534** | **522** | 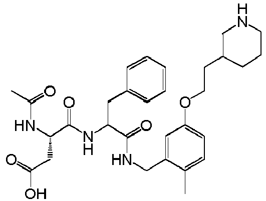 | (3S)-3-acetamido-4-((1-((2-methyl-5-(2-(piperidin-3-yl)ethoxy)benzyl)amino)-1-oxo-3-phenylpropan-2-yl)amino)-4-oxobutanoic acid | 552.3 | 553.4 |
| **535** | **523** | 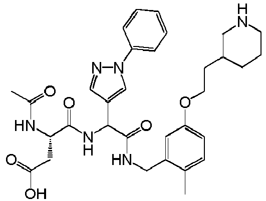 | (3S)-3-acetamido-4-((2-((2-methyl-5-(2-(piperidin-3-yl)ethoxy)benzyl)amino)-2-oxo-1-(1-phenyl-1H-pyrazol-4-yl)ethyl)amino)-4-oxobutanoic acid | 604.3 | 605.6 |
| **536** | **524** | 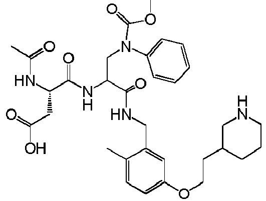 | (3S)-3-acetamido-4-((3-((methoxycarbonyl)(phenyl)a mino)-1-((2-methyl-5-(2-(piperidin-3-yl)ethoxy)benzyl)amino)-1-oxopropan-2-yl)amino)-4-oxobutanoic acid | 625.3 | 626.6 |
| **537** | **525** | 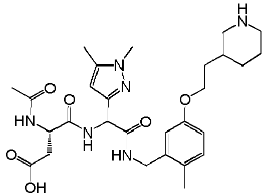 | (3S)-3-acetamido-4-((1-(1,5-dimethyl-1H-pyrazol-3-yl)-2-((2-methyl-5-(2-(piperidin-3-yl)ethoxy)benzyl)amino)-2-oxoethyl)amino)-4-oxobutanoic acid | 556.3 | 557.4 |
| **538** | **526** | 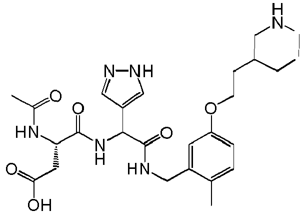 | (3S)-3-acetamido-4-((2-((2-methyl-5-(2-(piperidin-3-yl)ethoxy)benzyl)amino)-2-oxo-1-(1H-pyrazol-4-yl)ethyl)amino)-4-oxobutanoic acid | 528.3 | 529.4 |
| **539** | **527** | 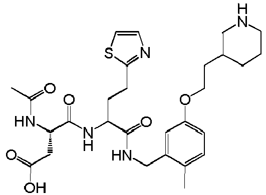 | (3S)-3-acetamido-4-((1-((2-methyl-5-(2-(piperidin-3-yl)ethoxy)benzyl)amino)-1-oxo-4-(thiazol-2-yl)butan-2-yl)amino)-4-oxobutanoic acid | 573.3 | 574.6 |
| **540** | **528** | 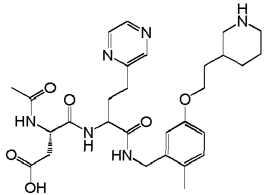 | (3S)-3-acetamido-4-((1-((2-methyl-5-(2-(piperidin-3-yl)ethoxy)benzyl)amino)-1-oxo-4-(pyrazin-2-yl)butan-2-yl)amino)-4-oxobutanoic acid | 568.3 | 569.3 |

### benzyl ((12S)-5⁴-methyl-8,11,14-trioxo-9-(2-(pyridin-4-yl)ethyl)-4-oxa-7,10-diaza-1(3,1)-piperidina-5(1,3)-benzenacyclotetradecaphane-12-yl)carbamate (336)

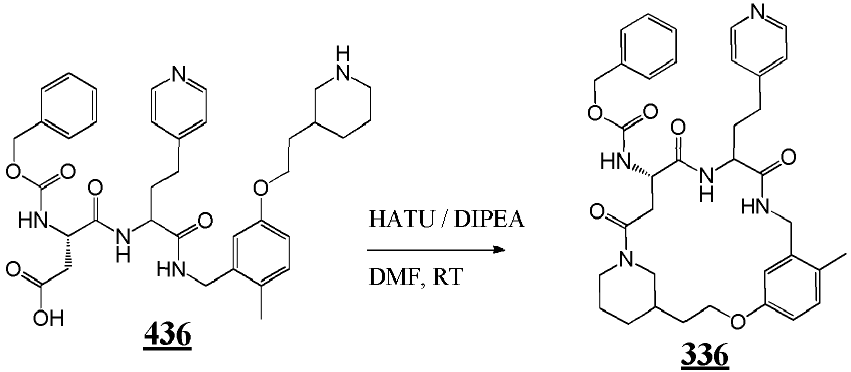

Macrocyclization was achieved according to general procedure A with 606 mg amino acid **436.** Reaction mixtures were diluted with some methanol and purified via reversed phase column chromatography (RP18, water/acetonitrile gradient) and HPLC.
Formula: C₃₆H₄₃N₅O₆, exact mass: 641.3, found: 642.3 [M+H]+

The examples in the following table were prepared similar to the procedure described for compound **336:**

**Table A-37**

| **Cpd no** | From Cpd | structure | name | Exact mass | [M+H]⁺ found |
|---|---|---|---|---|---|
| **549** | **545** | 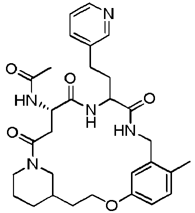 | N-((12S)-5⁴-methyl-8,11,14-trioxo-9-(2-(pyridin-3-yl)ethyl)-4-oxa-7,10-diaza-1(3,1)-piperidina-5(1,3)-benzenacyclotetradecaphane-12-yl)acetamide | 549 | 550 |
| **550** | **546** | 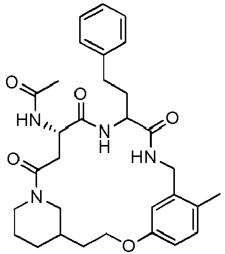 | N-((12S)-5⁴-methyl-8,11,14-trioxo-9-phenethyl-4-oxa-7,10-diaza-1(3,1)-piperidina-5(1,3)-benzenacyclotetradecaphane-12-yl)acetamide | 548 | 549 |
| **551** | **547** | 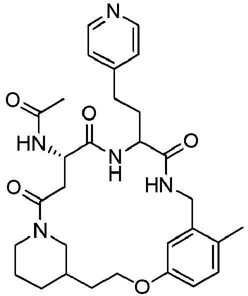 | N-((12S)-5⁴-methyl-8,11,14-trioxo-9-(2-(pyridin-4-yl)ethyl)-4-oxa-7,10-diaza-1(3,1)-piperidina-5(1,3)-benzenacyclotetradecaphane-12-yl)acetamide | 549 | 550 |
| **552** | **548** | 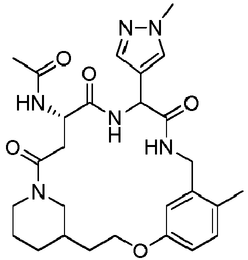 | N-((12S)-5⁴-methyl-9-(1-methyl-1H-pyrazol-4-yl)-8,11,14-trioxo-4-oxa-7,10-diaza-1(3,1)-piperidina-5(1,3)-benzenacyclotetradecaphane-12-yl)acetamide | 524 | 525 |
| **553** | **529** | 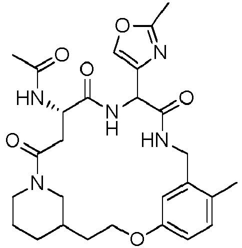 | N-((12S)-5⁴-methyl-9-(2-methyloxazol-4-yl)-8,11,14-trioxo-4-oxa-7,10-diaza-1(3,1)-piperidina-5(1,3)-benzenacyclotetradecaphane-12-yl)acetamide | 525 | 526 |
| **554** | **530** | 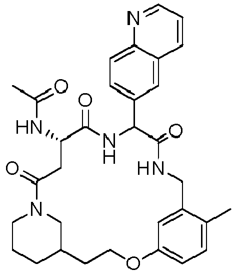 | N-((12S)-5⁴-methyl-8,11,14-trioxo-9-(quinolin-6-yl)-4-oxa-7,10-diaza-1(3,1)-piperidina-5(1,3)-benzenacyclotetradecaphane-12-yl)acetamide | 571 | 572 |
| **555** | **531** | 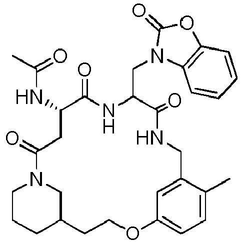 | N-((12S)-5⁴-methyl-8,11,14-trioxo-9-((2-oxobenzo[d]oxazol-3(2H)-yl)methyl)-4-oxa-7,10-diaza-1(3,1)-piperidina-5(1,3)-benzenacyclotetradecaphane-12-yl)acetamide | 591 | 592 |
| **556** | **532** | 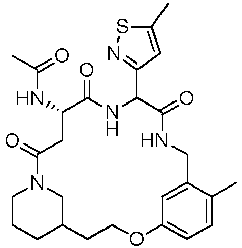 | N-((12S)-5⁴-methyl-9-(5-methylisothiazol-3-yl)-8,11,14-trioxo-4-oxa-7,10-diaza-1(3,1)-piperidina-5(1,3)-benzenacyclotetradecaphane-12-yl)acetamide | 541 | 542 |
| **557** | **533** | 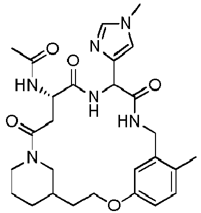 | N-((12S)-5⁴-methyl-9-(1-methyl-1H-imidazol-4-yl)-8,11,14-trioxo-4-oxa-7,10-diaza-1(3,1)-piperidina-5(1,3)-benzenacyclotetradecaphane-12-yl)acetamide | 524 | 525 |
| **558** | **534** | 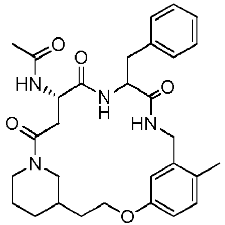 | N-((12S)-9-benzyl-5⁴-methyl-8,11,14-trioxo-4-oxa-7,10-diaza-1(3,1)-piperidina-5(1,3)-benzenacyclotetradecaphane-12-yl)acetamide | 534 | 535 |
| **559** | **535** | 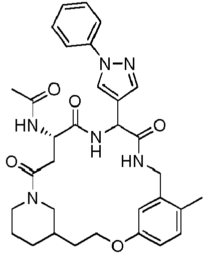 | N-((12S)-5⁴-methyl-8,11,14-trioxo-9-(1-phenyl-1H-pyrazol-4-yl)-4-oxa-7,10-diaza-1(3,1)-piperidina-5(1,3)-benzenacyclotetradecaphane-12-yl)acetamide | 586 | 587 |
| **560** | **536** | 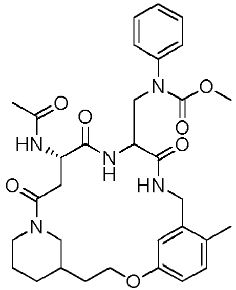 | methyl (((12S)-12-acetamido-5⁴-methyl-8,11,14-trioxo-4-oxa-7,10-diaza-1(3,1)-piperidina-5(1,3)-benzenacyclotetradecaphane-9-yl)methyl)(phenyl)carbamate | 607 | 608 |
| **561** | **537** | 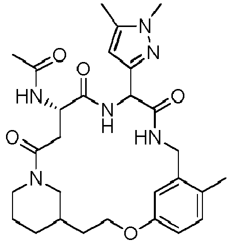 | N-((12S)-9-(1,5-dimethyl-1H-pyrazol-3-yl)-5⁴-methyl-8,11,14-trioxo-4-oxa-7,10-benzenacyclotetradecaphane-12-yl)acetamide | 538 | 539 |
| **562** | **538** | 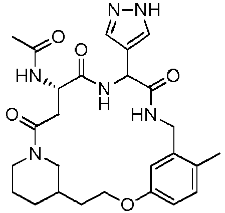 | N-((12S)-5⁴-methyl-8,11,14-trioxo-9-(1H-pyrazol-4-yl)-4-oxa-7,10-diaza-1(3,1)-piperidina-5(1,3)-benzenacyclotetradecaphane-12-yl)acetamide | 510 | 511 |
| **563** | **539** | 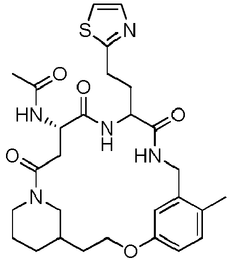 | N-((12S)-5⁴-methyl-8,11,14-trioxo-9-(2-(thiazol-2-yl)ethyl)-4-oxa-7,10-diaza-1(3,1)-piperidina-5(1,3)-benzenacyclotetradecaphane-12-yl)acetamide | 555 | 556 |
| **564** | **540** | 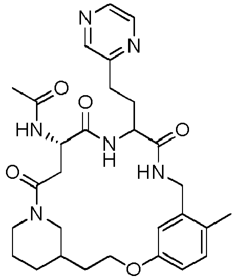 | N-((12S)-5⁴-methyl-8,11,14-trioxo-9-(2-(pyrazin-2-yl)ethyl)-4-oxa-7,10-diaza-1(3,1)-piperidina-5(1,3)-benzenacyclotetradecaphane-12-yl)acetamide | 550 | 551 |

### Example A-32: Preparation of macrocyclic compounds 338, 342- 344, 437 439, 443 and 444

### ((12S)-12-amino-5⁴-methyl-8,11,14-trioxo-9-(2-(pyridin-4-yl)ethyl)-4-oxa-7,10-diaza-1(3,1)-piperidina-5(1 ,3)-benzenacyclotetradecaphane-12-yl) (437)

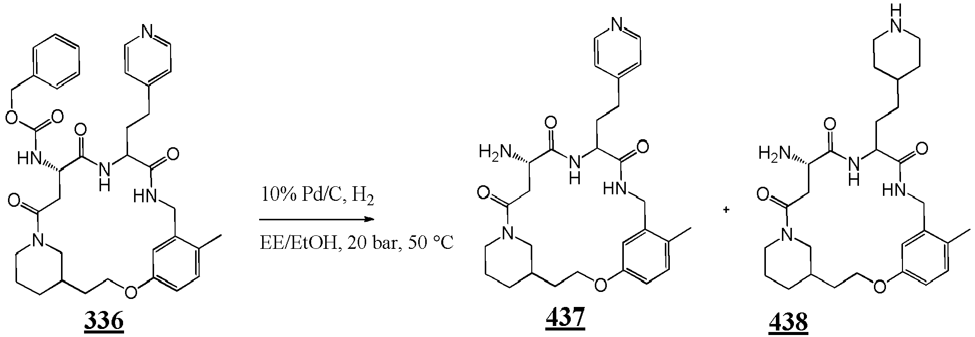

Following general procedure C using 190 mg Cbz-protected amine **336** were dissolved ethyl acetate / ethanol (1:1). H-Cube conditions: 1 ml/min, 50°C, 20 bar H₂. After removing of volatiles under reduced pressure the product was used without further purification. Some piperidyl side product **438** was removed after the following step.
**437-** Formula: C₂₈H₃₇N₅O₄, exact mass: 507.3, found: 508.3 [M+H]⁺
**438-** Formula: C₂₈H₄₃N₅O₄, exact mass: 513.3, found: 514.3 [M+H]⁺

### N-((12S)-9-(2-(1-acetylpiperidin-4-yl)ethyl)-5⁴-methyl-8,11,14-trioxo-4-oxa-7,10-diaza-1(3,1)-piperidina-5(1,3)-benzenacyclotetradecaphane-12-yl)acetamide (338)

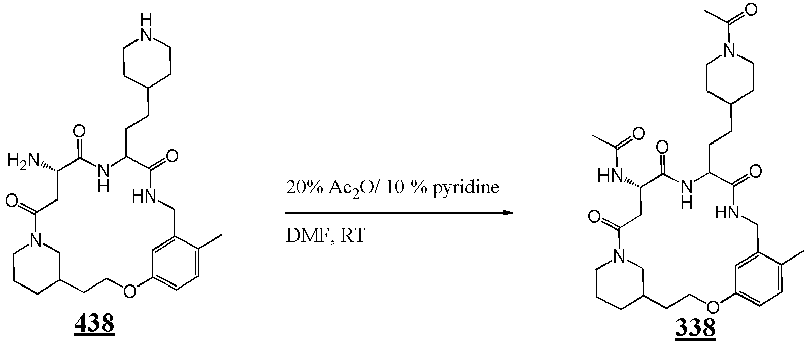

Both free amines in compound **438** were acetylated by dissolving amine 438 in Ac₂O/pyridine/DMF (20/10/70) at room temperature. Purification of the reaction mixture was achieved by HPLC to give compound **338.**
Formula: C₃₂H₄₇N₅O₆, exact mass: 597.4, found: 598.4 [M+H]⁺

### Amide derivatizations of compound 437 according to general procedure B

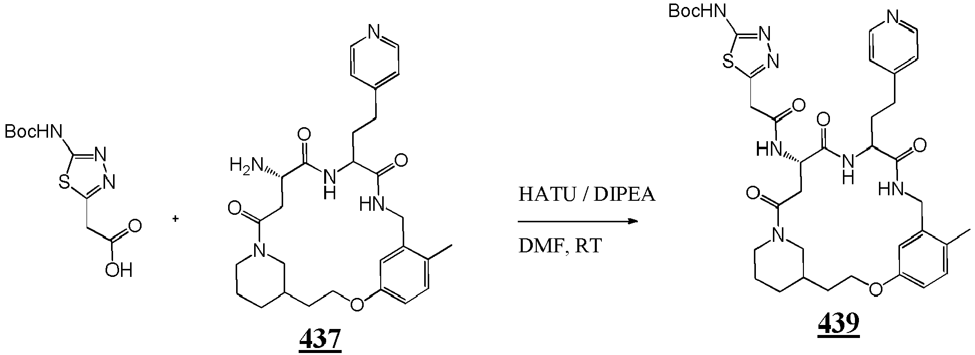

Amine **437** (32 mg) was coupled with carboxylic acids according to general procedure B to give amides disclosed in table below as exemplified for derivative **439.** Purifications were achieved by HPLC or reversed phase flash chromatography.

**Table A-38**

| **Cpd no** | structure | name | Exact mass | [M+H]⁺ found |
|---|---|---|---|---|
| **439** | 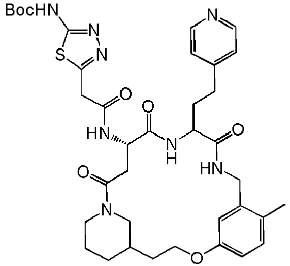 | tert-butyl (5-(2-(((12S)-5⁴-methyl-8,11,14-trioxo-9-(2-(pyridin-4-yl)ethyl)-4-oxa-7,10-diaza-1(3,1)-piperidina-5(1,3)-benzenacyclotetradecaphane-12-yl)amino)-2-oxoethyl)-1,3,4-thiadiazol-2-yl)carbamate | 748.3 | 749.3 |
| | | | C₃₇H₄₈ | |
| | | | N₈O₇S | |
| **443** | 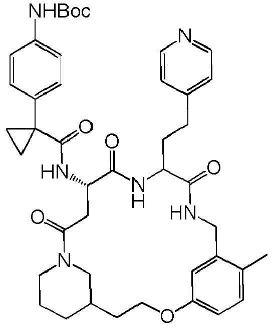 | tert-butyl (4-(1-(((12S)-5⁴-methyl-8,11,14-trioxo-9-(2-(pyridin-4-yl)ethyl)-4-oxa-7,10-diaza-1(3,1)-piperidina-5(1,3)-benzenacyclotetradecaphane-12-yl)carbamoyl)cyclopropyl)phenyl)carbamate | 766.4 | 767.4 |
| | | | C₄₃H₅₄ | |
| | | | N₆O₇ | |
| **444** | 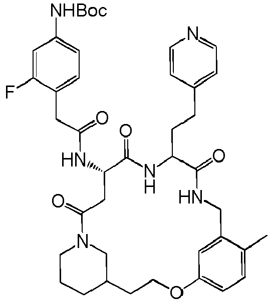 | tert-butyl (3-fluoro-4-(2-(((12S)-5⁴-methyl-8,11,14-trioxo-9-(2-(pyridin-4-yl)ethyl)-4-oxa-7,10-diaza-1 (3,1)-piperidina-5(1,3)-benzenacyclotetradecaphane-12-yl)amino)-2-oxoethyl)phenyl)carbamate | 758.4 | 759.4 |
| | | | C₄₁H₅₁F | |
| | | | N₆O₇ | |

### Removal of Boc-protecting groups

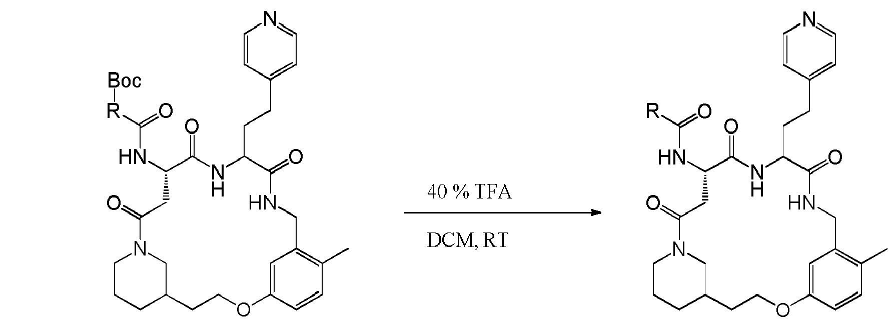

Boc protecting groups were cleaved from the compound according to general procedure D stirring compounds in 5 ml 40% TFA in DCM at room temperature. After deprotection was complete solvent was removed and the crude product was purified by reversed phase HPLC.

**Table A-39**

| **Cpd no** | From cpd | structure | name | Exact mass | [M+H]⁺ found |
|---|---|---|---|---|---|
| **342** | **439** | 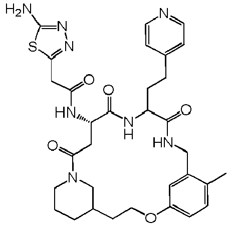 | 2-(5-amino-1,3,4-thiadiazol-2-yl)-N-((12S)-5⁴-methyl-8,11,14-trioxo-9-(2-(pyridin-4-yl)ethyl)-4-oxa-7,10-diaza-1(3,1)-piperidina-5(1,3)-benzenacyclotetradecaphane-12-yl)acetamide | 648.3 | 649.3 |
| | | | | C₃₂H₄₀ | |
| | | | | N₈O₅S | |
| **343** | **443** | 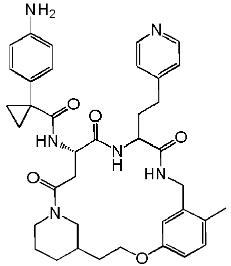 | 1-(4-aminophenyl)-N-((12S)-5⁴-methyl-8,11,14-trioxo-9-(2-(pyridin-4-yl)ethyl)-4-oxa-7,10-diaza-1(3,1)-piperidina-5(1 ,3)-benzenacyclotetradecaphane-12-yl)cyclopropane-1 - carboxamide | 666.4 | 667.4 |
| | | | | C₃₈H₄₆ | |
| | | | | N₆O₅ | |
| **344** | **444** | 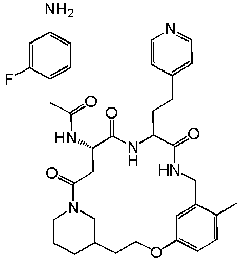 | 2-(4-amino-2-fluorophenyl)-N-((12S)-5⁴-methyl-8,11,14-trioxo-9-(2-(pyridin-4-yl)ethyl)-4-oxa-7,10-diaza-1(3,1)-piperidina-5(1,3)-benzenacyclotetradecaphane-12-yl)acetamide | 658.3 | 659.4 |
| | | | | C₃₆H₄₃ | |
| | | | | FN₆O₅ | |

### Example A-33: Preparation of macrocyclic compound 348

### 2,2-dimethyl-N-((9R,12R)-5⁴-methyl-8,11,14-trioxo-9-phenethyl-4-oxa-7,10-diaza-1(3,1)-piperidina-5(1,3)-benzenacyclotetradecaphane-12-yl)-3-(tritylthio)propanamide (450)

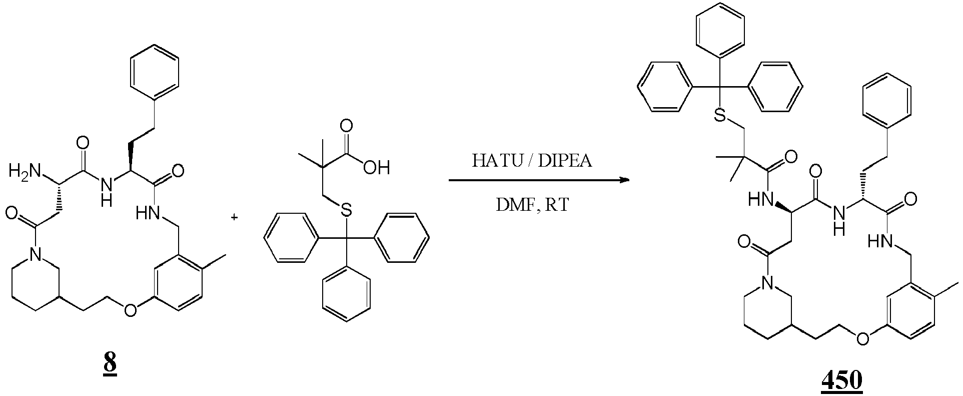

Amine 8 (100 mg) was coupled with the carboxylic acid according to general procedure B to give protected thiol **450.** Purification was achieved by reversed phase HPLC. Formula: C₃₂H₄₇N₅O₆, exact mass: 597.4, found: 598.4 [M+H]⁺

### 3-mercapto-2,2-dimethyl-N-((9S,12S)-5⁴-methyl-8,11,14-trioxo-9-phenethyl-4-oxa-7,10-diaza-1(3,1)-piperidina-5(1 ,3)-benzenacyclotetradecaphane-12-yl)propanamide (348)

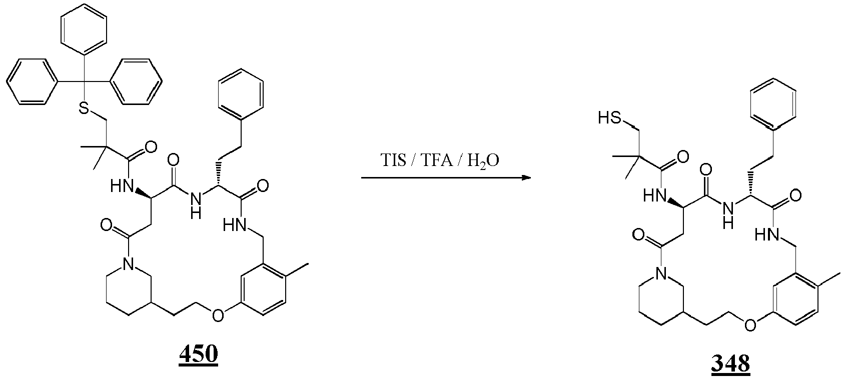

Compound **450** (140 mg) was dissolved in 1 ml TIS/TFA/H₂O (10/2/2) at room temperature and stirred for 1h. Purification by reversed phase column chromatography (RP18, water/acetonitrile gradient).
Formula: C₃₄H₄₆N₄O₆, exact mass: 622.3, found: 623.5 [M+H]⁺

### Example A-34: Preparation of macrocyclic compound 355

### ethyl 4-(3-(2-(3-((2-(((benzyloxy)carbonyl)amino)-2-(1-methyl-1H-pyrazol-4-vl)acetamido)methyl)-4,5-dimethylphenoxy)ethyl)piperidin-1-yl)-4-oxobutanoate (470)

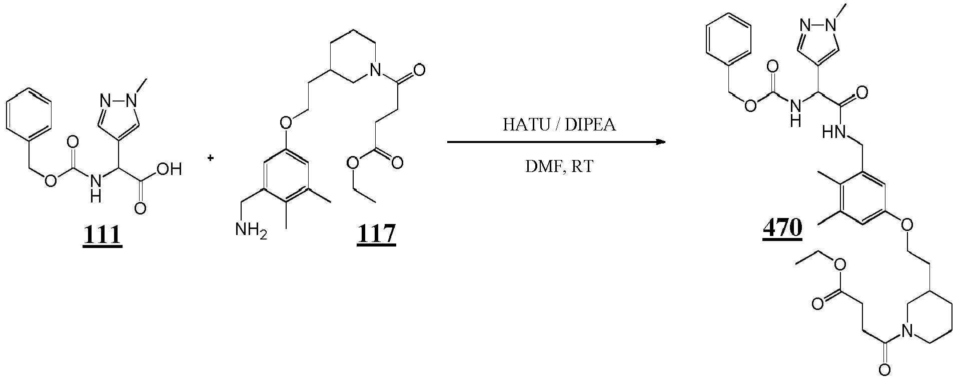

162 mg amine **117** and 100 mg carboxylic acid **111** were coupled according to general procedure B to give compound **470.** Purification was achieved by reversed phase column chromatography (RP18, water/acetonitrile gradient).
Formula: C₃₆H₄₇N₅O₇, exact mass: 661.3, found: 662.5 [M+H]⁺

### ethyl 4-(3-(2-(3-((2-amino-2-(1-methyl-1H-pyrazol-4-yl)acetamido)methyl)-4,5-dimethylphenoxy)ethyl)piperidin-1-yl)-4-oxobutanoate (471)

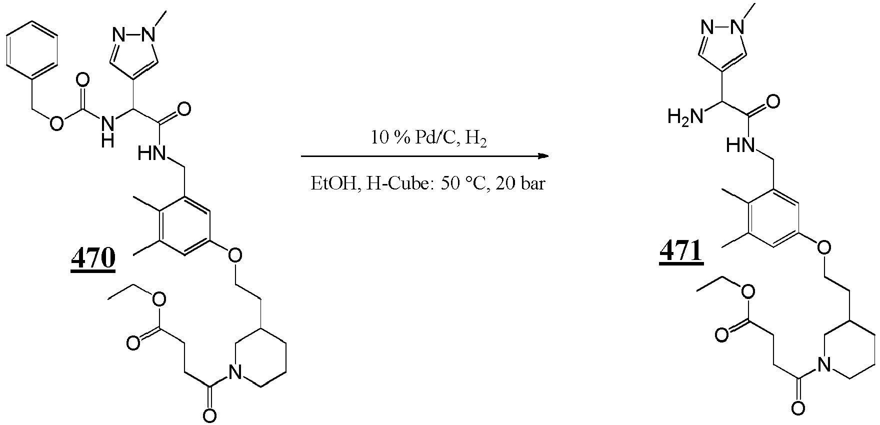

Cbz-protection group was removed according to general procedure C in EtOH at 50 °C and 20 bar H₂ pressure. Solvent was removed under reduced pressure to give a product pure enough to be used in the next reaction.
Formula: C₂₈H₄₁N₅O₅, exact mass: 527.3, found: 528.4 [M+H]⁺

### 4-(3-(2-(3-((2-amino-2-(1-methyl-1H-pyrazol-4-yl)acetamido)methyl)-4,5-dimethylphenoxy)ethyl) piperidin-1-yl)-4-oxobutanoicacid (472)

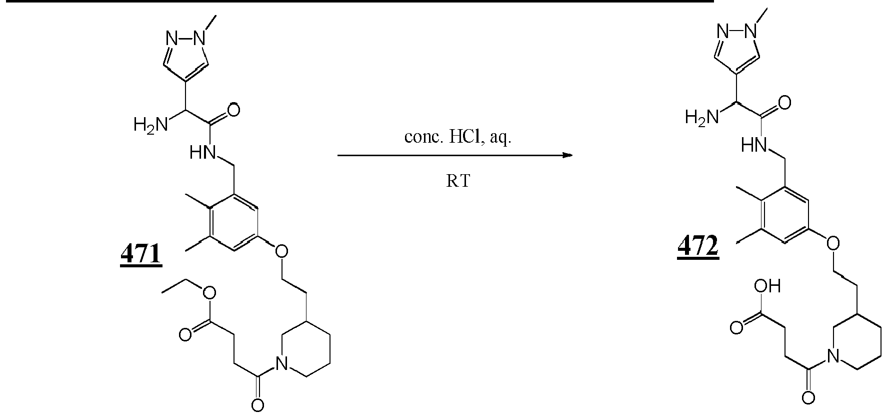

The crude ester **471** was dissolved in concentrated aqueous HCl solution at room temperature. The solution was stirred until the reaction was complete. Volatiles were removed and dissolved in acetonitrile. After drying the crude was used in the following cyclization.
Formula: C₂₆H₃₇N₅O₅, exact mass: 499.3, found: 500.2 [M+H]⁺

### 5⁴,5⁵-dimethyl-9-(1-methyl-1H-pyrazol-4-yl)-4-oxa-7,10-diaza-1(3,1)-piperidina-5(1,3)-benzenacyclotetradecaphane-8,11,14-trione (355)

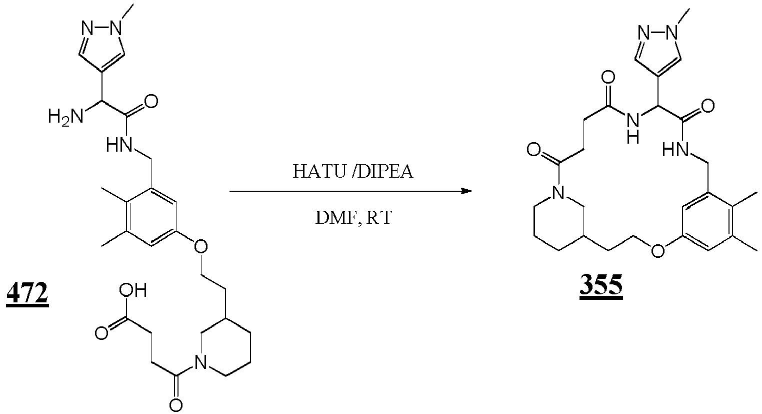

Macrocyclization was achieved according to general procedure A with 75 mg amino acid **472.** Reaction mixtures were diluted with some methanol and purified via reversed phase column chromatography (RP18, water/acetonitrile gradient) and HPLC.
Formula: C₂₆H₃₅N₅O₄, exact mass: 481.3, found: 482.3 [M+H]⁺

### Example A-35: Preparation of macrocyclic compound 356

### tert-butyl 3-(2-(3-((2-(((benzyloxy)carbonyl)amino)-2-(1-methyl-1H-pyrazol-4-vl)acetamido)methyl)-4-methylphenoxy)ethyl)piperidine-1-carboxylate (473)

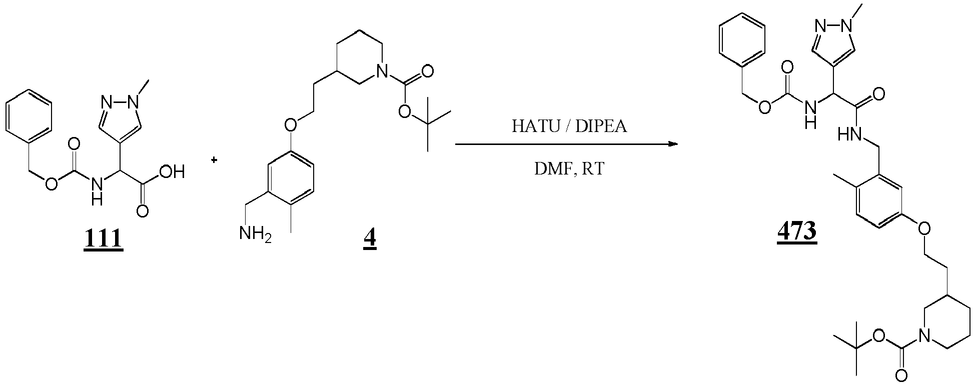

452 mg amine **4** and 341 mg carboxylic acid **111** were coupled according to general procedure B to give compound **473.** Purification was achieved by normal phase column chromatography (silica, cyclohexane/ethyl acetate gradient).
Formula: C₃₄H₄₅N₅O₆, exact mass: 619.3, found: 620.3 [M+H]⁺

### tert-butyl 3-(2-(3-((2-amino-2-(1-methyl-1H-pyrazol-4-yl)acetamido)methyl)-4-methylphenoxy)ethyl)piperidine-1-carboxylate (474)

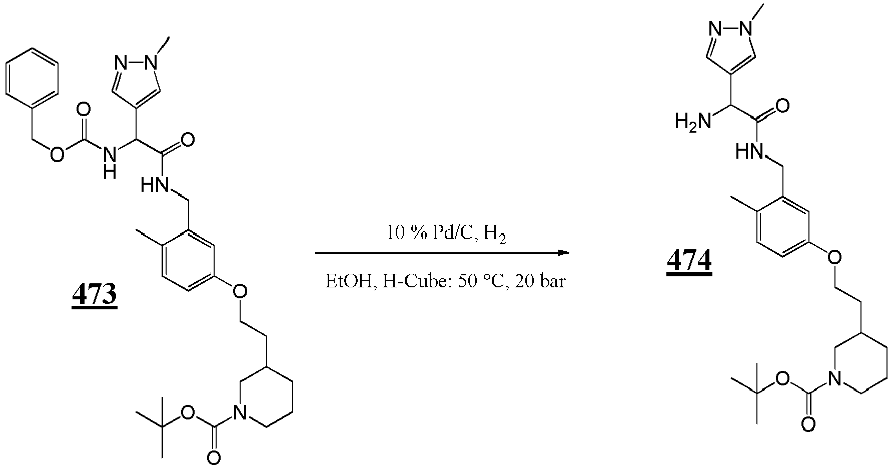

Cbz-protection group was removed according to general procedure C in EtOH at 50 °C and 20 bar H₂ pressure. Solvent was removed under reduced pressure to give a product pure enough to be used in the next reaction.
Formula: C₂₆H₃₉N₅O₄, exact mass: 485.3, found: 486.3 [M+H]⁺

### tert-butyl 3-(2-(3-((2-(6-ethoxy-6-oxohexanamido)-2-(1-methyl-1H-pyrazol-4-vl)acetamido)methyl)-4-methylphenoxy)ethyl)piperidine-1-carboxylate (475)

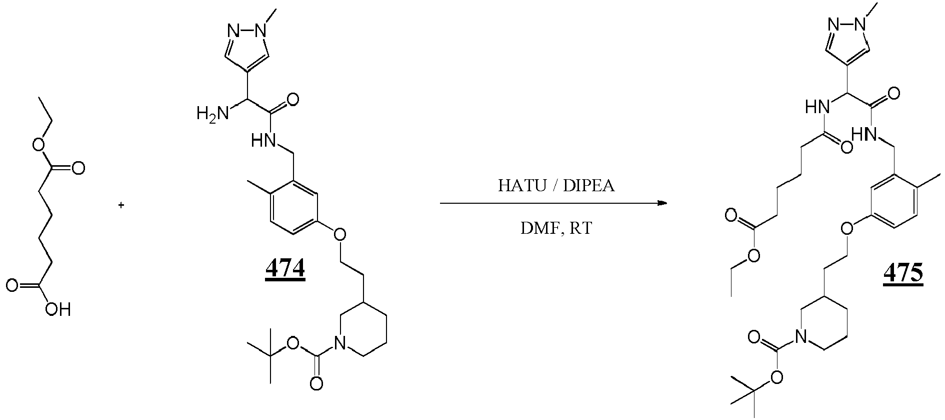

100 mg amine **474** and 43 mg 6-ethoxy-6-oxohexanoic acid were coupled according to general procedure B to give compound **475.** Purification was achieved by normal phase column chromatography (silica, cyclohexane/ethyl acetate gradient).
Formula: C₃₄H₅₁N₅O₇, exact mass: 641.4, found: 642.4 [M+H]⁺

### 6-((1-(1-methyl-1H-pyrazol-4-yl)-2-((2-methyl-5-(2-(piperidin-3-yl)ethoxy)benzyl)amino)-2-oxoethyl)amino)-6-oxohexanoic acid (476)

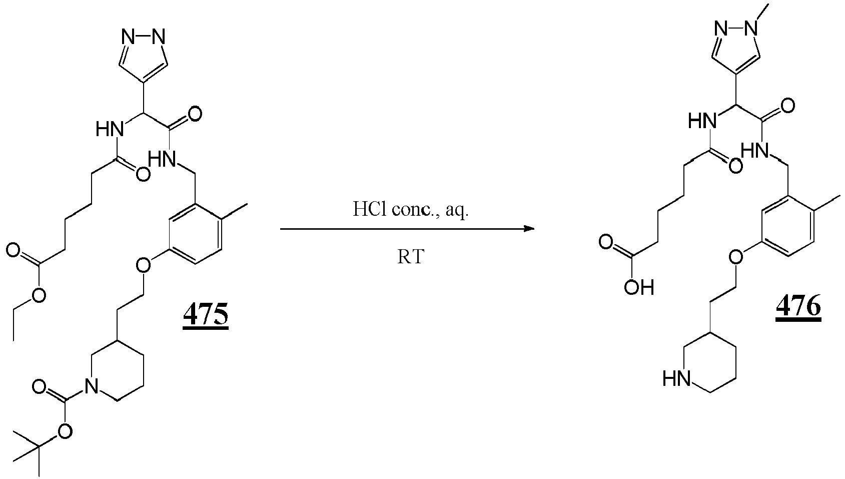

Ester **475** was dissolved in concentrated aqueous HCl solution at room temperature. The solution was stirred until the reaction was complete. Volatiles were removed and dissolved in acetonitrile. After drying the crude was used in the following cyclization. Formula: C₂₇H₃₉N₅O₅, exact mass: 513.3, found: 514.3 [M+H]⁺

### 5⁴-methyl-9-(1-methyl-1H-pyrazol-4-yl)-4-oxa-7,10-diaza-1(3,1)-piperidina-5(1,3)-benzenacyclohexadecaphane-8,11,16-trione (356)

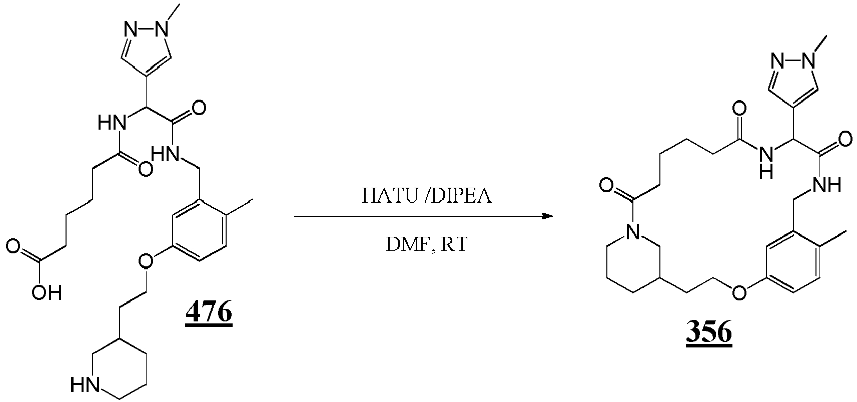

Macrocyclization was achieved according to general procedure A with 0.11 amino acid **476.** Reaction mixtures were diluted with some methanol and purified via HPLC.
Formula: C₂₇H₃₇N₅O₄, exact mass: 495.3, found: 496.3 [M+H]⁺

### Example A-36: Preparation of macrocyclic compounds 358-363, 479, 480, and 552

### tert-butyl 3-(2-(3-((5S)-5-(2-ethoxy-2-oxoethyl)-8-(1-methyl-1H-pyrazol-4-yl)-3,6,9-trioxo-1-phenyl-2-oxa-4,7,10-triazaundecan-11-yl)-4-methylphenoxy)ethyl)piperidine-1-carboxylate (477)

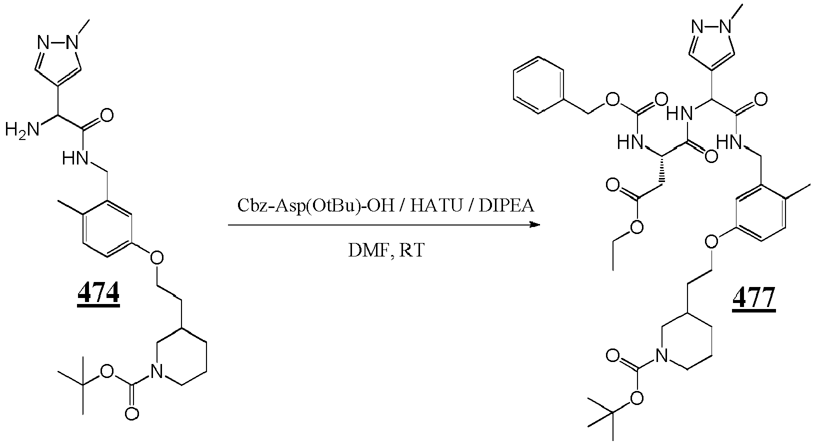

3 g amine **474** and 3 g Cbz-Asp(OtBu)-OH were coupled according to general procedure B to give compound **477.** Purification was achieved by normal phase column chromatography (silica, cyclohexane/ethyl acetate gradient).
Formula: C₄₀Hₛ₄N₆O₉, exact mass: 762.4, found: 763.5 [M+H]⁺

### (3S)-3-(((benzyloxy)carbonyl)amino)-4-((1-(1-methyl-1H-pyrazol-4-yl)-2-((2-methyl-5-(2-(piperidin-3-yl)ethoxy)benzyl)amino)-2-oxoethyl)amino)-4-oxobutanoic acid (478)

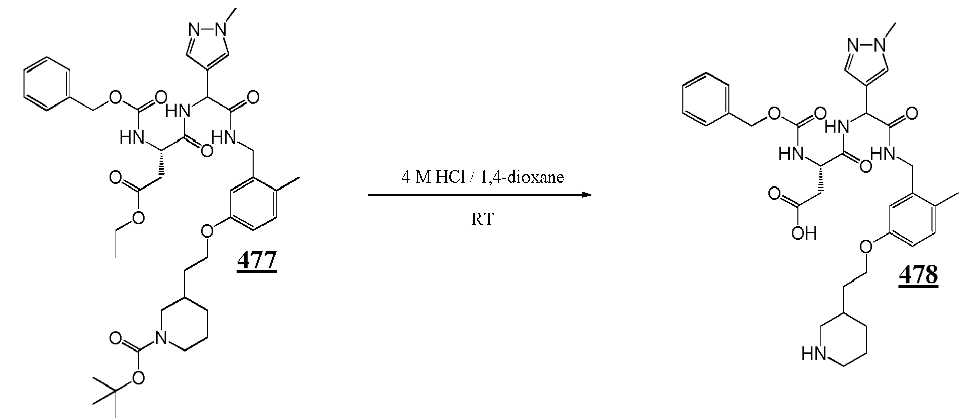

4.2 g ester **477** was dissolved in 40 ml 4 M HCl solution in 1,4-dioxane at room temperature. The solution was stirred until the reaction was complete. Volatiles were removed and dissolved in acetonitrile. After drying the crude was used in the following cyclization.
Formula: C₃₃H₄₂N₆O₇, exact mass: 634.3, found: 635.3 [M+H]⁺

### benzyl ((12S)-5⁴-methyl-9-(1-methyl-1H-pyrazol-4-yl)-8,11,14-trioxo-4-oxa-7,10-diaza-1(3,1)-piperidina-5(1,3)-benzenacyclotetradecaphane-12-yl)carbamate(479)

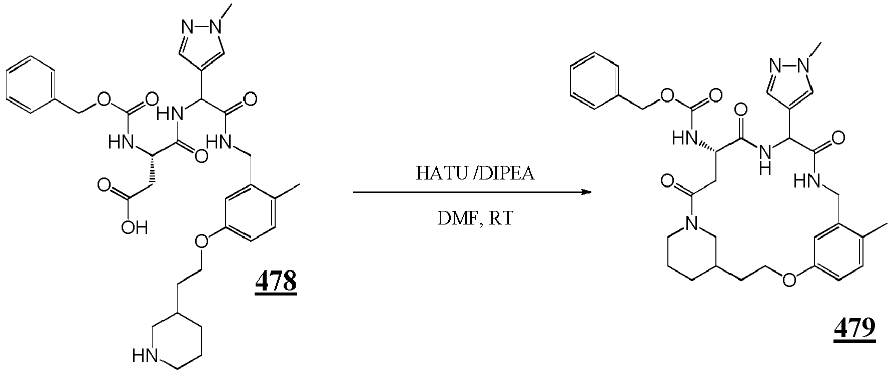

Macrocyclization was achieved according to general procedure A with 2.42 g amino acid **478.** Saturated NaHCO₃ solution was added to the reaction mixture and this was extracted with ethyl acetate. Organic phases were dried over MgSO₄ and concentrated under reduced pressure. Purification was achieved via normal phase column chromatography (silica, cyclohexane/ethyl acetate gradient).
Formula: C₃₃H₄₀N₆O₆, exact mass: 616.3, found: 617.3 [M+H]⁺

### (12S)-12-amino-5⁴-methyl-9-(1-methyl-1H-pyrazol-4-yl)-4-oxa-7,10-diaza-1(3,1)-piperidina-5(1,3)-benzenacyclotetradecaphane-8,11,14-trione (480)

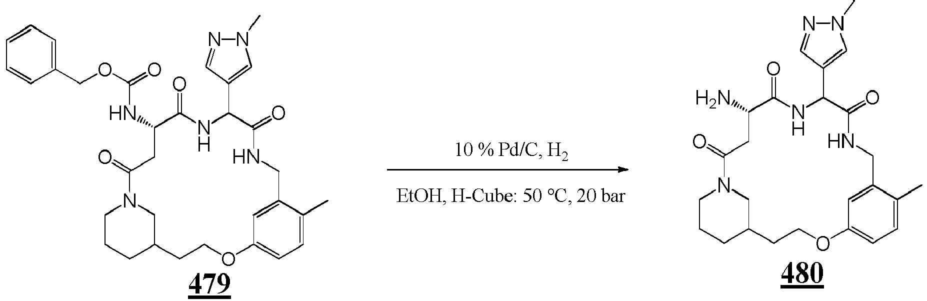

Cbz-group was removed according to general procedure C in EtOH at 50 °C and 20 bar H₂ pressure. Solvent was removed under reduced pressure to give a product pure enough to be used in the next reaction.
Formula: C₂₅H₃₄N₆O₄, exact mass: 482.3

### ethyl ((12S)-5⁴-methyl-9-(1-methyl-1H-pyrazol-4-yl)-8,11,14-trioxo-4-oxa-7,10-diaza-1(3,1)-piperidina-5(1,3)-benzenacyclotetradecaphane-12-yl)carbamate (363)

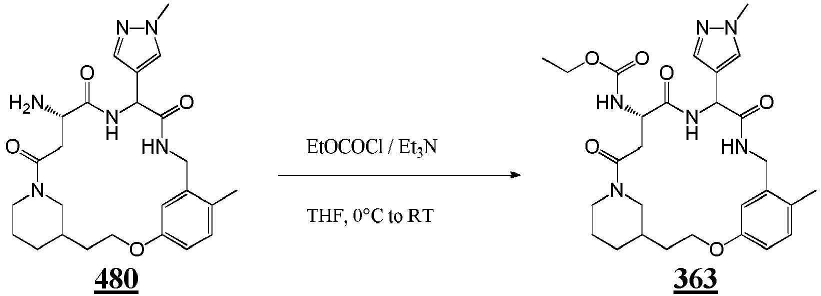

Compound **363** was synthesized according to general procedure G from 75 mg amine **480.** Final purification was achieved by HPLC.
Formula: C₂₈H₃₈N₆O₆, exact mass: 554.3, found: 555.3 [M+H]⁺

### N-((12S)-5⁴-methyl-9-(1-methyl-1H-pyrazol-4-yl)-8,11,14-trioxo-4-oxa-7,10-diaza-1(3,1)-piperidina-5(1,3)-benzenacyclotetradecaphane-12-yl)acetamide (552)

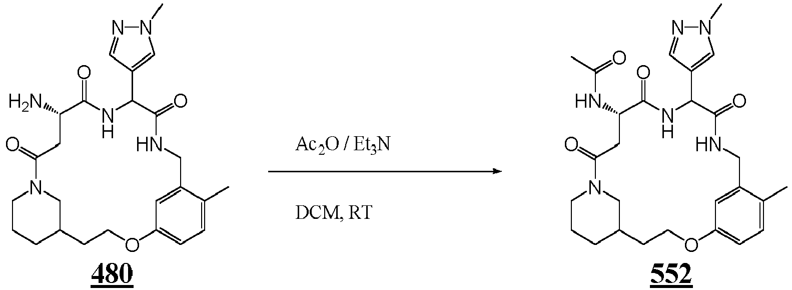

Compound **552** was synthesized from 75 mg amine **480.** Final purification was achieved by HPLC.
Formula: C₂₇H₃₆N₆O₅, exact mass: 524.3, found: 525.4 [M+H]⁺

### Amide derivatizations of compound 480 according to general procedure B

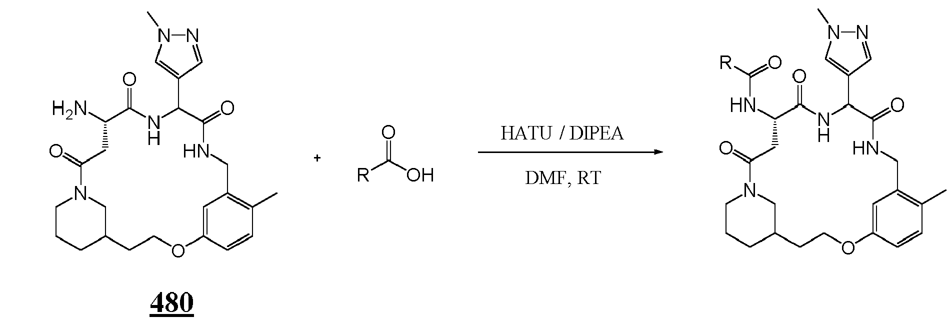

Amine **480** (e.g. 75 mg) was coupled with carboxylic acids according to general procedure B to give amides disclosed in table below. Purifications were achieved by HPLC.

**Table A-40**

| **Cpd no** | structure | name | Exact mass | [M+H]⁺ found |
|---|---|---|---|---|
| **358** | 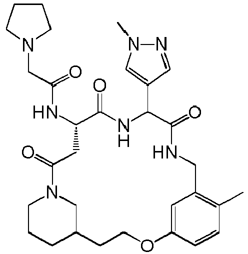 | N-((12S)-5⁴-methyl-9-(1-methyl-1H-pyrazol-4-yl)-8,11,14-trioxo-4-oxa-7,10-diaza-1(3,1)-piperidina-5(1,3)-benzenacyclotetradecaphane-12-yl)-2-(pyrrolidin-1-yl)acetamide | 593.3 | 594.4 |
| **359** | 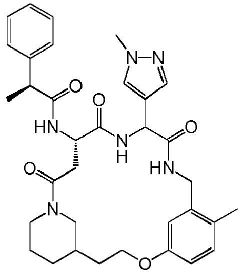 | (2S)-N-((12S)-5⁴-methyl-9-(1-methyl-1H-pyrazol-4-yl)-8,11,14-trioxo-4-oxa-7,10-diaza-1(3,1)-piperidina-5(1 ,3)-benzenacyclotetradecaphane-12-yl)-2-phenylpropanamide | 614.3 | 615.4 |
| **360** | 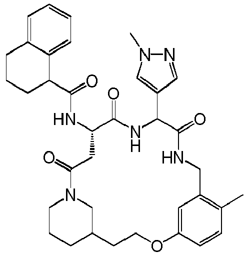 | N-((12S)-5⁴-methyl-9-(1-methyl-1H-pyrazol-4-yl)-8,11,14-trioxo-4-oxa-7,10-diaza-1(3,1)-piperidina-5(1 ,3)-benzenacyclotetradecaphane-12-yl)-1,2,3,4-tetrahydronaphthalene-1-carboxamide | 640.3 | 641.4 |
| **361** | 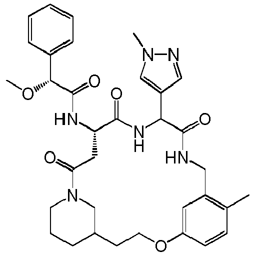 | (2R)-2-methoxy-N-((12S)-5⁴-methyl-9-(1-methyl-1H-pyrazol-4-yl)-8,11, 14-trioxo-4-oxa-7, 1 0-diaza-1(3,1)-piperidina-5(1,3)-benzenacyclotetradecaphane-12-yl)-2-phenylacetamide | 630.3 | 631.4 |
| **362** | 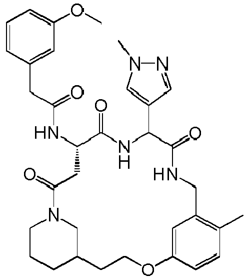 | 2-(3-methoxyphenyl)-N-((12S)-5⁴-methyl-9-(1-methyl-1H-pyrazol-4-yl)-8,11, 14-trioxo-4-oxa-7, 1 0-diaza-1(3,1)-piperidina-5(1,3)-benzenacyclotetradecaphane-12-yl)acetamide | 630.3 | 631.3 |

### Example A-37: Preparation of macrocyclic compound 495

### 4-(3-(2-(3-(isocyanomethyl)-4-methylphenoxy)ethyl)piperidin-1-yl)-4-oxobutanoic (481)

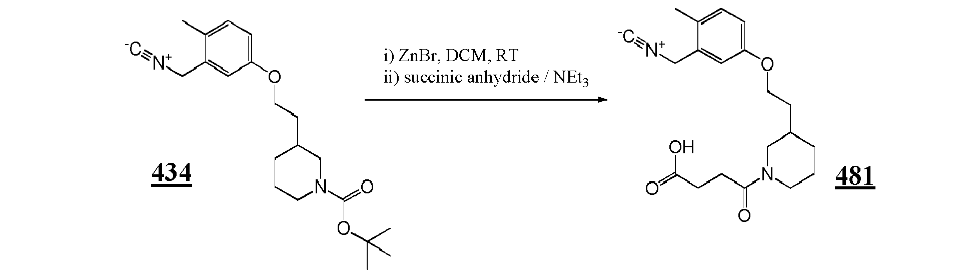

tert-butyl 3-(2-(3-(isocyanomethyl)-4-methylphenoxy)ethyl)piperidine-1-carboxylate (307 mg, 0.86 mmol, 1.0 eq.) was dissolved in DCM (10 mL) and zinc bromide (385 mg, 1.7 mmol, 2 eq.) was added to the solution. The reaction mixture was stirred for 72h to provide the free piperidine derivation in situ. MS (ES) C₁₆H₂₂N₂O requires: 258, found: 259 (M+H)+.

To the reaction mixture was added Et₃N (343 mg, 3.4 mmol, 4 eq.) and succinic anhydride (120 mg, 1.2 mmol, 1.4 eq.) and the reaction mixture was stirred for 2h. The reaction mixture was diluted with DCM and H₂O. The aqueous layer was adjusted to pH 2-4 using a 0.1 M HCl solution before it was extracted 3X with DCM. The combined organic layers were dried over MgSO₄ and the solvents were removed under reduced pressure.

The crude product was purified by flash chromatography on silica gel (0-100 % EtOAc/cHex, 0-100 % MeOH/DCM) to yield the title compound **481** (50 mg, 0.14 mmol, 16%). Compound **481** is very unstable and hydrolyses easily to the corresponding formamide.
MS (ES) C₂₀H₂₆N₂O₄ requires: 358 ,found: 359 (M+H)⁺.

### 5⁴-methyl-9-phenethyl-4-oxa-7,10-diaza-1(3,1)-piperidina-5(1,3)-benzenacyclotetradecaphane-8,11,14-trione (495)

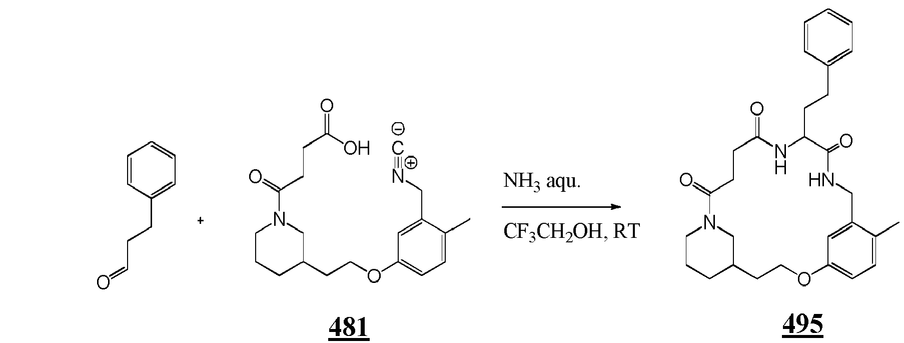

4-(3-(2-(3-(isocyanomethyl)-4-methylphenoxy)ethyl)piperidin-1-yl)-4-oxobutanoic acid (50 mg, 0.14 mmol, 1.0 eq.) was dissolved in 2,2,2-trifluoroethanol (1 mL), a solution of 3-phenylpropanal (52 mg, 0.40 mmol, 2.8 eq.) in 2,2,2-trifluoroethanol (1 mL), and aq. NH₃ (32%) (153 mg, 1.4 mmol, 10.0 eq.) were added to the solution. The reaction mixture was stirred for 10min at RT.

The reaction mixture was diluted with DCM and NaHCO₃ and the aqueous layer was extracted 3X with DCM before the combined organic layers were dried over MgSO₄ and the solvents were removed *in vacuo.*

The crude product was purified by flash chromatography on silica gel (0-100 % EtOAc/cHex, 0-100 % MeOH/DCM) and by a subsequent reversed-phase RP-HPLC (column: C18), using H₂O (0.1 %TFA) and ACN (0.1 %TFA) as eluents. The desired fractions were lyophilized to yield the compound **495** (30 mg, 0.06 mmol, 44%) as a white solid. MS (ES) C₂₉H₃₇N₃O₄ requires: 491, found: 492 (M+H)⁺.
¹H NMR (400MHz, d₆-DMSO, 300K) δ 8.42-7.70 (m, 2H), 7.33-7.24 (m, 2H), 7.24-7.13 (m, 3H), 7.06-7.00 (m, 1H), 6.99-6.66 (m, 2H), 4.77-3.72 (m, 6H), 3.10-2.54 (m, 4H), 2.54-2.26 (m, 3H), 2.20-2.12 (m, 3H), 2.10-0.97 (m, 11H).

### Example A-38: Preparation of macrocyclic compounds 503, 505, and 509 -513

### tert-butyl (2S)-2-(((benzyloxy)carbonyl)amino)-4-(3-(2-(3-(isocyanomethyl)-4-methylphenoxy)ethyl)piperidin-1-yl)-4-oxobutanoate (515)

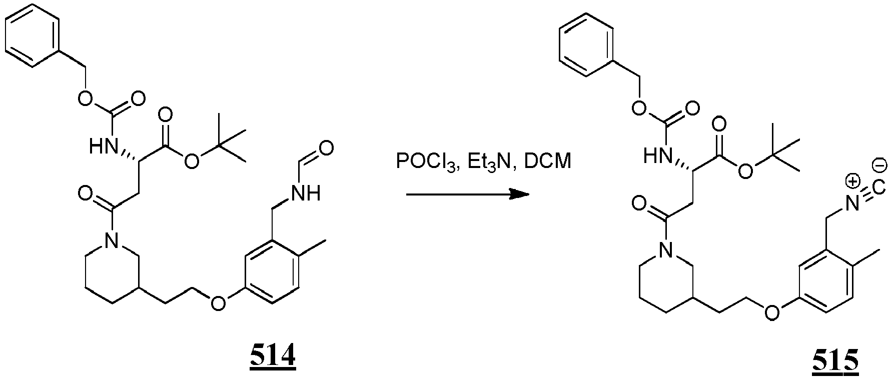

tert-butyl (2S)-2-(((benzyloxy)carbonyl)amino)-4-(3-(2-(3-(formamidomethyl)-4-methylphenoxy)ethyl)piperidin-1-yl)-4-oxobutanoate (100 mg, 0.172 mmol, 1.0 eq.) was dissolved in DCM (10 mL) and Et₃N (104 mg, 1.0 mmol, 6 eq.) was added to the solution. A solution of POCl₃ (53 mg, 0.34 mmol, 2 eq.) in DCM (1 mL) was added dropwise to the reaction mixture at 0°C. After 1h still 20 % of starting material was observed. An additional solution of POCl₃ (26 mg, 0.172 mmol, 1 eq.) in DCM (0.5 mL) was added dropwise to the reaction mixture at 0°C. The reaction was kept for further 30min.

The reaction mixture was diluted with DCM and NaHCO₃ and the aqueous layer was extracted 3X with DCM. The combined organic layers were dried over MgSO₄ and the solvents were removed under reduced pressure.

The crude product was purified by flash chromatography on silica gel (0-100 % EtOAc/cHex) to yield the title compound **515** (95 mg, 0.168 mmol, 98%). MS (ES) C₃₂H₄₁N₃O₆ requires: 563, found: 564 (M+H)⁺.

### (2S)-2-(((benzyloxy)carbonyl)amino)-4-(3-(2-(3-(isocyanomethyl)-4-methylphenoxy)ethyl)piperidin-1-yl)-4-oxobutanoicacid (516)

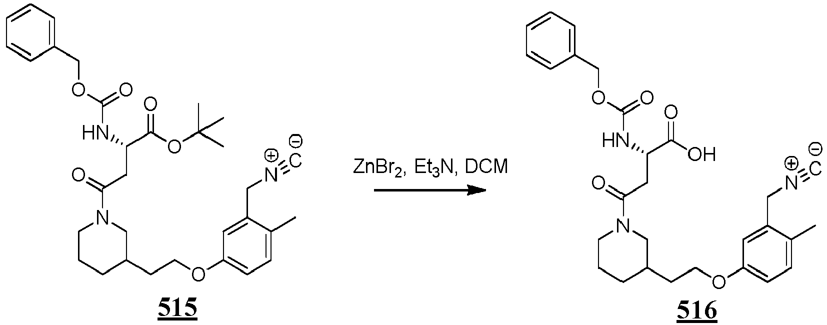

To a solution of tert-butyl (2S)-2-(((benzyloxy)carbonyl)amino)-4-(3-(2-(3-(isocyanomethyl)-4-methylphenoxy)ethyl)piperidin-1-yl)-4-oxobutanoate (1 g, 1.77 mmol, 1.0 eq.) in DCM (40 mL) was added Et₃N (358 mg, 3.54 mmol, 2 eq.) and zinc bromide (2 g, 8.9 mmol, 5 eq.). The reaction mixture was stirred for 16 h.

The reaction mixture was purified by reversed-phase chromatography (column: C18), using H₂O and ACN as eluents. The desired fractions were directly lyophilized to yield title compound **516** (672 mg, 1.32 mmol, 75%) as a white solid. MS (ES) C₂₈H₃₃N₃O₆ requires: 507, found: 508 (M+H)⁺.

### benzyl ((12S)-9-(2-(1,5-naphthyridin-3-yl)ethyl)-5⁴-methyl-8,11,14-trioxo-4-oxa-7,10-diaza-1(3,1)-piperidina-5(1,3)-benzenacyclotetradecaphane-12-yl)carbamate (511)

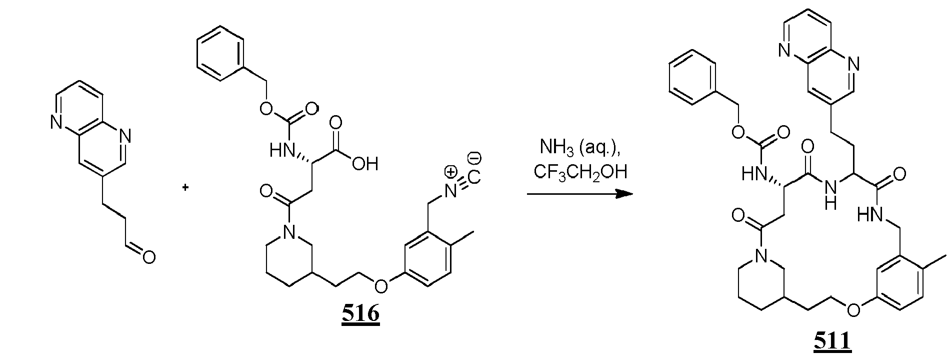

(2S)-2-(((benzyloxy)carbonyl)am ino)-4-(3-(2-(3-(isocyanomethyl)-4-methylphenoxy)ethyl)piperidin-1-yl)-4-oxobutanoic acid (77 mg, 0.15 mmol, 1.0 eq.) was dissolved in 2,2,2-trifluoroethanol (4 mL), 3-(1,5-naphthyridin-3-yl)propanal (39 mg, 0.21 mmol, 1.4 eq.) and aq. NH₃ (32%) (550 mg, 0.46 mmol, 3.0 eq.) were added to the solution. The reaction mixture was stirred for 12 h at RT.

The reaction mixture was diluted with DCM and NaHCO₃ and the aqueous layer was extracted 3X with DCM before the combined organic layers were dried over MgSO₄ and the solvents were removed in vacuo.

The crude product was purified by flash chromatography on silica gel (0-100 % EtOAc/cHex, 0-100 % MeOH/DCM) and by a subsequent reversed-phase RP-HPLC (column: C18), using H₂O (0.1 %TFA) and ACN (0.1 %TFA) as eluents to yield the title compound **511** (35 mg, 0.04 mmol, 29%) as a yellowish solid (TFA salt). MS (ES) C₃₉H₄₄N₆O₆ requires: 692, found: 693 (M+H)⁺.
¹H NMR (400MHz, d₆-DMSO, 300K) δ 9.14-8.71 (m, 2H), 8.63-7.56 (m, 5H), 7.52-6.96 (m, 7H), 6.98-6.54 (m, 2H), 5.22-4.87 (m, 2H), 4.68-3.62 (8H), 3.13-2.58 (m, 6H), 2.22-1.88 (m, 4H), 1.81-1.02 (m, 8H).

The examples in the following table were prepared according to the procedure described for compound **511.**

**Table A-41**

| **Cpd no** | structure | name | Exact mass | [M+H]⁺ found |
|---|---|---|---|---|
| **511** | 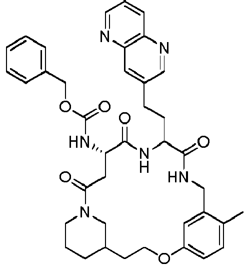 | benzyl ((12S)-9-(2-(1,5-naphthyridin-3-yl)ethyl)-5⁴-methyl-8,11,14-trioxo-4-oxa-7,10-diaza-1(3,1)-piperidina-5(1,3)-benzenacyclotetradecaphane-12-yl)carbamate | 692 | 693 |
| **503** | 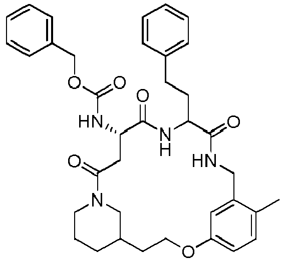 | benzyl ((12S)-9-(2-(1,5-naphthyridin-3-yl)ethyl)-54-methyl-8,11,14-trioxo-4-oxa-7,10-diaza-1(3,1)-pipehdina-5(1,3)-benzenacyclotetradecaphane-12-yl)carbamate | 640 | 641 |
| **505** | 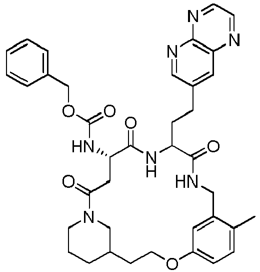 | benzyl ((12S)-5⁴-methyl-8,11,14-trioxo-9-(2-(pyrido[2,3-b]pyrazin-7-yl)ethyl)-4-oxa-7,10-diaza-1(3,1)-piperidina-5(1,3)-benzenacyclotetradecaphane-12-yl)carbamate | 693 | 694 |
| **509** | 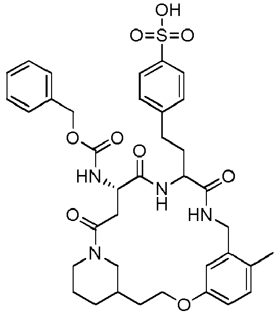 | 4-(2-((12S)-12-(((benzyloxy)carbonyl)amino)-5⁴-methyl-8,11,14-trioxo-4-oxa-7,10-diaza-1(3,1)-piperidina-5(1,3)-benzenacyclotetradecaphane-9-yl)ethyl)benzenesulfonic acid | 720 | 721 |
| **510** | 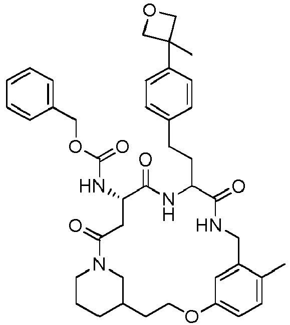 | benzyl ((12S)-5⁴-methyl-9-(4-(3-methyloxetan-3-yl)phenethyl)-8,11,14-trioxo-4-oxa-7,10-diaza-1(3,1)-piperidina-5(1,3)-benzenacyclotetradecaphane-12-yl)carbamate | 710 | 711 |
| **512** | 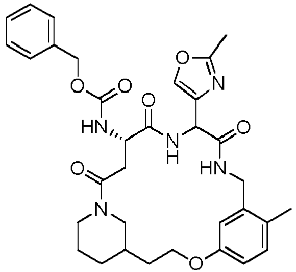 | benzyl ((12S)-5⁴-methyl-9-(2-methyloxazol-4-yl)-8,11,14-trioxo-4-oxa-7,10-diaza-1(3,1)-piperidina-5(1,3)-benzenacyclotetradecaphane-12-yl)carbamate | 617 | 618 |
| **513** | 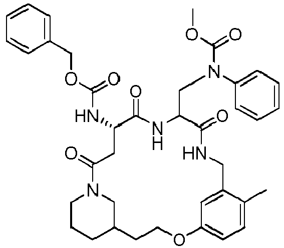 | methyl (((12R)-12-(((benzyloxy)carbonyl)amino)-5⁴-methyl-8,11,14-trioxo-4-oxa-7,10-diaza-1(3,1)-piperidina-5(1 ,3)-benzenacyclotetradecaphane-9-yl)methyl)(phenyl)carbamate | 699 | 700 |

### Example A-39: Preparation of macrocyclic compounds 565 -568

### N-((12S)-9-(1-benzyl-1H-pyrazol-4-yl)-5⁴-methyl-8,11,14-trioxo-4-oxa-7,10-diaza-1(3,1)-pipehdina-5(1,3)-benzenacyclotetradecaphane-12-yl)acetamide(565)

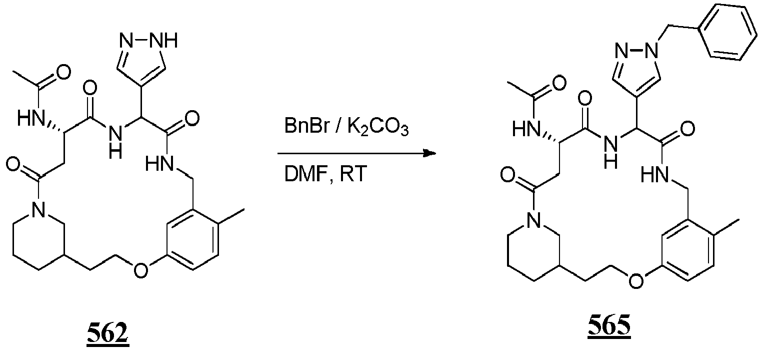

N-((12S)-5⁴-methyl-8,11,14-trioxo-9-(1H-pyrazol-4-yl)-4-oxa-7,10-diaza-1(3,1)-piperidina-5(1,3)-benzenacyclotetradecaphane-12-yl)acetamide **562** (50 mg, 0.098 mmol, 1.0 eq.) was dissolved in DMF (2 ml), potassium carbonate (41 mg, 0.29 mmol, 3.0 eq.), and benzyl bromide (21 mg, 0.12 mmol, 1.2 eq.) were added to the solution at RT and the reaction mixture was stirred for 12h.

The reaction mixture was diluted with EtOAc and NaHCO₃ and the aqueous layer was extracted 3X with EtOAc. The combined organic layers were dried over MgSO₄ and the solvent was removed *in vacuo.*

The crude product was purified by flash chromatography on silica gel (0-100 % EtOAc/ cHex, 0-100% MeOH/DCM) and by a subsequent reversed-phase RP-HPLC (column: C18), using H₂O (0.1 %TFA) and ACN (0.1 %TFA) as eluents. The desired fractions were lyophilized to yield title compound **565** (23.4 mg, 0.033 mmol, 34%) as a white solid (TFA salt). MS (ES) C₃₃H₄₀N₆O₅ requires: 600, found: 601 (M+H)⁺.
¹H NMR (400MHz, d₆-DMSO, 300K) δ 8.69-8.34 (m, 1H), 8.27-7.65 (m, 3H), 7.54-7.20 (m, 6H), 7.09-7.02 (m, 1H), 6.97-6.66 (m, 2H), 5.57-5.21 (m, 3H), 4.77-4.04 (m, 6H), 3.21-2.53 (m, 3H), 2.22-2.02 (m, 4H), 1.95-1.16 (m, 11H).

### Examples in the following table were prepared similar to the procedure described for 565.

**Table A-42**

| **Cpd No** | Structure | name | M | [M+H]⁺ |
|---|---|---|---|---|
| **566** | 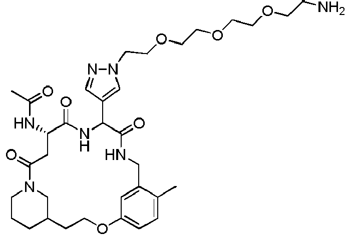 | N-((12S)-9-(1-(2-(2-(2-(2-aminoethoxy)ethoxy)ethoxy)ethyl)-1H-pyrazol-4-yl)-5⁴-methyl-8,11,14-trioxo-4-oxa-7,10-diaza-1(3,1)-piperidina-5(1,3)-benzenacyclotetradecaphane-12-yl)acetamide | 685 | 686 |
| **567** | 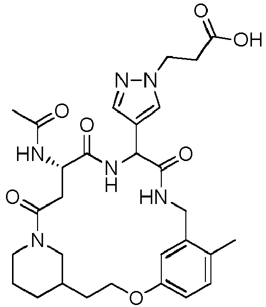 | 3-(4-((12S)-12-acetamido-5⁴-methyl-8,11,14-trioxo-4-oxa-7,10-diaza-1(3,1)-piperidina-5(1,3)-benzenacyclotetradecaphane-9-yl)-1H-pyrazol-1-yl)propanoic acid | 582 | 583 |
| **568** | 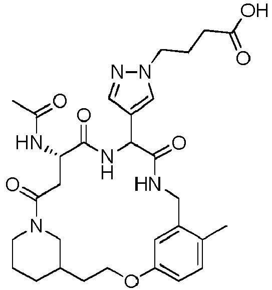 | 4-(4-((12S)-12-acetamido-5⁴-methyl-8,11,14-trioxo-4-oxa-7,10-diaza-1(3,1)-piperidina-5(1,3)-benzenacyclotetradecaphane-9-yl)-1H-pyrazol-1-yl)butanoic acid | 596 | 597 |

In order to obtain the examples **566, 567** and **568** subsequent standard acidic BOC (or tert-butyl)-deprotection according to general procedure D was performed.

### Example A-40: Preparation of macrocyclic compounds 570 -572

### N-((12S)-5⁴-methyl-8,11,14-trioxo-9-(1-(3-(tritylthio)propyl)-1H-pyrazol-4-yl)-4-oxa-7,10-diaza-1(3,1)-piperidina-5(1,3)-benzenacyclotetradecaphane-12-yl)acetamide (569)

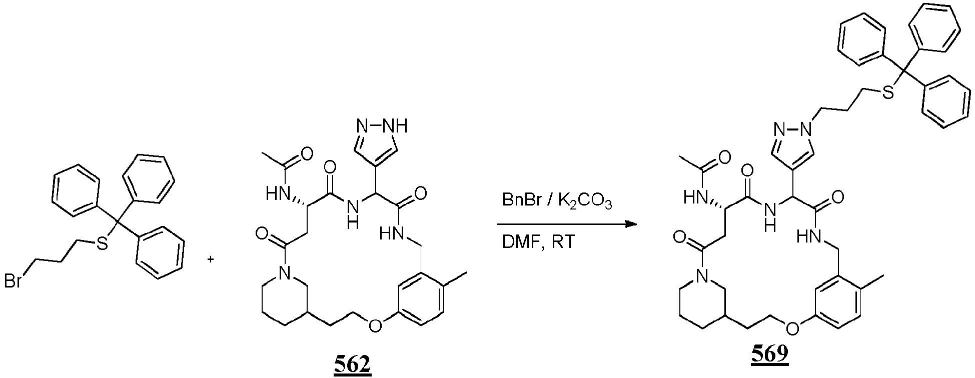

N-((12S)-5⁴-methyl-8,11,14-trioxo-9-(1H-pyrazol-4-yl)-4-oxa-7,10-diaza-1(3,1)-piperidina-5(1,3)-benzenacyclotetradecaphane-12-yl)acetamide **562** (100 mg, 0.20 mmol, 1.0 eq.) was dissolved in DMF (5 mL), potassium carbonate (81 mg, 0.59 mmol, 3.0 eq.), and (3-bromopropyl)(trityl)sulfane (187 mg, 0.47 mmol, 2.4 eq.) were added to the solution at RT and the reaction mixture was heated to 80°C and stirred for 18h.

The reaction mixture was diluted with DCM and NaHCO₃ and the aqueous layer was extracted 3X with DCM. The combined organic layers were dried over MgSO₄ and the solvent was removed in vacuo. The crude product was purified by flash chromatography on silica gel (0-100 % EtOAc/ cHex, 0-100% MeOH/DCM) and led to the desired title compound **569** (35.2 mg, 0.043 mmol, 22%). MS (ES) C₄₈H₅₄N₆O₅S requires: 826, found: 827 (M+H)⁺.

### N-((12S)-9-(1-(3-mercaptopropyl)-1H-pyrazol-4-yl)-5⁴-methyl-8,11,14-trioxo-4-oxa-7,10-diaza-1(3,1)-piperidina-5(1,3)-benzenacyclotetradecaphane-12-yl)acetamide (570)

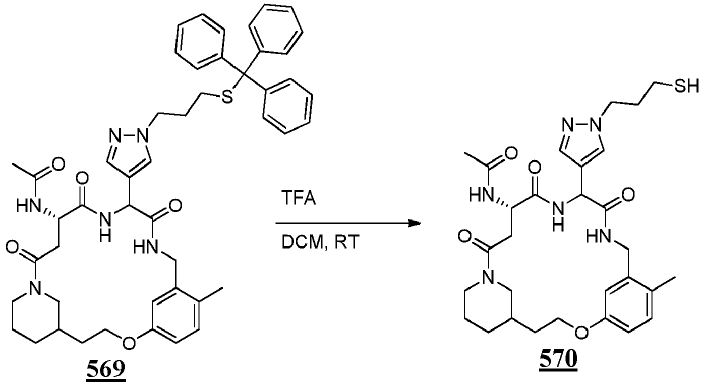

N-((12S)-5⁴-methyl-8,11,14-trioxo-9-(1-(3-(tritylthio)propyl)-1H-pyrazol-4-yl)-4-oxa-7,10-diaza-1(3,1)-piperidina-5(1,3)-benzenacyclotetradecaphane-12-yl)acetamide (35.2 mg, 0.043 mmol, 1.0 eq.) was dissolved in 25 % TFA/DCM (3 mL) and stirred for 30min at RT. Removing the solvents under reduced pressure led to the title compound **570** (30 mg, 0.043 mmol, quant.) as TFA salt. MS (ES) C₂₉H₄₀N₆O₅S requires: 584, found: 585 (M+H)⁺.

### 3-(4-((12S)-12-acetamido-5⁴-methyl-8,11,14-trioxo-4-oxa-7,10-diaza-1(3,1)-piperidina-5(1,3)-benzenacyclotetradecaphane-9-yl)-1H-pyrazol-1-yl)propane-1-sulfonic acid (571)

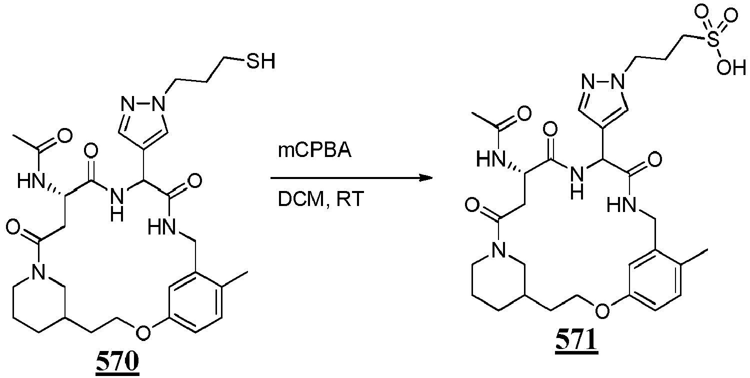

N-((12S)-9-(1-(3-mercaptopropyl)-1H-pyrazol-4-yl)-5⁴-methyl-8,11,14-trioxo-4-oxa-7,10-diaza-1(3,1)-piperidina-5(1,3)-benzenacyclotetradecaphane-12-yl)acetamide **570** (25 mg, 0.043 mmol, 1.0 eq.) was dissolved in DCM (5 mL) and 3-chlorobenzoperoxoic acid (45 mg, 0.26 mmol, 6.0 eq.) was added to the solution at 0°C and the reaction mixture was stirred for 12h at RT. Drops of H₂O were added to the reaction and the solvents were removed in vacuo.

The crude product was purified by reversed-phase RP-HPLC (column: C18), using H₂O (0.1 %TFA) and ACN (0.1 %TFA) as eluents. The desired fractions were lyophilized to yield the title compound **571** (7 mg, 0.009 mmol, 22%) as a yellowish solid (TFA salt). MS (ES) C₂₉H₄₀N₆O₈S requires: 632, found: 633 (M+H)⁺.

### N-(4-((12S)-12-acetamido-5⁴-methvl-8,11,14-trioxo-4-oxa-7,10-diaza-1(3,1)-piperidina-5(1,3)-benzenacyclotetradecaphane-9-yl)-1H-pyrazol-1-yl)ethane-1-sulfonic acid (572)

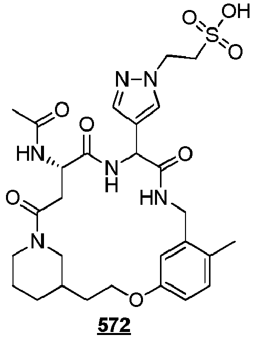

Compound 2-(4-((12S)-12-acetamido-5⁴-methyl-8,11,14-trioxo-4-oxa-7,10-diaza-1(3,1)-piperidina-5(1,3)-benzenacyclotetradecaphane-9-yl)-1H-pyrazol-1-yl)ethane-1-sulfonic acid **571** was prepared in analogy to compound **570.** MS (ES) C₂₉H₄₀N₆O₈S requires: 618, found: 619 (M+H)⁺.

### Biological Assays

### Example B-1: ß5 Inhibition Assay for the human constitutibe proeasome and the human immunoproteasome

### Assay Description constitutive proteasome and immunoproteasome

The enzymatic activity of the β5 subunit of the human constitutive proteasome (CPS) is analyzed. In another assay, which differs from the assay of the human constitutive proteasome only in using the human immunoproteasome (IPS) the enzymatic activity of the β5 subunit of the human immunoproteasome (IPS) is analyzed.

Incubation of the enzyme with its fluorogenic substrate Suc-LLVY-AMC leads to liberation of the fluorescent dye AMC which can be measured with a suitable plate reader.

### Reagents / Materials

| | | |
|---|---|---|
| Human cPS | E-360 | Boston Biochem or |
| Human IPS | E-370 | Boston Biochem |
| Suc-LLVY-AMC | I-1955 | Bachem |
| Bortezomib | 349320:01 | Proteros |
| Assay plate | 4514 | Corning |
| Assay Buffer | | |

- 100mM HEPES pH7,5
- 50mM NaCl
- 0,02% SDS
- 1mM DTT (add fresh)

### Microplate reader settings

Victor X5 (Perkin Elmer)
Label: Umbeliferone (0,1s)
Excitation wavelength: 355nm
Emission wavelength: 460nm

### Assay procedure (384 well plate)

- 4µl human cPS or IPS enzyme in assay buffer (final assay concentration 1nM) or 4µl assay buffer for negative controls
- Compound addition via acoustic dispenser (Echo 520, Labcyte)
- Mix and incubate @ RT for 10min
- 4µl Suc-LLVY-AMC substrate (final assay concentration 22.6µM)
- Mix and incubate @ RT for 4 h (dark)
- Stop reaction with 4µl Bortezomib (final assay concentration 1µM)
- 15min @ RT
- Measure (Excitation 355nm; Emission 460nm)

**Table 2** shows activity data in the Human cPS ß5 Inhibition Assay. Inhibition is indicated as IC₅₀ [nM] ("-" = not measured). Compounds having an activity designated as "A" provided an IC₅₀≤ 100 nM; compounds having an activity designated as "B" provided an 100 nM < IC₅₀≤ 500 nM; compounds having an activity designated as "C" provided an 500 nM < IC₅₀≤ 1000 nM; compounds having an activity designated as "D" provided an 1000nM < IC₅₀≤ 5000 nM; compounds having an activity designated as "E" provided an an IC₅₀> 5000nM.

**Table 2:**

| **Cpd** | **IPS[µM]** | **CPS [µM]** |
|---|---|---|
| **7** | A | A |
| **13** | A | A |
| **19** | A | A |
| **16** | A | A |
| **25** | A | A |
| **26** | A | A |
| **27** | A | A |
| **28** | A | A |
| **29** | A | A |
| **30** | A | A |
| **31** | A | A |
| **32** | A | A |
| **33** | A | A |
| **34** | A | A |
| **35** | A | A |
| **36** | A | A |
| **37** | A | A |
| **39** | A | A |
| **40** | A | A |
| **42** | A | A |
| **43** | A | A |
| **45** | A | A |
| **51** | A | A |
| **8** | A | A |
| **60** | A | A |
| **63** | A | A |
| **64** | A | - |
| **77** | A | A |
| **78** | B | D |
| **79** | A | B |
| **80** | A | A |
| **81** | A | A |
| **82** | A | A |
| **83** | A | A |
| **84** | A | A |
| **85** | A | A |
| **86** | A | A |
| **87** | A | A |
| **88** | A | A |
| **89** | A | A |
| **95** | A | A |
| **116** | B | E |
| **120** | A | A |
| **126** | A | A |
| **127** | A | A |
| **128** | A | A |
| **129** | A | A |
| **130** | A | A |
| **134** | A | A |
| **135** | A | A |
| **136** | A | A |
| **143** | A | A |
| **148** | A | D |
| **152** | A | A |
| **160** | A | A |
| **157** | A | A |
| **158** | A | A |
| **159** | A | A |
| **168** | D | E |
| **171** | A | B |
| **174** | A | A |
| **175** | A | A |
| **176** | A | A |
| **177** | A | A |
| **179** | A | A |
| **180** | A | A |
| **181** | A | A |
| **182** | A | A |
| **186** | A | A |
| **191** | A | C |
| **195** | A | A |
| **199** | A | A |
| **205** | A | A |
| **211** | A | B |
| **213** | A | A |
| **215** | A | A |
| **216** | A | A |
| **217** | A | A |
| **219** | A | A |
| **220** | A | A |
| **258** | A | B |
| **265** | B | - |
| **269** | A | D |
| **281** | D | D |
| **284** | A | A |
| **285** | A | A |
| **286** | A | A |
| **287** | A | A |
| **288** | A | A |
| **289** | B | B |
| **290** | C | D |
| **291** | A | - |
| **293** | A | - |
| **294** | A | A |
| **292** | A | - |
| **282** | C | E |
| **295** | A | A |
| **296** | B | C |
| **299** | C | E |
| **300** | A | B |
| **301** | C | D |
| **283** | A | C |
| **302** | A | A |
| **303** | A | A |
| **304** | A | A |
| **305** | A | A |
| **306** | A | A |
| **307** | A | A |
| **308** | A | A |
| **309** | A | A |
| **310** | A | A |
| **311** | A | A |
| **312** | B | E |
| **313** | C | E |
| **314** | A | A |
| **315** | A | A |
| **316** | A | A |
| **317** | A | A |
| **318** | A | A |
| **319** | A | A |
| **320** | A | A |
| **322** | A | A |
| **323** | A | A |
| **324** | A | A |
| **325** | A | A |
| **326** | A | A |
| **327** | A | A |
| **328** | A | A |
| **329** | A | A |
| **330** | A | A |
| **331** | A | A |
| **332** | A | A |
| **333** | A | A |
| **334** | A | A |
| **335** | A | A |
| **336** | A | A |
| **337** | A | A |
| **338** | A | A |
| **339** | A | A |
| **340** | A | A |
| **341** | A | A |
| **342** | A | A |
| **343** | A | A |
| **344** | A | A |
| **345** | A | A |
| **347** | A | A |
| **348** | A | A |
| **349** | A | A |
| **350** | A | A |
| **352** | A | A |
| **353** | A | A |
| **355** | A | D |
| **356** | A | C |
| **358** | A | B |
| **359** | A | A |
| **360** | A | A |
| **361** | A | A |
| **362** | A | A |
| **363** | A | A |
| **364** | A | A |
| **369** | A | A |
| **371** | A | A |
| **372** | A | A |
| **373** | A | A |
| **503** | A | A |
| **505** | A | A |
| **509** | A | A |
| **510** | A | A |
| **511** | A | A |
| **512** | A | A |
| **513** | A | A |
| **549** | A | A |
| **551** | A | A |
| **552** | A | A |
| **553** | A | A |
| **554** | A | A |
| **555** | A | A |
| **556** | A | A |
| **557** | A | A |
| **558** | A | A |
| **559** | A | A |
| **560** | A | A |
| **561** | A | A |
| **562** | A | A |
| **563** | A | A |
| **564** | A | - |
| **565** | A | - |
| **566** | A | A |
| **567** | A | A |
| **568** | A | A |
| **571** | A | A |

### Example B-2: CellTiter-Glo Luminescent Cell Viability Assay Cell Titer Glo Viability Assay with HT-29 cells (72h incubation)

The CellTiter-Glo Luminescent Cell Viability Assay (Promega) is a homogeneous method of determining the number of viable cells in culture. It is based on quantification of ATP, indicating the presence of metabolically active cells.

On day 1 400 HT-29 cells per well are seeded in white 384well plates (Greiner, # 781080) in 25µl cell culture medium (DMEM (PAN-Biotech # P04-03590) + 10% FCS + glutamine). After incubation for 24h at 37°C/5% CO₂ compounds or DMSO are added at different concentrations by Echo Liquid Handling Technology. Cells are further incubated in humidified chambers for 72 h at 37°C and 5% CO₂. Cells treated with the compound vehicle DMSO are used as positive controls and cells treated with 10 µM Staurosporine serve as negative controls.

At day 5 - 72h after compound addition - the CellTiter Glo Reagent is prepared according to the instructions of the kit (Promega Inc.): Reagent is mixed 1:1 with cell culture medium. Thereon, mixture and assay plates are equilibrated at room temperature for 20 min. Equal volumes of the reagent-medium-mixture is added to the volume of culture medium present in each well. The plates are mixed at ~300 rpm for 2 minutes on an orbital shaker. The microplates are then incubated at room temperature for 10 minutes for stabilization of the luminescent signal. Following incubation the luminescence is recorded on a Victor microplate reader (Perkin Elmer) using a 200 ms integration time. The data is then analyzed with Excel using the XLFIT Plugin (dose response Fit 205) for IC₅₀-determination.

As quality control the Z'-factor is calculated from 16 positive and negative control values. Only assay results showing a Z'-factor ≥ 0.5 are used for further analysis.

**Table 3** shows activity data in the biochemical Glo cell viability assays of A549 (human lung carcinoma *cell* line) and Karpas and CTG assay of HT29 (human colone cancer cell line) cell. Inhibition is indicated as IC₅₀ [nM] ("-" = not measured). Compounds having an activity designated as "A" provided an IC₅₀≤ 100 nM; compounds having an activity designated as "B" provided an 100 nM < IC₅₀≤ 500 nM; compounds having an activity designated as "C" provided an 500 nM < IC₅₀≤ 1000 nM; compounds having an activity designated as "D" provided an 1000nM < IC₅₀≤ 5000 nM; compounds having an activity designated as "E" provided an an IC₅₀> 5000nM.

**Table 3:**

| **Cpd No.** | **PGLo A549 [µM]** | **Pglo Karpas [µM]** | **CTG HT29 [µM]** |
|---|---|---|---|
| **7** | A | | A |
| **8** | C | A | B |
| **13** | C | | C |
| **19** | D | C | D |
| **16** | C | | B |
| **22** | - | | D |
| **25** | A | | A |
| **26** | A | | A |
| **27** | A | | A |
| **28** | A | A | A |
| **29** | A | | A |
| **30** | A | | A |
| **31** | A | | A |
| **32** | A | | A |
| **33** | A | | A |
| **34** | A | | A |
| **35** | B | | B |
| **36** | B | | B |
| **37** | A | | A |
| **38** | | | A |
| **39** | A | | A |
| **40** | A | | A |
| **41** | | | A |
| **42** | A | | A |
| **43** | B | | A |
| **44** | | | A |
| **45** | B | | B |
| **51** | B | | D |
| **59** | ND | | D |
| **60** | B | B | A |
| **61** | | | A |
| **62** | | | A |
| **63** | A | A | A |
| **64** | A | A | A |
| **77** | C | B | D |
| **78** | E | B | |
| **79** | D | C | D |
| **80** | A | A | A |
| **81** | A | A | A |
| **82** | A | A | A |
| **83** | A | A | A |
| **84** | A | A | A |
| **85** | A | A | A |
| 86 | A | A | A |
| **87** | A | A | A |
| **88** | A | A | A |
| **89** | A | A | A |
| **95** | D | | C |
| **107** | - | | A |
| **116** | E | | B |
| **120** | D | | B |
| **126** | A | | B |
| **127** | A | | A |
| **128** | A | | A |
| **129** | A | | A |
| **130** | B | | B |
| **134** | A | | A |
| **135** | A | | A |
| **136** | A | | B |
| **143** | B | | B |
| **148** | E | | B |
| **151** | | | A |
| **152** | A | | A |
| **160** | B | | A |
| **157** | B | | A |
| **158** | A | | A |
| **159** | B | | A |
| **168** | - | | B |
| **171** | C | | B |
| **172** | - | | A |
| **173** | - | | B |
| **174** | A | | A |
| **175** | A | | A |
| **176** | A | | A |
| **177** | A | | A |
| **179** | A | | A |
| **180** | A | | A |
| **181** | A | | A |
| **182** | A | | A |
| **186** | B | | B |
| **191** | D | | D |
| **195** | B | | A |
| **199** | A | | A |
| **205** | A | | A |
| **211** | C | | B |
| **213** | B | | A |
| **215** | B | | A |
| **216** | B | | A |
| **217** | A | | A |
| **218** | | | A |
| **219** | A | | A |
| **220** | A | | A |
| **252** | | | B |
| **253** | | | B |
| **254** | | | B |
| **255** | E | | B |
| **256** | | | B |
| **257** | | | B |
| **258** | C | | B |
| **259** | | | B |
| **260** | | | B |
| **261** | | | B |
| **265** | E | | B |
| **269** | D | | D |
| **281** | D | | B |
| **284** | B | | A |
| **285** | B | | A |
| **286** | B | | A |
| **287** | A | | A |
| **288** | B | | A |
| **289** | C | | B |
| **290** | E | | B |
| **291** | D | | A |
| **293** | A | | A |
| **294** | A | | A |
| **292** | A | | A |
| **282** | E | | B |
| **295** | B | | A |
| **296** | C | | B |
| **298** | | | B |
| **299** | E | | B |
| **300** | D | | B |
| **301** | D | | B |
| **283** | D | | B |
| **302** | A | | A |
| **303** | | | A |
| **304** | | | A |
| **305** | | | A |
| **306** | | | A |
| **307** | | | A |
| **308** | | | A |
| **309** | A | | A |
| **310** | A | | A |
| **311** | A | | A |
| **312** | | | B |
| **313** | | | B |
| **314** | A | | A |
| **315** | | | A |
| **316** | | | A |
| **317** | | | A |
| **318** | | | A |
| **319** | | | A |
| **320** | A | | A |
| **322** | D | | B |
| **323** | A | | A |
| **324** | A | | A |
| **325** | A | | A |
| **326** | A | | A |
| **327** | B | | A |
| **328** | B | | A |
| **329** | B | | A |
| **330** | A | | A |
| **331** | A | | A |
| **332** | A | | A |
| **333** | A | | A |
| **334** | B | | A |
| **335** | A | | A |
| **336** | A | | A |
| **337** | B | | A |
| **338** | B | | A |
| **339** | B | | A |
| **340** | A | | B |
| **341** | A | | A |
| **342** | A | | A |
| **343** | A | | A |
| **344** | A | | A |
| **345** | B | | A |
| **347** | B | | A |
| **348** | A | | A |
| **349** | B | | B |
| **350** | A | | A |
| **351** | B | | B |
| **352** | A | | A |
| **353** | A | | A |
| **354** | | | B |
| **355** | E | | D |
| **356** | C | | C |
| **358** | A | | B |
| **359** | A | | A |
| **360** | A | | A |
| **361** | B | | A |
| **362** | A | | A |
| **363** | A | | A |
| **364** | A | | A |
| **369** | A | | A |
| **371** | A | | A |
| **372** | A | | A |
| **373** | A | A | |
| **495** | | | B |
| **503** | B | | A |
| **571** | A | | C |
| **505** | A | | A |
| **572** | | | B |
| **509** | A | | B |
| **510** | A | | A |
| **511** | A | | A |
| **512** | A | | A |
| **513** | A | | A |
| **549** | B | | A |
| **550** | | | A |
| **551** | A | | A |
| **552** | B | | A |
| **553** | B | | A |
| **554** | B | | A |
| **555** | A | | A |
| **556** | A | | A |
| **557** | A | | B |
| **558** | B | | A |
| **559** | B | | A |
| **560** | B | | A |
| **561** | A | | A |
| **562** | B | | B |
| **563** | A | | A |
| **564** | A | | A |
| **565** | A | | A |
| **566** | A | | B |
| **567** | A | | B |
| **568** | A | | B |

### Example B-3 : Chymotrypsin-like ProteasomeGlo assay

Chymotrypsin-like ProteasomeGlo assay in the B-cells isolated from PBMC's following compound addition (IC₅₀).
1. PBMC's will be isolated from human blood followed by B-cell isolation from the PBMC's using the Miltenyi Isolation Kit.
2. This experiment will use the Chymotrypsin-like ProteasomeGlo read-out to generate IC50's folowing compound treatment.
3. Dilute all compounds 1:100 for the assay.
4. Do not make a staurosporine transfer for lane 12! Repeat the DMSO transfer with lane 12.
5. Lane 12 should not be seeded with any cells. Only medium!
6. For all steps, make sure the samples and reagents remain cool!
7. While dealing with the buffy coats, be careful to not let any exposed skin to be in direct contact with the blood.

### Reagents

Separating medium
Lymphocyte Separating Medium (Pancoll) (PAN Biotech; P04-60125)
RBC Lysis Buffer (10x) (BioLegend; 420301)
Prepare 1x RBC Lysis Buffer by diluting 10x RBC Lysis Buffer to 1x with ddH₂O.

### PBMC cell medium

| **Component** | **Manufacturer information** | **Concentration** | **Volume (ml)** | **Final concentration** |
|---|---|---|---|---|
| RPMI 1640 | PAN Biotech; P04-22100 | | 500 | |
| HyClone Heat inactivated FCS | Thermo Scientific; SV30180.03 (Lot No.: TXK40002) | 100 % | 50 | 10 % |
| L-Glutamine | PAN Biotech; P04-80100 | 100x | 5 | 1X |

### Cultivation medium for B-cells

| **Component** | **Manufacturer information** | **Concentration** | **Volume (ml)** | **Final concentration** |
|---|---|---|---|---|
| DMEM | Gibco; 41965039 | | 500 | |
| Heat inactivated FCS | Capricon; FBS-11A | 100% | 50 | 10% |
| L-Glutamine | PAN Biotech; P04-80100 | 100x | 5 | 1X |

### Wash buffer

| **Component** | **Manufacturer information** | **Concentration** | **Volume (ml)** | **Final concentration** |
|---|---|---|---|---|
| DPBS | Gibco; 14190136 | | 178 | 89 % |
| DMEM | Gibco; 41965039 | | 20 | 10 % |
| Heat inactivated FCS | Capricon; FBS-11A | 100 % | 2 | 1 % |

### B cell isolation buffer

| **Component** | **Manufacturer information** | **Concentration** | **Final concentration** | **Amount** | **Unit** |
|---|---|---|---|---|---|
| DPBS | Gibco; 14190136 | | | 300 | ml |
| Bovine Serum albumin (BSA) | | | 0.50% | 1.5 | 9 |
| EDTA Solution | Self-made | 250 mM | 2 mM | 2.4 | ml |

Chymotrypsin-Like Cell-Based Proteasome-Glo^{™} Assay (Promega; G8661)
Cell culture 384-well plates (Greiner BioOne; 781080)
Dispensing cassette, MultiDrop Standard tube dispensing cassette (Thermo Scientific; 24072670)
DPBS (Pan BioTech; P04-36500)
Cell culture 384-well plates (Greiner BioOne; 781080)
CPD source plate: diamond-shape plate (Labcyte; LP-0200)
Intermediate plate (Labcyte; P-05525)

| | |
|---|---|
| **Day 1** | Seed A549 cells |
| **Day 2** | Seed Karpas cells and ProteasomeGlo assay |

### B cell Isolation Kit II, human (Miltenyi Biotec; 130-091-151)

includes,
**1. QuadroMACS^{™} Starting Kit (LS) (#130-091-051)**
QuadroMACS^{™} Separator ((#130-090-976)
MACS MultiStand ((#130-042-303)
LS Columns (#130-042-401)
MACS^{®} 15 mL Tube Rack (#130-091-052)
One unit of MACS Microbeads, or one MACS
Microbead Kit, or one MACS Cell Isolation Kit.
**2. B cell Isolation Kit II**

| | |
|---|---|
| Components: | - 1mLB Cell Biotin-Antibody Cocktail, human: Cocktail of biotin-conjugated monoclonal antibodies against CD2, CD14, CD16, CD36, Cd43, and CD235a (Glycophorin A)- |
| | - 2 mL Anti-Biotin Microbeads: Microbeas conjugated to monoclonla anti-biotin antibodies (isotype: mouse IgG1). |
| Capacity | For 10⁹ total cells |
| Product format | All components are supplied in buffer containing stabilizer and 0.05 % sodium azide. |
| Storage | Store protected from light at 2-8 °C. Do not freeze. The expiration date is indicated on the vial labels. |

### Components of the Miltenyi Biotec Starting and B cell Isolation Kit

- MACS^{®} MultiStand
- LS Columns
- QuadroMACS^{™} Separator

### Method

| | |
|---|---|
| **Day 1:** | PBMC isolation followed by B-cell isolation using the Miltenyi Kit |
| **Day 2:** | B-cell plating, ECHO transfer and ProteasomeGlo assay |

### Day 1

**1. Prepare the wash buffer and cultivation medium for the assay.**
a. Cultivation medium: Prepare and store at 4°C. The cultivation medium will be used at the end of B-cell isolation for overnight incubation of the B-cells and subsequent experiment.
b. Wash buffer: Prepare fresh before each assay taking into care to be exact with the volumes prepared.
   ~ 200ml of the wash buffer is required per B-cell isolation from PBMCs.
c. Buffer for B cell isolation: Prepare the buffer fresh before each assay as it contains BSA which is unstable in a solution for long term storage. Cool the buffer own on ice before use!
d. PBMC cell medium: Prepare and store at 4°C. The medium is to be pre-warmed before starting the PBMC isolation from the buffy coats.
**2. Assemble the components of the Miltenyi Separator** + **MACS MultiStand as follows:**
a. Attach the QuadroMACS Separator to the MACS MultiStand by placing the separator adjacent to the MultiStand and slowly sliding the separator onto the stand. not to trap your fingers while attaching the two components together.
b. Check that the ejection blocks in the gap pf the magnet (separator) are attached before placing the MACS LS columns into the magnetic field of the separator.
c. Place the LS Columns with the column wings to the front into the slots of the separator.
**3. PBMC isolation from buffy coats** For the initial steps and to make processing the buffy coats easier, separately process the buffy coats.
a. Cut the tube attached to the buffy coats carefully using a clean, sterile and fresh scalpel.
b. Transfer the blood into 2 individual 50ml falcons.
c. Add an equal volume of DPBS to the blood in the falcons.
d. Add ~20ml of lymphocyte separating medium to two 50ml falcons.
e. Carefully pour 15-20ml of the blood + DPBS mix from step c. onto the lymphocyte separating medium from step d.
f. Centrifuge the falcons with the lymphocyte separating medium + blood mix at 800xg for 15minutes using a swing-out rotor.
g. Following centrifugation, carefully remove the falcons from the centrifuge.
h. The following layers will be observed:
i. Aspirate the plasma layer off carefully without disturbing the different layers.
j. Remove the cell pellet using a pipette into two fresh 50ml falcons.
k. Fill the falcons with the cells upto 50ml with DPBS.
l. Centrifuge the falcons at 250xg for 10minutes using a swing-out rotor (Brake-off).
m. Aspirate the complete DPBS off the cells without disturbing the cell pellet at the bottom of the falcon.
n. Re-suspend the cell pellet in 10ml of 1x RBC lysis buffer.
o. Incubate the falcons at room temperature in the dark for 10minutes.
p. After RBC lysis, centrifuge the tubes at 250xg for 10minutes using a swing-out rotor (brake-off).
   **(*Note:** If the cell pellet is still red or light red, repeat steps n. to p.!)
q. Re-suspend the cell pellet in DPBS up to 50ml in the falcon.
r. Centrifuge the falcons at 250xg for 10minutes using a swing-out rotor (Brake-off).
s. Aspirate the DPBS off the cells without disturbing the cell pellet at the bottom of the falcon.
t. Repeat the wash step as described in steps q. to s. 2x times more (total number of washes is 3x).
u. Aspirate the DPBS completely off the cell pellet after the last wash without disturbing the cell pellet.
v. Re-suspend the cell pellet in 20ml of PBMC cell medium.
w. Proceed to counting of cells and B-cell isolation from PBMCs.
   **(*Note:** Keep the cells isolated from the two donors or more always separate and do not pool the cells from multiple donors at any step!(
**4. B cell isolation from PBMCs**
a. Collect the PBMCs and re-suspend the entire amount of cells into 30ml of wash buffer in a 50ml falcon.
b. Count the PBMCs using the CEDEX.

| | Patient 1 (719) | Patient 2 (724) |
|---|---|---|
| Number of PBMC/ml | 17774000 | 13101000 |
| Total volume of suspension | 40 ml | 40 ml |
| Total number of PBMCs | 710960000 | 524040000 |

c. Leave the cell suspension at room temperature for 30mins.
d. Centrifuge the cell suspension at the end of 30 minutes for 10mins at 1500rpm.
e. Aspirate the wash buffer carefully from the cells without disturbing the pellet at the bottom of the falcon.
f. Re-suspend the pellet in volume of the B-cell isolation buffer calculated as follows:

| | | | |
|---|---|---|---|
| Volume of B-cell isolation buffer per 10⁷ total cells | 40 µl | | 40 µl |
| Total number of cells | 710960000 | | 524040000 |
| Total volume of B-cell isolation buffer needed for resuspension | 2843.84 µl | | 2096.16 µl |

g. Add the calculated volume of Biotin-Antibod Cocktail as follows:

| | | | |
|---|---|---|---|
| Volume of Biotin-Antibody cocktail per 10⁷ total cells | 10 µl | | 10 µl |
| Total number of cells | 710960000 | | 524040000 |
| Total volume of Biotin-Antibody cocktail to be added | 710.96 µl | | 524.04 µl |

h. Mix well using a 1ml pipette and incubate on ice for 10mins.
i. Add more B-cell isolation buffer to the cell + antibody suspension as follows:

| | | | |
|---|---|---|---|
| Volume of B-cell isolation buffer per 10⁷ total cells | 30 µl | | 30 µl |
| Total number of cells | 710960000 | | 524040000 |
| Total volume of B-cell isolation buffer to be added | 2132.88 µl | | 1572.12 µl |

j. Add the calculated volume of anti-Biotin Microbeads to the cell + antibody mix as follows:

| | | | |
|---|---|---|---|
| Volume of anti-Biotin microbeads per 10⁷ total cells | 20 µl | | 20 µl |
| Total number of cells | 710960000 | | 524040000 |
| Total volume of Biotin-Antibody cocktail to be added | 1421.92 µl | | 1048.08 µl |

k. Mix well using a 1ml pipette and incubate the mix on ice for 20mins.
l. In the meantime, equilibrate one LS Column as follows:
1. Insert one LS Column into one slot of the separator as depicted in the pictures above.
2. Place a 50ml falcon at the bottom of the LS column to collect the wash flow-through.
3. Add 3ml of the B-cell isolation buffer into the column.
4. Allow the buffer to flow-through making sure that the entire volume passes the column and that the column reservior is empty before the addition of the cells.
5. Remove the falcon with the flow-through and discard.
m. Place a fresh 50ml falcon under the LS Column to collect the untouched B-cells isolated from the PBMCs.
n. Apply the entire volume of the cell + antibody + microbeads mix to the LS Column.
o. Collect the flow-through from each application of the mix into the same 50ml falcon under the LS Column.
p. Once the complete volume of the cell + antibody + microbead mix has been passed through, proceed to washing of the column.
q. Add 3ml of the B-cell isolation buffer to the LS Column and allow the buffer to flow-through. Collect the flow-through and repeat the wash 2x times more (in total 3x washes).
r. Centrifuge the B-cells isolated in the collection falkan at 1500rpm and 4°C for 15mins.
s. Aspirate the B-cell isolation buffer off the pellet keeping in mind the relatively low attachment of the B-cells to tubes and that the B-cells are not aspirated off.
t. Re-suspend the B-cell pellet in 1000 µl of fresh B-cell isolation buffer.
u. Collect 50µl of the B-cell suspension into a fresh, labelled eppi and add 25 µl of 3x Laemmli buffer to the cell suspension (Sample)
v. Make up the volume of the B-cell suspension to 10ml with B-cell cultivation medium and store the suspension overnight at 4°C.
w. Place a fresh 2ml eppendorf under the LS Column. Remove the column from the separator and carefully depress the plunger through the column, collecting the unwanted cells removed from the B-cells (Pass).
x. Add 100 µl of 3x Laemmli buffer to the Pass from step u and 25 µl of 3x Laemmli buffer to the Input collected in step f.
y. Boil all three samples (Input, Sample and Pass) at 98°C for 15mins.
z. Proceed to western blotting from the samples prepared.
a1. For the B-cell suspension stored overnight, proceed to B-cell counting and plating for cell number titration on Day 2.

### Day 2

Proceed directly with the cell suspension from Day 1 for cell counting.
1. Count the cells using the CEDEX

| | | |
|---|---|---|
| Cell number required per well | 10.000 | /25µl |
| Number of cells/ml | 400.000 | |
| Number of wells | 384 | |
| Total number of plates | 3 | |
| Volume of cell suspension to be seeded per well | 25 | µl |
| Total volume of cell suspension required | 28800 | µl |
| Total Volume of cells required | 20000000 | |

| | **B-cells** | | **B-cells** |
|---|---|---|---|
| Number of cell/ml | 21777000 | | 14331000 |
| Number of cells/ml | | | |
| Total volume of cells suspension | 0.91840015 ml | | 1.39557602 ml |
| Volume of medium required | 49.0815999 ml | | 48.604424 ml |

2. Using a multi-channel pipette, pipette 25 µl of the cell suspensions
3. Place the cells **for 3hrs** in the high humidity incubator at 37°C and 5% CO₂.

### ECHO Compound transfer

**Destination plate** refers to the plates onto which the compounds will be transferred (meaning the plates with the cells already plated).
**1. Compound plate from compound management:**
   a. Pipette 2x 8µl of the compounds (10mM or 1mM stock solutions) into rows A1-P1, A2-P2 and A3-L3 of a diamond shaped plate.
   b. Once pipetted, seal the compound plate with an aluminum foil seal and spin down briefly. Place the plate in the dark till further use.
**2. DMSO intermediate plate:**
   a. Pipette 25µl of DMSO into columns 1-12 of an intermediate plate.
   b. Seal the lanes 1-12 using an aluminum foil seal.
   c. Spin the plate down briefly and store till further use.
**4. ECHO transfer**
   a. Remove the plates with cells from the incubator.
   b. Take all the plates including the compound plate, intermediate plate and Staurosporine plate to the ECHO.
   c. Click on "Open" in the ECHO software and select the protocol "QLI5_25ul_30uM-Grenier".
   d. Follow the on-screen prompts and instructions once you click "Start" to insert the compound plate, intermediate plate and destination plates.
   e. Change the final number of destination plates on the program.
   f. Follow the on-screen prompts and instructions once you click "Start" to load the Staurosporine and destination plates.
   g. Once all the compounds, DMSO and Staurosporine have been transferred, place the destination plates in a wet tissue box.
   h. Place the destination plates back into the incubator and incubate at 37°C, 5% CO₂ for 105 minutes.
   i. Re-seal all the compound + Stauro diamond and intermediate plates and store in the dark.

### Chymotrypsin like ProteasomeGlo Assay

1. Thaw the components of the ProteasomeGlo assay at room temperature including the ProteasomeGlo Buffer, the lyophilized Luciferin Detection Reagent and the Suc-LLVY-Glo Substrate.
2. Reconstitute the Luciferin Detection Reagent in the amber coloured bottle by adding 50ml of the ProteasomeGlo Buffer.
3. Vortex the Suc-LLVY-Glo Substrate briefly to reconstitute the Substrate well.
4. Spin down the Substrate and add 250µl of the Substrate to the reconstituted Luciferin Detection Reagent from step 2.
5. Briefly vortex the amber bottle with the mix to prepare a homogenous solution.
6. Place the mix prepared in step 5 to sit at room temperature for 60 minutes.
7. After 60minutes, pipette 25µl of the mix from step 5 into all wells with the cells.
8. Shake the plates for 2-5minutes to ensure the complete mixing of the ProteasomeGlo reagent with the cells.
9. Leave the plates in the dark for 10-15minutes.
10. Measure the luminescence read-out using the Viktor and the protocol "Luminescence_384Well_0.2sec".

**(Note:** The Protoeasome-Glo^{™} Reagent (combined Proteasome-Glo^{™} Substrate, Proteasome-Glo^{™} Buffer and Luciferin Detection Reagent) can be stored at 4°C or - 20°C for 1 month with minimal loss of activity.)

**Table 4** shows activity data in the Chymotrypsin-like ProteasomeGlo assay of B-cell and hPMBC. Inhibition is indicated as IC₅₀ [nM] ("-" = not measured). Compounds having an activity designated as "A" provided an IC₅₀ ≤ 100 nM; compounds having an activity designated as "B" provided an 100 nM < IC₅₀ ≤ 500 nM; compounds having an activity designated as "C" provided an 500 nM < IC₅₀ ≤ 1000 nM; compounds having an activity designated as "D" provided an 1000nM < IC₅₀ ≤ 5000 nM; compounds having an activity designated as "E" provided an an IC₅₀ > 5000nM.

**Table 4:**

| **Cpd No.** | **Pglo B-Cell [µM]** | **Pglo hPBMC [µM]** | **B cell diff. [µM]** | **PBMC IL17 stim. [µM]** |
|---|---|---|---|---|
| **7** | | A | | |
| **8** | A | B | | |
| **13** | A | | B | |
| **16** | A | | A | A |
| **22** | | | | |
| **25** | A | | | |
| **26** | A | | | |
| **27** | A | | A | A |
| **28** | | B | | |
| **29** | A | | A | A |
| **30** | A | | A | |
| **31** | A | | | |
| **32** | A | | | |
| **33** | A | | A | |
| **34** | A | | A | |
| **35** | A | | A | |
| **36** | B | | A | |
| **37** | A | | A | |
| **39** | A | | | |
| **40** | A | | | |
| **42** | A | | A | |
| **43** | A | | A | |
| **44** | | | | |
| **45** | B | | A | |
| **51** | A | | | |
| **59** | B | | | |
| **60** | B | B | | |
| **63** | A | A | | A |
| **64** | A | A | | |
| **77** | B | B | | |
| **78** | B | C | | |
| **79** | C | | | |
| **80** | A | A | | |
| **81** | A | A | | |
| **82** | A | A | | |
| **83** | A | A | | |
| **84** | | A | | |
| **85** | | A | | |
| **86** | | A | | |
| **87** | | A | | |
| **88** | A | A | | |
| **89** | | A | | |
| **116** | | C | | |
| **120** | | B | | |
| **126** | | A | | |
| **127** | | A | | |
| **128** | | A | | |
| **129** | | A | | |
| **130** | | A | | |
| **134** | | A | | |
| **135** | | A | | |
| **136** | | A | | |
| **143** | | A | | |
| **148** | | C | | |
| **151** | | | | |
| **152** | | A | | |
| **160** | | A | | |
| **157** | | A | | |
| **158** | | A | | |
| **159** | | A | | |
| **168** | | D | | |
| **171** | | A | | |
| **174** | | A | | |
| **175** | | A | | |
| **176** | | A | | |
| **177** | | A | | |
| **179** | | A | | |
| **180** | | A | | |
| **181** | | A | | |
| **182** | | A | | |
| **186** | | B | | |
| **191** | | B | | |
| **195** | | A | | |
| **199** | | A | | |
| **205** | | A | | |
| **211** | | B | | |
| **213** | | A | | |
| **215** | | A | | |
| **216** | | A | | |
| **217** | | A | | |
| **219** | | A | | |
| **220** | | A | | |
| **253** | | E | | |
| **255** | | E | | |
| **256** | | E | | |
| **257** | | D | | |
| **258** | | B | | |
| **259** | | E | | |
| **260** | | E | | |
| **281** | | D | | |
| **284** | | A | | |
| **285** | | A | | |
| **286** | | A | | |
| **287** | | A | | |
| **288** | | A | | |
| **289** | | C | | |
| **290** | | E | | |
| **291** | | D | | |
| **293** | | A | | |
| **294** | | A | | |
| **292** | | A | | |
| **282** | | C | | |
| **295** | | B | | |
| **296** | | B | | |
| **299** | | D | | |
| **300** | | C | | |
| **301** | | D | | |
| **283** | | C | | |
| **302** | | A | | |
| **309** | | A | | |
| **310** | | A | | |
| **311** | | A | | |
| **314** | | A | | |
| **320** | | A | | |
| **322** | | C | | |
| **326** | | A | | |
| **329** | | B | | |
| **332** | | A | | |
| **333** | | A | | |
| **334** | | B | | |
| **335** | | A | | |
| **336** | | A | | |
| **337** | | A | | |
| **338** | | A | | |
| **339** | | B | | |
| **340** | | A | | |
| **341** | | A | | |
| **342** | | A | | |
| **343** | | A | | |
| **344** | | A | | |
| **345** | | A | | |
| **347** | | A | | |
| **348** | | A | | |
| **349** | | A | | |
| **350** | | A | | |
| **351** | | A | | |
| **352** | | B | | |
| **353** | | A | | |
| **354** | | A | | |
| **355** | | A | | |
| **356** | | A | | |
| **358** | | A | | |
| **359** | | A | | |
| **360** | | A | | |
| **361** | | A | | |
| **362** | | A | | |
| **363** | | A | | |
| **364** | | A | | |
| **371** | | A | | |
| **372** | | A | | |
| **373** | A | A | | |
| **503** | | B | | |
| **505** | | B | | |
| **509** | | B | | |
| **510** | | A | | |
| **511** | | A | | |
| **512** | | A | | |
| **513** | | A | | |
| **549** | | A | | |
| **550** | | | | |
| **551** | | A | | |
| **552** | | A | | |
| **553** | | A | | |
| **554** | | B | | |
| **555** | | A | | |
| **556** | | A | | |
| **557** | | A | | |
| **558** | | A | | |
| **559** | | A | | |
| **560** | | A | | |
| **561** | | A | | |
| **562** | | A | | |
| **563** | | A | | |
| **564** | | A | | |
| **565** | | B | | |
| **566** | | B | | |
| **567** | | A | | |
| **568** | | A | | |
| **571** | | A | | |

## Claims

1. Compound of the general formula (I) 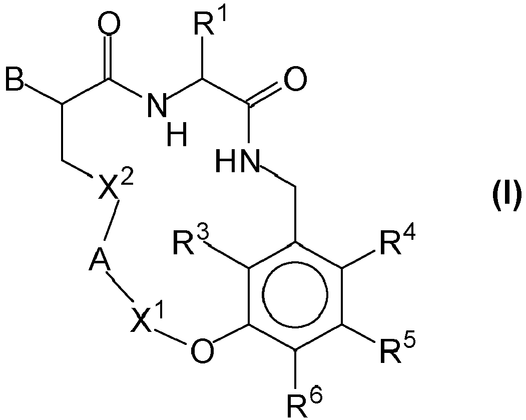 wherein
A represents -CO-N(**R**^{N6})-, 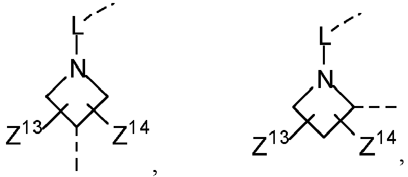 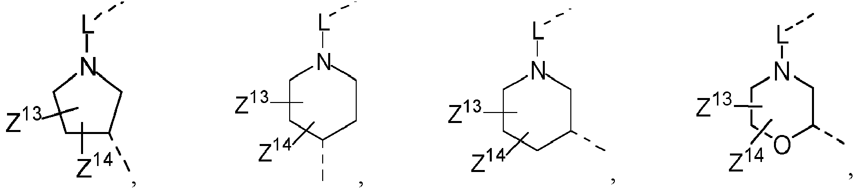 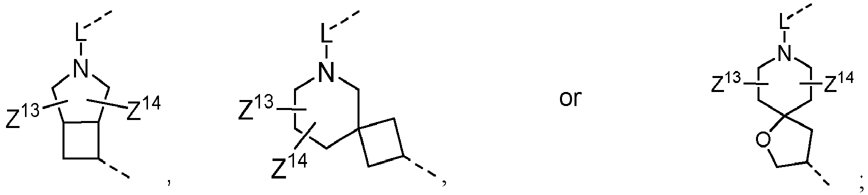
B represents -H, -NH(**R**²), -N(**R²**)(**R^{N5}**), 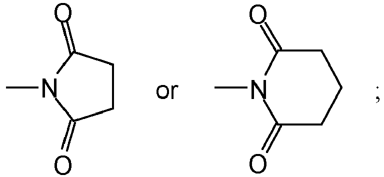
**L** represents -CO-, -CO-NH-, -CO-N(**R**^{N3})-, or -CO-O-,
**R¹** represents -H, -(CH₂)ₚ-**R**⁷, -(CH₂)ₚ-NH-**R⁷**, -(CH₂)ₚ-**R⁹**, or -(CH₂)ₚ-N**R^{N4}**-**R⁹**;
**R²** represents -H, -**R⁸**, -**R¹¹**, -**L¹**-**R¹¹**, -**L¹**-(CH₂)ᵣ-**R⁸**, -**L¹**-**R¹⁰**, -**L¹**-(C₂H₄O)ₛ-**R¹¹**, -**L¹**-(CH₂)ₜ-O-**R¹¹**, -**L¹**-(CH₂)ₜ-**NH**-(CH₂)ᵣ-**R⁸**, -**L¹**-(CH₂)ₜ-O-(CH₂)ᵣ-**R⁸**, -**L¹**-(CH₂)ₜ-**NR^{N6}R¹⁰**, -**L¹**-(CH₂)ₜ-O-(CH₂)ᵤ-**NR^{N6}R¹⁰**, -**L¹**-(CH₂)ᵣ-**R¹⁴**, -CO-C(**R¹²**)(**R¹³**)-**R⁸** or -CO-C(**R¹²**)(**R¹³**)-(CH₂)ᵤ-**R⁸**;
**L¹** represents a bond, -CO-, -CO₂-, -CONH-, or -SO₂-;
**R³** - **R⁶** represent independently of each other -H, -CH₃, -OCH₃, -F, or -Cl; or **R⁵** and **R⁶** form 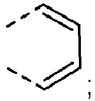
**R⁸** and **R⁹** represents independently of each other
C₆-C₁₄ aryl, C₁-C₁₀ heteroaryl, C₃-C₈ carbocyclyl, C₁-C₉ heterocyclyl, C₄-C₁₁ bicyclic carbocyclyl, C₄-C₁₁ bridged carbocyclyl, C₁-C₁₀ bicyclic heterocyclyl, C₁- C₁₀ bridged heterocyclyl, C₇-C₁₆-spiroalkyl, C₅-C₁₄-spiroheterocyclyl,
wherein all afore-mentioned ring systems can be substituted with 1 to 5 substituents selected from Z¹ , Z², Z³, Z⁴, Z⁵, Z⁶ , Z⁷, Z⁸ , Z⁹ , Z¹⁰, Z¹¹, Z¹² and R^{N1}, R^{N2}; and
C₁-C₁₀ heteroaryl, C₁-C₉ heterocyclyl, C₁-C₁₀ bicyclic heterocyclyl, C₁-C₁₀ bridged heterocyclyl, C₅-C₁₄-spiroheterocyclyl ring systems contain at least one of heteroatoms N, O, and S;
said C₃-C₈ carbocyclyl, C₁-C₉ heterocyclyl, C₁-C₁₀ bicyclic heterocyclyl, C₄-C₁₁ bridged carbocyclyl, C₁-C₁₀ bicyclic heterocyclyl, C₁-C₁₀ bridged heterocyclyl, C₇- C₁₆-spiroalkyl, C₅-C₁₄-spiroheterocyclyl ring systems can be partly saturated or unsaturated,
**R⁷** and **R¹⁰** represent independently of each other -H, -CH₃, -C₂H₅, -C₃H₇, -CH(CH₃)₂, -CH₂F, -CHF₂, -CF₃, -CH₂CN, -C(CH₃)₂-CN, -CH₂-C(CH₃)₂-CN, 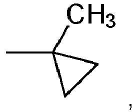 cyclo-C₃H₅, cyclo-C₄H₇, cyclo-C₅H₉, cyclo-C₆H₁₁, cyclo-C₇H₁₃, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -Ph, -CH₂-Ph, -CH₂-CH₂-Ph, -C₂H₄-CH=CH₂, -CH₂-CH=CH-CH₃, -CH₂-C(CH₃)=CH₂, -CH(CH₃)-CH=CH₂, -C(CH₃)=CH-CH₃, -CH₂-CH=C(CH₃)₂, -CO-CH=C(CH₃)₂, -CH₂-C≡CH, -C₂H₄-C≡CH, -CH₂-C≡C-CH₃, -CH₂-OCF₃, -C₂H₄-OCF₃, -C₃H₆-OCF₃, -CH₂-OCHF₂, -C₂H₄-OCHF₂, -C₃H₆-OCHF₂, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OH, -C₂H₄-OH, -C₃H₆-OH, -C(CH₃)₂-CN, -C(CH₃)₂-OH, -C(CH₃)₂-CH₂-OH, -C(C₂H₅)₂-CH₂-OH, -C(CH₂-OH)₂-CH₃, -C(CH₂-OH)₂-C₂H₅, -C(CH₃)₂-CH₂-SH, -C(C₂H₅)₂-CH₂-SH, -C(CH₂-SH)₂-CH₃, or -C(CH₂-SH)₂-C₂H₅;
**R¹¹** represents -H, -CH₃, -C₂H₅, -C₃H₇, -CH(CH₃)₂, -CH₂F, -CHF₂, -CF₃, -CH₂CN, -C(CH₃)₂-CN, -CH₂-C(CH₃)₂-CN, 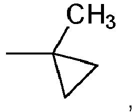 cyclo-C₃H₅, cyclo-C₄H₇, cyclo-C₅H₉, cyclo-C₆H₁₁, cyclo-C₇H₁₃, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -Ph, -CH₂-Ph, -CH₂-CH₂-Ph, -CH=CH₂, -CH₂-CH=CH₂, -C(CH₃)=CH₂, -CH=CH-CH₃, -C(CH₃)=CH-CH₃, -CH=C(CH₃)₂, -C(CH₃)=C(CH₃)₂, -C₂H₄-CH=CH₂, -CH₂-CH=CH-CH₃, -CH₂-C(CH₃)=CH₂, -CH(CH₃)-CH=CH₂, -C(CH₃)=CH-CH₃, -CH₂-CH=C(CH₃)₂, -C=CH, -CH₂-C≡CH, -C₂H₄-C≡CH, -CH₂-C≡C-CH₃, -C≡C-CH₃, -C≡C-C₂H₅, -CH₂-OCF₃, -C₂H₄-OCF₃, -C₃H₆-OCF₃, -CH₂-OCHF₂, -C₂H₄-OCHF₂, -C₃H₆-OCHF₂, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OH, -C₂H₄-OH, -C₃H₆-OH, -C(CH₃)₂-CH₂-OH, -C(C₂H₅)₂-CH₂-OH, -C(CH₂-OH)₂-CH₃, -C(CH₂-OH)₂-C₂H₅, -C(CH₃)₂-CH₂-SH, -C(C₂H₅)₂-CH₂-SH, -C(CH₂-SH)₂-CH₃, or -C(CH₂-SH)₂-C₂H₅;
**R¹²** and **R¹³** represent independently of each other -H, -CH₃, -C₂H₅, -C₃H₇, -CH(CH₃)₂, -Ph, -CH₂-Ph, -COOH, -NH₂, -NHCO₂(CCH₃)₃, -CH₂-NH₂, -CHF₂, -CF₃, -OCF₃, -OCHF₂, -OH, -OCH₃, -OC₂H₅, -OC₃H₇, and -OCH(CH₃)₂; or
**R¹²** and **R¹³** form together
**R¹⁴** represents 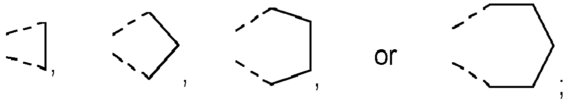 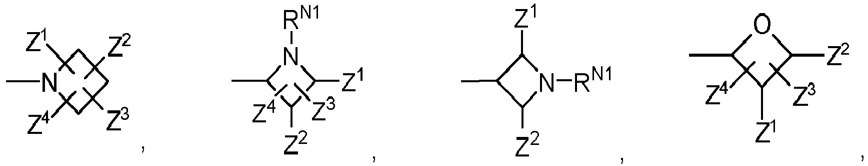 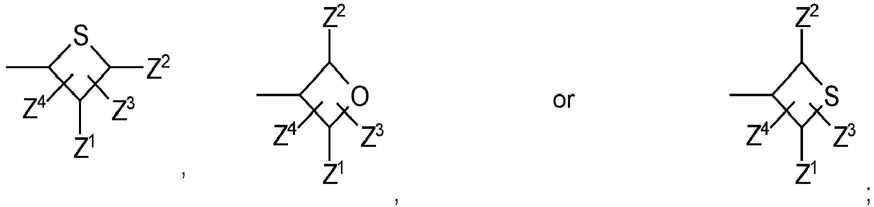
**R¹⁵** and **R¹⁶** represent independently of each other -**X³**-**L²**-**R¹⁷**, or -(OCH₂CH₂)_{w}-**R¹⁷**;
**L²** represents -(CH₂)ᵥ-, -(CH₂CH₂-O)_{w}-CH₂-, or -(CH₂CH₂-O)_{w}-CH₂CH₂-;
**R¹⁷** represents -OH, -SH, -SO₃H, -NH₂, or -CO₂H;
**R^{N1}**, **R^{N2}**, **R^{N3}**, and **R^{N4}** represent independently of each other -**R¹⁵**, -H, -CH₃, -C₂H₅, -C₃H₇, -CH(CH₃)₂, -CHF₂, -CF₃, 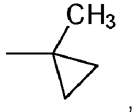 cyclo-C₃H₅, cyclo-C₄H₇, cyclo-C₅H₉, cyclo-C₆H₁₁, cyclo-C₇H₁₃, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -Ph, -CH₂-Ph, -CH₂-CH₂-Ph, -C₂H₄-CH=CH₂, -CH₂-CH=CH-CH₃, -CH₂-C(CH₃)=CH₂, -CH(CH₃)-CH=CH₂, -CH₂-CH=C(CH₃)₂, -CH₂-C≡CH, -C₂H₄-C≡CH, -CH₂-C≡C-CH₃, -COCH₃, -COC₂H₅, -COC₃H₇, -CO-cyclo-C₃H₅, -COCH(CH₃)₂, -COC(CH₃)₃, -COOH, -COOCH₃, -COOC₂H₅, -COOC₃H₇, -COO-cyclo-C₃H₅, -COOCH(CH₃)₂, -COOC(CH₃)₃, -COOCH₂Ph, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, or -SO₃H;
**R^{N5}** and **R^{N6}** represent independently of each other -H, -CH₃, -C₂H₅, -C₃H₇, -CH(CH₃)₂, cyclo-C₃H₅, -COOC(CH₃)₃, or -COOCH₂Ph;
**X¹** represents -(CH₂)ₘ-;
**X²** represents -(CH₂)ₙ-;
**X³** represents a bond, -O-, -NH-, or -S-;
**Z¹** - **Z¹⁴** represent independently of each other 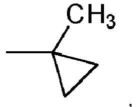 cyclo-C₃H₅, cyclo-C₄H₇, cyclo-C₅H₉, cyclo-C₆H₁₁, cyclo-C₇H₁₃, -**R¹⁶**, -H, -OH, -OCH₃, -OC₂H₅, -OC₃H₇, -O-cyclO-C₃H₅, -OCH(CH₃)₂, -OC(CH₃)₃, -OC₄H₉, -OPh, -OCH₂-Ph, -OCPh₃, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, —C₂H₄—OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -SH, -SCH₃, -SC₂H₅, -SC₃H₇, -S-cyclo-C₃H₅, -SCH(CH₃)₂, -SC(CH₃)₃, -F, -Cl, -Br, -I, -CN, -COCH₃, -COC₂H₅, -COC₃H₇, -CO-cyclo-C₃H₅, -COCH(CH₃)₂, -COC(CH₃)₃, -COOH, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -O-S(=O)CH₃, -O-S(=O)C₂H₅, -O-S(=O)C₃H₇, -O-S(=O)-cyclo-C₃H₅, -O-S(=O)CH(CH₃)₂, -O-S(=O)C(CH₃)₃, -S(=O)(=NH)CH₃, -S(=O)(=NH)C₂H₅, -S(=O)(=NH)C₃H₇, -S(=O)(=NH)-cyclo-C₃H₅, -S(=O)(=NH)CH(CH₃)₂, -S(=O)(=NH)C(CH₃)₃, -NH-SO₂-CH₃, -NH-SO₂-C₂H₅, -NH-SO₂-C₃H₇, -NH-SO₂-cyclo-C₃H₅, -NH-SO₂-CH(CH₃)₂, -NH-SO₂-C(CH₃)₃, -O-SO₂-CH₃, -O-SO₂-C₂H₅, -O-SO₂-C₃H₇, -O-SO₂-cyclo-C₃H₅, -O-SO₂-CH(CH₃)₂, -O-SO₂-C(CH₃)₃, -OCH₂F, -OCHF₂, -OCF₃, -CH₂-OCF₃, -C₂H₄-OCF₃, -C₃H₆-OCF₃, -CH₂-OCHF₂, -C₂H₄-OCHF₂, -C₃H₆-OCHF₂, -OC₂F₅, -CH₂-OC₂F₅, -C₂H₄-OC₂F₅, -C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-C(=NH)-NH₂, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-C(=NH)-NHCH₃, -NH-C(=NH)-NHC₂H₅, -NH-C(=NH)-NHC₃H₇, -O-CO-NH-cyclo-C₃H₅, -NH-C(=NH)-NH-cyclo-C₃H₅, -NH-C(=NH)-NH[CH(CH₃)₂], -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO- OCH₃, -O-CO-OC₂H₅, -O-CO-OC₃H₇, -O-CO-O-cyclo-C₃H₅, -O-CO- OCH(CH₃)₂, -O-CO-OC(CH₃)₃, -CH₂F, -CHF₂, -CF₃, -CH₂-CH₂F, -CH₂-CHF₂, -CH₂-CF₃, cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)- C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇ -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH=CH-CH=CH-CH₃, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH- CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH(CH₃)-CH₂-C≡CH, -CH(CH₃)-C≡C-CH₃, -C₂H₄-CH(CH₃)-C≡CH, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)- C=CH, -C(CH₃)=CH-CH=CH-CH₃, -C=CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H_{7,} -CH(CH₃)-C≡CH, -C₄H₈-C≡CH, -C₂H₄-C≡C-C₂H₅, -CH₂-C≡C-C₃H₇, -C≡C-C₄H₉, -C≡C-CH₂-CH(CH₃)₂, -CH(CH₃)-C₂H₄-C≡CH, -CH₂-CH(CH₃)-C≡C-CH₃, -C(CH₃)(C₂H₅)-C≡CH, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -CH₂-C≡C-C≡C-CH₃, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -CH₂-CH(C≡CH)₂, -C=C-C=CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -C≡C-C(CH₃)₃, -C≡C-CH₂-C≡C-CH₃, -C≡C-C≡C-C₂H₅, 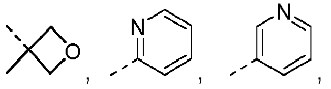 or 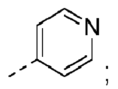
**Z³** and **Z⁴** may form together 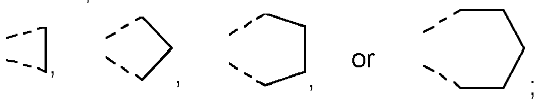 or 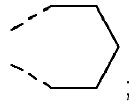
**Z¹³** and **Z¹⁴** may form together 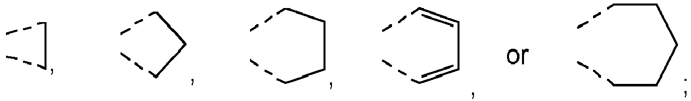
m is an integer selected from 0, 1, 2, 3, 4, 5, or 6;
n is an integer selected from 0, 1, 2, 3, 4, 5, or 6;
p is an integer selected from 0, 1, 2, 3, 4, 5, or 6;
r is an integer selected from 0, 1, 2, 3, or 4;
s is an integer selected from 1, 2, 3, 4, 5, 6, 7, 8, 9, or 10;
t is an integer selected from 1, 2, 3, or 4;
u is an integer selected from 1, 2, 3, or 4;
v is an integer selected from 0, 1, 2, 3, 4, 5, or 6;
w is an integer selected from 0, 1, 2, 3, 4, 5 or 6;
or an enantiomer, a stereoisomeric form, a mixture of enantiomers, a diastereomer, a mixture of diastereomers, a hydrate, a solvate, an acid salt form, a tautomer, a racemate of the above mentioned compounds, or a pharmaceutically acceptable salt thereof.

2. The compound according to claim 1, wherein **R⁸** and **R⁹** represents independently of each other: 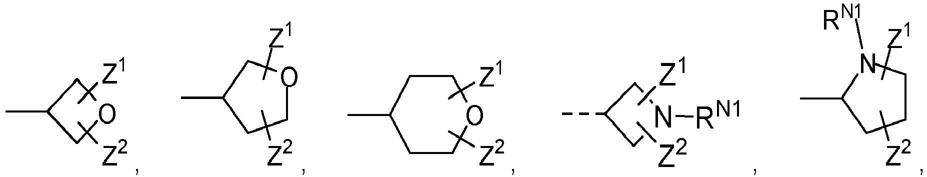 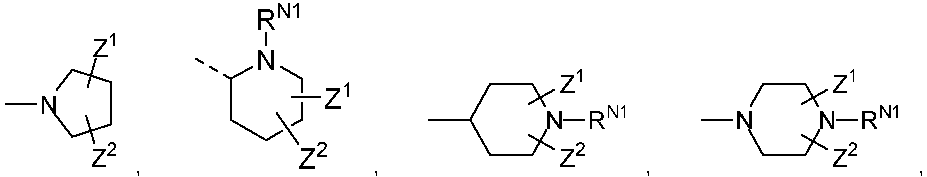 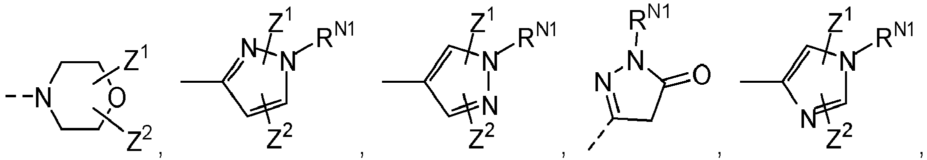 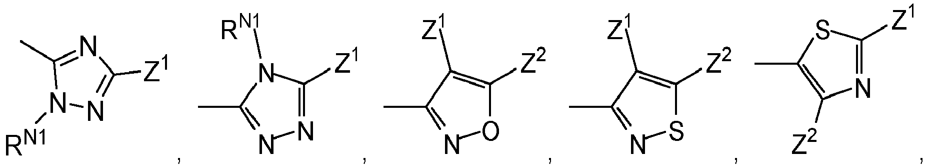 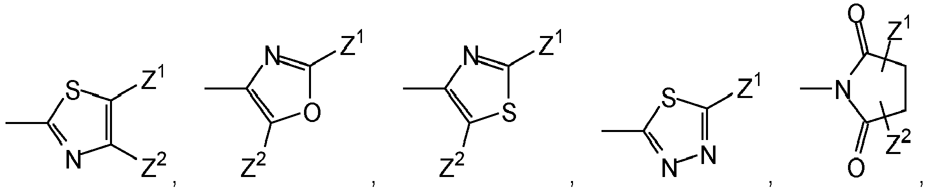 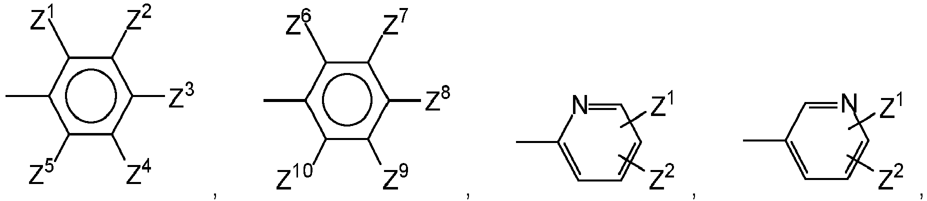 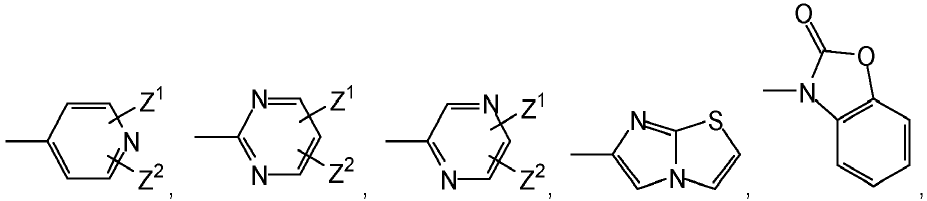 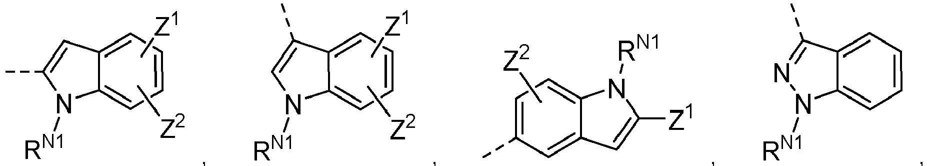 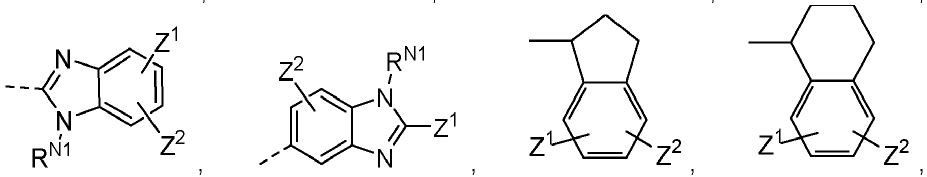 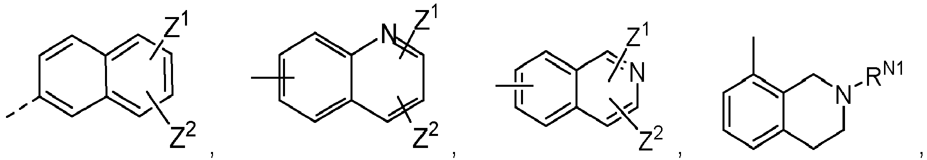 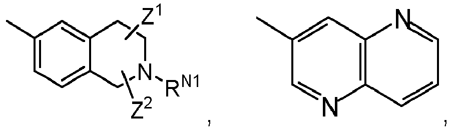 and

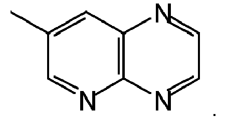

3. The compound according to claim 1 or 2, wherein **R⁸** represents 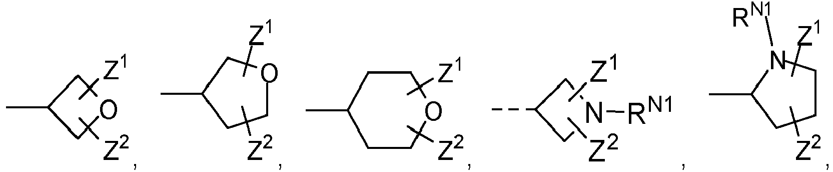 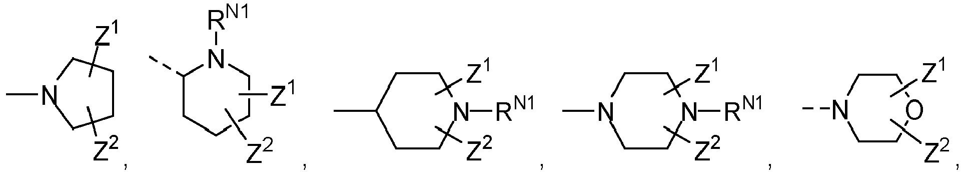 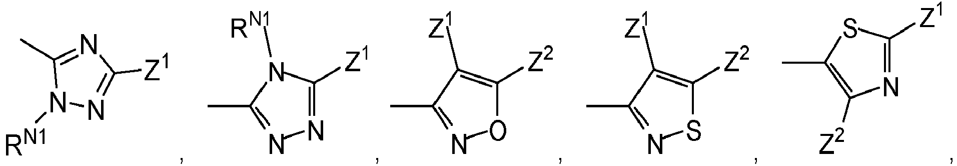 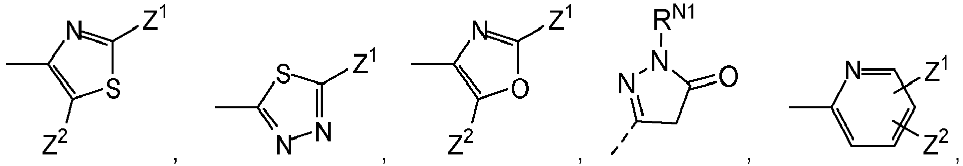 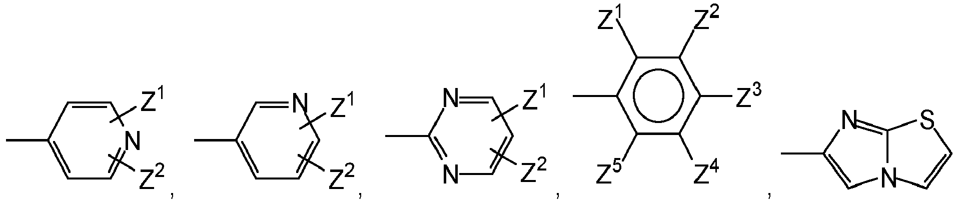 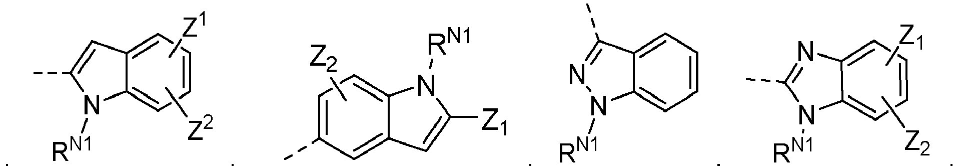 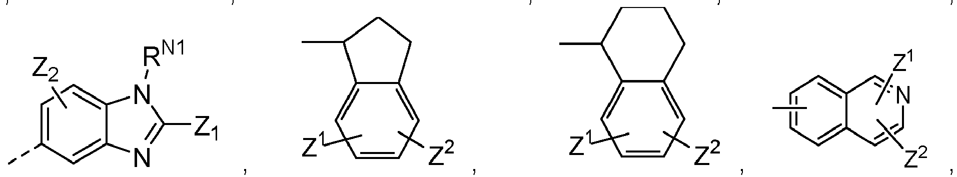 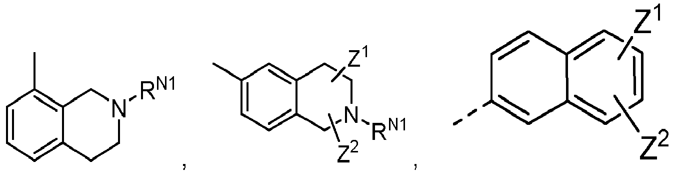 or

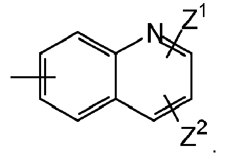

4. The compound according to any one of the claim 1 - 3, wherein **R⁹** represents 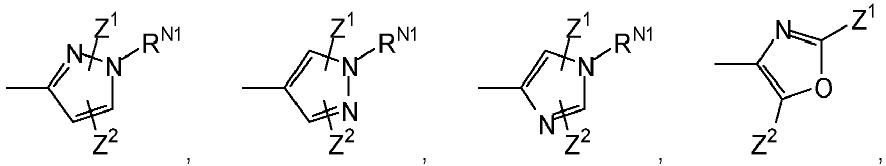 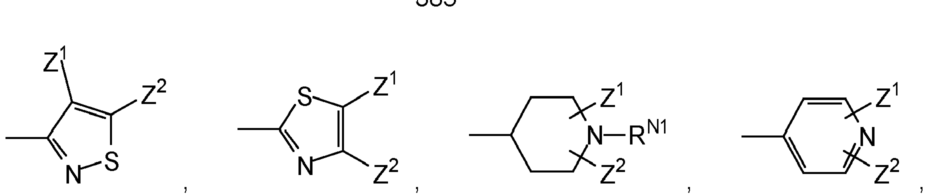 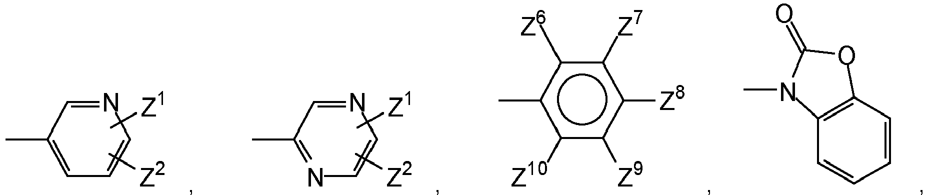 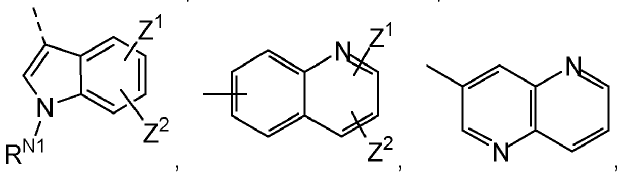 or

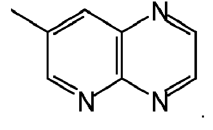

5. The compound according to any one of the claims 1 to 4, wherein the compound has any one of the formulae (**II-1**) - **(II-16),** (**III-1**) - **(III-8), (IV-1)** - **(IV-4),** and **(V-1)** - **(V-9)** 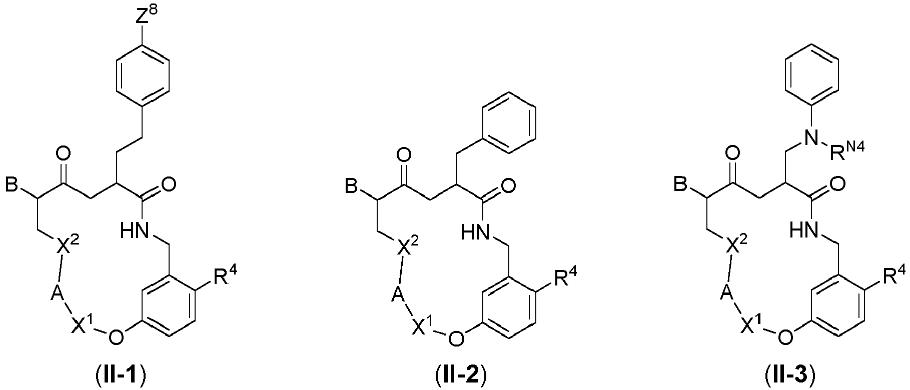 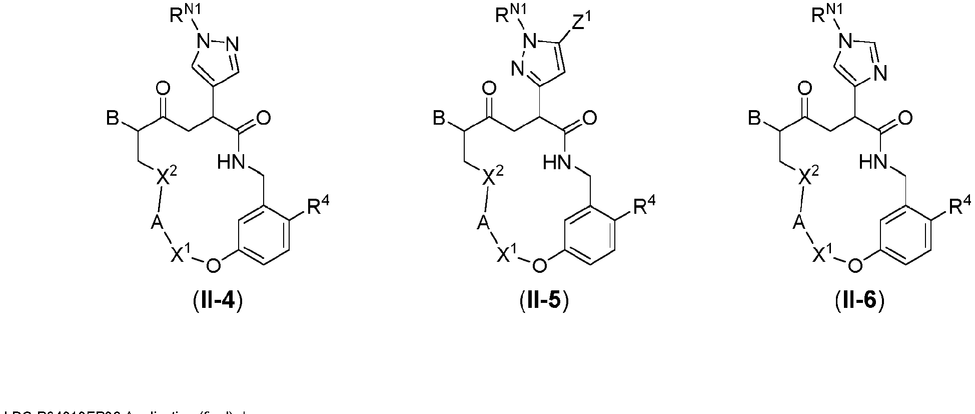 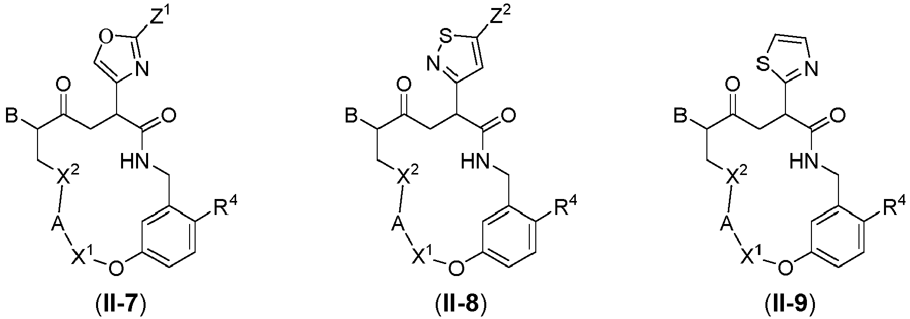 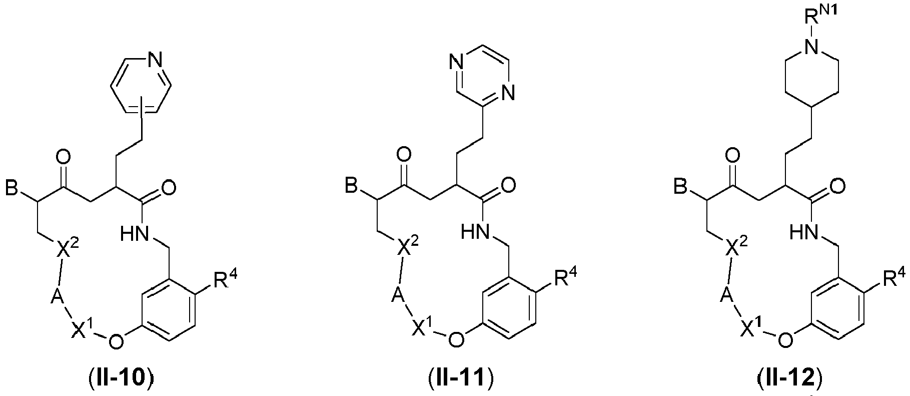 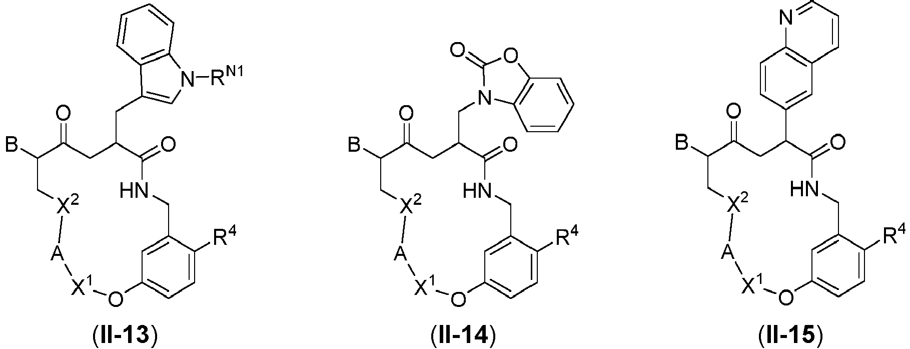 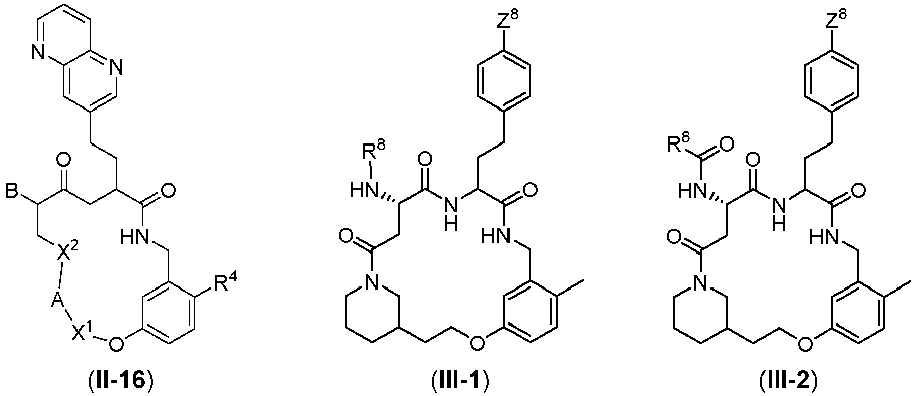 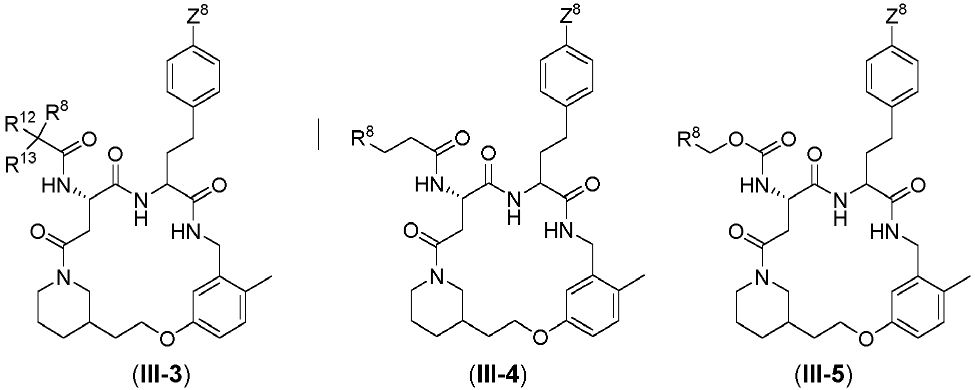 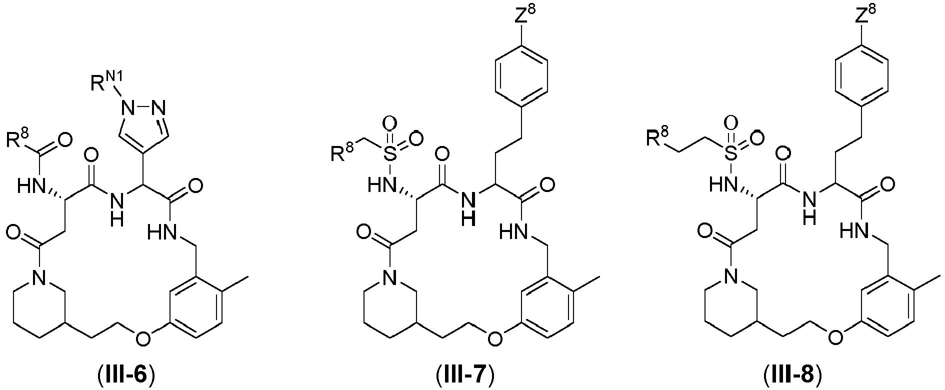 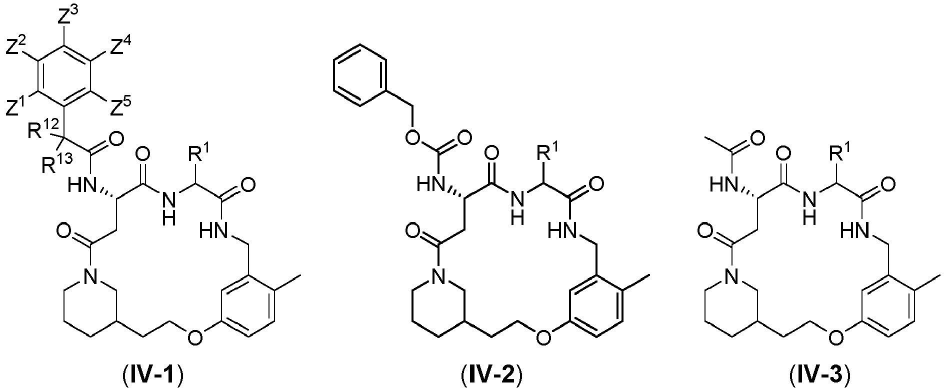 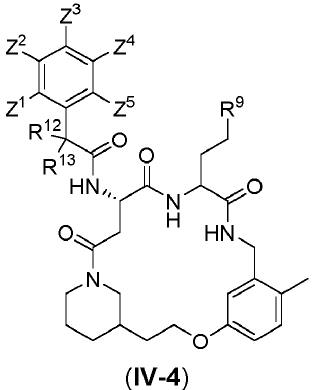 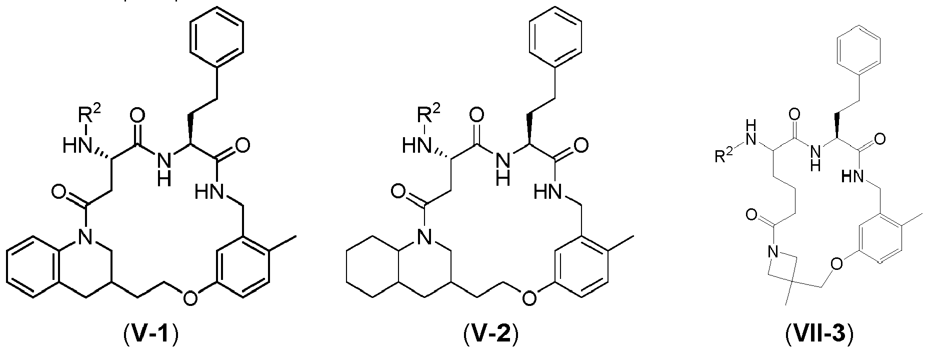 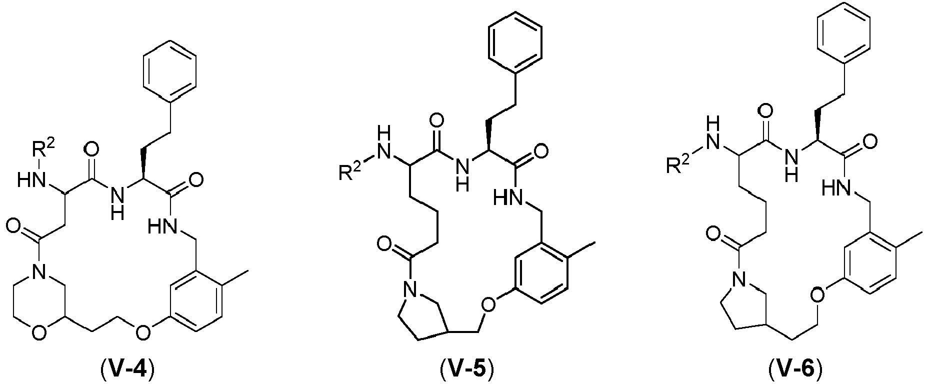 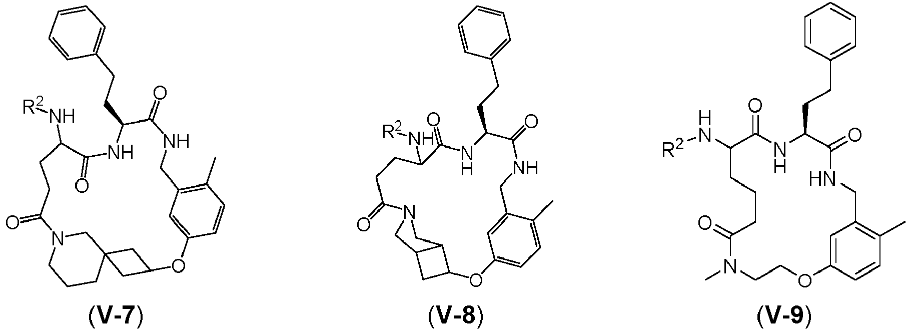 wherein A, B, R¹, R², R⁴, R⁸, R⁹, R¹², R¹³, R^{N1}, R^{N4}, X¹, X², Z¹, Z², Z³, Z⁴, Z⁵ and Z⁸ have the same meanings as defined in any one of the claims 1 to 4.

6. The compound according to any one of the claims 1 to 5, wherein A represents -CO-NH-, -CO-N(CH₃)-,

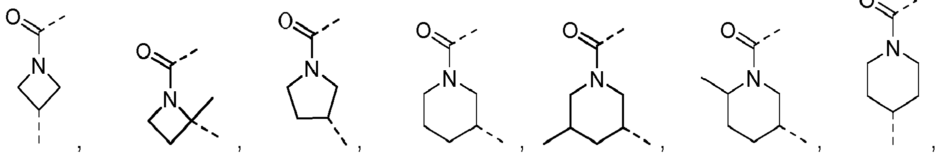

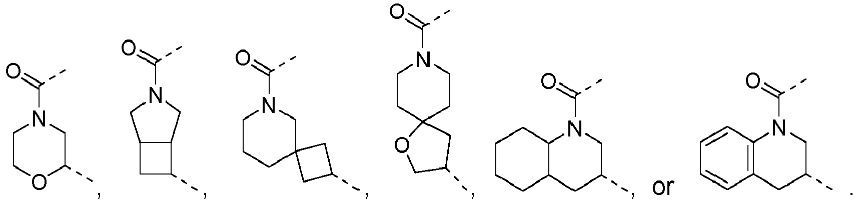

7. The compound according to claim 1 selected from the group consisting of:
| **Compound** | **IUPAC Name** |
|---|---|
| **7** | benzyl ((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamate |
| **8** | (9S,12S)-12-amino-5⁴-methyl-9-phenethyl-4-oxa-7,10-diaza-1(3,1)-piperidina-5(1,3)-benzenacyclotetradecaphane-8,11,14-trione |
| **13** | (9S)-5⁴-methyl-9-phenethyl-4-oxa-7,10-diaza-1(3,1)-piperidina-5(1,3)-benzenacyclotetradecaphane-8,11,14-trione |
| **16** | (9S)-5⁴-methyl-9-phenethyl-4-oxa-7,10-diaza-1(3,1)-piperidina-5(1 ,3)-benzenacyclohexadecaphane-8,11,16-trione |
| **19** | (9S)-5⁴-methyl-9-phenethyl-4-oxa-7,10-diaza-1(3,1)-piperidina-5(1,3)-benzenacyclopentadecaphane-8,11,15-trione |
| **22** | (9S)-5⁴-methyl-9-phenethyl-4-oxa-7,10-diaza-1(3,1)-piperidina-5(1,3)-benzenacycloheptadecaphane-8,11,17-trione |
| **25** | 3-(3-fluorophenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)propanamide |
| **26** | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)benzamide |
| **27** | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)pivalamide |
| **28** | 3,3-dimethyl-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)butanamide |
| **29** | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-phenylacetamide |
| **30** | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-phenylacetamide |
| **31** | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-1H-benzo[d]imidazole-2-carboxamide |
| **32** | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)pyrimidine-2-carboxamide |
| **33** | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **34** | (9S,12S)-5⁴-methyl-9-phenethyl-12-(pyrimidin-2-ylamino)-4-oxa-7,10-diaza-1(3,1)-piperidina-5(1,3)-benzenacyclotetradecaphane-8,11,14-trione |
| **35** | (9S,12S)-5⁴-methyl-12-(oxetan-3-ylamino)-9-phenethyl-4-oxa-7,10-diaza-1(3,1)-piperidina-5(1,3)-benzenacyclotetradecaphane-8,11,14-trione |
| **36** | (9S,12S)-5⁴-methyl-9-phenethyl-12-(pyridin-2-ylamino)-4-oxa-7,10-diaza-1(3,1)-piperidina-5(1,3)-benzenacyclotetradecaphane-8,11,14-trione |
| **37** | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)methanesulfonamide |
| **38** | 1-ethyl-3-((9S,12S)-5⁴-methyl-8,11,14-trioxo-9-phenethyl-4-oxa-7,10-diaza-1(3,1)-piperidina-5(1,3)-benzenacyclotetradecaphane-12-yl)urea |
| **39** | ethyl ((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamate |
| **40** | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)picolinamide |
| **41** | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)nicotinamide |
| **42** | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)isonicotinamide |
| **43** | (9S,12S)-12-(2,5-dioxopyrrolidin-1-yl)-5⁴-methyl-9-phenethyl-4-oxa-7,10-diaza-1(3,1)-piperidina-5(1,3)-benzenacyclotetradecaphane-8,11,14-trione |
| **44** | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-3-phenylpropane-1-sulfonamide |
| **45** | (9S,12S)-12-((1H-benzo[d]imidazol-2-yl)amino)-5⁴-methyl-9-phenethyl-4-oxa-7,10-diaza-1(3,1)-piperidina-5(1,3)-benzenacyclotetradecaphane-8,11,14-trione |
| **51** | 5⁴-methyl-9-(2-(pyridin-3-yl)ethyl)-4-oxa-7,10-diaza-1(3,1)-piperidina-5(1,3)-benzenacyclotetradecaphane-8,11,14-trione |
| **59** | (9R)-5⁴,5⁶-dimethyl-9-phenethyl-4-oxa-7,10-diaza-1(3,1)-piperidina-5(1,3)-benzenacyclotetradecaphane-8,11,14-trione |
| **60** | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-1H-indazole-3-carboxamide |
| **61** | 2-(3-chlorophenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **62** | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-(m-tolyl)acetamide |
| **63** | 2-(3,4-dimethoxyphenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **64** | 2-(3,4-dimethoxyphenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)butanamide |
| **77** | (9S)-9-((1H-indol-3-yl)methyl)-5⁴-methyl-4-oxa-7,10-diaza-1(3,1)-piperidina-5(1,3)-benzenacyclotetradecaphane-8,11,14-trione |
| **78** | (9S)-5⁴,9-dimethyl-4-oxa-7,10-diaza-1(3,1)-piperidina-5(1,3)-benzenacyclotetradecaphane-8,11,14-trione |
| **79** | (9S)-9-benzyl-5⁴-methyl-4-oxa-7,10-diaza-1(3,1)-piperidina-5(1,3)-benzenacyclotetradecaphane-8,11,14-trione |
| **80** | (2R)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-phenylpropanamide |
| **81** | (2S)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-phenylpropanamide |
| **82** | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-1,2,3,4-tetrahydronaphthalene-1-carboxamide |
| **83** | 2-(4-methoxyphenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **84** | 2-(2-methoxyphenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **85** | 2-(3-methoxyphenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **86** | (2R)-2-methoxy-N-((9S,12S)-5⁴-methyl-8,11, 14-trioxo-9-phenethyl-4-oxa-7,10-diaza-1(3,1)-piperidina-5(1,3)-benzenacyclotetradecaphane-12-yl)-2-phenylacetamide |
| **87** | 1-methyl-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-5-(trifluoromethyl)-1H-indole-2-carboxam ide |
| **88** | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-morpholinoacetamide |
| **89** | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-(pyrrolidin-1-yl)acetamide |
| **95** | (9S)-5⁴,5⁵-dimethyl-9-phenethyl-4-oxa-7,10-diaza-1(3,1)-piperidina-5(1,3)-benzenacyclotetradecaphane-8,11,14-trione (95) |
| **105** | benzyl ((12S)-5⁴-methyl-8,11,14-trioxo-9-(2-(pyrazin-2-yl)ethyl)-4-oxa-7,10-diaza-1(3,1)-piperidina-5(1,3)-benzenacyclotetradecaphane-12-yl)carbamate |
| **106** | (12S)-amino-5⁴-methyl-8,11,14-trioxo-9-(2-(pyrazin-2-yl)ethyl)-4-oxa-7,10-diaza-1 (3,1)-piperidina-5(1,3)-benzenacyclotetradecaphane |
| **107** | (2S)-N-((12S)-5⁴-methyl-8,11,14-trioxo-9-(2-(pyrazin-2-yl)ethyl)-4-oxa-7,10-diaza-1(3,1)-piperidina-5(1,3)-benzenacyclotetradecaphane-12-yl)-2-phenylpropanamide |
| **116** | 5⁴-methyl-9-(1-methyl-1H-pyrazol-4-yl)-4-oxa-7,10-diaza-1(3,1)-piperidina-5(1,3)-benzenacyclotetradecaphane-8,11,14-trione |
| **120** | (9S)-9-((1H-indol-3-yl)methyl)-5⁴,5⁵-dimethyl-4-oxa-7,10-diaza-1(3,1)-piperidina-5(1,3)-benzenacyclotetradecaphane-8,11,14-trione |
| **121** | tert-butyl 4-(2-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)am ino)-2-oxoethyl)piperazine-1-carboxylate |
| **122** | tert-butyl (3-(2-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)amino)-2-oxoethyl)phenyl)carbamate |
| **123** | tert-butyl (4-(2-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)amino)-2-oxoethyl)phenyl)carbamate |
| **124** | tert-butyl methyl(2-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)amino)-2-oxoethyl)carbamate |
| **125** | tert-butyl methyl(2-(2-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)amino)-2-oxoethoxy)ethyl)carbamate |
| **126** | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-(piperazin-1-yl)acetamide |
| **127** | 2-(3-aminophenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **128** | 2-(4-aminophenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **129** | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-(methylamino)acetamide |
| **130** | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-(2-(methylamino)ethoxy)acetamide |
| **131** | tert-butyl 4-fluoro-4-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)piperidine-1-carboxylate |
| **132** | tert-butyl 4,4-difluoro-2-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)pyrrolidine-1-carboxylate |
| **133** | tert-butyl 4-methyl-4-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)piperidine-1-carboxylate |
| **134** | 4-fluoro-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)piperidine-4-carboxamide |
| **135** | (2S)-4,4-difluoro-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)pyrrolidine-2-carboxamide |
| **136** | 4-methyl-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)piperidine-4-carboxamide |
| **143** | N-((9S,12S)-5⁴-methyl-8,11,15-trioxo-9-phenethyl-4-oxa-7,10-diaza-1(2,4)-morpholina-5(1,3)-benzenacyclopentadecaphane-12-yl)acetamide |
| **148** | N-((7S,10S)-3⁴-methyl-6,9,13-trioxo-7-phenethyl-2-oxa-5,8-diaza-1(3,1)-piperidina-3(1,3)-benzenacyclotridecaphane-10-yl)acetamide |
| **149** | tert-butyl 4-ethyl-4-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)piperidine-1-carboxylate |
| **150** | tert-butyl 3,3-difluoro-4-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)piperidine-1-carboxylate |
| **151** | 4-ethyl-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)piperidine-4-carboxamide |
| **152** | 3,3-difluoro-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)piperidine-4-carboxamide |
| **153** | tert-butyl 2-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)-5-(trifluoromethyl)pyrrolidine-1-carboxylate |
| **154** | tert-butyl 2-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)piperidine-1-carboxylate |
| **155** | tert-butyl (3-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)oxetan-3-yl)carbamate |
| **156** | tert-butyl (3-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)tetrahydrofuran-3-yl)carbamate |
| **157** | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-5-(trifluoromethyl)pyrrolidine-2-carboxamide |
| **158** | 3-amino-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)oxetane-3-carboxamide |
| **159** | 3-amino-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)tetrahydrofuran-3-carboxamide |
| **160** | (2R)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)azetidine-2-carboxamide |
| **161** | tert-butyl 2-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)azetidine-1-carboxylate |
| **168** | N-((10S,13S)-1⁴-methyl-8,11,14-trioxo-13-phenethyl-2-oxa-7,12,15-triaza-1(1,3)-benzenacyclohexadecaphane-10-yl)acetamide |
| **171** | N-((11S,14S)-1⁴-methyl-8,12,15-trioxo-14-phenethyl-2-oxa-7,13,16-triaza-1(1,3)-benzenacycloheptadecaphane-11-yl)acetamide |
| **172** | 2-(4-aminophenyl)-N-((13R,9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **173** | 2-(4-aminophenyl)-N-((13S,9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **174** | 3-(2-methyl-1H-benzo[d]imidazol-6-yl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)propanamide |
| **175** | 3-(4-bromophenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)propanamide |
| **176** | 3-([1,1'-biphenyl]-4-yl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)propanamide |
| **177** | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-3-(2-phenoxyphenyl)propanamide |
| **178** | tert-butyl ((2S)-1-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)amino)-1-oxo-3-phenylpropan-2-yl)carbamate |
| **179** | (2R)-2-amino-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-3-phenylpropanamide |
| **180** | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)ethenesulfonamide |
| **181** | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acrylamide |
| **182** | 3-(1H-indol-5-yl)-N-((9S,12S)-54-methyl-8,11,14-trioxo-9-phenethyl-4-oxa-7,10-diaza-1(3,1)-piperidina-5(1,3)-benzenacyclotetradecaphane-12-yl)propanamide |
| **186** | N-((9S,12S)-5⁴-methyl-8,11,16-trioxo-9-phenethyl-4-oxa-7,10-diaza-1(2,4)-morpholina-5(1,3)-benzenacyclohexadecaphane-12-yl)acetamide |
| **191** | N-((11R,15S,7S,10S)-3⁴-methyl-6,9,14-trioxo-7-phenethyl-2-oxa-13,5,8-triaza-1(7,3)-bicyclo[3.2.0]heptana-3(1,3)-benzenacyclotetradecaphane-10-yl)acetamide |
| **195** | benzyl ((9S,12S)-5⁴-methyl-8,11,16-trioxo-9-phenethyl-4-oxa-7,10-diaza-1(3,1)-piperidina-5(1,3)-benzenacyclohexadecaphane-12-yl)carbamate |
| **199** | benzyl ((9S,12S)-5⁴-methyl-8,11,15-trioxo-9-phenethyl-4-oxa-7,10-diaza-1(3,1)-piperidina-5(1,3)-benzenacyclopentadecaphane-12-yl)carbamate |
| **205** | N-((9S,12S)-5⁴-fluoro-8,11,14-trioxo-9-phenethyl-4-oxa-7,10-diaza-1(3,1)-piperidina-5(1,3)-benzenacyclotetradecaphane-12-yl)acetamide |
| **211** | N-((9S,12S)-5⁴-methoxy-8,11,14-trioxo-9-phenethyl-4-oxa-7,10-diaza-1(3,1)-piperidina-5(1,3)-benzenacyclotetradecaphane-12-yl)acetamide |
| **212** | (9S,12S)-5⁴-methyl-12-amino-8,11,16-trioxo-9-phenethyl-4-oxa-7,10-diaza-1(3,1)-piperidina-5(1,3)-benzenacyclohexadecaphane |
| **213** | N-((9S,12S)-5⁴-methyl-8,11,16-trioxo-9-phenethyl-4-oxa-7,10-diaza-1(3,1)-piperidina-5(1,3)-benzenacyclohexadecaphane-12-yl)acetamide |
| **214** | (9S,12S)-12-amino-5⁴-methyl-8,11,15-trioxo-9-phenethyl-4-oxa-7,10-diaza-1(3,1)-piperidina-5(1,3)-benzenacyclopentadecaphane) |
| **215** | N-((9S,12S)-5⁴-methyl-8,11,15-trioxo-9-phenethyl-4-oxa-7,10-diaza-1(3,1)-piperidina-5(1,3)-benzenacyclopentadecaphane-12-yl)acetamide |
| **216** | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-1-phenylmethanesulfonamide |
| **217** | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-phenylethane-1-sulfonamide |
| **218** | 2,5,8,11,14,17,20-heptaoxadocosan-22-yl ((9R,12R)-5⁴-methyl-8,11,14-trioxo-9-phenethyl-4-oxa-7,10-diaza-1(3,1)-piperidina-5(1,3)-benzenacyclotetradecaphane-12-yl)carbamate |
| **219** | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2,5,8,11,14-pentaoxaheptadecan-17-amide |
| **220** | ((9R,12R)-5⁴-methyl-8,11,14-trioxo-9-phenethyl-4-oxa-7,10-diaza-1(3,1)-piperidina-5(1,3)-benzenacyclotetradecaphane-12-yl)carbamate |
| **252** | N-((8S,11S)-1⁴-methyl-6,9,12-trioxo-11-phenethyl-2-oxa-5,10,13-triaza-1(1,3)-benzenacyclotetradecaphane-8-yl)acetamide |
| **253** | N-((9S,12S)-1⁴-methyl-7,10,13-trioxo-12-phenethyl-2-oxa-6,11,14-triaza-1(1,3)-benzenacyclopentadecaphane-9-yl)acetamide |
| **254** | N-((7S,10S)-3⁴-methyl-6,9,12-trioxo-7-phenethyl-2-oxa-5,8-diaza-1(4,1)-piperidina-3(1,3)-benzenacyclododecaphane-10-yl)acetamide |
| **255** | N-((7S,10S)-3⁴-methyl-6,9,12-trioxo-7-phenethyl-2-oxa-5,8-diaza-1(3,1)-piperidina-3(1,3)-benzenacyclododecaphane-10-yl)acetamide |
| **256** | N-((9S,12S)-14,6-dimethyl-7,10,13-trioxo-12-phenethyl-2-oxa-6,11,14-triaza-1(1,3)-benzenacyclopentadecaphane-9-yl)acetamide |
| **257** | N-((8S,11S)-4⁴-methyl-7,10,13-trioxo-8-phenethyl-3-oxa-6,9-diaza-1(3,1)-piperidina-4(1,3)-benzenacyclotridecaphane-11-yl)acetamide |
| **258** | N-((9S,12S)-5⁴-methyl-8,11,14-trioxo-9-phenethyl-4-oxa-7,10-diaza-1(2,4)-morpholina-5(1,3)-benzenacyclotetradecaphane-12-yl)acetam ide |
| **259** | N-((8S,11S)-4⁴-methyl-7,10,13-trioxo-8-phenethyl-3-oxa-6,9-diaza-1(3,1)-azetidina-4(1,3)-benzenacyclotridecaphane-11-yl)acetamide |
| **260** | N-((8S,11S)-1²,4⁴-dimethyl-7,10,13-trioxo-8-phenethyl-3-oxa-6,9-diaza-1(2,1)-azetidina-4(1,3)-benzenacyclotridecaphane-11-yl)acetamide |
| **261** | N-((7S,10S)-3⁴-methyl-6,9,12-trioxo-7-phenethyl-2-oxa-5,8-diaza-1(3,1)-pyrrolidina-3(1,3)-benzenacyclododecaphane-10-yl)acetamide |
| **265** | N-((8S,11S)-1²,4⁴-dimethyl-7,10,14-trioxo-8-phenethyl-3-oxa-6,9-diaza-1(2,1)-azetidina-4(1,3)-benzenacyclotetradecaphane-11-yl)acetamide |
| **269** | N-((8S,11S)-1²,4⁴-dimethyl-7,10,14-trioxo-8-phenethyl-3-oxa-6,9-diaza-1(2,1)-azetidina-4(1,3)-benzenacyclotetradecaphane-11-yl)acetamide |
| **280** | N-((11S,14S)-1⁴,6-dimethyl-7,12,15-trioxo-14-phenethyl-2-oxa-6,13,16-triaza-1(1,3)-benzenacycloheptadecaphane-11-yl)acetamide |
| **281** | N-((8S,11S)-1²,4⁴-dimethyl-7,10,15-trioxo-8-phenethyl-3-oxa-6,9-diaza-1(2,1)-azetidina-4(1,3)-benzenacyclopentadecaphane-11-yl)acetamide |
| **282** | N-((11S,14S)-1⁴,7-dimethyl-8,12,15-trioxo-14-phenethyl-2-oxa-7,13,16-triaza-1(1,3)-benzenacycloheptadecaphane-11-yl)acetamide |
| **283** | benzyl ((7S,10S)-3⁴-methyl-6,9,14-trioxo-7-phenethyl-2-oxa-5,8-diaza-15(3,1)-piperidina-3(1,3)-benzena-1(1,3)-cyclobutanapentadecaphane-10-yl)carbamate |
| **284** | 3-hydroxy-2,2-dimethyl-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)propanamide |
| **285** | 2-ethyl-2-(hydroxymethyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)butanamide |
| **286** | 3-hydroxy-2-(hydroxymethyl)-2-methyl-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)propanamide |
| **287** | 3-methyl-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)oxetane-3-carboxamide |
| **288** | 4-methyl-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)tetrahydro-2H-pyran-4-carboxamide |
| **289** | benzyl ((13R,9R,12S)-5⁴-methyl-8,11,14-trioxo-9-phenethyl-4-oxa-7,10-diaza-1(3,1)-piperidina-5(1,3)-benzenacyclotetradecaphane-12-yl)carbamate |
| **290** | benzyl ((13R,9R,12R)-5⁴-methyl-8,11,14-trioxo-9-phenethyl-4-oxa-7,10-diaza-1(3,1)-piperidina-5(1,3)-benzenacyclotetradecaphane-12-yl)carbamate |
| **291** | benzyl ((13R,9S,12R)-5⁴-methyl-8,11,14-trioxo-9-phenethyl-4-oxa-7,10-diaza-1(3,1)-piperidina-5(1,3)-benzenacyclotetradecaphane-12-yl)carbamate |
| **292** | benzyl ((13R,9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamate |
| **293** | (3-methyloxetan-3-yl)methyl ((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamate |
| **294** | 2-cyano-2-methyl-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)propanamide |
| **295** | N-(1⁴,7-dimethyl-8,13,16-trioxo-15-phenethyl-2-oxa-7,14,17-triaza-1(1,3)-benzenacyclooctadecaphane-12-yl)acetamide |
| **296** | N-(1⁴,7-dimethyl-8,11,14-trioxo-13-phenethyl-2-oxa-7,12,15-triaza-1(1,3)-benzenacyclohexadecaphane-10-yl)acetamide |
| **298** | N-(3⁴-methyl-6,9,12-trioxo-7-phenethyl-2-oxa-13,5,8-triaza-1(6,3)-bicyclo[3.2.0]heptana-3(1,3)-benzenacyclododecaphane-10-yl)acetamide |
| **299** | N-(3⁴-methyl-6,9,13-trioxo-7-phenethyl-2-oxa-13,5,8-triaza-1(6,3)-bicyclo[3.2.0]heptana-3(1,3)-benzenacyclotridecaphane-10-yl)acetamide |
| **300** | benzyl ((7S,10S)-3⁴-methyl-6,9,12-trioxo-7-phenethyl-2-oxa-5,8-diaza-13(3,1)-piperidina-3(1,3)-benzena-1(1,3)-cyclobutanatridecaphane-1 0-yl)carbamate |
| **301** | benzyl ((7S,10S)-3⁴-methyl-6,9,13-trioxo-7 -phenethyl-2-oxa-5,8-diaza-14(3,1)-piperidina-3(1,3)-benzena-1(1,3)-cyclobutanatetradecaphane-1 0-yl)carbamate |
| **302** | 2-(2-aminothiazol-4-yl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **303** | 2-(4-amino-3-methylphenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **304** | 2-(4-amino-2-methylphenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **305** | 1-(4-aminophenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)cyclopentane-1 -carboxamide |
| **306** | 1-(4-aminophenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)cyclobutane-1-carboxamide |
| **307** | 1-(4-aminophenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)cyclopropane-1-carboxamide |
| **308** | 2-(4-aminophenyl)-2-methyl-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)propanamide |
| **309** | 2-(4-aminophenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)propanamide |
| **310** | 2-(5-amino-1,3,4-thiadiazol-2-yl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **311** | 2-(2-aminothiazol-5-yl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **312** | benzyl ((8S,11S)-1⁴,5-dimethyl-6,9,12-trioxo-11-phenethyl-2-oxa-5,10,13-triaza-1(1,3)-benzenacyclotetradecaphane-8-yl)carbamate |
| **313** | benzyl ((9S,12S)-5⁴-methyl-8,11,14-trioxo-9-phenethyl-11,12,13,14-tetrahydro-4-oxa-7,10-diaza-1(3,1)-quinolina-5(1,3)-benzenacyclotetradecaphane-12-yl)carbamate |
| **314** | benzyl ((9S,12S)-5⁴-methyl-8,11,14-trioxo-9-phenethyl-11,12,13,14,14a,15,16,17,18,18a-decahydro-4-oxa-7,10-diaza-1(3,1)-quinolina-5(1,3)-benzenacyclotetradecaphane-12-yl)carbamate |
| **315** | benzyl ((9S,12S)-1⁶,5⁴-dimethyl-8,11,14-trioxo-9-phenethyl-4-oxa-7,10-diaza-1(3,1)-piperidina-5(1,3)-benzenacyclotetradecaphane-12-yl)carbamate |
| **316** | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-1,2,3,4-tetrahydroisoquinoline-5-carboxamide |
| **317** | 5-amino-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2,3-dihydro-1H-indene-1-carboxamide |
| **318** | 2-(4-amino-2-fluorophenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **319** | 3-amino-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)isoquinoline-8-carboxamide |
| **320** | 2-(4-amino-3,5-dichlorophenyl)-N-((9S,12S)-54-methyl-8,11,14-trioxo-9-phenethyl-4-oxa-7,10-diaza-1(3,1)-piperidina-5(1,3)-benzenacyclotetradecaphane-12-yl)acetamide |
| **321** | benzyl ((9S,12S)-5⁴-methyl-8,11,14-trioxo-9-phenethyl-4-oxa-7,10-diaza-1(3,1)-piperidina-5(1,3)-naphthalenacyclotetradecaphane-12-yl)carbamate |
| **322** | benzyl ((12S,15S)-1⁴-methyl-8,13,16-trioxo-15-phenethyl-2-oxa-7,14,17-triaza-1(1,3)-benzenacyclooctadecaphane-12-yl)carbamate |
| **323** | 2-(4-acetamidophenyl)-2-methoxy-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **324** | 2-(4-acetamidophenyl)-2-ethoxy-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **325** | benzyl ((9S,12S)-5⁴-methyl-8,11,14-trioxo-9-phenethyl-4-oxa-7,10-diaza-1(3,1)-pyrrolidina-5(1,3)-benzenacyclotetradecaphane-12-yl)carbamate |
| **326** | 2-(4-aminophenyl)-2-methoxy-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **327** | N-((9S,12R)-5⁴-methyl-8,11,14-trioxo-9-phenethyl-4-oxa-7,10-diaza-1(3,1)-piperidina-5(1,3)-benzenacyclotetradecaphane-12-yl)-2-(3-(pyridin-3-yl)-1H-1,2,4-triazol-5-yl)acetamide |
| **328** | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-(3-phenyl-1H-1,2,4-triazol-5-yl)acetamide |
| **329** | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-(3-(pyridin-4-yl)-1H-1,2,4-triazol-5-yl)acetamide |
| **330** | 2-(imidazo[2,1-b]thiazol-6-yl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **331** | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-(pyridin-2-yl)acetamide |
| **332** | 2-(3-methyl-1H-1,2,4-triazol-5-yl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **333** | 2-(2-fluorophenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **334** | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-(5-oxo-1-phenyl-4,5-dihydro-1H-pyrazol-3-yl)acetamide |
| **335** | 2-(5-hydroxyisoxazol-3-yl)-N-((9S,12S)-54-methyl-8,11,14-trioxo-9-phenethyl-4-oxa-7,10-diaza-1(3,1)-piperidina-5(1,3)-benzenacyclotetradecaphane-12-yl)acetamide |
| **336** | benzyl ((12S)-5⁴-methyl-8,11,14-trioxo-9-(2-(pyridin-4-yl)ethyl)-4-oxa-7,10-diaza-1(3,1)-piperidina-5(1,3)-benzenacyclotetradecaphane-12-yl)carbamate |
| **337** | benzyl ((9S,12S)-1⁵,5⁴-dimethyl-8,11,14-trioxo-9-phenethyl-4-oxa-7,10-diaza-1(3,1)-piperidina-5(1,3)-benzenacyclotetradecaphane-12-yl)carbamate |
| **338** | N-((12S)-9-(2-(1-acetylpiperidin-4-yl)ethyl)-5⁴-methyl-8,11,14-trioxo-4-oxa-7,10-diaza-1(3,1)-piperidina-5(1,3)-benzenacyclotetradecaphane-12-yl)acetamide |
| **339** | 6-amino-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-naphthamide |
| **340** | 2-(5-amino-1H-1,2,4-triazol-3-yl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **341** | 2-(4-acetylphenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **342** | 2-(5-amino-1,3,4-thiadiazol-2-yl)-N-((12S)-5⁴-methyl-8,11,14-trioxo-9-(2-(pyridin-4-yl)ethyl)-4-oxa-7,10-diaza-1(3,1)-piperidina-5(1,3)-benzenacyclotetradecaphane-12-yl)acetamide |
| **343** | 1-(4-aminophenyl)-N-((12S)-5⁴-methyl-8,11,14-trioxo-9-(2-(pyridin-4-yl)ethyl)-4-oxa-7,10-diaza-1(3,1)-piperidina-5(1,3)-benzenacyclotetradecaphane-12-yl)cyclopropane-1-carboxamide |
| **344** | 2-(4-amino-2-fluorophenyl)-N-((12S)-5⁴-methyl-8,11,14-trioxo-9-(2-(pyridin-4-yl)ethyl)-4-oxa-7,10-diaza-1(3,1)-piperidina-5(1,3)-benzenacyclotetradecaphane-12-yl)acetamide |
| **345** | 2-(4-aminophenyl)-2-ethoxy-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **347** | benzyl ((13S,16S)-1⁴-methyl-8,14,17-trioxo-16-phenethyl-2-oxa-7,15,18-triaza-1(1,3)-benzenacyclononadecaphane-13-yl)carbamate |
| **348** | 3-mercapto-2,2-dimethyl-N-((9S,12S)-54-methyl-8,11,14-trioxo-9-phenethyl-4-oxa-7,10-diaza-1(3,1)-piperidina-5(1,3)-benzenacyclotetradecaphane-12-yl)propanamide |
| **349** | benzyl ((8S,11S)-4⁴-methyl-7,10,14-trioxo-8-phenethyl-3-oxa-6,9-diaza-1(3,1)-pyrrolidina-4(1,3)-benzenacyclotetradecaphane-11-yl)carbamate |
| **350** | benzyl ((9S, 12S)-54-methyl-8,11,15-trioxo-9-phenethyl-4-oxa-7,10-diaza-1(3,1)-pyrrolidina-5(1,3)-benzenacyclopentadecaphane-12-yl)carbamate |
| **351** | benzyl ((8S,11S)-4⁴-methyl-7,10,15-trioxo-8-phenethyl-3-oxa-6,9-diaza-1(3,1)-pyrrolidina-4(1,3)-benzenacyclopentadecaphane-11-yl)carbamate |
| **352** | benzyl ((9S, 12S)-5⁴-methyl-8,11,16-trioxo-9-phenethyl-4-oxa-7,10-diaza-1(3,1)-pyrrolidina-5(1,3)-benzenacyclohexadecaphane-12-yl)carbamate |
| **353** | benzyl ((9S,12S)-5⁴-methyl-8,11,17-trioxo-9-phenethyl-4-oxa-7,10-diaza-1(3,1)-azetidina-5(1,3)-benzenacycloheptadecaphane-12-yl)carbamate |
| **354** | benzyl ((12S,15S)-1⁴,1⁵-dimethyl-8,13,16-trioxo-15-phenethyl-2-oxa-7,14,17-triaza-1(1,3)-benzenacyclooctadecaphane-12-yl)carbamate |
| **355** | 5⁴,5⁵-dimethyl-9-(1-methyl-1H-pyrazol-4-yl)-4-oxa-7,10-diaza-1(3,1)-piperidina-5(1,3)-benzenacyclotetradecaphane-8,11,14-trione |
| **356** | 5⁴-methyl-9-(1-methyl-1H-pyrazol-4-yl)-4-oxa-7,10-diaza-1(3,1)-piperidina-5(1,3)-benzenacyclohexadecaphane-8,11,16-trione |
| **358** | N-((12S)-5⁴-methyl-9-(1-methyl-1H-pyrazol-4-yl)-8,11,14-trioxo-4-oxa-7,10-diaza-1(3,1)-piperidina-5(1,3)-benzenacyclotetradecaphane-12-yl)-2-(pyrrolidin-1-yl)acetamide |
| **359** | (2S)-N-((12S)-5⁴-methyl-9-(1-methyl-1H-pyrazol-4-yl)-8,11,14-trioxo-4-oxa-7,10-diaza-1(3,1)-piperidina-5(1,3)-benzenacyclotetradecaphane-12-yl)-2-phenylpropanamide |
| **360** | N-((12S)-5⁴-methyl-9-(1-methyl-1H-pyrazol-4-yl)-8,11,14-trioxo-4-oxa-7,10-diaza-1(3,1)-piperidina-5(1,3)-benzenacyclotetradecaphane-12-yl)-1,2,3,4-tetrahydronaphthalene-1-carboxamide |
| **361** | (2R)-2-methoxy-N-((12S)-5⁴-methyl-9-(1-methyl-1H-pyrazol-4-yl)-8,11,14-trioxo-4-oxa-7,10-diaza-1(3,1)-piperidina-5(1,3)-benzenacyclotetradecaphane-12-yl)-2-phenylacetamide |
| **362** | 2-(3-methoxyphenyl)-N-((12S)-5⁴-methyl-9-(1-methyl-1H-pyrazol-4-yl)-8,11,14-trioxo-4-oxa-7,10-diaza-1(3,1)-piperidina-5(1,3)-benzenacyclotetradecaphane-12-yl)acetamide |
| **363** | Ethyl ((12S)-5⁴-methyl-9-(1-methyl-1H-pyrazol-4-yl)-8,11,14-trioxo-4-oxa-7,10-diaza-1(3,1)-piperidina-5(1,3)-benzenacyclotetradecaphane-12-yl)carbamate |
| **364** | N-((12S,15S)-1⁴-methyl-8,13,16-trioxo-15-phenethyl-2-oxa-7,14,17-triaza-1(1,3)-benzenacyclooctadecaphane-12-yl)acetamide |
| **369** | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-(quinolin-6-yl)propanamide |
| **370** | benzyl ((9S)-54-methyl-8,11,16-trioxo-9-phenethyl-4-oxa-7,10-diaza-1(3,1)-piperidina-5(1,3)-benzenacyclohexadecaphane-13-yl)carbamate |
| **371** | 1-(4-aminophenyl)-N-((9S,12S)-5⁴-methyl-8,11,14-trioxo-9-(2-(pyrazin-2-yl)ethyl)-4-oxa-7,10-diaza-1(3,1)-piperidina-5(1,3)-benzenacyclotetradecaphane-12-yl)cyclopropane-1-carboxamide |
| **372** | 2-(4-amino-2-fluorophenyl)-N-((9S,12S)-5⁴-methyl-8,11,14-trioxo-9-(2-(pyrazin-2-yl)ethyl)-4-oxa-7,10-diaza-1(3,1)-piperidina-5(1,3)-benzenacyclotetradecaphane-12-yl)acetamide |
| **373** | N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)-2-(o-tolyl)acetamide |
| **387** | tert-butyl (4-(2-(((9S,12S)-54-methyl-8,11,14-trioxo-9-phenethyl-4-oxa-7,10-diaza-1(3,1)-piperidina-5(1,3)-benzenacyclotetradecaphane-12-yl)amino)-2-oxoethyl)thiazol-2-yl)carbamate |
| **388** | tert-butyl (2-methyl-4-(2-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)amino)-2-oxoethyl)phenyl)carbamate |
| **389** | tert-butyl (3-methyl-4-(2-(((9S,12S)-54-methyl-8,11,14-trioxo-9-phenethyl-4-oxa-7,10-diaza-1(3,1)-piperidina-5(1,3)-benzenacyclotetradecaphane-12-yl)amino)-2-oxoethyl)phenyl)carbamate |
| **390** | tert-butyl (4-(1-(((9S,12S)-54-methyl-8,11,14-trioxo-9-phenethyl-4-oxa-7,10-diaza-1 (3,1)-piperidina-5(1,3)-benzenacyclotetradecaphane-12-yl)carbamoyl)cyclopentyl)phenyl)carbamate |
| **391** | tert-butyl (4-(1-(((9S,12S)-5⁴-methyl-8,11,14-trioxo-9-phenethyl-4-oxa-7,10-diaza-1 (3,1)-piperidina-5(1,3)-benzenacyclotetradecaphane-12-yl)carbamoyl)cyclobutyl)phenyl)carbamate |
| **392** | tert-butyl (4-(1-(((9S,12S)-5⁴-methyl-8,11,14-trioxo-9-phenethyl-4-oxa-7,10-diaza-1 (3,1)-piperidina-5(1,3)-benzenacyclotetradecaphane-12-yl)carbamoyl)cyclopropyl)phenyl)carbamate |
| **393** | tert-butyl (4-(2-methyl-1-(((9S,12S)-54-methyl-8,11,14-trioxo-9-phenethyl-4-oxa-7,10-diaza-1(3,1)-piperidina-5(1,3)-benzenacyclotetradecaphane-12-yl)amino)-1-oxopropan-2-yl)phenyl)carbamate |
| **394** | 2-(4-aminophenyl)-N-((9S,12S)-5⁴-methyl-8,11,14-trioxo-9-phenethyl-4-oxa-7,10-diaza-1(3,1)-piperidina-5(1,3)-benzenacyclotetradecaphane-12-yl)propanamide |
| **395** | tert-butyl (5-(2-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)amino)-2-oxoethyl)-1,3,4-thiadiazol-2-yl)carbamate |
| **396** | tert-butyl (5-(2-(((9S,12S)-5⁴-methyl-8,11,14-trioxo-9-phenethyl-4-oxa-7,10-diaza-1 (3,1)-piperidina-5(1,3)-benzenacyclotetradecaphane-12-yl)amino)-2-oxoethyl)thiazol-2-yl)carbamate |
| **402** | benzyl ((9S, 12S)-1⁴,5-dimethyl-6,10,13-trioxo-12-phenethyl-2-oxa-5,11,14-triaza-1(1,3)-benzenacyclopentadecaphane-9-yl)carbamate |
| **405** | benzyl ((10S,13S)-1⁴,5-dimethyl-6,11,14-trioxo-13-phenethyl-2-oxa-5,12,15-triaza-1(1,3)-benzenacyclohexadecaphane-10-yl)carbamate |
| **419** | tert-butyl 8-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)-3,4-dihydroisoquinoline-2(1H)-carboxylat |
| **420** | tert-butyl 8-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)-3,4-dihydroisoquinoline-2(1H)-carboxylat |
| **421** | tert-butyl 8-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)-3,4-dihydroisoquinoline-2(1H)-carboxylat |
| **422** | tert-butyl 8-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)-3,4-dihydroisoquinoline-2(1H)-carboxylat |
| **423** | tert-butyl (2,6-dichloro-4-(2-(((9S, 12S)-5⁴-methyl-8,11, 14-trioxo-9-phenethyl-4-oxa-7,10-diaza-1(3,1)-piperidina-5(1,3)-benzenacyclotetradecaphane-12-yl)amino)-2-oxoethyl)phenyl)carbamate |
| **429** | tert-butyl (4-(1-methoxy-2-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)amino)-2-oxoethyl)phenyl)carbamate |
| **437** | ((12S)-12-amino-5⁴-methyl-8,11,14-trioxo-9-(2-(pyridin-4-yl)ethyl)-4-oxa-7,10-diaza-1(3,1)-piperidina-5(1,3)-benzenacyclotetradecaphane |
| **438** | N-((12S)-12-amino-9-(2-(1-acetylpiperidin-4-yl)ethyl)-54-methyl-8,11,14-trioxo-4-oxa-7,10-diaza-1(3,1)-piperidina-5(1,3)-benzenacyclotetradecaphane |
| **439** | tert-butyl (5-(2-(((12S)-5⁴-methyl-8,11,14-trioxo-9-(2-(pyridin-4-yl)ethyl)-4-oxa-7,10-diaza-1(3,1)-piperidina-5(1,3)-benzenacyclotetradecaphane-12-yl)amino)-2-oxoethyl)-1,3,4-thiadiazol-2-yl)carbamate |
| **440** | tert-butyl (6-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)carbamoyl)naphthalen-2-yl)carbamate |
| **441** | tert-butyl (5-(2-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)amino)-2-oxoethyl)-4H-1,2,4-triazol-3-yl)carbamate |
| **442** | tert-butyl (4-(1-ethoxy-2-(((9S,12S)-5⁴-methyl-8,11,14-trioxo-9-phenethyl-4-oxa-7,10-diaza-1(3,1)-piperidina-5(1,3)-benzenacyclotetradecaphane-12-yl)amino)-2-oxoethyl)phenyl)carbamate |
| **443** | tert-butyl (4-(1-(((12S)-5⁴-methyl-8,11,14-trioxo-9-(2-(pyridin-4-yl)ethyl)-4-oxa-7,10-diaza-1(3,1)-piperidina-5(1,3)-benzenacyclotetradecaphane-12-yl)carbamoyl)cyclopropyl)phenyl)carbamate |
| **444** | tert-butyl (3-fluoro-4-(2-(((12S)-5⁴-methyl-8,11,14-trioxo-9-(2-(pyridin-4-yl)ethyl)-4-oxa-7,10-diaza-1(3,1)-piperidina-5(1,3)-benzenacyclotetradecaphane-12-yl)amino)-2-oxoethyl)phenyl)carbamate |
| **479** | benzyl ((12S)-5⁴-methyl-9-(1-methyl-1H-pyrazol-4-yl)-8,11,14-trioxo-4-oxa-7,10-diaza-1(3,1)-piperidina-5(1,3)-benzenacyclotetradecaphane-12-yl)carbamate |
| **480** | (12S)-12-amino-5⁴-methyl-9-(1-methyl-1H-pyrazol-4-yl)-4-oxa-7,10-diaza-1(3,1)-piperidina-5(1,3)-benzenacyclotetradecaphane-8,11,14-trione |
| **495** | 5⁴-methyl-9-phenethyl-4-oxa-7,10-diaza-1(3,1)-piperidina-5(1,3)-benzenacyclotetradecaphane-8,11,14-trione |
| **511** | benzyl ((12S)-9-(2-(1,5-naphthyridin-3-yl)ethyl)-5⁴-methyl-8,11,14-trioxo-4-oxa-7,10-diaza-1(3,1)-piperidina-5(1,3)-benzenacyclotetradecaphane-12-yl)carbamate |
| **503** | benzyl ((12S)-9-(2-(1,5-naphthyridin-3-yl)ethyl)-5⁴-methyl-8,11,14-trioxo-4-oxa-7,10-diaza-1(3,1)-piperidina-5(1,3)-benzenacyclotetradecaphane-12-yl)carbamate |
| **505** | benzyl ((12S)-5⁴-methyl-8,11,14-trioxo-9-(2-(pyrido[2,3-b]pyrazin-7-yl)ethyl)-4-oxa-7,10-diaza-1(3,1)-piperidina-5(1,3)-benzenacyclotetradecaphane-12-yl)carbamate |
| **509** | 4-(2-((12S)-12-(((benzyloxy)carbonyl)amino)-5⁴-methyl-8,11,14-trioxo-4-oxa-7,10-diaza-1(3,1)-piperidina-5(1,3)-benzenacyclotetradecaphane-9-yl)ethyl)benzenesulfonic acid |
| **510** | benzyl ((12S)-5⁴-methyl-9-(4-(3-methyloxetan-3-yl)phenethyl)-8,11,14-trioxo-4-oxa-7,10-diaza-1(3,1)-piperidina-5(1,3)-benzenacyclotetradecaphane-12-yl)carbamate |
| **512** | benzyl ((12S)-5⁴-methyl-9-(2-methyloxazol-4-yl)-8,11,14-trioxo-4-oxa-7,10-diaza-1(3,1)-piperidina-5(1,3)-benzenacyclotetradecaphane-12-yl)carbamate |
| **513** | methyl (((12R)-12-(((benzyloxy)carbonyl)amino)-5⁴-methyl-8,11,14-trioxo-4-oxa-7,10-diaza-1(3,1)-piperidina-5(1,3)-benzenacyclotetradecaphane-9-yl)methyl)(phenyl)carbamate |
| **549** | N-((12S)-5⁴-methyl-8,11,14-trioxo-9-(2-(pyridin-3-yl)ethyl)-4-oxa-7,10-diaza-1(3,1)-piperidina-5(1,3)-benzenacyclotetradecaphane-12-yl)acetamide |
| **550** | N-((12S)-5⁴-methyl-8,11,14-trioxo-9-phenethyl-4-oxa-7,10-diaza-1(3,1)-piperidina-5(1,3)-benzenacyclotetradecaphane-12-yl)acetamide |
| **551** | N-((12S)-5⁴-methyl-8,11, 14-trioxo-9-(2-(pyridin-4-yl)ethyl)-4-oxa-7, 10-diaza-1(3,1)-piperidina-5(1,3)-benzenacyclotetradecaphane-12-yl)acetamide |
| **552** | N-((12S)-5⁴-methyl-9-(1-methyl-1H-pyrazol-4-yl)-8,11,14-trioxo-4-oxa-7,10-diaza-1(3,1)-piperidina-5(1,3)-benzenacyclotetradecaphane-12-yl)acetamide |
| **553** | N-((12S)-5⁴-methyl-9-(2-methyloxazol-4-yl)-8,11,14-trioxo-4-oxa-7,10-diaza-1(3,1)-piperidina-5(1,3)-benzenacyclotetradecaphane-12-yl)acetamide |
| **554** | N-((12S)-5⁴-methyl-8,11,14-trioxo-9-(quinolin-6-yl)-4-oxa-7,10-diaza-1(3,1)-piperidina-5(1,3)-benzenacyclotetradecaphane-12-yl)acetamide |
| **555** | N-((12S)-5⁴-methyl-8,11,14-trioxo-9-((2-oxobenzo[d]oxazol-3(2H)-yl)methyl)-4-oxa-7,10-diaza-1(3,1)-piperidina-5(1,3)-benzenacyclotetradecaphane-12-yl)acetamide |
| **556** | N-((12S)-5⁴-methyl-9-(5-methylisothiazol-3-yl)-8,11,14-trioxo-4-oxa-7,10-diaza-1(3,1)-piperidina-5(1,3)-benzenacyclotetradecaphane-12-yl)acetamide |
| **557** | N-((12S)-5⁴-methyl-9-(1-methyl-1H-imidazol-4-yl)-8,11,14-trioxo-4-oxa-7,10-diaza-1(3,1)-piperidina-5(1,3)-benzenacyclotetradecaphane-12-yl)acetamide |
| **558** | N-((12S)-9-benzyl-5⁴-methyl-8,11,14-trioxo-4-oxa-7,10-diaza-1(3,1)-piperidina-5(1,3)-benzenacyclotetradecaphane-12-yl)acetamide |
| **559** | N-((12S)-5⁴-methyl-8,11,14-trioxo-9-(1-phenyl-1H-pyrazol-4-yl)-4-oxa-7,10-diaza-1(3,1)-piperidina-5(1,3)-benzenacyclotetradecaphane-12-yl)acetamide |
| **560** | methyl (((12S)-12-acetamido-5⁴-methyl-8,11,14-trioxo-4-oxa-7,10-diaza-1(3,1)-piperidina-5(1,3)-benzenacyclotetradecaphane-9-yl)methyl)(phenyl)carbamate |
| **561** | N-((12S)-9-(1,5-dimethyl-1H-pyrazol-3-yl)-5⁴-methyl-8,11,14-trioxo-4-oxa-7,10-diaza-1(3,1)-piperidina-5(1,3)-benzenacyclotetradecaphane-12-yl)acetamide |
| **562** | N-((12S)-5⁴-methyl-8,11,14-trioxo-9-(1H-pyrazol-4-yl)-4-oxa-7,10-diaza-1(3,1)-piperidina-5(1,3)-benzenacyclotetradecaphane-12-yl)acetamide |
| **563** | N-((12S)-5⁴-methyl-8,11, 14-trioxo-9-(2-(thiazol-2-yl)ethyl)-4-oxa-7, 10-diaza-1(3,1)-piperidina-5(1,3)-benzenacyclotetradecaphane-12-yl)acetamide |
| **564** | N-((12S)-5⁴-methyl-8,11,14-trioxo-9-(2-(pyrazin-2-yl)ethyl)-4-oxa-7,10-diaza-1(3,1)-piperidina-5(1,3)-benzenacyclotetradecaphane-12-yl)acetamide |
| **565** | N-((12S)-9-(1-benzyl-1H-pyrazol-4-yl)-5⁴-methyl-8,11,14-trioxo-4-oxa-7,10-diaza-1(3,1)-piperidina-5(1,3)-benzenacyclotetradecaphane-12-yl)acetamide |
| **566** | N-((12S)-9-(1-(2-(2-(2-(2-aminoethoxy)ethoxy)ethoxy)ethyl)-1H-pyrazol-4-yl)-5⁴-methyl-8,11,14-trioxo-4-oxa-7,10-diaza-1(3,1)-piperidina-5(1,3)-benzenacyclotetradecaphane-12-yl)acetamide |
| **567** | 3-(4-((12S)-12-acetamido-5⁴-methyl-8,11,14-trioxo-4-oxa-7,10-diaza-1(3,1)-piperidina-5(1,3)-benzenacyclotetradecaphane-9-yl)-1H-pyrazol-1-yl)propanoic acid |
| **568** | 4-(4-((12S)-12-acetamido-5⁴-methyl-8,11,14-trioxo-4-oxa-7,10-diaza-1(3,1)-piperidina-5(1,3)-benzenacyclotetradecaphane-9-yl)-1H-pyrazol-1-yl)butanoic acid |
| **570** | N-((12S)-9-(1-(3-mercaptopropyl)-1H-pyrazol-4-yl)-5⁴-methyl-8,11,14-trioxo-4-oxa-7,10-diaza-1(3,1)-piperidina-5(1,3)-benzenacyclotetradecaphane-12-yl)acetamide |
| **571** | 3-(4-((12S)-12-acetamido-5⁴-methyl-8,11,14-trioxo-4-oxa-7,10-diaza-1(3,1)-piperidina-5(1,3)-benzenacyclotetradecaphane-9-yl)-1H-pyrazol-1-yl)propane-1-sulfonic acid |
| **572** | N-(4-((12S)-12-acetamido-5⁴-methyl-8,11,14-trioxo-4-oxa-7,10-diaza-1(3,1)-piperidina-5(1,3)-benzenacyclotetradecaphane-9-yl)-1H-pyrazol-1-yl)ethane-1-sulfonic acid |
or an enantiomer, a stereoisomeric form, a mixture of enantiomers, a diastereomer, a mixture of diastereomers, a hydrate, a solvate, an acid salt form, a tautomer, a racemate of the above mentioned compounds, or a pharmaceutically acceptable salt thereof.

8. A pharmaceutical composition comprising at least one compound according to any one of the claims 1 to 7 as an active ingredient, together with at least one pharmaceutically acceptable carrier, excipient and/or diluent.

9. The compound according to any one of the claims 1 to 7 for use as a medicament.

10. The compound according to any one of the claims 1 to 7, or a pharmaceutical composition according to claim 8, for use in the prophylaxis and/or treatment of a disease associated with and/or caused by proteasome or immunoproteasome, selected from a cancer, an infectious disease, an inflammatory disease, autoimmune disease, and transplant rejection.

11. The compound for use or the pharmaceutical composition for use according to claim 10, wherein the cancer is selected from the group consisting of: adenocarcinoma, choroidal melanoma, acute leukemia, acoustic neurinoma, ampullary carcinoma, anal carcinoma, astrocytoma, basal cell carcinoma, pancreatic cancer, desmoid tumour, bladder cancer, bronchial carcinoma, non-small cell lung cancer (NSCLC), breast cancer, Burkitt's lymphoma, corpus cancer, CUP-syndrome (carcinoma of unknown primary), colorectal cancer, small intestine cancer, small intestinal tumours, ovarian cancer, endometrial carcinoma, ependymoma, epithelial cancer types, Ewing's tumours, gastrointestinal tumours, gastric cancer, gallbladder cancer, gall bladder carcinomas, uterine cancer, cervical cancer, cervix, glioblastomas, gynecologic tumours, ear, nose and throat tumours, hematologic neoplasias, hairy cell leukemia, urethral cancer, skin cancer, skin testis cancer, brain tumours (gliomas), brain metastases, testicle cancer, hypophysis tumour, carcinoids, Kaposi's sarcoma, laryngeal cancer, germ cell tumour, bone cancer, colorectal carcinoma, head and neck tumours (tumours of the ear, nose and throat area), colon carcinoma, craniopharyngiomas, oral cancer (cancer in the mouth area and on lips), cancer of the central nervous system, liver cancer, liver metastases, leukemia, eyelid tumor, lung cancer, lymph node cancer (Hodgkin's/Non-Hodgkin's), lymphomas, stomach cancer, malignant melanoma, malignant neoplasia, malignant tumours gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, esophageal carcinoma, osteolytic carcinomas and osteoplastic carcinomas, osteosarcomas, ovarial carcinoma, pancreatic carcinoma, penile cancer, plasmocytoma, squamous cell carcinoma of the head and neck (SCCHN), prostate cancer, pharyngeal cancer, rectal carcinoma, retinoblastoma, vaginal cancer, thyroid carcinoma, Schneeberger disease, esophageal cancer, spinalioms, T-cell lymphoma (mycosis fungoides), thymoma, tube carcinoma, eye tumours, urethral cancer, urologic tumours, urothelial carcinoma, vulva cancer, wart appearance, soft tissue tumours, soft tissue sarcoma, Wilm's tumour, cervical carcinoma and tongue cancer. Particularly suitable for treatment are, for example, astrocytomas, glioblastomas, pancreatic cancer, bronchial cancer, breast cancer, colorectal cancer, ovarian cancer, gastric cancer, laryngeal cancer, malignant melanoma, oesophageal cancer, cervical cancer, liver cancer, bladder cancer, cholangiocarcinoma and renal cell cancer.

12. The compound for use or the pharmaceutical composition for use according to claim 10, wherein the infectious disease is selected from the group consisting of: HIV, Echinococcosis, Amebiasis (Entamoeba histolytica Infection), Angiostrongylus Infection, Anisakiasis, Anthrax, Babesiosis (Babesia Infection), Balantidium Infection (Balantidiasis), Baylisascaris Infection (Raccoon Roundworm), Bilharzia (Schistosomiasis), Blastocystis hominis Infection (Blastomycosis), Borreliosis, Botulism, Brainerd Diarrhea, Brucellosis, BSE (Bovine Spongiform Encephalopathy), Candidiasis, Capillariasis (Capillaria Infection), CFS (Chronic Fatigue Syndrome), Chagas Disease (American Trypanosomiasis), Chickenpox (Varicella-Zoster virus), Chlamydia pneumoniae Infection, Cholera, CJD (Creutzfeldt-Jakob Disease), Clonorchiasis (Clonorchis Infection), CLM (Cutaneous Larva Migrans, Hookworm Infection), Coccidioidomycosis, Conjunctivitis, Coxsackievirus A16 (Hand, Foot and Mouth Disease), Cryptococcosis, Cryptosporidium Infection (Cryptosporidiosis), Culex mosquito (Vector of West Nile Virus), Cyclosporiasis (Cyclospora Infection), Cysticercosis (Neurocysticercosis), Cytomegalovirus Infection, Dengue / Dengue Fever, Dipylidium Infection (Dog and Cat Flea Tapeworm), Ebola Virus Hemorrhagic Fever, Echinococcosis (Alveolar Hydatid Disease), Encephalitis, Entamoeba coli Infection, Entamoeba dispar Infection, Entamoeba hartmanni Infection, Entamoeba histolytica Infection (Amebiasis), Entamoeba polecki Infection, Enterobiasis (Pinworm Infection), Enterovirus Infection (non-polio), Epstein-Barr Virus Infection, Escherichia coli Infection, Foodborne Infection, Foot and mouth Disease, Fungal Dermatitis, Gastroenteritis, Group A streptococcal Disease, Group B streptococcal Disease, Hansen's Disease (Leprosy), Hantavirus Pulmonary Syndrome, Head Lice Infestation (Pediculosis), Helicobacter pylori Infection, Hematologic Disease, Hendra Virus Infection, Hepatitis (HCV, HBV), Herpes Zoster (Shingles), Human Ehrlichiosis, Human Parainfluenza Virus Infection, Influenza, Isosporiasis (Isospora Infection), Lassa Fever, Leishmaniasis, Kala-azar (Kala-azar, Leishmania Infection), Leprosy, Lice (Body lice, Head lice, Pubic lice), Lyme Disease, Malaria, Marburg Hemorrhagic Fever, Measles, Meningitis, Mosquito-borne Diseases, Mycobacterium avium Complex (MAC) Infection, Naegleria Infection, Nosocomial Infections, Nonpathogenic Intestinal Amebae Infection, Onchocerciasis (River Blindness), Opisthorciasis (Opisthorcis Infection), Parvovirus Infection, Plague, PCP (Pneumocystis carinii Pneumonia), Polio, Q Fever, Rabies, Respiratory Syncytial Virus (RSV) Infection, Rheumatic Fever, Rift Valley Fever, Rotavirus Infection, Roundworms Infection, Salmonellosis, Salmonella Enteritidis, Scabies, Shigellosis, Shingles, Sleeping Sickness, Smallpox, Streptococcal Infection, Tapeworm Infection (Taenia Infection), Tetanus, Toxic Shock Syndrome, Tuberculosis, Ulcers (Peptic Ulcer Disease), Valley Fever, Vibrio parahaemolyticus Infection, Vibrio vulnificus Infection, Viral Hemorrhagic Fever, Warts, Waterborne infectious Diseases, West Nile Virus Infection (West Nile Encephalitis), Whooping Cough, Yellow Fever.

13. The compound for use or the pharmaceutical composition for use according to claim 10, wherein the autoimmune disease is selected from the group consisting of:
Achalasia, Addison's disease, Adult Still's disease, Agammaglobulinemia, Alopecia areata, Amyloidosis, Ankylosing spondylitis, Anti-GBM/Anti-TBM nephritis, Antiphospholipid syndrome, Autoimmune angioedema, Autoimmune dysautonomia, Autoimmune encephalomyelitis, Autoimmune hepatitis, Autoimmune inner ear disease (AIED), Autoimmune myocarditis, Autoimmune oophoritis, Autoimmune orchitis, Autoimmune pancreatitis, Autoimmune retinopathy, Autoimmune urticaria, Axonal & neuronal neuropathy (AMAN), Baló disease, Behcet's disease, Benign mucosal pemphigoid, Bullous pemphigoid, Castleman disease (CD), Celiac disease, Chagas disease, Chronic inflammatory demyelinating polyneuropathy (CIDP), Chronic recurrent multifocal osteomyelitis (CRMO), Churg-Strauss Syndrome (CSS), Eosinophilic Granulomatosis (EGPA), Cicatricial pemphigoid, Cogan's syndrome, Cold agglutinin disease, Congenital heart block, Coxsackie myocarditis, CREST syndrome, Crohn's disease, Dermatitis herpetiformis, Dermatomyositis, Devic's disease (neuromyelitis optica), Discoid lupus, Dressler's syndrome, Endometriosis, Eosinophilic esophagitis (EoE), Eosinophilic fasciitis, Erythema nodosum, Essential mixed cryoglobulinemia, Evans syndrome, Fibromyalgia, Fibrosing alveolitis, Giant cell arteritis (temporal arteritis), Giant cell myocarditis, Glomerulonephritis, Goodpasture's syndrome, Granulomatosis with Polyangiitis, Graves' disease, Guillain-Barre syndrome, Hashimoto's thyroiditis, Hemolytic anemia, Henoch-Schonlein purpura (HSP), Herpes gestationis or pemphigoid gestationis (PG), Hidradenitis Suppurativa (HS) (Acne Inversa), Hypogammalglobulinemia, IgA Nephropathy, IgG4-related sclerosing disease, Immune thrombocytopenic purpura (ITP), Inclusion body myositis (IBM), Interstitial cystitis (IC), Juvenile arthritis, Juvenile diabetes (Type 1 diabetes), Juvenile myositis (JM), Kawasaki disease, Lambert-Eaton syndrome, Leukocytoclastic vasculitis, Lichen planus, Lichen sclerosus, Ligneous conjunctivitis, Linear IgA disease (LAD), Lupus, Lyme disease chronic, Meniere's disease, Microscopic polyangiitis (MPA), Mixed connective tissue disease (MCTD), Mooren's ulcer, Mucha-Habermann disease, Multifocal Motor Neuropathy (MMN) or MMNCB, Multiple sclerosis, Myasthenia gravis, Myelin Oligodendrocyte Glycoprotein Antibody Disorder, Myositis, Narcolepsy, Neonatal Lupus, Neuromyelitis optica, Neutropenia, Ocular cicatricial pemphigoid, Optic neuritis, Palindromic rheumatism (PR), PANDAS, Paraneoplastic cerebellar degeneration (PCD), Paroxysmal nocturnal hemoglobinuria (PNH), Parry Romberg syndrome, Pars planitis (peripheral uveitis), Parsonage-Turner syndrome, Pemphigus, Peripheral neuropathy, Perivenous encephalomyelitis, Pernicious anemia (PA), POEMS syndrome, Polyarteritis nodosa, Polyglandular syndromes type I, II, III, Polymyalgia rheumatic, Polymyositis, Postmyocardial infarction syndrome, Postpericardiotomy syndrome, Primary Biliary Cholangitis, Primary sclerosing cholangitis, Progesterone dermatitis, Psoriasis, Psoriatic arthritis, Pure red cell aplasia (PRCA), Pyoderma gangrenosum, Raynaud's phenomenon, Reactive Arthritis, Reflex sympathetic dystrophy, Relapsing polychondritis, Restless legs syndrome (RLS), Retroperitoneal fibrosis, Rheumatic fever, Rheumatoid arthritis, Sarcoidosis, Schmidt syndrome, Scleritis, Scleroderma, Sjögren's syndrome, Sperm & testicular autoimmunity, Stiff person syndrome (SPS), Subacute bacterial endocarditis (SBE), Susac's syndrome, Sympathetic ophthalmia (SO), Takayasu's arteritis, Temporal arteritis/Giant cell arteritis, Thrombocytopenic purpura (TTP), Thyroid eye disease (TED), Tolosa-Hunt syndrome (THS), Transverse myelitis, Type 1 diabetes, Ulcerative colitis (UC), Undifferentiated connective tissue disease (UCTD), Uveitis, Vasculitis, Vitiligo, Vogt-Koyanagi-Harada Disease.

14. A method for producing a compound of the formula (**I**) comprising:
**Step 1A:** providing an intermediate compound (**I-1***): 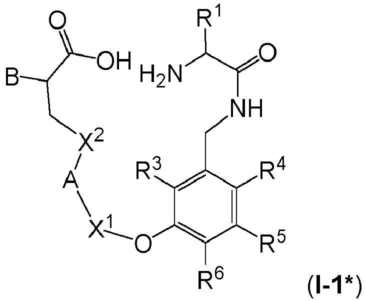 wherein **A**, **B, R¹**, **R³**, **R⁴**, **R⁵**, **R⁶**, **X¹**, and **X²** have the same meanings as defined in the formula (**I**) according to claim 1;
**Step 2A:** perform an intramolecular amide coupling reaction between a carboxylic acid group and an amine group of the intermediate compound (**I-1***) to obtain the compound of the formula (**I**) 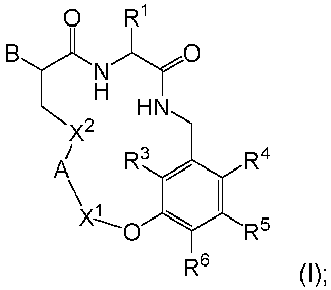 or
a method for producing the compound of the formula (**I**) comprising:
**Step 1B**: providing an intermediate compound (**I-2***): 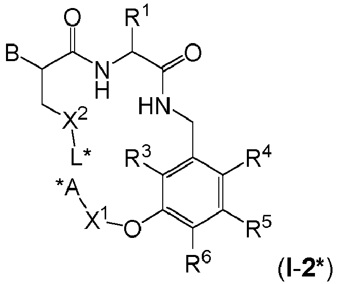 wherein
**A*** represents **-**NH(**R^{N6}**)-, 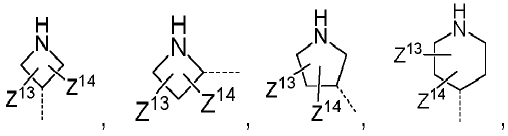 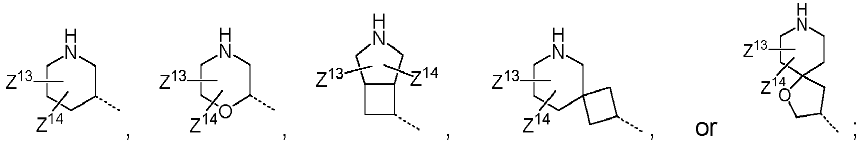
**L*** represents -CO₂H,
and **B**, **R¹**, **R²**, **R³, R⁴**, **R⁵**, **R⁶**, **R^{N6}**, **X¹**, **X²**, **Z¹³**, and **Z¹⁴** have the same meanings as defined in the formula (**I**) according to claim 1;
**Step 2B**: perform an intramolecular amide coupling reaction between the **L*** and an amino group of **A*** moiety of the intermediated compound (**I-2***) to obtain the compound of the formula (**I**);
or
a method for producing the compound of the formula (**I**) comprising:
**Step 1C:** providing an intermediate compound (**I-3***): 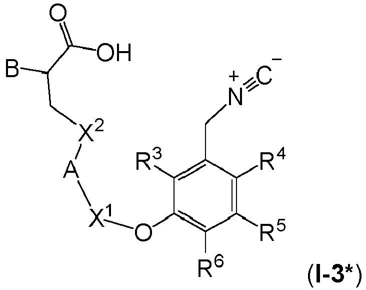 wherein
**A, B, R¹**, **R³**, **R⁴**, **R⁵**, **R⁶**, **X¹** and **X²** have the same meanings as defined in the formula (**I**) according to claim 1;
**Step 2C**: perform an intramolecular Ugi reaction of the intermediate compound (**I-3***) with **R¹**-CHO and aqueous ammonia (NH₃) to obtain the compound of the formula (**I**).

15. An intermediate compound selected from compounds **7***, **8***, **11***, **12***, **13***, **14***, **I-1***, **I-2***, and **I-3***: 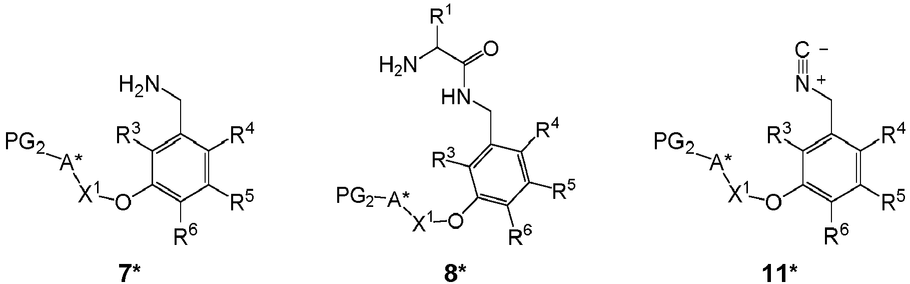 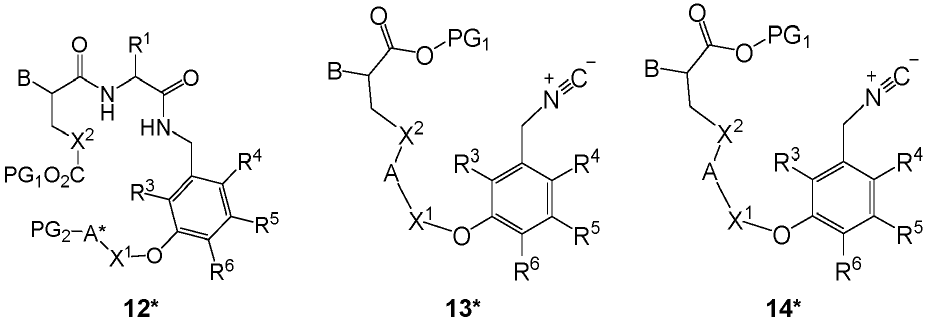 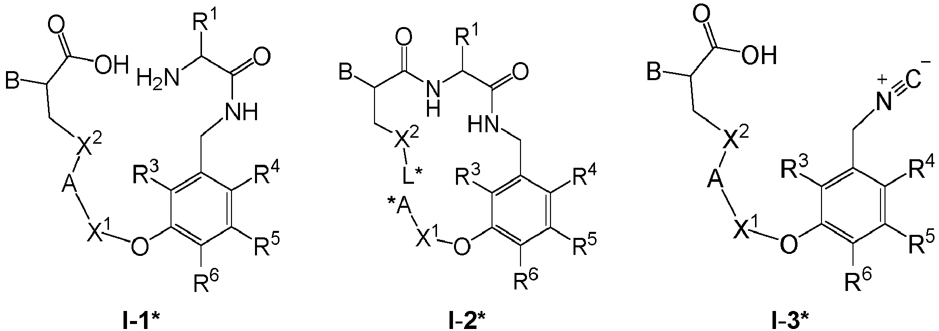 wherein
**A*** represents -NH(**R^{N6}**)-, 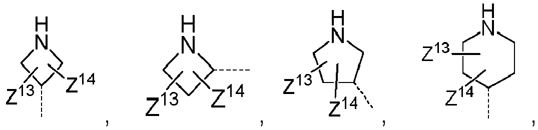 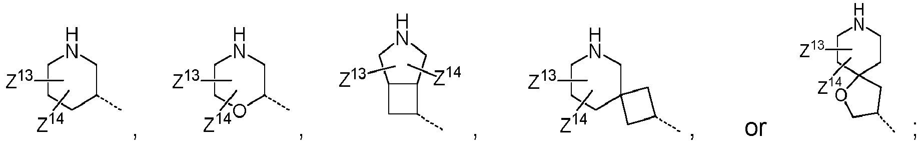
**L*** represents -CO₂H;
**PG₁** represents a carboxyl protecting group;
**PG₂** represents an amine protecting group;
and **B**, **R¹**, **R²**, **R³**, **R⁴**, **R⁵**, **R⁶**, **R^{N6}**, **X¹**, **X²**, **Z¹³**, and **Z¹⁴** have the same meanings as defined in the formula (**I**) according to claim 1.
